# EUROPÄISCHE PATENTSCHRIFT

(11) **EP 2 373 648 B1**
(45) Veröffentlichungstag und Bekanntmachung des Hinweises auf die Patenterteilung: **23.03.2016**
(21) Anmeldenummer: 09764188.0
(22) Anmeldetag: 28.11.2009
(51) Int. Cl.: A01N 43/80, C07D 417/04, C07D 417/14, A01N 43/78

(54) **SUBSTITUIERTE 1-(THIAZOLYL)- UND 1-(ISOTHIAZOLYL)PYRAZOL-4-YL-ESSIGSÄUREN, VERFAHREN ZU DEREN HERSTELLUNG UND DEREN VERWENDUNG ALS HERBIZIDE UND PFLANZENWACHSTUMSREGULATOREN**
SUBSTITUTED 1-(THIAZOLYL)- AND 1-(ISOTHIAZOLYL)PYRAZOL-4-YL ACETIC ACIDS, METHOD FOR THEIR PRODUCTION AND THEIR USE AS HERBICIDES AND PLANT GROWTH REGULATORS
ACIDES DE VINAIGRE 1-(THIAZOLYL) ET 1-(ISOTHIAZOLYL)PYRAZOLE-4-YL SUBSTITUÉS, LEUR PROCÉDÉ DE FABRICATION ET D'UTILISATION EN TANT QU'HERBICIDES ET RÉGULATEURS DE CROISSANCE DES PLANTES

(30) Priorität: 06.12.2008 EP 08021241
(43) Veröffentlichungstag der Anmeldung: 12.10.2011
(73) Patentinhaber: Bayer Intellectual Property GmbH, 40789 Monheim am Rhein (DE)
(72) Erfinder: MARTELLETTI, Arianna, 65843 Sulzbach (DE); JAKOBI, Harald, 60598 Frankfurt (DE); DITTGEN, Jan, 60316 Frankfurt (DE); HAÜSER-HAHN, Isolde, 51375 Leverkusen (DE); FEUCHT, Dieter, 65760 Eschborn (DE); ROSINGER, Christopher, Hugh, 65719 Hofheim (DE)
(74) Vertreter: BIP Patents
(86) Internationale Anmeldenummer: PCT/EP2009/008490
(87) Internationale Veröffentlichungsnummer: WO 2010/063422

(56) Entgegenhaltungen:
- EP-A- 0 822 187
- WO-A-2008/080504

## Beschreibung

Die Erfindung betrifft das technische Gebiet der Herbizide und Pflanzenwachstumsregulatoren, beispielsweise der Herbizide zur Bekämpfung von Unkräutern und Ungräsern in Nutzpflanzenkulturen oder der Pflanzenwachstumsregulatoren, welche zur Beeinflussung des Wachstums von Kulturpflanzen eingesetzt werden können.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es (a) dass sie keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen oder ein toxikologisch ungünstiges Profil besitzen. Andere Wirkstoffe, die als Pflanzenwachstumsregulatoren bei einigen Nutzpflanzen eingesetzt werden können, führen bei anderen Nutzpflanzen zu unerwünscht verminderten Ernteerträgen oder sind mit der Kulturpflanze nicht oder nur in einem engen Aufwandmengenbereich verträglich. Andere bekannte Wirkstoffe lassen sich wegen schwer zugänglicher Vorprodukte und Reagenzien im industriellen Maßstab nicht wirtschaftlich herstellen oder besitzen nur unzureichende chemische Stabilitäten.

Bei anderen Wirkstoffen hängt die Wirkung zu stark von Umweltbedingungen, wie Wetter- und Bodenverhältnissen ab.

Aus EP-A-0822187 und dort zitierter Literatur sind herbizide 3-(Hetero)Aryl-4-[(hetero)aryl-carbonyl]-pyrazolverbindungen bekannt. Speziell beschreibt EP-A-0822187 Pyrazolverbindungen, die in Position 4 einen Phenylcarbonyl-rest oder eine Heteroarylcarbonyl-gruppe Rest und in Position 5 einen ggf. substituierten Phenylrest oder Heterocyclylrest aufweisen. Die dort beschriebenen Verbindungen sind an der 1-Position (am Stickstoffatom) nicht N-substituiert. Allgemein wird in EP-A-0822187 gelehrt, dass an Position 1 auch eine abspaltbare Gruppe vorhanden sein kann, wobei beispielhaft diverse Acylgruppen genannt werden.

Aus US 4146721 sind Pyrazolylessigsäuren als Analgetika, Antipiretika und Entzündungshemmer bekannt, jedoch ist keine Anwendung als Pestizide, besonders Herbizide beschrieben.

In US 4,095,025 sind 1,3-Diaryl-pyrazol-4-yl-acrylsäure(derivate) für pharmazeutische (z.B. entzündungshemmende) Zwecke beschrieben.

WO 2004/089931 beschreibt substituierte Pyrazole mit gegebenenfalls substituierten Phenyl- oder Pyrid-3-yl-Resten am N-Atom in Position 1 des Pyrazols für die Behandlung und Prophylaxe von Krankheiten, die durch die Bindung der Verbindungen an 5 HT Rezeptoren beeinflusst werden.

WO2008/080504 beschreibt substituierte 1-(3-Pyridinyl)pyrazol-4-yl essigsäuren als Herbizide und Pflanzenwachstumsregulatoren.

Aus den genannten Gründen besteht weiterhin ein Bedarf nach alternativen wirkungsstarken Herbiziden für die selektive Anwendung in Pflanzenkulturen oder die Anwendung auf Nichtkulturland. Auch ist es wünschenswert, alternative chemische Wirkstoffe bereitzustellen, die gegebenenfalls mit Vorteilen als Herbizide oder Pflanzenwachstumsregulatoren eingesetzt werden können.

Gegenstand der vorliegenden Erfindung sind Verbindungen der Formel (I) oder deren Salze, 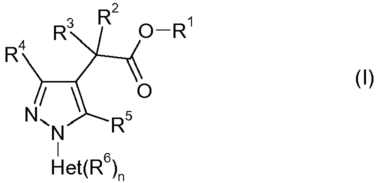 in welcher
- Het: einen fünfgliedrigen heteroaromatischen Rest mit zwei Heteroatomen als Ringatome, wobei eines der Heteroatome im Ring ein Stickstoffatom und das andere ein Schwefelatom ist und das Stickstoffatom im Ring in 1,3-Stellung zum Ring-C-atom steht, das am Pyrazolrest gebunden ist,
- R¹: Wasserstoff oder einen hydrolysierbaren Rest, vorzugsweise Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, vorzugsweise 1 bis 24 C-Atome, insbesondere 1 bis 20 C-Atome aufweist, oder
einen Rest der Formel SiR^{a}R^{b}R^{c}, -NR^{a}R^{b} oder -N=CR^{c}R^{d}, wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet oder R^{a} und R^{b} zusammen mit dem N-Atom einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten, wobei jeder der Reste R^{a}, R^{b}, R^{c} und R^{d} inklusive Substituenten bis 30 C-Atome, vorzugsweise bis 24 C-Atome, insbesondere bis 20 C-Atome aufweist,
- R²: Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist,
- R³: Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist, oder
- R² und R³: zusammen mit dem C-Atom, an das sie gebunden sind, einen carbocyclischen gesättigten oder teilweise ungesättigten Ring mit 3 bis 6 C-Atomen, der unsubstituiert oder durch einen oder mehrere Reste aus der Gruppe Halogen und (C₁-C₄)Alkyl substituiert ist, und
- R⁴: Wasserstoff, Halogen, Cyano, (C₁-C₆)Alkyl, (C₂-C₆)Alkenyl oder (C₂-C₆)Alkinyl,
wobei jeder der letztgenannten drei Reste unsubstituiert oder durch einen oder mehrere Reste aus der Gruppe bestehend aus Halogen, Cyano, Hydroxy, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₉)Cycloalkyl substituiert ist, oder
(C₃-C₉)Cycloalkyl, (C₅-C₉)Cycloalkenyl oder (C₅-C₉)Cycloalkinyl, wobei jeder der 3 letztgenannten Reste unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, oder
Phenyl, das unsubstituiert oder durch einen oder mehrere Reste aus der Gruppe bestehend aus Halogen, Cyano, Nitro, Carboxy, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₁-C₄)Alkanoyl, (C₁-C₄)Haloalkanoyl, [(C₁-C₄)Alkoxy]-carbonyl und [(C₁-C₄)Haloalkoxy]-carbonyl substituiert ist, oder
(C₁-C₆)Alkanoyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₁-C₄)Alkoxy]-carbonyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder [(C₃-C₉)Cycloalkoxy]-carbonyl, das unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist,
- R⁵: einen Phenylrest oder einen 5- oder 6-gliedrigen heteroaromatischen Rest mit 1 bis 3 Heteroringatomen aus der Gruppe N, O und S, wobei der Phenylrest oder der heterocyclische Rest unsubstituiert oder durch einen oder mehrere Reste aus der Gruppe bestehend aus den Resten
- (a): Halogen, Hydroxy, Amino, Nitro, Carboxy, Cyano und Carbamoyl,
- (b): (C₁-C₆)Alkyl, (C₂-C₆)Alkenyl, (C₂-C₆)Alkinyl, (C₁-C₆)Alkoxy, (C₁-C₆)Alkenyloxy und (C₁-C₆)Alkinyloxy, wobei jeder der letztgenannten 6 Reste unsubstituiert oder durch einen oder mehrere Reste aus der Gruppe Halogen, (C₁-C₄)Alkoxy, (C₁-C₆)Haloalkoxy, (C₁-C₄)Alkylthio, Mono- und Di-[(C₁-C₄)alkyl]-amino, Hydroxy, Carboxy, [(C₁-C₄)Alkoxy]-carbonyl, [(C₁-C₄)Haloalkoxy]-carbonyl, Mono- und Di-[(C₁-C₄)alkyl]-aminocarbonyl und Cyano substituiert ist,
- (c): (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₁-C₆)Alkylsulfinyloxy, (C₁-C₆)Haloalkylsulfinyloxy, (C₁-C₆)Alkylsulfonyloxy, (C₁-C₆)Haloalkylsulfonyloxy, (C₁-C₆)Alkylsulfato, (C₁-C₆)Haloalkylsulfato und
- (d): (C₃-C₆)Cycloalkyl, (C₃-C₆)Cycloalkyloxy, Phenyl und Phenoxy, wobei jeder der letztgenannten 4 Reste unsubstituiert oder durch einen oder mehrere Reste aus der Gruppe bestehend aus Halogen, (C₁-C₄)Alkyl, (C₁-C₄)Haloalkyl, (C₁-C₄)Alkoxy, (C₁-C₆)Haloalkoxy und (C₁-C₄)Alkylthio substituiert ist, und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₆)Haloalkoxy und (C₁-C₄)Alkylthio substituiert ist,
wobei der Phenylrest inklusive Substituenten 6 bis 30 C-Atome aufweist und der heteroaromatische Rest inklusive 1 bis 30 C-Atome aufweist, und (R⁶)ₙ
n Substituenten R⁶, wobei R⁶, im Falle dass n = 1 ist, oder jeder der Substituenten R⁶ unabhängig voneinander, im Falle dass n größer als 1 ist, einen Rest Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- oder Di-[(C₁-C₄)alkyl]-aminoalkyl, Hydroxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₁-C₆)Haloalkoxy, (C₁-C₄)Alkoxy-(C₁-C₄)alkoxy, (C₁-C₆)Alkylthio, (C₁-C₆)Haloalkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- oder Di-[(C₁-C₄)alkyl]-aminocarbonyl, Mono- oder Di-[(C₁-C₆)acyl]-amino, Mono- oder Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₉)Cycloalkyl oder (C₅-C₉)Cycloalkenyl, wobei jeder der letztgenannten beiden Reste unsubstituiert oder durch einen oder mehrere Reste aus der Gruppe bestehend aus Halogen, (C₁-C₄)Alkyl und (C₁-C₄)Haloalkyl substituiert ist, bedeutet, und
- n: 0, 1, oder 2 bedeuten.

Die Verbindungen der Formel (I) können durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium-und Kaliumsalze, oder auch Ammoniumsalze, Salze mit organischen Aminen oder quartäre (quaternäre) Ammoniumsalze.

In der Formel (I) und allen nachfolgenden Formeln werden Bezeichnungen für chemische Reste verwendet, die insbesondere die nachstehend erläuterten Bedeutungen haben.

Ein hydrolysierbarer Rest (siehe Definition von R¹) bedeutet einen unter den Anwendungsbedingungen hydrolysierbaren Rest, beispielsweise einen schon in der Spritzbrühe oder insbesondere unter den physiologischen Bedingungen in Pflanzen hydrolysierbaren Rest, wobei eine Verbindung der Formel (I) mit der Carbonsäureestergruppe -CO-OR¹ (R¹ ungleich Wasserstoff) zur Verbindung der Formel (I) mit der Carbonsäuregruppe -CO-OH (d. h. Verbindung (I) mit R¹ = H) hydrolysiert wird. In der Definition der hydrolysierbaren Reste sind auch ausdrücklich die Reste mit R¹ = Kohlenwasserstoffrest oder Heterocyclylrest, wobei die beiden letztgenannten Reste unsubstituiert oder substituiert sind, umfasst, auch wenn sie teilweise vergleichsweise langsam hydrolysierbar sind.

Ein Kohlenwasserstoffrest ist ein aliphatischer, cycloaliphatischer oder aromatischer monocyclischer oder, im Falle eines gegebenenfalls substituierten Kohlenwasserstoffrestes, auch ein bicyclischer oder polycyclischer organischer Rest auf Basis der Elemente Kohlenstoff und Wasserstoff, beispielsweise umfassend die Reste Alkyl, Alkenyl, Alkinyl, Cycloalkyl, Cycloalkenyl, Aryl, Phenyl, Naphthyl, Indanyl, Indenyl, etc.; Entsprechendes gilt für Kohlenwasserstoffreste in zusammengesetzte Bedeutungen wie Kohlenwasserstoffoxyresten oder anderen über Heteroatomgruppen gebundene Kohlenwasserstoffresten.

Wenn nicht näher definiert, weisen die Kohlenwasserstoffreste vorzugsweise 1 bis 20 C-Atome, weiter bevorzugt 1 bis 16 C-Atome, insbesondere 1 bis 12 C-Atome auf.

Die Kohlenwasserstoffreste, auch in den speziellen Resten Alkyl, Alkoxy, Haloalkyl, Haloalkoxy, Alkylamino und Alkylthio, sowie die entsprechenden ungesättigten und/oder substituierten Reste können im Kohlenstoffgerüst jeweils geradkettig oder verzweigt sein.

Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl" umfassen entsprechend auch gradkettige oder verzweigte Alkylreste mit einer größeren Zahl von C-Atomen, d. h. gemäß Beispiel auch die Alkylreste mit 5 und 6 C-Atomen. Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl. Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl; Alkinyl bedeutet z.B. Propargyl, But-2-in-1-yl, But-3-in-1-yl, 1-Methyl-but-3-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Ein 3- bis 9-gliedriger carbocyclischer Ring bedeutet (C₃-C₉)Cycloalkyl oder (C₅-C₉)Cycloalkenyl.
(C₃-C₉)Cycloalkyl bedeutet ein carbocyclisches, gesättigtes Ringsystem mit vorzugsweise 3-9 C-Atomen, z.B. Cyclopropyl, Cyclobutyl, Cyclopentyl, Cyclohexyl, Cycloheptyl, Cyclooctyl oder Cyclononyl. Im Falle von substituiertem Cycloalkyl werden cyclische Systeme mit Substituenten umfaßt, wobei die Substituenten auch mit einer Doppelbindung am Cycloalkylrest, z. B. eine Alkylidengruppe wie Methyliden, gebunden sein können.
(C₅-C₉)Cycloalkenyl bedeutet ein carbocyclisches, nicht aromatisches, partiell ungesättigtes Ringsystem mit 5-9 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Halogen bedeutet beispielsweise Fluor, Chlor, Brom oder Iod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.
Im Falle gegebenenfalls substituiertes Aryl sind auch mehrcyclische Systeme, wie Tetrahydronaphthyl, Indenyl, Indanyl, Fluorenyl, Biphenylyl, umfasst, wobei die Bindungsstelle am aromatischen System ist.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P, B, Si, Se) der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält.
Ist der Heterocyclylrest oder der heterocyclische Ring gegebenenfalls substituiert, kann er mit anderen carbocyclischen oder heterocyclischen Ringen annelliert sein. Bevorzugt sind benzokondensierte (benzoannellierte) heterocyclische bzw. heteroaromatische Ringe.
Im Falle von gegebenenfalls substituiertem Heterocyclyl werden auch mehrcyclische Systeme umfaßt, wie beispielsweise 8-Aza-bicyclo[3.2.1]octanyl oder 1-Aza-bicyclo[2.2.1]heptyl.
Im Falle von gegebenenfalls substituiertem Heterocyclyl werden auch spirocyclische Systeme umfaßt, wie beispielsweise 1-Oxa-5-aza-spiro[2.3]hexyl. Vorzugsweise ist er ein Rest eines heteroaromatischen Rings mit einem Heteroatom aus der Gruppe N, O und S, beispielsweise der Rest eines Fünf- oder Sechsrings, wie Pyridyl, Pyrrolyl, Thienyl oder Furyl;
weiterhin bevorzugt ist er ein Rest eines entsprechenden heteroaromatischen Rings mit 2, 3 oder 4 Heteroatomen, z. B. Pyrimidinyl, Pyridazinyl, Pyrazinyl, Triazinyl, Tetrazinyl, Thiazolyl, Thiadiazolyl, Oxazolyl, Isoxazolyl, Pyrazolyl, Imidazolyl oder Triazolyl oder Tetrazolyl.

Bevorzugt ist er dabei ein Rest eines heteroaromatischen Fünf- oder Sechsrings mit 1 bis 4 Heteroatomen, wie z. B. 1,2,3-Triazolyl, 1,2,4-Triazolyl, Tetrazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Tetrazolyl, 1,2,3-Triazinyl, 1,2,4-Triazinyl, 1,3,5-Triazinyl, 1,2,3,4-Tetrazinyl, 1,2,3,5-Tetrazinyl, 1,2,4,5-Tetrazinyl, Thiazolyl, Isothiazolyl, Oxazolyl, Isoxazolyl, Pyrazolyl, Imidazolyl.
Weiter bevorzugt sind dabei heteroaromatische Reste von Fünfring-Heterocyclen mit 3 N-Atomen, wie 1,2,3-Triazol-1-yl, 1,2,3-Triazol-4-yl, 1,2,3-Triazol-5-yl, 1,2,5-Triazol-1-yl, 1,2,5-Triazol-3-yl, 1,3,4-Triazol-1-yl, 1,3,4-Triazol-2-yl, 1,2,4-Triazol-3-yl, 1,2,4-Triazol-5-yl;
weiter bevorzugt sind auch heteroaromatische Reste von Sechsring-Heterocyclen mit 3 N-Atomen, wie 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl;
weiter bevorzugt sind auch heteroaromatische Reste von Fünfring-Heterocyclen mit zwei N-Atomen und einem O-Atom, wie 1,2,4-Oxadiazol-3-yl; 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl,
weiter bevorzugt sind auch heteroaromatische Reste von Fünfring-Heterocyclen mit zwei N-Atomen und einem S-Atom, wie 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl;
weiter bevorzugt sind auch heteroaromatische Reste von Fünfring-Heterocyclen mit vier N-Atomen, wie 1,2,3,4-Tetrazol-1-yl, 1,2,3,4-Tetrazol-5-yl, 1,2,3,5-Tetrazol-1-yl, 1,2,3,5-Tetrazol-4-yl, 2*H*-1,2,3,4-Tetrazol-5-yl, 1*H*-1,2,3,4-Tetrazol-5-yl, weiter bevorzugt sind auch heteroaromatische Reste von Sechsring-Heterocyclen, wie 1,2,4,5-Tetrazin-3-yl;
weiter bevorzugt sind auch heteroaromatische Reste von Fünfring-Heterocyclen mit drei N-Atomen und einem O- oder S-Atom, wie 1,2,3,4-Oxatriazol-5-yl; 1,2,3,5-Oxatriazol-4-yl; 1,2,3,4-Thiatriazol-5-yl;1,2,3,5-Thiatriazol-4-yl;
weiter bevorzugt sind auch heteroaromatische Reste von Sechsring-Heterocyclen, wie beispielsweise 1,2,4,6-Thiatriazin-1-yl; 1,2,4,6-Thiatriazin-3-yl; 1,2,4,6-Thiatriazin-5-yl.

Weiterhin bevorzugt ist der heterocyclische Rest oder Ring ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl,

Weiterhin bevorzugt ist er auch ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl.

Bevorzugte heterocyclische Reste sind auch benzokondensierte oder benzoannellierte heteroaromatische Ringe, beispielsweise Benzofuryl, Benzisofuryl, Benzothiophenyl, Benzisothiophenyl, Isobenzothiophenyl, Indolyl, Isoindolyl, Indazolyl, Benzimidazolyl, Benztriazolyl, Benzoxazolyl, 1,2-Benzisoxazolyl, 2,1-Benzisoxazolyl, Benzothiazolyl, 1,2-Benzisothiazolyl, 2,1-Benzoisothiazolyl, 1,2,3-Benzoxadiazolyl, 2,1,3-Benzoxadiazolyl, 1,2,3-Benzothiadiazolyl, 2,1,3-Benzothiadiazolyl, Chinolyl (Chinolinyl), Isochinolyl (Isochinolinyl), Chinnolinyl, Phtalazinyl, Chinazolinyl, Chinoxalinyl, Naphthyridinyl, Benzotriazinyl, Purinyl, Pteridinyl, Indolizinyl, Benzo-1,3-dioxylyl, 4H-Benzo-1,3-dioxinyl, und 4H-Benzo-1,4-dioxinyl, und wo es möglich ist, N-Oxide und Salze davon.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkyl-aminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden substituiert sind.

Der Ausdruck "Reste aus der Gruppe (gefolgt von der Gruppe = Liste der Substituenten)", wo immer verwendet, soll gleichbedeutend sein mit "Reste ausgewählt aus der Gruppe (...)" oder "Reste ausgewählt aus der Gruppe bestehend aus (...)".

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Mit "Basisrest" wird der jeweilige Grundkörper eines Restes bezeichnet, an dem Substituenten einer Substituentenebene gebunden sind.

Bevorzugte Substituenten für die Substituentenebenen sind beispielsweise Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkyl-aminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.
Zwei Substituenten auch gemeinsam eine gesättigte oder ungesättigte Kohlenwasserstoff-Brücke oder eine entsprechende Brücke, in denen C-Atome, CH-Gruppen oder CH₂-Gruppen durch Heteroatome ersetzt sind, bilden und damit einen ankondensierten oder annellierten Cyclus bilden. Bevorzugt werden dabei benzokondensierte Systeme auf Basis der Grundkörper gebildet.

Gegebenenfalls substituiertes Phenyl bedeutet vorzugsweise Phenyl oder Phenyl, das unsubstituiert oder durch einen oder mehrere Reste aus der Gruppe Halogen, Cyano, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio und Nitro substituiert ist, insbesondere Phenyl, das gegebenenfalls durch einen oder mehrere Reste aus der Gruppe Halogen, (C₁-C₄)Alkyl, (C₁-C₄)Haloalkyl und (C₁-C₄)Alkoxy substituiert ist.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren oder Phosphinsäuren.
Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.
Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.
Weiter bevorzugt bedeutet Acyl einen Alkanoylrest mit 1 bis 6 C-Atomen, insbesondere 1 bis 4 C-Atomen. (C₁-C₄)Alkanoyl bedeutet dabei den Rest einer Alkansäure mit 1 bis 4 C-Atomen nach Abtrennen der OH-Gruppe der Säuregruppe, d.h. Formyl, Acetyl, n-Propionyl, i-Propionyl oder n-, i-, sec. oder tert.-Butanoyl.

Die "yl-Position" eines Restes bezeichnet das C-Atom mit der freien Bindung. Erfindungsgemäße bzw. erfindungsgemäß verwendete Verbindungen der Formel (I) (und ggf deren Salze) werden kurz auch als "Verbindungen (I)" bezeichnet.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Gegenstand der Erfindung sind auch alle Tautomere der Verbindungen der Formel (I), die durch Verschiebung eines Wasserstoffatoms entstehen können (z. B. Keto-Enol-Tautomere). Die Tautomere sind ebenfalls von der Verbindung der Formel (I) umfasst, auch wenn die Formel (I) nur eines der jeweiligen im Gleichgewicht stehenden bzw. ineinander umwandelbaren Tautomere formal richtig beschreibt.

Die Verbindungen der Formel (I) umfassen auch alle physikalischen Formen, in denen diese in Reinsubstanz oder gegebenenfalls in Mischung mit anderen Stoffen auftreten können, insbesondere auch polymorphe Kristallformen der Verbindungen der Formel (I) oder deren Salze oder Lösungsmitteladditionsverbindungen (z. B. Hydrate).

Vor allem aus den Gründen der höheren herbiziden Wirkung, besseren Selektivität und/oder besseren Herstellbarkeit sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Unabhängig von den jeweils anderen Resten aus der Gruppe R¹, R², R³, R⁴, R⁵ und (R⁶)ₙ und der den allgemeinen Resten entsprechenden Unterbedeutungen, und vorzugsweise in Kombination mit bevorzugten Bedeutungen von einem oder mehreren dieser Reste, sind erfindungsgemäße Verbindungen bzw. erfindungsgemäße Verwendungen von Verbindungen mit nachfolgend aufgeführten bevorzugten Bedeutungen der betreffenden Reste von besonderem Interesse.

Bevorzugt sind die erfindungsgemäßen Verbindungen der Formel (I) oder deren Salze, in welcher
- R¹: Wasserstoff, Alkyl, Alkenyl, Alkinyl, Cycloalkyl, Cycloalkenyl, Cycloalkinyl oder Aryl, wobei jeder der letztgenannten 7 Reste unsubstituiert oder substituiert ist und inklusive Substituenten bis zu 30 C-Atome, vorzugsweise bis 24 C-Atome, insbesondere bis 20 C-Atome aufweist, oder einen Heterocyclylrest mit 3 bis 9 Ringatomen, der 1 bis 4 Heteroatome aus der Gruppe N, O und S enthält und der unsubstituiert oder oder substituiert ist und der inklusive Substituenten 1 bis 30 C-Atome, vorzugsweise 1 bis 24 C-Atome, insbesondere 1 bis 20 C-Atome aufweist, bedeutet.

Weiter bevorzugt sind dabei auch Verbindungen (I) oder deren Salze, worin
- R¹: Wasserstoff bedeutet.

Weiter bevorzugt sind dabei auch Verbindungen (I) oder deren Salze, worin
- R¹: H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl, wobei jeder der letztgenannten 7 Reste unsubstituiert oder substituiert ist und inklusive Substituenten bis zu 30 C-Atome, vorzugsweise bis 24 C-Atome, insbesondere bis 20 C-Atome aufweist.

Weiter bevorzugt sind dabei auch Verbindungen (I) oder deren Salze, worin
- R¹: H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, auch Carboxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, die letztgenannten 7 Reste nur im Falle cyclischer Basisreste, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, Reste der Formeln -NR*R**, -CO-NR*R** und -O-CO-NR*R**,
wobei jeder der Reste R* und R** in den letztgenannten 3 Formeln unabhängig voneinander H, (C₁-C₈)Alkyl, (C₂-C₈)Alkenyl, (C₂-C₈)Alkinyl, Benzyl, substituiertes Benzyl, Phenyl oder substituiertes Phenyl, oder zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeutet,
und [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist,
und Phenyl, Phenyl-(C₁-C₆)alkoxy, Phenyl-[(C₁-C₆)alkoxy]-carbonyl, Phenoxy, Phenoxy-(C₁-C₆)alkoxy, Phenoxy-[(C₁-C₆)alkoxy]-carbonyl, Phenoxycarbonyl, Phenylcarbonyloxy, auch Phenoxycarbonyloxy, Phenylcarbonylamino, Phenyl-[(C₁-C₆)alkyl]-carbonylamino, Phenyl-[(C₁-C₆)alkyl]-carbonyloxy, auch Phenyl-[(C₁-C₆)alkoxy]-carbonyloxy, (C₃-C₇)Cycloalkyl, (C₃-C₇)Cycloalkoxy, auch (C₃-C₆)Cycloalkyl-(C₁-C₆)alkoxy, auch (C₃-C₆)Cycloalkyl-[(C₁-C₆)alkoxy]-carbonyl, auch (C₃-C₆)Cycloalkoxy-(C₁-C₆)alkoxy, auch (C₃-C₆)Cycloalkoxy-[(C₁-C₆)alkoxy]-carbonyl, auch (C₃-C₆)Cycloalkoxy-carbonyl, auch (C₃-C₆)Cycloalkyl-carbonyloxy, auch (C₃-C₆)Cycloalkoxy-carbonyloxy, auch (C₃-C₆)Cycloalkyl-[(C₁-C₆)alkoxy]-carbonyloxy, auch (C₃-C₆)Cycloalkyl-carbonylamino, auch (C₃-C₆)Cycloalkyl-[(C₁-C₆)alkyl]-carbonylamino und auch (C₃-C₆)Cycloalkyl-[(C₁-C₆)alkyl]-carbonyloxy,
wobei jeder der letztgenannten 26 Reste gegebenenfalls auch mit einem carbocyclischen oder heterocyclischen Ring, vorzugsweise einem carbocyclischen Ring mit 3 bis 6 C-Atomen oder einem hetereocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)ₘ-CH(OR')₂,
in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R"' H oder (C₁-C₄)Alkyl bedeuten,
und auch Reste der Formel Het¹, wobei Het¹ jeweils unabhängig voneinander einen gesättigten, teilweise ungesättigten oder heteroaromatischen Heterocyclylrest mit 3 bis 9 Ringatomen, vorzugsweise mit 5 oder 6 Ringatomen bedeutet, wobei der jeweilige heterocyclische Rest 1 bis 4 Heteroatome, vorzugsweise 1 bis 3 Heteroringatome aus der Gruppe N, O und S enthält und gegebenenfalls auch mit einem carbocyclischen oder heterocyclischen Ring, vorzugsweise einem carbocyclischen Ring mit 3 bis 6 C-Atomen oder einem hetereocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist, substituiert ist, oder
- R¹: einen mehrcyclischen Rest auf Basis von (C₃-C₉)Cycloalkyl, (C₅-C₉)Cycloalkenyl, (C₅-C₉)Cycloalkinyl oder Phenyl, wobei der Basisring mit einem carbocyclischen oder heterocyclischen Ring, vorzugsweise einem 5-oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist, oder
- R¹: einen gesättigten, teilweise ungesättigten oder heteroaromatischen Heterocyclylrest mit 3 bis 9 Ringatomen, vorzugsweise mit 5 oder 6 Ringatomen, der 1 bis 4 Heteroatome, vorzugsweise 1 bis 3 Heteroringatome aus der Gruppe N, O und S enthält, gegebenenfalls auch mit einem carbocyclischen oder heterocyclischen Ring, vorzugsweise einem 5- oder 6-gliedrigen Ring mit 0 oder 1 bis 3 Heteroringatomen aus der Gruppe N, O und S kondensiert ist, vorzugsweise benzokondensiert ist, und der im Ring oder im mehrcyclischen System unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet.

Weiter bevorzugt sind dabei auch Verbindungen (I) oder deren Salze, worin
- R¹: H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, die letztgenannten 7 Reste nur im Falle cyclischer Basisreste, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, Reste der Formeln -NR*R**, -CO-NR*R** und -O-CO-NR*R**,
wobei jeder der Reste R* und R** in den letztgenannten 3 Formeln unabhängig voneinander H, (C₁-C₈)Alkyl, (C₂-C₈)Alkenyl, (C₂-C₈)Alkinyl, Benzyl, substituiertes Benzyl, Phenyl oder substituiertes Phenyl, oder zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeutet,
und [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist,
und Phenyl, Phenyl-(C₁-C₆)alkoxy, Phenyl-[(C₁-C₆)alkoxy]-carbonyl, Phenoxy, Phenoxy-(C₁-C₆)alkoxy, Phenoxy-[(C₁-C₆)alkoxy]-carbonyl, Phenoxycarbonyl, Phenylcarbonyloxy, Phenylcarbonylamino, Phenyl-[(C₁-C₆)alkyl]-carbonylamino, Phenyl-[(C₁-C₆)alkyl]-carbonyloxy, (C₃-C₇)Cycloalkyl und (C₃-C₇)Cycloalkoxy,
wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)ₘ-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R"' H oder (C₁-C₄)Alkyl bedeuten, substituiert ist,
bedeutet.

Weiter bevorzugt sind dabei auch Verbindungen (I) oder deren Salze, worin
- R¹: H, (C₁-C₁₂)Alkyl, (C₂-C₁₂)Alkenyl, (C₂-C₁₂)Alkinyl, (C₃-C₆)Cycloalkyl, (C₅-C₆)Cycloalkenyl, (C₅-C₆)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, auch Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, die letztgenannten 7 nur im Falle cyclischer Basisreste, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, Reste der Formel -NR*R**, -CO-NR*R** und -O-CO-NR*R**,
wobei jeder der Reste R* und R** in den letztgenannten 3 Formeln unabhängig voneinander H, (C₁-C₄)Alkyl, (C₂-C₄)Alkenyl, (C₂-C₄)Alkinyl, Benzyl, Phenyl, das unsubstituiert oder durch einen oder mehrere Reste aus der Gruppe Halogen, (C₁-C₄)Alkyl, (C₁-C₄)Haloalkyl und (C₁-C₄)Alkoxy substituiert ist, oder zusammen mit dem N-Atom einen Piperidin-, Piperazin-, Pyrrolidin-, Pyrazolidin-, Piperazolidin- oder Morpholinrest, welcher unsubstituiert oder durch einen oder mehrere Reste aus der Gruppe (C₁-C₄)Alkyl und (C₁-C₄)Haloalkyl substituiert ist, bedeutet,
und [(C₁-C₄)Alkoxy]-carbonyl, [(C₁-C₄)Alkyl]-carbonylamino, [(C₁-C₄)Alkoxy]-carbonylamino, [(C₁-C₄Alkylamino]-carbonylamino, [(C₁-C₄)Alkyl]-carbonyloxy, [(C₁-C₄)Alkoxy]-carbonyloxy und (C₁-C₄)Alkylsulfonyl,
wobei jeder der letztgenannten 7 Reste unsubstituiert oder durch einen oder mehrere Reste aus der Gruppe Halogen, NO₂, (C₁-C₄)Alkoxy und Phenyl, das unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Phenyl, Phenyl-(C₁-C₄)alkoxy, Phenyl-[(C₁-C₄)alkoxy]-carbonyl, Phenoxy, Phenoxy-(C₁-C₄)alkoxy, Phenoxy-[(C₁-C₄)alkoxy]-carbonyl, Phenoxycarbonyl, Phenylcarbonyloxy, auch Phenoxycarbonyloxy, auch Phenyl-[(C₁-C₆)alkoxy]-carbonyloxy, Phenylcarbonylamino, Phenyl-[(C₁-C₄)alkyl]-carbonylamino, Phenyl-[(C₁-C₄)alkyl]-carbonyloxy, (C₃-C₆)Cycloalkyl, (C₃-C₆)Cycloalkoxy, auch (C₃-C₆)Cycloalkoxy-carbonyl, auch (C₃-C₆)Cycloalkyl-carbonyloxy, auch
(C₃-C₆)Cycloalkoxy-carbonyloxy, auch (C₃-C₆)Cycloalkyl-[(C₁-C₆)alkyl]-carbonyloxy und auch (C₃-C₆)Cycloalkyl-[(C₁-C₆)alkoxy]-carbonyloxy, wobei jeder der letztgenannten 20 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -CO-O-NR'₂, -O-N=CR'₂, -N=CR'₂, -O-NR'₂, -CH(OR')₂ und -O-(CH₂)ₘ-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy, in welcher jeder der Reste R" unabhängig voneinander H oder (C₁-C₄)Alkyl oder gemeinsam eine (C₁-C₄)Alkylengruppe bedeuten und R"' H oder (C₁-C₂)Alkyl bedeuten,
und auch Reste der Formel Het¹, wobei Het¹ jeweils unabhängig voneinander einen gesättigten, teilweise ungesättigten oder heteroaromatischen Heterocyclylrest mit 5 oder 6 Ringatomen bedeutet, wobei der jeweilige heterocyclische Rest 1 bis 3 Heteroringatome aus der Gruppe N, O und S enthält und gegebenenfalls auch mit einem carbocyclischen oder heterocyclischen Ring, vorzugsweise einem 5-oder 6-gliedrigen Ring mit 0 oder 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, Hydroxy, (C₁-C₄)Alkyl, (C₁-C₄)Alkoxy, (C₁-C₄)Haloalkyl und (C₁-C₄)Haloalkoxy substituiert ist, substituiert ist, bedeutet.

Weiter bevorzugt sind dabei auch Verbindungen (I) oder deren Salze, worin
- R¹: H, (C₁-C₁₂)Alkyl, (C₂-C₁₂)Alkenyl, (C₂-C₁₂)Alkinyl, (C₃-C₆)Cycloalkyl, (C₅-C₆)Cycloalkenyl, (C₅-C₆)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, die letztgenannten 7 nur im Falle cyclischer Basisreste, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, Reste der Formel -NR*R**, -CO-NR*R** und -O-CO-NR*R**,
wobei jeder der Reste R* und R** in den letztgenannten 3 Formeln unabhängig voneinander H, (C₁-C₄)Alkyl, (C₂-C₄)Alkenyl, (C₂-C₄)Alkinyl, Benzyl, Phenyl, das unsubstituiert oder durch einen oder mehrere Reste aus der Gruppe Halogen, (C₁-C₄)Alkyl, (C₁-C₄)Haloalkyl und (C₁-C₄)Alkoxy substituiert ist, oder zusammen mit dem N-Atom einen Piperidin-, Piperazin-, Pyrrolidin-, Pyrazolidin-, Piperazolidin- oder Morpholinrest, welcher unsubstituiert oder durch einen oder mehrere Reste aus der Gruppe (C₁-C₄)Alkyl und (C₁-C₄)Haloalkyl substituiert ist, bedeutet,
und [(C₁-C₄)Alkoxy]-carbonyl, [(C₁-C₄)Alkyl]-carbonylamino, [(C₁-C₄)Alkoxy]-carbonylamino, [(C₁-C₄Alkylamino]-carbonylamino, [(C₁-C₄)Alkyl]-carbonyloxy, [(C₁-C₄)Alkoxy]-carbonyloxy und (C₁-C₄)Alkylsulfonyl, wobei jeder der letztgenannten 7 Reste unsubstituiert oder durch einen oder mehrere Reste aus der Gruppe Halogen, NO₂, (C₁-C₄)Alkoxy und Phenyl, das unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Phenyl, Phenyl-(C₁-C₄)alkoxy, Phenyl-[(C₁-C₄)alkoxy]-carbonyl, Phenoxy, Phenoxy-(C₁-C₄)alkoxy, Phenoxy-[(C₁-C₄)alkoxy]-carbonyl, Phenoxycarbonyl, Phenylcarbonyloxy, Phenylcarbonylamino, Phenyl-[(C₁-C₄)alkyl]-carbonylamino, Phenyl-[(C₁-C₄)alkyl]-carbonyloxy, (C₃-C₆)Cycloalkyl und (C₃-C₆)Cycloalkoxy, wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -CO-O-NR'₂, -O-N=CR'₂, -N=CR'₂, -O-NR'₂, -CH(OR')₂ und -O-(CH₂)ₘ-CH(OR')₂,
in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy,
in welcher jeder der Reste R" unabhängig voneinander H oder (C₁-C₄)Alkyl oder gemeinsam eine (C₁-C₄)Alkylengruppe bedeuten und R"' H oder (C₁-C₂)Alkyl bedeuten, substituiert ist,
bedeutet.

Weiter bevorzugt sind dabei auch Verbindungen (I) oder deren Salze und deren Verwendung, worin
- R¹: H, (C₁-C₆)Alkyl, (C₂-C₆)Alkenyl, (C₂-C₆)Alkinyl oder (C₃-C₆)Cycloalkyl, wobei jeder der letztgenannten 4 Reste unsubstituiert oder durch einen oder mehrere Reste aus der Gruppe Halogen, (C₁-C₆)Alkyl, letzterer nur Substituent im Fall cyclischer Basisreste, (C₁-C₄)Alkoxy, (C₁-C₄)Alkylthio, (C₃-C₆)Cycloalkyl, das unsubstituiert oder durch einen oder mehrere Reste aus der Gruppe Halogen und (C₁-C₄)Alkyl substituiert ist, und Phenyl, das unsubstituiert oder durch einen oder mehrere Reste aus der Gruppe Halogen, (C₁-C₄)Alkyl, (C₁-C₄)Alkoxy und (C₁-C₄)Haloalkyl substituiert ist, substituiert ist,
bedeutet.

Besonders bevorzugt sind dabei auch Verbindungen (I) oder deren Salze, worin
- R¹: H, (C₁-C₄)Alkyl, (C₂-C₄)Alkenyl oder (C₂-C₄)Alkinyl, wobei jeder der letztgenannten 3 Reste unsubstituiert oder durch einen oder mehrere Reste aus der Gruppe Halogen, (C₁-C₄)Alkoxy, (C₁-C₄)Alkylthio, Cyclopropyl, Cyclobutyl, wobei jeder der letztgenannten beiden Reste unsubstituiert oder durch einen oder mehrere Reste aus der Gruppe Halogen und (C₁-C₄)Alkyl substituiert ist, und Phenyl, das unsubstituiert oder durch einen oder mehrere Reste aus der Gruppe Halogen, (C₁-C₄)Alkyl, (C₁-C₄)Alkoxy und (C₁-C₄)Haloalkyl substituiert ist, substituiert ist,
bedeutet.

Weiter bevorzugt bedeutet
- R¹: auch einen mehrcyclischen Rest auf Basis von (C₃-C₉)Cycloalkyl, (C₅-C₉)Cycloalkenyl, (C₅-C₉)Cycloalkinyl oder Phenyl, wobei der Basisring mit einem carbocyclischen oder heterocyclischen Ring, vorzugsweise einem 5-oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere Reste aus der Gruppe Halogen, Cyano, Nitro, (C₁-C₆)Alkyl, (C₁-C₆)Haloalkyl, (C₁-C₄)Alkoxy-(C₁-C₄)alkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₃-C₆)Cycloalkyl, (C₃-C₆)Cycloalkoxy, [(C₁-C₄)Alkoxy]-carbonyl und [(C₁-C₄)Haloalkoxy]-carbonyl substituiert ist.

Bevorzugt sind auch Verbindungen (I) oder deren Salze, worin
- R¹: einen gesättigten, teilweise ungesättigten oder heteroaromatischen Heterocyclylrest mit 3 bis 9 Ringatomen, vorzugsweise mit 5 oder 6 Ringatomen, der 1 bis 4 Heteroatome, vorzugsweise 1 bis 3 Heteroringatome aus der Gruppe N, O und S enthält und der unsubstituiert oder durch einen oder mehrere Reste aus der Gruppe Halogen, Cyano, Thio, Nitro, Hydroxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet.

Bevorzugt sind auch Verbindungen (I) oder deren Salze, worin
- R¹: einen Rest der Formel SiR^{a}R^{b}R^{c}, -NR^{a}R^{b} oder -N=CR^{c}R^{d}, vorzugsweise der Formel -NR^{a}R^{b} oder -N=CR^{c}R^{d}, wobei in den letztgenannten 5 Formeln jeder der Reste R^{a}, R^{b}, R^{c} und R^{d} unabhängig voneinander Wasserstoff, (C₁-C₄)Alkyl, (C₂-C₄)Alkenyl, (C₂-C₄)Alkinyl, Benzyl, substituiertes Benzyl, Phenyl oder substituiertes Phenyl bedeutet oder R^{a} und R^{b} zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeuten oder R^{c} und R^{d} zusammen mit dem C-Atom einen 3- bis 8-gliedrigen carbocyclischen Rest oder heterocyclischen Rest, welcher 1 bis 3 Heteroringatome aus der Gruppe N, O und S enthalten kann, wobei der carbocyclische oder heterocyclischen Rest unsubstituiert oder durch einen oder mehrere Reste aus der Gruppe (C₁-C₄)Alkyl und (C₁-C₄)Haloalkyl substituiert ist, bedeuten,
bedeutet.

Besonders bevorzugt sind auch Verbindungen (I) oder deren Salze, worin
- R¹: H, Methyl, Ethyl, n-Propyl, i-Propyl, n-Butyl, s-Butyl, i-Butyl, t-Butyl, Allyl, Propargyl (Prop-2-in-1-yl), But-2-in-1-yl, But-3-in-1-yl, 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, 1- (2E)-1-methylbut-2-en-1-yl, (E)-Pent-3-en-2-yl oder (Z)-Pent-3-en-2-yl, Phenyl, 2-Carboxy-phenyl, 2-Chlorphenyl, 3-Chlorphenyl, 4-Chlorphenyl, 2-Fluorphenyl, 3-Fluorphenyl, 4-Fluorphenyl, 3-Methoxyphenyl, 4-Methoxyphenyl, 2-Methoxyphenyl, 3-Methoxyphenyl, 4-Methoxyphenyl, Benzyl, 2-Phenyl-ethyl, 1-Phenyl-ethyl, (4-Chlorphenyl)-methyl [d. h. = CH₂(4-CI-Ph)], (4-Fluorphenyl)-methyl [d. h. = CH₂(4-F-Ph)], (4-Methoxyphenyl)-methyl [d. h. = CH₂(4-OMe-Ph)], 2-Methoxyethyl, 2,2,2-Trifluorethyl, 1,1,1-Trifluorprop-2-yl, 2,2-Difluorethyl, 1,3-Difluorprop-2-yl, 2,3-Dimethoxypropyl, 2,3-Dimethoxyprop-2-yl, 2,2-Dimethoxy-eth-2-yl, 2-(2,2,2-Trifluorethoxy)-ethyl, 2-Fluorethyl, 2-Chlorethyl, 2-Bromethyl, 2-lodethyl, 2,2,3,3,3-Pentafluorpropyl, 1-Hydroxy-prop-2-yl, 2-Hydroxy-prop-2-yl, 2-Hydroxy-prop-1-yl, 3-Hydroxy-propyl, 3-Hydroxy-prop-2-yl, (2-Methoxyethoxy)-methyl; 2-(2-Methoxyethoxy)-ethyl; (2-Ethoxyethoxy)-methyl; 2-(2-Ethoxyethoxy)-ethyl, (Acetoxy)-methyl, (Propanoyloxy)-methyl, (2-Methylpropanoyloxy)-methyl, (2,2-Dimethylpropanoyloxy)-methyl, 1-(Acetoxy)-ethyl, 2-(Acetoxy)-ethyl, 2-(Propanoyloxy)-ethyl, 1-(Propanoyloxy)-ethyl, 1-(2-Methylpropanoyloxy)-eth-1-yl, 2-(2-Methylpropanoyloxy)-eth-1-yl, 2-(2,2-Dimethylpropanoyloxy)-ethyl [d. h. 1-(t-Butylcarbonyloxy)-ethyl], 2-(2,2-Dimethylpropanoyloxy)-ethyl; 1-(2,2-Dimethylpropanoyloxy)-2-methylprop-1-yl, 1-(t-Butylcarbonyloxy)-2-methyl prop-1-yl, (Methoxycarbonyl)-methyl, (Ethoxycarbonyl)-methyl, (n-Propoxycarbonyl)-methyl, (i-Propoxycarbonyl)-methyl, (n-Butoxycarbonyl)-methyl, (s-Butoxycarbonyl)-methyl, (i-Butoxycarbonyl)-methyl, (t-Butoxycarbonyl)-methyl, 1-(Methoxycarbonyl)-ethyl, 2-(Methoxycarbonyl)-ethyl, 1-(Ethoxycarbonyl)-ethyl, 2-(Ethoxycarbonyl)-ethyl, 1-(n-Propoxycarbonyl)-ethyl, 2-(n-Propoxycarbonyl)-ethyl, 1-(i-Propoxycarbonyl)-ethyl, 2-(i-Propoxycarbonyl)-ethyl, 1-(n-Butoxycarbonyl)-ethyl, 2-(n-Butoxycarbonyl)-ethyl, 1-(s-Butoxycarbonyl)-ethyl, 2-(s-Butoxycarbonyl)-ethyl, 1-(i-Butoxycarbonyl)-ethyl, 2-(i-Butoxycarbonyl)-ethyl, 1-(t-Butoxycarbonyl)-ethyl, 2-(t-Butoxycarbonyl)-ethyl, (Methoxycarbonyloxy)-methyl, (Ethoxycarbonyloxy)-methyl, (n-Propoxycarbonyloxy)-methyl, (i-Propoxycarbonyloxy)-methyl, (n-Butoxycarbonyloxy)-methyl, (s-Butoxycarbonyloxy)-methyl, (i-Butoxycarbonyloxy)-methyl, (t-Butoxycarbonyloxy)-methyl, 1-(Methoxycarbonyloxy)-ethyl, 2-(Methoxycarbonyloxy)-ethyl, 1-(Ethoxycarbonyloxy)-ethyl, 2-(Ethoxycarbonyloxy)-ethyl, 1-(n-Propoxycarbonyloxy)-ethyl, 2-(n-Propoxycarbonyloxy)-ethyl, 1-(i-Propoxycarbonyloxy)-ethyl, 2-(i-Propoxycarbonyloxy)-ethyl, 1-(n-Butoxycarbonyloxy)-ethyl, 2-(n-Butoxycarbonyloxy)-ethyl, 1-(s-Butoxycarbonyloxy)-ethyl, 2-(s-Butoxycarbonyloxy)-ethyl, 1-(i-Butoxycarbonyloxy)-ethyl, 2-(i-Butoxycarbonyloxy)-ethyl, 1-(t-Butoxycarbonyloxy)-ethyl, 2-(t-Butoxycarbonyloxy)-ethyl, (Cyclohexoxycarbonyloxy)-methyl, 1-(Cyclohexoxycarbonyloxy)-eth-1-yl, 2-(Cyclohexoxycarbonyloxy)-eth-1-yl, (Acetyl)-methyl, 1-(Acetyl)-ethyl, 2-(Acetyl)-ethyl, 1-(Acetyl)-propyl, 2-(Acetyl)-propyl, 3-(Acetyl)-propyl, (Propanoyl)-methyl, 1-(Propanoyl)-ethyl, 2-(Propanoyl)-ethyl, 1-(Propanoyl)-propyl, 2-(Propanoyl)-propyl, 3-(Propanoyl)-propyl, 1-(Propanoyl)-2-methyl-propyl, 2-(Ethylidenaminooxy)-ethyl, 2-(Prop-2-ylidenaminooxy)-ethyl, 2-(But-2-ylidenaminooxy)-ethyl, 2-(Pent-3-ylidenaminooxy)-ethyl, (N,N-Dimethylamino)-methyl, 2-(N,N-Dimethylamino)-eth-1-yl, 1-(N,N-Dimethylamino)-eth-1-yl, 2-(N,N-Diethylamino)-eth-1-yl, 1-(N,N-Diethylamino)-eth-1-yl, (N,N-Diethylamino)-methyl, (N,N-Dimethylaminocarbonyl)-methyl, 1-(N,N-Dimethylaminocarbonyl)-ethyl, 2-(N,N-Dimethylaminocarbonyl)-ethyl, (N,N-Diethylaminocarbonyl)-methyl, 1-(N,N-Diethylaminocarbonyl)-ethyl, 2-(N,N-Diethylaminocarbonyl)-ethyl, 1-(Dimethylamino)-prop-2-yl [d. h. 2-(Dimethylamino)-1-methyl-ethyl], 1-(Diethylamino)-prop-2-yl, Trimethylsilyl-methyl, 1-(Trimethylsilyl)-ethyl, 2-(Trimethylsilyl)-ethyl, Triethylsilyl-methyl, 1-(Triethylsilyl)-ethyl, 2-(Triethylsilyl)-ethyl, Cyclopropyl, Cyclopropylmethyl, 1-Cyclopropyl-ethyl, 2-Cyclopropyl-ethyl, (1-Methyl-cyclopropyl)-methyl, 1-(1-Methyl-cyclopropyl)-ethyl, 2-(1-Methyl-cyclopropyl)-ethyl, (2,2-Dichlorcyclopropyl)-methyl, 1-(2,2-Dichlorcyclopropyl)-ethyl, 2-(2,2-Dichlorcyclopropyl)-ethyl, (2,2-Dimethyl-cyclopropyl)-methyl, 1-(2,2-Dimethyl-cyclopropyl)-ethyl, 2-(2,2-Dimethyl-cyclopropyl)-ethyl, Cyclobutyl-methyl, Cyclopentyl-methyl, Cyclohexyl-methyl oder Pyrid-2-yl, Pyrid-3-yl, Pyrid-4-yl, 2-Chlorpyrid-3-yl, 3-Chlorpyrid-2-yl, Thien-2-yl, Thien-3-yl, 2-Chlorthien-3-yl, 3-Chlorthien-2-yl, 4-Chlorthien-2-yl, (1-Ethyl-5-methyl-1 H-pyrazol-4-yl)-methyl, 1-(1-Ethyl-5-methyl-1H-pyrazol-4-yl)-ethyl, 2-(1-Ethyl-5-methyl-1 H-pyrazol-4-yl)-ethyl (1-Ethyl-3-methyl-1 H-pyrazol-4-yl)-methyl, 1-(1-Ethyl-3-methyl-1H-pyrazol-4-yl)-ethyl, 2-(1-Ethyl-3-methyl-1 H-pyrazol-4-yl)-ethyl, Tetrahydrofuran-2-yl, Tetrahydrofuran-3-yl, Tetrahydrofuran-2-yl-methyl, Tetrahydrofuran-3-yl-methyl, (5-Methyl-2-oxo-1,3-dioxol-4-yl)methyl; Oxetan-3-yl, (Oxetan-3-yl)methyl, (Oxetan-2-yl)methyl, (1,3-Dioxolan-2-yl)-methyl, (1,3-Dioxolan-4-yl)-methyl, 5-Methyl-2-oxo-1,3-dioxolan-4-yl)methyl, (Morpholin-4-yl)-methyl; 1-(Morpholin-4-yl)-ethyl, 2-(Morpholin-4-yl)-ethyl, 2,3-Dihydro-1H-inden-2-yl, Dihydro-1H-inden-3-yl, Dihydro-1H-inden-4-yl, Dihydro-1H-inden-5-yl, 1H-Inden-2-yl, 1H-Inden-3-yl, 1H-Inden-4-yl, 1H-Inden-5-yl, 1H-Inden-6-yl oder 1H-Inden-7-yl
bedeutet.

Ganz besonders bevorzugt sind dabei Verbindungen (I) und deren Salze, worin
- R¹: H, Methyl, Ethyl, n-Propyl, i-Propyl, Phenyl, Benzyl, CH₂(4-Cl-Ph), d. h. (4-Chlorphenyl)-methyl, CH₂(4-F-Ph), d. h. (4-Fluorphenyl)-methyl, CH₂(4-OMe-Ph), d. h. (4-Methoxyphenyl)-methyl, 2-Methoxyethyl, Tetrahydrofuran-2-yl-methyl, 2-(Dimethylamino)ethyl, Oxetan-3-yl, (3-Methyloxetan-3-yl)methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Allyl, Propargyl (Prop-2-in-1-yl), 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methyl prop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, 1-(2E)-1-methylbut-2-en-1-yl, (E)-Pent-3-en-2-yl oder (Z)-Pent-3-en-2-yl, Cyclobutylmethyl, Cyclopentylmethyl, Cyclohexylmethyl oder 1-Ethyl-5-methyl-1 H-pyrazol-4-methyl, d. h. (1-Ethyl-5-methyl-1 H-pyrazol-4-yl)-methyl,
bedeutet.

Bevorzugt sind auch Verbindungen der Formel (I) oder deren Salze, worin
- R²: Wasserstoff, Halogen oder (C₁-C₄)Alkyl, das unsubstituiert oder durch einen oder mehrere Reste aus der Gruppe Halogen, wie Fluor oder Chlor substituiert ist, vorzugweise Wasserstoff oder (C₁-C₄)Alkyl, insbesondere Wasserstoff, Methyl oder Ethyl, ganz besonders Wasserstoff oder Methyl
bedeutet.

Bevorzugt sind auch die Verbindungen der Formel (I) oder deren Salze, worin
- R³: Wasserstoff, Halogen oder (C₁-C₄)Alkyl, das unsubstituiert oder durch einen oder mehrere Reste aus der Gruppe Halogen, wie Fluor oder Chlor, substituiert ist, vorzugweise Wasserstoff oder (C₁-C₄)Alkyl, insbesondere Wasserstoff oder Methyl, ganz besonders Wasserstoff
bedeutet.

Bevorzugt sind auch Verbindungen der Formel (I) oder deren Salze, worin
- R²: Wasserstoff und
- R³: Wasserstoff
bedeuten.

Bevorzugt sind auch die Verbindungen der Formel (I) oder deren Salze, worin R² und R³ zusammen mit dem C-Atom, an das sie gebunden sind, (C₃-C₆)Cycloalkyl oder (C₅-C₆)Cy_{C}loalkenyl, vorzugsweise (C₃-C₆)Cycloalkyl, wobei jeder der letztgenannten 3 Reste unsubstituiert oder durch einen oder mehrere Reste aus der Gruppe Halogen und (C₁-C₄)Alkyl substituiert ist, bedeuten.
Bevorzugt bedeuten dabei R² und R³ zusammen mit dem C-Atom, an das sie gebunden sind, Cyclopropyl, Cyclobutyl, Cyclopentyl oder Cyclohexyl, insbesondere Cyclopropyl, wobei jeder der letztgenannten 4 Reste unsubstituiert oder durch einen oder mehrere Reste aus der Gruppe Halogen und Methyl, vorzugsweise Fluor, Chlor und Methyl substituiert ist.

Bevorzugt sind auch die Verbindungen der Formel (I) oder deren Salze, worin
- R⁴: Wasserstoff, Halogen, Cyano, (C₁-C₄)Alkyl, (C₂-C₄)Alkenyl oder (C₂-C₄)Alkinyl, wobei jeder der letztgenannten drei Reste unsubstituiert oder durch einen oder mehrere Reste aus der Gruppe bestehend aus Halogen und Hydroxy substituiert ist, vorzugsweise unsubstituiert oder durch einen oder mehrere Reste aus der Gruppe Halogen, wie Fluor und Chlor substituiert ist, oder
(C₃-C₆)Cycloalkyl, das unsubstituiert oder durch einen oder mehrere Reste aus der Gruppe bestehend aus Halogen und (C₁-C₄)Alkyl substituiert ist, oder
Phenyl, das unsubstituiert oder durch einen oder mehrere Reste aus der Gruppe bestehend aus Halogen, Nitro, (C₁-C₄)Alkyl, (C₁-C₄)Haloalkyl, (Cr₁-C₄)Alkoxy, (C₁-C₄)Haloalkoxy, (C₁-C₄)Alkoxy-(C₁-C₄)alkoxy, (C₁-C₄)Alkylthio, [(C₁-C₄)Alkoxy]-carbonyl und [(C₁-C₄)Haloalkoxy]-carbonyl substituiert ist, oder
(C₁-C₄)Alkanoyl, das unsubstituiert oder durch einen oder mehrere Reste aus der Gruppe bestehend aus Halogen, wie Fluor und Chlor, Cyano, (C₁-C₄)Alkoxy, (C₁-C₄)Haloalkoxy und (C₁-C₂)Alkoxy-(C₁-C₂)alkoxy substituiert ist, vorzugsweise Formyl, oder
[(C₁-C₄)Alkoxy]-carbonyl, das unsubstituiert oder durch einen oder mehrere Reste aus der Gruppe bestehend aus Halogen, wie Fluor und Chlor, substituiert ist, oder
[(C₃-C₆)Cycloalkoxy]-carbonyl, das unsubstituiert oder durch einen oder mehrere Reste aus der Gruppe bestehend aus Halogen und (C₁-C₄)Alkyl substituiert ist,
bedeutet.

Weiter bevorzugt sind auch die Verbindungen der Formel (I) oder deren Salze, worin
- R⁴: Wasserstoff, Halogen, wie Fluor oder Chlor, Cyano, (C₁-C₄)Alkyl, das gegebenenfalls durch Hydroxy substituiert ist [= (C₁-C₄)Hydroxyalkyl], (C₁-C₄)Haloalkyl, Cyclopropyl oder Cyclobutyl, wobei jeder der letztgenannten beiden Reste unsubstituiert oder durch einen oder mehrere Reste aus der Gruppe bestehend aus Halogen, wie Fluor und Chlor, und (C₁-C₄)Alkyl substituiert ist, oder
Phenyl, das unsubstituiert oder durch einen oder mehrere Reste aus der Gruppe bestehend aus Halogen, (C₁-C₄)Alkyl, (C₁-C₄)Haloalkyl, (C₁-C₄)Alkoxy und (C₁-C₄)Alkylthio substituiert ist, oder
(C₁-C₄)Alkanoyl, das unsubstituiert oder durch einen oder mehrere Reste aus der Gruppe bestehend aus Halogen, wie Fluor und Chlor, substituiert ist, vorzugsweise Formyl, oder [(C₁-C₄)Alkoxy]-carbonyl oder [(C₁-C₄)Haloalkoxy]-carbonyl bedeutet, vorzugsweise R⁴ Wasserstoff, Halogen, wie Fluor oder Chlor, Cyano, Methyl, Ethyl, n-Propyl, i-Propyl, CH₂Cl, CHCl₂, CCl₃, CH₂F, CHF₂, CF₃ oder Formyl
bedeutet.

Weiter bevorzugt ist
- R⁴: Wasserstoff, Fluor, Chlor, Methyl, Ethyl, n-Propyl, i-Propyl, CH₂Cl, CHCl₂, CCl₃, CH₂F, CHF₂ oder CF₃., insbesondere Methyl.

Bevorzugt sind auch Verbindungen der Formel (I), worin
- R⁵: Phenyl, das unsubstituiert oder vorzugsweise durch einen oder mehrere Reste aus der Gruppe bestehend aus den Resten
(a) Halogen, Hydroxy, Amino, Nitro, Carboxy, Cyano und Carbamoyl,
(b) (C₁-C₆)Alkyl, (C₂-C₆)Alkenyl, (C₂-C₆)Alkinyl und (C₁-C₆)Alkoxy, wobei jeder der letztgenannten 4 Reste unsubstituiert oder durch einen oder mehrere Reste aus der Gruppe Halogen, (C₁-C₄)Alkoxy, (C₁-C₆)Haloalkoxy, (C₁-C₄)Alkylthio, Hydroxy, Carboxy, [(C₁-C₄)Alkoxy]-carbonyl und Cyano substituiert ist,
(c) (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₁-C₆)Alkylsulfinyloxy, (C₁-C₆)Alkylsulfonyloxy und (C₁-C₆)Haloalkylsulfonyloxy und
- (d): (C₃-C₆)Cycloalkyl, (C₃-C₆)Cycloalkyloxy, Phenyl und Phenoxy, wobei jeder der letztgenannten 4 Reste unsubstituiert oder durch einen oder mehrere Reste aus der Gruppe bestehend aus Halogen, (C₁-C₄)Alkyl, (C₁-C₄)Haloalkyl, (C₁-C₄)Alkoxy, (C₁-C₆)Haloalkoxy und (C₁-C₄)Alkylthio substituiert ist, substituiert ist, wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₆)Haloalkoxy und (C₁-C₄)Alkylthio substituiert ist,
oder bedeutet
- R⁵: einen 5- oder 6-gliedrigen heteroaromatischen Rest mit 1 bis 3 Heteroringatomen aus der Gruppe N, O und S, der unsubstituiert oder durch einen oder mehrere Reste aus der Gruppe bestehend aus den Resten
(a) Halogen, Hydroxy, Amino, Nitro, Carboxy, Cyano und Carbamoyl,
(b) (C₁-C₆)Alkyl, (C₂-C₆)Alkenyl, (C₂-C₆)Alkinyl und (C₁-C₆)Alkoxy, wobei jeder der letztgenannten 6 Reste unsubstituiert oder durch einen oder mehrere Reste aus der Gruppe Halogen, (C₁-C₄)Alkoxy, (C₁-C₆)Haloalkoxy, (C₁-C₄)Alkylthio, Mono- und Di-[(C₁-C₄)alkyl]-amino, Hydroxy, Carboxy, [(C₁-C₄)Alkoxy]-carbonyl und Cyano substituiert ist,
(c) (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₁-C₆)Alkylsulfinyloxy, (C₁-C₆)Alkylsulfonyloxy und (C₁-C₆)Haloalkylsulfonyloxy und
(d) (C₃-C₆)Cycloalkyl, (C₃-C₆)Cycloalkyloxy, Phenyl und Phenoxy, wobei jeder der letztgenannten 4 Reste unsubstituiert oder durch einen oder mehrere Reste aus der Gruppe bestehend aus Halogen, (C₁-C₄)Alkyl, (C₁-C₄)Haloalkyl, (C₁-C₄)Alkoxy, (C₁-C₆)Haloalkoxy und (C₁-C₄)Alkylthio substituiert ist, substituiert ist, wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₆)Haloalkoxy und (C₁-C₄)Alkylthio substituiert ist

Weiter bevorzugt sind auch die Verbindungen der Formel (I) oder deren Salze und deren Anwendungen, worin
- R⁵: Phenyl,
das unsubstituiert oder vorzugsweise durch einen oder mehrere Reste aus der Gruppe bestehend aus Halogen, Hydroxy, Amino, Nitro, Carboxy, Cyano, Carbamoyl, (C₁-C₆)Alkyl, auch (C₂-C₄)Alkenyl, auch (C₂-C₄)Alkinyl, (C₁-C₆)Haloalkyl, (C₁-C₄)Alkoxy-(C₁-C₄)alkyl, (C₁-C₄)Alkylthi₀-(C₁-C₄)alkyl, Mono- und Di-[(C₁-C₄)alkyl]-amino-(C₁-C₄)alkyl, Hydroxy-(C₁-C₄)alkyl, Carboxy-(C₁-C₄)alkyl, Cyano-(C₁-C₄)alkyl, (C₁-C₆)Alkoxy, das gegebenenfalls auch halogeniert sein kann [= (C₁-C₆)Haloalkoxy)], (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono-und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, auch (C₁-C₄)Alkylsulfonyloxy, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
oder
- R⁵: einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, auch (C₂-C₄)Alkenyl, auch (C₂-C₄)Alkinyl, (C₁-C₆)Haloalkyl, (C₁-C₄)Alkoxy-(C₁-C₄)alkyl, (C₁-C₄)Alkylthio-(C₁-C₄)alkyl, Mono- und Di-[(C₁-C₄)alkyl]-amino-(C₁-C₄)alkyl, Hydroxy-(C₁-C₄)alkyl, Carboxy-(C₁-C₄)alkyl, Cyano-(C₁-C₄)alkyl, (C₁-C₆)Alkoxy, das gegebenenfalls auch halogeniert sein kann [= (C₁-C₆)Haloalkoxy], (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono-und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, auch (C₁-C₄)Alkylsulfonyloxy, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist, substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
bedeutet.

Weiter bevorzugt sind dabei auch die Verbindungen der Formel (I) oder deren Salze, worin
- R⁵: Phenyl, das unsubstituiert oder vorzugsweise durch einen oder mehrere Reste aus der Gruppe bestehend aus Halogen, Hydroxy, Nitro, Carboxy, Cyano, (C₁-C₄)Alkyl, (C₁-C₄)Haloalkyl, (C₁-C₄)Alkoxy-(C₁-C₄)alkyl, (C₁-C₄)Alkylthi₀-(C₁-C₄)alkyl, Hydroxy-(C₁-C₄)alkyl, (C₁-C₄)Alkoxy, das gegebenenfalls auch halogeniert sein kann [= (C₁-C₄)Haloalkoxy], (C₁-C₄)Alkoxy-(C₁-C₄)alkoxy, (C₁-C₄)Alkylthio, [(C₁-C₄)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl, (C₁-C₄)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₄)Alkyl substituiert ist, oder
- R⁵: einen 5- oder 6-gliedrigen heteroaromatischen Rest mit 1 bis 3 Heteroringatomen aus der Gruppe N, O und S, der unsubstituiert oder durch einen oder mehrere Reste aus der Gruppe bestehend aus Halogen, Hydroxy, Carboxy, Cyano, auch Amino, (C₁-C₄)Alkyl, auch (C₂-C₄)Alkenyl, auch (C₂-C₄)Alkinyl, (C₁-C₄)Haloalkyl, (C₁-C₄)Alkoxy-(C₁-C₄)alkyl, (C₁-C₄)Alkylthio-(C₁-C₄)alkyl, Hydroxy-(C₁-C₄)alkyl, (C₁-C₄)Alkoxy, das gegebenenfalls auch halogeniert sein kann [= (C₁-C₄)Haloalkoxy], (C₁-C₄)Alkoxy-(C₁-C₄)alkoxy, (C₁-C₄)Alkylthio, [(C₁-C₆)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl, (C₁-C₄)Haloalkylsulfonyl, auch (C₁-C₄)Alkylsulfonyloxy, auch Mono- und Di-[(C₁-C₄)alkyl]-amino, auch Phenyl und (C₃-C₆)Cycloalkyl, das unsubstituiert oder durch einen oder mehrere Reste aus der Gruppe bestehend aus Halogen, (C₁-C₄)Alkyl und (C₁-C₄)Haloalkyl substituiert ist, substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₄)Alkyl substituiert ist,
bedeutet.

Noch weiter bevorzugt sind dabei auch die Verbindungen der Formel (I) oder deren Salze, worin
- R⁵: Phenyl, das unsubstituiert oder vorzugsweise durch einen oder mehrere Reste aus der Gruppe bestehend aus Halogen, Cyano, (C₁-C₄)Alkyl, (C₁-C₄)Haloalkyl, (C₁-C₄)Alkoxy, (C₁-C₄)Alkylthio, auch (C₂-C₄)Alkenyl, (C₂-C₄)Alkinyl, (C₃-C₆)Cycloalkyl, Phenyl, Amino, Mono- und Di-[(C₁-C₄)alkyl]-amino und (C₁-C₄)Alkylsulfonyloxy substituiert und gegebenenfalls benzokondensiert ist,
oder
- R⁵: einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, (C₁-C₄)Alkyl, (C₁-C₄)Haloalkyl, (C₁-C₄)Alkoxy, (C₁-C₄)Alkylthio, auch (C₂-C₄)Alkenyl, (C₂-C₄)Alkinyl, (C₃-C₆)Cycloalkyl, Phenyl, Amino, Mono- und Di-[(C₁-C₄)alkyl]-amino und (C₁-C₄)Alkylsulfonyloxy substituiert und gegebenenfalls benzokondensiert oder benzoannelliert ist,
bedeutet.

Noch weiter bevorzugt sind dabei auch die Verbindungen der Formel (I) oder deren Salze, worin
- R⁵: Phenyl, das unsubstituiert oder vorzugsweise durch einen oder mehrere Reste aus der Gruppe bestehend aus Halogen, Cyano, (C₁-C₄)Alkyl, (C₁-C₄)Haloalkyl, (C₁-C₄)Alkoxy und (C₁-C₄)Alkylthio substituiert und gegebenenfalls benzokondensiert ist,
oder
- R⁵: einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, (C₁-C₄)Alkyl, (C₁-C₄)Haloalkyl, (C₁-C₄)Alkoxy und (C₁-C₄)Alkylthio substituiert und gegebenenfalls benzokondensiert oder benzoannelliert ist,
bedeutet.

Besonders bevorzugt sind dabei Verbindungen der Formel (I) oder deren Salze, worin
- R⁵: Phenyl, das unsubstituiert oder vorzugsweise durch einen oder mehrere Reste aus der Gruppe bestehend aus Halogen, wie Fluor, Chlor, Brom und lod, Cyano, Methyl, Ethyl, n-Propyl, i-Propyl, n-Butyl, sec-Butyl, i-Butyl, t-Butyl, Trifluormethyl, Trichlormethyl, Methoxy und Ethoxy substituiert und gegebenenfalls benzokondensiert ist,
bedeutet, vorzugsweise
- R⁵: Phenyl, 2-Fluorphenyl, 2-Chlorphenyl, 2-Bromphenyl, 2-lodphenyl, 2-Methylphenyl, 4-(tert.-Butyl)-phenyl, 2-Trifluormethyl-phenyl oder 2-Methoxyphenyl oder auch 2-Cyanophenyl oder auch 2-Nitrophenyl oder auch 4-Nitrophenyl oder 3-Fluorphenyl, 3-Chlorphenyl, 3-Bromphenyl, 3-lodphenyl, 3-Methylphenyl, 3-Trifluormethyl-phenyl, 3-Methoxyphenyl, 4-Carboxy-phenyl, 4-Fluorphenyl, 4-Chlorphenyl, 4-Bromphenyl, 4-lodphenyl, 4-Methylphenyl, 4-Trifluormethyl-phenyl, 4-Methoxyphenyl, 2,3-Dichlorphenyl, 2,3-Dimethylphenyl, 2,4-Dichlorphenyl, 2,4-Dimethylphenyl, 2,5-Dichlorphenyl, 2,5-Dimethylphenyl, 2,6-Dichlorphenyl, oder auch 2,4-Difluorphenyl, 2,6-Dimethylphenyl, 3,4-Dichlorphenyl oder auch 3,4-Difluorphenyl, oder 2,6-Dimethylphenyl, 3,4-Dichlorphenyl, 3,4-Dimethylphenyl, 3,5-Dichlorphenyl, 3,5-Dimethylphenyl, 2-Chlor-3-methylphenyl, 2-Chlor-4-methylphenyl, 2-Chlor-5-methylphenyl, 2-Chlor-6-methylphenyl, 3-Chlor-4-methylphenyl, 3-Chlor-5-methylphenyl, 3-Chlor-2-methylphenyl, 4-Chlor-2-methylphenyl oder 4-Chlor-3-methylphenyl oder auch 4-Brom-3-methylphenyl oder 5-Chlor-2-methylphenyl, 4-Phenyl-phenyl, 3-Trifluormethyl-4-chlor-phenyl, 4-Phenoxy-phenyl, 4-Carboxymethyl-phenyl, 4-Acetyl-phenyl (= 4-Methylcarbonyl-phenyl) oder 1,3-Benzodioxol-5-yl
bedeutet.

Bevorzugt sind dabei auch Verbindungen der Formel (I) oder deren Salze, worin
- R⁵: 2-Pyridyl, 3-Pyridyl, 4-Pyridyl, 2-Thienyl, 3-Thienyl, 2-Furyl, 3-Furyl, 2-Pyrrolyl, 3-Pyrrolyl, 2-Pyrimidinyl, 4-Pyrimidinyl, 5-Pyrimidinyl, 3-Pyridazinyl, 4-Pyridazinyl, 2-Pyrazinyl, 3-Pyrazinyl, 2-Imidazolinyl, 4-Imidazolinyl, 1-Pyrazolyl, 3-Pyrazolyl, 4-Pyrazolyl, 5-Pyrazolyl, 1,3,5-Triazin-2-yl, 1,2,4-Triazin-3-yl, 1,2,4-Triazin-5-yl, 2-Thiazolyl, 1,3-Benzothiazol-2-yl, 4-Thiazolyl, 5-Thiazolyl, 3-Isothiazolyl, 4-Isothiazolyl, 5-Isothiazolyl, 2-Oxazolyl, 4-Oxazolyl, 5-Oxazolyl, 3-Isoxazolyl, 4-Isoxazolyl, 5-Isoxazolyl, Thiadiazolyl oder Triazolyl, oder auch 3-Isochinolinyl, 2-Chinolinyl, 1,3-Benzthiazol-2-yl oder 1,3-Benzoxazol-2-yl, vorzugsweise 2-Pyridyl, 3-Pyridyl, 4-Pyridyl, 2-Pyrimidinyl, 2-Pyrazinyl, 2-Thienyl, 3-Thienyl, 2-Furyl oder 2-Thiazolyl oder auch 3-Isochinolinyl oder 2-Chinolinyl, wobei jeder der vorstehend genannten heteroaromatischen Reste unsubstituiert oder substituiert ist, vorzugsweise durch die oben bereits bevorzugt genannten Reste substituiert ist, insbesondere durch einen oder mehrere Reste aus der Gruppe bestehend aus Halogen, Hydroxy, (C₁-C₄)Alkyl, (C₁-C₄)Haloalkyl, (C₁-C₄)Alkoxy und (C₁-C₄)Alkylthio und auch (C₂-C₄)Alkenyl, (C₂-C₄)Alkinyl, (C₁-C₄)Alkylamino, Di-[(C₁-C₄)alkyl]-amino und (C₁-C₄)Alkylsulfonyloxy substituiert ist,
bedeutet.

Bevorzugt sind dabei auch Verbindungen der Formel (I) oder deren Salze, worin R⁵ 2-Pyridyl, 3-Pyridyl, 4-Pyridyl, 2-Thienyl, 3-Thienyl, 2-Furyl, 3-Furyl, 2-Pyrrolyl, 3-Pyrrolyl, 2-Pyrimidinyl, 4-Pyrimidinyl, 5-Pyrimidinyl, 3-Pyridazinyl, 4-Pyridazinyl, 2-Pyrazinyl, 3-Pyrazinyl, 2-Imidazolinyl, 4-Imidazolinyl, 1-Pyrazolyl, 3-Pyrazolyl, 4-Pyrazolyl, 5-Pyrazolyl, 1,3,5-Triazin-2-yl, 1,2,4-Triazin-3-yl, 1,2,4-Triazin-5-yl, 2-Thiazolyl, 2-(1,3-Benzothiazolyl), Isochinolin-3-yl, Chinolin-2-yl, 4-Thiazolyl, 5-Thiazolyl, 3-Isothiazolyl, 4-Isothiazolyl, 5-Isothiazolyl, 2-Oxazolyl, 4-Oxazolyl, 5-Oxazolyl, 3-Isoxazolyl, 4-Isoxazolyl, 5-Isoxazolyl, Thiadiazolyl oder Triazolyl, vorzugsweise 2-Pyridyl, 3-Pyridyl, 4-Pyridyl, 2-Pyrimidinyl, 2-Pyrazinyl, 2-Thienyl, 3-Thienyl, 2-Furyl oder 2-Thiazolyl, wobei jeder der vorstehend genannten heteroaromatischen Reste unsubstituiert oder substituiert ist, vorzugsweise durch die oben bereits bevorzugt genannten Reste (d. h. als Substituenten genannte Reste) substituiert ist, weiter bevorzugt durch einen oder mehrere Reste aus der Gruppe bestehend aus Halogen, Hydroxy, (C₁-C₄)Alkyl, (C₁-C₄)Haloalkyl, (C₁-C₄)Alkoxy und (C₁-C₄)Alkylthio und auch (C₂-C₄)Alkenyl, (C₂-C₄)Alkinyl, (C₁-C₄)Alkylamino, Di-[(C₁-C₄)alkyl]-amino und (C₁-C₄)Alkylsulfonyloxy substituiert ist, insbesondere durch einen oder mehrere Reste aus der Gruppe bestehend aus Halogen, Hydroxy, (C₁-C₄)Alkyl, (C₁-C₄)Haloalkyl, (C₁-C₄)Alkoxy, (C₁-C₄)Alkylthio substituiert ist,
bedeutet.

Besonders bevorzugt sind dabei auch Verbindungen der Formel (I) oder deren Salze, worin
- R⁵: 2-Pyridyl, 3-Fluor-pyrid-2-yl, 3-Chlor-pyrid-2-yl, 3-Brom-pyrid-2-yl, 3-Methyl-pyrid-2-yl, 3-Methoxy-pyrid-2-yl, 3-Trifluormethyl-pyrid-2-yl, 4-Fluor-pyrid-2-yl, 4-Chlor-pyrid-2-yl, 4-Brom-pyrid-2-yl, 4-Methyl-pyrid-2-yl, 4-Methoxy-pyrid-2-yl, 4-Trifluormethyl-pyrid-2-yl, 5-Fluor-pyrid-2-yl, 5-Chlor-pyrid-2-yl, 5-Brom-pyrid-2-yl, 5-Methyl-pyrid-2-yl, 5-Methoxy-pyrid-2-yl, 5-Trifluormethyl-pyrid-2-yl, 6-Fluor-pyrid-2-yl, 6-Chlor-pyrid-2-yl, 6-Brom-pyrid-2-yl, 6-Methyl-pyrid-2-yl, 6-Methoxy-pyrid-2-yl, 6-Trifluormethyl-pyrid-2-yl, 4,6-Dimethyl-pyridin-2-yl, 4-Chlor-6-methyl-pyrid-2-yl, 4-Methyl-5-chlor-pyrid-2-yl oder
3-Pyridyl, 2-Fluor-pyrid-3-yl, 2-Chlor-pyrid-3-yl, 2-Brom-pyrid-3-yl, 2-Methyl-pyrid-3-yl, 2-Methoxy-pyrid-3-yl, 2-Trifluormethyl-pyrid-3-yl, 4-Fluor-pyrid-3-yl, 4-Chlor-pyrid-3-yl, 4-Brom-pyrid-3-yl, 4-Methyl-pyrid-3-yl, 4-Methoxy-pyrid-3-yl, 4-Trifluormethyl-pyrid-3-yl, 5-Fluor-pyrid-3-yl, 5-Chlor-pyrid-3-yl, 5-Brom-pyrid-3-yl, 5-Methyl-pyrid-3-yl, 5-Methoxy-pyrid-3-yl, 5-Trifluormethyl-pyrid-3-yl, 6-Fluor-pyrid-3-yl, 6-Chlor-pyrid-3-yl, 6-Brom-pyrid-3-yl, 6-Methyl-pyrid-3-yl, 6-Methoxy-pyrid-3-yl, 6-Trifluormethyl-pyrid-3-yl, 6-Hydroxy-pyridin-3-yl, 4-Pyridyl, 2-Fluor-pyrid-4-yl, 2-Chlor-pyrid-4-yl, 2-Brom-pyrid-4-yl, 2-Methyl-pyrid-4-yl, 2-Methoxy-pyrid-4-yl, 2-Trifluormethyl-pyrid-4-yl, 3-Fluor-pyrid-4-yl, 3-Chlor-pyrid-4-yl, 3-Brom-pyrid-4-yl, 3-Methyl-pyrid-3-yl, 3-Methoxy-pyrid-4-yl, 3-Trifluormethyl-pyrid-4-yl, 5-Iod-pyrid-2-yl, 5-Dimethylamino-pyrid-2-yl, 5-Methylamino-pyrid-2-yl, 5-Methylthio-pyrid-2-yl, 5-Difluormethoxy-pyrid-2-yl, 5-Hydroxy-pyrid-2-yl, 5-Ethinyl-pyrid-2-yl, 5-Cyclopropyl-pyrid-2-yl, 5-Allyl-pyrid-2-yl, 5-Phenyl-pyrid-2-yl, 5-Amino-pyrid-2-yl oder 5-Methylsulfonyloxy-pyrid-2-yl oder
2-Thienyl, 3-Fluor-thien-2-yl, 3-Chlor-thien-2-yl, 3-Brom-thien-2-yl, 3-Methyl-thien-2-yl, 3-Methoxy-thien-2-yl, 3-Trifluormethyl-thien-2-yl, 4-Fluor-thien-2-yl, 4-Chlor-thien-2-yl, 4-Brom-thien-2-yl, 4-Methyl-thien-2-yl, 4-Methoxy-thien-2-yl, 4-Trifluormethyl-thien-2-yl, 5-Fluor-thien-2-yl, 5-Chlor-thien-2-yl, 5-Brom-thien-2-yl, 5-lod-2-thienyl, 5-Methyl-thien-2-yl, 5-Methoxy-thien-2-yl, 5-Trifluormethyl-thien-2-yl oder
3-Thienyl, 2-Fluor-thien-3-yl, 2-Chlor-thien-3-yl, 2-Brom-thien-3-yl, 2-Methyl-thien-3-yl, 2-Methoxy-thien-3-yl, 2-Trifluormethyl-thien-3-yl, 4-Fluor-thien-3-yl, 4-Chlor-thien-3-yl, 4-Brom-thien-3-yl, 4-Methyl-thien-3-yl, 4-Methoxy-thien-3-yl, 4-Trifluormethyl-thien-3-yl, 5-Fluor-thien-3-yl, 5-Chlor-thien-3-yl, 5-Brom-thien-3-yl, 5-Methyl-thien-3-yl, 5-Methoxy-thien-3-yl, 5-Trifluormethyl-thien-3-yl oder
2-Furyl, 3-Fluor-fur-2-yl, 3-Chlor-fur-2-yl, 3-Brom-fur-2-yl, 3-Methyl-fur-2-yl, 3-Methoxy-fur-2-yl, 3-Trifluormethyl-fur-2-yl, 4-Fluor-fur-2-yl, 4-Chlor-fur-2-yl, 4-Brom-fur-2-yl, 4-Methyl-fur-2-yl, 4-Methoxy-fur-2-yl, 4-Trifluormethyl-fur-2-yl, 5-Fluor-fur-2-yl, 5-Chlor-fur-2-yl, 5-Brom-fur-2-yl, 5-Methyl-fur-2-yl, 5-Methoxy-fur-2-yl oder 5-Trifluormethyl-fur-2-yl oder
2-Thiazolyl, 4-Methyl-thiazol-2-yl, 5-Methyl-thiazol-2-yl, 5-Brom-thiazol-2-yl, 5-Chlor-thiazol-2-yl, 4,5-Dimethyl-thiazol-2-yl, 4,5-Dichlor-thiazol-2-yl, 2-(1,3-Benzothiazolyl), 7-Chlor-(1,3-benzothiazol-2-yl), Isochinolin-3-yl, Chinolin-2-yl oder 1,3-Benzothiazol-2-yl, 7-Chlor-1,3-benzothiazol-2-yl, 2-Brom-thiazol-4-yl, 2-Chlor-thiazol-4-yl, 4-Thiazolyl, 2-Methyl-thiazol-4-yl, Thiazol-5-yl, 2-Methyl-thiazol-5-yl, 6-Chlor-1,3-benzothiazol-2-yl, 7-Brom-1,3-benzothiazol-2-yl, 6-Brom-1,3-benzothiazol-2-yl, 1,3-Benzoxazol-2-yl, 7-Chlor-1,3-benzoxazol-2-yl, 6-Chlor-1,3-benzoxazol-2-yl, 7-Brom-1,3-benzoxazol-2-yl, 6-Brom-1,3-benzoxazol-2-yl oder
2-Pyrazinyl, 5-Methyl-pyrazin-2-yl, 1,5-Dimethyl-pyrazol-3-yl, 1-Methyl-pyrazol-3-yl oder 1-Methyl-pyrazol-5-yl oder
2-Pyrimidinyl, 5-F-pyrimidin-2-yl, 5-Chlor-pyrimidin-2-yl, 5-Brom-pyrimidin-2-yl, 5-Methyl-pyrimidin-2-yl, 4,6-Dimethyl-pyrimidin-2-yl, 5-Fluor-pyrimidin-2-yl, 5-Iod-pyrimidin-2-yl oder
3-Pyridazinyl, 6-Methyl-pyridazin-3-yl, 3-(1,2,4)-triazinyl, d. h. 1,2,4-Triazin-3-yl, oder 6-Methyl-(1,2,4)-triazin-3-yl, d. h. 6-Methyl-1,2,4-triazin-3-yl
bedeutet, vorzugsweise
R⁵ 2-Pyridyl, 5-Fluor-pyrid-2-yl, 5-Chlor-pyrid-2-yl, 5-Brom-pyrid-2-yl, 5-Methyl-pyrid-2-yl, 5-Methoxy-pyrid-2-yl, 5-Trifluormethyl-pyrid-2-yl, 3-Pyridyl, 6-Fluor-pyrid-3-yl, 6-Chlor-pyrid-3-yl, 6-Brom-pyrid-3-yl, 6-Methyl-pyrid-3-yl, 6-Methoxy-pyrid-3-yl oder 6-Trifluormethyl-pyrid-3-yl, 4,6-Me₂-pyridin-2-yl,2-Thienyl, 3-Chlor-thien-2-yl, 3-Methyl-thien-2-yl, 4-Chlor-thien-2-yl, 4-Methyl-thien-2-yl, 5-Chlor-thien-2-yl, 5-Brom-thien-2-yl, 5-lod-2-thienyl, 5-Methyl-thien-2-yl 2-Thiazolyl, 5-Brom-thiazol-2-yl, 5-Chlor-thiazol-2-yl, 4,5-Dimethylthiazol-2-yl, 4,5-Dichlor-thiazol-2-yl, 2-(1,3-Benzothiazolyl), Isochinolin-3-yl (= 3-Isochinolinyl), Chinolin-2-yl (= 2-Chinolinyl), 2-Pyrazinyl, 5-Methyl-pyrazin-2-yl, 1,5-Dimethyl-pyrazol-3-yl, 2-Pyrimidinyl, 5-Brom-pyrimidin-2-yl, 5-Methyl-pyrimidin-2-yl, 4,6-Dimethyl-pyrimidin-2-yl, 3-Pyridazinyl, oder 6-Methyl-pyridazin-3-yl oder auch 4-Fluor-pyrid-2-yl, 4-Chlor-pyrid-2-yl, 4-Brom-pyrid-2-yl, 4-Methyl-pyrid-2-yl oder 4-Trifluor-pyrid-2-yl
bedeutet.

Bevorzugt sind auch die Verbindungen der Formel (I) oder deren Salze, worin
- (R⁶)ₙ: n Substituenten R⁶, wobei R⁶, im Falle dass n = 1 ist, oder jeder der Substituenten R⁶ unabhängig voneinander, im Falle dass n größer als 1 ist, einen Rest Halogen, Cyano, (C₁-C₄)Alkyl, (C₁-C₄)Haloalkyl, (C₁-C₄)Alkoxy, (C₁-C₄)Alkylthio, (C₁-C₄)Alkylsulfinyl, (C₁-C₄)Haloalkylsulfinyl, (C₁-C₄)Alkylsulfonyl oder (C₁-C₄)Haloalkylsulfonyl, (C₁-C₄)Alkoxycarbonyl bedeutet, und
- n: 0, 1, oder 2, vorzugweise 0 oder 1
bedeuten.

Bevorzugt sind dabei auch die Verbindungen der Formel (I) oder deren Salze, worin
- (R⁶)ₙ: n Substituenten R⁶, wobei R⁶, im Falle dass n = 1 ist, oder jeder der Substituenten R⁶ unabhängig voneinander, im Falle dass n größer als 1 ist, einen Rest Halogen, wie Fluor, Chlor, Brom oder Iod, Methyl, Ethyl, Trifluormethyl, Methoxy, Ethoxy, Methylthio, Ethylthio, Methylsulfinyl, Ethylsulfinyl, Methylsulfonyl, Ethylsulfonyl, Methoxycarbonyl oder Ethoxycarbonyl bedeutet, und
- n: 0, 1, oder 2, vorzugweise 0 oder 1
bedeuten.

Bevorzugt sind Verbindungen der Formel (I) oder deren Salzen, worin Het einen Rest der Formel (Het-a), (Het-b) oder (Het-c) 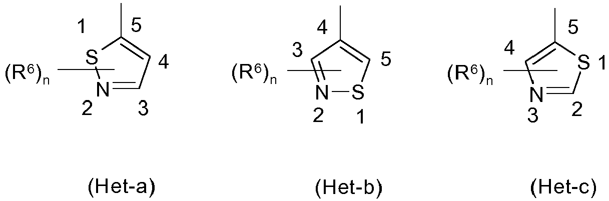 bedeutet und darin (R⁶)ₙ die genannte bzw. bevorzugt genannte Bedeutung haben.

Weiter bevorzugt sind Verbindungen der Formel (I) oder deren Salze, worin
- Het: den genannten Rest (Het-a) bedeutet, worin
- n: 0 (= die Zahl Null, d. h. keine Substituenten R⁶ vorhanden, d. h. alle freien Bindungen am Ring sind mit Wasserstoff besetzt) bedeutet oder
- (R⁶)ₙ: 3-Brom, 3-Chlor, 3-Fluor, 3-Cyano, 3-Methyl, 3-Ethyl, 3-CF₃, 3-Methoxy, 3-Ethoxy, 3-Methylthio, 3-Methylsulfinyl, 3-Methylsulfonyl, 4-Fluor, 4-Chlor, 4-Brom, 4-Cyano, 4-Methyl, 4-Ethyl, 4-CF₃, 4-Methoxy, 4-Ethoxy, 4-Methylthio, 4-Methylsulfinyl, 4-Methylsulfonyl, 3,4-Dimethyl, 3,4-Difluor, 3,4-Dichlor bedeutet,
wobei die Bezifferung der Reste sich auf die Position des Restes am Isothiazol-5-yl-Rest bezieht, in dem die S und N-Ringatome jeweils in 1- und 2-Position im Ring liegen.

Besonders bevorzugt sind dabei die Verbindungen der Formel (I) oder deren Salze, worin
- Het: den genannten Rest (Het-a) bedeutet, worin n = 0 bedeutet oder
- (R⁶)ₙ: 4-Fluor, 4-Chlor, 4-Methyl, 4-Trifluormethyl, 4-Methoxy, 4-Methylsulfonyl, 4-Methylsulfinyl oder 4-Methylthio, vorzugsweise 4-Methyl bedeutet.

Weiter bevorzugt sind Verbindungen der Formel (I) oder deren Salze, worin
- Het: den genannten Rest (Het-b) bedeutet, worin
- n: 0 (= die Zahl Null, d. h. keine Substituenten R⁶ vorhanden, d. h. alle freien Bindungen am Ring sind mit Wasserstoff besetzt) oder
- (R⁶)ₙ: 5-Fluor, 5-Chlor, 5-Brom, 5-Cyano, 5-Methyl, 5-Ethyl, 5-Trifluormethyl, 5-Methoxy, 5-Ethoxy, 5-Methylthio, 5-Methylsulfinyl, 5-Methylsulfonyl, 3-Fluor, 3-Chlor, 3-Brom, 3-Cyano, 3-Methyl, 3-Ethyl, 3-Trifluormethyl, 3-Methoxycarbonyl, 3-Methoxy, 3-Ethoxy, 3-Methylthio, 3-Methylsulfinyl, 3-Methylsulfonyl, 3,5-Dimethoxycarbonyl, 3,5-Dimethyl, 3,5-Difluor oder 3,5-Dichlor
bedeutet, wobei die Bezifferung der Reste sich auf die Position des Restes am Isothiazol-4-yl-Rest bezieht, in dem die S und N-Ringatome jeweils in 1- und 2-Position im Ring liegen.

Besonders bevorzugt sind dabei die Verbindungen der Formel (I) oder deren Salze, worin
- Het: den genannten Rest (Het-b) bedeutet, worin n = 0 bedeutet oder
- (R⁶)ₙ: 5-Fluor, 5-Chlor, 5-Methyl, 5-Trifluromethyl, 5-Methoxy, 5-Methylsulfonyl, 5-Methylsulfinyl oder 5-Methylthio, vorzugsweise 5-Methyl bedeutet.

Weiter bevorzugt sind Verbindungen der Formel (I) oder deren Salze, worin
- Het: den genannten Rest (Het-c) bedeutet, worin
- n: 0 (= die Zahl Null, d. h. keine Substituenten R⁶ vorhanden, d. h. alle freien Bindungen am Ring sind mit Wasserstoff besetzt) oder
- (R⁶)ₙ: 2-Brom, 2-Chlor, 2-Fluor, 2-Cyano, 2-Methyl, 2-Ethyl, 2-CF₃, 2-Methoxy, 2-Ethoxy, 2-Methylthio, 2-Methylsulfinyl, 2-Methylsulfonyl, 4-Fluor, 4-Chlor, 4-Brom, 4-Cyano, 4-Methyl, 4-Ethyl, 4-CF₃, 4-Methoxy, 4-Ethoxy, 4-Methylthio, 4-Methylsulfinyl, 4-Methylsulfonyl, 4-Ethoxycarbonyl, 2,4-Dimethyl, 2,4-Difluor, 2,4-Dichlor bedeutet,
wobei die Bezifferung der Reste sich auf die Position des Restes am Thiazol-5-yl bezieht, in dem die S und N-Ringatome jeweils in 1- und 3-Position im Ring liegen.

Besonders bevorzugt sind dabei die Verbindungen der Formel (I) oder deren Salze, worin
- Het: den genannten Rest (Het-c) bedeutet, worin n = 0 bedeutet oder worin
- (R⁶)ₙ: 4-Fluor, 4-Chlor, 4-Methyl, 4-Trifluormethyl, 4-Methoxy, 4-Methylsulfonyl, 4-Methylsulfinyl oder 4-Methylthio, vorzugsweise 4-Methyl, bedeutet.

Bevorzugt sind auch die Verbindungen der Formel (I) oder deren Salze, worin Het den genannten Rest (Het-a), (Het-b), oder (Het-c), worin jeweils n = 0 ist, bedeutet.

Bevorzugt sind auch die Verbindungen der Formel (I) oder deren Salze, worin die Reste Het, R¹, R², R³, R⁴, R⁵, R⁶ und n gemäß zwei oder mehreren der genannten bevorzugten Bedeutungen ausgewählt worden sind.

Bevorzugt sind als Verbindungen der Formel (I) oder deren Salze die Verbindungen der Formel (Ia), (Ib), oder (Ic) oder deren Salze, 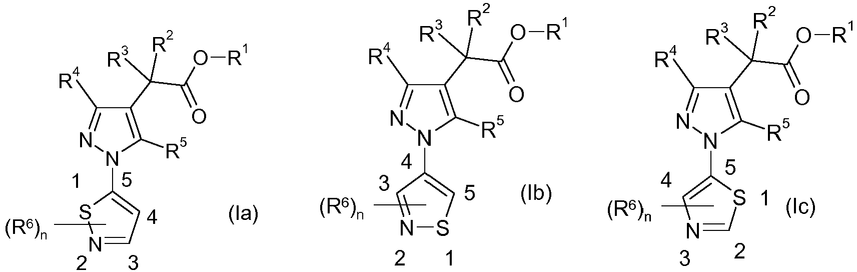 worin R¹, R², R³, R⁴, R⁵, R⁶ und n wie in Formel (I) bzw. gemäß den genannten bevorzugten Bedeutungen definiert sind.

Besonders bevorzugt sind die Verbindungen der allgemeinen Formel (Ia) oder deren Salze, worin
- R¹: Wasserstoff bedeutet und R², R³, R⁴, R⁵, R⁶ und n wie in Formel (I) definiert sind [= Verbindungen der Formel (la")] oder
- R¹: Methyl bedeutet und R², R³, R⁴, R⁵, R⁶ und n wie in Formel (I) definiert sind, [= Verbindungen der Formel (Ia"')] oder
R² und R³ jeweils Wasserstoff bedeuten und R¹, R⁴, R⁵, R⁶ und n wie in Formel (I) definiert sind [= Verbindungen der Formel (Ia"")].

Besonders bevorzugt sind auch die Verbindungen der allgemeinen Formel (Ib) oder deren Salze, worin
- R¹: Wasserstoff bedeutet und R², R³, R⁴, R⁵, R⁶ und n wie in Formel (I) definiert sind [= Verbindungen der Formel (Ib")] oder
- R¹: Methyl bedeutet und R², R³, R⁴, R⁵, R⁶ und n wie in Formel (I) definiert sind, [= Verbindungen der Formel (Ib"')] oder
R² und R³ jeweils Wasserstoff bedeuten und R¹, R⁴, R⁵, R⁶ und n wie in Formel (I) definiert sind [= Verbindungen der Formel (Ib"")].

Besonders bevorzugt sind die Verbindungen der allgemeinen Formel (Ic) oder deren Salze, worin
- R¹: Wasserstoff bedeutet und R², R³, R⁴, R⁵, R⁶ und n wie in Formel (I) definiert sind [= Verbindungen der Formel (Ic")] oder
- R¹: Methyl bedeutet und R², R³, R⁴, R⁵, R⁶ und n wie in Formel (I) definiert sind, [= Verbindungen der Formel (Ic"')] oder
R² und R³ jeweils Wasserstoff bedeuten und R¹, R⁴, R⁵, R⁶ und n wie in Formel (I) definiert sind [= Verbindungen der Formel (Ic"")].

Dabei sind besonders die Verbindungen der Formel (I), (Ia), (Ib), oder (Ic) oder deren Salze bevorzugt, in welcher ein oder mehrere der Reste R¹ bis R⁶ die in den Beispieltabellen verwendeten Restebedeutungen aufweisen.

Dabei sind besonders die Verbindungen der Formel (I) und deren Salze bevorzugt, in welcher ein oder mehrere der Reste R¹ bis R⁶ die in den Beispieltabellen verwendeten Restebedeutungen aufweisen.

Die erfindungsgemäßen Verbindungen der Formel (I) umfassen alle Stereoisomeren, welche aufgrund der Asymmetriezentren oder Doppelbindungen im Molekül, deren Konfiguration in der Formel nicht speziell bezeichnet oder die nicht speziell angegeben sind, auftreten können, und deren Mischung, inklusive der racemischen Verbindungen und der teilweise mit bestimmten Stereoisomeren angereicherten Mischungen.

Die Erfindung umfasst auch alle Tautomeren, wie Keto- und Enol-Tautomeren, und deren Mischungen und Salze, wenn entsprechende funktionelle Gruppen vorhanden sind.

Gegenstand der vorliegenden Erfindung sind auch Verfahren zur Herstellung der Verbindungen der allgemeinen Formel (I) und/oder deren Salze.

Die erfindungsgemäßen Verbindungen der Formel (I) können alternativ durch verschiedene Verfahren dargestellt werden.

In den nachfolgenden Verfahren werden partiell Lösemittel (gleichbedeutend mit "Lösungsmittel") verwendet. In diesem Zusammenhang bezeichnen "inerte Lösemittel" jeweils Lösemittel, die unter den jeweiligen Reaktionsbedingungen inert sind, jedoch nicht unter beliebigen Reaktionsbedingungen inert sein müssen.

In den nachfolgenden Verfahren a), b), c), d), e), f), g), h), i) können die beschriebenen Reaktionen alternativ auch in einem Mikrowellenofen durchgeführt werden.
(a) Zur Herstellung von Verbindungen der allgemeinen Formel (I) oder deren Salzen, worin Het, R¹, R², R³, R⁴, R⁵, R⁶ die gemäß Formel (I) zuvor angegebenen Bedeutungen haben, wird nach Herstellungsverfahren (a) eine Verbindung der Formel (II),

   H₂N-NH-Het(R⁶)ₙ (II)

   worin Het und (R⁶)ₙ wie in Formel (I) definiert ist,
   mit einer Verbindung der Formel (III), 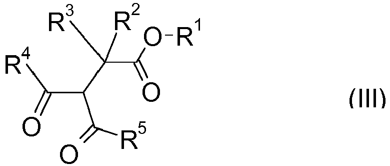
worin R¹, R², R³, R⁴ und R⁵ wie in Formel (I) definiert sind,
zur Verbindung der Formel (I) oder deren Salz umgesetzt.

Die beim erfindungsgemäßen Verfahren (a) zur Herstellung von Verbindungen der

Formel (I) als Ausgangsstoffe verwendeten substituierten 1,3-Dicarbonylverbindungen der Formel (III) sind vorzugsweise solche, bei denen die Reste R¹, R², R³, R⁴ und R⁵ die bevorzugten Bedeutungen aufweisen, die bereits oben im Zusammenhang mit der Beschreibung der erfindungsgemäßen Verbindungen der Formel (I) als bevorzugt angegeben worden sind. Die beim erfindungsgemäßen Verfahren (a) zur Herstellung von Verbindungen der Formel (I) als Ausgangsstoffe verwendeten substituierten Heteroarylhydrazine der Formel (II) hat daher auch vorzugsweise diejenigen Bedeutungen für (R⁶)ₙ, die bereits oben im Zusammenhang mit der Beschreibung der erfindungsgemäßen Verbindungen der Formel (I) vorzugsweise für (R⁶)ₙ,und insbesondere bevorzugt in Abhängigkeit der Reste Het angegeben worden sind.

Hydrazine der Formel (II) oder deren Salze als Ausgangsstoffe sind bekannt und/oder können nach bekannten Verfahren hergestellt werden (vgl. z.B. Methoden der organischen Chemie (Houben-Weyl, D. Klamann, Ed.) Band E16a, Teil 1, S. 421 ff., Georg Thieme Verlag, Stuttgart 1990 und dort zitierte Literatur; J. Am. Chem. Soc., 1954, 76, 596; Monatshefte für Chemie 1988, 119, 333; J. Heterocyclic Chem. 1988, 25, 1055; J. Heterocyclic Chem. 1989, 26, 475; Heterocycles 1994, 37, 379).

Die Umsetzung der Verbindungen der Formel (II) und (III) kann ohne Katalysator oder in Gegenwart von Katalysatoren, bespielsweise von einer Säure als Katalysator, erfolgen, vorzugsweise in einem organischen Lösungsmittel, wie z.B. Tetrahydrofuran (THF), Dioxan, Acetonitril, Dimethylformamid (DMF), Methanol und Ethanol, bei Temperaturen zwischen 20 °C bis zum Siedepunkt des Lösungsmittels, vorzugsweise bei 50 °C bis 150 °C. Falls Säureadditionssalze der Formel (II) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalihydrogencarbonate, Alkalialkoholate, Erdalkalihydroxide, Erdalkalihydride, Erdalkalicarbonate oder organische Basen wie Triethylamin, Diisopropyl-ethylamin oder 1,8-Diazabicyclo[5.4.0]undec-7-en (DBU).

Analoge Verfahren sind in der Literatur z. B. beschrieben in WO 2004/037793.
(b) Im Falle, dass R¹ in Formel (I) von Wasserstoff verschieden ist, ist das Herstellungsverfahren (b) dadurch gekennzeichnet, dass man eine Verbindung der Formel (I'), 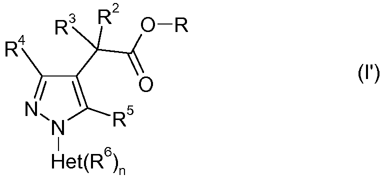 in welcher Het, R², R³, R⁴, R⁵ und R⁶ wie in Formel (I) definiert sind und
   - R: einen vom Rest R¹ unterschiedlichen, von Wasserstoff verschiedenen Rest, der aus der Gruppe der Reste, wie sie für R¹ definiert ist, ausgewählt ist, oder ein Anhydrid, Säurehalogenid oder einen aktivierten Ester der Verbindung der Formel (I'), worin R = H bedeutet, bedeutet,
   mit einer Verbindung der Formel (IV),

   R¹ - OH (IV)

   worin R¹ wie in Formel (I) definiert ist,
   zur Verbindung der Formel (I) umsetzt oder
(c) im Falle, dass R¹ in Formel (I) von Wasserstoff verschieden ist, ist das Herstellungsverfahren (c) dadurch gekennzeichnet, dass man eine Verbindung der Formel (I") 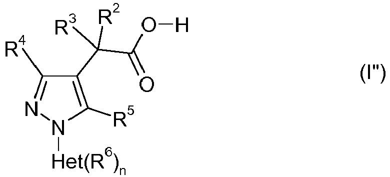 in welcher Het, R², R³, R⁴, R⁵ und R⁶ wie in Formel (I) definiert sind,
   gegebenenfalls nach Aktivierung der Säuregruppe, mit einer Verbindung der Formel (IV),

   R¹ - OH (IV)

   worin R¹ wie in Formel (I) definiert ist,
   zur Verbindung der Formel (I) umsetzt (verestert) oder
(d) im Falle, dass die Verbindung der Formel (I), worin R = H, oder deren Salz hergestellt wird, ist das Herstellungsverfahren (d) dadurch gekennzeichnet, dass man eine Verbindung der Formel (I') [siehe Definition in Variante (b)] zur Verbindung der Formel (I) oder deren Salz hydrolysiert.

Die Ausgangsverbindungen der Formeln (II), (III) und (IV) sind in der Regel bekannt oder können analog bekannten Verfahren hergestellt werden.

Die Umsetzung der Verbindungen der Formel (I') und (IV) kann nach Standardmethoden der Umesterung oder der Veresterung über aktivierte Carbonsäuren erfolgen.
Die Umsetzung der Verbindungen der Formel (I") und (IV) kann nach Standardmethoden der Versterungen oder gegebenenfalls über aktivierte Carbonsäuren erfolgen.
Die Herstellung von Verbindungen der Formel (I") aus Verbindungen (I') kann nach Standardmethoden der Verseifung erfolgen.
e) Die Verbindungen der Formel (III) lassen sich beispielsweise herstellen durch Umsetzung einer Dicarbonylverbindung der Formel (V)

   R⁴-CO-CH₂-CO-R⁵ (V)

   mit einer Verbindung der Formel (VI),

   R²R³C(Hal)-CO-OR¹ (VI)

   wobei R¹, R², R³, R⁴ und R⁵ wie in Formel (III) definiert sind, vorzugsweise R¹ Methyl oder Ethyl bedeutet, und Hal eine Abgangsgruppe, vorzugsweise ein reaktives Halogen wie ein Chlor-atom oder insbesondere ein Brom-atom, oder auch p-Toluolsulfonyl (Tosyl) oder Methylsulfonyl (Mesyl) bedeutet.

Die erfindungsgemäßen Verbindungen der Formel (I) können gemäß den genannten Verfahren a) bis e)analog bekannten Methoden dargestellt werden, wie sie z. B. in Methoden der organischen Chemie (Houben-Weyl, E. Schaumann, Ed.) Band E8b, Hetarene III, Teil 2, S. 399-710, Georg Thieme Verlag, Stuttgart 1994 und dort zitierter Literatur beschrieben sind, wobei die Synthesen nach Methoden der organischen Chemie (Houben-Weyl, E. Schaumann, Ed.) Band E8b, Hetarene III, Teil 2, S. 420 ff., Georg Thieme Verlag, Stuttgart 1994 und dort zitierte Literatur; Synthesis, 1986, 409; J. Chinese Chem. Soc., 2001, 48, 45 und besonders US 4146721, DE2141124 , DOS 1946370 und Justus Liebigs Ann. Chem. 1973, 1919 von besonderem Interesse sind.
f) Die Verbindungen der Formel (V) lassen sich beispielsweise auch herstellen durch Umsetzung einer Verbindung der Formel (VII)

   R⁵ - CO- OR⁷ (VII)

   mit einer Verbindung der Formel (VIII),

   CH₃-CO-R⁴ (VIII)

   wobei R⁴ und R⁵ wie in Formel (I) definiert sind, R⁷ (C₁-C₆)Alkyl bedeutet, vorzugsweise Methyl oder Ethyl bedeutet, in Gegenwart einer geeigneten organischen Base wie z. B. Natriummethanolat oder Natriumethanolat, in einem geigneten Lösemittel, z. B. Methanol, Ethanol, oder bevorzugt Tetrahydrofuran, in einem Temperaturbereich zwischen -10 und 50°C, bevorzugt 0°C und gegebenenfalls unter einer Inertgas-Atmosphäre, z.B. Stickstoff.

Analoge Reaktionen für die Umsetzung sind in der Literatur beschrieben, z.B. Supramolecular Chemistry (2003), 15(7-8), 529-547; J. Am. Chem. Soc. (1951), 73 5614-16; J. of Med. Chem. (1990), 33(7), 1859-65; WO 00/08002.

Alternativ können Verbindungen der Formel (V) auch durch Umsetzung einer Verbindung der Formel (IX)

R⁴-CO-OR⁷ (IX)

mit einer Verbindung der Formel (X),

CH₃ - CO - R⁵ (X)

unter analogen Bedingungen wie oben unter f) beschrieben, erhalten werden, wobei R⁴ und R⁵ wie in Formel (I) definiert sind, R⁷ (C₁-C₆)Alkyl bedeutet, vorzugsweise Methyl oder Ethyl bedeutet.

Analoge Reaktionen für die Umsetzung sind in der Literatur beschrieben, z. B. in J. Am. Chem. Soc. (1950), 72 1352-6.
g) Zur Herstellung einer Verbindung der allgemeinen Formel (I), 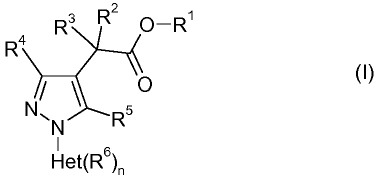 worin Het, R¹, R², R³, R⁴, R⁵ und R⁶ wie in Formel (I) definiert sind, kann
   beispielsweise auch eine Verbindung der allgemeinen Formel (XI), mit einem Bor-Derivat der Formel (XII), in Gegenwart eines geeigneten Cu(I) oder Cu(II) Salzes und einer organischen Base gegebenenfalls in einem Lösemittel zur Reaktion gebracht werden, wie in folgenden Schema dargestellt: 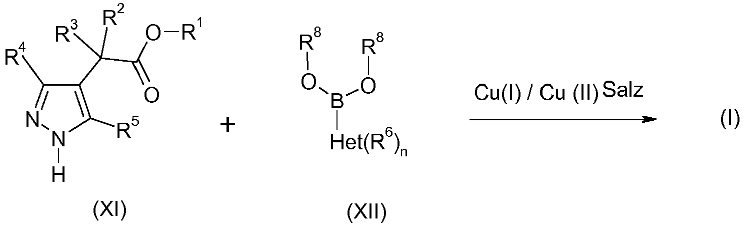
worin Het, R¹, R², R³, R⁴, R⁵, R⁶, die gemäß Formel (I) zuvor angegebenen Bedeutungen haben, und R⁸ gleich H oder (C₁-C₆)-Alkyl, bevorzugt Methyl, oder beide Alkylreste R⁸ zyklisch miteinander verknüpft sind.

Die Umsetzung wird in Gegenwart eines geeigneten anorganischen oder organischen Kupfer(I)- oder Kupfer(II)-Salzes, bevorzugt Cul, Cu₂O, besonders bevorzugt Cu(OAc)₂, eingesetzt als 0.1 bis 1.5 Äquivalenten, mit mehr als einem Äquivalent Bor-Derivat (XII), bevorzugt zwischen 1.5 und 2.5 Äquivalenten, durchgeführt. Im Fall wo der Kupfer-Katalysator nicht stöchiometrisch eingesetzt wird, kann den Zusatz eines milden Oxidationsmittels helfen, geeignet sind z.B 2,2,6,6-Tetramethyl-1-piperiniloxy (TEMPO), Pyridin-Oxid, Sauerstoff oder trockene Luft.
Dazu gibt man eine geeignete organischen Base wie z.B. Pyridin, Triethylamin oder Kalium-tert-butoxid und, um die Transmetallierung effizienter zu gestalten, eine Fluorid-Anionen Quelle, bevorzugt Cäsiumfluorid zu.
Man arbeitet in einem geeigneten Lösemittel, bevorzugt einem halogenierten Lösemittel z.B. Trichlormethan oder bevorzugt Dichlormethan, in einem Temperaturbereich zwischen 0 und 40°C, bevorzugt zwischen 20 und 30°C und gegebenenfalls unter einer Inertgas-Atmosphäre, z.B. Stickstoff, bis zur erfolgten Umsetzung, wobei teilweise lange Reaktionszeiten erforderlich sein können..

Analoge Methoden für Kupfer-induzierte C-N Kupplungen sind in der Literatur beschrieben, z.B. in Tet. Lett. 1998, 39, 2941; Tet. Lett. 1998, 39, 2933; Tet. Lett. 44 (2003) 3863-3865; J. Comb. Chem. 2004, 6, 385-390; Tet. Lett. 41 (2000) 9053 bis 9057.

Analoge Methoden für Kupfer-induzierte C-N Kupplungen in Gegenwart von Fluorid-Anionen sind in der Literatur beschrieben, z.B. in Eur. J. Org. Chem. 2007, 1318-1323 und Org. Lett. 2007, 9 (5), 761.

Analoge Methoden für katalytische Kupfer-induzierte C-N Kupplungen sind in der Literatur beschrieben, z.B. in Tet. Lett. 2001, 3415; Org. Lett. 2003, 5 (23), 4397 und Org. Lett. 2001, 3 (13), 2077.

Analoge Methoden für Kupfer-induzierte C-N Kupplungen in einem Microwellenreaktor sind in der Literatur beschrieben, z.B. J. Comb. Chem. 358 2008, 10, 358-360.

Verbindungen der allgemeinen Formel (XI) sind nach dem Fachmann bekannten Verfahren darstellbar, beispielsweise durch Umsetzung von einer Verbindung der allgemeine Formel (III), worin R¹, R², R³, R⁴ und R⁵ wie in Formel (I) definiert sind, mit Hydrazin(hydrat) wie beschrieben in Can. J. Chem. 2001, 79 (2), 183-194.

Verbindungen der allgemeinen Formel (XII) sind dem Fachmann bekannt und teilweise kommerziell erhältlich oder nach dem Fachmann bekannten Verfahren darstellbar, beispielsweise wie beschrieben in a) Science of Synthesis, Houben-Weyl (Methods of Molecular Transformations), Category 2, Volume 6, Ed. E. Schaumann; b) Houben-Weyl (Methoden der organische Chemie), Band 13, Organoborverbindungen I-Teil 3a, Ed. E. Schaumann.
h) Zur Herstellung einer Verbindung der allgemeinen Formel (I), 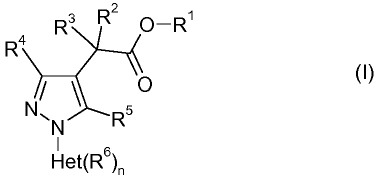 worin Het, R¹, R², R³, R⁴, R⁵ und R⁶ wie in Formel (I) definiert sind, kann beispielsweise auch eine Verbindung der allgemeinen Formel (XI), mit einer Verbindung der Formel (XIII), worin R⁶ die gemäß Formel (I) zuvor angegebene Bedeutung hat, in Gegenwart eines geeigneten Katalysator/Liganden-Systems, mit einer geeigneten Base und in einem geeigneten Lösemittel zur Reaktion gebracht werden, wie in folgendem Schema dargestellt: 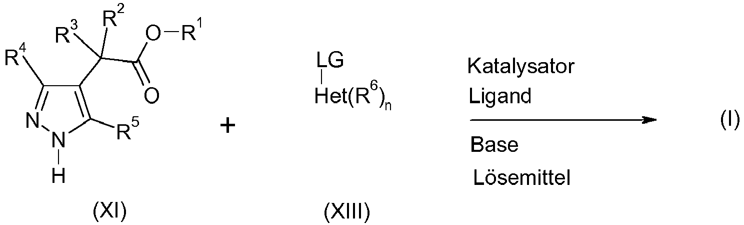
worin Het, R¹, R², R³, R⁴, R⁵, R⁶, die gemäß Formel (I) zuvor angegebenen Bedeutungen haben. LG bedeutet eine Abgangsgruppe, wobei als Abgangsgruppen beispielsweise Chlor, Brom, Iod, Phenylsulfonat, Tosylat oder Triflat fungieren können.

Verbindungen der Formel (XIII) können mit Verbindungen der Formel (XI) in Gegenwart eines geeigneten Katalysator/Liganden-System zu Verbindungen der Formel (I) umgesetzt werden. Die Reaktion wird bevorzugt unter Anwendung eines geeigneten anorganischen oder organischen Kupfer(I)- oder Kupfer(II)-Salzes, bevorzugt, z. B. Cu(OAc)₂ (Kupferdiacetat), Cu(Acac)₂ (Kupferdiacetylacetat), Cul (Kupferiodid), CuBr (Kupferbromid), Cu₂O (Kupfer-(I)-oxid), [Cu(OH)TMEDA]₂Cl₂ (ein Kupferkomplexsalz, TMEDA bedeutet "Tetramethylethylendiamin"), oder Cu(0) (Kupfer-(II)-oxid), durchgeführt, mit einem geeigneten Liganden wie z.B. L-Proline, N,N'-Dimethylethan-1,2-diamin, trans- N,N'-Dimethylcyclohexan-1,2-diamin, Dimethylglycin, Salicylaldoxim, 1,1'-Binaphthalen-2,2'-diol (BINOL) und einer organischen oder anorganische Base wie z.B. Triethylamin, Pyridin, 2,6-Lutidine, Cäsiumcarbonat, Kaliumcarbonat, Kaliumphosphat gegebenenfalls in einem Lösemittel, wie z.B. Toluol, 1,4-Dioxan, Dichlormethan, Dimethylformamid, Dimethylacetamid, Acetonitril.

Man kann die Reaktion auch in Gegenwart eines geeignetes Palladium-Katalysators, z. B. Palladium(II)acetat, oder Dipalladium-tri-[(1E,4E)-1,5-diphenylpenta-1,4-dien-3-on] durchführen, mit einem Phosphin Liganden wie z. B. 2,2'-Bis(diphenylphospino)-1,1'-binaphthyl (BINAP), 1,1'-Diphenylphosphinoferrocene (DPPF), 2-Di-tert-butylphosphinobiphenyl (JohnPhos), 2-Dicyclohexylphosphino-2'-dimethylaminobiphenyl (DavePhos), 2-Dicyclohexylphosphino-2',4',6'-triisopropylbiphenyl (XPhos), 2-Dicyclohexylphosphino-2'-methylbiphenyl (MePhos), 4,5-Bis(diphenylphosphino)xanthene (XANTPHOS), Tri-tert-butylphosphoniumtetrafluoroborat oder Di-tert-butylphosphinsäure mit einer anorganische Base wie z.B. Cäsiumcarbonat, Kaliumcarbonat, Kaliumphosphat gegebenenfalls in einem Lösemittel, wie z.B. Toluol, 1,4-Dioxan, Dichlormethan, Dimethylformamid, Dimethylacetamid oder Acetonitril oder deren Gemischen.

Man kann die Reaktion auch in Gegenwart eines geeigneten Fe(III) Komplexes, durchführen, wie z.B. Eisen(III)oxid, Eisentrichlorid oder Eisentriacetylacetonat mit einem geeignetem Liganden wie z.B. L-Prolin, N,N'-Dimethylethan-1,2-diamin, trans- N,N'-Dimethylcyclohexan-1,2-diamin, oder Dimethylglycin und einer anorganische Base wie z.B. Cäsiumcarbonat, Kaliumcarbonat, Kaliumphosphat gegebenenfalls in einem Lösemittel, wie z.B. Toluol, 1,4-Dioxan, Dichlormethan, Dimethylformamid, Dimethylacetamid oder Acetonitril oder deren Gemischen.

Man kann die Reaktion auch in Gegenwart eines Gemisches der Palladium- und Kupfer- oder Eisen- und Kupfer -basierten Katalysatoren durchführen.

Die Umsetzung wird in der Regel in Gegenwart von mehr als einem Äquivalent einer Verbindung der Formel (XI), bevorzugt zwischen 1 und 2 Äquivalenten, oder in Gegenwart von mehr als einem Äquivalent einer Verbindung der Formel (XIII), bevorzugt zwischen 1 und 1.5 Äquivalenten, durchgeführt.
Man arbeitet beispielsweise in einem Temperaturbereich zwischen 0 und 150°C, bevorzugt zwischen 60 und 120°C und gegebenenfalls unter einer Inertgas-Atmosphäre, z.B. Stickstoff, bis zur erfolgten Umsetzung, wobei teilweise lange Reaktionszeiten erforderlich sein können.

Analoge Methoden für Kupfer-induzierte C-N Kupplungen sind in der Literatur beschrieben, z. B. Tet. Lett. 49 (2008) 948-951; Eur. J. Org. Chem. 2004, 695, 709; J. Org. Chem. 2007, 72, 2737- 2743; Heterocycles, 61, 2003, 505 - 512; Heterocycles, 48 (11), 1998, 2225; J. Am. Chem. Soc. 2002, 124, 11684-11688; J. Org. Chem. 2004, 69, 5578-5587; J. Org. Chem. 2007, 72, 8535-8538; Org. Lett. 2000, 2 (9), 1233-1236; Journal of Molecular Catalysis A: Chemical (2006), 256(1-2), 256-260; Acc.Chem. Res. (2008), 41(11), 1450-1460, J. Mol. Catal. A: Chemical 256 (2006) 256-260).

Analoge Methoden für Palladium-induzierte C-N Kupplungen sind in der Literatur beschrieben, z.B. in J. Org. Chem. 2001, 66, 8677; J. Org. Chem. 1999, 64, 6019-6022; Angew. Chem. Int. Ed. 2005, 44, 1371 -1375; Heterocycles, 48, 11, 1998, 847; Tetrahedron 61 (2005) 2931-2939; Angew. Chem. Int. Ed. 2006, 45, 6523 - 6527.

Analoge Methoden für Eisen-induzierte C-N Kupplungen sind in der Literatur beschrieben, z.B. in Angew. Chem. Int. Ed. 2007, 46, 934; Angew. Chem. Int. Ed. 2007, 46, 8862 -8865.

Analoge Methoden für Kupfer/Eisen-induzierte C-N Kupplungen sind in der Literatur beschrieben, z.B. in Angew. Chem. Int. Ed. 2007, 46, 934; Tet. Lett. 39 (1998) 5617-5620.

Analoge Methoden für Kupfer-induzierte C-N Kupplungen in einem Microwellenreaktor sind in der Literatur beschrieben, z.B. J. Comb. Chem. 358 2008, 10, 358-360.

Verbindungen der allgemeinen Formel (XI) sind nach dem Fachmann bekannten Verfahren darstellbar, beispielsweise durch Umsetzung von einer Verbindung der allgemeine Formel (III), worin R¹, R², R³, R⁴ und R⁵ wie in Formel (I) definiert sind, mit Hydrazin(hydrat), wie beschrieben in Can. J. Chem. 2001, 79 (2), 183-194.

Verbindungen der allgemeinen Formel (XIII) sind dem Fachmann bekannt und teilweise kommerziell erhältlich oder nach dem Fachmann bekannten Verfahren darstellbar, beispielsweise wie beschrieben in Science of Synthesis, Houben-Weyl (Methods of Molecular Transformations), Category 2, Volume 16, Ed. E. Schaumann.
i) Als Ausgangsstoff zur Herstellung einer Verbindung der genannten allgemeinen Formel (I), worin Het, R¹, R², R³, R⁴, R⁵ und R⁶, wie in Formel (I) definiert sind, kann beispielsweise auch eine Verbindung der allgemeinen Formel (XV) dienen, die durch Umsetzung einer Verbindung der Formel (XIII), worin R⁶ die gemäß Formel (I) zuvor angegebene Bedeutung hat, mit Benzophenonhydrazon (XIV), in Gegenwart eines geeigneten Katalysator/Liganden-Systems, mit einer geeigneten Base und in einem geeigneten Lösemittel hergestellt wird. Verbindungen der allgemeinen Formel (XV) ergeben mit einer Verbindung der allgemeinen Formel (III), in Gegenwart einer Säure, gegebenenfalls in einem Lösemittel, die Verbindung der allgemeinenFormel (I), wie in folgendem Schema dargestellt:

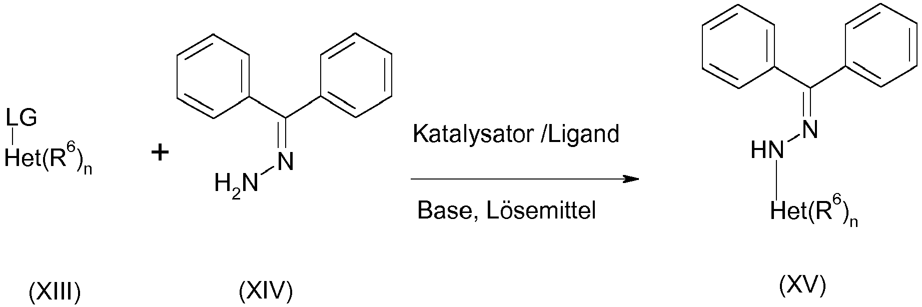

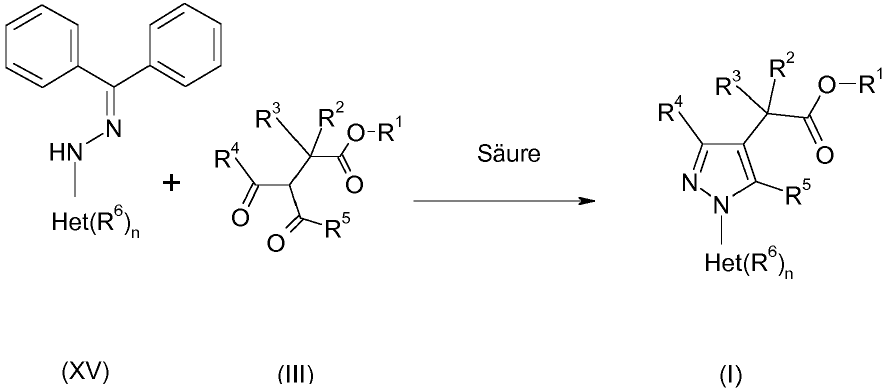

Hierin haben Het, R¹, R², R³, R⁴, R⁵, R⁶ die gemäß Formel (I) zuvor angegebenen Bedeutungen, LG bedeutet eine Abgangsgruppe, wobei als Abgangsgruppen beispielsweise Chlor, Brom, Iod, Phenylsulfonat, Tosylat oder Triflat fungieren können.

Verbindungen der Formel (XIII) können mit Benzophenonhydrazon (XIV) in Gegenwart eines Katalysators und einem geeigneten Katalysator/Liganden-System zu Verbindungen der Formel (XV) umgesetzt werden. Die Reaktion wird bevorzugt unter Anwendung eines Palladium-Katalysators, z. B. Palladium(II)acetat, durchgeführt, mit einem Phosphin Liganden wie z. B. 2,2'-Bis(diphenylphospino)-1,1'-binaphthyl (BINAP), 1,1'-Diphenylphosphinoferrocene (DPPF), 2-Di-tert-butylphosphinobiphenyl (JohnPhos), 2-DiCyclohexylphosphino-2'-dimethylaminobiphenyl (DavePhos), 2-Dicyclohexylphosphino-2',4',6'-triisopropylbiphenyl (XPhos), 2-Dicyclohexylphosphino-2'-methylbiphenyl (MePhos), 4,5-Bis(diphenylphosphino)xanthene (XANTPHOS). Das Verwenden einer Base ist von Vorteil, z. B. Natrium-tert-butoxid. Die Reaktion wird in der Regel unter einer Inertgas-Atmosphäre, z.B. Stickstoff, unter Ausschluss von Wasser in einem geeigneten Lösemittel, z. B. Toluol, durchgeführt.

Benzophenonhydrazon ist kommerziell erhältlich.

Verbindungen der Formel (XV) können direkt weiter mit Verbindungen der Formel (III) zu Verbindungen der Formel (I) umgesetzt werden. Die Reaktion findet in Gegenwart einer geeigneten anorganischen oder organischen (nicht)wässrigen Säure statt, bevorzugt p-Toluolsulfonsäure, Schwefelsäure, besonders bevorzugt Salzsäure, wobei beispielsweise zwischen 1 und 10, bevorzugt zwischen 3 bis 7, besonders bevorzugt etwa 5 Äquivalente der Säure eingesetzt werden.
Die Reaktion wird beispielsweise in einem geeigneten Lösemittel, z.B. Diethylether, Dioxan oder bevorzugt Tetrahydrofuran, in einem Temperaturbereich zwischen 0 und 80°C, bevorzugt 50°C und gegebenenfalls unter einer Inertgas-Atmosphäre, z.B. Stickstoff, durchgeführt.

Analoge Zyklisierungsreaktionen eines Hydrazons mit einem 1,3-Diketon zu einem Pyrazol sind in der Literatur beschrieben, z.B. in WO-A-2001/32627; Angew. Chem. 110 (1998) 2249-2252; Tet. Lett. 43 (2002) 2171-2173; J.Am. Chem. Soc. 1981, 103, 7743 - 7752; Organic Process Research and Development 2004, 8, 1065 - 1071; Tet. Lett. 45 (2004) 5935-5937; WO-A-2007/064872, US 6489512, WO-A-2006/114213.

Verbindungen der Formel (XV) können auch zu Verbindungen der Formel (II) umgesetzt werden, beispielsweise nach den dem Fachmann bekannten Verfahren, in Anwesenheit von Säure, bevorzugt unter teils wässrigen Bedingungen. Die Verbindungen der Formel (II) können weiter zu Verbindungen der Formel (I) nach obengenanntem Verfahren a) reagieren.

Verbindungen der allgemeinen Formel (XIII) sind dem Fachmann bekannt und teilweise kommerziell erhältlich oder gemäß dem Fachmann bekannten Verfahren darstellbar, beispielsweise wie beschrieben in Science of Synthesis, Houben-Weyl (Methods of Molecular Transformations), Category 2, Volume 16, Ed. E. Schaumann.

Verbindungen der allgemeinen Formel (XV) sind herstellbar wie z. B. beschrieben in Tet. Lett. 45 (2004) 5935-5937; Angew. Chem. Int. Ed. 2006, 45, 6523-6527; J. Am. Chem. Soc. (2003) 125, 13978-13980; J. Am. Chem. Soc. (2003), 125, 6653-6655; Org. Lett. 3 (9) (2001) 1351; Tet. Lett. 45 (2004) 5935-5937; Tetr. Lett. 43 (2002) 2171-2173; Angew. Chem. Int. Ed. 1998, 37 (15) 2090; WO2001/32627; J. Am. Chem. Soc. (1998) 120, 6621; WO-2007/064872; US-6489512; WO-2006/114213; US-2005/0192294, J. Am. Chem. Soc. 2003, 125, 6653 - 6655; Org. Lett. 2001, 3 (9), 1351 - 1354.
j) Zur Herstellung einer Verbindung der genannten Formel (I), worin Het, R¹, R², R³, R⁴, R⁵ und R⁶ wie in Formel (I) definiert sind, kann beispielsweise auch eine Verbindung der allgemeinen Formel (XVI), wobei R⁶ wie in Formel (I) definiert ist, mit Di-tert-butyl-azo-dicarboxylat (DBAD, XVII) in Gegenwart eines geeigneten Kupfer-Salzes in einem geeigneten Lösemittel zu einer Verbindung der allgemeinen Formel (XVIII) umgesetzt werden, worin R⁶ wie in Formel (I) definiert ist. Man erhält Verbindungen der Formel (II) oder deren Salze, worin R⁶ wie in Formel (I) definiert ist, die nach Verfahren a) zu Verbindungen der Formel (I), worin R¹, R², R³, R⁴, R⁵ und R⁶ wie in Formel (I) definiert sind, umgesetzt werden können, wie in folgenden Schema dargestellt:

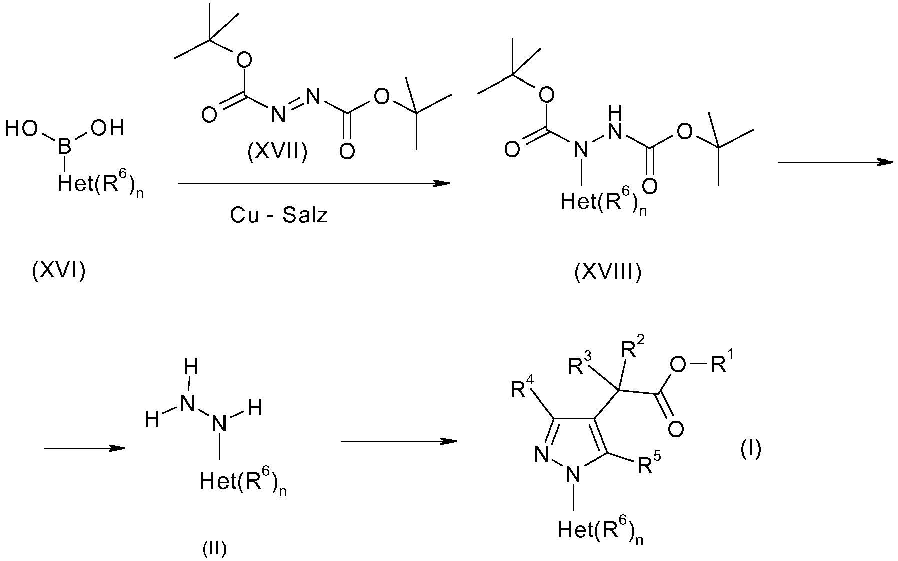

Die Umsetzung wird beispielsweise in Gegenwart eines geeigneten anorganischen Kupfer-Salzes, beispielsweise Cu(OAc)₂ (Kupferdiacetat) oder dessen Monohydrat Cu(OAc)₂.H₂O, in einem geeigneten Lösemittel, z.B. in einem Alkohol, wie Methanol, in einem Temperaturbereich zwischen 0 und 40°C, bevorzugt 20 - 25°C und gegebenenfalls unter einer Inertgas-Atmosphäre, z.B. Stickstoff, durchgeführt. Analoge Reaktionen unter Verwendung von kommerziell erhältlichem Di-tert-butyl-(E)-diazen-1,2-dicarboxylat (DTBAD) sind in der Literatur beschrieben, z.B.. Org. Lett. (2006) 8 (1), 43-45; J. Org. Chem. 2005, 70, 8631-8634.

Verbindungen der allgemeinen Formel (XVI) sind kommerziell erhältlich und/oder nach dem Fachmann bekannten Verfahren darstellbar, z.B. wie beschrieben in a) Science of Synthesis, Houben-Weyl (Methods of Molecular Transformations), Category 2, Volume 6, Ed. E. Schaumann; b) Houben-Weyl (Methoden der organische Chemie), Band 13, Organoborverbindungen I-Teil 3a, Ed. E. Schaumann.

Verbindungen der allgemeinen Formel (XVIII) können zu Verbindungen der allgemeinen Formel (II) nach dem Fachmann bekannten Verfahren umgesetzt werden, z. B wie beschrieben in J. Med. Chem. 1998, 41, 2858-2871; Tetrahedron 44 (17), 5525 (1988); J. Med. Chem. 1996, 39, 1172-1188; J. Org. Chem. 2004, 69, 5778-5781. Verbindungen der allgemeine Formel (II) oder deren Salze können nach obengenanntem Verfahren a) zu Verbindungen der Formel (I) umgesetzt werden.
k) Zur Herstellung einer Verbindung der genannten Formel (I), worin Het, R¹, R², R³, R⁴, R⁵ und R⁶ wie in Formel (I) definiert sind, kann beispielsweise auch eine Verbindung der allgemeinen Formel (XIX), worin R⁶ wie in Formel (I) definiert ist und LG' eine geeignete Gruppe bedeutet, wobei als geeignete Gruppen beispielsweise Brom und Iod fungieren können, mit einem geeigneten Metall oder einem geeigneten Transmetallierungsreagenz zu einer Verbindung der Formel (XX) umgesetzt werden, die wiederum, mit Di-tert-butyl-(E)-diazen-1,2-dicarboxylat (DTBAD, XVII) in Gegenwart eines geeigneten Lösemittels zu einer Verbindung der allgemeinen Formel (XVIII) umgesetzt wird, worin R⁶ wie in Formel (I) definiert ist. Man erhält Verbindungen der Formel (II) oder deren Salze, worin R⁶ wie in Formel (I) definiert ist, die nach Verfahren a) zu Verbindungen der Formel (I), R¹, R², R³, R⁴, R⁵ und R⁶ wie in Formel (I) definiert sind, umgesetzt werden können wie in folgenden Schema dargestellt:

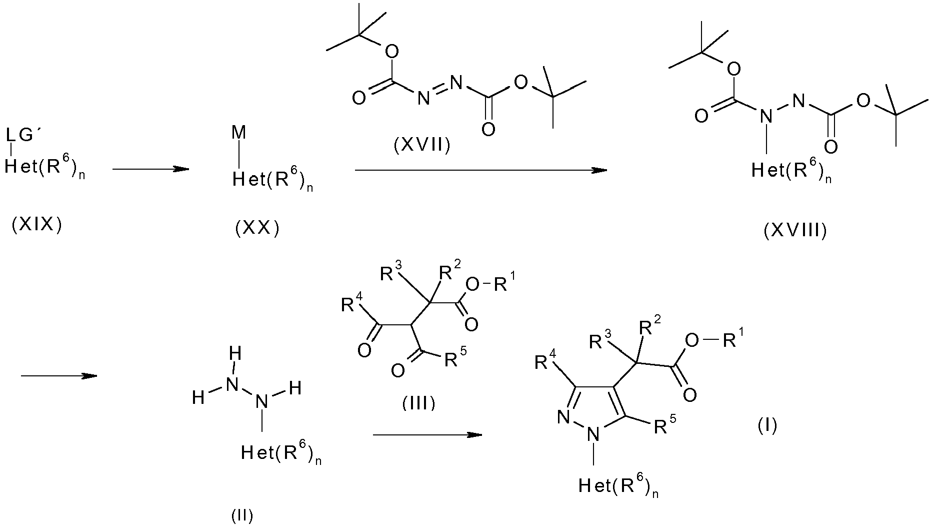

Die Umsetzung zu einer Verbindung der allgemeinen Formel (XX) wird beispielsweise in Gegenwart eines geeigneten Transmetallierungsreagenzes, z. B. einer Alkyllithium-Base, bevorzugt BuLi (Butyllithium); oder eines Metalls, bevorzugt Li, Mg oder Zn, durchgeführt. Das so entstandene Nucleophil wird mit Di-tert-butyl-(E)-diazen-1,2-dicarboxylat (DTBAD, XVIII) zu einer Verbindung der allgemeine Formel (XVIII) weiter umgesetzt. Analoge Reaktionen unter Verwendung von kommerziell erhältlichem Di-tert-butyl-azo-dicarboxylat (DBAD) sind in der Literatur beschrieben, z.B.. Tet. Lett. 1987, 28 (42), 4933; Tet. Lett. 39 (1998) 9157-9160.

Verbindungen der allgemeinen Formel (XIX) sind kommerziell erhältlich und/oder nach dem Fachmann bekannten Verfahren darstellbar, z.B. wie beschrieben in Science of Synthesis, Houben-Weyl (Methods of Molecular Transformations), Category 2, Volume 16, Ed. E. Schaumann.

Verbindungen der allgemeinen Formel (XVIII) können zu Verbindungen der allgemeine Formel (II) nach dem Fachmann bekannten Verfahren umgesetzt werden, z.B wie beschrieben in J. Med. Chem. 1998, 41, 2858-2871; Tetrahedron 44 (17), 5525 (1988); J. Med. Chem. 1996, 39, 1172-1188; J. Org. Chem. 2004, 69, 5778-5781. Verbindungen der allgemeine Formel (II) oder deren Salze können nach obengenannten Verfahren a) zu Verbindungen der Formel (I) umgesetzt werden.

Die Ausgangsstoffe der allgemeinen Formel (III) können nach allgemein bekannten Verfahren durch Alkylierung entsprechender 1,3-Diketone mit 2-halogenierten Essigsäurederivaten, beispielsweise Bromessigsäure-Derivaten erhalten werden (vgl. z.B. DE-OS 1946370, S. 13). Die hierfür als Ausgangsstoffe verwendeten 1,3-Diketone (V) sind nach obengenannten Verfahren f) herstellbar oder kommerziell erhältlich oder bekannt und/oder können nach bekannten Verfahren hergestellt werden (siehe z.B. US 4146721, DE2141124, DOS1946370 oder J. Am. Chem. Soc., 1948, 70, 4023; Justus Liebigs Ann. Chem. 1973, 1919; Justus Liebigs Ann. Chem. 1976, 13; J. Chem. Soc. Perkin Trans. 2, 1993, 6, 1067; Heteroatom Chemistry, 1997, 8, 147).

Für die Herstellung von Enantiomeren der Verbindungen (I) kommen auch übliche Racemattrennungsmethoden in Frage (vgl. Handbücher der Stereochemie), z. B. im Anschluss an Verfahren zur Trennung von Gemischen in Diastereomere, z. B. physikalische Verfahren wie Kristallisation, Chromatographieverfahren, vor allem Säulenchromatographie und Hochdruckflüssigchromatographie, Destillation, gegebenenfalls unter reduziertem Druck, Extraktion und andere Verfahren, können verbleibende Gemische von Enantiomeren in der Regel durch chromatographische Trennung an chiralen Festphasen getrennt werden. Für präparative Mengen oder im industriellen Maßstab kommen Verfahren wie die Kristallisation diastereomerer Salze, die aus den Verbindungen (I) mit optisch aktiven Säuren und gegebenenfalls bei vorhandenen sauren Gruppen mit optisch aktiven Basen erhalten werden können, in Frage.

Zur Racemattrennung durch Kristallisation diastereomerer Salze kommen als optisch aktive Säure z. B. Camphersulfonsäure, Camphersäure, Bromcamphersulfonsäure, Chinasäure, Weinsäure, Dibenzoylweinsäure und andere analoge Säure in Betracht; als optisch aktive Basen kommen z. B. Chinin, Cinchonin, Chinidin, Brucin, 1-Phenylethylamin und andere analoge Basen in Frage.
Die Kristallisationen werden dann meist in wässrigen oder wässrig-organischen Lösungsmittel durchgeführt, wobei das Diastereomer mit der geringeren Löslichkeit gegebenenfalls nach Animpfen zunächst ausfällt. Das eine Enantiomer der Verbindung der Formel (I) wird danach aus dem ausgefällten Salz oder das andere aus dem Kristallisat durch Ansäuern bzw. mit Base freigesetzt.

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzol und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat oder Kalium-tert.-butylat, oder Ammoniak, Ethanolamin oder quartäres Ammoniumhydroxid der Formel [NRR'R"R"']⁺ OH-.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und/oder deren Salzen enthalten.

Die erfindungsgemäßen Verbindungen der Formel (I) (und/oder deren Salze), im folgenden zusammen als "erfindungsgemäße Verbindungen", "erfindungsgemäße Verbindungen (I)" oder kurz als "Verbindungen (I)" bezeichnet, weisen eine ausgezeichnete herbizide Wirksamkeit gegen ein breites Spektrum wirtschaftlich wichtiger mono und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie monooder dikotyle Unkräuter oder unerwünschte Kulturpflanzen), das Saatgut (z.B. Körner, Samen oder vegetative Vermehrungsorgane wie Knollen oder Sprossteile mit Knospen), den Boden, in dem oder auf dem die Pflanzen wachsen, (z. B. den Boden von Kulturland oder Nicht-Kulturland) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen und/oder deren Salze in wirtschaftlich bedeutenden transgenen Kulturen von Nutz und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).

- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.
Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und/oder deren Salze als Herbizide zur Bekämpfung von Schadpflanzen in transgenen Kulturpflanzen.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Die erfindungsgemäßen Verbindungen (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) und/oder deren Salze enthalten.

Die Verbindungen der Formel (I) und/oder deren Salze können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf ÖI- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.
Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.
Emulsionen, z.B. ÖI-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden. Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I) und/oder dessen Salze.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.
Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifopbutyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate (Glufosinate-P), L-Glufosinate-ammonium (Glufosinate-P-ammonium), Glufosinate-P-natrium, Glufosinate-ammonium, Glyphosate, Glyphosate-isopropylammonium, H-9201, Halosafen, Halosulfuron, Halosulfuronmethyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HW-02, Imazamethabenz, Imazamethabenz-methyl, Imazamox, Imazamox-ammonium, Imazapic, Imazapyr, Imazaquin, Imazethapyr, Imazethapyr-ammonium, Imazosulfuron, Inabenfide, Indanofan, Indaziflam, Indolessigsäure (IAA), 4-Indol-3-ylbuttersäure (IBA), lodosulfuron, lodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methiozolin, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT 128, MT-5950, d. h. N-[3-Chlor-4-(1-methylethyl)-phenyl]-2-methylpentanamid, NGGC-011, Naproanilide, Napropamide, Naptalam, NC-620, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenzisopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobacnatrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosate-trimesium), Sulfosulfuron, SYN-449, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuronmethyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, ZJ-0862 sowie die folgenden Verbindungen

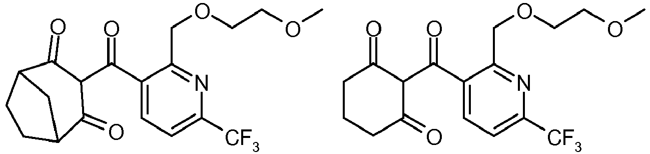

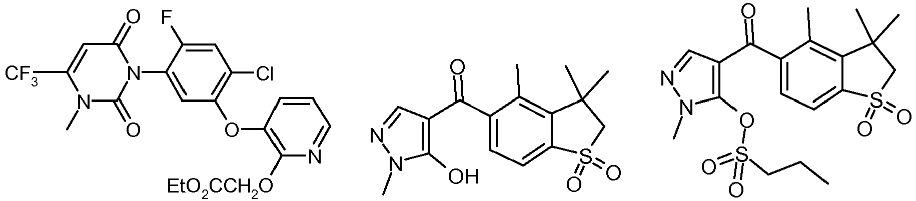

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
A) Verbindungen der Formel (S-1), 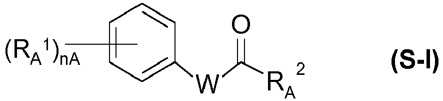 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl,
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W_{A}¹) bis (W_{A}⁴),

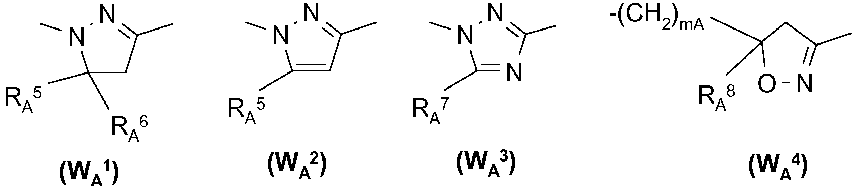

- m_{A}: ist 0 oder 1;
- R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-I) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
- R_{A}³: ist Wasserstoff oder ein unsubstituierter oder substituierter aliphatischer Kohlenwasserstoffrest, vorzugsweise mit insgesamt 1 bis 18 C-Atomen;
- R_{A}⁴: ist Wasserstoff, (C₁-C₆)Alkyl, (C₁-C₆)Alkoxy oder substituiertes oder unsubstituiertes Phenyl;
- R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
- R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;

### vorzugsweise:

a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäureethylester (S1-1) ("Mefenpyr-diethyl", siehe Pestic. Man.), und verwandte Verbindungen, wie sie in der WO 91/07874 beschrieben sind;
b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1 -(2,4-Dichlorphenyl)-5-(1,1 -dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind;
c) Verbindungen vom Typ der Triazolcarbonsäuren, vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-6), und verwandte Verbindungen wie sie in EP-A-174 562 und EP-A-346 620 beschrieben sind;
d) Verbindungen vom Typ der 5-Benzyl- oder 5-Phenyl-2-isoxazolin-3- carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-isoxazolin-carbonsäureethylester (S1-9) ("Isoxadifen-ethyl") oder -n-propylester (S1-10) oder der 5-(4-Fluorphenyl)-5-phenyl-2-isoxazolin-3-carbonsäureethylester (S1-11), wie sie in der Patentanmeldung WO-A-95/07897 beschrieben sind.

B) Chinolinderivate der Formel (S-II), 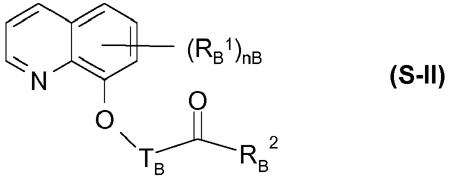 wobei die Symbole und Indizes folgende Bedeutungen haben:
   - R_{B}¹: Halogen, (C₁-C₄)Alkyl, (C₁-C₄)Alkoxy, Nitro oder (C₁-C₄)Haloalkyl,
   - n_{B}: ist eine natürliche Zahl von 0 bis 5, vorzugsweise 0 bis 3;
   - R_{B}²: OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-II) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;

### vorzugsweise:

a) Verbindungen vom Typ der 8-Chinolinoxyessigsäure (S2), vorzugsweise (5-Chlor-8-chinolinoxy)essigsäure-(1-methylhexyl)-ester (Common name "Cloquintocet-mexyl" (S2-1) (siehe Pestic. Man.), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie deren Hydrate und Salze wie sie in der WO-A-2002/034048 beschrieben sind.
b) Verbindungen vom Typ der (5-Chlor-8-chinolinoxy)malonsäure, vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.

C) Verbindungen der Formel (S-III) 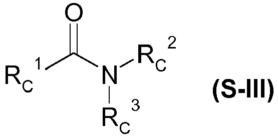 wobei die Symbole und Indizes folgende Bedeutungen haben:
   - Rc¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{c}², R_{c}³: ist gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder Rc² und Rc³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;

### vorzugsweise:

Wirkstoffe vom Typ der Dichloracetamide, die häufig als Vorauflaufsafener (bodenwirksame Safener) angewendet werden, wie z. B.
"Dichlormid" (siehe Pestic.Man.) (= N,N-Diallyl-2,2-dichloracetamid),
"R-29148" (= 3-Dichloracetyl-2,2,5-trimethyl-1,3-oxazolidin von der Firma Stauffer),
"R-28725" (= 3-Dichloracetyl-2,2,-dimethyl-1,3-oxazolidin von der Firma Stauffer),
"Benoxacor" (siehe Pestic. Man.) (= 4-Dichloracetyl-3,4-dihydro-3-methyl-2H-1,4-benzoxazin),
"PPG-1292" (= N-Allyl-N-[(1,3-dioxolan-2-yl)methyl]-dichloracetamid von der Firma PPG Industries),
"DKA-24" (= N-Allyl-N-[(allylaminocarbonyl)methyl]-dichloracetamid von der Firma Sagro-Chem),
"AD-67" oder "MON 4660" (= 3-Dichloracetyl-1-oxa-3-aza-spiro[4,5]decan von der Firma Nitrokemia bzw. Monsanto),
"TI-35" (= 1-Dichloracetyl-azepan von der Firma TRI-Chemical RT)
"Diclonon" (Dicyclonon) oder "BAS145138" oder "LAB145138" (= 3-Dichloracetyl-2,5,5-trimethyl-1,3-diazabicyclo[4.3.0]nonan von der Firma BASF) und
"Furilazol" oder "MON 13900" (siehe Pestic. Man.) (= (RS)-3-Dichloracetyl-5-(2-furyl)-2,2-dimethyloxazolidin)
D) N-Acylsulfonamide der Formel (S-IV) und ihre Salze, 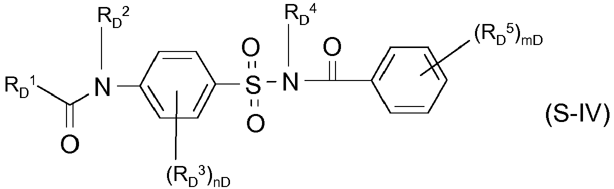 worin
   - R_{D}¹: Wasserstoff, einen Kohlenwasserstoffrest, einen Kohlenwasserstoffoxyrest, einen Kohlenwasserstoffthiorest oder einen Heterocyclylrest, der vorzugsweise über ein C-Atom gebunden ist, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist,
   wobei jeder Kohlenwasserstoffteil vorzugsweise 1 bis 20 C-Atome aufweist und ein C-haltiger Rest R_{D}¹ inklusive Substituenten vorzugsweise 1 bis 30 C-Atome aufweist;
   - R_{D}²: Wasserstoff oder (C₁-C₄)Alkyl, vorzugsweise Wasserstoff, oder
   - R_{D}¹ und R_{D}²: zusammen mit der Gruppe der Formel -CO-N- den Rest eines 3- bis 8-gliedrigen gesättigten oder ungesättigten Rings;
   - R_{D}³: gleich oder verschieden Halogen, Cyano, Nitro, Amino, Hydroxy, Carboxy, Formyl, CONH₂, SO₂NH₂ oder einen Rest der Formel -Z^{b}-R^{b} ;
   - R_{D}⁴: Wasserstoff oder (C₁-C₄)Alkyl, vorzugsweise H;
   - R_{D}⁵: gleich oder verschieden Halogen, Cyano, Nitro, Amino, Hydroxy, Carboxy, CHO, CONH₂, SO₂NH₂ oder einen Rest der Formel -Z^{c}-R^{c},
   - R^{a}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - R^{b},R^{c}: gleich oder verschieden einen Kohlenwasserstofifrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - Z^{a}: eine divalente Gruppe der Formel -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*- oder -NR*-SO₂-, wobei die rechts angegebene Bindung der jeweiligen divalenten Gruppe die Bindung zum Rest R^{a} ist und wobei die R* in den letztgenannten 5 Resten unabhängig voneinander jeweils H, (C₁-C₄)-Alkyl oder Halo-(C₁-C₄)-alkyl bedeuten;
   - Z^{b},Z^{c}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*-oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-alkyl bedeuten;
   - n_{D}: eine ganze Zahl von 0 bis 4, vorzugsweise 0, 1 oder 2, insbesondere 0 oder 1, und
   - m_{D}: eine ganze Zahl von 0 bis 5, vorzugsweise 0, 1, 2 oder 3, insbesondere 0, 1 oder 2;
   bedeuten;
E) Acylsulfamoylbenzoesäureamide der allgemeinen Formel (S-V), gegebenenfalls auch in Salzform, 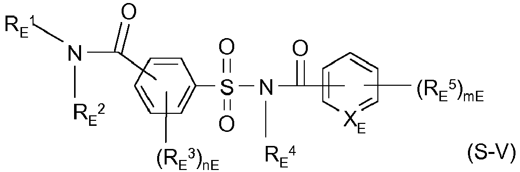 worin
   - X_{E}: CH oder N;
   - R_{E}¹: Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
   - R_{E}²: Wasserstoff, Hydroxy, (C₁-C₆)Alkyl, (C₂-C₆)Alkenyl, (C₂-C₆)Alkinyl, (C₁-C₆)Alkoxy, (C₂-C₆)Alkenyloxy, wobei die fünf letztgenannten Reste gegebenenfalls durch einen oder mehrere, gleiche oder verschiedene Reste aus der Gruppe Halogen, Hydroxy, (C₁-C₄)Alkyl, (C₁-C₄)Alkoxy und (C₁-C₄)Alkylthio substituiert sind, oder
   - R_{E}¹ und R_{E}²: zusammen mit dem sie tragenden Stickstoffatom einen 3- bis 8-gliedrigen gesättigten oder ungesättigten Ring;
   - R_{E}³: Halogen, Cyano, Nitro, Amino, Hydroxy, Carboxy, CHO, CONH₂, SO₂NH₂ oder Z^{b}-R^{b};
   - R_{E}⁴: Wasserstoff, (C₁-C₄)Alkyl, (C₂-C₄)Alkenyl oder (C₂-C₄)Alkinyl;
   - R_{E}⁵: Halogen, Cyano, Nitro, Amino, Hydroxy, Carboxy, Phosphoryl, CHO, CONH₂, SO₂NH₂ oder Z^{c}-R^{c};
   - R^{a}: einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind;
   - R^{b}, R^{c}: gleich oder verschieden einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
   - Z^{a}: eine divalente Einheit aus der Gruppe O, S, CO, CS, C(O)O, C(O)S, SO, SO₂, NR^{d}, C(O)NR^{d} oder SO₂NR^{d};
   - Z^{b}, Z^{c}: gleich oder verschieden eine direkte Bindung oder eine divalente Einheit aus der Gruppe O, S, CO, CS, C(O)O, C(O)S, SO, SO₂, NR^{d}, SO₂NR^{d} oder C(O)NR^{d};
   - R^{d}: Wasserstoff, (C₁-C₄)-Alkyl oder (C₁-C₄)-Haloalkyl,
   - n_{E}: eine ganze Zahl von 0 bis 4, und
   - m_{E}: für den Fall, daß X für CH steht, eine ganze Zahl von 0 bis 5, und für den Fall, daß X für N steht, eine ganze Zahl von 0 bis 4
   bedeuten; davon bevorzugt sind Verbindungen (auch in Form ihrer Salze) vom Typ der Acylsulfamoylbenzoesäureamide, z.B. der nachfolgenden Formel (S-VI), die z.B. bekannt sind aus WO 99/16744, 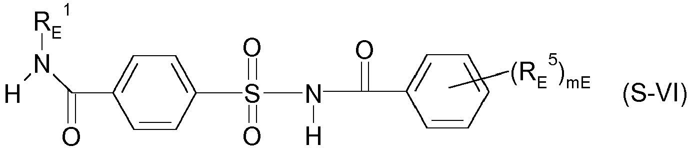
   z.B. solche worin
   R_{E}¹ = Cyclopropyl und R_{E}⁵ = 2-OMe ist ("Cyprosulfamide", S3-1),
   R_{E}¹ = Cyclopropyl und R_{E}⁵ = 5-Cl-2-OMe ist (S3-2),
   R_{E}¹ = Ethyl und R_{E}⁵ = 2-OMe ist (S3-3),
   R_{E}¹ = Isopropyl und R_{E}⁵ = 5-Cl-2-OMe ist (S3-4) und
   R_{E}¹ = Isopropyl und R_{E}⁵ = 2-OMe ist (S3-5);
F) Verbindungen vom Typ der N-Acylsulfamoylphenylharnstoffe der Formel (S-VII), die z.B. bekannt sind aus der EP-A-365484, 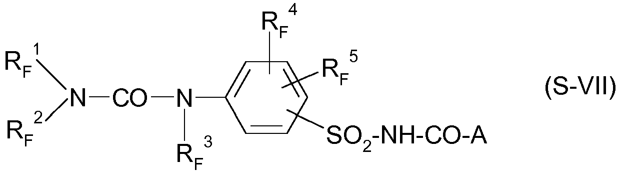 worin
   - A: für einen Rest aus der Gruppe 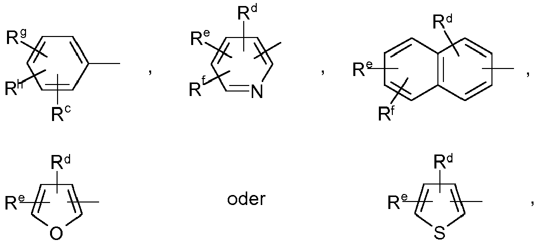
   R_{F}¹ und R_{F}² unabhängig voneinander für Wasserstoff, (C₁-C₈)Alkyl, (C₃-C₈)Cycloalkyl, (C₃-C₆)Alkenyl, (C₃-C₆)Alkinyl, 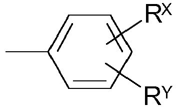 oder durch (C₁-C₄)Alkoxy oder 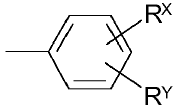 substituiertes (C₁-C₄)Alkoxy, oder
   - R_{F}¹ und R_{F}²: gemeinsam für eine (C₄-C₆)Alkylenbrücke oder eine durch Sauerstoff, Schwefel, SO, SO₂, NH oder -N(C₁-C₄-Alkyl)- unterbrochene (C₄-C₆)Alkylenbrücke,
   - R_{F}³: für Wasserstoff oder (C₁-C₄)Alkyl,
   - R_{F}⁴ und R_{F}⁵: unabhängig voneinander für Wasserstoff, Halogen, Cyan, Nitro, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl, -COOR^{j}, -CONR^{k}R^{m}, -CORⁿ, -SO₂NR^{k}R^{m} oder -OSO₂-C₁-C₄-Alkyl, oder R^{a} und R^{b} gemeinsam für eine (C₃-C₄)Alkylenbrücke, die durch Halogen oder C₁-C₄-Alkyl substituiert sein kann, oder eine (C₃-C₄)Alkenylenbrücke, die durch Halogen oder (C₁-C₄)Alkyl substituiert sein kann, oder eine C₄-Alkadienylenbrücke, die durch Halogen oder (C₁-C₄)Alkyl substituiert sein kann, und
   - R^{g} und R^{h}: unabhängig voneinander für Wasserstoff, Halogen, C₁-C₄-Alkyl, Trifluormethyl, Methoxy, Methylthio oder -COOR^{j} stehen, wobei
   - R^{c}: Wasserstoff, Halogen, (C₁-C₄)Alkyl oder Methoxy,
   - R^{d}: Wasserstoff, Halogen, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, -COOR^{j} oder -CONR^{k}R^{m},
   - R^{e}: Wasserstoff, Halogen, C₁-C₄-Alkyl, -COOR^{j}, Trifluormethyl oder Methoxy, oder R^{d} und R^{e} gemeinsam für eine (C₃-C₄)Alkylenbrücke,
   - R^{f}: Wasserstoff, Halogen oder (C₁-C₄)Alkyl,
   - R^{X} und R^{Y}: unabhängig voneinander Wasserstoff, Halogen, (C₁-C₄)Alkyl, (C₁-C₄)Alkoxy, (C₁-C₄)Alkylthio, -COOR⁴, Trifluormethyl, Nitro oder Cyan,
   - R^{j}, R^{k} und R^{m}: unabhängig voneinander Wasserstoff oder (C₁-C₄)Alkyl,
   - R^{k} und R^{m}: gemeinsam eine (C₄-C₆)Alkylenbrücke oder eine durch Sauerstoff, NH oder -N(C₁-C₄-Alkyl)- unterbrochene C₄-C₆-Alkylenbrücke, und
   - Rⁿ: (C₁-C₄)Alkyl, Phenyl oder durch Halogen, (C₁-C₄)Alkyl, Methoxy, Nitro oder Trifluormethyl substituiertes Phenyl bedeuten,
   davon bevorzugt sind:
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   1-[4-(N-Naphthoylsulfamoyl)phenyl]-3,3-dimethylharnstoff, einschließlich der
   Stereoisomeren und der in der Landwirtschaft gebräuchlichen Salze,
G) Wirkstoffe aus der Klasse der Hydroxyaromaten und der aromatischaliphatischen Carbonsäurederivate, z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 1,2-Dihydro-2-oxo-6-trifluoromethylpyridin-3-carboxamid, 2-Hydroxyzimtsäure, 2,4-Dichlorzimtsäure, wie sie in der WO 2004084631, WO 2005015994, WO 2006007981, WO 2005016001 beschrieben sind;
H) Wirkstoffe aus der Klasse der 1,2-Dihydrochinoxalin-2-one, z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO 2005112630 beschrieben sind,
I) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (siehe Pestic. Man.) (= Piperidin-1-thiocarbonsäure-S-1-methyl-1-phenylethylester), das als Safener für Reis gegen Schäden des Herbizids Molinate bekannt ist,
   "Daimuron" oder "SK 23" (siehe Pestic. Man.) (= 1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (= 3-(2-Chlorphenylmethyl)-1-(1-methyl-1-phenylethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist, "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
K) Verbindungen der Formel (S-IX), wie sie in der WO-A-1998/38856 beschrieben sind 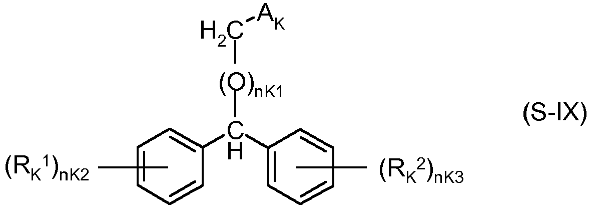 worin die Symbole und Indizes folgende Bedeutungen haben:
   - R_{K}¹, R_{K}²: unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{K}: COOR_{K}³ oder COOR_{K}⁴
   - R_{K}³, R_{K}⁴: unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{K}¹: 0 oder 1 und
   - n_{K}², n_{K}³: unabhängig voneinander 0, 1 oder 2;

### vorzugsweise:

Methyl-(diphenylmethoxy)acetat (CAS-Regno: 41858-19-9),
L) Verbindungen der Formel (S-X), wie sie in der WO A-98/27049 beschrieben sind 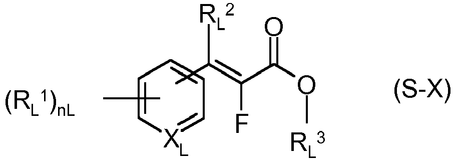 worin die Symbole und Indizes folgende Bedeutungen haben:
   - X_{L}: CH oder N,
   - n_{L}: für den Fall, dass X=N ist, eine ganze Zahl von 0 bis 4 und für den Fall, dass X=CH ist, eine ganze Zahl von 0 bis 5 ,
   - R_{L}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)Alkylsulfonyl, (C₁-C₄)Alkoxycarbonyl, ggf. substituiertes. Phenyl, ggf. substituiertes Phenoxy,
   - R_{L}²: Wasserstoff oder (C₁-C₄)Alkyl
   - R_{L}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze.
M) Wirkstoffe aus der Klasse der 3-(5-Tetrazolylcarbonyl)-2-chinolone, z.B. 1,2-Dihydro-4-hydroxy-1-ethyl-3-(5-tetrazolylcarbonyl)-2-chinolon (CAS-Regno: 219479-18-2), 1,2-Dihydro-4-hydroxy-1-methyl-3-(5-tetrazolylcarbonyl)-2-chinolon (CAS-Regno: 95855-00-8), wie sie in der WO-A-1999000020 beschrieben sind,
N) Verbindungen der Formeln (S-XI) oder (S-XII)
   wie sie in der WO-A-2007023719 und WO-A-2007023764 beschrieben sind 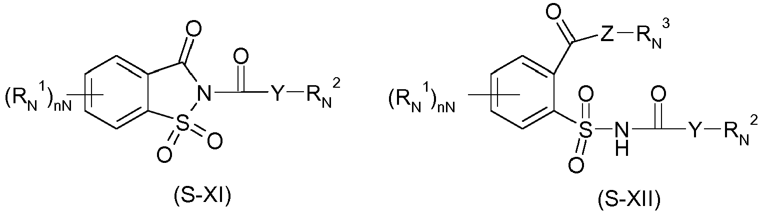 worin
   - R_{N}¹: Halogen, (C₁-C₄)Alkyl, Methoxy, Nitro, Cyano, CF₃, OCF₃
   - Y, Z: unabhängig voneinander O oder S,
   - n_{N}: eine ganze Zahl von 0 bis 4,
   - R_{N}²: (C₁-C₁₆)Alkyl, (C₂-C₆)Alkenyl, (C₃-C₆)Cycloalkyl, Aryl; Benzyl, Halobenzyl,
   - R_{N}³: Wasserstoff, (C₁-C₆)Alkyl bedeuten;
O) eine oder mehreren Verbindungen aus Gruppe: 1,8-Naphthalsäureanhydrid,
   O,O-Diethyl S-2-ethylthioethyl phosphordithioat (Disulfoton),
   4-Chlorphenyl-methylcarbamat (Mephenate),
   O,O-Diethyl-O-phenylphosphorotioat (Dietholate),
   4-Carboxy-3,4-dihydro-2H-1-benzopyran-4-essigsäure (CL-304415, CASRegno: 31541-57-8),
   2-propenyl 1-oxa-4-azaspiro[4.5]decane-4-carbodithioate (MG-838, CAS-Regno: 133993-74-5),
   Methyl-[(3-oxo-1H-2-benzothiopyran-4(3H)-yliden)methoxy]acetate (aus WO-A-98/13361; CAS-Regno: 205121-04-6),
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon-O-1,3-dioxolan-2-ylmethyloxim (Fluxofenim),
   4,6-Dichlor-2-phenylpyrimidin (Fenclorim),
   Benzyl-2-chlor-4-trifluormethyl-1,3-thiazol-5-carboxylat (Flurazole), 2-Dichlormethyl-2-methyl-1,3-dioxolan (MG-191),
   einschließlich der Stereoisomeren und der in der Landwirtschaft gebräuchlichen Salze.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I) und/oder deren Salze. Sie kann innerhalb weiter Grenzen schwanken. Für die Anwendung als Herbizid zur Bekämpfung von Schadpflanzen liegt sie beispielsweise im Bereich von 0,001 bis 10,0 kg/ha oder mehr Aktivsubstanz, vorzugsweise im Bereich von 0,005 bis 5 kg/ha, insbesondere im Bereich von 0,01 bis 1 kg/ha Aktivsubstanz. Dies gilt sowohl für die Anwendung im Vorauflauf oder im Nachauflauf.

Bei der Anwendung als Pflanzenwachstumsregulator, beispielsweise als Halmverkürzer bei Kulturpflanzen, wie sie oben genannt worden sind, vorzugsweise Getreidepflanzen wie Weizen, Gerste, Roggen, Triticale, Hirse, Reis oder Mais, liegt die Aufwandmenge beispielsweise im Bereich von 0,001 bis 2 kg/ha oder mehr Aktivsubstanz, vorzugsweise im Bereich von 0,005 bis 1 kg/ha, insbesondere im Bereich von 10 bis 500 g/ha Aktivsubstanz, ganz besonders von 20 bis 250 g/ha Aktivsubstanz. Dies gilt sowohl für die Anwendung im Vorauflauf oder im Nachauflauf, wobei die Nachauflaufbehandlung in der Regel bevorzugt ist.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Nachfolgend sind beispielhaft einige Synthesebeispiele von Verbindungen der allgemeinen Formel (I) beschrieben. In den Beispielen beziehen sich Mengenangaben (auch Prozentangaben) auf das Gewicht, sofern nichts anderes speziell angegeben ist. Wenn im Rahmen der Beschreibung und der Beispiele Bezeichnungen "R" und "S" für die absolute Konfiguration an einem Chiralitätszentrum der Stereoisomeren der Formel (I) angegeben ist, folgt diese der RS-Nomenklatur nach der Cahn-Ingold- Prelog-Regel, wenn nichts anderes näher definiert ist.

### (A) Synthesebeispiele

### Beispiel A1 [5-(4-Chlorphenyl)-3-methyl-1-(1,3-thiazol-5-yl)-1H-pyrazol-4-yl]essigsäure (siehe Tabelle 7, Beispiel 7-29)

### a) Herstellung von Methyl-[5-(4-chlorphenyl)-3-methyl-1H-pyrazol-4-yl]acetat

Zu einer Lösung von 10 g (37 mmol) Methyl-3-[(4-chlorphenyl)carbonyl]-4-oxopentanoat in Ethanol (100 ml) gab man 2,236 g (45 mmol) Hydrazinhydrat. Die Mischung wurde 6 Stunden unter Rückfluss erhitzt, dann auf Wasser gegeben und mit Dichlormethan extrahiert. Die vereinigten organischen Phasen wurden mit Magnesiumsulfat getrocknet, filtriert und eingeengt. Man erhielt 6,9 g Produkt (63 % d. Th.); NMR (CDCl₃, 400 MHz): 2.28 (s, 3H); 3.5 (s, 2H); 3.7 (s, 3H); 7.39 (d, 2H); 7.5 (d, 2H).

### b) Herstellung von Methyl-[5-(4-chlorphenyl)-3-methyl-1-(1,3-thiazol-5-yl)-1H-pyrazol-4-yl]acetat

Methyl-[5-(4-chlorphenyl)-3-methyl-1H-pyrazol-4-yl]acetat (1,0 g, 3,77 mmol), 5-Bromthiazol (0,526 g, 3,21 mmol), Salicylaldoxim (0,103 g, 0,75 mmol), Cu₂O (0,027 g, 0,18 mmol), Cs₂CO₃ (1,477 g, 4,53 mmol) und Acetonitril (4,8 ml) wurden in einem ausgeheitzen und über Argonstrom abgekühlten Gefäß nacheinander eingefüllt. Das geschlossene Gefäß wurde im Mikrowellenofen für 6 Stunden auf 85°C erhitzt. Danach wurde das Reaktionsgemisch auf Eiswasser gegossen, mit Dichloromethan extrahiert, die organische Phase getrocknet und eingeengt. Nach Chromatographie des Rückstands erhielt man 0,096 g Produkt (6,5 % d. Th.); NMR (CDCl₃, 400 MHz): 2.31 (s, 3H); 3.35 (s, 2H); 3.70 (s, 3H); 7.22 (d, 2H); 7.29 (br s, 1 H); 7.42 (d, 2H); 8.53 (br s, 1 H).

### c) Herstellung von [5-(4-Chlorphenyl)-3-methyl-1-(1,3-thiazol-5-yl)-1H-pyrazol-4-yl]essigsäure

Zu 0,289 g (0,83 mmol) Methyl-[5-(4-chlorphenyl)-3-methyl-1-(1,3-thiazol-5-yl)-1H-pyrazol-4-yl]acetat in 10 ml Methanol gab man 1 ml 2 molare wässrige Natronlauge, rührte vier Stunden bei Raumtemperatur und ließ über Nacht stehen. Der Ansatz wurde dann eingeengt. Zum Rückstand gab man Eiswasser, stellte das Gemisch durch Zugabe von 2 molarer wässriger Salzsäure auf pH 3-4 und extrahierte mit Dichloromethan. Die vereinigten organischen Phasen wurden mit Magnesiumsulfat getrocknet, filtriert und eingeengt. Das Rohprodukt wurde am Hochvakuum getrocknet. Man erhielt 0,187 g Produkt (60,7 % d. Th.); NMR (CDCl₃, 400 MHz): 2.33 (s, 3H); 3.39 (s, 2H); 7.24 (d, 2H); 7.31 (br s, 1 H); 7.42 (d, 2H); 8.56 (br s, 1 H).

### Beispiel A2 Cyclopropylmethyl-[5-(4-chlorphenyl)-3-methyl-1-(1,3-thiazol-5-yl)-1 H-pyrazol-4-yl]acetat (siehe Tabelle 9, Beispiel 9-122)

In einen Mikrowellenreaktor wurden nacheinander 0,40 g (0,41 mmol) [5-(4-Chlorphenyl)-3-methyl-1-(1,3-thiazol-5-yl)-1H-pyrazol-4-yl]essigsäure, 3 ml Cyclopropylcarbinol und 2 Tropfen konzentrierte Schwelsäure gegeben.
Das geschlossene Gefäß wurde im Mikrowellenofen für 4 Stunden auf 100°C erhitzt. Danach wurde der Ansatz am Hochvakuum eingeengt. Man gab Eiswasser und Dichloromethan zu und stellte mit ges. NaHCO₃-Lsg auf pH 8. Die vereinigten organischen Phasen wurden mit Magnesiumsulfat getrocknet, filtriert und eingeengt. Nach Chromatographie des Rückstands erhielt man 0,047 g Produkt (26 % d. Th.). NMR (CDCl₃, 400 MHz): 0.28 (m, 2H); 0.58 (m, 2H); 1.10 (m, 1 H); 2.33 (s, 3H); 3.37 (s, 2H); 3.92 (d, 2H); 7.27 (d, 2H); 7.29 (br s, 1 H); 7.42 (d, 2H); 8.52 (br s, 1 H).

### Beispiel A3 Methyl-4-oxo-3-(pyridin-2-ylcarbonyl)pentanoat (siehe Tabelle 10, Beispiel 10-68)

### a) Herstellung von 1-(Pyridin-2-yl)butan-1,3-dion

Zu einer Mischung von 10 g (73 mmol) Picolinsäuremethylester und 25 ml (124 mmol) Aceton in 150 ml Tetrahydrofuran wurde eine Natriummethylat Lösung (28% in Methanol) zugetropft. Man rührte 3 Stunden bei 20°C und entfernte das Lösemittel im Vakuum. Der Rückstand wurde in Wasser aufgenommen, mit 2 molarer wässriger Salzsäure angesäuert und mit Dichlormethan extrahiert. Nach Trocknen der vereinigten organischen Phasen und Entfernen des Lösemittels im Vakuum erhielt man 7,940 g Produkt (60% d. Th.); NMR (CDCl₃, 400 MHz): 2.23 (s, 3H); 6.81 (s, 1H); 7.41 (m, 1 H); 7.83 (m, 1H); 8.09 (m, 1H); 8.68 (m, 1 H); 15.7 (br, 1 H).

### b) Methyl-4-oxo-3-(pyridin-2-ylcarbonyl)pentanoat

Zu 0,911 g (22,78 mmol) Natriumhydrid in 25 ml Dimethylsulfoxid tropfte man eine Lösung von 3,38 g (20,7 mmol) 1-(Pyridin-2-yl)butan-1,3-dion gelöst in Dimethylsulfoxid so langsam zu, dass die Temperatur nicht höher als 30°C anstieg. Es wurde 30 Minuten bei 20°C weitergerührt. Dann wurden 3,486 g (22,78 mmol) Bromessigsäuremethylester in etwas Dimethylsulfoxid bei 0°C langsam zugetropft. Man rührte weitere 4 Stunden bei 20°C. Die Reaktionsmischung wurde auf Eiswasser gegossen, und mit Dichlormethan extrahiert. Die organische Phase wurde mehrmals mit Wasser gewaschen. Nach Trocknen der vereinigten organischen Phasen, Entfernen des Lösemittels im Vakuum und Chromatographie des Rückstands erhielt man 3,48 g Produkt (71,4% d. Th.); NMR (CDCl₃, 400 MHz): 2.4 (s, 3H); 2.9 (d, 1 H); 3.05 (d, 1 H); 3.69 (s, 3H); 5.49 (dd, 1 H); 7.50 (m, 1 H); 7.86 (m, 1 H); 8.07 (m, 1H); 8.7 (m, 1 H).

### Beispiel A4 Ethyl-5-[4-(2-methoxy-2-oxoethyl)-3-methyl-5-(pyridin-2-yl)-1H-pyrazol-1-yl]-1,3-thiazol-4-carboxylat (s. Tab. 8, Beispiel 8-241)

a) Herstellung von Methyl-4-oxo-3-(pyridin-2-ylcarbonyl)pentanoat Die Herstellung verläuft gemäß Beispiel A3.
b) Herstellung von Ethyl-5-hydrazinyl-1,3-thiazol-4-carboxylat Ethyl-5-hydrazinyl-1,3-thiazol-4-carboxylat wurde nach dem Fachmann bekannten Methoden via Diazotierung von Ethyl-5-amino-1,3-thiazol-4-carboxylat und anschliessende Reduktion hergestellt.
c) Herstellung von Ethyl-5-[4-(2-methoxy-2-oxoethyl)-3-methyl-5-(pyridin-2-yl)-1H-pyrazol-1-yl]-1,3-thiazol-4-carboxylat

In einem Mikrowellenreaktor wurden 0.450 g (1.91 mmol) Methyl-4-oxo-3-(pyridin-2-ylcarbonyl)pentanoat in 4 ml Methanol mit 0.358 g (1.91 mmol) Ethyl-5-hydrazinyl-1,3-thiazol-4-carboxylat versetzt. Das geschlossene Gefäß wurde im Mikrowellenofen für 2 Stunden auf 90°C erhitzt. Nach Entfernen des Lösemittels extrahierte man mehrfach mit Dichlormethan / Wasser. Die vereinigten organischen Phasen wurden über Natriumsulfat getrocknet und das Lösemittel im Vakuum entfernt. Nach Chromatographie des Rückstands erhielt man 0,508 g Produkt (61,8% d. Th.), NMR (CDCl₃, 300 MHz): 1.12 (t, 3H); 2.35 (s, 3H); 3.66 (s, 2H); 3.69 (s, 3H); 4.08 (s, 3H); 7.19 (m, 1 H); 7.32 (m, 1 H); 7.66 (m, 1 H); 8.51 (m, 1 H); 8.72 (s, 1 H).

### Beispiel A5 Methyl-3-[(4-chlorphenyl)carbonyl]-4-oxopentanoat (siehe Tabelle 10, Beispiel 10-13)

Zu 2,237 g (56 mmol) Natriumhydrid in 200 ml Dimethylsulfoxid tropfte man eine Lösung von 10 g (51 mmol) 1-(4-Chlorphenyl)butan-1,3-dion (kommerziell erhältlich) gelöst in Dimethylsulfoxid so langsam zu, dass die Temperatur nicht höher als 30°C anstieg. Es wurde 30 Minuten bei 20°C weitergerührt. Dann wurden 8,558 g (56 mmol) Bromessigsäuremethylester in etwas Dimethylsulfoxid bei 0°C langsam zugetropft. Man rührte 4 Stunden bei 20°C. Die Reaktionsmischung wurde auf Eiswasser gegossen, und mit Dichlormethan extrahiert. Die organische Phase wurde mehrmals mit Wasser gewaschen. Nach Trocknen der vereinigten organischen Phasen, Entfernen des Lösemittels im Vakuum und Chromatographie des Rückstands erhielt man 7,750 g Produkt (56,7% d. Th.); NMR (CDCl₃, 400 MHz): 2.19 (s, 3H); 2.99 (d, 1 H); 3.03 (d, 1 H); 3.69 (s, 3H); 4.95 (dd, 1 H); 7.49 (d, 2H); 7.98 (d, 2H).

### Beispiel A6 Methyl-[5-(4-chlorphenyl)-3-methyl-1-(3-methylisothiazol-5-yl)-1 H-pyrazol-4-yl]acetat (siehe Tabelle 2, Beispiel 2-147)

a) Herstellung von Methyl-3-[(4-chlorphenyl)carbonyl]-4-oxopentanoat Die Herstellung verläuft gemäß Beispiel A5.
b) Herstellung von 5-Hydrazinyl-3-methylisothiazol 5-Hydrazinyl-3-methylisothiazol wurde nach dem Fachmann bekannten Methoden via Diazotierung von 5-Amino-3-methylisothiazol und anschliessende Reduktion hergestellt.
c) Herstellung von Methyl-[5-(4-chlorphenyl)-3-methyl-1-(3-methylisothiazol-5-yl)-1 H-pyrazol-4-yl]acetat
   Zu 0.500 g (2.0 mmol) Methyl-3-[(4-chlorphenyl)carbonyl]-4-oxopentanoat in 10.00 ml Methanol gab man 0,301 g (2.0 mmol) 5-Hydrazinyl-3-methylisothiazol und 0.099 ml (2.0 mmol) konzentrierte Schwefelsäure und rührte 6 Stunden unter Rückfluss. Der Ansatz wurde auf Wasser gegossen und mit Ethylacetat extrahiert.
   Nach Trocknen der vereinigten organischen Phasen, Entfernen des Lösemittels im Vakuum und HPLC Trennung des Rückstands erhielt man 0.106 g Produkt (14.9% d. Th.); NMR (CDCl3, 400 MHz): 2.29 (s, 3H); 2.30 (s, 3H); 3.29 (s, 2H); 3.68 (s, 3H); 6.30 (s, 1 H); 7.31 (d, 2H); 7.49 (d, 2H).

Die in den nachfolgenden Tabellen 1 bis 10 beschriebenen Verbindungen erhält man gemäß den oder analog zu den oben beschriebenen Beispielen.
In der Tabelle 10 sind Zwischenprodukte der Formel (III) aufgeführt, die gemäß den oben beschriebenen Verfahren eingesetzt werden können.

In den Tabellen 1 bis 10 bedeuten:
- F, Cl, Br, J: = Fluor, Chlor, Brom bzw. Jod entsprechend den üblichen chemischen Atomsymbolen
- Me: = Methyl
- MeO oder OMe: = Methoxy
- 3,5-Me₂: = 3,5-Dimethyl (z. B. als Substitution am Phenylring)
- 4,5-Cl₂: = 4,5-Dichloro (z. B. als Substitution am Phenylring)
- Et: = Ethyl
- Pr: = nPr = n-Propyl
- iPr: = Isopropyl
- iOPr: = O-iPr = iPrO = Isopropyloxy
- cyPr: = Cyclopropyl
- Bu: = nBu = n-Butyl = But-1-yl
- iBu: = Isobutyl = 2-Methyl-prop-1-yl
- sBu: = sec-Bu
- tBu: = t-Butyl = tertiär-Butyl = 2-Methyl-prop-2-yl
- Ph: = Phenyl
- PhO: = Phenoxy
- Ac: = COCH₃ = Acetyl
- Allyl: = Prop-2-en-1-yl
- COOH: = Carboxy
- COOEt: = Ethoxycarbonyl
- COOMe: = Methoxycarbonyl
- 3,5-(COOMe)₂: = 3,5-Dimethoxycarbonyl
- OSO₂Me: = -O-S(=O)₂-CH₃, Methylsulfonyloxy, Methansulfonat
- "(R⁶)ₙ = H": = unsubstituierter Cyclus (n = 0)

Im Übrigen gelten die üblichen chemischen Symbole und Formeln, wie z. B. CH₂ für Methylen oder CF₃ für Trifluormethyl oder OH für Hydroxyl. Zusammengesetzte Bedeutungen sind entsprechend aus den genannten Abkürzungen zusammengesetzt definiert.

Physikalische Daten ("Daten") der Verbindungen zu den Tabellen sind gegebenenfalls in den ausführlichen Herstellungsbeispielen (siehe oben) oder am Ende der Tabellen angegeben. Dabei bedeuten:
- "NMR": = Daten gemäß ¹H-NMR-Spektum (¹H-Kernresonanz-Daten)
- "Schmp.": = Schmelzpunkt

**Tabelle 1: Verbindungen der Formel (la")**

| | | | | | |
|---|---|---|---|---|---|
| 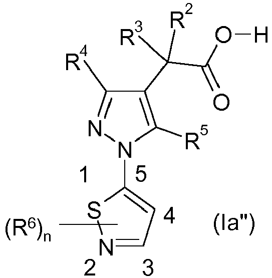 | | | | | |

| Nr. | R² | R³ | R⁴ | R⁵ | (R⁶)ₙ |
|---|---|---|---|---|---|
| 1-1 | H | H | Ph | Ph | H |
| 1-2 | H | H | Me | Ph | H |
| 1-3 | H | H | Me | 5-J-2-thienyl | H |
| 1-4 | H | H | Me | 2-Furyl | H |
| 1-5 | H | H | Me | Ph | 3-OMe |
| 1-6 | Me | H | Me | Ph | 4-Me |
| 1-7 | H | H | Me | Ph | 3-CI |
| 1-8 | H | H | Me | Ph | 4-CF₃ |
| 1-9 | H | H | Me | Ph | 3-CF₃ |
| 1-10 | H | H | Me | Ph | 4-Me |
| 1-11 | H | H | Me | Ph | 3,4-Me₂ |
| 1-12 | H | H | Me | Ph | 3,4-Cl₂ |
| 1-13 | H | H | Me | 4-MeO-Ph | 4-Me |
| 1-14 | H | H | Me | 4-MeO-Ph | H |
| 1-15 | Me | H | Me | Ph | H |
| 1-16 | H | H | Me | 4-Me-Ph | 4-Me |
| 1-17 | H | H | Me | 4-Me-Ph | 4-CI |
| 1-18 | H | H | Me | 4-Me-Ph | H |
| 1-19 | H | H | Me | 3-CI-Ph | H |
| 1-20 | H | H | Me | 3-CF₃-Ph | H |
| 1-21 | H | H | Me | 3-CF₃-Ph | 4-Me |
| 1-22 | H | H | Me | 3,4-Cl₂-Ph | 4-Me |
| 1-23 | H | H | Me | 3-CI-Ph | 4-Me |
| 1-24 | H | H | Me | 2-CI-Ph | 4-Me |
| 1-25 | H | H | Me | 2,4-Cl₂-Ph | 4-Me |
| 1-26 | H | H | Me | 4-CF₃-Ph | 4-Me |
| 1-27 | H | H | Me | 4-CI-Ph | 4-Me |
| 1-28 | H | H | Me | 4-CI-Ph | H |
| 1-29 | H | H | Me | 3,4-Cl₂-Ph | H |
| 1-30 | H | H | Me | 4-CF₃-Ph | H |
| 1-31 | H | H | Me | 4-CI-Ph | 4-CI |
| 1-32 | H | H | Me | Ph | 4-CI |
| 1-33 | H | H | Me | 2-CI-Ph | H |
| 1-34 | H | H | Me | 4-tBu-Ph | 4-Me |
| 1-35 | H | H | Me | 3,5-Me₂-Ph | 4-Me |
| 1-36 | H | H | Me | Ph | 4-OMe |
| 1-37 | H | H | Me | 4-CI-Ph | 4-OMe |
| 1-38 | H | H | Me | 4-Me-Ph | 4-Me |
| 1-39 | H | H | Me | 4-F-Ph | 4-CI |
| 1-40 | H | H | Me | 4-F-Ph | 4-Me |
| 1-41 | H | H | Me | 3-Me-Ph | 4-Me |
| 1-42 | H | H | Me | 4-(COOH)-Ph | 4-Me |
| 1-43 | H | H | Me | 3-Br-Ph | 4-Me |
| 1-44 | H | H | Me | 4-Ph-Ph | 4-Me |
| 1-45 | H | H | Me | 4-(COOH)-Ph | H |
| 1-46 | H | H | Me | 3,5-Me₂-Ph | H |
| 1-47 | H | H | Me | Ph | 4-SMe |
| 1-48 | H | H | Me | 4-CI-Ph | 4-SMe |
| 1-49 | H | H | Me | 3-Cl-4-Me-Ph | H |
| 1-50 | H | H | Me | 3-CF₃-4-Cl-Ph | H |
| 1-51 | H | H | Me | 3-CF₃-4-Cl-Ph | 4-Me |
| 1-52 | H | H | Me | 3-Cl-4-Me-Ph | 4-Me |
| 1-53 | H | H | Me | 2-Pyridyl | 4-CI |
| 1-54 | H | H | Me | 4-CI-Ph | 4-F |
| 1-55 | H | H | Me | 2-Thienyl | 4-Me |
| 1-56 | H | H | Me | 3-Me-2-thienyl | 4-Me |
| 1-57 | H | H | Me | 4-Me-2-thienyl | 4-Me |
| 1-58 | H | H | Me | 5-Cl-2-thienyl | 4-Me |
| 1-59 | H | H | Me | 5-Cl-2-thienyl | 4-CI |
| 1-60 | H | H | Me | 3-Thienyl | 4-Me |
| 1-61 | H | H | Me | 2-Thienyl | H |
| 1-62 | H | H | Me | 3-Me-2-thienyl | H |
| 1-63 | H | H | Me | 4-Me-2-thienyl | H |
| 1-64 | H | H | Me | 5-Cl-2-thienyl | H |
| 1-65 | H | H | Me | 5-Me-2-thienyl | H |
| 1-66 | H | H | Me | 6-MeO-pyridin-3-yl | H |
| 1-67 | H | H | Me | 5-Br-2-thienyl | H |
| 1-68 | H | H | Me | 5-Br-2-thienyl | 4-Me |
| 1-69 | H | H | Me | 3-Thienyl | H |
| 1-70 | H | H | Me | 4-Cl-Ph | 4-S(O)Me |
| 1-71 | H | H | Me | 4-Br-Ph | 4-Me |
| 1-72 | H | H | Me | 1,3-Benzodioxol-5-yl | 4-Me |
| 1-73 | H | H | Me | 4-J-Ph | 4-Me |
| 1-74 | H | H | Me | 3,5-Cl₂-Ph | 4-Me |
| 1-75 | H | H | Me | 4-PhO-Ph | 4-Me |
| 1-76 | H | H | Me | 6-OH-pyridin-3-yl | H |
| 1-77 | H | H | Me | Ph | 4-S(O)Me |
| 1-78 | H | H | H | Ph | H |
| 1-79 | H | H | H | Ph | 4-Me |
| 1-80 | H | H | Et | Ph | H |
| 1-81 | H | H | n-Pr | Ph | H |
| 1-82 | H | H | CH₂Cl | Ph | H |
| 1-83 | H | H | CHCl₂ | Ph | H |
| 1-84 | H | H | CH₂F | Ph | H |
| 1-85 | H | H | CHF₂ | Ph | H |
| 1-86 | H | H | Cl | Ph | H |
| 1-87 | H | H | Et | Ph | 4-Me |
| 1-88 | H | H | n-Pr | Ph | 4-Me |
| 1-89 | H | H | CH₂Cl | Ph | 4-Me |
| 1-90 | H | H | CHCl₂ | Ph | 4-Me |
| 1-91 | H | H | CH₂F | Ph | 4-Me |
| 1-92 | H | H | CHF₂ | Ph | 4-Me |
| 1-93 | H | H | Cl | Ph | 4-Me |
| 1-94 | H | H | Et | 4-CI-Ph | H |
| 1-95 | H | H | n-Pr | 4-CI-Ph | H |
| 1-96 | H | H | CH₂Cl | 4-CI-Ph | H |
| 1-97 | H | H | CHCl₂ | 4-CI-Ph | H |
| 1-98 | H | H | CH₂F | 4-CI-Ph | H |
| 1-99 | H | H | CHF₂ | 4-CI-Ph | H |
| 1-100 | H | H | Cl | 4-CI-Ph | H |
| 1-101 | H | H | Et | 4-Me-Ph | H |
| 1-102 | H | H | n-Pr | 4-Me-Ph | H |
| 1-103 | H | H | CH₂Cl | 4-Me-Ph | H |
| 1-104 | H | H | CHCl₂ | 4-Me-Ph | H |
| 1-105 | H | H | CH₂F | 4-Me-Ph | H |
| 1-106 | H | H | CHF₂ | 4-Me-Ph | H |
| 1-107 | H | H | Cl | 4-Me-Ph | H |
| 1-108 | H | H | Et | 2-Pyridyl | H |
| 1-109 | H | H | n-Pr | 2-Pyridyl | H |
| 1-110 | H | H | CH₂Cl | 2-Pyridyl | H |
| 1-111 | H | H | CHCl₂ | 2-Pyridyl | H |
| 1-112 | H | H | CH₂F | 2-Pyridyl | H |
| 1-113 | H | H | CHF₂ | 2-Pyridyl | H |
| 1-114 | H | H | Cl | 2-Pyridyl | H |
| 1-115 | H | H | Me | 2-Pyridyl | H |
| 1-116 | H | H | Me | 5-Cl-pyridin-2-yl | H |
| 1-117 | H | H | Me | 5-Cl-pyridin-2-yl | 4-CI |
| 1-118 | H | H | Me | 5-Cl-pyridin-2-yl | 4-Me |
| 1-119 | H | H | Me | 5-Br-pyridin-2-yl | H |
| 1-120 | H | H | Me | 5-Br-pyridin-2-yl | 4-CI |
| 1-121 | H | H | Me | 5-Br-pyridin-2-yl | 4-Me |
| 1-122 | H | H | Me | 5-F-pyridin-2-yl | H |
| 1-123 | H | H | Me | 5-Me-pyridin-2-yl | H |
| 1-124 | H | H | Me | 5-Me-pyridin-2-yl | 4-Me |
| 1-125 | H | H | Me | 2,4-Cl₂-Ph | H |
| 1-126 | H | H | Me | 4-CH₂COOH-Ph | 4-Me |
| 1-127 | H | H | Me | 3,4-Me₂-Ph | 4-Me |
| 1-128 | H | H | Me | 4-Br-Ph | H |
| 1-129 | H | H | Me | 3,4-Me₂-Ph | H |
| 1-130 | H | H | Me | 3-Me-Ph | H |
| 1-131 | H | H | Me | 4-F-Ph | H |
| 1-132 | H | H | Me | 4-(Me-CO)-Ph | H |
| 1-133 | H | H | Me | 4-tBu-Ph | H |
| 1-134 | H | H | Me | 4-Cl-3-Me-Ph | H |
| 1-135 | H | H | n-Pr | 4-CI-Ph | 4-Me |
| 1-136 | H | H | Me | 3-Pyridyl | H |
| 1-137 | H | H | Me | 4-Pyridyl | H |
| 1-138 | H | H | C(O)OMe | Ph | H |
| 1-139 | H | H | Me | 6-Me-pyridin-3-yl | H |
| 1-140 | H | H | Me | 4-CI-Ph | 4-SO₂Me |
| 1-141 | H | H | Me | 3-Pyridyl | 4-Me |
| 1-142 | H | H | Me | 2,3-Cl₂-Ph | 4-Me |
| 1-143 | H | H | Me | 2-Pyridyl | 4-Me |
| 1-144 | H | H | H | 4-CI-Ph | 4-Me |
| 1-145 | H | H | Me | 6-Cl-pyridin-3-yl | H |
| 1-146 | H | H | Me | 4-CI-Ph | 3-Me |
| 1-147 | H | H | Me | Ph | 3-Me |
| 1-148 | H | H | Me | 4-Me-pyridin-2-yl | H |
| 1-149 | H | H | Me | 4-Me-pyridin-2-yl | 4-Me |
| 1-150 | H | H | Me | 4-Me-pyridin-2-yl | 4-CI |
| 1-151 | H | H | Me | 4-Me-pyridin-2-yl | 4-F |
| 1-152 | H | H | Me | 4-F-pyridin-2-yl | H |
| 1-153 | H | H | Me | 4-Cl-pyridin-2-yl | H |
| 1-154 | H | H | Me | 4-Br-pyridin-2-yl | H |
| 1-155 | H | H | Me | 4-OMe-pyridin-2-yl | H |
| 1-156 | H | H | Me | 5-CF₃-pyridin-2-yl | H |
| 1-157 | H | H | Me | 6-OMe-pyridin-2-yl | H |
| 1-158 | H | H | cyPr | 4-Cl-Ph | H |
| 1-159 | H | H | CN | 4-Cl-Ph | H |
| 1-160 | H | H | CN | 4-CI-Ph | 4-Me |
| 1-161 | H | H | CN | 4-Me-Ph | H |
| 1-162 | H | H | CN | 4-Me-Ph | 4-Me |
| 1-163 | H | H | CN | Ph | H |
| 1-164 | H | H | CN | Ph | 4-Me |
| 1-165 | H | H | CN | 2-pyridyl | H |
| 1-166 | H | H | CN | 3-pyridyl | H |
| 1-167 | H | H | CN | 5-Cl-pyridin-2-yl | H |
| 1-168 | H | H | CN | 5-Br-pyridin-2-yl | H |
| 1-169 | H | H | CN | 5-F-pyridin-2-yl | H |
| 1-170 | H | H | CN | 5-Me-pyridin-2-yl | H |
| 1-171 | H | H | CN | 6-Me-pyridin-3-yl | H |
| 1-172 | H | H | CN | 4-Me-pyridin-2-yl | H |
| 1-173 | H | H | CN | 4-F-pyridin-2-yl | H |
| 1-174 | H | H | CN | 4-Cl-pyridin-2-yl | H |
| 1-175 | H | H | CN | 4-Br-pyridin-2-yl | H |
| 1-176 | H | H | CN | 4-OMe-pyridin-2-yl | H |
| 1-177 | H | H | formyl | 4-CI-Ph | H |
| 1-178 | H | H | formyl | 4-CI-Ph | 4-Me |
| 1-179 | H | H | formyl | 4-Me-Ph | H |
| 1-180 | H | H | formyl | 4-Me-Ph | 4-Me |
| 1-181 | H | H | formyl | Ph | H |
| 1-182 | H | H | formyl | Ph | 4-Me |
| 1-183 | H | H | formyl | 2-pyridyl | H |
| 1-184 | H | H | formyl | 3-pyridyl | H |
| 1-185 | H | H | formyl | 5-Cl-pyridin-2-yl | H |
| 1-186 | H | H | formyl | 5-Br-pyridin-2-yl | H |
| 1-187 | H | H | formyl | 5-F-pyridin-2-yl | H |
| 1-188 | H | H | formyl | 5-Me-pyridin-2-yl | H |
| 1-189 | H | H | formyl | 6-Me-pyridin-3-yl | H |
| 1-190 | H | H | formyl | 4-Me-pyridin-2-yl | H |
| 1-191 | H | H | formyl | 4-F-pyridin-2-yl | H |
| 1-192 | H | H | formyl | 4-Cl-pyridin-2-yl | H |
| 1-193 | H | H | formyl | 4-Br-pyridin-2-yl | H |
| 1-194 | H | H | formyl | 4-OMe-pyridin-2-yl | H |
| 1-195 | H | H | CH₂OH | 5-Me-pyridin-2-yl | H |
| 1-196 | H | H | CH₂OH | 4-CI-Ph | H |
| 1-197 | H | H | CH₂OH | 4-Me-pyridin-2-yl | H |
| 1-198 | H | H | CH₂OH | 4-Me-Ph | H |
| 1-199 | H | H | CH₂OH | Ph | H |
| 1-200 | H | H | CH₂OH | 2-Pyridyl | H |
| 1-201 | H | H | Me | 2-Thiazolyl | H |
| 1-202 | H | H | Me | 2-Thiazolyl | 4-CI |
| 1-203 | H | H | Me | 2-Thiazolyl | 4-Me |
| 1-204 | H | H | Me | 4-Me-thiazol-2-yl | H |
| 1-205 | H | H | Me | 4-Me-thiazol-2-yl | 4-CI |
| 1-206 | H | H | Me | 4-Me-thiazol-2-yl | 4-Me |
| 1-207 | H | H | Me | 5-Me-thiazol-2-yl | H |
| 1-208 | H | H | Me | 5-Br-thiazol-2-yl | H |
| 1-209 | H | H | Me | 5-Br-thiazol-2-yl | 4-Me |
| 1-210 | H | H | Me | 5-Cl-thiazol-2-yl | H |
| 1-211 | H | H | Me | 4,6-Me₂-pyridin-2-yl | H |
| 1-212 | H | H | Me | 4,6-Me₂-pyridin-2-yl | 4-Me |
| 1-213 | H | H | Me | 2-Pyridyl | 4-F |
| 1-214 | H | H | Me | 2-Pyrazinyl | H |
| 1-215 | H | H | Me | 5-Me-Pyrazin-2-yl | H |
| 1-216 | H | H | Me | 2-Pyrazinyl | 4-Me |
| 1-217 | H | H | Me | 1,3-Benzothiazol-2-yl | H |
| 1-218 | H | H | Me | 1,3-Benzothiazol-2-yl | 4-Me |
| 1-219 | H | H | Me | 7-Cl-1,3-benzothiazol-2-yl | H |
| 1-220 | H | H | Me | 1,5-Me₂-pyrazol-3-yl | H |
| 1-221 | H | H | Me | 1,5-Me₂-pyrazol-3-yl) | 4-Me |
| 1-222 | H | H | Me | 4,5-Me₂-thiazol-2-yl | H |
| 1-223 | H | H | Me | 4,5-Cl₂-thiazol-2-yl | H |
| 1-224 | H | H | Me | 2-Pyrimidinyl | H |
| 1-225 | H | H | Me | 2-Pyrimidinyl | 4-Me |
| 1-226 | H | H | Me | 5-F-pyrimidin-2-yl | H |
| 1-227 | H | H | Me | 5-Cl-pyrimidin-2-yl | H |
| 1-228 | H | H | Me | 5-Br-pyrimidin-2-yl | H |
| 1-229 | H | H | Me | 5-Me-pyrimidin-2-yl | H |
| 1-230 | H | H | Me | 5-Me-pyrimidin-2-yl | 4-Me |
| 1-231 | H | H | Me | 4,6-Me₂-pyrimidin-2-yl | H |
| 1-232 | H | H | Me | 4,6-Me₂-pyrimidin-2-yl | 4-Me |
| 1-233 | H | H | Me | 3-Pyridazinyl | H |
| 1-234 | H | H | Me | 6-Me-Pyridazin-3-yl | H |
| 1-235 | H | H | Me | 1,2,4-Triazin-3-yl | H |
| 1-236 | H | H | Me | 6-Me-1,2,4-Triazin-3-yl | H |
| 1-237 | H | H | Me | Chinolin-2-yl | H |
| 1-238 | H | H | Me | Isochinolin-3-yl | H |
| 1-239 | H | H | Me | 3,5-Cl₂-Ph | H |
| 1-240 | H | H | Me | 2-Me-pyridin-4-yl | H |
| 1-241 | H | H | Me | 4-Cl-6-Me-pyridin-2-yl | H |
| 1-242 | H | H | Me | 4-Br-3-Me-Ph | H |
| 1-243 | H | H | Me | 5-Cl-pyridin-3-yl | H |
| 1-244 | H | H | Me | 5-Allyl-pyridin-2-yl | H |
| 1-245 | H | H | Me | 5-Cyclopropyl-pyridin-2-yl | H |
| 1-246 | H | H | Me | 5-Ethinyl-pyridin-2-yl | H |
| 1-247 | H | H | Me | 5-Ph-pyridin-2-yl | H |
| 1-248 | H | H | Me | 6-Br-pyridin-3-yl | H |
| 1-249 | H | H | Me | 4-Cl-3-thienyl | H |
| 1-250 | H | H | Me | 4-Br-3-thienyl | H |
| 1-251 | H | H | Me | 4-Me-3-thienyl | H |
| 1-252 | H | H | Me | 4-Thiazolyl | H |
| 1-253 | H | H | Me | 5-Thiazolyl | H |
| 1-254 | H | H | Me | 2-Me-thiazol-4-yl | H |
| 1-255 | H | H | Me | 2-Me-thiazol-5-yl | H |
| 1-256 | H | H | Me | 5-Cl-3-thienyl | H |
| 1-257 | H | H | Me | 5-Br-3-thienyl | H |
| 1-258 | H | H | Me | 5-Me-3-thienyl | H |
| 1-259 | H | H | Me | 2-F-Ph | H |
| 1-260 | H | H | Me | 2-CN-Ph | H |
| 1-261 | H | H | Me | 2-NO₂-Ph | H |
| 1-262 | H | H | Me | 2,4-F₂-Ph | H |
| 1-263 | H | H | Me | 3,4-F₂-Ph | H |
| 1-264 | H | H | Me | 1-Me-pyrazol-3-yl | H |
| 1-265 | H | H | Me | 1-Me-pyrazol-5-yl | H |
| 1-266 | H | H | Me | 3-Br-Ph | H |
| 1-267 | H | H | Me | 4-Ph-Ph | H |
| 1-268 | H | H | Me | 1,3-Benzodioxol-5-yl | H |
| 1-269 | H | H | Me | 4-J-Ph | H |
| 1-270 | H | H | Me | 4-PhO-Ph | H |
| 1-271 | H | H | Me | 4-CH₂COOH-Ph | H |
| 1-272 | H | H | Me | 2,3-Cl₂-Ph | H |
| 1-273 | H | H | Me | 5-I-pyridin-2-yl | H |
| 1-274 | H | H | Me | 5-I-pyrimidin-2-yl | H |
| 1-275 | H | H | Me | 2-Cl-thiazol-4-yl | H |
| 1-276 | H | H | Me | 2-Br-thiazol-4-yl | H |
| 1-277 | H | H | Me | 5-OSO₂Me-pyridin-2-yl | H |
| 1-278 | H | H | Me | 6-Cl-1,3-benzothiazol-2-yl | H |
| 1-279 | H | H | Me | 6-Br-1,3-benzothiazol-2-yl | H |
| 1-280 | H | H | Me | 1,3-Benzoxazol-2-yl | H |
| 1-281 | H | H | Me | 6-Cl-1,3-benzoxazol-2-yl | H |
| 1-282 | H | H | Me | 6-Br-1,3-benzoxazol-2-yl | H |
| 1-283 | H | H | Me | 7-Cl-1,3-benzoxazol-2-yl | H |
| 1-284 | H | H | Me | 5-NH₂-pyridin-2-yl | H |
| 1-285 | H | H | Me | 5-OH-pyridin-2-yl | H |
| 1-286 | H | H | Me | 5-OCHF₂-pyridin-2-yl | H |
| 1-287 | H | H | Me | 5-MeO-pyridin-2-yl | H |
| 1-288 | H | H | Me | 5-MeS-pyridin-2-yl | H |
| 1-289 | H | H | Me | 5-NHMe-pyridin-2-yl | H |
| 1-290 | H | H | Me | 5-NMe₂-pyridin-2-yl | H |
| 1-291 | H | H | Me | 4-NO₂-Ph | H |
| 1-292 | H | H | Me | 4-Me-5-Cl-pyridin-2-yl | H |

Darüber hinaus wurden NMR-Daten für erfindungsgemäße Verbindungen der allgemeinen Formel (I), erzeugt. "NMR" der Beispielverbindungen wurde jeweils als ¹H-NMR-Spektum bei 400 MHz (CDCl₃) (¹H-Kernresonanz-Daten) gemessen. Nachfolgend sind charakteristische chemische Verschiebungen δ (ppm) für einige Beispielverbindungen aufgeführt:
NMR Verbindung 1-67 (CDCl₃, 400 MHz, δ in ppm): 2.34 (*s,* 3H); 3.43 (*s,* 2H); 6.71 (*d,* 1 H); 6.96 (*d,* 1 H); 7.18 (*d,* 1 H); 8.18 (*d,* 1 H).
NMR Verbindung 1-119 (CDCl₃, 400 MHz, δ in ppm): 2.42 (*s,* 3H); 3.47 (*s,* 2H); 6.74 (*d,* 1 H); 7.35 (*d,* 1 H); 8.02 (*dd,*1H); 8.27 (*d,* 1 H); 8.85 (*d,* 1H).

**Tabelle 2: Verbindungen der Formel (Ia"')**

| | | | | | |
|---|---|---|---|---|---|
| 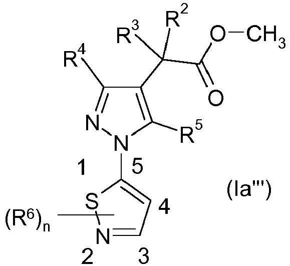 | | | | | |

| Nr. | R² | R³ | R⁴ | R⁵ | (R⁶)ₙ |
|---|---|---|---|---|---|
| 2-1 | H | H | Ph | Ph | H |
| 2-2 | H | H | Me | Ph | H |
| 2-3 | H | H | Me | 5-J-2-thienyl | H |
| 2-4 | H | H | Me | 2-Furyl | H |
| 2-5 | H | H | Me | Ph | 3-OMe |
| 2-6 | Me | H | Me | Ph | 4-Me |
| 2-7 | H | H | Me | Ph | 3-CI |
| 2-8 | H | H | Me | Ph | 4-CF₃ |
| 2-9 | H | H | Me | Ph | 3-CF₃ |
| 2-10 | H | H | Me | Ph | 4-Me |
| 2-11 | H | H | Me | Ph | 3,4-Me₂ |
| 2-12 | H | H | Me | Ph | 3,4-Cl₂ |
| 2-13 | H | H | Me | 4-MeO-Ph | 4-Me |
| 2-14 | H | H | Me | 4-MeO-Ph | H |
| 2-15 | Me | H | Me | Ph | H |
| 2-16 | H | H | Me | 4-Me-Ph | H |
| 2-17 | H | H | Me | 4-Me-Ph | 4-Me |
| 2-18 | H | H | Me | 4-Me-Ph | 4-CI |
| 2-19 | H | H | Me | 3-CI-Ph | H |
| 2-20 | H | H | Me | 3-CF₃-Ph | H |
| 2-21 | H | H | Me | 3-CF₃-Ph | 4-Me |
| 2-22 | H | H | Me | 3,4-Cl₂-Ph | 4-Me |
| 2-23 | H | H | Me | 3-Cl-Ph | 4-Me |
| 2-24 | H | H | Me | 2-Cl-Ph | 4-Me |
| 2-25 | H | H | Me | 2,4-Cl₂-Ph | 4-Me |
| 2-26 | H | H | Me | 4-CF₃-Ph | 4-Me |
| 2-27 | H | H | Me | 4-Cl-Ph | 4-Me |
| 2-28 | H | H | Me | 4-Cl-Ph | H |
| 2-29 | H | H | Me | 4-Cl-Ph | 3-CI |
| 2-30 | H | H | Me | 3,4-Cl₂-Ph | H |
| 2-31 | H | H | Me | 4-CF₃-Ph | H |
| 2-32 | H | H | Me | 4-Cl-Ph | 4-CI |
| 2-33 | H | H | Me | Ph | 4-CI |
| 2-34 | H | H | Me | 2-Cl-Ph | H |
| 2-35 | H | H | Me | 4-tBu-Ph | 4-Me |
| 2-36 | H | H | Me | 3,5-Me₂-Ph | 4-Me |
| 2-37 | H | H | Me | Ph | 4-OMe |
| 2-38 | H | H | Me | 4-Cl-Ph | 4-OMe |
| 2-39 | H | H | Me | 4-Me-Ph | 4-Me |
| 2-40 | H | H | Me | 4-F-Ph | 4-Me |
| 2-41 | H | H | Me | 4-F-Ph | 4-CI |
| 2-42 | H | H | Me | 3-Me-Ph | 4-Me |
| 2-43 | H | H | Me | 4-COOH-Ph | 4-Me |
| 2-44 | H | H | Me | 3-Br-Ph | 4-Me |
| 2-45 | H | H | Me | 4-Ph-Ph | 4-Me |
| 2-46 | H | H | Me | 4-COOH-Ph | H |
| 2-47 | H | H | Me | 3,5-Me₂-Ph | H |
| 2-48 | H | H | Me | Ph | 4-SMe |
| 2-49 | H | H | Me | 4-Cl-Ph | 4-SMe |
| 2-50 | H | H | Me | 3-Cl-4-Me-Ph | H |
| 2-51 | H | H | Me | 3-CF₃-4-Cl-Ph | H |
| 2-52 | H | H | Me | 3-CF₃-4-Cl-Ph | 4-Me |
| 2-53 | H | H | Me | 3-Cl-4-Me-Ph | 4-Me |
| 2-54 | H | H | Me | 2-Pyridyl | 4-Cl |
| 2-55 | H | H | Me | 4-CI-Ph | 4-F |
| 2-56 | H | H | Me | 2-Thienyl | 4-Me |
| 2-57 | H | H | Me | 3-Me-2-thienyl | 4-Me |
| 2-58 | H | H | Me | 4-Me-2-thienyl | 4-Me |
| 2-59 | H | H | Me | 5-Cl-2-thienyl | 4-Me |
| 2-60 | H | H | Me | 5-Cl-2-thienyl | 4-Cl |
| 2-61 | H | H | Me | 3-Thienyl | 4-Me |
| 2-62 | H | H | Me | 2-Thienyl | H |
| 2-63 | H | H | Me | 3-Me-2-thienyl | H |
| 2-64 | H | H | Me | 4-Me-2-thienyl | H |
| 2-65 | H | H | Me | 5-Cl-2-thienyl | H |
| 2-66 | H | H | Me | 5-Me-2-thienyl | H |
| 2-67 | H | H | Me | 6-MeO-pyridin-3-yl | H |
| 2-68 | H | H | Me | 5-Br-2-thienyl | H |
| 2-69 | H | H | Me | 5-Br-2-thienyl | 4-Me |
| 2-70 | H | H | Me | 3-Thienyl | H |
| 2-71 | H | H | Me | 4-Cl-Ph | 4-S(O)Me |
| 2-72 | H | H | Me | 4-Br-Ph | 4-Me |
| 2-73 | H | H | Me | 1,3-Benzodioxol-5-yl | 4-Me |
| 2-74 | H | H | Me | 4-J-Ph | 4-Me |
| 2-75 | H | H | Me | 3,5-Cl₂-Ph | 4-Me |
| 2-76 | H | H | Me | 4-PhO-Ph | 4-Me |
| 2-77 | H | H | Me | 6-OH-pyridin-3-yl | H |
| 2-78 | H | H | Me | Ph | 4-S(O)Me |
| 2-79 | H | H | H | Ph | H |
| 2-80 | H | H | H | Ph | 4-Me |
| 2-81 | H | H | Et | Ph | H |
| 2-82 | H | H | n-Pr | Ph | H |
| 2-83 | H | H | CH₂Cl | Ph | H |
| 2-84 | H | H | CHCl₂ | Ph | H |
| 2-85 | H | H | CH₂F | Ph | H |
| 2-86 | H | H | CHF₂ | Ph | H |
| 2-87 | H | H | Cl | Ph | H |
| 2-88 | H | H | Et | Ph | 4-Me |
| 2-89 | H | H | n-Pr | Ph | 4-Me |
| 2-90 | H | H | CH₂Cl | Ph | 4-Me |
| 2-91 | H | H | CHCl₂ | Ph | 4-Me |
| 2-92 | H | H | CH₂F | Ph | 4-Me |
| 2-93 | H | H | CHF₂ | Ph | 4-Me |
| 2-94 | H | H | Cl | Ph | 4-Me |
| 2-95 | H | H | Et | 4-Cl-Ph | H |
| 2-96 | H | H | n-Pr | 4-Cl-Ph | H |
| 2-97 | H | H | CH₂Cl | 4-Cl-Ph | H |
| 2-98 | H | H | CHCl₂ | 4-Cl-Ph | H |
| 2-99 | H | H | CH₂F | 4-Cl-Ph | H |
| 2-100 | H | H | CHF₂ | 4-Cl-Ph | H |
| 2-101 | H | H | Cl | 4-Cl-Ph | H |
| 2-102 | H | H | Et | 4-Me-Ph | H |
| 2-103 | H | H | n-Pr | 4-Me-Ph | H |
| 2-104 | H | H | CH₂Cl | 4-Me-Ph | H |
| 2-105 | H | H | CHCl₂ | 4-Me-Ph | H |
| 2-106 | H | H | CH₂F | 4-Me-Ph | H |
| 2-107 | H | H | CHF₂ | 4-Me-Ph | H |
| 2-108 | H | H | Cl | 4-Me-Ph | H |
| 2-109 | H | H | Et | 2-Pyridyl | H |
| 2-110 | H | H | n-Pr | 2-Pyridyl | H |
| 2-111 | H | H | CH₂Cl | 2-Pyridyl | H |
| 2-112 | H | H | CHCl₂ | 2-Pyridyl | H |
| 2-113 | H | H | CH₂F | 2-Pyridyl | H |
| 2-114 | H | H | CHF₂ | 2-Pyridyl | H |
| 2-115 | H | H | Cl | 2-Pyridyl | H |
| 2-116 | H | H | Me | 2-Pyridyl | H |
| 2-117 | H | H | Me | 5-Cl-pyridin-2-yl | H |
| 2-118 | H | H | Me | 5-Cl-pyridin-2-yl | 4-Cl |
| 2-119 | H | H | Me | 5-Cl-pyridin-2-yl | 4-Me |
| 2-120 | H | H | Me | 5-Br-pyridin-2-yl | H |
| 2-121 | H | H | Me | 5-Br-pyridin-2-yl | 4-Cl |
| 2-122 | H | H | Me | 5-Br-pyridin-2-yl | 4-Me |
| 2-123 | H | H | Me | 5-F-pyridin-2-yl | H |
| 2-124 | H | H | Me | 5-Me-pyridin-2-yl | H |
| 2-125 | H | H | Me | 5-Me-pyridin-2-yl | 4-Me |
| 2-126 | H | H | Me | 2,4-Cl₂-Ph | H |
| 2-127 | H | H | Me | 4-(CH₂COOH)-Ph | 4-Me |
| 2-128 | H | H | Me | 3,4-Me₂-Ph | 4-Me |
| 2-129 | H | H | Me | 4-Br-Ph | H |
| 2-130 | H | H | Me | 3,4-Me₂-Ph | H |
| 2-131 | H | H | Me | 3-Me-Ph | H |
| 2-132 | H | H | Me | 4-F-Ph | H |
| 2-133 | H | H | Me | 4-(Me-CO)-Ph | H |
| 2-134 | H | H | Me | 4-tBu-Ph | H |
| 2-135 | H | H | Me | 4-Cl-3-Me-Ph | H |
| 2-136 | H | H | n-Pr | 4-Cl-Ph | 4-Me |
| 2-137 | H | H | Me | 3-Pyridyl | H |
| 2-138 | H | H | Me | 4-Pyridyl | H |
| 2-139 | H | H | C(O)OMe | Ph | H |
| 2-140 | H | H | Me | 6-Me-pyridin-3-yl | H |
| 2-141 | H | H | Me | 4-Cl-Ph | 4-SO₂Me |
| 2-142 | H | H | Me | 3-Pyridyl | 4-Me |
| 2-143 | H | H | Me | 2,3-Cl₂-Ph | 4-Me |
| 2-144 | H | H | Me | 2-Pyridyl | 4-Me |
| 2-145 | H | H | H | 4-Cl-Ph | 4-Me |
| 2-146 | H | H | Me | 6-Cl-pyridin-3-yl | H |
| 2-147 | H | H | Me | 4-Cl-Ph | 3-Me |
| 2-148 | H | H | Me | Ph | 3-Me |
| 2-149 | H | H | Me | 4-Me-pyridin-2-yl | H |
| 2-150 | H | H | Me | 4-Me-pyridin-2-yl | 4-Me |
| 2-151 | H | H | Me | 4-Me-pyridin-2-yl | 4-Cl |
| 2-152 | H | H | Me | 4-Me-pyridin-2-yl | 4-F |
| 2-153 | H | H | Me | 4-F-pyridin-2-yl | H |
| 2-154 | H | H | Me | 4-Cl-pyridin-2-yl | H |
| 2-155 | H | H | Me | 4-Br-pyridin-2-yl | H |
| 2-156 | H | H | Me | 4-OMe-pyridin-2-yl | H |
| 2-157 | H | H | Me | 5-CF₃-pyridin-2-yl | H |
| 2-158 | H | H | Me | 6-OMe-pyridin-2-yl | H |
| 2-159 | H | H | cyPr | 4-Cl-Ph | H |
| 2-160 | H | H | CN | 4-Cl-Ph | H |
| 2-161 | H | H | CN | 4-Cl-Ph | 4-Me |
| 2-162 | H | H | CN | 4-Me-Ph | H |
| 2-163 | H | H | CN | 4-Me-Ph | 4-Me |
| 2-164 | H | H | CN | Ph | H |
| 2-165 | H | H | CN | Ph | 4-Me |
| 2-166 | H | H | CN | 2-pyridyl | H |
| 2-167 | H | H | CN | 3-pyridyl | H |
| 2-168 | H | H | CN | 5-Cl-pyridin-2-yl | H |
| 2-169 | H | H | CN | 5-Br-pyridin-2-yl | H |
| 2-170 | H | H | CN | 5-F-pyridin-2-yl | H |
| 2-171 | H | H | CN | 5-Me-pyridin-2-yl | H |
| 2-172 | H | H | CN | 6-Me-pyridin-3-yl | H |
| 2-173 | H | H | CN | 4-Me-pyridin-2-yl | H |
| 2-174 | H | H | CN | 4-F-pyridin-2-yl | H |
| 2-175 | H | H | CN | 4-Cl-pyridin-2-yl | H |
| 2-176 | H | H | CN | 4-Br-pyridin-2-yl | H |
| 2-177 | H | H | CN | 4-OMe-pyridin-2-yl | H |
| 2-178 | H | H | Formyl | 4-Cl-Ph | H |
| 2-179 | H | H | Formyl | 4-Cl-Ph | 4-Me |
| 2-180 | H | H | Formyl | 4-Me-Ph | H |
| 2-181 | H | H | Formyl | 4-Me-Ph | 4-Me |
| 2-182 | H | H | Formyl | Ph | H |
| 2-183 | H | H | Formyl | Ph | 4-Me |
| 2-184 | H | H | Formyl | 2-pyridyl | H |
| 2-185 | H | H | Formyl | 3-pyridyl | H |
| 2-186 | H | H | Formyl | 5-Cl-pyridin-2-yl | H |
| 2-187 | H | H | Formyl | 5-Br-pyridin-2-yl | H |
| 2-188 | H | H | Formyl | 5-F-pyridin-2-yl | H |
| 2-189 | H | H | Formyl | 5-Me-pyridin-2-yl | H |
| 2-190 | H | H | Formyl | 6-Me-pyridin-3-yl | H |
| 2-191 | H | H | Formyl | 4-Me-pyridin-2-yl | H |
| 2-192 | H | H | Formyl | 4-F-pyridin-2-yl | H |
| 2-193 | H | H | Formyl | 4-Cl-pyridin-2-yl | H |
| 2-194 | H | H | Formyl | 4-Br-pyridin-2-yl | H |
| 2-195 | H | H | Formyl | 4-OMe-pyridin-2-yl | H |
| 2-196 | H | H | CH₂OH | 5-Me-pyridin-2-yl | H |
| 2-197 | H | H | CH₂OH | 4-CI-Ph | H |
| 2-198 | H | H | CH₂OH | 4-Me-pyridin-2-yl | H |
| 2-199 | H | H | CH₂OH | 4-Me-Ph | H |
| 2-200 | H | H | CH₂OH | Ph | H |
| 2-201 | H | H | CH₂OH | 2-Pyridyl | H |
| 2-202 | H | H | Me | 2-Thiazolyl | H |
| 2-203 | H | H | Me | 2-Thiazolyl | 4-Cl |
| 2-204 | H | H | Me | 2-Thiazolyl | 4-Me |
| 2-205 | H | H | Me | 4-Me-thiazol-2-yl | H |
| 2-206 | H | H | Me | 4-Me-thiazol-2-yl | 4-Cl |
| 2-207 | H | H | Me | 4-Me-thiazol-2-yl | 4-Me |
| 2-208 | H | H | Me | 5-Me-thiazol-2-yl | H |
| 2-209 | H | H | Me | 5-Br-thiazol-2-yl | H |
| 2-210 | H | H | Me | 5-Br-thiazol-2-yl | 4-Me |
| 2-211 | H | H | Me | 5-Cl-thiazol-2-yl | H |
| 2-212 | H | H | Me | 4,6-Me₂-pyridin-2-yl | H |
| 2-213 | H | H | Me | 4,6-Me₂-pyridin-2-yl | 4-Me |
| 2-214 | H | H | Me | 2-Pyridyl | 4-F |
| 2-215 | H | H | Me | 2-Pyrazinyl | H |
| 2-216 | H | H | Me | 5-Me-Pyrazin-2-yl | H |
| 2-217 | H | H | Me | 2-Pyrazinyl | 4-Me |
| 2-218 | H | H | Me | 1,3-Benzothiazol-2-yl | H |
| 2-219 | H | H | Me | 1,3-Benzothiazol-2-yl | 4-Me |
| 2-220 | H | H | Me | 7-Cl-1,3-benzothiazol-2-yl | H |
| 2-221 | H | H | Me | 1,5-Me₂-pyrazol-3-yl | H |
| 2-222 | H | H | Me | 1,5-Me₂-pyrazol-3-yl | 4-Me |
| 2-223 | H | H | Me | 4,5-Me₂-thiazol-2-yl | H |
| 2-224 | H | H | Me | 4,5-Cl₂-thiazol-2-yl | H |
| 2-225 | H | H | Me | 2-Pyrimidinyl | H |
| 2-226 | H | H | Me | 2-Pyrimidinyl | 4-Me |
| 2-227 | H | H | Me | 5-F-pyrimidin-2-yl | H |
| 2-228 | H | H | Me | 5-Cl-pyrimidin-2-yl | H |
| 2-229 | H | H | Me | 5-Br-pyrimidin-2-yl | H |
| 2-230 | H | H | Me | 5-Me-pyrimidin-2-yl | H |
| 2-231 | H | H | Me | 5-Me-pyrimidin-2-yl | 4-Me |
| 2-232 | H | H | Me | 4,6-Me₂-pyrimidin-2-yl | H |
| 2-233 | H | H | Me | 4,6-Me₂-pyrimidin-2-yl | 4-Me |
| 2-234 | H | H | Me | 3-Pyridazinyl | H |
| 2-235 | H | H | Me | 6-Me-Pyridazin-3-yl | H |
| 2-236 | H | H | Me | 1,2,4-Triazin-3-yl | H |
| 2-237 | H | H | Me | 6-Me-1,2,4-Triazin-3-yl | H |
| 2-238 | H | H | Me | Chinolin-2-yl | H |
| 2-239 | H | H | Me | Isochinolin-3-yl | H |
| 2-240 | H | H | Me | 4-NO₂-Ph | H |
| 2-241 | H | H | Me | 3,5-Cl₂-Ph | H |
| 2-242 | H | H | Me | 2-Me-pyridin-4-yl | H |
| 2-243 | H | H | Me | 4-Cl-6-Me-pyridin-2-yl | H |
| 2-244 | H | H | Me | 4-Br-3-Me-Ph | H |
| 2-245 | H | H | Me | 5-Cl-pyridin-3-yl | H |
| 2-246 | H | H | Me | 5-Allyl-pyridin-2-yl | H |
| 2-247 | H | H | Me | 5-Cyclopropyl-pyridin-2-yl | H |
| 2-248 | H | H | Me | 5-Ethinyl-pyridin-2-yl | H |
| 2-249 | H | H | Me | 5-Ph-pyridin-2-yl | H |
| 2-250 | H | H | Me | 5-OH-pyridin-2-yl | H |
| 2-251 | H | H | Me | 5-OCHF₂-pyridin-2-yl | H |
| 2-252 | H | H | Me | 5-MeO-pyridin-2-yl | H |
| 2-253 | H | H | Me | 5-MeS-pyridin-2-yl | H |
| 2-254 | H | H | Me | 5-NHMe-pyridin-2-yl | H |
| 2-255 | H | H | Me | 5-NMe₂-pyridin-2-yl | H |
| 2-256 | H | H | Me | 6-Cl-1,3-benzoxazol-2-yl | H |
| 2-257 | H | H | Me | 6-Br-1,3-benzoxazol-2-yl | H |
| 2-258 | H | H | Me | 7-Cl-1,3-benzoxazol-2-yl | H |
| 2-259 | H | H | Me | 5-NH₂-pyridin-2-yl | H |
| 2-260 | H | H | Me | 2-Cl-thiazol-4-yl | H |
| 2-261 | H | H | Me | 2-Br-thiazol-4-yl | H |
| 2-262 | H | H | Me | 5-OSO₂Me-pyridin-2-yl | H |
| 2-263 | H | H | Me | 6-Cl-1,3-benzothiazol-2-yl | H |
| 2-264 | H | H | Me | 6-Br-1,3-benzothiazol-2-yl | H |
| 2-265 | H | H | Me | 1,3-Benzoxazol-2-yl | H |
| 2-266 | H | H | Me | 4-PhO-Ph | H |
| 2-267 | H | H | Me | 2,3-Cl₂-Ph | H |
| 2-268 | H | H | Me | 5-I-pyridin-2-yl | H |
| 2-269 | H | H | Me | 5-I-pyrimidin-2-yl | H |
| 2-270 | H | H | Me | 3,4-F₂-Ph | H |
| 2-271 | H | H | Me | 1-Me-pyrazol-3-yl | H |
| 2-272 | H | H | Me | 1-Me-pyrazol-5-yl | H |
| 2-273 | H | H | Me | 3-Br-Ph | H |
| 2-274 | H | H | Me | 4-Ph-Ph | H |
| 2-275 | H | H | Me | 1,3-Benzodioxol-5-yl | H |
| 2-276 | H | H | Me | 4-J-Ph | H |
| 2-277 | H | H | Me | 5-Br-3-thienyl | H |
| 2-278 | H | H | Me | 5-Me-3-thienyl | H |
| 2-279 | H | H | Me | 2-F-Ph | H |
| 2-280 | H | H | Me | 2-CN-Ph | H |
| 2-281 | H | H | Me | 2-NO₂-Ph | H |
| 2-282 | H | H | Me | 2,4-F₂-Ph | H |
| 2-283 | H | H | Me | 5-Thiazolyl | H |
| 2-284 | H | H | Me | 2-Me-thiazol-4-yl | H |
| 2-285 | H | H | Me | 2-Me-thiazol-5-yl | H |
| 2-286 | H | H | Me | 5-Cl-3-thienyl | H |
| 2-287 | H | H | Me | 6-Br-pyridin-3-yl | H |
| 2-288 | H | H | Me | 4-Cl-3-thienyl | H |
| 2-289 | H | H | Me | 4-Br-3-thienyl | H |
| 2-290 | H | H | Me | 4-Me-3-thienyl | H |
| 2-291 | H | H | Me | 4-Thiazolyl | H |
| 2-292 | H | H | Me | 4-Me-5-Cl-pyridin-2-yl | H |

Darüber hinaus wurden NMR-Daten für erfindungsgemäße Verbindungen der allgemeinen Formel (I), erzeugt. "NMR" der Beispielverbindungen wurde jeweils als ¹H-NMR-Spektum bei 400 MHz (CDCl₃) (¹H-Kernresonanz-Daten) gemessen. Nachfolgend sind charakteristische chemische Verschiebungen δ (ppm) für einige Beispielverbindungen aufgeführt:
NMR Verbindung 2-2 (CDCl₃, 400 MHz, δ in ppm): 2.34 (s, 3H); 3.32 (*s*, 2H); 3.68 (s, 3H); 6.39 (*d*, 1 H); 7,36 (*m,* 2H); 7.52 (*m*, 3H); 8.04 *(d,* 1 H).
NMR Verbindung 2-28 (CDCl₃, 400 MHz, δ in ppm): 2.33 (*s*, 3H); 3.31 (*s*, 2H); 3.69 (*s*, 3H); 6.43 (*d,* 1H); 7.31 (*d,* 2H); 7.50 (*d,* 2H); 8.08 (*d,* 1 H).
NMR Verbindung 2-68 (CDCl₃, 400 MHz, δ* in ppm): 2.33 (*s*, 3H); 3.49 (*s*, 2H); 3.70 (*s*, 3H); 6.71 (*d*, 1 H, *J* =1,97 Hz); 6.95 (*d*, 1 H, *J* =3,79 Hz); 7.18 (*d*, 1 H, *J* =3,79 Hz); 8.16 (*d*, 1 H, *J* =1,97 Hz).
NMR Verbindung 2-116 (CDCl₃, 400 MHz, δ in ppm): 2.35 (*s*, 3H); 3.57 (*s*, 2H); 3.69 (*s*, 3H); 6.52 (*d*, 1 H); 7.44 *(ddd,* 1 H); 7.50 (*dt,* 1 H); 7,85 (*td,* 1 H); 8.10 (*d,* 1H); 8.79 *(dt,* 1 H).
NMR Verbindung 2-120 (CDCl₃, 400 MHz, δ in ppm): 2.35 (s, 3H); 3.45 (*s*, 2H); 3.69 (*s*, 3H); 6.57 (*d*, 1 H, *J* =1,97 Hz); 7.41 *(d,* 1 H); 7,97 (*dd,* 1 H); 8.15 (d, 1H, *J* =1,97 Hz); 8.84 (*d*, 1H).

**Tabelle 3: Verbindungen der Formel (la"")**

| | | | | |
|---|---|---|---|---|
| 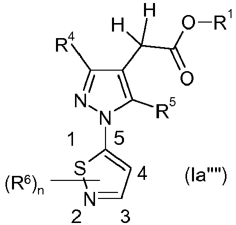 | | | | |

| Nr. | R¹ | R⁴ | R⁵ | (R⁶)ₙ |
|---|---|---|---|---|
| 3-1 | Et | Me | Ph | H |
| 3-2 | Et | Me | Ph | 4-Me |
| 3-3 | Et | Me | 3-Cl-Ph | H |
| 3-4 | Et | Me | 4-Cl-Ph | H |
| 3-5 | Et | Me | 4-Cl-Ph | 4-Me |
| 3-6 | Et | Me | 2-Thienyl | H |
| 3-7 | Et | Me | 3-Thienyl | H |
| 3-8 | Et | Me | 3-Me-2-thienyl | H |
| 3-9 | Et | Me | 4-Me-2-thienyl | H |
| 3-10 | Et | Me | 5-Br-2-thienyl | H |
| 3-11 | Et | Me | 5-Br-2-thienyl | 4-Me |
| 3-12 | Et | Me | 5-Cl-2-thienyl | H |
| 3-13 | Et | Me | 5-Cl-2-thienyl | 4-Me |
| 3-14 | Et | Me | 5-J-2-thienyl | H |
| 3-15 | Et | Me | 5-Me-2-thienyl | H |
| 3-16 | Et | Me | 3-Pyridyl | H |
| 3-17 | Et | Me | 6-MeO-pyridin-3-yl | H |
| 3-18 | Et | Me | 6-OH-pyridin-3-yl | H |
| 3-19 | Et | Me | 6-Me-pyridin-3-yl | H |
| 3-20 | Et | Me | 4-Me-Ph | H |
| 3-21 | Et | Me | 4-Me-Ph | 4-Me |
| 3-22 | Et | Me | 4-Br-Ph | H |
| 3-23 | Et | Me | 4-F-Ph | H |
| 3-24 | Et | Me | 4-F-Ph | 4-Me |
| 3-25 | Et | Me | 5-Cl-pyridin-2-yl | H |
| 3-26 | Et | Me | 5-Br-pyridin-2-yl | H |
| 3-27 | Et | Me | 5-F-pyridin-2-yl | H |
| 3-28 | Et | Me | 5-F-pyridin-2-yl | 4-Me |
| 3-29 | Et | Me | 5-Cl-pyridin-2-yl | 4-Me |
| 3-30 | Et | Me | 5-Br-pyridin-2-yl | 4-Me |
| 3-31 | Et | Me | 5-Me-pyridin-2-yl | H |
| 3-32 | Et | Me | 5-Me-pyridin-2-yl | 4-Me |
| 3-33 | Et | Me | 2-Pyridyl | 4-Me |
| 3-34 | Et | Me | 2-Pyridyl | H |
| 3-35 | Et | Me | 4-Pyridyl | H |
| 3-36 | Et | Me | 4-Me-pyridin-2-yl | H |
| 3-37 | Et | Me | 4-Me-pyridin-2-yl | 4-Me |
| 3-38 | Et | Me | 2-Thiazolyl | H |
| 3-39 | Et | Me | 4-Me-thiazol-2-yl | H |
| 3-40 | Et | Me | 5-Br-thiazol-2-yl | H |
| 3-41 | Et | Me | 5-Cl-thiazol-2-yl | H |
| 3-42 | Et | Me | 5-Me-thiazol-2-yl | H |
| 3-43 | Et | Me | 4,5-Me₂-thiazol-2-yl | H |
| 3-44 | Et | Me | 4,5-Cl₂-thiazol-2-yl | H |
| 3-45 | Et | Me | 4,6-Me₂-pyridin-2-yl | H |
| 3-46 | Et | Me | 2-Pyrazinyl | H |
| 3-47 | Et | Me | 2-Pyrimidinyl | H |
| 3-48 | Et | Me | 2-Pyrimidinyl | 4-Me |
| 3-49 | Et | Me | 5-Cl-pyrimidin-2-yl | H |
| 3-50 | Et | Me | 5-Br-pyrimidin-2-yl | H |
| 3-51 | Et | Me | 5-Me-pyrimidin-2-yl | H |
| 3-52 | Et | Me | 5-Me-pyrimidin-2-yl | 4-Me |
| 3-53 | Et | Me | 4,6-Me₂-pyrimidin-2-yl | H |
| 3-54 | Et | Me | 4,6-Me₂-pyrimidin-2-yl | 4-Me |
| 3-55 | Et | Me | 1,3-Benzothiazol-2-yl | H |
| 3-56 | Et | Me | 7-Cl-1,3-benzothiazol-2-yl | H |
| 3-57 | Et | Me | 1,5-Me₂-pyrazol-3-yl | H |
| 3-58 | Et | Me | 5-Me-Pyrazin-2-yl | H |
| 3-59 | Et | Me | 5-F-pyrimidin-2-yl | H |
| 3-60 | Et | Me | 3-Pyridazinyl | H |
| 3-61 | Et | Me | 6-Me-Pyridazin-3-yl | H |
| 3-62 | Et | Me | 1,2,4-Triazin-3-yl | H |
| 3-63 | Et | Me | 6-Me-1,2,4-triazin-3-yl | H |
| 3-64 | Et | Me | Chinolin-2-yl | H |
| 3-65 | Et | Me | Isochinolin-3-yl | H |
| 3-66 | Pr | Me | Ph | H |
| 3-67 | Pr | Me | 4-Cl-Ph | H |
| 3-68 | Pr | Me | 2-Thienyl | H |
| 3-69 | Pr | Me | 3-Pyridyl | H |
| 3-70 | Pr | Me | 6-Me-pyridin-3-yl | H |
| 3-71 | Pr | Me | 4-Me-Ph | H |
| 3-72 | Pr | Me | 4-Br-Ph | H |
| 3-73 | Pr | Me | 4-F-Ph | H |
| 3-74 | Pr | Me | 5-Cl-pyridin-2-yl | H |
| 3-75 | Pr | Me | 5-Br-pyridin-2-yl | H |
| 3-76 | Pr | Me | 5-F-pyridin-2-yl | H |
| 3-77 | Pr | Me | 5-Me-pyridin-2-yl | H |
| 3-78 | Pr | Me | 2-Pyridyl | H |
| 3-79 | Pr | Me | 4-Pyridyl | H |
| 3-80 | i-Pr | Me | Ph | H |
| 3-81 | i-Pr | Me | 4-Cl-Ph | H |
| 3-82 | i-Pr | Me | 2-Thienyl | H |
| 3-83 | i-Pr | Me | 3-Pyridyl | H |
| 3-84 | i-Pr | Me | 6-Me-pyridin-3-yl | H |
| 3-85 | i-Pr | Me | 4-Me-Ph | H |
| 3-86 | i-Pr | Me | 4-Br-Ph | H |
| 3-87 | i-Pr | Me | 4-F-Ph | H |
| 3-88 | i-Pr | Me | 5-Cl-pyridin-2-yl | H |
| 3-89 | i-Pr | Me | 5-Br-pyridin-2-yl | H |
| 3-90 | i-Pr | Me | 5-F-pyridin-2-yl | H |
| 3-91 | i-Pr | Me | 5-Me-pyridin-2-yl | H |
| 3-92 | i-Pr | Me | 2-Pyridyl | H |
| 3-93 | i-Pr | Me | 4-Pyridyl | H |
| 3-94 | CH₂Ph | Me | Ph | H |
| 3-95 | CH₂Ph | Me | 4-Cl-Ph | H |
| 3-96 | CH₂Ph | Me | 2-Thienyl | H |
| 3-97 | CH₂Ph | Me | 2-Pyridyl | H |
| 3-98 | Prop-2-in-1-yl | Me | Ph | H |
| 3-99 | Prop-2-in-1-yl | Me | 4-Cl-Ph | H |
| 3-100 | Prop-2-in-1-yl | Me | 2-Thienyl | H |
| 3-101 | Prop-2-in-1-yl | Me | 3-Thienyl | H |
| 3-102 | Prop-2-in-1-yl | Me | 3-Me-2-thienyl | H |
| 3-103 | Prop-2-in-1-yl | Me | 4-Me-2-thienyl | H |
| 3-104 | Prop-2-in-1-yl | Me | 5-Cl-2-thienyl | H |
| 3-105 | Prop-2-in-1-yl | Me | 5-Me-2-thienyl | H |
| 3-106 | Prop-2-in-1-yl | Me | 3-Pyridyl | H |
| 3-107 | Prop-2-in-1-yl | Me | 6-MeO-pyridin-3-yl | H |
| 3-108 | Prop-2-in-1-yl | H | Ph | H |
| 3-109 | Prop-2-in-1-yl | Me | 6-Me-pyridin-3-yl | H |
| 3-110 | Prop-2-in-1-yl | Me | 4-Me-Ph | H |
| 3-111 | Prop-2-in-1-yl | Me | 4-Br-Ph | H |
| 3-112 | Prop-2-in-1-yl | Me | 4-F-Ph | H |
| 3-113 | Prop-2-in-1-yl | Me | 5-Cl-pyridin-2-yl | H |
| 3-114 | Prop-2-in-1-yl | Me | 5-Br-pyridin-2-yl | H |
| 3-115 | Prop-2-in-1-yl | Me | 5-F-pyridin-2-yl | H |
| 3-116 | Prop-2-in-1-yl | Me | 5-Me-pyridin-2-yl | H |
| 3-117 | Prop-2-in-1-yl | Me | 2-Pyridyl | H |
| 3-118 | Prop-2-in-1-yl | Me | 4-Pyridyl | H |
| 3-119 | Prop-2-in-1-yl | Me | 4-Cl-Ph | 4-Me |
| 3-120 | Prop-2-in-1-yl | Me | Ph | 4-Me |
| 3-121 | Cyclopropylmethyl | Me | Ph | H |
| 3-122 | Cyclopropylmethyl | Me | 4-Cl-Ph | H |
| 3-123 | Cyclopropylmethyl | Me | 2-Thienyl | H |
| 3-124 | Cyclopropylmethyl | Me | 3-Thienyl | H |
| 3-125 | Cyclopropylmethyl | Me | 3-Me-2-thienyl | H |
| 3-126 | Cyclopropylmethyl | Me | 3-Pyridyl | H |
| 3-127 | Cyclopropylmethyl | Me | 5-Cl-2-thienyl | H |
| 3-128 | Cyclopropylmethyl | Me | 5-Me-2-thienyl | H |
| 3-129 | Cyclopropylmethyl | Me | 4-Me-2-thienyl | H |
| 3-130 | Cyclopropylmethyl | Me | 6-MeO-pyridin-3-yl | H |
| 3-131 | Cyclopropylmethyl | Me | 6-OH-pyridin-3-yl | H |
| 3-132 | Cyclopropylmethyl | Me | 6-Me-pyridin-3-yl | H |
| 3-133 | Cyclopropylmethyl | Me | 4-Me-Ph | H |
| 3-134 | Cyclopropylmethyl | Me | 4-Br-Ph | H |
| 3-135 | Cyclopropylmethyl | Me | 4-F-Ph | H |
| 3-136 | Cyclopropylmethyl | Me | 5-Cl-pyridin-2-yl | H |
| 3-137 | Cyclopropylmethyl | Me | 5-Br-pyridin-2-yl | H |
| 3-138 | Cyclopropylmethyl | Me | 5-F-pyridin-2-yl | H |
| 3-139 | Cyclopropylmethyl | Me | 5-Me-pyridin-2-yl | H |
| 3-140 | Cyclopropylmethyl | Me | 2-Pyridyl | H |
| 3-141 | Cyclopropylmethyl | Me | 4-Pyridyl | H |
| 3-142 | Cyclopropylmethyl | Me | 4-Cl-Ph | 4-Me |
| 3-143 | Cyclopropylmethyl | Me | Ph | 4-Me |
| 3-144 | Cyclopropylmethyl | H | Ph | H |
| 3-145 | Cyclopropylmethyl | H | Chinolin-2-yl | H |
| 3-146 | Cyclopropylmethyl | H | Isochinolin-3-yl | H |
| 3-147 | 3,3-Dichlor-2-fluorprop-2-en-1-yl | Me | Ph | H |
| 3-148 | 3,3-Dichlor-2-fluorprop-2-en-1-yl | Me | 4-Cl-Ph | H |
| 3-149 | 3,3-Dichlor-2-fluorprop-2-en-1-yl | Me | 2-Thienyl | H |
| 3-150 | 3,3-Dichlor-2-fluorprop-2-en-1-yl | Me | 2-Pyridyl | H |
| 3-151 | (1-Methylcyclopropyl)-methyl | Me | Ph | H |
| 3-152 | (1-Methylcyclopropyl)-methyl | Me | 4-Cl-Ph | H |
| 3-153 | (1-Methylcyclopropyl)-methyl | Me | 2-Thienyl | H |
| 3-154 | (1-Methylcyclopropyl)-methyl | Me | 2-Pyridyl | H |
| 3-155 | 4-Chlorbut-2-in-1-yl | Me | Ph | H |
| 3-156 | 4-Chlorbut-2-in-1-yl | Me | 4-Cl-Ph | H |
| 3-157 | 4-Chlorbut-2-in-1-yl | Me | 2-Thienyl | H |
| 3-158 | 4-Chlorbut-2-in-1-yl | Me | 2-Pyridyl | H |
| 3-159 | (2,2-Dichlorcyclopropyl)-methyl | Me | Ph | H |
| 3-160 | (2,2-Dichlorcyclopropyl)-methyl | Me | 4-Cl-Ph | H |
| 3-161 | (2,2-Dichlorcyclopropyl)-methyl | Me | 2-Thienyl | H |
| 3-162 | (2,2-Dichlorcyclopropyl)-methyl | Me | 2-Pyridyl | H |
| 3-163 | But-2-in-1-yl | Me | Ph | H |
| 3-164 | But-2-in-1-yl | Me | 4-Cl-Ph | H |
| 3-165 | But-2-in-1-yl | Me | 2-Thienyl | H |
| 3-166 | But-2-in-1-yl | Me | 3-Thienyl | H |
| 3-167 | But-2-in-1-yl | Me | 3-Me-2-thienyl | H |
| 3-168 | But-2-in-1-yl | Me | 4-Me-2-thienyl | H |
| 3-169 | But-2-in-1-yl | Me | 5-Cl-2-thienyl | H |
| 3-170 | But-2-in-1-yl | Me | 5-Me-2-thienyl | H |
| 3-171 | But-2-in-1-yl | Me | 3-Pyridyl | H |
| 3-172 | But-2-in-1-yl | Me | 6-MeO-pyridin-3-yl | H |
| 3-173 | But-2-in-1-yl | H | Ph | H |
| 3-174 | But-2-in-1-yl | Me | 6-Me-pyridin-3-yl | H |
| 3-175 | But-2-in-1-yl | Me | 4-Me-Ph | H |
| 3-176 | But-2-in-1-yl | Me | 4-Br-Ph | H |
| 3-177 | But-2-in-1-yl | Me | 4-F-Ph | H |
| 3-178 | But-2-in-1-yl | Me | 5-Cl-pyridin-2-yl | H |
| 3-179 | But-2-in-1-yl | Me | 5-Br-pyridin-2-yl | H |
| 3-180 | But-2-in-1-yl | Me | 5-F-pyridin-2-yl | H |
| 3-181 | But-2-in-1-yl | Me | 5-Me-pyridin-2-yl | H |
| 3-182 | But-2-in-1-yl | Me | 2-Pyridyl | H |
| 3-183 | But-2-in-1-yl | Me | 4-Pyridyl | H |
| 3-184 | But-2-in-1-yl | Me | 4-Cl-Ph | 4-Me |
| 3-185 | But-2-in-1-yl | Me | Ph | 4-Me |
| 3-186 | 1-Methylprop-2-in-1-yl | Me | Ph | H |
| 3-187 | 1-Methylprop-2-in-1-yl | Me | 4-Cl-Ph | H |
| 3-188 | 1-Methylprop-2-in-1-yl | Me | 2-Thienyl | H |
| 3-189 | 1-Methylprop-2-in-1-yl | Me | 2-Pyridyl | H |
| 3-190 | 1-Cyclopropylethyl | Me | Ph | H |
| 3-191 | 1-Cyclopropylethyl | Me | 4-Cl-Ph | H |
| 3-192 | 1-Cyclopropylethyl | Me | 2-Thienyl | H |
| 3-193 | 1-Cyclopropylethyl | Me | 2-Pyridyl | H |
| 3-194 | Allyl | Me | Ph | H |
| 3-195 | Allyl | Me | 4-Cl-Ph | H |
| 3-196 | Allyl | Me | 2-Thienyl | H |
| 3-197 | Allyl | Me | 2-Pyridyl | H |
| 3-198 | 3-Methylbut-2-en-1-yl | Me | Ph | H |
| 3-199 | 3-Methylbut-2-en-1-yl | Me | 4-Cl-Ph | H |
| 3-200 | 3-Methylbut-2-en-1-yl | Me | 2-Thienyl | H |
| 3-201 | 3-Methylbut-2-en-1-yl | Me | 2-Pyridyl | H |
| 3-202 | 2-Methylprop-2-en-1-yl | Me | Ph | H |
| 3-203 | 2-Methylprop-2-en-1-yl | Me | 4-Cl-Ph | H |
| 3-204 | 2-Methylprop-2-en-1-yl | Me | 2-Thienyl | H |
| 3-205 | 2-Methylprop-2-en-1-yl | Me | 2-Pyridyl | H |
| 3-206 | (2E)-1-Methylbut-2-en-1-yl | Me | Ph | H |
| 3-207 | (2E)-1-Methylbut-2-en-1-yl | Me | 4-Cl-Ph | H |
| 3-208 | (2E)-1-Methylbut-2-en-1-yl | Me | 2-Thienyl | H |
| 3-209 | (2E)-1-Methylbut-2-en-1-yl | Me | 2-Pyridyl | H |
| 3-210 | 3-Phenylprop-2-in-1-yl | Me | Ph | H |
| 3-211 | 3-Phenylprop-2-in-1-yl | Me | 4-Cl-Ph | H |
| 3-212 | 3-Phenylprop-2-in-1-yl | Me | 2-Thienyl | H |
| 3-213 | 3-Phenylprop-2-in-1-yl | Me | 2-Pyridyl | H |
| 3-214 | Cyclobutylmethyl | Me | Ph | H |
| 3-215 | Cyclobutylmethyl | Me | 4-Cl-Ph | H |
| 3-216 | Cyclobutylmethyl | Me | 2-Thienyl | H |
| 3-217 | Cyclobutylmethyl | Me | 2-Pyridyl | H |
| 3-218 | Cyclopentylmethyl | Me | Ph | H |
| 3-219 | Cyclopentylmethyl | Me | 4-Cl-Ph | H |
| 3-220 | Cyclopentylmethyl | Me | 2-Thienyl | H |
| 3-221 | Cyclopentylmethyl | Me | 2-Pyridyl | H |
| 3-222 | Cyclohexylmethyl | Me | Ph | H |
| 3-223 | Cyclohexylmethyl | Me | 4-Cl-Ph | H |
| 3-224 | Cyclohexylmethyl | Me | 2-Thienyl | H |
| 3-225 | Cyclohexylmethyl | Me | 2-Pyridyl | H |
| 3-226 | But-3-en-1-yl | Me | Ph | H |
| 3-227 | But-3-en-1-yl | Me | 4-Cl-Ph | H |
| 3-228 | But-3-en-1-yl | Me | 2-Thienyl | H |
| 3-229 | But-3-en-1-yl | Me | 2-Pyridyl | H |
| 3-230 | 2-Chloroprop-2-en-1-yl | Me | Ph | H |
| 3-231 | 2-Ch loroprop-2-en-1-yl | Me | 4-Cl-Ph | H |
| 3-232 | 2-Chloroprop-2-en-1-yl | Me | 2-Thienyl | H |
| 3-233 | 2-Chloroprop-2-en-1-yl | Me | 3-Thienyl | H |
| 3-234 | 2-Chloroprop-2-en-1-yl | Me | 3-Me-2-thienyl | H |
| 3-235 | 2-Chloroprop-2-en-1-yl | Me | 4-Me-2-thienyl | H |
| 3-236 | 2-Chloroprop-2-en-1-yl | Me | 5-Cl-2-thienyl | H |
| 3-237 | 2-Chloroprop-2-en-1-yl | Me | 5-Me-2-thienyl | H |
| 3-238 | 2-Chloroprop-2-en-1-yl | Me | 3-Pyridyl | H |
| 3-239 | 2-Chloroprop-2-en-1-yl | Me | 6-MeO-pyridin-3-yl | H |
| 3-240 | 2-Chloroprop-2-en-1-yl | Me | 6-OH-pyridin-3-yl | H |
| 3-241 | 2-Chloroprop-2-en-1-yl | Me | 6-Me-pyridin-3-yl | H |
| 3-242 | 2-Chloroprop-2-en-1-yl | Me | 4-Me-Ph | H |
| 3-243 | 2-Chloroprop-2-en-1-yl | Me | 4-Br-Ph | H |
| 3-244 | 2-Chioroprop-2-en-1-yl | Me | 4-F-Ph | H |
| 3-245 | 2-Chloroprop-2-en-1-yl | Me | 5-Cl-pyridin-2-yl | H |
| 3-246 | 2-Chloroprop-2-en-1-yl | Me | 5-Br-pyridin-2-yl | H |
| 3-247 | 2-Chloroprop-2-en-1-yl | Me | 5-F-pyridin-2-yl | H |
| 3-248 | 2-Chloroprop-2-en-1-yl | Me | 5-Me-pyridin-2-yl | H |
| 3-249 | 2-Chloroprop-2-en-1-yl | Me | 2-Pyridyl | H |
| 3-250 | 2-Chloroprop-2-en-1-yl | Me | 4-Pyridyl | H |
| 3-251 | 2-Chloroprop-2-en-1-yl | Me | 4-Cl-Ph | 4-Me |
| 3-252 | 2-Chloroprop-2-en-1-yl | Me | Ph | 4-Me |
| 3-253 | 2-Chloroprop-2-en-1-yl | H | Ph | H |
| 3-254 | 2-Chloroprop-2-en-1-yl | H | Chinolin-2-yl | H |
| 3-255 | 2-Chloroprop-2-en-1-yl | H | Isochinolin-3-yl | H |
| 3-256 | 2-Methoxyethyl | Me | Ph | H |
| 3-257 | 2-Methoxyethyl | Me | 4-Cl-Ph | H |
| 3-258 | 2-Methoxyethyl | Me | 2-Thienyl | H |
| 3-259 | 2-Methoxyethyl | Me | 2-Pyridyl | H |
| 3-260 | Tetrahydrofuran-2-yl-methyl | Me | Ph | H |
| 3-261 | Tetrahydrofuran-2-yl-methyl | Me | 4-Cl-Ph | H |
| 3-262 | Tetrahydrofuran-2-yl-methyl | Me | 2-Thienyl | H |
| 3-263 | Tetrahydrofuran-2-yl-methyl | Me | 2-Pyridyl | H |
| 3-264 | 2-(Dimethylamino)ethyl | Me | Ph | H |
| 3-265 | 2-(Dimethylamino)ethyl | Me | 4-Cl-Ph | H |
| 3-266 | 2-(Dimethylamino)ethyl | Me | 2-Thienyl | H |
| 3-267 | 2-(Dimethylamino)ethyl | Me | 2-Pyridyl | H |
| 3-268 | Oxetan-3-yl | Me | Ph | H |
| 3-269 | Oxetan-3-yl | Me | 4-Cl-Ph | H |
| 3-270 | Oxetan-3-yl | Me | 2-Thienyl | H |
| 3-271 | Oxetan-3-yl | Me | 2-Pyridyl | H |
| 3-272 | (3-Methyloxetan-3-yl)methyl | Me | Ph | H |
| 3-273 | (3-Methyloxetan-3-yl)methyl | Me | 4-Cl-Ph | H |
| 3-274 | (3-Methyloxetan-3-yl)methyl | Me | 2-Thienyl | H |
| 3-275 | (3-Methyloxetan-3-yl)methyl | Me | 2-Pyridyl | H |
| 3-276 | 2,2,2-Trifluorethyl | Me | Ph | H |
| 3-277 | 2,2,2-Trifluorethyl | Me | 4-Cl-Ph | H |
| 3-278 | 2,2,2-Trifluorethyl | Me | 2-Thienyl | H |
| 3-279 | 2,2,2-Trifluorethyl | Me | 3-Pyridyl | H |
| 3-280 | 2,2,2-Trifluorethyl | Me | 6-Me-pyridin-3-yl | H |
| 3-281 | 2,2,2-Trifluorethyl | Me | 4-Me-Ph | H |
| 3-282 | 2,2,2-Trifluorethyl | Me | 4-Br-Ph | H |
| 3-283 | 2,2,2-Trifluorethyl | Me | 4-F-Ph | H |
| 3-284 | 2,2,2-Trifluorethyl | Me | 5-Cl-pyridin-2-yl | H |
| 3-285 | 2,2,2-Trifluorethyl | Me | 5-Br-pyridin-2-yl | H |
| 3-286 | 2,2,2-Trifluorethyl | Me | 5-F-pyridin-2-yl | H |
| 3-287 | 2,2,2-Trifluorethyl | Me | 5-Me-pyridin-2-yl | H |
| 3-288 | 2,2,2-Trifluorethyl | Me | 2-Pyridyl | H |
| 3-289 | 2,2,2-Trifluorethyl | Me | 4-Pyridyl | H |
| 3-290 | CH₂(4-Cl-Ph) | Me | Ph | H |
| 3-291 | CH₂(4-Cl-Ph) | Me | 4-Cl-Ph | H |
| 3-292 | CH₂(4-Cl-Ph) | Me | 2-Thienyl | H |
| 3-293 | CH₂(4-Cl-Ph) | Me | 3-Pyridyl | H |
| 3-294 | CH₂(4-Cl-Ph) | Me | 6-Me-pyridin-3-yl | H |
| 3-295 | CH₂(4-Cl-Ph) | Me | 4-Me-Ph | H |
| 3-296 | CH₂(4-Cl-Ph) | Me | 4-Br-Ph | H |
| 3-297 | CH₂(4-Cl-Ph) | Me | 4-F-Ph | H |
| 3-298 | CH₂(4-Cl-Ph) | Me | 5-Cl-pyridin-2-yl | H |
| 3-299 | CH₂(4-Cl-Ph) | Me | 5-Br-pyridin-2-yl | H |
| 3-300 | CH₂(4-Cl-Ph) | Me | 5-F-pyridin-2-yl | H |
| 3-301 | CH₂(4-Cl-Ph) | Me | 5-Me-pyridin-2-yl | H |
| 3-302 | CH₂(4-Cl-Ph) | Me | 2-Pyridyl | H |
| 3-303 | CH₂(4-Cl-Ph) | Me | 4-Pyridyl | H |
| 3-304 | CH₂(4-F-Ph) | Me | Ph | H |
| 3-305 | CH₂(4-F-Ph) | Me | 4-Cl-Ph | H |
| 3-306 | CH₂(4-F-Ph) | Me | 2-Thienyl | H |
| 3-307 | CH₂(4-F-Ph) | Me | 3-Pyridyl | H |
| 3-308 | CH₂(4-F-Ph) | Me | 6-Me-pyridin-3-yl | H |
| 3-309 | CH₂(4-F-Ph) | Me | 4-Me-Ph | H |
| 3-310 | CH₂(4-F-Ph) | Me | 4-Br-Ph | H |
| 3-311 | CH₂(4-F-Ph) | Me | 4-F-Ph | H |
| 3-312 | CH₂(4-F-Ph) | Me | 5-Cl-pyridin-2-yl | H |
| 3-313 | CH₂(4-F-Ph) | Me | 5-Br-pyridin-2-yl | H |
| 3-314 | CH₂(4-F-Ph) | Me | 5-F-pyridin-2-yl | H |
| 3-315 | CH₂(4-F-Ph) | Me | 5-Me-pyridin-2-yl | H |
| 3-316 | CH₂(4-F-Ph) | Me | 2-Pyridyl | H |
| 3-317 | CH₂(4-F-Ph) | Me | 4-Pyridyl | H |
| 3-318 | CH₂(4-OMe-Ph) | Me | Ph | H |
| 3-319 | CH₂(4-OMe-Ph) | Me | 4-Cl-Ph | H |
| 3-320 | CH₂(4-OMe-Ph) | Me | 2-Thienyl | H |
| 3-321 | CH₂(4-OMe-Ph) | Me | 3-Pyridyl | H |
| 3-322 | CH₂(4-OMe-Ph) | Me | 6-Me-pyridin-3-yl | H |
| 3-323 | CH₂(4-OMe-Ph) | Me | 4-Me-Ph | H |
| 3-324 | CH₂(4-OMe-Ph) | Me | 4-Br-Ph | H |
| 3-325 | CH₂(4-OMe-Ph) | Me | 4-F-Ph | H |
| 3-326 | CH₂(4-OMe-Ph) | Me | 5-Cl-pyridin-2-yl | H |
| 3-327 | CH₂(4-OMe-Ph) | Me | 5-Br-pyridin-2-yl | H |
| 3-328 | CH₂(4-OMe-Ph) | Me | 5-F-pyridin-2-yl | H |
| 3-329 | CH₂(4-OMe-Ph) | Me | 5-Me-pyridin-2-yl | H |
| 3-330 | CH₂(4-OMe-Ph) | Me | 2-Pyridyl | H |
| 3-331 | CH₂(4-OMe-Ph) | Me | 4-Pyridyl | H |
| 3-332 | 2,2-Difluorethyl | Me | Ph | H |
| 3-333 | 2,2-Difluorethyl | Me | 4-Cl-Ph | H |
| 3-334 | 2,2-Difluorethyl | Me | 2-Thienyl | H |
| 3-335 | 2,2-Difluorethyl | Me | 2-Pyridyl | H |
| 3-336 | Ph | Me | Ph | H |
| 3-337 | Ph | Me | 4-Cl-Ph | H |
| 3-338 | Ph | Me | 2-Thienyl | H |
| 3-339 | Ph | Me | 2-Pyridyl | H |
| 3-340 | 2-Fluorethyl | Me | Ph | H |
| 3-341 | 2-Fluorethyl | Me | 4-Cl-Ph | H |
| 3-342 | 2-Fluorethyl | Me | 2-Thienyl | H |
| 3-343 | 2-Fluorethyl | Me | 2-Pyridyl | H |
| 3-344 | 2,2,3,3,3-Pentafluorpropyl | Me | Ph | H |
| 3-345 | 2,2,3,3,3-Pentafluorpropyl | Me | 4-Cl-Ph | H |
| 3-346 | 2,2,3,3,3-Pentafluorpropyl | Me | 2-Thienyl | H |
| 3-347 | 2,2,3,3,3-Pentafluorpropyl | Me | 2-Pyridyl | H |
| 3-348 | 1-Ethyl-5-methyl-1 H-pyrazol-4-yl-methyl | Me | Ph | H |
| 3-349 | 1-Ethyl-5-methyl-1 H-pyrazol-4-yl-methyl | Me | 4-Cl-Ph | H |
| 3-350 | 1-Ethyl-5-methyl-1 H-pyrazol-4-yl-methyl | Me | 2-Thienyl | H |
| 3-351 | 1-Ethyl-5-methyl-1 H-pyrazol-4-yl-methyl | Me | 2-Pyridyl | H |
| 3-352 | 1-Ethyl-5-methyl-1 H-pyrazol-4-yl-methyl | Me | Ph | H |
| 3-353 | 1-Ethyl-5-methyl-1 H-pyrazol-4-yl-methyl | Me | 4-Cl-Ph | H |
| 3-354 | 1-Ethyl-5-methyl-1 H-pyrazol-4-yl-methyl | Me | 2-Thienyl | H |
| 3-355 | 1-Ethyl-5-methyl-1 H-pyrazol-4-yl-methyl | Me | 2-Pyridyl | H |
| 3-356 | Prop-2-in-1-yl | Me | Isochinolin-3-yl | H |
| 3-357 | Prop-2-in-1-yl | Me | Chinolin-2-yl | H |
| 3-358 | But-2-in-1-yl | Me | Isochinolin-3-yl | H |
| 3-359 | But-2-in-1-yl | Me | Chinolin-2-yl | H |
| 3-360 | 2,2-Difluorethyl | Me | 5-Cl-pyridin-2-yl | H |
| 3-361 | But-3-in-2-yl | Me | 5-Cl-pyridin-2-yl | H |
| 3-362 | But-3-in-2-yl | Me | Isochinolin-3-yl | H |
| 3-363 | But-3-in-2-yl | Me | Chinolin-2-yl | H |
| 3-364 | But-3-in-2-yl | Me | Ph | H |
| 3-365 | But-3-in-2-yl | Me | 4-Cl-Ph | H |
| 3-366 | But-3-in-2-yl | Me | 2-Thienyl | H |
| 3-367 | But-3-in-2-yl | Me | 3-Pyridyl | H |
| 3-368 | But-3-in-2-yl | Me | 6-Me-pyridin-3-yl | H |
| 3-369 | But-3-in-2-yl | Me | 4-Me-Ph | H |
| 3-370 | But-3-in-2-yl | Me | 4-Br-Ph | H |
| 3-371 | But-3-in-2-yl | Me | 4-F-Ph | H |
| 3-372 | But-3-in-2-yl | Me | 5-Br-pyridin-2-yl | H |
| 3-373 | But-3-in-2-yl | Me | 5-F-pyridin-2-yl | H |
| 3-374 | But-3-in-2-yl | Me | 5-Me-pyridin-2-yl | H |
| 3-375 | But-3-in-2-yl | Me | 2-Pyridyl | H |
| 3-376 | But-3-in-2-yl | Me | 4-Pyridyl | H |
| 3-377 | Pr | Me | Isochinolin-3-yl | H |
| 3-378 | Pr | Me | Chinolin-2-yl | H |
| 3-379 | iPr | Me | Isochinolin-3-yl | H |
| 3-380 | iPr | Me | Chinolin-2-yl | H |
| 3-381 | CH₂Ph | Me | Isochinolin-3-yl | H |
| 3-382 | CH₂Ph | Me | Chinolin-2-yl | H |
| 3-383 | 3,3-Dichlor-2-fluorprop-2-en-1-yl | Me | Isochinolin-3-yl | H |
| 3-384 | 3,3-Dichlor-2-fluorprop-2-en-1-yl | Me | Chinolin-2-yl | H |
| 3-385 | (1-Methylcyclopropyl)-methyl | Me | Isochinolin-3-yl | H |
| 3-386 | (1-Methylcyclopropyl)-methyl | Me | Chinolin-2-yl | H |
| 3-387 | 4-Chlorbut-2-in-1-yl | Me | Isochinolin-3-yl | H |
| 3-388 | 4-Chlorbut-2-in-1-yl | Me | Chinolin-2-yl | H |
| 3-389 | (2,2-Dichlorcyclopropyl)-methyl | Me | Isochinolin-3-yl | H |
| 3-390 | (2,2-Dichlorcyclopropyl)-methyl | Me | Chinolin-2-yl | H |
| 3-391 | 1-Methylprop-2-in-1-yl | Me | Isochinolin-3-yl | H |
| 3-392 | 1-Methylprop-2-in-1-yl | Me | Chinolin-2-yl | H |
| 3-393 | 1-Cyclopropylethyl | Me | Isochinolin-3-yl | H |
| 3-394 | 1-Cyclopropylethyl | Me | Chinolin-2-yl | H |
| 3-395 | Allyl | Me | Isochinolin-3-yl | H |
| 3-396 | Allyl | Me | Chinolin-2-yl | H |
| 3-397 | 3-Methylbut-2-en-1-yl | Me | Isochinolin-3-yl | H |
| 3-398 | 3-Methylbut-2-en-1-yl | Me | Chinolin-2-yl | H |
| 3-399 | Cyclobutylmethyl | Me | Isochinolin-3-yl | H |
| 3-400 | Cyclobutylmethyl | Me | Chinolin-2-yl | H |
| 3-401 | Cyclopentylmethyl | Me | Isochinolin-3-yl | H |
| 3-402 | Cyclopentylmethyl | Me | Chinolin-2-yl | H |
| 3-403 | Tetrahydrofuran-2-yl-methyl | Me | Isochinolin-3-yl | H |
| 3-404 | Tetrahydrofuran-2-yl-methyl | Me | Chinolin-2-yl | H |
| 3-405 | Tetrahydrofuran-2-yl-methyl | Me | 5-Cl-pyridin-2-yl | H |
| 3-406 | Tetrahydrofuran-2-yl-methyl | Me | 5-Br-pyridin-2-yl | H |
| 3-407 | Oxetan-3-yl | Me | Isochinolin-3-yl | H |
| 3-408 | Oxetan-3-yl | Me | Chinolin-2-yl | H |
| 3-409 | Oxetan-3-yl | Me | 5-Cl-pyridin-2-yl | H |
| 3-410 | Oxetan-3-yl | Me | 5-Br-pyridin-2-yl | H |
| 3-411 | (3-Methyloxetan-3-yl)methyl | Me | Isochinolin-3-yl | H |
| 3-412 | (3-Methyloxetan-3-yl)methyl | Me | Chinolin-2-yl | H |
| 3-413 | (3-Methyloxetan-3-yl)methyl | Me | 5-Cl-pyridin-2-yl | H |
| 3-414 | (3-Methyloxetan-3-yl)methyl | Me | 5-Br-pyridin-2-yl | H |
| 3-415 | 2,2,2-Trifluorethyl | Me | Isochinolin-3-yl | H |
| 3-416 | 2,2,2-Trifluorethyl | Me | Chinolin-2-yl | H |
| 3-417 | 2,2,2-Trifluorethyl | Me | 5-Cl-pyridin-2-yl | H |
| 3-418 | 2,2,2-Trifluorethyl | Me | 5-Br-pyridin-2-yl | H |
| 3-419 | 2,2-Difluorethyl | Me | Isochinolin-3-yl | H |
| 3-420 | 2,2-Difluorethyl | Me | Chinolin-2-yl | H |
| 3-421 | 2,2-Difluorethyl | Me | 5-Cl-pyridin-2-yl | H |
| 3-422 | 2,2-Difluorethyl | Me | 5-Br-pyridin-2-yl | H |
| 3-423 | Et | Me | 6-Cl-1,3-benzothiazol-2-yl | H |
| 3-424 | Et | Me | 6-Br-1,3-benzothiazol-2-yl | H |
| 3-425 | Et | Me | 4-OMe-pyridin-2-yl | H |
| 3-426 | Et | Me | 4-F-pyridin-2-yl | H |
| 3-427 | Et | Me | 4-Cl-pyridin-2-yl | H |
| 3-428 | Et | Me | 4-Br-pyridin-2-yl | H |
| 3-429 | Et | Me | 6-Cl-pyridin-3-yl | H |
| 3-430 | Et | Me | 6-Br-pyridin-3-yl | H |
| 3-431 | Et | Me | 4-Cl-3-thienyl | H |
| 3-432 | Et | Me | 4-Br-3-thienyl | H |
| 3-433 | Et | Me | 4-Me-3-thienyl | H |
| 3-434 | Et | Me | 4-Thiazolyl | H |
| 3-435 | Et | Me | 5-Thiazolyl | H |
| 3-436 | Et | Me | 2-Me-thiazol-4-yl | H |
| 3-437 | Et | Me | 2-Me-thiazol-5-yl | H |
| 3-438 | Et | Me | 5-Cl-3-thienyl | H |
| 3-439 | Et | Me | 5-Br-3-thienyl | H |
| 3-440 | Et | Me | 5-Me-3-thienyl | H |
| 3-441 | Et | Me | 2-Cl-Ph | H |
| 3-442 | Et | Me | 2,4-Cl₂-Ph | H |
| 3-443 | Et | Me | 2-F-Ph | H |
| 3-444 | Et | Me | 2-CN-Ph | H |
| 3-445 | Et | Me | 2-NO₂-Ph | H |
| 3-446 | Et | Me | 2,4-F₂-Ph | H |
| 3-447 | Et | Me | 3,4-F₂-Ph | H |
| 3-448 | Et | Me | 1-Me-pyrazol-3-yl | H |
| 3-449 | Et | Me | 2-Furyl | H |
| 3-450 | Et | Me | 4-MeO-Ph | H |
| 3-451 | Et | Me | 3-CF₃-Ph | H |
| 3-452 | Et | Me | 3,4-Cl₂-Ph | H |
| 3-453 | Et | Me | 4-CF₃-Ph | H |
| 3-454 | Et | Me | 4-tBu-Ph | H |
| 3-455 | Et | Me | 3,5-Me₂-Ph | H |
| 3-456 | Et | Me | 3-Me-Ph | H |
| 3-457 | Et | Me | 3-Br-Ph | H |
| 3-458 | Et | Me | 4-Ph-Ph | H |
| 3-459 | Et | Me | 3-Cl-4-Me-Ph | H |
| 3-460 | Et | Me | 3-CF₃-4-Cl-Ph | H |
| 3-461 | Et | Me | 1,3-Benzodioxol-5-yl | H |
| 3-462 | Et | Me | 4-J-Ph | H |
| 3-463 | Et | Me | 3,5-Cl₂-Ph | H |
| 3-464 | Et | Me | 4-PhO-Ph | H |
| 3-465 | Et | Me | 3,4-Me₂-Ph | H |
| 3-466 | Et | Me | 4-(Me-CO)-Ph | H |
| 3-467 | Et | Me | 4-Cl-3-Me-Ph | H |
| 3-468 | Et | Me | 2,3-Cl₂-Ph | H |
| 3-469 | Et | Me | 5-CF₃-pyridin-2-yl | H |
| 3-470 | Et | Me | 6-OMe-pyridin-2-yl | H |
| 3-471 | Et | Me | 2-Me-pyridin-4-yl | H |
| 3-472 | Et | Me | 4-Cl-6-Me-pyridin-2-yl | H |
| 3-473 | Et | Me | 4-Br-3-Me-Ph | H |
| 3-474 | Et | Me | 5-Cl-pyridin-3-yl | H |
| 3-475 | Et | Me | 5-Allyl-pyridin-2-yl | H |
| 3-476 | Et | Me | 5-Cyclopropyl-pyridin-2-yl | H |
| 3-477 | Et | Me | 5-Ethinyl-pyridin-2-yl | H |
| 3-478 | Et | Me | 5-Ph-pyridin-2-yl | H |
| 3-479 | Et | Me | 5-I-pyridin-2-yl | H |
| 3-480 | Et | Me | 5-I-pyrimidin-2-yl | H |
| 3-481 | Et | Me | 2-Cl-thiazol-4-yl | H |
| 3-482 | Et | Me | 2-Br-thiazol-4-yl | H |
| 3-483 | Et | Me | 5-OSO₂Me-pyridin-2-yl | H |
| 3-484 | Et | Me | 1,3-Benzoxazol-2-yl | H |
| 3-485 | Et | Me | 6-Cl-1,3-benzoxazol-2-yl | H |
| 3-486 | Et | Me | 6-Br-1,3-benzoxazol-2-yl | H |
| 3-487 | Et | Me | 7-Cl-1,3-benzoxazol-2-yl | H |
| 3-488 | Et | Me | 5-NH₂-pyridin-2-yl | H |
| 3-489 | Et | Me | 5-OH-pyridin-2-yl | H |
| 3-490 | Et | Me | 5-OCHF₂-pyridin-2-yl | H |
| 3-491 | Et | Me | 5-MeO-pyridin-2-yl | H |
| 3-492 | Et | Me | 5-MeS-pyridin-2-yl | H |
| 3-493 | Et | Me | 5-NHMe-pyridin-2-yl | H |
| 3-494 | Et | Me | 5-NMe₂-pyridin-2-yl | H |
| 3-495 | Et | Me | 4-NO₂-Ph | H |
| 3-496 | Cyclopropylmethyl | Me | 4-Thiazolyl | H |
| 3-497 | Prop-2-in-1-yl | Me | 4-Thiazolyl | H |
| 3-498 | But-2-in-1-yl | Me | 4-Thiazolyl | H |
| 3-499 | But-3-in-2-yl | Me | 4-Thiazolyl | H |
| 3-500 | Pr | Me | 4-Thiazolyl | H |
| 3-501 | iPr | Me | 4-Thiazolyl | H |
| 3-502 | CH₂Ph | Me | 4-Thiazolyl | H |
| 3-503 | 3,3-Dichlor-2-fluorprop-2-en-1-yl | Me | 4-Thiazolyl | H |
| 3-504 | (1-Methylcyclopropyl)-methyl | Me | 4-Thiazolyl | H |
| 3-505 | 4-Chlorbut-2-in-1-yl | Me | 4-Thiazolyl | H |
| 3-506 | (2,2-Dichlorcyclopropyl)-methyl | Me | 4-Thiazolyl | H |
| 3-507 | 1-Methylprop-2-in-1-yl | Me | 4-Thiazolyl | H |
| 3-508 | 1-Cyclopropylethyl | Me | 4-Thiazolyl | H |
| 3-509 | Allyl | Me | 4-Thiazolyl | H |
| 3-510 | 3-Methylbut-2-en-1-yl | Me | 4-Thiazolyl | H |
| 3-511 | Cyclobutylmethyl | Me | 4-Thiazolyl | H |
| 3-512 | Cyclopentylmethyl | Me | 4-Thiazolyl | H |
| 3-513 | 2-Chloroprop-2-en-1-yl | Me | 4-Thiazolyl | H |
| 3-514 | Tetrahydrofuran-2-yl-methyl | Me | 4-Thiazolyl | H |
| 3-515 | (3-Methyloxetan-3-yl)-methyl | Me | 4-Thiazolyl | H |
| 3-516 | 2,2,2-Trifluorethyl | Me | 4-Thiazolyl | H |
| 3-517 | 2,2-Difluorethyl | Me | 4-Thiazolyl | H |
| 3-518 | Oxetan-3-yl | Me | 4-Thiazolyl | H |
| 3-519 | Cyclopropylmethyl | Me | 3-Br-Ph | H |
| 3-520 | Prop-2-in-1-yl | Me | 3-Br-Ph | H |
| 3-521 | But-2-in-1-yl | Me | 3-Br-Ph | H |
| 3-522 | But-3-in-2-yl | Me | 3-Br-Ph | H |
| 3-523 | Pr | Me | 3-Br-Ph | H |
| 3-524 | iPr | Me | 3-Br-Ph | H |
| 3-525 | CH₂Ph | Me | 3-Br-Ph | H |
| 3-526 | 3,3-Dichlor-2-fluorprop-2-en-1-yl | Me | 3-Br-Ph | H |
| 3-527 | (1-Methylcyclopropyl)-methyl | Me | 3-Br-Ph | H |
| 3-528 | 4-Chlorbut-2-in-1-yl | Me | 3-Br-Ph | H |
| 3-529 | (2,2-Dichlorcyclopropyl)-methyl | Me | 3-Br-Ph | H |
| 3-530 | 1-Methylprop-2-in-1-yl | Me | 3-Br-Ph | H |
| 3-531 | 1-Cyclopropylethyl | Me | 3-Br-Ph | H |
| 3-532 | Allyl | Me | 3-Br-Ph | H |
| 3-533 | 3-Methylbut-2-en-1-yl | Me | 3-Br-Ph | H |
| 3-534 | Cyclobutylmethyl | Me | 3-Br-Ph | H |
| 3-535 | Cyclopentylmethyl | Me | 3-Br-Ph | H |
| 3-536 | 2-Chloroprop-2-en-1-yl | Me | 3-Br-Ph | H |
| 3-537 | Tetrahydrofuran-2-yl-methyl | Me | 3-Br-Ph | H |
| 3-538 | (3-Methyloxetan-3-yl)-methyl | Me | 3-Br-Ph | H |
| 3-539 | 2,2,2-Trifluorethyl | Me | 3-Br-Ph | H |
| 3-540 | 2,2-Difluorethyl | Me | 3-Br-Ph | H |
| 3-541 | Oxetan-3-yl | Me | 3-Br-Ph | H |
| 3-542 | Cyclopropylmethyl | Me | 2-Cl-thiazol-4-yl | H |
| 3-543 | Prop-2-in-1-yl | Me | 2-Cl-thiazol-4-yl | H |
| 3-544 | But-2-in-1-yl | Me | 2-Cl-thiazol-4-yl | H |
| 3-545 | But-3-in-2-yl | Me | 2-Cl-thiazol-4-yl | H |
| 3-546 | Pr | Me | 2-Cl-thiazol-4-yl | H |
| 3-547 | iPr | Me | 2-Cl-thiazol-4-yl | H |
| 3-548 | CH₂Ph | Me | 2-Cl-thiazol-4-yl | H |
| 3-549 | 3,3-Dichlor-2-fluorprop-2-en-1-yl | Me | 2-Cl-thiazol-4-yl | H |
| 3-550 | (1-Methylcyclopropyl)-methyl | Me | 2-Cl-thiazol-4-yl | H |
| 3-551 | 4-Chlorbut-2-in-1-yl | Me | 2-Cl-thiazol-4-yl | H |
| 3-552 | (2,2-Dichlorcyclopropyl)-methyl | Me | 2-Cl-thiazol-4-yl | H |
| 3-553 | 1-Methylprop-2-in-1-yl | Me | 2-Cl-thiazol-4-yl | H |
| 3-554 | 1-Cyclopropylethyl | Me | 2-Cl-thiazol-4-yl | H |
| 3-555 | Allyl | Me | 2-Cl-thiazol-4-yl | H |
| 3-556 | 3-Methylbut-2-en-1-yl | Me | 2-Cl-thiazol-4-yl | H |
| 3-557 | Cyclobutylmethyl | Me | 2-Cl-thiazol-4-yl | H |
| 3-558 | Cyclopentylmethyl | Me | 2-Cl-thiazol-4-yl | H |
| 3-559 | 2-Chloroprop-2-en-1-yl | Me | 2-Cl-thiazol-4-yl | H |
| 3-560 | Tetrahydrofuran-2-yl-methyl | Me | 2-Cl-thiazol-4-yl | H |
| 3-561 | (3-Methyloxetan-3-yl)-methyl | Me | 2-Cl-thiazol-4-yl | H |
| 3-562 | 2,2,2-Trifluorethyl | Me | 2-Cl-thiazol-4-yl | H |
| 3-563 | 2,2-Difluorethyl | Me | 2-Cl-thiazol-4-yl | H |
| 3-564 | Oxetan-3-yl | Me | 2-Cl-thiazol-4-yl | H |
| 3-565 | Cyclopropylmethyl | Me | 2-Br-thiazol-4-yl | H |
| 3-566 | Prop-2-in-1-yl | Me | 2-Br-thiazol-4-yl | H |
| 3-567 | But-2-in-1-yl | Me | 2-Br-thiazol-4-yl | H |
| 3-568 | But-3-in-2-yl | Me | 2-Br-thiazol-4-yl | H |
| 3-569 | Pr | Me | 2-Br-thiazol-4-yl | H |
| 3-570 | iPr | Me | 2-Br-thiazol-4-yl | H |
| 3-571 | CH₂Ph | Me | 2-Br-thiazol-4-yl | H |
| 3-572 | 3,3-Dichlor-2-fluorprop-2-en-1-yl | Me | 2-Br-thiazol-4-yl | H |
| 3-573 | (1-Methylcyclopropyl)-methyl | Me | 2-Br-thiazol-4-yl | H |
| 3-574 | 4-Chlorbut-2-in-1-yl | Me | 2-Br-thiazol-4-yl | H |
| 3-575 | (2,2-Dichlorcyclopropyl)-methyl | Me | 2-Br-thiazol-4-yl | H |
| 3-576 | 1-Methylprop-2-in-1-yl | Me | 2-Br-thiazol-4-yl | H |
| 3-577 | 1-Cyclopropylethyl | Me | 2-Br-thiazol-4-yl | H |
| 3-578 | Allyl | Me | 2-Br-thiazol-4-yl | H |
| 3-579 | 3-Methylbut-2-en-1-yl | Me | 2-Br-thiazol-4-yl | H |
| 3-580 | Cyclobutylmethyl | Me | 2-Br-thiazol-4-yl | H |
| 3-581 | Cyclopentylmethyl | Me | 2-Br-thiazol-4-yl | H |
| 3-582 | 2-Chloroprop-2-en-1-yl | Me | 2-Br-thiazol-4-yl | H |
| 3-583 | Tetrahydrofuran-2-yl-methyl | Me | 2-Br-thiazol-4-yl | H |
| 3-584 | (3-Methyloxetan-3-yl)-methyl | Me | 2-Br-thiazol-4-yl | H |
| 3-585 | 2,2,2-Trifluorethyl | Me | 2-Br-thiazol-4-yl | H |
| 3-586 | 2,2-Difluorethyl | Me | 2-Br-thiazol-4-yl | H |
| 3-587 | Oxetan-3-yl | Me | 2-Br-thiazol-4-yl | H |
| 3-588 | Cyclopropylmethyl | Me | 5-Br-thiazol-2-yl | H |
| 3-589 | Prop-2-in-1-yl | Me | 5-Br-thiazol-2-yl | H |
| 3-590 | But-2-in-1-yl | Me | 5-Br-thiazol-2-yl | H |
| 3-591 | But-3-in-2-yl | Me | 5-Br-thiazol-2-yl | H |
| 3-592 | Pr | Me | 5-Br-thiazol-2-yl | H |
| 3-593 | iPr | Me | 5-Br-thiazol-2-yl | H |
| 3-594 | CH₂Ph | Me | 5-Br-thiazol-2-yl | H |
| 3-595 | 3,3-Dichlor-2-fluorprop-2-en-1-yl | Me | 5-Br-thiazol-2-yl | H |
| 3-596 | (1-Methylcyclopropyl)-methyl | Me | 5-Br-thiazol-2-yl | H |
| 3-597 | 4-Chlorbut-2-in-1-yl | Me | 5-Br-thiazol-2-yl | H |
| 3-598 | (2,2-Dichlorcyclopropyl)-methyl | Me | 5-Br-thiazol-2-yl | H |
| 3-599 | 1-Methylprop-2-in-1-yl | Me | 5-Br-thiazol-2-yl | H |
| 3-600 | 1-Cyclopropylethyl | Me | 5-Br-thiazol-2-yl | H |
| 3-601 | Allyl | Me | 5-Br-thiazol-2-yl | H |
| 3-602 | 3-Methylbut-2-en-1-yl | Me | 5-Br-thiazol-2-yl | H |
| 3-603 | Cyclobutylmethyl | Me | 5-Br-thiazol-2-yl | H |
| 3-604 | Cyclopentylmethyl | Me | 5-Br-thiazol-2-yl | H |
| 3-605 | 2-Chloroprop-2-en-1-yl | Me | 5-Br-thiazol-2-yl | H |
| 3-606 | Tetrahydrofuran-2-yl-methyl | Me | 5-Br-thiazol-2-yl | H |
| 3-607 | (3-Methyloxetan-3-yl)-methyl | Me | 5-Br-thiazol-2-yl | H |
| 3-608 | 2,2,2-Trifluorethyl | Me | 5-Br-thiazol-2-yl | H |
| 3-609 | 2,2-Difluorethyl | Me | 5-Br-thiazol-2-yl | H |
| 3-610 | Oxetan-3-yl | Me | 5-Br-thiazol-2-yl | H |
| 3-611 | Cyclopropylmethyl | Me | 5-Cl-thiazol-2-yl | H |
| 3-612 | Prop-2-in-1-yl | Me | 5-Cl-thiazol-2-yl | H |
| 3-613 | But-2-in-1-yl | Me | 5-Cl-thiazol-2-yl | H |
| 3-614 | But-3-in-2-yl | Me | 5-Cl-thiazol-2-yl | H |
| 3-615 | Pr | Me | 5-Cl-thiazol-2-yl | H |
| 3-616 | iPr | Me | 5-Cl-thiazol-2-yl | H |
| 3-617 | CH₂Ph | Me | 5-Cl-thiazol-2-yl | H |
| 3-618 | 3,3-Dichlor-2-fluorprop-2-en-1-yl | Me | 5-Cl-thiazol-2-yl | H |
| 3-619 | (1-Methylcyclopropyl)-methyl | Me | 5-Cl-thiazol-2-yl | H |
| 3-620 | 4-Chlorbut-2-in-1-yl | Me | 5-Cl-thiazol-2-yl | H |
| 3-621 | (2,2-Dichlorcyclopropyl)-methyl | Me | 5-Cl-thiazol-2-yl | H |
| 3-622 | 1-Methylprop-2-in-1-yl | Me | 5-Cl-thiazol-2-yl | H |
| 3-623 | 1-Cyclopropylethyl | Me | 5-Cl-thiazol-2-yl | H |
| 3-624 | Allyl | Me | 5-Cl-thiazol-2-yl | H |
| 3-625 | 3-Methylbut-2-en-1-yl | Me | 5-Cl-thiazol-2-yl | H |
| 3-626 | Cyclobutylmethyl | Me | 5-Cl-thiazol-2-yl | H |
| 3-627 | Cyclopentylmethyl | Me | 5-Cl-thiazol-2-yl | H |
| 3-628 | 2-Chloroprop-2-en-1-yl | Me | 5-Cl-thiazol-2-yl | H |
| 3-629 | Tetrahydrofuran-2-yl-methyl | Me | 5-Cl-thiazol-2-yl | H |
| 3-630 | (3-Methyloxetan-3-yl)-methyl | Me | 5-Cl-thiazol-2-yl | H |
| 3-631 | 2,2,2-Trifluorethyl | Me | 5-Cl-thiazol-2-yl | H |
| 3-632 | 2,2-Difluorethyl | Me | 5-Cl-thiazol-2-yl | H |
| 3-633 | Oxetan-3-yl | Me | 5-Cl-thiazol-2-yl | H |
| 3-634 | Cyclopropylmethyl | Me | 5-OSO₂Me-pyridin-2-yl | H |
| 3-635 | Prop-2-in-1-yl | Me | 5-OSO₂Me-pyridin-2-yl | H |
| 3-636 | But-3-in-2-yl | Me | 5-OSO₂Me-pyridin-2-yl | H |
| 3-637 | iPr | Me | 5-OSO₂Me-pyridin-2-yl | H |
| 3-638 | CH₂Ph | Me | 5-OSO₂Me-pyridin-2-yl | H |
| 3-639 | (2,2-Dichlorcyclopropyl)-methyl | Me | 5-OSO₂Me-pyridin-2-yl | H |
| 3-640 | Allyl | Me | 5-OSO₂Me-pyridin-2-yl | H |
| 3-641 | 2,2,2-Trifluorethyl | Me | 5-OSO₂Me-pyridin-2-yl | H |
| 3-642 | 2,2-Difluorethyl | Me | 5-OSO₂Me-pyridin-2-yl | H |
| 3-643 | Oxetan-3-yl | Me | 5-OSO₂Me-pyridin-2-yl | H |
| 3-644 | Cyclopropylmethyl | Me | 6-Cl-1,3-benzothiazol-2-yl | H |
| 3-645 | Prop-2-in-1-yl | Me | 6-Cl-1,3-benzothiazol-2-yl | H |
| 3-646 | But-3-in-2-yl | Me | 6-Cl-1,3-benzothiazol-2-yl | H |
| 3-647 | iPr | Me | 6-Cl-1,3-benzothiazol-2-yl | H |
| 3-648 | CH₂Ph | Me | 6-Cl-1,3-benzothiazol-2-yl | H |
| 3-649 | (2,2-Dichlorcyclopropyl)-methyl | Me | 6-Cl-1,3-benzothiazol-2-yl | H |
| 3-650 | Allyl | Me | 6-Cl-1,3-benzothiazol-2-yl | H |
| 3-651 | 2,2,2-Trifluorethyl | Me | 6-Cl-1,3-benzothiazol-2-yl | H |
| 3-652 | 2,2-Difluorethyl | Me | 6-Cl-1,3-benzothiazol-2-yl | H |
| 3-653 | Oxetan-3-yl | Me | 6-Cl-1,3-benzothiazol-2-yl | H |
| 3-654 | Cyclopropylmethyl | Me | 6-Br-1,3-benzothiazol-2-yl | H |
| 3-655 | Prop-2-in-1-yl | Me | 6-Br-1,3-benzothiazol-2-yl | H |
| 3-656 | But-3-in-2-yl | Me | 6-Br-1,3-benzothiazol-2-yl | H |
| 3-657 | iPr | Me | 6-Br-1,3-benzothiazol-2-yl | H |
| 3-658 | CH₂Ph | Me | 6-Br-1,3-benzothiazol-2-yl | H |
| 3-659 | (2,2-Dichlorcyclopropyl)-methyl | Me | 6-Br-1,3-benzothiazol-2-yl | H |
| 3-660 | Allyl | Me | 6-Br-1,3-benzothiazol-2-yl | H |
| 3-661 | 2,2,2-Trifluorethyl | Me | 6-Br-1,3-benzothiazol-2-yl | H |
| 3-662 | 2,2-Difluorethyl | Me | 6-Br-1,3-benzothiazol-2-yl | H |
| 3-663 | Oxetan-3-yl | Me | 6-Br-1,3-benzothiazol-2-yl | H |
| 3-664 | Cyclopropylmethyl | Me | 1,3-Benzoxazol-2-yl | H |
| 3-665 | Prop-2-in-1-yl | Me | 1,3-Benzoxazol-2-yl | H |
| 3-666 | But-3-in-2-yl | Me | 1,3-Benzoxazol-2-yl | H |
| 3-667 | iPr | Me | 1,3-Benzoxazol-2-yl | H |
| 3-668 | CH₂Ph | Me | 1,3-Benzoxazol-2-yl | H |
| 3-669 | (2,2-Dichlorcyclopropyl)-methyl | Me | 1,3-Benzoxazol-2-yl | H |
| 3-670 | Allyl | Me | 1,3-Benzoxazol-2-yl | H |
| 3-671 | 2,2,2-Trifluorethyl | Me | 1,3-Benzoxazol-2-yl | H |
| 3-672 | 2,2-Difluorethyl | Me | 1,3-Benzoxazol-2-yl | H |
| 3-673 | Oxetan-3-yl | Me | 1,3-Benzoxazol-2-yl | H |
| 3-674 | Cyclopropylmethyl | Me | 6-Cl-1,3-benzoxazol-2-yl | H |
| 3-675 | Prop-2-in-1-yl | Me | 6-Cl-1,3-benzoxazol-2-yl | H |
| 3-676 | But-3-in-2-yl | Me | 6-Cl-1,3-benzoxazol-2-yl | H |
| 3-677 | iPr | Me | 6-Cl-1,3-benzoxazol-2-yl | H |
| 3-678 | CH₂Ph | Me | 6-Cl-1,3-benzoxazol-2-yl | H |
| 3-679 | (2,2-Dichlorcyclopropyl)-methyl | Me | 6-Cl-1,3-benzoxazol-2-yl | H |
| 3-680 | Allyl | Me | 6-Cl-1,3-benzoxazol-2-yl | H |
| 3-681 | 2,2,2-Trifluorethyl | Me | 6-Cl-1,3-benzoxazol-2-yl | H |
| 3-682 | 2,2-Difluorethyl | Me | 6-Cl-1,3-benzoxazol-2-yl | H |
| 3-683 | Oxetan-3-yl | Me | 6-Cl-1,3-benzoxazol-2-yl | H |
| 3-684 | Cyclopropylmethyl | Me | 6-Br-1,3-benzoxazol-2-yl | H |
| 3-685 | Prop-2-in-1-yl | Me | 6-Br-1,3-benzoxazol-2-yl | H |
| 3-686 | But-3-in-2-yl | Me | 6-Br-1,3-benzoxazol-2-yl | H |
| 3-687 | iPr | Me | 6-Br-1,3-benzoxazol-2-yl | H |
| 3-688 | CH₂Ph | Me | 6-Br-1,3-benzoxazol-2-yl | H |
| 3-689 | (2,2-Dichlorcyclopropyl)-methyl | Me | 6-Br-1,3-benzoxazol-2-yl | H |
| 3-690 | Allyl | Me | 6-Br-1,3-benzoxazol-2-yl | H |
| 3-691 | 2,2,2-Trifluorethyl | Me | 6-Br-1,3-benzoxazol-2-yl | H |
| 3-692 | 2,2-Difluorethyl | Me | 6-Br-1,3-benzoxazol-2-yl | H |
| 3-693 | Oxetan-3-yl | Me | 6-Br-1,3-benzoxazol-2-yl | H |
| 3-694 | Cyclopropylmethyl | Me | 7-Cl-1,3-benzoxazol-2-yl | H |
| 3-695 | Prop-2-in-1-yl | Me | 7-Cl-1,3-benzoxazol-2-yl | H |
| 3-696 | But-3-in-2-yl | Me | 7-Cl-1,3-benzoxazol-2-yl | H |
| 3-697 | iPr | Me | 7-Cl-1,3-benzoxazol-2-yl | H |
| 3-698 | CH₂Ph | Me | 7-Cl-1,3-benzoxazol-2-yl | H |
| 3-699 | (2,2-Dichlorcyclopropyl)-methyl | Me | 7-Cl-1,3-benzoxazol-2-yl | H |
| 3-700 | Allyl | Me | 7-Cl-1,3-benzoxazol-2-yl | H |
| 3-701 | 2,2,2-Trifluorethyl | Me | 7-Cl-1,3-benzoxazol-2-yl | H |
| 3-702 | 2,2-Difluorethyl | Me | 7-Cl-1,3-benzoxazol-2-yl | H |
| 3-703 | Oxetan-3-yl | Me | 7-Cl-1,3-benzoxazol-2-yl | H |
| 3-704 | Cyclopropylmethyl | Me | 5-I-pyridin-2-yl | H |
| 3-705 | Prop-2-in-1-yl | Me | 5-I-pyridin-2-yl | H |
| 3-706 | But-3-in-2-yl | Me | 5-I-pyridin-2-yl | H |
| 3-707 | iPr | Me | 5-I-pyridin-2-yl | H |
| 3-708 | CH₂Ph | Me | 5-I-pyridin-2-yl | H |
| 3-709 | (2,2-Dichlorcyclopropyl)-methyl | Me | 5-I-pyridin-2-yl | H |
| 3-710 | Allyl | Me | 5-I-pyridin-2-yl | H |
| 3-711 | 2,2,2-Trifluorethyl | Me | 5-I-pyridin-2-yl | H |
| 3-712 | 2,2-Difluorethyl | Me | 5-I-pyridin-2-yl | H |
| 3-713 | Oxetan-3-yl | Me | 5-I-pyridin-2-yl | H |
| 3-714 | Cyclopropylmethyl | Me | 5-NH₂-pyridin-2-yl | H |
| 3-715 | Prop-2-in-1-yl | Me | 5-NH₂-pyridin-2-yl | H |
| 3-716 | But-3-in-2-yl | Me | 5-NH₂-pyridin-2-yl | H |
| 3-717 | iPr | Me | 5-NH₂-pyridin-2-yl | H |
| 3-718 | CH₂Ph | Me | 5-NH₂-pyridin-2-yl | H |
| 3-719 | (2,2-Dichlorcyclopropyl)-methyl | Me | 5-NH₂-pyridin-2-yl | H |
| 3-720 | Allyl | Me | 5-NH₂-pyridin-2-yl | H |
| 3-721 | 2,2,2-Trifluorethyl | Me | 5-NH₂-pyridin-2-yl | H |
| 3-722 | 2,2-Difluorethyl | Me | 5-NH₂-pyridin-2-yl | H |
| 3-723 | Oxetan-3-yl | Me | 5-NH₂-pyridin-2-yl | H |
| 3-724 | Cyclopropylmethyl | Me | 5-OH-pyridin-2-yl | H |
| 3-725 | Prop-2-in-1-yl | Me | 5-OH-pyridin-2-yl | H |
| 3-726 | But-3-in-2-yl | Me | 5-OH-pyridin-2-yl | H |
| 3-727 | iPr | Me | 5-OH-pyridin-2-yl | H |
| 3-728 | CH₂Ph | Me | 5-OH-pyridin-2-yl | H |
| 3-729 | (2,2-Dichlorcyclopropyl)-methyl | Me | 5-OH-pyridin-2-yl | H |
| 3-730 | Allyl | Me | 5-OH-pyridin-2-yl | H |
| 3-731 | 2,2,2-Trifluorethyl | Me | 5-OH-pyridin-2-yl | H |
| 3-732 | 2,2-Difluorethyl | Me | 5-OH-pyridin-2-yl | H |
| 3-733 | Oxetan-3-yl | Me | 5-OH-pyridin-2-yl | H |
| 3-734 | Cyclopropylmethyl | Me | 5-OCHF₂-pyridin-2-yl | H |
| 3-735 | Prop-2-in-1-yl | Me | 5-OCHF₂-pyridin-2-yl | H |
| 3-736 | But-3-in-2-yl | Me | 5-OCHF₂-pyridin-2-yl | H |
| 3-737 | iPr | Me | 5-OCHF₂-pyridin-2-yl | H |
| 3-738 | CH₂Ph | Me | 5-OCHF₂-pyridin-2-yl | H |
| 3-739 | (2,2-Dichlorcyclopropyl)-methyl | Me | 5-OCHF₂-pyridin-2-yl | H |
| 3-740 | Allyl | Me | 5-OCHF₂-pyridin-2-yl | H |
| 3-741 | 2,2,2-Trifluorethyl | Me | 5-OCHF₂-pyridin-2-yl | H |
| 3-742 | 2,2-Difluorethyl | Me | 5-OCHF₂-pyridin-2-yl | H |
| 3-743 | Oxetan-3-yl | Me | 5-OCHF₂-pyridin-2-yl | H |
| 3-744 | Cyclopropylmethyl | Me | 5-MeO-pyridin-2-yl | H |
| 3-745 | Prop-2-in-1-yl | Me | 5-MeO-pyridin-2-yl | H |
| 3-746 | But-3-in-2-yl | Me | 5-MeO-pyridin-2-yl | H |
| 3-747 | iPr | Me | 5-MeO-pyridin-2-yl | H |
| 3-748 | CH₂Ph | Me | 5-MeO-pyridin-2-yl | H |
| 3-749 | (2,2-Dichlorcyclopropyl)-methyl | Me | 5-MeO-pyridin-2-yl | H |
| 3-750 | Allyl | Me | 5-MeO-pyridin-2-yl | H |
| 3-751 | 2,2,2-Trifluorethyl | Me | 5-MeO-pyridin-2-yl | H |
| 3-752 | 2,2-Difluorethyl | Me | 5-MeO-pyridin-2-yl | H |
| 3-753 | Oxetan-3-yl | Me | 5-MeO-pyridin-2-yl | H |
| 3-754 | Cyclopropylmethyl | Me | 5-MeS-pyridin-2-yl | H |
| 3-755 | Prop-2-in-1-yl | Me | 5-MeS-pyridin-2-yl | H |
| 3-756 | But-3-in-2-yl | Me | 5-MeS-pyridin-2-yl | H |
| 3-757 | iPr | Me | 5-MeS-pyridin-2-yl | H |
| 3-758 | CH₂Ph | Me | 5-MeS-pyridin-2-yl | H |
| 3-759 | (2,2-Dichlorcyclopropyl)-methyl | Me | 5-MeS-pyridin-2-yl | H |
| 3-760 | Allyl | Me | 5-MeS-pyridin-2-yl | H |
| 3-761 | 2,2,2-Trifluorethyl | Me | 5-MeS-pyridin-2-yl | H |
| 3-762 | 2,2-Difluorethyl | Me | 5-MeS-pyridin-2-yl | H |
| 3-763 | Oxetan-3-yl | Me | 5-MeS-pyridin-2-yl | H |
| 3-764 | Cyclopropylmethyl | Me | 5-NHMe-pyridin-2-yl | H |
| 3-765 | Prop-2-in-1-yl | Me | 5-NHMe-pyridin-2-yl | H |
| 3-766 | But-3-in-2-yl | Me | 5-NHMe-pyridin-2-yl | H |
| 3-767 | iPr | Me | 5-NHMe-pyridin-2-yl | H |
| 3-768 | CH₂Ph | Me | 5-NHMe-pyridin-2-yl | H |
| 3-769 | (2,2-Dichlorcyclopropyl)-methyl | Me | 5-NHMe-pyridin-2-yl | H |
| 3-770 | Allyl | Me | 5-NHMe-pyridin-2-yl | H |
| 3-771 | 2,2,2-Trifluorethyl | Me | 5-NHMe-pyridin-2-yl | H |
| 3-772 | 2,2-Difluorethyl | Me | 5-NHMe-pyridin-2-yl | H |
| 3-773 | Oxetan-3-yl | Me | 5-NHMe-pyridin-2-yl | H |
| 3-774 | Cyclopropylmethyl | Me | 5-NMe₂-pyridin-2-yl | H |
| 3-775 | Prop-2-in-1-yl | Me | 5-NMe₂-pyridin-2-yl | H |
| 3-776 | But-3-in-2-yl | Me | 5-NMe₂-pyridin-2-yl | H |
| 3-777 | iPr | Me | 5-NMe₂-pyridin-2-yl | H |
| 3-778 | CH₂Ph | Me | 5-NMe₂-pyridin-2-yl | H |
| 3-779 | (2,2-Dichlorcyclopropyl)-methyl | Me | 5-NMe₂-pyridin-2-yl | H |
| 3-780 | Allyl | Me | 5-NMe₂-pyridin-2-yl | H |
| 3-781 | 2,2,2-Trifluorethyl | Me | 5-NMe₂-pyridin-2-yl | H |
| 3-782 | 2,2-Difluorethyl | Me | 5-NMe₂-pyridin-2-yl | H |
| 3-783 | Oxetan-3-yl | Me | 5-NMe₂-pyridin-2-yl | H |
| 3-784 | 3-Hydroxy-but-2-yl | Me | 4-Cl-Ph | H |
| 3-785 | 3-Hydroxy-but-2-yl | Me | 4-Br-Ph | H |
| 3-786 | 3-Hydroxy-but-2-yl | Me | 5-Cl-pyridin-2-yl | H |
| 3-787 | 3-Hydroxy-but-2-yl | Me | 5-Br-pyridin-2-yl | H |
| 3-788 | 3-Hydroxy-but-2-yl | Me | 5-Cl-2-thienyl | H |
| 3-789 | 3-Hydroxy-but-2-yl | Me | 5-Br-2-thienyl | H |
| 3-790 | 3-Ethylpent-1-in-3-yl | Me | 4-Cl-Ph | H |
| 3-791 | 3-Ethylpent-1-in-3-yl | Me | 4-Br-Ph | H |
| 3-792 | 3-Ethylpent-1-in-3-yl | Me | 5-Cl-pyridin-2-yl | H |
| 3-793 | 3-Ethylpent-1-in-3-yl | Me | 5-Br-pyridin-2-yl | H |
| 3-794 | 3-Ethylpent-1-in-3-yl | Me | 5-Cl-2-thienyl | H |
| 3-795 | 3-Ethylpent-1-in-3-yl | Me | 5-Br-2-thienyl | H |
| 3-796 | Difluormethyl | Me | 4-Cl-Ph | H |
| 3-797 | Difluormethyl | Me | 4-Br-Ph | H |
| 3-798 | Difluormethyl | Me | 5-Cl-pyridin-2-yl | H |
| 3-799 | Difluormethyl | Me | 5-Br-pyridin-2-yl | H |
| 3-800 | Difluormethyl | Me | 5-Cl-2-thienyl | H |
| 3-801 | Difluormethyl | Me | 5-Br-2-thienyl | H |
| 3-802 | 2,2,3,3-Tetrafluorpropyl | Me | 4-Cl-Ph | H |
| 3-803 | 2,2,3,3-Tetrafluorpropyl | Me | 4-Br-Ph | H |
| 3-804 | 2,2,3,3-Tetrafluorpropyl | Me | 5-Cl-pyridin-2-yl | H |
| 3-805 | 2,2,3,3-Tetrafluorpropyl | Me | 5-Br-pyridin-2-yl | H |
| 3-806 | 2,2,3,3-Tetrafluorpropyl | Me | 5-Cl-2-thienyl | H |
| 3-807 | 2,2,3,3-Tetrafluorpropyl | Me | 5-Br-2-thienyl | H |
| 3-808 | 4,4,4-Trifluorbutyl | Me | 4-Cl-Ph | H |
| 3-809 | 4,4,4-Trifluorbutyl | Me | 4-Br-Ph | H |
| 3-810 | 4,4,4-Trifluorbutyl | Me | 5-Cl-pyridin-2-yl | H |
| 3-811 | 4,4,4-Trifluorbutyl | Me | 5-Br-pyridin-2-yl | H |
| 3-812 | 4,4,4-Trifluorbutyl | Me | 5-Cl-2-thienyl | H |
| 3-813 | 4,4,4-Trifluorbutyl | Me | 5-Br-2-thienyl | H |
| 3-814 | Acetoxy-methyl | Me | 4-Cl-Ph | H |
| 3-815 | Acetoxy-methyl | Me | 4-Br-Ph | H |
| 3-816 | Acetoxy-methyl | Me | 5-Cl-pyridin-2-yl | H |
| 3-817 | Acetoxy-methyl | Me | 5-Br-pyridin-2-yl | H |
| 3-818 | Acetoxy-methyl | Me | 5-Cl-2-thienyl | H |
| 3-819 | Acetoxy-methyl | Me | 5-Br-2-thienyl | H |
| 3-820 | 2-Chlorethyl | Me | 4-Cl-Ph | H |
| 3-821 | 2-Chlorethyl | Me | 4-Br-Ph | H |
| 3-822 | 2-Chlorethyl | Me | 5-Cl-pyridin-2-yl | H |
| 3-823 | 2-Chlorethyl | Me | 5-Br-pyridin-2-yl | H |
| 3-824 | 2-Chlorethyl | Me | 5-Cl-2-thienyl | H |
| 3-825 | 2-Chlorethyl | Me | 5-Br-2-thienyl | H |
| 3-826 | 3-Fluorpropyl | Me | 4-Cl-Ph | H |
| 3-827 | 3-Fluorpropyl | Me | 4-Br-Ph | H |
| 3-828 | 3-Fluorpropyl | Me | 5-Cl-pyridin-2-yl | H |
| 3-829 | 3-Fluorpropyl | Me | 5-Br-pyridin-2-yl | H |
| 3-830 | 3-Fluorpropyl | Me | 5-Cl-2-thienyl | H |
| 3-831 | 3-Fluorpropyl | Me | 5-Br-2-thienyl | H |
| 3-832 | 2-Ethoxyethyl | Me | 4-Cl-Ph | H |
| 3-833 | 2-Ethoxyethyl | Me | 4-Br-Ph | H |
| 3-834 | 2-Ethoxyethyl | Me | 5-Cl-pyridin-2-yl | H |
| 3-835 | 2-Ethoxyethyl | Me | 5-Br-pyridin-2-yl | H |
| 3-836 | 2-Ethoxyethyl | Me | 5-Cl-2-thienyl | H |
| 3-837 | 2-Ethoxyethyl | Me | 5-Br-2-thienyl | H |
| 3-838 | 2-Propan-1-ol | Me | 4-Cl-Ph | H |
| 3-839 | 2-Propan-1-ol | Me | 4-Br-Ph | H |
| 3-840 | 1-Hydroxy-prop-2-yl | Me | 5-Cl-pyridin-2-yl | H |
| 3-841 | 1-Hydroxy-prop-2-yl | Me | 5-Br-pyridin-2-yl | H |
| 3-842 | 1-Hydroxy-prop-2-yl | Me | 5-Cl-2-thienyl | H |
| 3-843 | 1-Hydroxy-prop-2-yl | Me | 5-Br-2-thienyl | H |
| 3-844 | 2-Methoxybut-1-yl | Me | 4-Cl-Ph | H |
| 3-845 | 2-Methoxybut-1-yl | Me | 4-Br-Ph | H |
| 3-846 | 2-Methoxybut-1-yl | Me | 5-Cl-pyridin-2-yl | H |
| 3-847 | 2-Methoxybut-1-yl | Me | 5-Br-pyridin-2-yl | H |
| 3-848 | 2-Methoxybut-1-yl | Me | 5-Cl-2-thienyl | H |
| 3-849 | 2-Methoxybut-1-yl | Me | 5-Br-2-thienyl | H |
| 3-850 | 1,3-Difluorpropan-2-yl | Me | 4-Cl-Ph | H |
| 3-851 | 1,3-Difluorpropan-2-yl | Me | 4-Br-Ph | H |
| 3-852 | 1,3-Difluorpropan-2-yl | Me | 5-Cl-pyridin-2-yl | H |
| 3-853 | 1,3-Difluorpropan-2-yl | Me | 5-Br-pyridin-2-yl | H |
| 3-854 | 1,3-Difluorpropan-2-yl | Me | 5-Cl-2-thienyl | H |
| 3-855 | 1,3-Difluorpropan-2-yl | Me | 5-Br-2-thienyl | H |
| 3-856 | 2,3-Dimethoxypropyl | Me | 4-Cl-Ph | H |
| 3-857 | 2,3-Dimethoxypropyl | Me | 4-Br-Ph | H |
| 3-858 | 2,3-Dimethoxypropyl | Me | 5-Cl-pyridin-2-yl | H |
| 3-859 | 2,3-Dimethoxypropyl | Me | 5-Br-pyridin-2-yl | H |
| 3-860 | 2,3-Dimethoxypropyl | Me | 5-Cl-2-thienyl | H |
| 3-861 | 2,3-Dimethoxypropyl | Me | 5-Br-2-thienyl | H |
| 3-862 | 1,3-dioxolan-4-yl-methyl | Me | 4-Cl-Ph | H |
| 3-863 | 1,3-dioxolan-4-yl-methyl | Me | 4-Br-Ph | H |
| 3-864 | 1,3-dioxolan-4-yl-methyl | Me | 5-Cl-pyridin-2-yl | H |
| 3-865 | 1,3-dioxolan-4-yl-methyl | Me | 5-Br-pyridin-2-yl | H |
| 3-866 | 1,3-dioxolan-4-yl-methyl | Me | 5-Cl-2-thienyl | H |
| 3-867 | 1,3-dioxolan-4-yl-methyl | Me | 5-Br-2-thienyl | H |
| 3-868 | 1,1,1,4,4,4-Hexafluorbutan-2-yl | Me | 4-Cl-Ph | H |
| 3-869 | 1,1,1,4,4,4-Hexafluorbutan-2-yl | Me | 4-Br-Ph | H |
| 3-870 | 1,1,1,4,4,4-Hexafluorbutan-2-yl | Me | 5-Cl-pyridin-2-yl | H |
| 3-871 | 1,1,1,4,4,4-Hexafluorbutan-2-yl | Me | 5-Br-pyridin-2-yl | H |
| 3-872 | 1,1,1,4,4,4-Hexafluorbutan-2-yl | Me | 5-Cl-2-thienyl | H |
| 3-873 | 1,1,1,4,4,4-Hexafluorbutan-2-yl | Me | 5-Br-2-thienyl | H |
| 3-874 | 1,1-Difluorpropan-2-yl | Me | 4-Cl-Ph | H |
| 3-875 | 1,1-Difluorpropan-2-yl | Me | 4-Br-Ph | H |
| 3-876 | 1,1-Difluorpropan-2-yl | Me | 5-Cl-pyridin-2-yl | H |
| 3-877 | 1,1-Difluorpropan-2-yl | Me | 5-Br-pyridin-2-yl | H |
| 3-878 | 1,1-Difluorpropan-2-yl | Me | 5-Cl-2-thienyl | H |
| 3-879 | 1,1-Difluorpropan-2-yl | Me | 5-Br-2-thienyl | H |
| 3-880 | 1-Fluorpropan-2-yl | Me | 4-Cl-Ph | H |
| 3-881 | 1-Fluorpropan-2-yl | Me | 4-Br-Ph | H |
| 3-882 | 1-Fluorpropan-2-yl | Me | 5-Cl-pyridin-2-yl | H |
| 3-883 | 1-Fluorpropan-2-yl | Me | 5-Br-pyridin-2-yl | H |
| 3-884 | 1-Fluorpropan-2-yl | Me | 5-Cl-2-thienyl | H |
| 3-885 | 1-Fluorpropan-2-yl | Me | 5-Br-2-thienyl | H |
| 3-886 | 1-Brompropan-2-yl | Me | 4-Cl-Ph | H |
| 3-887 | 1-Brompropan-2-yl | Me | 4-Br-Ph | H |
| 3-888 | 1-Brompropan-2-yl | Me | 5-Cl-pyridin-2-yl | H |
| 3-889 | 1-Brompropan-2-yl | Me | 5-Br-pyridin-2-yl | H |
| 3-890 | 1-Brompropan-2-yl | Me | 5-Cl-2-thienyl | H |
| 3-891 | 1-Brompropan-2-yl | Me | 5-Br-2-thienyl | H |
| 3-892 | 1-Chlorpropan-2-yl | Me | 4-Cl-Ph | H |
| 3-893 | 1-Chlorpropan-2-yl | Me | 4-Br-Ph | H |
| 3-894 | 1-Chlorpropan-2-yl | Me | 5-Cl-pyridin-2-yl | H |
| 3-895 | 1-Chlorpropan-2-yl | Me | 5-Br-pyridin-2-yl | H |
| 3-896 | 1-Chlorpropan-2-yl | Me | 5-Cl-2-thienyl | H |
| 3-897 | 1-Chlorpropan-2-yl | Me | 5-Br-2-thienyl | H |
| 3-898 | 2-Isopropoxyethyl | Me | 4-Cl-Ph | H |
| 3-899 | 2-Isopropoxyethyl | Me | 4-Br-Ph | H |
| 3-900 | 2-Isopropoxyethyl | Me | 5-Cl-pyridin-2-yl | H |
| 3-901 | 2-Isopropoxyethyl | Me | 5-Br-pyridin-2-yl | H |
| 3-902 | 2-Isopropoxyethyl | Me | 5-Cl-2-thienyl | H |
| 3-903 | 2-Isopropoxyethyl | Me | 5-Br-2-thienyl | H |
| 3-904 | Tetrahydrofuran-3-yl | Me | 4-Cl-Ph | H |
| 3-905 | Tetrahydrofuran-3-yl | Me | 4-Br-Ph | H |
| 3-906 | Tetrahydrofuran-3-yl | Me | 5-Cl-pyridin-2-yl | H |
| 3-907 | Tetrahydrofuran-3-yl | Me | 5-Br-pyridin-2-yl | H |
| 3-908 | Tetrahydrofuran-3-yl | Me | 5-Cl-2-thienyl | H |
| 3-909 | Tetrahydrofuran-3-yl | Me | 5-Br-2-thienyl | H |
| 3-910 | 2-(2,2,2-Trifluorethoxy)ethyl | Me | 4-Cl-Ph | H |
| 3-911 | 2-(2,2,2-Trifluorethoxy)ethyl | Me | 4-Br-Ph | H |
| 3-912 | 2-(2,2,2-Trifluorethoxy)ethyl | Me | 5-Cl-pyridin-2-yl | H |
| 3-913 | 2-(2,2,2-Trifluorethoxy)ethyl | Me | 5-Br-pyridin-2-yl | H |
| 3-914 | 2-(2,2,2-Trifluorethoxy)ethyl | Me | 5-Cl-2-thienyl | H |
| 3-915 | 2-(2,2,2-Trifluorethoxy)ethyl | Me | 5-Br-2-thienyl | H |
| 3-916 | (3,3,4,4,4-Pentafluorbutan-2-yl | Me | 4-Cl-Ph | H |
| 3-917 | (3,3,4,4,4-Pentafluorbutan-2-yl | Me | 4-Br-Ph | H |
| 3-918 | (3,3,4,4,4-Pentafluorbutan-2-yl | Me | 5-Cl-pyridin-2-yl | H |
| 3-919 | (3,3,4,4,4-Pentafluorbutan-2-yl | Me | 5-Br-pyridin-2-yl | H |
| 3-920 | (3,3,4,4,4-Pentafluorbutan-2-yl | Me | 5-Cl-2-thienyl | H |
| 3-921 | (3,3,4,4,4-Pentafluorbutan-2-yl | Me | 5-Br-2-thienyl | H |
| 3-922 | 1-(N, N-Dimethylaminocarbonyl)-eth-1-yl | Me | 4-Cl-Ph | H |
| 3-923 | 1-(N, N-Dimethylaminocarbonyl)-eth-1-yl | Me | 4-Br-Ph | H |
| 3-924 | 1-(N, N-Dimethylaminocarbonyl)-eth-1-yl | Me | 5-Cl-pyridin-2-yl | H |
| 3-925 | 1-(N, N-Dimethylaminocarbonyl)-eth-1-yl | Me | 5-Br-pyridin-2-yl | H |
| 3-926 | 1-(N, N-Dimethylaminocarbonyl)-eth-1-yl | Me | 5-Cl-2-thienyl | H |
| 3-927 | 1-(N, N-Dimethylaminocarbonyl)-eth-1-yl | Me | 5-Br-2-thienyl | H |
| 3-928 | (1,3-dioxan-2-yl)-methyl | Me | 4-Cl-Ph | H |
| 3-929 | (1,3-dioxan-2-yl)-methyl | Me | 4-Br-Ph | H |
| 3-930 | (1,3-dioxan-2-yl)-methyl | Me | 5-Cl-pyridin-2-yl | H |
| 3-931 | (1,3-dioxan-2-yl)-methyl | Me | 5-Br-pyridin-2-yl | H |
| 3-932 | (1,3-dioxan-2-yl)-methyl | Me | 5-Cl-2-thienyl | H |
| 3-933 | (1,3-dioxan-2-yl)-methyl | Me | 5-Br-2-thienyl | H |
| 3-934 | 1,1,1-Trifluorbutan-2-yl | Me | 4-Cl-Ph | H |
| 3-935 | 1,1,1-Trifluorbutan-2-yl | Me | 4-Br-Ph | H |
| 3-936 | 1,1,1-Trifluorbutan-2-yl | Me | 5-Cl-pyridin-2-yl | H |
| 3-937 | 1,1,1-Trifluorbutan-2-yl | Me | 5-Br-pyridin-2-yl | H |
| 3-938 | 1,1,1-Trifluorbutan-2-yl | Me | 5-Cl-2-thienyl | H |
| 3-939 | 1,1,1-Trifluorbutan-2-yl | Me | 5-Br-2-thienyl | H |
| 3-940 | 2-(But-2-yliden-aminooxy)-eth-1-yl | Me | 4-Cl-Ph | H |
| 3-941 | 2-(But-2-yliden-aminooxy)-eth-1-yl | Me | 4-Br-Ph | H |
| 3-942 | 2-(But-2-yliden-aminooxy)-eth-1-yl | Me | 5-Cl-pyridin-2-yl | H |
| 3-943 | 2-(But-2-yliden-aminooxy)-eth-1-yl | Me | 5-Br-pyridin-2-yl | H |
| 3-944 | 2-(But-2-yliden-aminooxy)-eth-1-yl | Me | 5-Cl-2-thienyl | H |
| 3-945 | 2-(But-2-yliden-aminooxy)-eth-1-yl | Me | 5-Br-2-thienyl | H |
| 3-946 | Oxetan-2-yl-methyl | Me | 4-Cl-Ph | H |
| 3-947 | Oxetan-2-yl-methyl | Me | 4-Br-Ph | H |
| 3-948 | Oxetan-2-yl-methyl | Me | 5-Cl-pyridin-2-yl | H |
| 3-949 | Oxetan-2-yl-methyl | Me | 5-Br-pyridin-2-yl | H |
| 3-950 | Oxetan-2-yl-methyl | Me | 5-Cl-2-thienyl | H |
| 3-951 | Oxetan-2-yl-methyl | Me | 5-Br-2-thienyl | H |
| 3-952 | 2,2-Dimethoxyethyl | Me | 4-Cl-Ph | H |
| 3-953 | 2,2-Dimethoxyethyl | Me | 4-Br-Ph | H |
| 3-954 | 2,2-Dimethoxyethyl | Me | 5-Cl-pyridin-2-yl | H |
| 3-955 | 2,2-Dimethoxyethyl | Me | 5-Br-pyridin-2-yl | H |
| 3-956 | 2,2-Dimethoxyethyl | Me | 5-Cl-2-thienyl | H |
| 3-957 | 2,2-Dimethoxyethyl | Me | 5-Br-2-thienyl | H |
| 3-958 | 1-Chlorpropyl | Me | 4-Cl-Ph | H |
| 3-959 | 1-Chlorpropyl | Me | 4-Br-Ph | H |
| 3-960 | 1-Chlorpropyl | Me | 5-Cl-pyridin-2-yl | H |
| 3-961 | 1-Chlorpropyl | Me | 5-Br-pyridin-2-yl | H |
| 3-962 | 1-Chlorpropyl | Me | 5-Cl-2-thienyl | H |
| 3-963 | 1-Chlorpropyl | Me | 5-Br-2-thienyl | H |
| 3-964 | 4-Chlorbutan-2-yl | Me | 4-Cl-Ph | H |
| 3-965 | 4-Chlorbutan-2-yl | Me | 4-Br-Ph | H |
| 3-966 | 4-Chlorbutan-2-yl | Me | 5-Cl-pyridin-2-yl | H |
| 3-967 | 4-Chlorbutan-2-yl | Me | 5-Br-pyridin-2-yl | H |
| 3-968 | 4-Chlorbutan-2-yl | Me | 5-Cl-2-thienyl | H |
| 3-969 | 4-Chlorbutan-2-yl | Me | 5-Br-2-thienyl | H |
| 3-970 | 3-Chlorpropan-2-yl | Me | 4-Cl-Ph | H |
| 3-971 | 3-Chlorpropan-2-yl | Me | 4-Br-Ph | H |
| 3-972 | 3-Chlorpropan-2-yl | Me | 5-Cl-pyridin-2-yl | H |
| 3-973 | 3-Chlorpropan-2-yl | Me | 5-Br-pyridin-2-yl | H |
| 3-974 | 3-Chlorpropan-2-yl | Me | 5-Cl-2-thienyl | H |
| 3-975 | 3-Chlorpropan-2-yl | Me | 5-Br-2-thienyl | H |
| 3-976 | 2-(2-Chlorethoxy)-ethyl | Me | 4-Cl-Ph | H |
| 3-977 | 2-(2-Chlorethoxy)-ethyl | Me | 4-Br-Ph | H |
| 3-978 | 2-(2-Chlorethoxy)-ethyl | Me | 5-Cl-pyridin-2-yl | H |
| 3-979 | 2-(2-Chlorethoxy)-ethyl | Me | 5-Br-pyridin-2-yl | H |
| 3-980 | 2-(2-Chlorethoxy)-ethyl | Me | 5-Cl-2-thienyl | H |
| 3-981 | 2-(2-Chlorethoxy)-ethyl | Me | 5-Br-2-thienyl | H |
| 3-982 | 2,2-Dichlorethyl | Me | 4-Cl-Ph | H |
| 3-983 | 2,2-Dichlorethyl | Me | 4-Br-Ph | H |
| 3-984 | 2,2-Dichlorethyl | Me | 5-Cl-pyridin-2-yl | H |
| 3-985 | 2,2-Dichlorethyl | Me | 5-Br-pyridin-2-yl | H |
| 3-986 | 2,2-Dichlorethyl | Me | 5-Cl-2-thienyl | H |
| 3-987 | 2,2-Dichlorethyl | Me | 5-Br-2-thienyl | H |
| 3-988 | 2,3-Dichlorpropyl | Me | 4-Cl-Ph | H |
| 3-989 | 2,3-Dichlorpropyl | Me | 4-Br-Ph | H |
| 3-990 | 2,3-Dichlorpropyl | Me | 5-Cl-pyridin-2-yl | H |
| 3-991 | 2,3-Dichlorpropyl | Me | 5-Br-pyridin-2-yl | H |
| 3-992 | 2,3-Dichlorpropyl | Me | 5-Cl-2-thienyl | H |
| 3-993 | 2,3-Dichlorpropyl | Me | 5-Br-2-thienyl | H |
| 3-994 | 1,3-Dichlorprop-2-yl | Me | 4-Cl-Ph | H |
| 3-995 | 1,3-Dichlorprop-2-yl | Me | 4-Br-Ph | H |
| 3-996 | 1,3-Dichlorprop-2-yl | Me | 5-Cl-pyridin-2-yl | H |
| 3-997 | 1,3-Dichlorprop-2-yl | Me | 5-Br-pyridin-2-yl | H |
| 3-998 | 1,3-Dichlorprop-2-yl | Me | 5-Cl-2-thienyl | H |
| 3-999 | 1,3-Dichlorprop-2-yl | Me | 5-Br-2-thienyl | H |
| 3-1000 | 2-Chlor-2,2-difluorethyl | Me | 4-Cl-Ph | H |
| 3-1001 | 2-Chlor-2,2-difluorethyl | Me | 4-Br-Ph | H |
| 3-1002 | 2-Chlor-2,2-difluorethyl | Me | 5-Cl-pyridin-2-yl | H |
| 3-1003 | 2-Chlor-2,2-difluorethyl | Me | 5-Br-pyridin-2-yl | H |
| 3-1004 | 2-Chlor-2,2-difluorethyl | Me | 5-Cl-2-thienyl | H |
| 3-1005 | 2-Chlor-2,2-difluorethyl | Me | 5-Br-2-thienyl | H |
| 3-1006 | 1-Chlor-2-methylpropan-2-yl | Me | 4-Cl-Ph | H |
| 3-1007 | 1-Chlor-2-methylpropan-2-yl | Me | 4-Br-Ph | H |
| 3-1008 | 1-Chlor-2-methylpropan-2-yl | Me | 5-Cl-pyridin-2-yl | H |
| 3-1009 | 1-Chlor-2-methylpropan-2-yl | Me | 5-Br-pyridin-2-yl | H |
| 3-1010 | 1-Chlor-2-methylpropan-2-yl | Me | 5-Cl-2-thienyl | H |
| 3-1011 | 1-Chlor-2-methylpropan-2-yl | Me | 5-Br-2-thienyl | H |
| 3-1012 | 1-Fluor-3-methoxypropan-2-yl | Me | 4-Cl-Ph | H |
| 3-1013 | 1-Fluor-3-methoxypropan-2-yl | Me | 4-Br-Ph | H |
| 3-1014 | 1-Fluor-3-methoxypropan-2-yl | Me | 5-Cl-pyridin-2-yl | H |
| 3-1015 | 1-Fluor-3-methoxypropan-2-yl | Me | 5-Br-pyridin-2-yl | H |
| 3-1016 | 1-Fluor-3-methoxypropan-2-yl | Me | 5-Cl-2-thienyl | H |
| 3-1017 | 1-Fluor-3-methoxypropan-2-yl | Me | 5-Br-2-thienyl | H |
| 3-1018 | 3,3,3-Trifluorpropyl | Me | 4-Cl-Ph | H |
| 3-1019 | 3,3,3-Trifluorpropyl | Me | 4-Br-Ph | H |
| 3-1020 | 3,3,3-Trifluorpropyl | Me | 5-Cl-pyridin-2-yl | H |
| 3-1021 | 3,3,3-Trifluorpropyl | Me | 5-Br-pyridin-2-yl | H |
| 3-1022 | 3,3,3-Trifluorpropyl | Me | 5-Cl-2-thienyl | H |
| 3-1023 | 3,3,3-Trifluorpropyl | Me | 5-Br-2-thienyl | H |
| 3-1024 | 2-Chlor-phenyl | Me | 4-Cl-Ph | H |
| 3-1025 | 2-Chlor-phenyl | Me | 4-Br-Ph | H |
| 3-1026 | 2-Chlor-phenyl | Me | 5-Cl-pyridin-2-yl | H |
| 3-1027 | 2-Chlor-phenyl | Me | 5-Br-pyridin-2-yl | H |
| 3-1028 | 2-Chlor-phenyl | Me | 5-Cl-2-thienyl | H |
| 3-1029 | 2-Chlor-phenyl | Me | 5-Br-2-thienyl | H |
| 3-1030 | 2-Chlorpyridin-3-yl | Me | 4-Cl-Ph | H |
| 3-1031 | 2-Chlorpyridin-3-yl | Me | 4-Br-Ph | H |
| 3-1032 | 2-Chlorpyridin-3-yl | Me | 5-Cl-pyridin-2-yl | H |
| 3-1033 | 2-Chlorpyridin-3-yl | Me | 5-Br-pyridin-2-yl | H |
| 3-1034 | 2-Chlorpyridin-3-yl | Me | 5-Cl-2-thienyl | H |
| 3-1035 | 2-Chlorpyridin-3-yl | Me | 5-Br-2-thienyl | H |
| 3-1036 | 3-Chlorpyridin-2-yl | Me | 4-Cl-Ph | H |
| 3-1037 | 3-Chlorpyridin-2-yl | Me | 4-Br-Ph | H |
| 3-1038 | 3-Chlorpyridin-2-yl | Me | 5-Cl-pyridin-2-yl | H |
| 3-1039 | 3-Chlorpyridin-2-yl | Me | 5-Br-pyridin-2-yl | H |
| 3-1040 | 3-Chlorpyridin-2-yl | Me | 5-Cl-2-thienyl | H |
| 3-1041 | 3-Chlorpyridin-2-yl | Me | 5-Br-2-thienyl | H |
| 3-1042 | Pentafluorethyl | Me | 4-Cl-Ph | H |
| 3-1043 | Pentafluorethyl | Me | 4-Br-Ph | H |
| 3-1044 | Pentafluorethyl | Me | 5-Cl-pyridin-2-yl | H |
| 3-1045 | Pentafluorethyl | Me | 5-Br-pyridin-2-yl | H |
| 3-1046 | Pentafluorethyl | Me | 5-Cl-2-thienyl | H |
| 3-1047 | Pentafluorethyl | Me | 5-Br-2-thienyl | H |
| 3-1048 | 1,2,2,2-Tetrafluorethyl | Me | 4-Cl-Ph | H |
| 3-1049 | 1,2,2,2-Tetrafluorethyl | Me | 4-Br-Ph | H |
| 3-1050 | 1,2,2,2-Tetrafluorethyl | Me | 5-Cl-pyridin-2-yl | H |
| 3-1051 | 1,2,2,2-Tetrafluorethyl | Me | 5-Br-pyridin-2-yl | H |
| 3-1052 | 1,2,2,2-Tetrafluorethyl | Me | 5-Cl-2-thienyl | H |
| 3-1053 | 1,2,2,2-Tetrafluorethyl | Me | 5-Br-2-thienyl | H |
| 3-1054 | 1,1,2,2-Tetrafluorethyl | Me | 4-Cl-Ph | H |
| 3-1055 | 1,1,2,2-Tetrafluorethyl | Me | 4-Br-Ph | H |
| 3-1056 | 1,1,2,2-Tetrafluorethyl | Me | 5-Cl-pyridin-2-yl | H |
| 3-1057 | 1,1,2,2-Tetrafluorethyl | Me | 5-Br-pyridin-2-yl | H |
| 3-1058 | 1,1,2,2-Tetrafluorethyl | Me | 5-Cl-2-thienyl | H |
| 3-1059 | 1,1,2,2-Tetrafluorethyl | Me | 5-Br-2-thienyl | H |
| 3-1060 | 1,1,2-Trifluorethyl | Me | 4-Cl-Ph | H |
| 3-1061 | 1,1,2-Trifluorethyl | Me | 4-Br-Ph | H |
| 3-1062 | 1,1,2-Trifluorethyl | Me | 5-Cl-pyridin-2-yl | H |
| 3-1063 | 1,1,2-Trifluorethyl | Me | 5-Br-pyridin-2-yl | H |
| 3-1064 | 1,1,2-Trifluorethyl | Me | 5-Cl-2-thienyl | H |
| 3-1065 | 1,1,2-Trifluorethyl | Me | 5-Br-2-thienyl | H |
| 3-1066 | 2-Methylbut-3-in-2-yl | Me | 4-Cl-Ph | H |
| 3-1067 | 2-Methylbut-3-in-2-yl | Me | 4-Br-Ph | H |
| 3-1068 | 2-Methylbut-3-in-2-yl | Me | 5-Cl-pyridin-2-yl | H |
| 3-1069 | 2-Methylbut-3-in-2-yl | Me | 5-Br-pyridin-2-yl | H |
| 3-1070 | 2-Methylbut-3-in-2-yl | Me | 5-Cl-2-thienyl | H |
| 3-1071 | 2-Methylbut-3-in-2-yl | Me | 5-Br-2-thienyl | H |
| 3-1072 | 1-(Ethoxycarbonyl)-eth-1-yl | Me | 4-Cl-Ph | H |
| 3-1073 | 1-(Ethoxycarbonyl)-eth-1-yl | Me | 4-Br-Ph | H |
| 3-1074 | 1-(Ethoxycarbonyl)-eth-1-yl | Me | 5-Cl-pyridin-2-yl | H |
| 3-1075 | 1-(Ethoxycarbonyl)-eth-1-yl | Me | 5-Br-pyridin-2-yl | H |
| 3-1076 | 1-(Ethoxycarbonyl)-eth-1-yl | Me | 5-Cl-2-thienyl | H |
| 3-1077 | 1-(Ethoxycarbonyl)-eth-1-yl | Me | 5-Br-2-thienyl | H |
| 3-1078 | 1,1,2,3,3,3-Hexafluorpropyl | Me | 4-Cl-Ph | H |
| 3-1079 | 1,1,2,3,3,3-Hexafluorpropyl | Me | 4-Br-Ph | H |
| 3-1080 | 1,1,2,3,3,3-Hexafluorpropyl | Me | 5-Cl-pyridin-2-yl | H |
| 3-1081 | 1,1,2,3,3,3-Hexafluorpropyl | Me | 5-Br-pyridin-2-yl | H |
| 3-1082 | 1,1,2,3,3,3-Hexafluorpropyl | Me | 5-Cl-2-thienyl | H |
| 3-1083 | 1,1,2,3,3,3-Hexafluorpropyl | Me | 5-Br-2-thienyl | H |
| 3-1084 | Isobutyl | Me | 4-Cl-Ph | H |
| 3-1085 | Isobutyl | Me | 4-Br-Ph | H |
| 3-1086 | Isobutyl | Me | 5-Cl-pyridin-2-yl | H |
| 3-1087 | Isobutyl | Me | 5-Br-pyridin-2-yl | H |
| 3-1088 | Isobutyl | Me | 5-Cl-2-thienyl | H |
| 3-1089 | Isobutyl | Me | 5-Br-2-thienyl | H |
| 3-1090 | n-Pentyl | Me | 4-Cl-Ph | H |
| 3-1091 | n-Pentyl | Me | 4-Br-Ph | H |
| 3-1092 | n-Pentyl | Me | 5-Cl-pyridin-2-yl | H |
| 3-1093 | n-Pentyl | Me | 5-Br-pyridin-2-yl | H |
| 3-1094 | n-Pentyl | Me | 5-Cl-2-thienyl | H |
| 3-1095 | n-Pentyl | Me | 5-Br-2-thienyl | H |
| 3-1096 | n-Heptyl | Me | 4-Cl-Ph | H |
| 3-1097 | n-Heptyl | Me | 4-Br-Ph | H |
| 3-1098 | n-Heptyl | Me | 5-Cl-pyridin-2-yl | H |
| 3-1099 | n-Heptyl | Me | 5-Br-pyridin-2-yl | H |
| 3-1100 | n-Heptyl | Me | 5-Cl-2-thienyl | H |
| 3-1101 | n-Heptyl | Me | 5-Br-2-thienyl | H |
| 3-1102 | n-Nonyl | Me | 4-Cl-Ph | H |
| 3-1103 | n-Nonyl | Me | 4-Br-Ph | H |
| 3-1104 | n-Nonyl | Me | 5-Cl-pyridin-2-yl | H |
| 3-1105 | n-Nonyl | Me | 5-Br-pyridin-2-yl | H |
| 3-1106 | n-Nonyl | Me | 5-Cl-2-thienyl | H |
| 3-1107 | n-Nonyl | Me | 5-Br-2-thienyl | H |
| 3-1108 | Cyclopentyl | Me | 4-Cl-Ph | H |
| 3-1109 | Cyclopentyl | Me | 4-Br-Ph | H |
| 3-1110 | Cyclopentyl | Me | 5-Cl-pyridin-2-yl | H |
| 3-1111 | Cyclopentyl | Me | 5-Br-pyridin-2-yl | H |
| 3-1112 | Cyclopentyl | Me | 5-Cl-2-thienyl | H |
| 3-1113 | Cyclopentyl | Me | 5-Br-2-thienyl | H |
| 3-1114 | Cyclohexyl | Me | 4-Cl-Ph | H |
| 3-1115 | Cyclohexyl | Me | 4-Br-Ph | H |
| 3-1116 | Cyclohexyl | Me | 5-Cl-pyridin-2-yl | H |
| 3-1117 | Cyclohexyl | Me | 5-Br-pyridin-2-yl | H |
| 3-1118 | Cyclohexyl | Me | 5-Cl-2-thienyl | H |
| 3-1119 | Cyclohexyl | Me | 5-Br-2-thienyl | H |
| 3-1120 | sBu | Me | 4-Cl-Ph | H |
| 3-1121 | sBu | Me | 4-Br-Ph | H |
| 3-1122 | sBu | Me | 5-Cl-pyridin-2-yl | H |
| 3-1123 | sBu | Me | 5-Br-pyridin-2-yl | H |
| 3-1124 | sBu | Me | 5-Cl-2-thienyl | H |
| 3-1125 | sBu | Me | 5-Br-2-thienyl | H |
| 3-1126 | Pentan-3-yl | Me | 4-Cl-Ph | H |
| 3-1127 | Pentan-3-yl | Me | 4-Br-Ph | H |
| 3-1128 | Pentan-3-yl | Me | 5-Cl-pyridin-2-yl | H |
| 3-1129 | Pentan-3-yl | Me | 5-Br-pyridin-2-yl | H |
| 3-1130 | Pentan-3-yl | Me | 5-Cl-2-thienyl | H |
| 3-1131 | Pentan-3-yl | Me | 5-Br-2-thienyl | H |
| 3-1132 | 1-(Methoxycarbonyl)-eth-1-yl | Me | 4-Cl-Ph | H |
| 3-1133 | 1-(Methoxycarbonyl)-eth-1-yl | Me | 4-Br-Ph | H |
| 3-1134 | 1-(Methoxycarbonyl)-eth-1-yl | Me | 5-Cl-pyridin-2-yl | H |
| 3-1135 | 1-(Methoxycarbonyl)-eth-1-yl | Me | 5-Br-pyridin-2-yl | H |
| 3-1136 | 1-(Methoxycarbonyl)-eth-1-yl | Me | 5-Cl-2-thienyl | H |
| 3-1137 | 1-(Methoxycarbonyl)-eth-1-yl | Me | 5-Br-2-thienyl | H |
| 3-1138 | 2,2,2-Trichlorethyl | Me | 4-Cl-Ph | H |
| 3-1139 | 2,2,2-Trichlorethyl | Me | 4-Br-Ph | H |
| 3-1140 | 2,2,2-Trichlorethyl | Me | 5-Cl-pyridin-2-yl | H |
| 3-1141 | 2,2,2-Trichlorethyl | Me | 5-Br-pyridin-2-yl | H |
| 3-1142 | 2,2,2-Trichlorethyl | Me | 5-Cl-2-thienyl | H |
| 3-1143 | 2,2,2-Trichlorethyl | Me | 5-Br-2-thienyl | H |
| 3-1144 | 3-Chlorpropyl | Me | 4-Cl-Ph | H |
| 3-1145 | 3-Chlorpropyl | Me | 4-Br-Ph | H |
| 3-1146 | 3-Chlorpropyl | Me | 5-Cl-pyridin-2-yl | H |
| 3-1147 | 3-Chlorpropyl | Me | 5-Br-pyridin-2-yl | H |
| 3-1148 | 3-Chlorpropyl | Me | 5-Cl-2-thienyl | H |
| 3-1149 | 3-Chlorpropyl | Me | 5-Br-2-thienyl | H |
| 3-1150 | 2-(2-Methoxyethoxy)-ethyl | Me | 4-Cl-Ph | H |
| 3-1151 | 2-(2-Methoxyethoxy)-ethyl | Me | 4-Br-Ph | H |
| 3-1152 | 2-(2-Methoxyethoxy)-ethyl | Me | 5-Cl-pyridin-2-yl | H |
| 3-1153 | 2-(2-Methoxyethoxy)-ethyl | Me | 5-Br-pyridin-2-yl | H |
| 3-1154 | 2-(2-Methoxyethoxy)-ethyl | Me | 5-Cl-2-thienyl | H |
| 3-1155 | 2-(2-Methoxyethoxy)-ethyl | Me | 5-Br-2-thienyl | H |
| 3-1156 | Butyl-2-yl-methyl | Me | 4-Cl-Ph | H |
| 3-1157 | Butyl-2-yl-methyl | Me | 4-Br-Ph | H |
| 3-1158 | Butyl-2-yl-methyl | Me | 5-Cl-pyridin-2-yl | H |
| 3-1159 | Butyl-2-yl-methyl | Me | 5-Br-pyridin-2-yl | H |
| 3-1160 | Butyl-2-yl-methyl | Me | 5-Cl-2-thienyl | H |
| 3-1161 | Butyl-2-yl-methyl | Me | 5-Br-2-thienyl | H |
| 3-1162 | But-3-in-1-yl | Me | 4-Cl-Ph | H |
| 3-1163 | But-3-in-1-yl | Me | 4-Br-Ph | H |
| 3-1164 | But-3-in-1-yl | Me | 5-Cl-pyridin-2-yl | H |
| 3-1165 | But-3-in-1-yl | Me | 5-Br-pyridin-2-yl | H |
| 3-1166 | But-3-in-1-yl | Me | 5-Cl-2-thienyl | H |
| 3-1167 | But-3-in-1-yl | Me | 5-Br-2-thienyl | H |
| 3-1168 | (2,2-Dichlorcyclopropyl)-methyl | Me | 4-Cl-Ph | H |
| 3-1169 | (2,2-Dichlorcyclopropyl)-methyl | Me | 4-Br-Ph | H |
| 3-1170 | (2,2-Dichlorcyclopropyl)-methyl | Me | 5-Cl-pyridin-2-yl | H |
| 3-1171 | (2,2-Dichlorcyclopropyl)-methyl | Me | 5-Br-pyridin-2-yl | H |
| 3-1172 | (2,2-Dichlorcyclopropyl)-methyl | Me | 5-Cl-2-thienyl | H |
| 3-1173 | (2,2-Dichlorcyclopropyl)-methyl | Me | 5-Br-2-thienyl | H |
| 3-1174 | 2-(N,N-Diethylamino)-eth-1-yl | Me | 4-Cl-Ph | H |
| 3-1175 | 2-(N,N-Diethylamino)-eth-1-yl | Me | 4-Br-Ph | H |
| 3-1176 | 2-(N,N-Diethylamino)-eth-1-yl | Me | 5-Cl-pyridin-2-yl | H |
| 3-1177 | 2-(N,N-Diethylamino)-eth-1-yl | Me | 5-Br-pyridin-2-yl | H |
| 3-1178 | 2-(N,N-Diethylamino)-eth-1-yl | Me | 5-Cl-2-thienyl | H |
| 3-1179 | 2-(N,N-Diethylamino)-eth-1-yl | Me | 5-Br-2-thienyl | H |
| 3-1180 | 2-Carboxy-phenyl | Me | 4-Cl-Ph | H |
| 3-1181 | 2-Carboxy-phenyl | Me | 4-Br-Ph | H |
| 3-1182 | 2-Carboxy-phenyl | Me | 5-Cl-pyridin-2-yl | H |
| 3-1183 | 2-Carboxy-phenyl | Me | 5-Br-pyridin-2-yl | H |
| 3-1184 | 2-Carboxy-phenyl | Me | 5-Cl-2-thienyl | H |
| 3-1185 | 2-Carboxy-phenyl | Me | 5-Br-2-thienyl | H |
| 3-1186 | tButyl | Me | 4-Cl-Ph | H |
| 3-1187 | tBu | Me | 4-Br-Ph | H |
| 3-1188 | tBu | Me | 5-Cl-pyridin-2-yl | H |
| 3-1189 | tBu | Me | 5-Br-pyridin-2-yl | H |
| 3-1190 | tBu | Me | 5-Cl-2-thienyl | H |
| 3-1191 | tBu | Me | 5-Br-2-thienyl | H |
| 3-1192 | 1-Methyl-cyclopropyl | Me | 4-Cl-Ph | H |
| 3-1193 | 1-Methyl-cyclopropyl | Me | 4-Br-Ph | H |
| 3-1194 | 1-Methyl-cyclopropyl | Me | 5-Cl-pyridin-2-yl | H |
| 3-1195 | 1-Methyl-cyclopropyl | Me | 5-Br-pyridin-2-yl | H |
| 3-1196 | 1-Methyl-cyclopropyl | Me | 5-Cl-2-thienyl | H |
| 3-1197 | 1-Methyl-cyclopropyl | Me | 5-Br-2-thienyl | H |
| 3-1198 | Trimethylsilylmethyl | Me | 4-Cl-Ph | H |
| 3-1199 | Trimethylsilylmethyl | Me | 4-Br-Ph | H |
| 3-1200 | Trimethylsilylmethyl | Me | 5-Cl-pyridin-2-yl | H |
| 3-1201 | Trimethylsilylmethyl | Me | 5-Br-pyridin-2-yl | H |
| 3-1202 | Trimethylsilylmethyl | Me | 5-Cl-2-thienyl | H |
| 3-1203 | Trimethylsilylmethyl | Me | 5-Br-2-thienyl | H |
| 3-1204 | 2,3-Dihydro-1H-inden-5-yl | Me | 4-Cl-Ph | H |
| 3-1205 | 2,3-Dihydro-1H-inden-5-yl | Me | 4-Br-Ph | H |
| 3-1206 | 2,3-Dihydro-1H-inden-5-yl | Me | 5-Cl-pyridin-2-yl | H |
| 3-1207 | 2,3-Dihydro-1H-inden-5-yl | Me | 5-Br-pyridin-2-yl | H |
| 3-1208 | 2,3-Dihydro-1H-inden-5-yl | Me | 5-Cl-2-thienyl | H |
| 3-1209 | 2,3-Dihydro-1H-inden-5-yl | Me | 5-Br-2-thienyl | H |
| 3-1210 | 1-Methylcyclobutyl | Me | 4-Cl-Ph | H |
| 3-1211 | 1-Methylcyclobutyl | Me | 4-Br-Ph | H |
| 3-1212 | 1-Methylcyclobutyl | Me | 5-Cl-pyridin-2-yl | H |
| 3-1213 | 1-Methylcyclobutyl | Me | 5-Br-pyridin-2-yl | H |
| 3-1214 | 1-Methylcyclobutyl | Me | 5-Cl-2-thienyl | H |
| 3-1215 | 1-Methylcyclobutyl | Me | 5-Br-2-thienyl | H |
| 3-1216 | 2-(Oxetan-3-yl)-eth-1-yl | Me | 4-Cl-Ph | H |
| 3-1217 | 2-(Oxetan-3-yl)-eth-1-yl | Me | 4-Br-Ph | H |
| 3-1218 | 2-(Oxetan-3-yl)-eth-1-yl | Me | 5-Cl-pyridin-2-yl | H |
| 3-1219 | 2-(Oxetan-3-yl)-eth-1-yl | Me | 5-Br-pyridin-2-yl | H |
| 3-1220 | 2-(Oxetan-3-yl)-eth-1-yl | Me | 5-Cl-2-thienyl | H |
| 3-1221 | 2-(Oxetan-3-yl)-eth-1-yl | Me | 5-Br-2-thienyl | H |
| 3-1222 | Bu | Me | 4-Cl-Ph | H |
| 3-1223 | Bu | Me | 4-Br-Ph | H |
| 3-1224 | Bu | Me | 5-Cl-pyridin-2-yl | H |
| 3-1225 | Bu | Me | 5-Br-pyridin-2-yl | H |
| 3-1226 | Bu | Me | 5-Cl-2-thienyl | H |
| 3-1227 | Bu | Me | 5-Br-2-thienyl | H |
| 3-1228 | 2-(N,N-Diethylamino)-eth-1-yl | Me | 4-Cl-Ph | H |
| 3-1229 | 2-(N,N-Diethylamino)-eth-1-yl | Me | 4-Br-Ph | H |
| 3-1230 | 2-(N, N-Diethylamino)-eth-1-yl | Me | 5-Cl-pyridin-2-yl | H |
| 3-1231 | 2-(N,N-Diethylamino)-eth-1-yl | Me | 5-Br-pyridin-2-yl | H |
| 3-1232 | 2-(N,N-Diethylamino)-eth-1-yl | Me | 5-Cl-2-thienyl | H |
| 3-1233 | 2-(N,N-Diethylamino)-eth-1-yl | Me | 5-Br-2-thienyl | H |
| 3-1234 | 2-(Morpholin-4-yl)-eth-1-yl | Me | 4-Cl-Ph | H |
| 3-1235 | 2-(Morpholin-4-yl)-eth-1-yl | Me | 4-Br-Ph | H |
| 3-1236 | 2-(Morpholin-4-yl)-eth-1-yl | Me | 5-Cl-pyridin-2-yl | H |
| 3-1237 | 2-(Morpholin-4-yl)-eth-1-yl | Me | 5-Br-pyridin-2-yl | H |
| 3-1238 | 2-(Morpholin-4-yl)-eth-1-yl | Me | 5-Cl-2-thienyl | H |
| 3-1239 | 2-(Morpholin-4-yl)-eth-1-yl | Me | 5-Br-2-thienyl | H |
| 3-1240 | 2-Chlor-thiophen-3-yl | Me | 4-Cl-Ph | H |
| 3-1241 | 2-Chlor-thiophen-3-yl | Me | 4-Br-Ph | H |
| 3-1242 | 2-Chlor-thiophen-3-yl | Me | 5-Cl-pyridin-2-yl | H |
| 3-1243 | 2-Chlor-thiophen-3-yl | Me | 5-Br-pyridin-2-yl | H |
| 3-1244 | 2-Chlor-thiophen-3-yl | Me | 5-Cl-2-thienyl | H |
| 3-1245 | 2-Chlor-thiophen-3-yl | Me | 5-Br-2-thienyl | H |
| 3-1246 | (N,N-Dimethylaminocarbonyl)-methyl | Me | 4-Cl-Ph | H |
| 3-1247 | (N,N-Dimethylaminocarbonyl)-methyl | Me | 4-Br-Ph | H |
| 3-1248 | (N,N-Dimethylaminocarbonyl)-methyl | Me | 5-Cl-pyridin-2-yl | H |
| 3-1249 | (N,N-Dimethylaminocarbonyl)-methyl | Me | 5-Br-pyridin-2-yl | H |
| 3-1250 | (N,N-Dimethylaminocarbonyl)-methyl | Me | 5-Cl-2-thienyl | H |
| 3-1251 | (N,N-Dimethylaminocarbonyl)-methyl | Me | 5-Br-2-thienyl | H |
| 3-1252 | 1-(t-Butylcarbonyloxy)-2-methylprop-1-yl | Me | 4-Cl-Ph | H |
| 3-1253 | 1-(t-Butylcarbonyloxy)-2-methylprop-1-yl | Me | 4-Br-Ph | H |
| 3-1254 | 1-(t-Butylcarbonyloxy)-2-methylprop-1-yl | Me | 5-Cl-pyridin-2-yl | H |
| 3-1255 | 1-(t-Butylcarbonyloxy)-2-methylprop-1-yl | Me | 5-Br-pyridin-2-yl | H |
| 3-1256 | 1-(t-Butylcarbonyloxy)-2-methylprop-1-yl | Me | 5-Cl-2-thienyl | H |
| 3-1257 | 1-(t-Butylcarbonyloxy)-2-methylprop-1-yl | Me | 5-Br-2-thienyl | H |
| 3-1258 | (5-Methyl-2-oxo-1,3-dioxol-4-yl)-methyl | Me | 4-CI-Ph | H |
| 3-1259 | (5-Methyl-2-oxo-1,3-dioxol-4-yl)-methyl | Me | 4-Br-Ph | H |
| 3-1260 | (5-Methyl-2-oxo-1,3-dioxol-4-yl)-methyl | Me | 5-Cl-pyridin-2-yl | H |
| 3-1261 | (5-Methyl-2-oxo-1,3-dioxol-4-yl)-methyl | Me | 5-Br-pyridin-2-yl | H |
| 3-1262 | (5-Methyl-2-oxo-1,3-dioxol-4-yl)-methyl | Me | 5-Cl-2-thienyl | H |
| 3-1263 | (5-Methyl-2-oxo-1,3-dioxol-4-yl)-methyl | Me | 5-Br-2-thienyl | H |
| 3-1264 | [(t-Butoxycarbonyl)oxy]-methyl | Me | 4-Cl-Ph | H |
| 3-1265 | [(t-Butoxycarbonyl)oxy]-methyl | Me | 4-Br-Ph | H |
| 3-1266 | [(t-Butoxycarbonyl)oxy]-methyl | Me | 5-Cl-pyridin-2-yl | H |
| 3-1267 | [(t-Butoxycarbonyl)oxy]-methyl | Me | 5-Br-pyridin-2-yl | H |
| 3-1268 | [(t-Butoxycarbonyl)oxy]-methyl | Me | 5-Cl-2-thienyl | H |
| 3-1269 | [(t-Butoxycarbonyl)oxy]-methyl | Me | 5-Br-2-thienyl | H |
| 3-1270 | [(Isopropoxycarbonyl)oxy]-methyl | Me | 4-Cl-Ph | H |
| 3-1271 | [(Isopropoxycarbonyl)oxy]-methyl | Me | 4-Br-Ph | H |
| 3-1272 | [(Isopropoxycarbonyl)oxy]-methyl | Me | 5-Cl-pyridin-2-yl | H |
| 3-1273 | [(Isopropoxycarbonyl)oxy]-methyl | Me | 5-Br-pyridin-2-yl | H |
| 3-1274 | [(Isopropoxycarbonyl)oxy]-methyl | Me | 5-Cl-2-thienyl | H |
| 3-1275 | [(Isopropoxycarbonyl)oxy]-methyl | Me | 5-Br-2-thienyl | H |
| 3-1276 | [(Methoxycarbonyl)oxy]-methyl | Me | 4-Cl-Ph | H |
| 3-1277 | [(Methoxycarbonyl)oxy]-methyl | Me | 4-Br-Ph | H |
| 3-1278 | [(Methoxycarbonyl)oxy]-methyl | Me | 5-Cl-pyridin-2-yl | H |
| 3-1279 | [(Methoxycarbonyl)oxy]-methyl | Me | 5-Br-pyridin-2-yl | H |
| 3-1280 | [(Methoxycarbonyl)oxy]-methyl | Me | 5-Cl-2-thienyl | H |
| 3-1281 | [(Methoxycarbonyl)oxy]-methyl | Me | 5-Br-2-thienyl | H |
| 3-1282 | 1-[(Ethoxycarbonyl)oxy]-ethyl | Me | 4-Cl-Ph | H |
| 3-1283 | 1-[(Ethoxycarbonyl)oxy]-ethyl | Me | 4-Br-Ph | H |
| 3-1284 | 1-[(Ethoxycarbonyl)oxy]-ethyl | Me | 5-Cl-pyridin-2-yl | H |
| 3-1285 | 1-[(Ethoxycarbonyl)oxy]-ethyl | Me | 5-Br-pyridin-2-yl | H |
| 3-1286 | 1-[(Ethoxycarbonyl)oxy]-ethyl | Me | 5-Cl-2-thienyl | H |
| 3-1287 | 1-[(Ethoxycarbonyl)oxy]-ethyl | Me | 5-Br-2-thienyl | H |
| 3-1288 | 1-Acetoxy-eth-1-yl | Me | 4-Cl-Ph | H |
| 3-1289 | 1-Acetoxy-eth-1-yl | Me | 4-Br-Ph | H |
| 3-1290 | 1-Acetoxy-eth-1-yl | Me | 5-Cl-pyridin-2-yl | H |
| 3-1291 | 1-Acetoxy-eth-1-yl | Me | 5-Br-pyridin-2-yl | H |
| 3-1292 | 1-Acetoxy-eth-1-yl | Me | 5-Cl-2-thienyl | H |
| 3-1293 | 1-Acetoxy-eth-1-yl | Me | 5-Br-2-thienyl | H |
| 3-1294 | 1-(2-Methylpropanoyloxy)-eth-1-yl | Me | 4-Cl-Ph | H |
| 3-1295 | 1-(2-Methylpropanoyloxy)-eth-1-yl | Me | 4-Br-Ph | H |
| 3-1296 | 1-(2-Methylpropanoyloxy)-eth-1-yl | Me | 5-Cl-pyridin-2-yl | H |
| 3-1297 | 1-(2-Methylpropanoyloxy)-eth-1-yl | Me | 5-Br-pyridin-2-yl | H |
| 3-1298 | 1-(2-Methylpropanoyloxy)-eth-1-yl | Me | 5-Cl-2-thienyl | H |
| 3-1299 | 1-(2-Methylpropanoyloxy)-eth-1-yl | Me | 5-Br-2-thienyl | H |
| 3-1300 | 1-Propanoyl-2-methyl-prop-1-yl | Me | 4-Cl-Ph | H |
| 3-1301 | 1-Propanoyl-2-methyl-prop-1-yl | Me | 4-Br-Ph | H |
| 3-1302 | 1-Propanoyl-2-methyl-prop-1-yl | Me | 5-Cl-pyridin-2-yl | H |
| 3-1303 | 1-Propanoyl-2-methyl-prop-1-yl | Me | 5-Br-pyridin-2-yl | H |
| 3-1304 | 1-Propanoyl-2-methyl-prop-1-yl | Me | 5-Cl-2-thienyl | H |
| 3-1305 | 1-Propanoyl-2-methyl-prop-1-yl | Me | 5-Br-2-thienyl | H |
| 3-1306 | 1-(Cyclohexoxycarbonyloxy)-eth-1-yl | Me | 4-Cl-Ph | H |
| 3-1307 | 1-(Cyclohexoxycarbonyloxy)-eth-1-yl | Me | 4-Br-Ph | H |
| 3-1308 | 1-(Cyclohexoxycarbonyloxy)-eth-1-yl | Me | 5-Cl-pyridin-2-yl | H |
| 3-1309 | 1-(Cyclohexoxycarbonyloxy)-eth-1-yl | Me | 5-Br-pyridin-2-yl | H |
| 3-1310 | 1-(Cyclohexoxycarbonyloxy)-eth-1-yl | Me | 5-Cl-2-thienyl | H |
| 3-1311 | 1-(Cyclohexoxycarbonyloxy)-eth-1-yl | Me | 5-Br-2-thienyl | H |
| 3-1312 | Cyclobutyl | Me | 4-Cl-Ph | H |
| 3-1313 | Cyclobutyl | Me | 4-Br-Ph | H |
| 3-1314 | Cyclobutyl | Me | 5-Cl-pyridin-2-yl | H |
| 3-1315 | Cyclobutyl | Me | 5-Br-pyridin-2-yl | H |
| 3-1316 | Cyclobutyl | Me | 5-Cl-2-thienyl | H |
| 3-1317 | Cyclobutyl | Me | 5-Br-2-thienyl | H |
| 3-1318 | CH₂(4-Me-Ph) | Me | 4-Cl-Ph | H |
| 3-1319 | CH₂(4-Me-Ph) | Me | 4-Br-Ph | H |
| 3-1320 | CH₂(4-Me-Ph) | Me | 5-Cl-pyridin-2-yl | H |
| 3-1321 | CH₂(4-Me-Ph) | Me | 5-Br-pyridin-2-yl | H |
| 3-1322 | CH₂(4-Me-Ph) | Me | 5-Cl-2-thienyl | H |
| 3-1323 | CH₂(4-Me-Ph) | Me | 5-Br-2-thienyl | H |
| 3-1324 | CHMe(4-Cl-Ph) | Me | 4-Cl-Ph | H |
| 3-1325 | CHMe(4-Cl-Ph) | Me | 4-Br-Ph | H |
| 3-1326 | CHMe(4-Cl-Ph) | Me | 5-Cl-pyridin-2-yl | H |
| 3-1327 | CHMe(4-Cl-Ph) | Me | 5-Br-pyridin-2-yl | H |
| 3-1328 | CHMe(4-Cl-Ph) | Me | 5-Cl-2-thienyl | H |
| 3-1329 | CHMe(4-Cl-Ph) | Me | 5-Br-2-thienyl | H |
| 3-1330 | CHMePh | Me | 4-Cl-Ph | H |
| 3-1331 | CHMePh | Me | 4-Br-Ph | H |
| 3-1332 | CHMePh | Me | 5-Cl-pyridin-2-yl | H |
| 3-1333 | CHMePh | Me | 5-Br-pyridin-2-yl | H |
| 3-1334 | CHMePh | Me | 5-Cl-2-thienyl | H |
| 3-1335 | CHMePh | Me | 5-Br-2-thienyl | H |
| 3-1336 | 1,1,1-Trifluorpropan-2-yl | Me | 4-Cl-Ph | H |
| 3-1337 | 1,1,1-Trifluorpropan-2-yl | Me | 4-Br-Ph | H |
| 3-1338 | 1,1,1-Trifluorpropan-2-yl | Me | 5-Cl-pyridin-2-yl | H |
| 3-1339 | 1,1,1-Trifluorpropan-2-yl | Me | 5-Br-pyridin-2-yl | H |
| 3-1340 | 1,1,1-Trifluorpropan-2-yl | Me | 5-Cl-2-thienyl | H |
| 3-1341 | 1,1,1-Trifluorpropan-2-yl | Me | 5-Br-2-thienyl | H |
| 3-1342 | (1-Ethyl-3-methyl-1 H-pyrazol-4-yl)methyl | Me | 4-Cl-Ph | H |
| 3-1343 | (1-Ethyl-3-methyl-1 H-pyrazol-4-yl)-methyl | Me | 4-Br-Ph | H |
| 3-1344 | (1-Ethyl-3-methyl-1 H-pyrazol-4-yl)-methyl | Me | 5-Cl-pyridin-2-yl | H |
| 3-1345 | (1-Ethyl-3-methyl-1 H-pyrazol-4-yl)-methyl | Me | 5-Br-pyridin-2-yl | H |
| 3-1346 | (1-Ethyl-3-methyl-1 H-pyrazol-4-yl)-methyl | Me | 5-Cl-2-thienyl | H |
| 3-1347 | (1-Ethyl-3-methyl-1 H-pyrazol-4-yl)-methyl | Me | 5-Br-2-thienyl | H |
| 3-1348 | Pr | Me | 4-Cl-Ph | H |
| 3-1349 | Pr | Me | 4-Br-Ph | H |
| 3-1350 | Pr | Me | 5-Cl-pyridin-2-yl | H |
| 3-1351 | Pr | Me | 5-Br-pyridin-2-yl | H |
| 3-1352 | Pr | Me | 5-Cl-2-thienyl | H |
| 3-1353 | Pr | Me | 5-Br-2-thienyl | H |
| 3-1354 | n-Octadecyl | Me | 4-Cl-Ph | H |
| 3-1355 | n-Octadecyl | Me | 4-Br-Ph | H |
| 3-1356 | n-Octadecyl | Me | 5-Cl-pyridin-2-yl | H |
| 3-1357 | n-Octadecyl | Me | 5-Br-pyridin-2-yl | H |
| 3-1358 | n-Octadecyl | Me | 5-Cl-2-thienyl | H |
| 3-1359 | n-Octadecyl | Me | 5-Br-2-thienyl | H |
| 3-1360 | n-Hexadecyl | Me | 4-Cl-Ph | H |
| 3-1361 | n-Hexadecyl | Me | 4-Br-Ph | H |
| 3-1362 | n-Hexadecyl | Me | 5-Cl-pyridin-2-yl | H |
| 3-1363 | n-Hexadecyl | Me | 5-Br-pyridin-2-yl | H |
| 3-1364 | n-Hexadecyl | Me | 5-Cl-2-thienyl | H |
| 3-1365 | n-Hexadecyl | Me | 5-Br-2-thienyl | H |
| 3-1366 | Oxetan-3-yl-methyl | Me | 4-Cl-Ph | H |
| 3-1367 | Oxetan-3-yl-methyl | Me | 4-Br-Ph | H |
| 3-1368 | Oxetan-3-yl-methyl | Me | 5-Cl-pyridin-2-yl | H |
| 3-1369 | Oxetan-3-yl-methyl | Me | 5-Br-pyridin-2-yl | H |
| 3-1370 | Oxetan-3-yl-methyl | Me | 5-Cl-2-thienyl | H |
| 3-1371 | Oxetan-3-yl-methyl | Me | 5-Br-2-thienyl | H |
| 3-1372 | 3-Methyloxetan-3-yl | Me | 4-Cl-Ph | H |
| 3-1373 | 3-Methyloxetan-3-yl | Me | 4-Br-Ph | H |
| 3-1374 | 3-Methyloxetan-3-yl | Me | 5-Cl-pyridin-2-yl | H |
| 3-1375 | 3-Methyloxetan-3-yl | Me | 5-Br-pyridin-2-yl | H |
| 3-1376 | 3-Methyloxetan-3-yl | Me | 5-Cl-2-thienyl | H |
| 3-1377 | 3-Methyloxetan-3-yl | Me | 5-Br-2-thienyl | H |
| 3-1378 | 2-Chlorprop-2-en-1-yl | Me | 4-Cl-Ph | H |
| 3-1379 | 2-Chlorprop-2-en-1-yl | Me | 4-Br-Ph | H |
| 3-1380 | 2-Chlorprop-2-en-1-yl | Me | 5-Cl-pyridin-2-yl | H |
| 3-1381 | 2-Chlorprop-2-en-1-yl | Me | 5-Br-pyridin-2-yl | H |
| 3-1382 | 2-Chlorprop-2-en-1-yl | Me | 5-Cl-2-thienyl | H |
| 3-1383 | 2-Chlorprop-2-en-1-yl | Me | 5-Br-2-thienyl | H |
| 3-1384 | (3E)-Pent-3-en-2-yl | Me | 4-Cl-Ph | H |
| 3-1385 | (3E)-Pent-3-en-2-yl | Me | 4-Br-Ph | H |
| 3-1386 | (3E)-Pent-3-en-2-yl | Me | 5-Cl-pyridin-2-yl | H |
| 3-1387 | (3E)-Pent-3-en-2-yl | Me | 5-Br-pyridin-2-yl | H |
| 3-1388 | (3E)-Pent-3-en-2-yl | Me | 5-Cl-2-thienyl | H |
| 3-1389 | (3E)-Pent-3-en-2-yl | Me | 5-Br-2-thienyl | H |
| 3-1390 | (2,2-Dimethylpropanoyloxy)-methyl | Me | 4-Cl-Ph | H |
| 3-1391 | (2,2-Dimethylpropanoyloxy)-methyl | Me | 4-Br-Ph | H |
| 3-1392 | (2,2-Dimethylpropanoyloxy)-methyl | Me | 5-Cl-pyridin-2-yl | H |
| 3-1393 | (2,2-Dimethylpropanoyloxy)-methyl | Me | 5-Br-pyridin-2-yl | H |
| 3-1394 | (2,2-Dimethylpropanoyloxy)-methyl | Me | 5-Cl-2-thienyl | H |
| 3-1395 | (2,2-Dimethylpropanoyloxy)-methyl | Me | 5-Br-2-thienyl | H |
| 3-1396 | 2-(Isopropoxycarbonyloxy)-eth-1-yl | Me | 4-Cl-Ph | H |
| 3-1397 | 2-(Isopropoxycarbonyloxy)-eth-1-yl | Me | 4-Br-Ph | H |
| 3-1398 | 2-(Isopropoxycarbonyloxy)-eth-1-yl | Me | 5-Cl-pyridin-2-yl | H |
| 3-1399 | 2-(Isopropoxycarbonyloxy)-eth-1-yl | Me | 5-Br-pyridin-2-yl | H |
| 3-1400 | 2-(Isopropoxycarbonyloxy)-eth-1-yl | Me | 5-Cl-2-thienyl | H |
| 3-1401 | 2-(Isopropoxycarbonyloxy)-eth-1-yl | Me | 5-Br-2-thienyl | H |

**Tabelle 4: Verbindungen der Formel (Ib")**

| | | | | | |
|---|---|---|---|---|---|
| 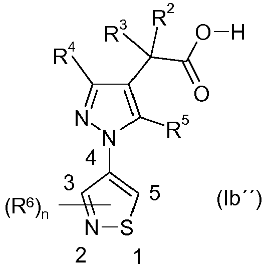 | | | | | |

| Nr. | R² | R³ | R⁴ | R⁵ | (R⁶)ₙ |
|---|---|---|---|---|---|
| 4-1 | H | H | Ph | Ph | H |
| 4-2 | H | H | Me | Ph | H |
| 4-3 | H | H | Me | 5-J-2-thienyl | H |
| 4-4 | H | H | Me | 2-Furyl | H |
| 4-5 | H | H | Me | Ph | 3-OMe |
| 4-6 | Me | H | Me | Ph | 5-Me |
| 4-7 | H | H | Me | Ph | 3-CI |
| 4-8 | H | H | Me | Ph | 5-CF₃ |
| 4-9 | H | H | Me | Ph | 3-CF₃ |
| 4-10 | H | H | Me | Ph | 5-Me |
| 4-11 | H | H | Me | Ph | 3,5-Me₂ |
| 4-12 | H | H | Me | Ph | 3,5-Cl₂ |
| 4-13 | H | H | Me | 4-MeO-Ph | 5-Me |
| 4-14 | H | H | Me | 4-MeO-Ph | H |
| 4-15 | Me | H | Me | Ph | H |
| 4-16 | H | H | Me | 4-Me-Ph | 5-Me |
| 4-17 | H | H | Me | 4-Me-Ph | 5-CI |
| 4-18 | H | H | Me | 4-Me-Ph | H |
| 4-19 | H | H | Me | 3-CI-Ph | H |
| 4-20 | H | H | Me | 3-CF₃-Ph | H |
| 4-21 | H | H | Me | 3-CF₃-Ph | 4-Me |
| 4-22 | H | H | Me | 3,4-Cl₂-Ph | 5-Me |
| 4-23 | H | H | Me | 3-Cl-Ph | 5-Me |
| 4-24 | H | H | Me | 2-Cl-Ph | 5-Me |
| 4-25 | H | H | Me | 2,4-Cl₂-Ph | 5-Me |
| 4-26 | H | H | Me | 4-CF₃-Ph | 5-Me |
| 4-27 | H | H | Me | 4-Cl-Ph | 5-Me |
| 4-28 | H | H | Me | 4-Cl-Ph | H |
| 4-29 | H | H | Me | 3,4-Cl₂-Ph | H |
| 4-30 | H | H | Me | 4-CF₃-Ph | H |
| 4-31 | H | H | Me | 4-Cl-Ph | 5-Cl |
| 4-32 | H | H | Me | Ph | 5-Cl |
| 4-33 | H | H | Me | 2-Cl-Ph | H |
| 4-34 | H | H | Me | 4-tBu-Ph | 5-Me |
| 4-35 | H | H | Me | 3,5-Me₂-Ph | 5-Me |
| 4-36 | H | H | Me | Ph | 5-OMe |
| 4-37 | H | H | Me | 4-Cl-Ph | 5-OMe |
| 4-38 | H | H | Me | 4-Me-Ph | 5-Me |
| 4-39 | H | H | Me | 4-F-Ph | 5-CI |
| 4-40 | H | H | Me | 4-F-Ph | 5-Me |
| 4-41 | H | H | Me | 3-Me-Ph | 5-Me |
| 4-42 | H | H | Me | 4-(COOH)-Ph | 5-Me |
| 4-43 | H | H | Me | 3-Br-Ph | 5-Me |
| 4-44 | H | H | Me | 4-Ph-Ph | 5-Me |
| 4-45 | H | H | Me | 4-(COOH)-Ph | H |
| 4-46 | H | H | Me | 3,5-Me₂-Ph | H |
| 4-47 | H | H | Me | Ph | 5-SMe |
| 4-48 | H | H | Me | 4-Cl-Ph | 5-SMe |
| 4-49 | H | H | Me | 3-Cl-4-Me-Ph | H |
| 4-50 | H | H | Me | 3-CF₃-4-Cl-Ph | H |
| 4-51 | H | H | Me | 3-CF₃-4-Cl-Ph | 5-Me |
| 4-52 | H | H | Me | 3-Cl-4-Me-Ph | 5-Me |
| 4-53 | H | H | Me | 2-Pyridyl | 5-Cl |
| 4-54 | H | H | Me | 4-Cl-Ph | 5-F |
| 4-55 | H | H | Me | 2-Thienyl | 5-Me |
| 4-56 | H | H | Me | 3-Me-2-thienyl | 5-Me |
| 4-57 | H | H | Me | 4-Me-2-thienyl | 5-Me |
| 4-58 | H | H | Me | 5-Cl-2-thienyl | 5-Me |
| 4-59 | H | H | Me | 5-Cl-2-thienyl | 5-CI |
| 4-60 | H | H | Me | 3-Thienyl | 5-Me |
| 4-61 | H | H | Me | 2-Thienyl | H |
| 4-62 | H | H | Me | 3-Me-2-thienyl | H |
| 4-63 | H | H | Me | 4-Me-2-thienyl | H |
| 4-64 | H | H | Me | 5-Cl-2-thienyl | H |
| 4-65 | H | H | Me | 5-Me-2-thienyl | H |
| 4-66 | H | H | Me | 6-MeO-pyridin-3-yl | H |
| 4-67 | H | H | Me | 5-Br-2-thienyl | H |
| 4-68 | H | H | Me | 5-Br-2-thienyl | 5-Me |
| 4-69 | H | H | Me | 3-Thienyl | H |
| 4-70 | H | H | Me | 4-CI-Ph | 5-S(O)Me |
| 4-71 | H | H | Me | 4-Br-Ph | 5-Me |
| 4-72 | H | H | Me | 1,3-Benzodioxol-5-yl | 5-Me |
| 4-73 | H | H | Me | 4-J-Ph | 5-Me |
| 4-74 | H | H | Me | 3,5-Cl₂-Ph | 5-Me |
| 4-75 | H | H | Me | 4-PhO-Ph | 5-Me |
| 4-76 | H | H | Me | 6-OH-pyridin-3-yl | H |
| 4-77 | H | H | Me | Ph | 5-S(O)Me |
| 4-78 | H | H | H | Ph | H |
| 4-79 | H | H | H | Ph | 5-Me |
| 4-80 | H | H | Et | Ph | H |
| 4-81 | H | H | n-Pr | Ph | H |
| 4-82 | H | H | CH₂Cl | Ph | H |
| 4-83 | H | H | CHCl₂ | Ph | H |
| 4-84 | H | H | CH₂F | Ph | H |
| 4-85 | H | H | CHF₂ | Ph | H |
| 4-86 | H | H | Cl | Ph | H |
| 4-87 | H | H | Et | Ph | 5-Me |
| 4-88 | H | H | n-Pr | Ph | 5-Me |
| 4-89 | H | H | CH₂Cl | Ph | 5-Me |
| 4-90 | H | H | CHCl₂ | Ph | 5-Me |
| 4-91 | H | H | CH₂F | Ph | 5-Me |
| 4-92 | H | H | CHF₂ | Ph | 5-Me |
| 4-93 | H | H | Cl | Ph | 5-Me |
| 4-94 | H | H | Et | 4-Cl-Ph | H |
| 4-95 | H | H | n-Pr | 4-Cl-Ph | H |
| 4-96 | H | H | CH₂Cl | 4-Cl-Ph | H |
| 4-97 | H | H | CHCl₂ | 4-Cl-Ph | H |
| 4-98 | H | H | CH₂F | 4-Cl-Ph | H |
| 4-99 | H | H | CHF₂ | 4-Cl-Ph | H |
| 4-100 | H | H | Cl | 4-Cl-Ph | H |
| 4-101 | H | H | Et | 4-Me-Ph | H |
| 4-102 | H | H | n-Pr | 4-Me-Ph | H |
| 4-103 | H | H | CH₂Cl | 4-Me-Ph | H |
| 4-104 | H | H | CHl₂ | 4-Me-Ph | H |
| 4-105 | H | H | CH₂F | 4-Me-Ph | H |
| 4-106 | H | H | CHF₂ | 4-Me-Ph | H |
| 4-107 | H | H | Cl | 4-Me-Ph | H |
| 4-108 | H | H | Et | 2-Pyridyl | H |
| 4-109 | H | H | n-Pr | 2-Pyridyl | H |
| 4-110 | H | H | CH₂Cl | 2-Pyridyl | H |
| 4-111 | H | H | CHCl₂ | 2-Pyridyl | H |
| 4-112 | H | H | CH₂F | 2-Pyridyl | H |
| 4-113 | H | H | CHF₂ | 2-Pyridyl | H |
| 4-114 | H | H | Cl | 2-Pyridyl | H |
| 4-115 | H | H | Me | 2-Pyridyl | H |
| 4-116 | H | H | Me | 5-Cl-pyridin-2-yl | H |
| 4-117 | H | H | Me | 5-Cl-pyridin-2-yl | 5-Cl |
| 4-118 | H | H | Me | 5-Cl-pyridin-2-yl | 5-Me |
| 4-119 | H | H | Me | 5-Br-pyridin-2-yl | H |
| 4-120 | H | H | Me | 5-Br-pyridin-2-yl | 5-Cl |
| 4-121 | H | H | Me | 5-Br-pyridin-2-yl | 5-Me |
| 4-122 | H | H | Me | 5-F-pyridin-2-yl | H |
| 4-123 | H | H | Me | 5-Me-pyridin-2-yl | H |
| 4-124 | H | H | Me | 5-Me-pyridin-2-yl | 5-Me |
| 4-125 | H | H | Me | 2,4-Cl₂-Ph | H |
| 4-126 | H | H | Me | 4-CH₂COOH-Ph | 5-Me |
| 4-127 | H | H | Me | 3,4-Me₂-Ph | 5-Me |
| 4-128 | H | H | Me | 4-Br-Ph | H |
| 4-129 | H | H | Me | 3,4-Me₂-Ph | H |
| 4-130 | H | H | Me | 3-Me-Ph | H |
| 4-131 | H | H | Me | 4-F-Ph | H |
| 4-132 | H | H | Me | 4-(Me-CO)-Ph | H |
| 4-133 | H | H | Me | 4-tBu-Ph | H |
| 4-134 | H | H | Me | 4-Cl-3-Me-Ph | H |
| 4-135 | H | H | n-Pr | 4-Cl-Ph | 5-Me |
| 4-136 | H | H | Me | 3-Pyridyl | H |
| 4-137 | H | H | Me | 4-Pyridyl | H |
| 4-138 | H | H | C(O)OMe | Ph | H |
| 4-139 | H | H | Me | 6-Me-pyridin-3-yl | H |
| 4-140 | H | H | Me | 4-Cl-Ph | 5-SO₂Me |
| 4-141 | H | H | Me | 3-Pyridyl | 5-Me |
| 4-142 | H | H | Me | 2,3-Cl₂-Ph | 5-Me |
| 4-143 | H | H | Me | 2-Pyridyl | 5-Me |
| 4-144 | H | H | H | 4-Cl-Ph | 5-Me |
| 4-145 | H | H | Me | 6-Cl-pyridin-3-yl | H |
| 4-146 | H | H | Me | Ph | 5-Me |
| 4-147 | H | H | Me | 4-Me-pyridin-2-yl | H |
| 4-148 | H | H | Me | 4-Me-pyridin-2-yl | 5-Me |
| 4-149 | H | H | Me | 4-Me-pyridin-2-yl | 5-Cl |
| 4-150 | H | H | Me | 4-Me-pyridin-2-yl | 5-F |
| 4-151 | H | H | Me | 4-F-pyridin-2-yl | H |
| 4-152 | H | H | Me | 4-Cl-pyridin-2-yl | H |
| 4-153 | H | H | Me | 4-Br-pyridin-2-yl | H |
| 4-154 | H | H | Me | 4-OMe-pyridin-2-yl | H |
| 4-155 | H | H | Me | 5-CF₃-pyridin-2-yl | H |
| 4-156 | H | H | Me | 6-OMe-pyridin-2-yl | H |
| 4-157 | H | H | cyPr | 4-Cl-Ph | H |
| 4-158 | H | H | CN | 4-Cl-Ph | H |
| 4-159 | H | H | CN | 4-Cl-Ph | 5-Me |
| 4-160 | H | H | CN | 4-Me-Ph | H |
| 4-161 | H | H | CN | 4-Me-Ph | 5-Me |
| 4-162 | H | H | CN | Ph | H |
| 4-163 | H | H | CN | Ph | 5-Me |
| 4-164 | H | H | CN | 2-pyridyl | H |
| 4-165 | H | H | CN | 3-pyridyl | H |
| 4-166 | H | H | CN | 5-Cl-pyridin-2-yl | H |
| 4-167 | H | H | CN | 5-Br-pyridin-2-yl | H |
| 4-168 | H | H | CN | 5-F-pyridin-2-yl | H |
| 4-169 | H | H | CN | 5-Me-pyridin-2-yl | H |
| 4-170 | H | H | CN | 6-Me-pyridin-3-yl | H |
| 4-171 | H | H | CN | 4-Me-pyridin-2-yl | H |
| 4-172 | H | H | CN | 4-F-pyridin-2-yl | H |
| 4-173 | H | H | CN | 4-Cl-pyridin-2-yl | H |
| 4-174 | H | H | CN | 4-Br-pyridin-2-yl | H |
| 4-175 | H | H | CN | 4-OMe-pyridin-2-yl | H |
| 4-176 | H | H | formyl | 4-Cl-Ph | H |
| 4-177 | H | H | formyl | 4-Cl-Ph | 5-Me |
| 4-178 | H | H | formyl | 4-Me-Ph | H |
| 4-179 | H | H | formyl | 4-Me-Ph | 5-Me |
| 4-180 | H | H | formyl | Ph | H |
| 4-181 | H | H | formyl | Ph | 5-Me |
| 4-182 | H | H | formyl | 2-pyridyl | H |
| 4-183 | H | H | formyl | 3-pyridyl | H |
| 4-184 | H | H | formyl | 5-Cl-pyridin-2-yl | H |
| 4-185 | H | H | formyl | 5-Br-pyridin-2-yl | H |
| 4-186 | H | H | formyl | 5-F-pyridin-2-yl | H |
| 4-187 | H | H | formyl | 5-Me-pyridin-2-yl | H |
| 4-188 | H | H | formyl | 6-Me-pyridin-3-yl | H |
| 4-189 | H | H | formyl | 4-Me-pyridin-2-yl | H |
| 4-190 | H | H | formyl | 4-F-pyridin-2-yl | H |
| 4-191 | H | H | formyl | 4-Cl-pyridin-2-yl | H |
| 4-192 | H | H | formyl | 4-Br-pyridin-2-yl | H |
| 4-193 | H | H | formyl | 4-OMe-pyridin-2-yl | H |
| 4-194 | H | H | CH₂OH | 5-Me-pyridin-2-yl | H |
| 4-195 | H | H | CH₂OH | 4-Cl-Ph | H |
| 4-196 | H | H | CH₂OH | 4-Me-pyridin-2-yl | H |
| 4-197 | H | H | CH₂OH | 4-Me-Ph | H |
| 4-198 | H | H | CH₂OH | Ph | H |
| 4-199 | H | H | CH₂OH | 2-Pyridyl | H |
| 4-200 | H | H | Me | 2-Thiazolyl | H |
| 4-201 | H | H | Me | 2-Thiazolyl | 5-Cl |
| 4-202 | H | H | Me | 2-Thiazolyl | 5-Me |
| 4-203 | H | H | Me | 4-Me-thiazol-2-yl | H |
| 4-204 | H | H | Me | 4-Me-thiazol-2-yl | 5-Cl |
| 4-205 | H | H | Me | 4-Me-thiazol-2-yl | 5-Me |
| 4-206 | H | H | Me | 5-Me-thiazol-2-yl | H |
| 4-207 | H | H | Me | 5-Br-thiazol-2-yl | H |
| 4-208 | H | H | Me | 5-Br-thiazol-2-yl | 5-Me |
| 4-209 | H | H | Me | 5-Cl-thiazol-2-yl | H |
| 4-210 | H | H | Me | 4,6-Me₂-pyridin-2-yl | H |
| 4-211 | H | H | Me | 4,6-Me₂-pyridin-2-yl | 5-Me |
| 4-212 | H | H | Me | 2-Pyridyl | 5-F |
| 4-213 | H | H | Me | 2-Pyrazinyl | H |
| 4-214 | H | H | Me | 5-Me-Pyrazin-2-yl | H |
| 4-215 | H | H | Me | 2-Pyrazinyl | 5-Me |
| 4-216 | H | H | Me | 1,3-Benzothiazol-2-yl | H |
| 4-217 | H | H | Me | 1,3-Benzothiazol-2-yl | 5-Me |
| 4-218 | H | H | Me | 7-Cl-1,3-benzothiazol-2-yl | H |
| 4-219 | H | H | Me | 1,5-Me₂-pyrazol-3-yl | H |
| 4-220 | H | H | Me | 1,5-Me₂-pyrazol-3-yl | 5-Me |
| 4-221 | H | H | Me | 4,5-Me₂-thiazol-2-yl | H |
| 4-222 | H | H | Me | 4,5-Cl₂-thiazol-2-yl | H |
| 4-223 | H | H | Me | 2-Pyrimidinyl | H |
| 4-224 | H | H | Me | 2-Pyrimidinyl | 5-Me |
| 4-225 | H | H | Me | 5-F-pyrimidin-2-yl | H |
| 4-226 | H | H | Me | 5-Cl-pyrimidin-2-yl | H |
| 4-227 | H | H | Me | 5-Br-pyrimidin-2-yl | H |
| 4-228 | H | H | Me | 5-Me-pyrimidin-2-yl | H |
| 4-229 | H | H | Me | 5-Me-pyrimidin-2-yl | 5-Me |
| 4-230 | H | H | Me | 4,6-Me₂-pyrimidin-2-yl | H |
| 4-231 | H | H | Me | 4,6-Me₂-pyrimidin-2-yl | 5-Me |
| 4-232 | H | H | Me | 3-Pyridazinyl | H |
| 4-233 | H | H | Me | 6-Me-Pyridazin-3-yl | H |
| 4-234 | H | H | Me | 1,2,4-Triazin-3-yl | H |
| 4-235 | H | H | Me | 6-Me-1,2,4-triazin-3-yl | H |
| 4-236 | H | H | Me | 4-Cl-Ph | 3-COOMe |
| 4-237 | H | H | Me | 4-Cl-Ph | 3,5-(COOMe)₂ |
| 4-238 | H | H | Me | Chinolin-2-yl | H |
| 4-239 | H | H | Me | Isochinolin-3-yl | H |
| 4-240 | H | H | Me | 4-NO₂-Ph | H |
| 4-241 | H | H | Me | 3,5-Cl₂-Ph | H |
| 4-242 | H | H | Me | 2-Me-pyridin-4-yl | H |
| 4-243 | H | H | Me | 4-Cl-6-Me-pyridin-2-yl | H |
| 4-244 | H | H | Me | 4-Br-3-Me-Ph | H |
| 4-245 | H | H | Me | 5-Cl-pyridin-3-yl | H |
| 4-246 | H | H | Me | 5-Allyl-pyridin-2-yl | H |
| 4-247 | H | H | Me | 5-Cyclopropyl-pyridin-2-yl | H |
| 4-248 | H | H | Me | 5-Ethinyl-pyridin-2-yl | H |
| 4-249 | H | H | Me | 5-Ph-pyridin-2-yl | H |
| 4-250 | H | H | Me | 5-OH-pyridin-2-yl | H |
| 4-251 | H | H | Me | 5-OCHF₂-pyridin-2-yl | H |
| 4-252 | H | H | Me | 5-MeO-pyridin-2-yl | H |
| 4-253 | H | H | Me | 5-MeS-pyridin-2-yl | H |
| 4-254 | H | H | Me | 5-NHMe-pyridin-2-yl | H |
| 4-255 | H | H | Me | 5-NMe₂-pyridin-2-yl | H |
| 4-256 | H | H | Me | 6-Cl-1,3-benzoxazol-2-yl | H |
| 4-257 | H | H | Me | 6-Br-1,3-benzoxazol-2-yl | H |
| 4-258 | H | H | Me | 7-Cl-1,3-benzoxazol-2-yl | H |
| 4-259 | H | H | Me | 5-NH₂-pyridin-2-yl | H |
| 4-260 | H | H | Me | 2-Cl-thiazol-4-yl | H |
| 4-261 | H | H | Me | 2-Br-thiazol-4-yl | H |
| 4-262 | H | H | Me | 5-OSO₂Me-pyridin-2-yl | H |
| 4-263 | H | H | Me | 6-Cl-1,3-benzothiazol-2-yl | H |
| 4-264 | H | H | Me | 6-Br-1,3-benzothiazol-2-yl | H |
| 4-265 | H | H | Me | 1,3-Benzoxazol-2-yl | H |
| 4-266 | H | H | Me | 4-PhO-Ph | H |
| 4-267 | H | H | Me | 4-CH₂COOH-Ph | H |
| 4-268 | H | H | Me | 2,3-Cl₂-Ph | H |
| 4-269 | H | H | Me | 5-I-pyridin-2-yl | H |
| 4-270 | H | H | Me | 5-I-pyrimidin-2-yl | H |
| 4-271 | H | H | Me | 3,4-F₂-Ph | H |
| 4-272 | H | H | Me | 1-Me-pyrazol-3-yl | H |
| 4-273 | H | H | Me | 1-Me-pyrazol-5-yl | H |
| 4-274 | H | H | Me | 3-Br-Ph | H |
| 4-275 | H | H | Me | 4-Ph-Ph | H |
| 4-276 | H | H | Me | 1,3-Benzodioxol-5-yl | H |
| 4-277 | H | H | Me | 4-J-Ph | H |
| 4-278 | H | H | Me | 5-Br-3-thienyl | H |
| 4-279 | H | H | Me | 5-Me-3-thienyl | H |
| 4-280 | H | H | Me | 2-F-Ph | H |
| 4-281 | H | H | Me | 2-CN-Ph | H |
| 4-282 | H | H | Me | 2-NO₂-Ph | H |
| 4-283 | H | H | Me | 2,4-F₂-Ph | H |
| 4-284 | H | H | Me | 5-Thiazolyl | H |
| 4-285 | H | H | Me | 2-Me-thiazol-4-yl | H |
| 4-286 | H | H | Me | 2-Me-thiazol-5-yl | H |
| 4-287 | H | H | Me | 5-Cl-3-thienyl | H |
| 4-288 | H | H | Me | 6-Br-pyridin-3-yl | H |
| 4-289 | H | H | Me | 4-Cl-3-thienyl | H |
| 4-290 | H | H | Me | 4-Br-3-thienyl | H |
| 4-291 | H | H | Me | 4-Me-3-thienyl | H |
| 4-292 | H | H | Me | 4-Thiazolyl | H |
| 4-293 | H | H | Me | 4-Me-5-Cl-pyridin-2-yl | H |

**Tabelle 5: Verbindungen der Formel (Ib''')**

| | | | | | |
|---|---|---|---|---|---|
| 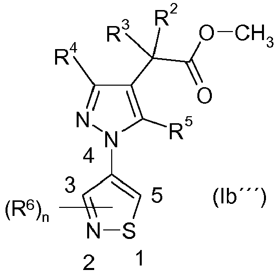 | | | | | |

| Nr. | R² | R³ | R⁴ | R⁵ | (R⁶)ₙ |
|---|---|---|---|---|---|
| 5-1 | H | H | Ph | Ph | H |
| 5-2 | H | H | Me | Ph | H |
| 5-3 | H | H | Me | 5-J-2-thienyl | H |
| 5-4 | H | H | Me | 2-Furyl | H |
| 5-5 | H | H | Me | Ph | 3-OMe |
| 5-6 | Me | H | Me | Ph | 5-Me |
| 5-7 | H | H | Me | Ph | 3-CI |
| 5-8 | H | H | Me | Ph | 5-CF₃ |
| 5-9 | H | H | Me | Ph | 3-CF₃ |
| 5-10 | H | H | Me | Ph | 5-Me |
| 5-11 | H | H | Me | Ph | 3,5-Me₂ |
| 5-12 | H | H | Me | Ph | 3,5-Cl₂ |
| 5-13 | H | H | Me | 4-MeO-Ph | 5-Me |
| 5-14 | H | H | Me | 4-MeO-Ph | H |
| 5-15 | Me | H | Me | Ph | H |
| 5-16 | H | H | Me | 4-Me-Ph | H |
| 5-17 | H | H | Me | 4-Me-Ph | 5-Me |
| 5-18 | H | H | Me | 4-Me-Ph | 5-CI |
| 5-19 | H | H | Me | 3-CI-Ph | H |
| 5-20 | H | H | Me | 3-CF₃-Ph | H |
| 5-21 | H | H | Me | 3-CF₃-Ph | 5-Me |
| 5-22 | H | H | Me | 3,4-Cl₂-Ph | 5-Me |
| 5-23 | H | H | Me | 3-CI-Ph | 5-Me |
| 5-24 | H | H | Me | 2-CI-Ph | 5-Me |
| 5-25 | H | H | Me | 2,4-Cl₂-Ph | 5-Me |
| 5-26 | H | H | Me | 4-CF₃-Ph | 5-Me |
| 5-27 | H | H | Me | 4-CI-Ph | 5-Me |
| 5-28 | H | H | Me | 4-CI-Ph | H |
| 5-29 | H | H | Me | 3,4-Cl₂-Ph | H |
| 5-30 | H | H | Me | 4-CF₃-Ph | H |
| 5-31 | H | H | Me | 4-CI-Ph | 5-CI |
| 5-32 | H | H | Me | Ph | 5-CI |
| 5-33 | H | H | Me | 2-CI-Ph | H |
| 5-34 | H | H | Me | 4-tBu-Ph | 5-Me |
| 5-35 | H | H | Me | 3,5-Me₂-Ph | 5-Me |
| 5-36 | H | H | Me | Ph | 5-OMe |
| 5-37 | H | H | Me | 4-CI-Ph | 5-OMe |
| 5-38 | H | H | Me | 4-Me-Ph | 5-Me |
| 5-39 | H | H | Me | 4-F-Ph | 5-Me |
| 5-40 | H | H | Me | 4-F-Ph | 5-CI |
| 5-41 | H | H | Me | 3-Me-Ph | 5-Me |
| 5-42 | H | H | Me | 4-COOH-Ph | 5-Me |
| 5-43 | H | H | Me | 3-Br-Ph | 5-Me |
| 5-44 | H | H | Me | 4-Ph-Ph | 5-Me |
| 5-45 | H | H | Me | 4-COOH-Ph | H |
| 5-46 | H | H | Me | 3,5-Me₂-Ph | H |
| 5-47 | H | H | Me | Ph | 5-SMe |
| 5-48 | H | H | Me | 4-CI-Ph | 5-SMe |
| 5-49 | H | H | Me | 3-Cl-4-Me-Ph | H |
| 5-50 | H | H | Me | 3-CF₃-4-Cl-Ph | H |
| 5-51 | H | H | Me | 3-CF₃-4-Cl-Ph | 5-Me |
| 5-52 | H | H | Me | 3-Cl-4-Me-Ph | 5-Me |
| 5-53 | H | H | Me | 2-Pyridyl | 5-CI |
| 5-54 | H | H | Me | 4-CI-Ph | 5-F |
| 5-55 | H | H | Me | 2-Thienyl | 5-Me |
| 5-56 | H | H | Me | 3-Me-2-thienyl | 5-Me |
| 5-57 | H | H | Me | 4-Me-2-thienyl | 5-Me |
| 5-58 | H | H | Me | 5-Cl-2-thienyl | 5-Me |
| 5-59 | H | H | Me | 5-Cl-2-thienyl | 5-CI |
| 5-60 | H | H | Me | 3-Thienyl | 5-Me |
| 5-61 | H | H | Me | 2-Thienyl | H |
| 5-62 | H | H | Me | 3-Me-2-thienyl | H |
| 5-63 | H | H | Me | 4-Me-2-thienyl | H |
| 5-64 | H | H | Me | 5-Cl-2-thienyl | H |
| 5-65 | H | H | Me | 5-Me-2-thienyl | H |
| 5-66 | H | H | Me | 6-MeO-pyridin-3-yl | H |
| 5-67 | H | H | Me | 5-Br-2-thienyl | H |
| 5-68 | H | H | Me | 5-Br-2-thienyl | 5-Me |
| 5-69 | H | H | Me | 3-Thienyl | H |
| 5-70 | H | H | Me | 4-CI-Ph | 5-S(O)Me |
| 5-71 | H | H | Me | 4-Br-Ph | 5-Me |
| 5-72 | H | H | Me | 1,3-Benzodioxol-5-yl | 5-Me |
| 5-73 | H | H | Me | 4-J-Ph | 5-Me |
| 5-74 | H | H | Me | 3,5-Cl₂-Ph | 5-Me |
| 5-75 | H | H | Me | 4-PhO-Ph | 5-Me |
| 5-76 | H | H | Me | 6-OH-pyridin-3-yl | H |
| 5-77 | H | H | Me | Ph | 5-S(O)Me |
| 5-78 | H | H | H | Ph | H |
| 5-79 | H | H | H | Ph | 5-Me |
| 5-80 | H | H | Et | Ph | H |
| 5-81 | H | H | n-Pr | Ph | H |
| 5-82 | H | H | CH₂Cl | Ph | H |
| 5-83 | H | H | CHCl₂ | Ph | H |
| 5-84 | H | H | CH₂F | Ph | H |
| 5-85 | H | H | CHF₂ | Ph | H |
| 5-86 | H | H | Cl | Ph | H |
| 5-87 | H | H | Et | Ph | 5-Me |
| 5-88 | H | H | n-Pr | Ph | 5-Me |
| 5-89 | H | H | CH₂Cl | Ph | 5-Me |
| 5-90 | H | H | CHCl₂ | Ph | 5-Me |
| 5-91 | H | H | CH₂F | Ph | 5-Me |
| 5-92 | H | H | CHF₂ | Ph | 5-Me |
| 5-93 | H | H | Cl | Ph | 5-Me |
| 5-94 | H | H | Et | 4-CI-Ph | H |
| 5-95 | H | H | n-Pr | 4-CI-Ph | H |
| 5-96 | H | H | CH₂Cl | 4-CI-Ph | H |
| 5-97 | H | H | CHCl₂ | 4-CI-Ph | H |
| 5-98 | H | H | CH₂F | 4-CI-Ph | H |
| 5-99 | H | H | CHF₂ | 4-CI-Ph | H |
| 5-100 | H | H | Cl | 4-CI-Ph | H |
| 5-101 | H | H | Et | 4-Me-Ph | H |
| 5-102 | H | H | n-Pr | 4-Me-Ph | H |
| 5-103 | H | H | CH₂Cl | 4-Me-Ph | H |
| 5-104 | H | H | CHCl₂ | 4-Me-Ph | H |
| 5-105 | H | H | CH₂F | 4-Me-Ph | H |
| 5-106 | H | H | CHF₂ | 4-Me-Ph | H |
| 5-107 | H | H | Cl | 4-Me-Ph | H |
| 5-108 | H | H | Et | 2-Pyridyl | H |
| 5-109 | H | H | n-Pr | 2-Pyridyl | H |
| 5-110 | H | H | CH₂Cl | 2-Pyridyl | H |
| 5-111 | H | H | CHCl₂ | 2-Pyridyl | H |
| 5-112 | H | H | CH₂F | 2-Pyridyl | H |
| 5-113 | H | H | CHF₂ | 2-Pyridyl | H |
| 5-114 | H | H | Cl | 2-Pyridyl | H |
| 5-115 | H | H | Me | 2-Pyridyl | H |
| 5-116 | H | H | Me | 5-Cl-pyridin-2-yl | H |
| 5-117 | H | H | Me | 5-Cl-pyridin-2-yl | 5-CI |
| 5-118 | H | H | Me | 5-Cl-pyridin-2-yl | 5-Me |
| 5-119 | H | H | Me | 5-Br-pyridin-2-yl | H |
| 5-120 | H | H | Me | 5-Br-pyridin-2-yl | 5-CI |
| 5-121 | H | H | Me | 5-Br-pyridin-2-yl | 5-Me |
| 5-122 | H | H | Me | 5-F-pyridin-2-yl | H |
| 5-123 | H | H | Me | 5-Me-pyridin-2-yl | H |
| 5-124 | H | H | Me | 5-Me-pyridin-2-yl | 5-Me |
| 5-125 | H | H | Me | 2,4-Cl₂-Ph | H |
| 5-126 | H | H | Me | 4-(CH₂COOH)-Ph | 5-Me |
| 5-127 | H | H | Me | 3,4-Me₂-Ph | 5-Me |
| 5-128 | H | H | Me | 4-Br-Ph | H |
| 5-129 | H | H | Me | 3,4-Me₂-Ph | H |
| 5-130 | H | H | Me | 3-Me-Ph | H |
| 5-131 | H | H | Me | 4-F-Ph | H |
| 5-132 | H | H | Me | 4-(Me-CO)-Ph | H |
| 5-133 | H | H | Me | 4-tBu-Ph | H |
| 5-134 | H | H | Me | 4-Cl-3-Me-Ph | H |
| 5-135 | H | H | n-Pr | 4-CI-Ph | 5-Me |
| 5-136 | H | H | Me | 3-Pyridyl | H |
| 5-137 | H | H | Me | 4-Pyridyl | H |
| 5-138 | H | H | C(O)OMe | Ph | H |
| 5-139 | H | H | Me | 6-Me-pyridin-3-yl | H |
| 5-140 | H | H | Me | 4-CI-Ph | 5-SO₂Me |
| 5-141 | H | H | Me | 3-Pyridyl | 5-Me |
| 5-142 | H | H | Me | 2,3-Cl₂-Ph | 5-Me |
| 5-143 | H | H | Me | 2-Pyridyl | 5-Me |
| 5-144 | H | H | H | 4-CI-Ph | 5-Me |
| 5-145 | H | H | Me | 6-Cl-pyridin-3-yl | H |
| 5-146 | H | H | Me | Ph | 5-Me |
| 5-147 | H | H | Me | 4-Me-pyridin-2-yl | H |
| 5-148 | H | H | Me | 4-Me-pyridin-2-yl | 5-Me |
| 5-149 | H | H | Me | 4-Me-pyridin-2-yl | 5-CI |
| 5-150 | H | H | Me | 4-Me-pyridin-2-yl | 5-F |
| 5-151 | H | H | Me | 4-F-pyridin-2-yl | H |
| 5-152 | H | H | Me | 4-Cl-pyridin-2-yl | H |
| 5-153 | H | H | Me | 4-Br-pyridin-2-yl | H |
| 5-154 | H | H | Me | 4-OMe-pyridin-2-yl | H |
| 5-155 | H | H | Me | 5-CF₃-pyridin-2-yl | H |
| 5-156 | H | H | Me | 6-OMe-pyridin-2-yl | H |
| 5-157 | H | H | cyPr | 4-CI-Ph | H |
| 5-158 | H | H | CN | 4-CI-Ph | H |
| 5-159 | H | H | CN | 4-CI-Ph | 5-Me |
| 5-160 | H | H | CN | 4-Me-Ph | H |
| 5-161 | H | H | CN | 4-Me-Ph | 5-Me |
| 5-162 | H | H | CN | Ph | H |
| 5-163 | H | H | CN | Ph | 5-Me |
| 5-164 | H | H | CN | 2-pyridyl | H |
| 5-165 | H | H | CN | 3-pyridyl | H |
| 5-166 | H | H | CN | 5-Cl-pyridin-2-yl | H |
| 5-167 | H | H | CN | 5-Br-pyridin-2-yl | H |
| 5-168 | H | H | CN | 5-F-pyridin-2-yl | H |
| 5-169 | H | H | CN | 5-Me-pyridin-2-yl | H |
| 5-170 | H | H | CN | 6-Me-pyridin-3-yl | H |
| 5-171 | H | H | CN | 4-Me-pyridin-2-yl | H |
| 5-172 | H | H | CN | 4-F-pyridin-2-yl | H |
| 5-173 | H | H | CN | 4-Cl-pyridin-2-yl | H |
| 5-174 | H | H | CN | 4-Br-pyridin-2-yl | H |
| 5-175 | H | H | CN | 4-OMe-pyridin-2-yl | H |
| 5-176 | H | H | formyl | 4-CI-Ph | H |
| 5-177 | H | H | formyl | 4-CI-Ph | 5-Me |
| 5-178 | H | H | formyl | 4-Me-Ph | H |
| 5-179 | H | H | formyl | 4-Me-Ph | 5-Me |
| 5-180 | H | H | formyl | Ph | H |
| 5-181 | H | H | formyl | Ph | 5-Me |
| 5-182 | H | H | formyl | 2-pyridyl | H |
| 5-183 | H | H | formyl | 3-pyridyl | H |
| 5-184 | H | H | formyl | 5-Cl-pyridin-2-yl | H |
| 5-185 | H | H | formyl | 5-Br-pyridin-2-yl | H |
| 5-186 | H | H | formyl | 5-F-pyridin-2-yl | H |
| 5-187 | H | H | formyl | 5-Me-pyridin-2-yl | H |
| 5-188 | H | H | formyl | 6-Me-pyridin-3-yl | H |
| 5-189 | H | H | formyl | 4-Me-pyridin-2-yl | H |
| 5-190 | H | H | formyl | 4-F-pyridin-2-yl | H |
| 5-191 | H | H | formyl | 4-Cl-pyridin-2-yl | H |
| 5-192 | H | H | formyl | 4-Br-pyridin-2-yl | H |
| 5-193 | H | H | formyl | 4-OMe-pyridin-2-yl | H |
| 5-194 | H | H | CH₂OH | 5-Me-pyridin-2-yl | H |
| 5-195 | H | H | CH₂OH | 4-CI-Ph | H |
| 5-196 | H | H | CH₂OH | 4-Me-pyridin-2-yl | H |
| 5-197 | H | H | CH₂OH | 4-Me-Ph | H |
| 5-198 | H | H | CH₂OH | Ph | H |
| 5-199 | H | H | CH₂OH | 2-Pyridyl | H |
| 5-200 | H | H | Me | 2-Thiazolyl | H |
| 5-201 | H | H | Me | 2-Thiazolyl | 5-CI |
| 5-202 | H | H | Me | 2-Thiazolyl | 5-Me |
| 5-203 | H | H | Me | 4-Me-thiazol-2-yl | H |
| 5-204 | H | H | Me | 4-Me-thiazol-2-yl | 5-CI |
| 5-205 | H | H | Me | 4-Me-thiazol-2-yl | 5-Me |
| 5-206 | H | H | Me | 5-Me-thiazol-2-yl | H |
| 5-207 | H | H | Me | 5-Br-thiazol-2-yl | H |
| 5-208 | H | H | Me | 5-Br-thiazol-2-yl | 5-Me |
| 5-209 | H | H | Me | 5-Cl-thiazol-2-yl | H |
| 5-210 | H | H | Me | 4,6-Me₂-pyridin-2-yl | H |
| 5-211 | H | H | Me | 4,6-Me₂-pyridin-2-yl | 5-Me |
| 5-212 | H | H | Me | 2-Pyridyl | 5-F |
| 5-213 | H | H | Me | 2-Pyrazinyl | H |
| 5-214 | H | H | Me | 5-Me-Pyrazin-2-yl | H |
| 5-215 | H | H | Me | 2-Pyrazinyl | 5-Me |
| 5-216 | H | H | Me | 1,3-Benzothiazol-2-yl | H |
| 5-217 | H | H | Me | 1,3-Benzothiazol-2-yl | 5-Me |
| 5-218 | H | H | Me | 7-Cl-1,3-benzothiazol-2-yl | H |
| 5-219 | H | H | Me | 1,5-Me₂-pyrazol-3-yl | H |
| 5-220 | H | H | Me | 1,5-Me₂-pyrazol-3-yl | 5-Me |
| 5-221 | H | H | Me | 4,5-Me₂-thiazol-2-yl | H |
| 5-222 | H | H | Me | 4,5-Cl₂-thiazol-2-yl | H |
| 5-223 | H | H | Me | 2-Pyrimidinyl | H |
| 5-224 | H | H | Me | 2-Pyrimidinyl | 5-Me |
| 5-225 | H | H | Me | 5-F-pyrimidin-2-yl | H |
| 5-226 | H | H | Me | 5-Cl-pyrimidin-2-yl | H |
| 5-227 | H | H | Me | 5-Br-pyrimidin-2-yl | H |
| 5-228 | H | H | Me | 5-Me-pyrimidin-2-yl | H |
| 5-229 | H | H | Me | 5-Me-pyrimidin-2-yl | 5-Me |
| 5-230 | H | H | Me | 4,6-Me₂-pyrimidin-2-yl | H |
| 5-231 | H | H | Me | 4,6-Me₂-pyrimidin-2-yl | 5-Me |
| 5-232 | H | H | Me | 3-Pyridazinyl | H |
| 5-233 | H | H | Me | 6-Me-Pyridazin-3-yl | H |
| 5-234 | H | H | Me | 1,2,4-Triazin-2-yl | H |
| 5-235 | H | H | Me | 6-Me-1,2,4-triazin-3-yl | H |
| 5-236 | H | H | Me | 4-CI-Ph | 3-COOMe |
| 5-237 | H | H | Me | 4-CI-Ph | 3,5-(COOMe)₂ |
| 5-238 | H | H | Me | 2-Pyridyl | 3,5-(COOMe)₂ |
| 5-239 | H | H | Me | Chinolin-2-yl | H |
| 5-240 | H | H | Me | Isochinolin-3-yl | H |
| 5-241 | H | H | Me | 2-Pyridyl | 3,5-(COOEt)₂ |
| 5-242 | H | H | Me | 4-NO₂-Ph | H |
| 5-243 | H | H | Me | 3,5-Cl₂-Ph | H |
| 5-244 | H | H | Me | 2-Me-pyridin-4-yl | H |
| 5-245 | H | H | Me | 4-Cl-6-Me-pyridin-2-yl | H |
| 5-246 | H | H | Me | 4-Br-3-Me-Ph | H |
| 5-247 | H | H | Me | 5-Cl-pyridin-3-yl | H |
| 5-248 | H | H | Me | 5-Allyl-pyridin-2-yl | H |
| 5-249 | H | H | Me | 5-Cyclopropyl-pyridin-2-yl | H |
| 5-250 | H | H | Me | 5-Ethinyl-pyridin-2-yl | H |
| 5-251 | H | H | Me | 5-Ph-pyridin-2-yl | H |
| 5-252 | H | H | Me | 5-OH-pyridin-2-yl | H |
| 5-253 | H | H | Me | 5-OCHF₂-pyridin-2-yl | H |
| 5-254 | H | H | Me | 5-MeO-pyridin-2-yl | H |
| 5-255 | H | H | Me | 5-MeS-pyridin-2-yl | H |
| 5-256 | H | H | Me | 5-NHMe-pyridin-2-yl | H |
| 5-257 | H | H | Me | 5-NMe₂-pyridin-2-yl | H |
| 5-258 | H | H | Me | 6-Cl-1,3-benzoxazol-2-yl | H |
| 5-259 | H | H | Me | 6-Br-1,3-benzoxazol-2-yl | H |
| 5-260 | H | H | Me | 7-Cl-1,3-benzoxazol-2-yl | H |
| 5-261 | H | H | Me | 5-NH₂-pyridin-2-yl | H |
| 5-262 | H | H | Me | 2-Cl-thiazol-4-yl | H |
| 5-263 | H | H | Me | 2-Br-thiazol-4-yl | H |
| 5-264 | H | H | Me | 5-OSO₂Me-pyridin-2-yl | H |
| 5-265 | H | H | Me | 6-Cl-1,3-benzothiazol-2-yl | H |
| 5-266 | H | H | Me | 6-Br-1,3-benzothiazol-2-yl | H |
| 5-267 | H | H | Me | 1,3-Benzoxazol-2-yl | H |
| 5-268 | H | H | Me | 4-PhO-Ph | H |
| 5-269 | H | H | Me | 2,3-Cl₂-Ph | H |
| 5-270 | H | H | Me | 5-I-pyridin-2-yl | H |
| 5-271 | H | H | Me | 5-I-pyrimidin-2-yl | H |
| 5-272 | H | H | Me | 3,4-F₂-Ph | H |
| 5-273 | H | H | Me | 1-Me-pyrazol-3-yl | H |
| 5-274 | H | H | Me | 1-Me-pyrazol-5-yl | H |
| 5-275 | H | H | Me | 3-Br-Ph | H |
| 5-276 | H | H | Me | 4-Ph-Ph | H |
| 5-277 | H | H | Me | 1,3-Benzodioxol-5-yl | H |
| 5-278 | H | H | Me | 4-J-Ph | H |
| 5-279 | H | H | Me | 5-Br-3-thienyl | H |
| 5-280 | H | H | Me | 5-Me-3-thienyl | H |
| 5-281 | H | H | Me | 2-F-Ph | H |
| 5-282 | H | H | Me | 2-CN-Ph | H |
| 5-283 | H | H | Me | 2-NO₂-Ph | H |
| 5-284 | H | H | Me | 2,4-F₂-Ph | H |
| 5-285 | H | H | Me | 5-Thiazolyl | H |
| 5-286 | H | H | Me | 2-Me-thiazol-4-yl | H |
| 5-287 | H | H | Me | 2-Me-thiazol-5-yl | H |
| 5-288 | H | H | Me | 5-Cl-3-thienyl | H |
| 5-289 | H | H | Me | 6-Br-pyridin-3-yl | H |
| 5-290 | H | H | Me | 4-Cl-3-thienyl | H |
| 5-291 | H | H | Me | 4-Br-3-thienyl | H |
| 5-292 | H | H | Me | 4-Me-3-thienyl | H |
| 5-293 | H | H | Me | 4-Thiazolyl | H |
| 5-294 | H | H | Me | 4-Me-5-Cl-pyridin-2-yl | H |

Für einige Verbindungen der allgemeinen Formel (I) aus der Tabelle 5 wurden ¹H-NMR-Spektren bei 400 MHz (CDCl₃) (¹H-Kernresonanz-Daten) gemessen. Nachfolgend sind charakteristische chemische Verschiebungen δ (ppm) für einige Beispielverbindungen aufgeführt (die Verbindungsnummer entspricht der laufenden Nr. aus Tabelle 5):
NMR Verbindung 5-2 (CDCl₃, 400 MHz, δ in ppm): 2.35 (*s*, 3H); 3.39 (*s*, 2H); 3.70 (*s*, 3H); 7,29 (*m,* 2H); 7.46 (*m*, 3H); 8.09 (*s*, 1 H); 8.35 (*s*, 1H).
NMR Verbindung 5-28 (CDCl₃, 400 MHz, δ in ppm): 2.35 (*s*, 3H); 3.38 (*s*, 2H); 3.70 (*s,* 3H); 7.22 (*d*, 2H); 7.41 (*d*, 2H); 8.13 (*s*, 1H); 8.37 (*s*, 1H).
NMR Verbindung 5-115 (CDCl₃, 400 MHz, δ in ppm): 2.37 (*s*, 3H); 3.57 (*s*, 2H); 3.69 (*s*, 3H); 7.32 (*dd,* 1 H); 7.38 (*d*, 1 H); 7,77 (*t,* 1 H); 8.33 (*s*, 1 H); 8.35 (*s*, 1 H); 8.70 (*d*, 1 H).
NMR Verbindung 5-116 (CDCl₃, 400 MHz, δ in ppm): 2.35 (*s*, 3H); 3.55 (*s*, 2H); 3.70 (*s*, 3H); 7.32 (*d*, 1 H); 7,72 (*dd,* 1H); 8.35 (*s*, 1H); 8.39 (*s*, 1 H); 8.63 (d, 1 H).
NMR Verbindung 5-119 (CDCl₃, 400 MHz, δ in ppm): 2.35 (*s*, 3H); 3.55 (*s*, 2H); 3.70 (*s*, 3H); 7.26 (*d*, 1 H); 7,88 (*dd,* 1H); 8.36 (*s*, 1H); 8.39 (*s*, 1 H); 8.72 (*d*, 1 H).
NMR Verbindung 5-131 (CDCl₃, 400 MHz, δ in ppm): 2.35 (*s*, 3H); 3.38 (*s*, 2H); 3.70 (*s*, 3H); 7.15 (*t*, 2H); 7.28 (*m*, 2H); 8.11 (*s*, 1H); 8.35 (*s*, 1H).
NMR Verbindung 5-216 (CDCl₃, 400 MHz, δ in ppm): 2.38 (*s*, 3H); 3.71 (*s*, 3H); 3.84 (*s*, 2H); 7.45 (*t,* 1 H); 7,53 (*t,* 1 H); 7.88 (*d*, 1 H); 8.08 (*d,* 1 H); 8.57 (*s*, 1 H); 8.69 (*s*, 1 H).
NMR Verbindung 5-227 (CDCl₃, 400 MHz, δ in ppm): 2.38 (*s*, 3H); 3.69 (*s*, 3H); 3.99 (*s*, 2H); 8.28 (*s*, 1 H); 8.30 (*s*, 1 H); 8.34 (*s*, 2H).
NMR Verbindung 5-238 (CDCl₃, 400 MHz, δ in ppm): 2.37 (*s*, 3H); 3.66 (*s*, 2H); 3.76 (*s*, 6H); 3.79 (*s*, 3H); 7.13 (*m*, 1 H); 7.47 (*m*, 1 H); 7.66 (*m*, 1 H); 8.38 (br s, 1 H).
NMR Verbindung 5-239 (CDCl₃, 400 MHz, δ in ppm): 2.38 (*s*, 3H); 3.64 (*s*, 3H); 3.68 (*s*, 2H); 7.37 (*d*, 1 H); 7.62 (*t,* 1 H); 7.78 (*t*, 1 H); 7.86 (*d, 1H*)*;* 8.08 (*d*, 1H); 8.18 (*d*, 1H); 8.37 (*s*, 1H); 8.43 (*s*, 1H).
NMR Verbindung 5-240 (CDCl₃, 400 MHz, δ in ppm): 2.39 (*s*, 3H); 3.57 (*s*, 2H); 3.70 (*s*, 3H); 7.70 (*t,* 1 H); 7.78 (*t,* 1 H); 7,83 (*s*, 1H); 7.84 (*d*, *1H);* 8.04 (*d*, 1 H); 8,34 (*s*, 1H); 8.35 (*s*, 1H); 9.28 (*s*, 1H).
NMR Verbindung 5-241 (CDCl₃, 400 MHz, δ in ppm): 2.36 (*s*, 3H); 3.64 (*s*, 2H); 3.73 (*s*, 6H); 3.78 (*s*, 3H); 7.13 (*m*, 1H); 7.47 (*m*, 1H); 7.65 (*m*, 1H); 8.38 (*m*, 1H).

**Tabelle 6: Verbindungen der Formel (Ib'''')**

| | | | | |
|---|---|---|---|---|
| 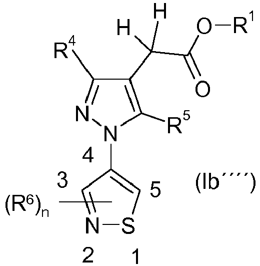 | | | | |

| Nr. | R¹ | R⁴ | R⁵ | (R⁶)ₙ |
|---|---|---|---|---|
| 6-1 | Et | Me | Ph | H |
| 6-2 | Et | Me | Ph | 5-Me |
| 6-3 | Et | Me | 3-Cl-Ph | H |
| 6-4 | Et | Me | 4-Cl-Ph | H |
| 6-5 | Et | Me | 4-Cl-Ph | 5-Me |
| 6-6 | Et | Me | 2-Thienyl | H |
| 6-7 | Et | Me | 3-Thienyl | H |
| 6-8 | Et | Me | 3-Me-2-thienyl | H |
| 6-9 | Et | Me | 4-Me-2-thienyl | H |
| 6-10 | Et | Me | 5-Br-2-thienyl | H |
| 6-11 | Et | Me | 5-Br-2-thienyl | 5-Me |
| 6-12 | Et | Me | 5-Cl-2-thienyl | H |
| 6-13 | Et | Me | 5-Cl-2-thienyl | 5-Me |
| 6-14 | Et | Me | 5-J-2-thienyl | H |
| 6-15 | Et | Me | 5-Me-2-thienyl | H |
| 6-16 | Et | Me | 3-Pyridyl | H |
| 6-17 | Et | Me | 6-MeO-pyridin-3-yl | H |
| 6-18 | Et | Me | 6-OH-pyridin-3-yl | H |
| 6-19 | Et | Me | 6-Me-pyridin-3-yl | H |
| 6-20 | Et | Me | 4-Me-Ph | H |
| 6-21 | Et | Me | 4-Me-Ph | 5-Me |
| 6-22 | Et | Me | 4-Br-Ph | H |
| 6-23 | Et | Me | 4-F-Ph | H |
| 6-24 | Et | Me | 4-F-Ph | 5-Me |
| 6-25 | Et | Me | 5-Cl-pyridin-2-yl | H |
| 6-26 | Et | Me | 5-Br-pyridin-2-yl | H |
| 6-27 | Et | Me | 5-F-pyridin-2-yl | H |
| 6-28 | Et | Me | 5-F-pyridin-2-yl | 5-Me |
| 6-29 | Et | Me | 5-Cl-pyridin-2-yl | 5-Me |
| 6-30 | Et | Me | 5-Br-pyridin-2-yl | 5-Me |
| 6-31 | Et | Me | 5-Me-pyridin-2-yl | H |
| 6-32 | Et | Me | 5-Me-pyridin-2-yl | 5-Me |
| 6-33 | Et | Me | 2-Pyridyl | 5-Me |
| 6-34 | Et | Me | 2-Pyridyl | H |
| 6-35 | Et | Me | 4-Pyridyl | H |
| 6-36 | Et | Me | 4-Me-pyridin-2-yl | H |
| 6-37 | Et | Me | 4-Me-pyridin-2-yl | 5-Me |
| 6-38 | Et | Me | 2-Thiazolyl | H |
| 6-39 | Et | Me | 4-Me-thiazol-2-yl | H |
| 6-40 | Et | Me | 5-Br-thiazol-2-yl | H |
| 6-41 | Et | Me | 5-Cl-thiazol-2-yl | H |
| 6-42 | Et | Me | 5-Me-thiazol-2-yl | H |
| 6-43 | Et | Me | 4,5-Me₂-thiazol-2-yl | H |
| 6-44 | Et | Me | 4,5-Cl₂-thiazol-2-yl | H |
| 6-45 | Et | Me | 4,6-Me₂-pyridin-2-yl | H |
| 6-46 | Et | Me | 2-Pyrazinyl | H |
| 6-47 | Et | Me | 2-Pyrimidinyl | H |
| 6-48 | Et | Me | 2-Pyrimidinyl | 5-Me |
| 6-49 | Et | Me | 5-Cl-pyrimidin-2-yl | H |
| 6-50 | Et | Me | 5-Br-pyrimidin-2-yl | H |
| 6-51 | Et | Me | 5-Me-pyrimidin-2-yl | H |
| 6-52 | Et | Me | 5-Me-pyrimidin-2-yl | 5-Me |
| 6-53 | Et | Me | 4,6-Me₂-pyrimidin-2-yl | H |
| 6-54 | Et | Me | 4,6-Me₂-pyrimidin-2-yl | 5-Me |
| 6-55 | Et | Me | 1,3-Benzothiazol-2-yl | H |
| 6-56 | Et | Me | 7-Cl-1,3-benzothiazol-2-yl | H |
| 6-57 | Et | Me | 1,5-Me₂-pyrazol-3-yl | H |
| 6-58 | Et | Me | 5-Me-Pyrazin-2-yl | H |
| 6-59 | Et | Me | 5-F-pyrimidin-2-yl | H |
| 6-60 | Et | Me | 3-Pyridazinyl | H |
| 6-61 | Et | Me | 6-Me-Pyridazin-3-yl | H |
| 6-62 | Et | Me | 1,2,4-Triazin-3-yl | H |
| 6-63 | Et | Me | 6-Me-1,2,4-triazin-3-yl | H |
| 6-64 | Et | Me | Chinolin-2-yl | H |
| 6-65 | Et | Me | Isochinolin-3-yl | H |
| 6-66 | Pr | Me | Ph | H |
| 6-67 | Pr | Me | 4-Cl-Ph | H |
| 6-68 | Pr | Me | 2-Thienyl | H |
| 6-69 | Pr | Me | 3-Pyridyl | H |
| 6-70 | Pr | Me | 6-Me-pyridin-3-yl | H |
| 6-71 | Pr | Me | 4-Me-Ph | H |
| 6-72 | Pr | Me | 4-Br-Ph | H |
| 6-73 | Pr | Me | 4-F-Ph | H |
| 6-74 | Pr | Me | 5-Cl-pyridin-2-yl | H |
| 6-75 | Pr | Me | 5-Br-pyridin-2-yl | H |
| 6-76 | Pr | Me | 5-F-pyridin-2-yl | H |
| 6-77 | Pr | Me | 5-Me-pyridin-2-yl | H |
| 6-78 | Pr | Me | 2-Pyridyl | H |
| 6-79 | Pr | Me | 4-Pyridyl | H |
| 6-80 | i-Pr | Me | Ph | H |
| 6-81 | i-Pr | Me | 4-CI-Ph | H |
| 6-82 | i-Pr | Me | 2-Thienyl | H |
| 6-83 | i-Pr | Me | 3-Pyridyl | H |
| 6-84 | i-Pr | Me | 6-Me-pyridin-3-yl | H |
| 6-85 | i-Pr | Me | 4-Me-Ph | H |
| 6-86 | i-Pr | Me | 4-Br-Ph | H |
| 6-87 | i-Pr | Me | 4-F-Ph | H |
| 6-88 | i-Pr | Me | 5-Cl-pyridin-2-yl | H |
| 6-89 | i-Pr | Me | 5-Br-pyridin-2-yl | H |
| 6-90 | i-Pr | Me | 5-F-pyridin-2-yl | H |
| 6-91 | i-Pr | Me | 5-Me-pyridin-2-yl | H |
| 6-92 | i-Pr | Me | 2-Pyridyl | H |
| 6-93 | i-Pr | Me | 4-Pyridyl | H |
| 6-94 | CH₂Ph | Me | Ph | H |
| 6-95 | CH₂Ph | Me | 4-Cl-Ph | H |
| 6-96 | CH₂Ph | Me | 2-Thienyl | H |
| 6-97 | CH₂Ph | Me | 2-Pyridyl | H |
| 6-98 | Prop-2-in-1-yl | Me | Ph | H |
| 6-99 | Prop-2-in-1-yl | Me | 4-Cl-Ph | H |
| 6-100 | Prop-2-in-1-yl | Me | 2-Thienyl | H |
| 6-101 | Prop-2-in-1-yl | Me | 3-Thienyl | H |
| 6-102 | Prop-2-in-1-yl | Me | 3-Me-2-thienyl | H |
| 6-103 | Prop-2-in-1-yl | Me | 4-Me-2-thienyl | H |
| 6-104 | Prop-2-in-1-yl | Me | 5-Cl-2-thienyl | H |
| 6-105 | Prop-2-in-1-yl | Me | 5-Me-2-thienyl | H |
| 6-106 | Prop-2-in-1-yl | Me | 3-Pyridyl | H |
| 6-107 | Prop-2-in-1-yl | Me | 6-MeO-pyridin-3-yl | H |
| 6-108 | Prop-2-in-1-yl | H | Ph | H |
| 6-109 | Prop-2-in-1-yl | Me | 6-Me-pyridin-3-yl | H |
| 6-110 | Prop-2-in-1-yl | Me | 4-Me-Ph | H |
| 6-111 | Prop-2-in-1-yl | Me | 4-Br-Ph | H |
| 6-112 | Prop-2-in-1-yl | Me | 4-F-Ph | H |
| 6-113 | Prop-2-in-1-yl | Me | 5-Cl-pyridin-2-yl | H |
| 6-114 | Prop-2-in-1-yl | Me | 5-Br-pyridin-2-yl | H |
| 6-115 | Prop-2-in-1-yl | Me | 5-F-pyridin-2-yl | H |
| 6-116 | Prop-2-in-1-yl | Me | 5-Me-pyridin-2-yl | H |
| 6-117 | Prop-2-in-1-yl | Me | 2-Pyridyl | H |
| 6-118 | Prop-2-in-1-yl | Me | 4-Pyridyl | H |
| 6-119 | Prop-2-in-1-yl | Me | 4-Cl-Ph | 5-Me |
| 6-120 | Prop-2-in-1-yl | Me | Ph | 5-Me |
| 6-121 | Cyclopropylmethyl | Me | Ph | H |
| 6-122 | Cyclopropylmethyl | Me | 4-Cl-Ph | H |
| 6-123 | Cyclopropylmethyl | Me | 2-Thienyl | H |
| 6-124 | Cyclopropylmethyl | Me | 3-Thienyl | H |
| 6-125 | Cyclopropylmethyl | Me | 3-Me-2-thienyl | H |
| 6-126 | Cyclopropylmethyl | Me | 3-Pyridyl | H |
| 6-127 | Cyclopropylmethyl | Me | 5-Cl-2-thienyl | H |
| 6-128 | Cyclopropylmethyl | Me | 5-Me-2-thienyl | H |
| 6-129 | Cyclopropylmethyl | Me | 4-Me-2-thienyl | H |
| 6-130 | Cyclopropylmethyl | Me | 6-MeO-pyridin-3-yl | H |
| 6-131 | Cyclopropylmethyl | Me | 6-OH-pyridin-3-yl | H |
| 6-132 | Cyclopropylmethyl | Me | 6-Me-pyridin-3-yl | H |
| 6-133 | Cyclopropylmethyl | Me | 4-Me-Ph | H |
| 6-134 | Cyclopropylmethyl | Me | 4-Br-Ph | H |
| 6-135 | Cyclopropylmethyl | Me | 4-F-Ph | H |
| 6-136 | Cyclopropylmethyl | Me | 5-Cl-pyridin-2-yl | H |
| 6-137 | Cyclopropylmethyl | Me | 5-Br-pyridin-2-yl | H |
| 6-138 | Cyclopropylmethyl | Me | 5-F-pyridin-2-yl | H |
| 6-139 | Cyclopropylmethyl | Me | 5-Me-pyridin-2-yl | H |
| 6-140 | Cyclopropylmethyl | Me | 2-Pyridyl | H |
| 6-141 | Cyclopropylmethyl | Me | 4-Pyridyl | H |
| 6-142 | Cyclopropylmethyl | Me | 4-CI-Ph | 5-Me |
| 6-143 | Cyclopropylmethyl | Me | Ph | 5-Me |
| 6-144 | Cyclopropylmethyl | H | Ph | H |
| 6-145 | Cyclopropylmethyl | H | Chinolin-2-yl | H |
| 6-146 | Cyclopropylmethyl | H | Isochinolin-3-yl | H |
| 6-147 | 3,3-Dichlor-2-fluorprop-2-en-1-yl | Me | Ph | H |
| 6-148 | 3,3-Dichlor-2-fluorprop-2-en-1-yl | Me | 4-Cl-Ph | H |
| 6-149 | 3,3-Dichlor-2-fluorprop-2-en-1-yl | Me | 2-Thienyl | H |
| 6-150 | 3,3-Dichlor-2-fluorprop-2-en-1-yl | Me | 2-Pyridyl | H |
| 6-151 | (1-Methylcyclopropyl)-methyl | Me | Ph | H |
| 6-152 | (1-Methylcyclopropyl)-methyl | Me | 4-Cl-Ph | H |
| 6-153 | (1-Methylcyclopropyl)-methyl | Me | 2-Thienyl | H |
| 6-154 | (1-Methylcyclopropyl)-methyl | Me | 2-Pyridyl | H |
| 6-155 | 4-Chlorbut-2-in-1-yl | Me | Ph | H |
| 6-156 | 4-Chlorbut-2-in-1-yl | Me | 4-Cl-Ph | H |
| 6-157 | 4-Chlorbut-2-in-1-yl | Me | 2-Thienyl | H |
| 6-158 | 4-Chlorbut-2-in-1-yl | Me | 2-Pyridyl | H |
| 6-159 | (2,2-Dichlorcyclopropyl)- methyl | Me | Ph | H |
| 6-160 | (2,2-Dichlorcyclopropyl)- methyl | Me | 4-Cl-Ph | H |
| 6-161 | (2,2-Dichlorcyclopropyl)- methyl | Me | 2-Thienyl | H |
| 6-162 | (2,2-Dichlorcyclopropyl)- methyl | Me | 2-Pyridyl | H |
| 6-163 | But-2-in-1-yl | Me | Ph | H |
| 6-164 | But-2-in-1-yl | Me | 4-Cl-Ph | H |
| 6-165 | But-2-in-1-yl | Me | 2-Thienyl | H |
| 6-166 | But-2-in-1-yl | Me | 3-Thienyl | H |
| 6-167 | But-2-in-1-yl | Me | 3-Me-2-thienyl | H |
| 6-168 | But-2-in-1-yl | Me | 4-Me-2-thienyl | H |
| 6-169 | But-2-in-1-yl | Me | 5-Cl-2-thienyl | H |
| 6-170 | But-2-in-1-yl | Me | 5-Me-2-thienyl | H |
| 6-171 | But-2-in-1-yl | Me | 3-Pyridyl | H |
| 6-172 | But-2-in-1-yl | Me | 6-MeO-pyridin-3-yl | H |
| 6-173 | But-2-in-1-yl | H | Ph | H |
| 6-174 | But-2-in-1-yl | Me | 6-Me-pyridin-3-yl | H |
| 6-175 | But-2-in-1-yl | Me | 4-Me-Ph | H |
| 6-176 | But-2-in-1-yl | Me | 4-Br-Ph | H |
| 6-177 | But-2-in-1-yl | Me | 4-F-Ph | H |
| 6-178 | But-2-in-1-yl | Me | 5-Cl-pyridin-2-yl | H |
| 6-179 | But-2-in-1-yl | Me | 5-Br-pyridin-2-yl | H |
| 6-180 | But-2-in-1-yl | Me | 5-F-pyridin-2-yl | H |
| 6-181 | But-2-in-1-yl | Me | 5-Me-pyridin-2-yl | H |
| 6-182 | But-2-in-1-yl | Me | 2-Pyridyl | H |
| 6-183 | But-2-in-1-yl | Me | 4-Pyridyl | H |
| 6-184 | But-2-in-1-yl | Me | 4-CI-Ph | 5-Me |
| 6-185 | But-2-in-1-yl | Me | Ph | 5-Me |
| 6-186 | 1-Methylprop-2-in-1-yl | Me | Ph | H |
| 6-187 | 1-Methylprop-2-in-1-yl | Me | 4-Cl-Ph | H |
| 6-188 | 1-Methylprop-2-in-1-yl | Me | 2-Thienyl | H |
| 6-189 | 1-Methylprop-2-in-1-yl | Me | 2-Pyridyl | H |
| 6-190 | 1-Cyclopropylethyl | Me | Ph | H |
| 6-191 | 1-Cyclopropylethyl | Me | 4-Cl-Ph | H |
| 6-192 | 1-Cyclopropylethyl | Me | 2-Thienyl | H |
| 6-193 | 1-Cyclopropylethyl | Me | 2-Pyridyl | H |
| 6-194 | Allyl | Me | Ph | H |
| 6-195 | Allyl | Me | 4-Cl-Ph | H |
| 6-196 | Allyl | Me | 2-Thienyl | H |
| 6-197 | Allyl | Me | 2-Pyridyl | H |
| 6-198 | 3-Methylbut-2-en-1-yl | Me | Ph | H |
| 6-199 | 3-Methylbut-2-en-1-yl | Me | 4-Cl-Ph | H |
| 6-200 | 3-Methylbut-2-en-1-yl | Me | 2-Thienyl | H |
| 6-201 | 3-Methylbut-2-en-1-yl | Me | 2-Pyridyl | H |
| 6-202 | 2-Methylprop-2-en-1-yl | Me | Ph | H |
| 6-203 | 2-Methylprop-2-en-1-yl | Me | 4-Cl-Ph | H |
| 6-204 | 2-Methylprop-2-en-1-yl | Me | 2-Thienyl | H |
| 6-205 | 2-Methylprop-2-en-1-yl | Me | 2-Pyridyl | H |
| 6-206 | (2E)-1-Methylbut-2-en-1-yl | Me | Ph | H |
| 6-207 | (2E)-1-Methylbut-2-en-1-yl | Me | 4-Cl-Ph | H |
| 6-208 | (2E)-1-Methylbut-2-en-1-yl | Me | 2-Thienyl | H |
| 6-209 | (2E)-1-Methylbut-2-en-1-yl | Me | 2-Pyridyl | H |
| 6-210 | 3-Phenylprop-2-in-1-yl | Me | Ph | H |
| 6-211 | 3-Phenylprop-2-in-1-yl | Me | 4-Cl-Ph | H |
| 6-212 | 3-Phenylprop-2-in-1-yl | Me | 2-Thienyl | H |
| 6-213 | 3-Phenylprop-2-in-1-yl | Me | 2-Pyridyl | H |
| 6-214 | Cyclobutylmethyl | Me | Ph | H |
| 6-215 | Cyclobutylmethyl | Me | 4-Cl-Ph | H |
| 6-216 | Cyclobutylmethyl | Me | 2-Thienyl | H |
| 6-217 | Cyclobutylmethyl | Me | 2-Pyridyl | H |
| 6-218 | Cyclopentylmethyl | Me | Ph | H |
| 6-219 | Cyclopentylmethyl | Me | 4-Cl-Ph | H |
| 6-220 | Cyclopentylmethyl | Me | 2-Thienyl | H |
| 6-221 | Cyclopentylmethyl | Me | 2-Pyridyl | H |
| 6-222 | Cyclohexylmethyl | Me | Ph | H |
| 6-223 | Cyclohexylmethyl | Me | 4-Cl-Ph | H |
| 6-224 | Cyclohexylmethyl | Me | 2-Thienyl | H |
| 6-225 | Cyclohexylmethyl | Me | 2-Pyridyl | H |
| 6-226 | But-3-en-1-yl | Me | Ph | H |
| 6-227 | But-3-en-1-yl | Me | 4-CI-Ph | H |
| 6-228 | But-3-en-1-yl | Me | 2-Thienyl | H |
| 6-229 | But-3-en-1-yl | Me | 2-Pyridyl | H |
| 6-230 | 2-Ch loroprop-2-en-1-yl | Me | Ph | H |
| 6-231 | 2-Chloroprop-2-en-1-yl | Me | 4-Cl-Ph | H |
| 6-232 | 2-Chloroprop-2-en-1-yl | Me | 2-Thienyl | H |
| 6-233 | 2-Chloroprop-2-en-1-yl | Me | 3-Thienyl | H |
| 6-234 | 2-Chloroprop-2-en-1-yl | Me | 3-Me-2-thienyl | H |
| 6-235 | 2-Chloroprop-2-en-1-yl | Me | 4-Me-2-thienyl | H |
| 6-236 | 2-Chloroprop-2-en-1-yl | Me | 5-Cl-2-thienyl | H |
| 6-237 | 2-Chloroprop-2-en-1-yl | Me | 5-Me-2-thienyl | H |
| 6-238 | 2-Chloroprop-2-en-1-yl | Me | 3-Pyridyl | H |
| 6-239 | 2-Chloroprop-2-en-1-yl | Me | 6-MeO-pyridin-3-yl | H |
| 6-240 | 2-Chloroprop-2-en-1-yl | Me | 6-OH-pyridin-3-yl | H |
| 6-241 | 2-Chloroprop-2-en-1-yl | Me | 6-Me-pyridin-3-yl | H |
| 6-242 | 2-Chloroprop-2-en-1-yl | Me | 4-Me-Ph | H |
| 6-243 | 2-Chloroprop-2-en-1-yl | Me | 4-Br-Ph | H |
| 6-244 | 2-Chloroprop-2-en-1-yl | Me | 4-F-Ph | H |
| 6-245 | 2-Chloroprop-2-en-1-yl | Me | 5-Cl-pyridin-2-yl | H |
| 6-246 | 2-Chloroprop-2-en-1-yl | Me | 5-Br-pyridin-2-yl | H |
| 6-247 | 2-Chloroprop-2-en-1-yl | Me | 5-F-pyridin-2-yl | H |
| 6-248 | 2-Chloroprop-2-en-1-yl | Me | 5-Me-pyridin-2-yl | H |
| 6-249 | 2-Chloroprop-2-en-1-yl | Me | 2-Pyridyl | H |
| 6-250 | 2-Chloroprop-2-en-1-yl | Me | 4-Pyridyl | H |
| 6-251 | 2-Chloroprop-2-en-1-yl | Me | 4-Cl-Ph | 5-Me |
| 6-252 | 2-Chloroprop-2-en-1-yl | Me | Ph | 5-Me |
| 6-253 | 2-Chloroprop-2-en-1-yl | H | Ph | H |
| 6-254 | 2-Chloroprop-2-en-1-yl | H | Chinolin-2-yl | H |
| 6-255 | 2-Chloroprop-2-en-1-yl | H | Isochinolin-3-yl | H |
| 6-256 | 2-Methoxyethyl | Me | Ph | H |
| 6-257 | 2-Methoxyethyl | Me | 4-Cl-Ph | H |
| 6-258 | 2-Methoxyethyl | Me | 2-Thienyl | H |
| 6-259 | 2-Methoxyethyl | Me | 2-Pyridyl | H |
| 6-260 | Tetrahydrofuran-2-yl-methyl | Me | Ph | H |
| 6-261 | Tetrahydrofuran-2-yl-methyl | Me | 4-Cl-Ph | H |
| 6-262 | Tetrahydrofuran-2-yl-methyl | Me | 2-Thienyl | H |
| 6-263 | Tetrahydrofuran-2-yl-methyl | Me | 2-Pyridyl | H |
| 6-264 | 2-(Dimethylamino)ethyl | Me | Ph | H |
| 6-265 | 2-(Dimethylamino)ethyl | Me | 4-Cl-Ph | H |
| 6-266 | 2-(Dimethylamino)ethyl | Me | 2-Thienyl | H |
| 6-267 | 2-(Dimethylamino)ethyl | Me | 2-Pyridyl | H |
| 6-268 | Oxetan-3-yl | Me | Ph | H |
| 6-269 | Oxetan-3-yl | Me | 4-Cl-Ph | H |
| 6-270 | Oxetan-3-yl | Me | 2-Thienyl | H |
| 6-271 | Oxetan-3-yl | Me | 2-Pyridyl | H |
| 6-272 | (3-Methyloxetan-3-yl)methyl | Me | Ph | H |
| 6-273 | (3-Methyloxetan-3-yl)methyl | Me | 4-Cl-Ph | H |
| 6-274 | (3-Methyloxetan-3-yl)methyl | Me | 2-Thienyl | H |
| 6-275 | (3-Methyloxetan-3-yl)methyl | Me | 2-Pyridyl | H |
| 6-276 | 2,2,2-Trifluorethyl | Me | Ph | H |
| 6-277 | 2,2,2-Trifluorethyl | Me | 4-Cl-Ph | H |
| 6-278 | 2,2,2-Trifluorethyl | Me | 2-Thienyl | H |
| 6-279 | 2,2,2-Trifluorethyl | Me | 3-Pyridyl | H |
| 6-280 | 2,2,2-Trifluorethyl | Me | 6-Me-pyridin-3-yl | H |
| 6-281 | 2,2,2-Trifluorethyl | Me | 4-Me-Ph | H |
| 6-282 | 2,2,2-Trifluorethyl | Me | 4-Br-Ph | H |
| 6-283 | 2,2,2-Trifluorethyl | Me | 4-F-Ph | H |
| 6-284 | 2,2,2-Trifluorethyl | Me | 5-Cl-pyridin-2-yl | H |
| 6-285 | 2,2,2-Trifluorethyl | Me | 5-Br-pyridin-2-yl | H |
| 6-286 | 2,2,2-Trifluorethyl | Me | 5-F-pyridin-2-yl | H |
| 6-287 | 2,2,2-Trifluorethyl | Me | 5-Me-pyridin-2-yl | H |
| 6-288 | 2,2,2-Trifluorethyl | Me | 2-Pyridyl | H |
| 6-289 | 2,2,2-Trifluorethyl | Me | 4-Pyridyl | H |
| 6-290 | CH₂(4-Cl-Ph) | Me | Ph | H |
| 6-291 | CH₂(4-Cl-Ph) | Me | 4-Cl-Ph | H |
| 6-292 | CH₂(4-Cl-Ph) | Me | 2-Thienyl | H |
| 6-293 | CH₂(4-Cl-Ph) | Me | 3-Pyridyl | H |
| 6-294 | CH₂(4-Cl-Ph) | Me | 6-Me-pyridin-3-yl | H |
| 6-295 | CH₂(4-Cl-Ph) | Me | 4-Me-Ph | H |
| 6-296 | CH₂(4-Cl-Ph) | Me | 4-Br-Ph | H |
| 6-297 | CH₂(4-Cl-Ph) | Me | 4-F-Ph | H |
| 6-298 | CH₂(4-Cl-Ph) | Me | 5-Cl-pyridin-2-yl | H |
| 6-299 | CH₂(4-Cl-Ph) | Me | 5-Br-pyridin-2-yl | H |
| 6-300 | CH₂(4-Cl-Ph) | Me | 5-F-pyridin-2-yl | H |
| 6-301 | CH₂(4-Cl-Ph) | Me | 5-Me-pyridin-2-yl | H |
| 6-302 | CH₂(4-Cl-Ph) | Me | 2-Pyridyl | H |
| 6-303 | CH₂(4-Cl-Ph) | Me | 4-Pyridyl | H |
| 6-304 | CH₂(4-F-Ph) | Me | Ph | H |
| 6-305 | CH₂(4-F-Ph) | Me | 4-Cl-Ph | H |
| 6-306 | CH₂(4-F-Ph) | Me | 2-Thienyl | H |
| 6-307 | CH₂(4-F-Ph) | Me | 3-Pyridyl | H |
| 6-308 | CH₂(4-F-Ph) | Me | 6-Me-pyridin-3-yl | H |
| 6-309 | CH₂(4-F-Ph) | Me | 4-Me-Ph | H |
| 6-310 | CH₂(4-F-Ph) | Me | 4-Br-Ph | H |
| 6-311 | CH₂(4-F-Ph) | Me | 4-F-Ph | H |
| 6-312 | CH₂(4-F-Ph) | Me | 5-Cl-pyridin-2-yl | H |
| 6-313 | CH₂(4-F-Ph) | Me | 5-Br-pyridin-2-yl | H |
| 6-314 | CH₂(4-F-Ph) | Me | 5-F-pyridin-2-yl | H |
| 6-315 | CH₂(4-F-Ph) | Me | 5-Me-pyridin-2-yl | H |
| 6-316 | CH₂(4-F-Ph) | Me | 2-Pyridyl | H |
| 6-317 | CH₂(4-F-Ph) | Me | 4-Pyridyl | H |
| 6-318 | CH₂(4-OMe-Ph) | Me | Ph | H |
| 6-319 | CH₂(4-OMe-Ph) | Me | 4-Cl-Ph | H |
| 6-320 | CH₂(4-OMe-Ph) | Me | 2-Thienyl | H |
| 6-321 | CH₂(4-OMe-Ph) | Me | 3-Pyridyl | H |
| 6-322 | CH₂(4-OMe-Ph) | Me | 6-Me-pyridin-3-yl | H |
| 6-323 | CH₂(4-OMe-Ph) | Me | 4-Me-Ph | H |
| 6-324 | CH₂(4-OMe-Ph) | Me | 4-Br-Ph | H |
| 6-325 | CH₂(4-OMe-Ph) | Me | 4-F-Ph | H |
| 6-326 | CH₂(4-OMe-Ph) | Me | 5-Cl-pyridin-2-yl | H |
| 6-327 | CH₂(4-OMe-Ph) | Me | 5-Br-pyridin-2-yl | H |
| 6-328 | CH₂(4-OMe-Ph) | Me | 5-F-pyridin-2-yl | H |
| 6-329 | CH₂(4-OMe-Ph) | Me | 5-Me-pyridin-2-yl | H |
| 6-330 | CH₂(4-OMe-Ph) | Me | 2-Pyridyl | H |
| 6-331 | CH₂(4-OMe-Ph) | Me | 4-Pyridyl | H |
| 6-332 | 2,2-Difluorethyl | Me | Ph | H |
| 6-333 | 2,2-Difluorethyl | Me | 4-Cl-Ph | H |
| 6-334 | 2,2-Difluorethyl | Me | 2-Thienyl | H |
| 6-335 | 2,2-Difluorethyl | Me | 2-Pyridyl | H |
| 6-336 | Ph | Me | Ph | H |
| 6-337 | Ph | Me | 4-Cl-Ph | H |
| 6-338 | Ph | Me | 2-Thienyl | H |
| 6-339 | Ph | Me | 2-Pyridyl | H |
| 6-340 | 2-Fluorethyl | Me | Ph | H |
| 6-341 | 2-Fluorethyl | Me | 4-Cl-Ph | H |
| 6-342 | 2-Fluorethyl | Me | 2-Thienyl | H |
| 6-343 | 2-Fluorethyl | Me | 2-Pyridyl | H |
| 6-344 | 2,2,3,3,3-Pentafluorpropyl | Me | Ph | H |
| 6-345 | 2,2,3,3,3-Pentafluorpropyl | Me | 4-Cl-Ph | H |
| 6-346 | 2,2,3,3,3-Pentafluorpropyl | Me | 2-Thienyl | H |
| 6-347 | 2,2,3,3,3-Pentafluorpropyl | Me | 2-Pyridyl | H |
| 6-348 | 1-Ethyl-5-methyl-1H-pyrazol-4-yl-methyl | Me | Ph | H |
| 6-349 | 1-Ethyl-5-methyl-1H-pyrazol-4-yl-methyl | Me | 4-Cl-Ph | H |
| 6-350 | 1-Ethyl-5-methyl-1H-pyrazol-4-yl-methyl | Me | 2-Thienyl | H |
| 6-351 | 1-Ethyl-5-methyl-1H-pyrazol-4-yl-methyl | Me | 2-Pyridyl | H |
| 6-352 | Et | Me | 6-Cl-1,3-benzothiazol-2-yl | H |
| 6-353 | Et | Me | 6-Br-1,3-benzothiazol-2-yl | H |
| 6-354 | Prop-2-in-1-yl | Me | Isochinolin-3-yl | H |
| 6-355 | Prop-2-in-1-yl | Me | Chinolin-2-yl | H |
| 6-356 | But-2-in-1-yl | Me | Isochinolin-3-yl | H |
| 6-357 | But-2-in-1-yl | Me | Chinolin-2-yl | H |
| 6-358 | 2,2-Difluorethyl | Me | 5-Cl-pyridin-2-yl | H |
| 6-359 | But-3-in-2-yl | Me | 5-Cl-pyridin-2-yl | H |
| 6-360 | But-3-in-2-yl | Me | Isochinolin-3-yl | H |
| 6-361 | But-3-in-2-yl | Me | Chinolin-2-yl | H |
| 6-362 | But-3-in-2-yl | Me | Ph | H |
| 6-363 | But-3-in-2-yl | Me | 4-Cl-Ph | H |
| 6-364 | But-3-in-2-yl | Me | 2-Thienyl | H |
| 6-365 | But-3-in-2-yl | Me | 3-Pyridyl | H |
| 6-366 | But-3-in-2-yl | Me | 6-Me-pyridin-3-yl | H |
| 6-367 | But-3-in-2-yl | Me | 4-Me-Ph | H |
| 6-368 | But-3-in-2-yl | Me | 4-Br-Ph | H |
| 6-369 | But-3-in-2-yl | Me | 4-F-Ph | H |
| 6-370 | But-3-in-2-yl | Me | 5-Br-pyridin-2-yl | H |
| 6-371 | But-3-in-2-yl | Me | 5-F-pyridin-2-yl | H |
| 6-372 | But-3-in-2-yl | Me | 5-Me-pyridin-2-yl | H |
| 6-373 | But-3-in-2-yl | Me | 2-Pyridyl | H |
| 6-374 | But-3-in-2-yl | Me | 4-Pyridyl | H |
| 6-375 | Pr | Me | Isochinolin-3-yl | H |
| 6-376 | Pr | Me | Chinolin-2-yl | H |
| 6-377 | iPr | Me | Isochinolin-3-yl | H |
| 6-378 | iPr | Me | Chinolin-2-yl | H |
| 6-379 | CH₂Ph | Me | Isochinolin-3-yl | H |
| 6-380 | CH₂Ph | Me | Chinolin-2-yl | H |
| 6-381 | 3,3-Dichlor-2-fluorprop-2-en-1-yl | Me | Isochinolin-3-yl | H |
| 6-382 | 3,3-Dichlor-2-fluorprop-2-en-1-yl | Me | Chinolin-2-yl | H |
| 6-383 | (1-Methylcyclopropyl)-methyl | Me | Isochinolin-3-yl | H |
| 6-384 | (1-Methylcyclopropyl)-methyl | Me | Chinolin-2-yl | H |
| 6-385 | 4-Chlorbut-2-in-1-yl | Me | Isochinolin-3-yl | H |
| 6-386 | 4-Chlorbut-2-in-1-yl | Me | Chinolin-2-yl | H |
| 6-387 | (2,2-Dichlorcyclopropyl)-methyl | Me | Isochinolin-3-yl | H |
| 6-388 | (2,2-Dichlorcyclopropyl)-methyl | Me | Chinolin-2-yl | H |
| 6-389 | 1-Methylprop-2-in-1-yl | Me | Isochinolin-3-yl | H |
| 6-390 | 1-Methylprop-2-in-1-yl | Me | Chinolin-2-yl | H |
| 6-391 | 1-Cyclopropylethyl | Me | Isochinolin-3-yl | H |
| 6-392 | 1-Cyclopropylethyl | Me | Chinolin-2-yl | H |
| 6-393 | Allyl | Me | Isochinolin-3-yl | H |
| 6-394 | Allyl | Me | Chinolin-2-yl | H |
| 6-395 | 3-Methylbut-2-en-1-yl | Me | Isochinolin-3-yl | H |
| 6-396 | 3-Methylbut-2-en-1-yl | Me | Chinolin-2-yl | H |
| 6-397 | Cyclobutylmethyl | Me | Isochinolin-3-yl | H |
| 6-398 | Cyclobutylmethyl | Me | Chinolin-2-yl | H |
| 6-399 | Cyclopentylmethyl | Me | Isochinolin-3-yl | H |
| 6-400 | Cyclopentylmethyl | Me | Chinolin-2-yl | H |
| 6-401 | Tetrahydrofuran-2-yl-methyl | Me | Isochinolin-3-yl | H |
| 6-402 | Tetrahydrofuran-2-yl-methyl | Me | Chinolin-2-yl | H |
| 6-403 | Tetrahydrofuran-2-yl-methyl | Me | 5-Cl-pyridin-2-yl | H |
| 6-404 | Tetrahydrofuran-2-yl-methyl | Me | 5-Br-pyridin-2-yl | H |
| 6-405 | Oxetan-3-yl | Me | Isochinolin-3-yl | H |
| 6-406 | Oxetan-3-yl | Me | Chinolin-2-yl | H |
| 6-407 | Oxetan-3-yl | Me | 5-Cl-pyridin-2-yl | H |
| 6-408 | Oxetan-3-yl | Me | 5-Br-pyridin-2-yl | H |
| 6-409 | (3-Methyloxetan-3-yl)methyl | Me | Isochinolin-3-yl | H |
| 6-410 | (3-Methyloxetan-3-yl)methyl | Me | Chinolin-2-yl | H |
| 6-411 | (3-Methyloxetan-3-yl)methyl | Me | 5-Cl-pyridin-2-yl | H |
| 6-412 | (3-Methyloxetan-3-yl)methyl | Me | 5-Br-pyridin-2-yl | H |
| 6-413 | 2,2,2-Trifluorethyl | Me | Isochinolin-3-yl | H |
| 6-414 | 2,2,2-Trifluorethyl | Me | Chinolin-2-yl | H |
| 6-415 | 2,2,2-Trifluorethyl | Me | 5-Cl-pyridin-2-yl | H |
| 6-416 | 2,2,2-Trifluorethyl | Me | 5-Br-pyridin-2-yl | H |
| 6-417 | 2,2-Difluorethyl | Me | Isochinolin-3-yl | H |
| 6-418 | 2,2-Difluorethyl | Me | Chinolin-2-yl | H |
| 6-419 | 2,2-Difluorethyl | Me | 5-Cl-pyridin-2-yl | H |
| 6-420 | 2,2-Difluorethyl | Me | 5-Br-pyridin-2-yl | H |
| 6-421 | Et | Me | 4-OMe-pyridin-2-yl | H |
| 6-422 | Et | Me | 4-F-pyridin-2-yl | H |
| 6-423 | Et | Me | 4-Cl-pyridin-2-yl | H |
| 6-424 | Et | Me | 4-Br-pyridin-2-yl | H |
| 6-425 | Et | Me | 6-Cl-pyridin-3-yl | H |
| 6-426 | Et | Me | 6-Br-pyridin-3-yl | H |
| 6-427 | Et | Me | 4-Cl-3-thienyl | H |
| 6-428 | Et | Me | 4-Br-3-thienyl | H |
| 6-429 | Et | Me | 4-Me-3-thienyl | H |
| 6-430 | Et | Me | 4-Thiazolyl | H |
| 6-431 | Et | Me | 5-Thiazolyl | H |
| 6-432 | Et | Me | 2-Me-thiazol-4-yl | H |
| 6-433 | Et | Me | 2-Me-thiazol-5-yl | H |
| 6-434 | Et | Me | 5-Cl-3-thienyl | H |
| 6-435 | Et | Me | 5-Br-3-thienyl | H |
| 6-436 | Et | Me | 5-Me-3-thienyl | H |
| 6-437 | Et | Me | 2-Cl-Ph | H |
| 6-438 | Et | Me | 2,4-Cl₂-Ph | H |
| 6-439 | Et | Me | 2-F-Ph | H |
| 6-440 | Et | Me | 2-CN-Ph | H |
| 6-441 | Et | Me | 2-NO₂-Ph | H |
| 6-442 | Et | Me | 2,4-F₂-Ph | H |
| 6-443 | Et | Me | 3,4-F₂-Ph | H |
| 6-444 | Et | Me | 1-Me-pyrazol-3-yl | H |
| 6-445 | Et | Me | 2-Furyl | H |
| 6-446 | Et | Me | 4-MeO-Ph | H |
| 6-447 | Et | Me | 3-CF₃-Ph | H |
| 6-448 | Et | Me | 3,4-Cl₂-Ph | H |
| 6-449 | Et | Me | 4-CF₃-Ph | H |
| 6-450 | Et | Me | 4-tBu-Ph | H |
| 6-451 | Et | Me | 3,5-Me₂-Ph | H |
| 6-452 | Et | Me | 3-Me-Ph | H |
| 6-453 | Et | Me | 3-Br-Ph | H |
| 6-454 | Et | Me | 4-Ph-Ph | H |
| 6-455 | Et | Me | 3-Cl-4-Me-Ph | H |
| 6-456 | Et | Me | 3-CF₃-4-Cl-Ph | H |
| 6-457 | Et | Me | 1,3-Benzodioxol-5-yl | H |
| 6-458 | Et | Me | 4-J-Ph | H |
| 6-459 | Et | Me | 3,5-Cl₂-Ph | H |
| 6-460 | Et | Me | 4-PhO-Ph | H |
| 6-461 | Et | Me | 3,4-Me₂-Ph | H |
| 6-462 | Et | Me | 4-(Me-CO)-Ph | H |
| 6-463 | Et | Me | 4-Cl-3-Me-Ph | H |
| 6-464 | Et | Me | 2,3-Cl₂-Ph | H |
| 6-465 | Et | Me | 5-CF₃-pyridin-2-yl | H |
| 6-466 | Et | Me | 6-OMe-pyridin-2-yl | H |
| 6-467 | Et | Me | 2-Me-pyridin-4-yl | H |
| 6-468 | Et | Me | 4-Cl-6-Me-pyridin-2-yl | H |
| 6-469 | Et | Me | 4-Br-3-Me-Ph | H |
| 6-470 | Et | Me | 5-Cl-pyridin-3-yl | H |
| 6-471 | Et | Me | 5-Allyl-pyridin-2-yl | H |
| 6-472 | Et | Me | 5-Cyclopropyl-pyridin-2-yl | H |
| 6-473 | Et | Me | 5-Ethinyl-pyridin-2-yl | H |
| 6-474 | Et | Me | 5-Ph-pyridin-2-yl | H |
| 6-475 | Et | Me | 5-I-pyridin-2-yl | H |
| 6-476 | Et | Me | 5-I-pyrimidin-2-yl | H |
| 6-477 | Et | Me | 2-Cl-thiazol-4-yl | H |
| 6-478 | Et | Me | 2-Br-thiazol-4-yl | H |
| 6-479 | Et | Me | 5-OSO₂Me-pyridin-2-yl | H |
| 6-480 | Et | Me | 1,3-Benzoxazol-2-yl | H |
| 6-481 | Et | Me | 6-Cl-1,3-benzoxazol-2-yl | H |
| 6-482 | Et | Me | 6-Br-1,3-benzoxazol-2-yl | H |
| 6-483 | Et | Me | 7-Cl-1,3-benzoxazol-2-yl | H |
| 6-484 | Et | Me | 5-NH₂-pyridin-2-yl | H |
| 6-485 | Et | Me | 5-OH-pyridin-2-yl | H |
| 6-486 | Et | Me | 5-OCHF₂-pyridin-2-yl | H |
| 6-487 | Et | Me | 5-MeO-pyridin-2-yl | H |
| 6-488 | Et | Me | 5-MeS-pyridin-2-yl | H |
| 6-489 | Et | Me | 5-NHMe-pyridin-2-yl | H |
| 6-490 | Et | Me | 5-NMe₂-pyridin-2-yl | H |
| 6-491 | Et | Me | 4-NO₂-Ph | H |
| 6-492 | Cyclopropylmethyl | Me | 4-Thiazolyl | H |
| 6-493 | Prop-2-in-1-yl | Me | 4-Thiazolyl | H |
| 6-494 | But-2-in-1-yl | Me | 4-Thiazolyl | H |
| 6-495 | But-3-in-2-yl | Me | 4-Thiazolyl | H |
| 6-496 | Pr | Me | 4-Thiazolyl | H |
| 6-497 | iPr | Me | 4-Thiazolyl | H |
| 6-498 | CH₂Ph | Me | 4-Thiazolyl | H |
| 6-499 | 3,3-Dichlor-2-fluorprop-2-en-1-yl | Me | 4-Thiazolyl | H |
| 6-500 | (1-Methylcyclopropyl)-methyl | Me | 4-Thiazolyl | H |
| 6-501 | 4-Chlorbut-2-in-1-yl | Me | 4-Thiazolyl | H |
| 6-502 | (2,2-Dichlorcyclopropyl)-methyl | Me | 4-Thiazolyl | H |
| 6-503 | 1-Methylprop-2-in-1-yl | Me | 4-Thiazolyl | H |
| 6-504 | 1-Cyclopropylethyl | Me | 4-Thiazolyl | H |
| 6-505 | Allyl | Me | 4-Thiazolyl | H |
| 6-506 | 3-Methylbut-2-en-1-yl | Me | 4-Thiazolyl | H |
| 6-507 | Cyclobutylmethyl | Me | 4-Thiazolyl | H |
| 6-508 | Cyclopentylmethyl | Me | 4-Thiazolyl | H |
| 6-509 | 2-Chloroprop-2-en-1-yl | Me | 4-Thiazolyl | H |
| 6-510 | Tetrahydrofuran-2-yl-methyl | Me | 4-Thiazolyl | H |
| 6-511 | (3-Methyloxetan-3-yl)-methyl | Me | 4-Thiazolyl | H |
| 6-512 | 2,2,2-Trifluorethyl | Me | 4-Thiazolyl | H |
| 6-513 | 2,2-Difluorethyl | Me | 4-Thiazolyl | H |
| 6-514 | Oxetan-3-yl | Me | 4-Thiazolyl | H |
| 6-515 | Cyclopropylmethyl | Me | 3-Br-Ph | H |
| 6-516 | Prop-2-in-1-yl | Me | 3-Br-Ph | H |
| 6-517 | But-2-in-1-yl | Me | 3-Br-Ph | H |
| 6-518 | But-3-in-2-yl | Me | 3-Br-Ph | H |
| 6-519 | Pr | Me | 3-Br-Ph | H |
| 6-520 | iPr | Me | 3-Br-Ph | H |
| 6-521 | CH₂Ph | Me | 3-Br-Ph | H |
| 6-522 | 3,3-Dichlor-2-fluorprop-2-en-1-yl | Me | 3-Br-Ph | H |
| 6-523 | (1-Methylcyclopropyl)-methyl | Me | 3-Br-Ph | H |
| 6-524 | 4-Chlorbut-2-in-1-yl | Me | 3-Br-Ph | H |
| 6-525 | (2,2-Dichlorcyclopropyl)-methyl | Me | 3-Br-Ph | H |
| 6-526 | 1-Methylprop-2-in-1-yl | Me | 3-Br-Ph | H |
| 6-527 | 1-Cyclopropylethyl | Me | 3-Br-Ph | H |
| 6-528 | Allyl | Me | 3-Br-Ph | H |
| 6-529 | 3-Methylbut-2-en-1-yl | Me | 3-Br-Ph | H |
| 6-530 | Cyclobutylmethyl | Me | 3-Br-Ph | H |
| 6-531 | Cyclopentylmethyl | Me | 3-Br-Ph | H |
| 6-532 | 2-Chloroprop-2-en-1-yl | Me | 3-Br-Ph | H |
| 6-533 | Tetrahydrofuran-2-yl-methyl | Me | 3-Br-Ph | H |
| 6-534 | (3-Methyloxetan-3-yl)-methyl | Me | 3-Br-Ph | H |
| 6-535 | 2,2,2-Trifluorethyl | Me | 3-Br-Ph | H |
| 6-536 | 2,2-Difluorethyl | Me | 3-Br-Ph | H |
| 6-537 | Oxetan-3-yl | Me | 3-Br-Ph | H |
| 6-538 | Cyclopropylmethyl | Me | 2-Cl-thiazol-4-yl | H |
| 6-539 | Prop-2-in-1-yl | Me | 2-Cl-thiazol-4-yl | H |
| 6-540 | But-2-in-1-yl | Me | 2-Cl-thiazol-4-yl | H |
| 6-541 | But-3-in-2-yl | Me | 2-Cl-thiazol-4-yl | H |
| 6-542 | Pr | Me | 2-Cl-thiazol-4-yl | H |
| 6-543 | iPr | Me | 2-Cl-thiazol-4-yl | H |
| 6-544 | CH₂Ph | Me | 2-Cl-thiazol-4-yl | H |
| 6-545 | 3,3-Dichlor-2-fluorprop-2-en-1-yl | Me | 2-Cl-thiazol-4-yl | H |
| 6-546 | (1-Methylcyclopropyl)-methyl | Me | 2-Cl-thiazol-4-yl | H |
| 6-547 | 4-Chlorbut-2-in-1-yl | Me | 2-Cl-thiazol-4-yl | H |
| 6-548 | (2,2-Dichlorcyclopropyl)-methyl | Me | 2-Cl-thiazol-4-yl | H |
| 6-549 | 1-Methylprop-2-in-1-yl | Me | 2-Cl-thiazol-4-yl | H |
| 6-550 | 1-Cyclopropylethyl | Me | 2-Cl-thiazol-4-yl | H |
| 6-551 | Allyl | Me | 2-Cl-thiazol-4-yl | H |
| 6-552 | 3-Methylbut-2-en-1-yl | Me | 2-Cl-thiazol-4-yl | H |
| 6-553 | Cyclobutylmethyl | Me | 2-Cl-thiazol-4-yl | H |
| 6-554 | Cyclopentylmethyl | Me | 2-Cl-thiazol-4-yl | H |
| 6-555 | 2-Chloroprop-2-en-1-yl | Me | 2-Cl-thiazol-4-yl | H |
| 6-556 | Tetrahydrofuran-2-yl-methyl | Me | 2-Cl-thiazol-4-yl | H |
| 6-557 | (3-Methyloxetan-3-yl)-methyl | Me | 2-Cl-thiazol-4-yl | H |
| 6-558 | 2,2,2-Trifluorethyl | Me | 2-Cl-thiazol-4-yl | H |
| 6-559 | 2,2-Difluorethyl | Me | 2-Cl-thiazol-4-yl | H |
| 6-560 | Oxetan-3-yl | Me | 2-Cl-thiazol-4-yl | H |
| 6-561 | Cyclopropylmethyl | Me | 2-Br-thiazol-4-yl | H |
| 6-562 | Prop-2-in-1-yl | Me | 2-Br-thiazol-4-yl | H |
| 6-563 | But-2-in-1-yl | Me | 2-Br-thiazol-4-yl | H |
| 6-564 | But-3-in-2-yl | Me | 2-Br-thiazol-4-yl | H |
| 6-565 | Pr | Me | 2-Br-thiazol-4-yl | H |
| 6-566 | iPr | Me | 2-Br-thiazol-4-yl | H |
| 6-567 | CH₂Ph | Me | 2-Br-thiazol-4-yl | H |
| 6-568 | 3,3-Dichlor-2-fluorprop-2-en-1-yl | Me | 2-Br-thiazol-4-yl | H |
| 6-569 | (1-Methylcyclopropyl)-methyl | Me | 2-Br-thiazol-4-yl | H |
| 6-570 | 4-Chlorbut-2-in-1-yl | Me | 2-Br-thiazol-4-yl | H |
| 6-571 | (2,2-Dichlorcyclopropyl)-methyl | Me | 2-Br-thiazol-4-yl | H |
| 6-572 | 1-Methylprop-2-in-1-yl | Me | 2-Br-thiazol-4-yl | H |
| 6-573 | 1-Cyclopropylethyl | Me | 2-Br-thiazol-4-yl | H |
| 6-574 | Allyl | Me | 2-Br-thiazol-4-yl | H |
| 6-575 | 3-Methylbut-2-en-1-yl | Me | 2-Br-thiazol-4-yl | H |
| 6-576 | Cyclobutylmethyl | Me | 2-Br-thiazol-4-yl | H |
| 6-577 | Cyclopentylmethyl | Me | 2-Br-thiazol-4-yl | H |
| 6-578 | 2-Chloroprop-2-en-1-yl | Me | 2-Br-thiazol-4-yl | H |
| 6-579 | Tetrahydrofuran-2-yl-methyl | Me | 2-Br-thiazol-4-yl | H |
| 6-580 | (3-Methyloxetan-3-yl)-methyl | Me | 2-Br-thiazol-4-yl | H |
| 6-581 | 2,2,2-Trifluorethyl | Me | 2-Br-thiazol-4-yl | H |
| 6-582 | 2,2-Difluorethyl | Me | 2-Br-thiazol-4-yl | H |
| 6-583 | Oxetan-3-yl | Me | 2-Br-thiazol-4-yl | H |
| 6-584 | Cyclopropylmethyl | Me | 5-Br-thiazol-2-yl | H |
| 6-585 | Prop-2-in-1-yl | Me | 5-Br-thiazol-2-yl | H |
| 6-586 | But-2-in-1-yl | Me | 5-Br-thiazol-2-yl | H |
| 6-587 | But-3-in-2-yl | Me | 5-Br-thiazol-2-yl | H |
| 6-588 | Pr | Me | 5-Br-thiazol-2-yl | H |
| 6-589 | iPr | Me | 5-Br-thiazol-2-yl | H |
| 6-590 | CH₂Ph | Me | 5-Br-thiazol-2-yl | H |
| 6-591 | 3,3-Dichlor-2-fluorprop-2-en-1-yl | Me | 5-Br-thiazol-2-yl | H |
| 6-592 | (1-Methylcyclopropyl)-methyl | Me | 5-Br-thiazol-2-yl | H |
| 6-593 | 4-Chlorbut-2-in-1-yl | Me | 5-Br-thiazol-2-yl | H |
| 6-594 | (2,2-Dichlorcyclopropyl)-methyl | Me | 5-Br-thiazol-2-yl | H |
| 6-595 | 1-Methylprop-2-in-1-yl | Me | 5-Br-thiazol-2-yl | H |
| 6-596 | 1-Cyclopropylethyl | Me | 5-Br-thiazol-2-yl | H |
| 6-597 | Allyl | Me | 5-Br-thiazol-2-yl | H |
| 6-598 | 3-Methylbut-2-en-1-yl | Me | 5-Br-thiazol-2-yl | H |
| 6-599 | Cyclobutylmethyl | Me | 5-Br-thiazol-2-yl | H |
| 6-600 | Cyclopentylmethyl | Me | 5-Br-thiazol-2-yl | H |
| 6-601 | 2-Chloroprop-2-en-1-yl | Me | 5-Br-thiazol-2-yl | H |
| 6-602 | Tetrahydrofuran-2-yl-methyl | Me | 5-Br-thiazol-2-yl | H |
| 6-603 | (3-Methyloxetan-3-yl)-methyl | Me | 5-Br-thiazol-2-yl | H |
| 6-604 | 2,2,2-Trifluorethyl | Me | 5-Br-thiazol-2-yl | H |
| 6-605 | 2,2-Difluorethyl | Me | 5-Br-thiazol-2-yl | H |
| 6-606 | Oxetan-3-yl | Me | 5-Br-thiazol-2-yl | H |
| 6-607 | Cyclopropylmethyl | Me | 5-Cl-thiazol-2-yl | H |
| 6-608 | Prop-2-in-1-yl | Me | 5-Cl-thiazol-2-yl | H |
| 6-609 | But-2-in-1-yl | Me | 5-Cl-thiazol-2-yl | H |
| 6-610 | But-3-in-2-yl | Me | 5-Cl-thiazol-2-yl | H |
| 6-611 | Pr | Me | 5-Cl-thiazol-2-yl | H |
| 6-612 | iPr | Me | 5-Cl-thiazol-2-yl | H |
| 6-613 | CH₂Ph | Me | 5-Cl-thiazol-2-yl | H |
| 6-614 | 3,3-Dichlor-2-fluorprop-2-en-1-yl | Me | 5-Cl-thiazol-2-yl | H |
| 6-615 | (1-Methylcyclopropyl)-methyl | Me | 5-Cl-thiazol-2-yl | H |
| 6-616 | 4-Chlorbut-2-in-1-yl | Me | 5-Cl-thiazol-2-yl | H |
| 6-617 | (2,2-Dichlorcyclopropyl)-methyl | Me | 5-Cl-thiazol-2-yl | H |
| 6-618 | 1-Methylprop-2-in-1-yl | Me | 5-Cl-thiazol-2-yl | H |
| 6-619 | 1-Cyclopropylethyl | Me | 5-Cl-thiazol-2-yl | H |
| 6-620 | Allyl | Me | 5-Cl-thiazol-2-yl | H |
| 6-621 | 3-Methylbut-2-en-1-yl | Me | 5-Cl-thiazol-2-yl | H |
| 6-622 | Cyclobutylmethyl | Me | 5-Cl-thiazol-2-yl | H |
| 6-623 | Cyclopentylmethyl | Me | 5-Cl-thiazol-2-yl | H |
| 6-624 | 2-Chloroprop-2-en-1-yl | Me | 5-Cl-thiazol-2-yl | H |
| 6-625 | Tetrahydrofuran-2-yl-methyl | Me | 5-Cl-thiazol-2-yl | H |
| 6-626 | (3-Methyloxetan-3-yl)-methyl | Me | 5-Cl-thiazol-2-yl | H |
| 6-627 | 2,2,2-Trifluorethyl | Me | 5-Cl-thiazol-2-yl | H |
| 6-628 | 2,2-Difluorethyl | Me | 5-Cl-thiazol-2-yl | H |
| 6-629 | Oxetan-3-yl | Me | 5-Cl-thiazol-2-yl | H |
| 6-630 | Cyclopropylmethyl | Me | 5-OSO₂Me-pyridin-2-yl | H |
| 6-631 | Prop-2-in-1-yl | Me | 5-OSO₂Me-pyridin-2-yl | H |
| 6-632 | But-3-in-2-yl | Me | 5-OSO₂Me-pyridin-2-yl | H |
| 6-633 | iPr | Me | 5-OSO₂Me-pyridin-2-yl | H |
| 6-634 | CH₂Ph | Me | 5-OSO₂Me-pyridin-2-yl | H |
| 6-635 | (2,2-Dichlorcyclopropyl)-methyl | Me | 5-OSO₂Me-pyridin-2-yl | H |
| 6-636 | Allyl | Me | 5-OSO₂Me-pyridin-2-yl | H |
| 6-637 | 2,2,2-Trifluorethyl | Me | 5-OSO₂Me-pyridin-2-yl | H |
| 6-638 | 2,2-Difluorethyl | Me | 5-OSO₂Me-pyridin-2-yl | H |
| 6-639 | Oxetan-3-yl | Me | 5-OSO₂Me-pyridin-2-yl | H |
| 6-640 | Cyclopropylmethyl | Me | 6-Cl-1,3-benzothiazol-2-yl | H |
| 6-641 | Prop-2-in-1-yl | Me | 6-Cl-1,3-benzothiazol-2-yl | H |
| 6-642 | But-3-in-2-yl | Me | 6-Cl-1,3-benzothiazol-2-yl | H |
| 6-643 | iPr | Me | 6-Cl-1,3-benzothiazol-2-yl | H |
| 6-644 | CH₂Ph | Me | 6-Cl-1,3-benzothiazol-2-yl | H |
| 6-645 | (2,2-Dichlorcyclopropyl)-methyl | Me | 6-Cl-1,3-benzothiazol-2-yl | H |
| 6-646 | Allyl | Me | 6-Cl-1,3-benzothiazol-2-yl | H |
| 6-647 | 2,2,2-Trifluorethyl | Me | 6-Cl-1,3-benzothiazol-2-yl | H |
| 6-648 | 2,2-Difluorethyl | Me | 6-Cl-1,3-benzothiazol-2-yl | H |
| 6-649 | Oxetan-3-yl | Me | 6-Cl-1,3-benzothiazol-2-yl | H |
| 6-650 | Cyclopropylmethyl | Me | 6-Br-1,3-benzothiazol-2-yl | H |
| 6-651 | Prop-2-in-1-yl | Me | 6-Br-1,3-benzothiazol-2-yl | H |
| 6-652 | But-3-in-2-yl | Me | 6-Br-1,3-benzothiazol-2-yl | H |
| 6-653 | iPr | Me | 6-Br-1,3-benzothiazol-2-yl | H |
| 6-654 | CH₂Ph | Me | 6-Br-1,3-benzothiazol-2-yl | H |
| 6-655 | (2,2-Dichlorcyclopropyl)-methyl | Me | 6-Br-1,3-benzothiazol-2-yl | H |
| 6-656 | Allyl | Me | 6-Br-1,3-benzothiazol-2-yl | H |
| 6-657 | 2,2,2-Trifluorethyl | Me | 6-Br-1,3-benzothiazol-2-yl | H |
| 6-658 | 2,2-Difluorethyl | Me | 6-Br-1,3-benzothiazol-2-yl | H |
| 6-659 | Oxetan-3-yl | Me | 6-Br-1,3-benzothiazol-2-yl | H |
| 6-660 | Cyclopropylmethyl | Me | 1,3-Benzoxazol-2-yl | H |
| 6-661 | Prop-2-in-1-yl | Me | 1,3-Benzoxazol-2-yl | H |
| 6-662 | But-3-in-2-yl | Me | 1,3-Benzoxazol-2-yl | H |
| 6-663 | iPr | Me | 1,3-Benzoxazol-2-yl | H |
| 6-664 | CH₂Ph | Me | 1,3-Benzoxazol-2-yl | H |
| 6-665 | (2,2-Dichlorcyclopropyl)-methyl | Me | 1,3-Benzoxazol-2-yl | H |
| 6-666 | Allyl | Me | 1,3-Benzoxazol-2-yl | H |
| 6-667 | 2,2,2-Trifluorethyl | Me | 1,3-Benzoxazol-2-yl | H |
| 6-668 | 2,2-Difluorethyl | Me | 1,3-Benzoxazol-2-yl | H |
| 6-669 | Oxetan-3-yl | Me | 1,3-Benzoxazol-2-yl | H |
| 6-670 | Cyclopropylmethyl | Me | 6-Cl-1,3-benzoxazol-2-yl | H |
| 6-671 | Prop-2-in-1-yl | Me | 6-Cl-1,3-benzoxazol-2-yl | H |
| 6-672 | But-3-in-2-yl | Me | 6-Cl-1,3-benzoxazol-2-yl | H |
| 6-673 | iPr | Me | 6-Cl-1,3-benzoxazol-2-yl | H |
| 6-674 | CH₂Ph | Me | 6-Cl-1,3-benzoxazol-2-yl | H |
| 6-675 | (2,2-Dichlorcyclopropyl)-methyl | Me | 6-Cl-1,3-benzoxazol-2-yl | H |
| 6-676 | Allyl | Me | 6-Cl-1,3-benzoxazol-2-yl | H |
| 6-677 | 2,2,2-Trifluorethyl | Me | 6-Cl-1,3-benzoxazol-2-yl | H |
| 6-678 | 2,2-Difluorethyl | Me | 6-Cl-1,3-benzoxazol-2-yl | H |
| 6-679 | Oxetan-3-yl | Me | 6-Cl-1,3-benzoxazol-2-yl | H |
| 6-680 | Cyclopropylmethyl | Me | 6-Br-1,3-benzoxazol-2-yl | H |
| 6-681 | Prop-2-in-1-yl | Me | 6-Br-1,3-benzoxazol-2-yl | H |
| 6-682 | But-3-in-2-yl | Me | 6-Br-1,3-benzoxazol-2-yl | H |
| 6-683 | iPr | Me | 6-Br-1,3-benzoxazol-2-yl | H |
| 6-684 | CH₂Ph | Me | 6-Br-1,3-benzoxazol-2-yl | H |
| 6-685 | (2,2-Dichlorcyclopropyl)-methyl | Me | 6-Br-1,3-benzoxazol-2-yl | H |
| 6-686 | Allyl | Me | 6-Br-1,3-benzoxazol-2-yl | H |
| 6-687 | 2,2,2-Trifluorethyl | Me | 6-Br-1,3-benzoxazol-2-yl | H |
| 6-688 | 2,2-Difluorethyl | Me | 6-Br-1,3-benzoxazol-2-yl | H |
| 6-689 | Oxetan-3-yl | Me | 6-Br-1,3-benzoxazol-2-yl | H |
| 6-690 | Cyclopropylmethyl | Me | 7-Cl-1,3-benzoxazol-2-yl | H |
| 6-691 | Prop-2-in-1-yl | Me | 7-Cl-1,3-benzoxazol-2-yl | H |
| 6-692 | But-3-in-2-yl | Me | 7-Cl-1,3-benzoxazol-2-yl | H |
| 6-693 | iPr | Me | 7-Cl-1,3-benzoxazol-2-yl | H |
| 6-694 | CH₂Ph | Me | 7-Cl-1,3-benzoxazol-2-yl | H |
| 6-695 | (2,2-Dichlorcyclopropyl)-methyl | Me | 7-Cl-1,3-benzoxazol-2-yl | H |
| 6-696 | Allyl | Me | 7-Cl-1,3-benzoxazol-2-yl | H |
| 6-697 | 2,2,2-Trifluorethyl | Me | 7-Cl-1,3-benzoxazol-2-yl | H |
| 6-698 | 2,2-Difluorethyl | Me | 7-Cl-1,3-benzoxazol-2-yl | H |
| 6-699 | Oxetan-3-yl | Me | 7-Cl-1,3-benzoxazol-2-yl | H |
| 6-700 | Cyclopropylmethyl | Me | 5-I-pyridin-2-yl | H |
| 6-701 | Prop-2-in-1-yl | Me | 5-I-pyridin-2-yl | H |
| 6-702 | But-3-in-2-yl | Me | 5-I-pyridin-2-yl | H |
| 6-703 | iPr | Me | 5-I-pyridin-2-yl | H |
| 6-704 | CH₂Ph | Me | 5-I-pyridin-2-yl | H |
| 6-705 | (2,2-Dichlorcyclopropyl)-methyl | Me | 5-I-pyridin-2-yl | H |
| 6-706 | Allyl | Me | 5-I-pyridin-2-yl | H |
| 6-707 | 2,2,2-Trifluorethyl | Me | 5-I-pyridin-2-yl | H |
| 6-708 | 2,2-Difluorethyl | Me | 5-I-pyridin-2-yl | H |
| 6-709 | Oxetan-3-yl | Me | 5-I-pyridin-2-yl | H |
| 6-710 | Cyclopropylmethyl | Me | 5-NH₂-pyridin-2-yl | H |
| 6-711 | Prop-2-in-1-yl | Me | 5-NH₂-pyridin-2-yl | H |
| 6-712 | But-3-in-2-yl | Me | 5-NH₂-pyridin-2-yl | H |
| 6-713 | iPr | Me | 5-NH₂-pyridin-2-yl | H |
| 6-714 | CH₂Ph | Me | 5-NH₂-pyridin-2-yl | H |
| 6-715 | (2,2-Dichlorcyclopropyl)-methyl | Me | 5-NH₂-pyridin-2-yl | H |
| 6-716 | Allyl | Me | 5-NH₂-pyridin-2-yl | H |
| 6-717 | 2,2,2-Trifluorethyl | Me | 5-NH₂-pyridin-2-yl | H |
| 6-718 | 2,2-Difluorethyl | Me | 5-NH₂-pyridin-2-yl | H |
| 6-719 | Oxetan-3-yl | Me | 5-NH₂-pyridin-2-yl | H |
| 6-720 | Cyclopropylmethyl | Me | 5-OH-pyridin-2-yl | H |
| 6-721 | Prop-2-in-1-yl | Me | 5-OH-pyridin-2-yl | H |
| 6-722 | But-3-in-2-yl | Me | 5-OH-pyridin-2-yl | H |
| 6-723 | iPr | Me | 5-OH-pyridin-2-yl | H |
| 6-724 | CH₂Ph | Me | 5-OH-pyridin-2-yl | H |
| 6-725 | (2,2-Dichlorcyclopropyl)-methyl | Me | 5-OH-pyridin-2-yl | H |
| 6-726 | Allyl | Me | 5-OH-pyridin-2-yl | H |
| 6-727 | 2,2,2-Trifluorethyl | Me | 5-OH-pyridin-2-yl | H |
| 6-728 | 2,2-Difluorethyl | Me | 5-OH-pyridin-2-yl | H |
| 6-729 | Oxetan-3-yl | Me | 5-OH-pyridin-2-yl | H |
| 6-730 | Cyclopropylmethyl | Me | 5-OCHF₂-pyridin-2-yl | H |
| 6-731 | Prop-2-in-1-yl | Me | 5-OCHF₂-pyridin-2-yl | H |
| 6-732 | But-3-in-2-yl | Me | 5-OCHF₂-pyridin-2-yl | H |
| 6-733 | iPr | Me | 5-OCHF₂-pyridin-2-yl | H |
| 6-734 | CH₂Ph | Me | 5-OCHF₂-pyridin-2-yl | H |
| 6-735 | (2,2-Dichlorcyclopropyl)-methyl | Me | 5-OCHF₂-pyridin-2-yl | H |
| 6-736 | Allyl | Me | 5-OCHF₂-pyridin-2-yl | H |
| 6-737 | 2,2,2-Trifluorethyl | Me | 5-OCHF₂-pyridin-2-yl | H |
| 6-738 | 2,2-Difluorethyl | Me | 5-OCHF₂-pyridin-2-yl | H |
| 6-739 | Oxetan-3-yl | Me | 5-OCHF₂-pyridin-2-yl | H |
| 6-740 | Cyclopropylmethyl | Me | 5-MeO-pyridin-2-yl | H |
| 6-741 | Prop-2-in-1-yl | Me | 5-MeO-pyridin-2-yl | H |
| 6-742 | But-3-in-2-yl | Me | 5-MeO-pyridin-2-yl | H |
| 6-743 | iPr | Me | 5-MeO-pyridin-2-yl | H |
| 6-744 | CH₂Ph | Me | 5-MeO-pyridin-2-yl | H |
| 6-745 | (2,2-Dichlorcyclopropyl)-methyl | Me | 5-MeO-pyridin-2-yl | H |
| 6-746 | Allyl | Me | 5-MeO-pyridin-2-yl | H |
| 6-747 | 2,2,2-Trifluorethyl | Me | 5-MeO-pyridin-2-yl | H |
| 6-748 | 2,2-Difluorethyl | Me | 5-MeO-pyridin-2-yl | H |
| 6-749 | Oxetan-3-yl | Me | 5-MeO-pyridin-2-yl | H |
| 6-750 | Cyclopropylmethyl | Me | 5-MeS-pyridin-2-yl | H |
| 6-751 | Prop-2-in-1-yl | Me | 5-MeS-pyridin-2-yl | H |
| 6-752 | But-3-in-2-yl | Me | 5-MeS-pyridin-2-yl | H |
| 6-753 | iPr | Me | 5-MeS-pyridin-2-yl | H |
| 6-754 | CH₂Ph | Me | 5-MeS-pyridin-2-yl | H |
| 6-755 | (2,2-Dichlorcyclopropyl)-methyl | Me | 5-MeS-pyridin-2-yl | H |
| 6-756 | Allyl | Me | 5-MeS-pyridin-2-yl | H |
| 6-757 | 2,2,2-Trifluorethyl | Me | 5-MeS-pyridin-2-yl | H |
| 6-758 | 2,2-Difluorethyl | Me | 5-MeS-pyridin-2-yl | H |
| 6-759 | Oxetan-3-yl | Me | 5-MeS-pyridin-2-yl | H |
| 6-760 | Cyclopropylmethyl | Me | 5-NHMe-pyridin-2-yl | H |
| 6-761 | Prop-2-in-1-yl | Me | 5-NHMe-pyridin-2-yl | H |
| 6-762 | But-3-in-2-yl | Me | 5-NHMe-pyridin-2-yl | H |
| 6-763 | iPr | Me | 5-NHMe-pyridin-2-yl | H |
| 6-764 | CH₂Ph | Me | 5-NHMe-pyridin-2-yl | H |
| 6-765 | (2,2-Dichlorcyclopropyl)-methyl | Me | 5-NHMe-pyridin-2-yl | H |
| 6-766 | Allyl | Me | 5-NHMe-pyridin-2-yl | H |
| 6-767 | 2,2,2-Trifluorethyl | Me | 5-NHMe-pyridin-2-yl | H |
| 6-768 | 2,2-Difluorethyl | Me | 5-NHMe-pyridin-2-yl | H |
| 6-769 | Oxetan-3-yl | Me | 5-NHMe-pyridin-2-yl | H |
| 6-770 | Cyclopropylmethyl | Me | 5-NMe₂-pyridin-2-yl | H |
| 6-771 | Prop-2-in-1-yl | Me | 5-NMe₂-pyridin-2-yl | H |
| 6-772 | But-3-in-2-yl | Me | 5-NMe₂-pyridin-2-yl | H |
| 6-773 | iPr | Me | 5-NMe₂-pyridin-2-yl | H |
| 6-774 | CH₂Ph | Me | 5-NMe₂-pyridin-2-yl | H |
| 6-775 | (2,2-Dichlorcyclopropyl)-methyl | Me | 5-NMe₂-pyridin-2-yl | H |
| 6-776 | Allyl | Me | 5-NMe₂-pyridin-2-yl | H |
| 6-777 | 2,2,2-Trifluorethyl | Me | 5-NMe₂-pyridin-2-yl | H |
| 6-778 | 2,2-Difluorethyl | Me | 5-NMe₂-pyridin-2-yl | H |
| 6-779 | Oxetan-3-yl | Me | 5-NMe₂-pyridin-2-yl | H |
| 6-780 | 3-Hydroxy-but-2-yl | Me | 4-Cl-Ph | H |
| 6-781 | 3-Hydroxy-but-2-yl | Me | 4-Br-Ph | H |
| 6-782 | 3-Hydroxy-but-2-yl | Me | 5-Cl-pyridin-2-yl | H |
| 6-783 | 3-Hydroxy-but-2-yl | Me | 5-Br-pyridin-2-yl | H |
| 6-784 | 3-Hydroxy-but-2-yl | Me | 5-Cl-2-thienyl | H |
| 6-785 | 3-Hydroxy-but-2-yl | Me | 5-Br-2-thienyl | H |
| 6-786 | 3-Ethylpent-1-in-3-yl | Me | 4-Cl-Ph | H |
| 6-787 | 3-Ethylpent-1-in-3-yl | Me | 4-Br-Ph | H |
| 6-788 | 3-Ethylpent-1-in-3-yl | Me | 5-Cl-pyridin-2-yl | H |
| 6-789 | 3-Ethylpent-1-in-3-yl | Me | 5-Br-pyridin-2-yl | H |
| 6-790 | 3-Ethylpent-1-in-3-yl | Me | 5-Cl-2-thienyl | H |
| 6-791 | 3-Ethylpent-1-in-3-yl | Me | 5-Br-2-thienyl | H |
| 6-792 | Difluormethyl | Me | 4-Cl-Ph | H |
| 6-793 | Difluormethyl | Me | 4-Br-Ph | H |
| 6-794 | Difluormethyl | Me | 5-Cl-pyridin-2-yl | H |
| 6-795 | Difluormethyl | Me | 5-Br-pyridin-2-yl | H |
| 6-796 | Difluormethyl | Me | 5-Cl-2-thienyl | H |
| 6-797 | Difluormethyl | Me | 5-Br-2-thienyl | H |
| 6-798 | 2,2,3,3-Tetrafluorpropyl | Me | 4-Cl-Ph | H |
| 6-799 | 2,2,3,3-Tetrafluorpropyl | Me | 4-Br-Ph | H |
| 6-800 | 2,2,3,3-Tetrafluorpropyl | Me | 5-Cl-pyridin-2-yl | H |
| 6-801 | 2,2,3,3-Tetrafluorpropyl | Me | 5-Br-pyridin-2-yl | H |
| 6-802 | 2,2,3,3-Tetrafluorpropyl | Me | 5-Cl-2-thienyl | H |
| 6-803 | 2,2,3,3-Tetrafluorpropyl | Me | 5-Br-2-thienyl | H |
| 6-804 | 4,4,4-Trifluorbutyl | Me | 4-Cl-Ph | H |
| 6-805 | 4,4,4-Trifluorbutyl | Me | 4-Br-Ph | H |
| 6-806 | 4,4,4-Trifluorbutyl | Me | 5-Cl-pyridin-2-yl | H |
| 6-807 | 4,4,4-Trifluorbutyl | Me | 5-Br-pyridin-2-yl | H |
| 6-808 | 4,4,4-Trifluorbutyl | Me | 5-Cl-2-thienyl | H |
| 6-809 | 4,4,4-Trifluorbutyl | Me | 5-Br-2-thienyl | H |
| 6-810 | Acetoxy-methyl | Me | 4-Cl-Ph | H |
| 6-811 | Acetoxy-methyl | Me | 4-Br-Ph | H |
| 6-812 | Acetoxy-methyl | Me | 5-Cl-pyridin-2-yl | H |
| 6-813 | Acetoxy-methyl | Me | 5-Br-pyridin-2-yl | H |
| 6-814 | Acetoxy-methyl | Me | 5-Cl-2-thienyl | H |
| 6-815 | Acetoxy-methyl | Me | 5-Br-2-thienyl | H |
| 6-816 | 2-Chlorethyl | Me | 4-Cl-Ph | H |
| 6-817 | 2-Chlorethyl | Me | 4-Br-Ph | H |
| 6-818 | 2-Chlorethyl | Me | 5-Cl-pyridin-2-yl | H |
| 6-819 | 2-Chlorethyl | Me | 5-Br-pyridin-2-yl | H |
| 6-820 | 2-Chlorethyl | Me | 5-Cl-2-thienyl | H |
| 6-821 | 2-Chlorethyl | Me | 5-Br-2-thienyl | H |
| 6-822 | 3-Fluorpropyl | Me | 4-Cl-Ph | H |
| 6-823 | 3-Fluorpropyl | Me | 4-Br-Ph | H |
| 6-824 | 3-Fluorpropyl | Me | 5-Cl-pyridin-2-yl | H |
| 6-825 | 3-Fluorpropyl | Me | 5-Br-pyridin-2-yl | H |
| 6-826 | 3-Fluorpropyl | Me | 5-Cl-2-thienyl | H |
| 6-827 | 3-Fluorpropyl | Me | 5-Br-2-thienyl | H |
| 6-828 | 2-Ethoxyethyl | Me | 4-Cl-Ph | H |
| 6-829 | 2-Ethoxyethyl | Me | 4-Br-Ph | H |
| 6-830 | 2-Ethoxyethyl | Me | 5-Cl-pyridin-2-yl | H |
| 6-831 | 2-Ethoxyethyl | Me | 5-Br-pyridin-2-yl | H |
| 6-832 | 2-Ethoxyethyl | Me | 5-Cl-2-thienyl | H |
| 6-833 | 2-Ethoxyethyl | Me | 5-Br-2-thienyl | H |
| 6-834 | 2-Propan-1-ol | Me | 4-Cl-Ph | H |
| 6-835 | 2-Propan-1-ol | Me | 4-Br-Ph | H |
| 6-836 | 1-Hydroxy-prop-2-yl | Me | 5-Cl-pyridin-2-yl | H |
| 6-837 | 1-Hydroxy-prop-2-yl | Me | 5-Br-pyridin-2-yl | H |
| 6-838 | 1-Hydroxy-prop-2-yl | Me | 5-Cl-2-thienyl | H |
| 6-839 | 1-Hydroxy-prop-2-yl | Me | 5-Br-2-thienyl | H |
| 6-840 | 2-Methoxybut-1-yl | Me | 4-Cl-Ph | H |
| 6-841 | 2-Methoxybut-1-yl | Me | 4-Br-Ph | H |
| 6-842 | 2-Methoxybut-1-yl | Me | 5-Cl-pyridin-2-yl | H |
| 6-843 | 2-Methoxybut-1-yl | Me | 5-Br-pyridin-2-yl | H |
| 6-844 | 2-Methoxybut-1-yl | Me | 5-Cl-2-thienyl | H |
| 6-845 | 2-Methoxybut-1-yl | Me | 5-Br-2-thienyl | H |
| 6-846 | 1,3-Difluorpropan-2-yl | Me | 4-Cl-Ph | H |
| 6-847 | 1,3-Difluorpropan-2-yl | Me | 4-Br-Ph | H |
| 6-848 | 1,3-Difluorpropan-2-yl | Me | 5-Cl-pyridin-2-yl | H |
| 6-849 | 1,3-Difluorpropan-2-yl | Me | 5-Br-pyridin-2-yl | H |
| 6-850 | 1,3-Difluorpropan-2-yl | Me | 5-Cl-2-thienyl | H |
| 6-851 | 1,3-Difluorpropan-2-yl | Me | 5-Br-2-thienyl | H |
| 6-852 | 2,3-Dimethoxypropyl | Me | 4-Cl-Ph | H |
| 6-853 | 2,3-Dimethoxypropyl | Me | 4-Br-Ph | H |
| 6-854 | 2,3-Dimethoxypropyl | Me | 5-Cl-pyridin-2-yl | H |
| 6-855 | 2,3-Dimethoxypropyl | Me | 5-Br-pyridin-2-yl | H |
| 6-856 | 2,3-Dimethoxypropyl | Me | 5-Cl-2-thienyl | H |
| 6-857 | 2,3-Dimethoxypropyl | Me | 5-Br-2-thienyl | H |
| 6-858 | 1,3-dioxolan-4-yl-methyl | Me | 4-Cl-Ph | H |
| 6-859 | 1,3-dioxolan-4-yl-methyl | Me | 4-Br-Ph | H |
| 6-860 | 1,3-dioxolan-4-yl-methyl | Me | 5-Cl-pyridin-2-yl | H |
| 6-861 | 1,3-dioxolan-4-yl-methyl | Me | 5-Br-pyridin-2-yl | H |
| 6-862 | 1,3-dioxolan-4-yl-methyl | Me | 5-Cl-2-thienyl | H |
| 6-863 | 1,3-dioxolan-4-yl-methyl | Me | 5-Br-2-thienyl | H |
| 6-864 | 1,1,1,4,4,4-Hexafluorbutan-2-yl | Me | 4-Cl-Ph | H |
| 6-865 | 1,1,1,4,4,4-Hexafluorbutan-2-yl | Me | 4-Br-Ph | H |
| 6-866 | 1,1,1,4,4,4-Hexafluorbutan-2-yl | Me | 5-Cl-pyridin-2-yl | H |
| 6-867 | 1,1,1,4,4,4-Hexafluorbutan-2-yl | Me | 5-Br-pyridin-2-yl | H |
| 6-868 | 1,1,1,4,4,4-Hexafluorbutan-2-yl | Me | 5-Cl-2-thienyl | H |
| 6-869 | 1,1,1,4,4,4-Hexafluorbutan-2-yl | Me | 5-Br-2-thienyl | H |
| 6-870 | 1,1-Difluorpropan-2-yl | Me | 4-Cl-Ph | H |
| 6-871 | 1,1-Difluorpropan-2-yl | Me | 4-Br-Ph | H |
| 6-872 | 1,1-Difluorpropan-2-yl | Me | 5-Cl-pyridin-2-yl | H |
| 6-873 | 1,1-Difluorpropan-2-yl | Me | 5-Br-pyridin-2-yl | H |
| 6-874 | 1,1-Difluorpropan-2-yl | Me | 5-Cl-2-thienyl | H |
| 6-875 | 1,1-Difluorpropan-2-yl | Me | 5-Br-2-thienyl | H |
| 6-876 | 1-Fluorpropan-2-yl | Me | 4-Cl-Ph | H |
| 6-877 | 1-Fluorpropan-2-yl | Me | 4-Br-Ph | H |
| 6-878 | 1-Fluorpropan-2-yl | Me | 5-Cl-pyridin-2-yl | H |
| 6-879 | 1-Fluorpropan-2-yl | Me | 5-Br-pyridin-2-yl | H |
| 6-880 | 1-Fluorpropan-2-yl | Me | 5-Cl-2-thienyl | H |
| 6-881 | 1-Fluorpropan-2-yl | Me | 5-Br-2-thienyl | H |
| 6-882 | 1-Brompropan-2-yl | Me | 4-Cl-Ph | H |
| 6-883 | 1-Brompropan-2-yl | Me | 4-Br-Ph | H |
| 6-884 | 1-Brompropan-2-yl | Me | 5-Cl-pyridin-2-yl | H |
| 6-885 | 1-Brompropan-2-yl | Me | 5-Br-pyridin-2-yl | H |
| 6-886 | 1-Brompropan-2-yl | Me | 5-Cl-2-thienyl | H |
| 6-887 | 1-Brompropan-2-yl | Me | 5-Br-2-thienyl | H |
| 6-888 | 1-Chlorpropan-2-yl | Me | 4-Cl-Ph | H |
| 6-889 | 1-Chlorpropan-2-yl | Me | 4-Br-Ph | H |
| 6-890 | 1-Chlorpropan-2-yl | Me | 5-Cl-pyridin-2-yl | H |
| 6-891 | 1-Chlorpropan-2-yl | Me | 5-Br-pyridin-2-yl | H |
| 6-892 | 1-Chlorpropan-2-yl | Me | 5-Cl-2-thienyl | H |
| 6-893 | 1-Chlorpropan-2-yl | Me | 5-Br-2-thienyl | H |
| 6-894 | 2-Isopropoxyethyl | Me | 4-Cl-Ph | H |
| 6-895 | 2-Isopropoxyethyl | Me | 4-Br-Ph | H |
| 6-896 | 2-Isopropoxyethyl | Me | 5-Cl-pyridin-2-yl | H |
| 6-897 | 2-Isopropoxyethyl | Me | 5-Br-pyridin-2-yl | H |
| 6-898 | 2-Isopropoxyethyl | Me | 5-Cl-2-thienyl | H |
| 6-899 | 2-Isopropoxyethyl | Me | 5-Br-2-thienyl | H |
| 6-900 | Tetrahydrofuran-3-yl | Me | 4-Cl-Ph | H |
| 6-901 | Tetrahydrofuran-3-yl | Me | 4-Br-Ph | H |
| 6-902 | Tetrahydrofuran-3-yl | Me | 5-Cl-pyridin-2-yl | H |
| 6-903 | Tetrahydrofuran-3-yl | Me | 5-Br-pyridin-2-yl | H |
| 6-904 | Tetrahydrofuran-3-yl | Me | 5-Cl-2-thienyl | H |
| 6-905 | Tetrahydrofuran-3-yl | Me | 5-Br-2-thienyl | H |
| 6-906 | 2-(2,2,2-Trifluorethoxy)ethyl | Me | 4-Cl-Ph | H |
| 6-907 | 2-(2,2,2-Trifluorethoxy)ethyl | Me | 4-Br-Ph | H |
| 6-908 | 2-(2,2,2-Trifluorethoxy)ethyl | Me | 5-Cl-pyridin-2-yl | H |
| 6-909 | 2-(2,2,2-Trifluorethoxy)ethyl | Me | 5-Br-pyridin-2-yl | H |
| 6-910 | 2-(2,2,2-Trifluorethoxy)ethyl | Me | 5-Cl-2-thienyl | H |
| 6-911 | 2-(2,2,2-Trifluorethoxy)ethyl | Me | 5-Br-2-thienyl | H |
| 6-912 | (3,3,4,4,4-Pentafluorbutan-2-yl | Me | 4-Cl-Ph | H |
| 6-913 | (3,3,4,4,4-Pentafluorbutan-2-yl | Me | 4-Br-Ph | H |
| 6-914 | (3,3,4,4,4-Pentafluorbutan-2-yl | Me | 5-Cl-pyridin-2-yl | H |
| 6-915 | (3,3,4,4,4-Pentafluorbutan-2-yl | Me | 5-Br-pyridin-2-yl | H |
| 6-916 | (3,3,4,4,4-Pentafluorbutan-2-yl | Me | 5-Cl-2-thienyl | H |
| 6-917 | (3,3,4,4,4-Pentafluorbutan-2-yl | Me | 5-Br-2-thienyl | H |
| 6-918 | 1-(N,N-Dimethylaminocarbonyl)-eth-1-yl | Me | 4-Cl-Ph | H |
| 6-919 | 1-(N,N-Dimethylaminocarbonyl)-eth-1-yl | Me | 4-Br-Ph | H |
| 6-920 | 1-(N,N-Dimethylaminocarbonyl)-eth-1-yl | Me | 5-Cl-pyridin-2-yl | H |
| 6-921 | 1-(N,N-Dimethylaminocarbonyl)-eth-1-yl | Me | 5-Br-pyridin-2-yl | H |
| 6-922 | 1-(N,N-Dimethylaminocarbonyl)-eth-1-yl | Me | 5-Cl-2-thienyl | H |
| 6-923 | 1-(N,N-Dimethylaminocarbonyl)-eth-1-yl | Me | 5-Br-2-thienyl | H |
| 6-924 | (1,3-dioxan-2-yl)-methyl | Me | 4-Cl-Ph | H |
| 6-925 | (1,3-dioxan-2-yl)-methyl | Me | 4-Br-Ph | H |
| 6-926 | (1,3-dioxan-2-yl)-methyl | Me | 5-Cl-pyridin-2-yl | H |
| 6-927 | (1,3-dioxan-2-yl)-methyl | Me | 5-Br-pyridin-2-yl | H |
| 6-928 | (1,3-dioxan-2-yl)-methyl | Me | 5-Cl-2-thienyl | H |
| 6-929 | (1,3-dioxan-2-yl)-methyl | Me | 5-Br-2-thienyl | H |
| 6-930 | 1,1,1-Trifluorbutan-2-yl | Me | 4-Cl-Ph | H |
| 6-931 | 1,1,1-Trifluorbutan-2-yl | Me | 4-Br-Ph | H |
| 6-932 | 1,1,1-Trifluorbutan-2-yl | Me | 5-Cl-pyridin-2-yl | H |
| 6-933 | 1,1,1-Trifluorbutan-2-yl | Me | 5-Br-pyridin-2-yl | H |
| 6-934 | 1,1,1-Trifluorbutan-2-yl | Me | 5-Cl-2-thienyl | H |
| 6-935 | 1,1,1-Trifluorbutan-2-yl | Me | 5-Br-2-thienyl | H |
| 6-936 | 2-(But-2-yliden-aminooxy)-eth-1-yl | Me | 4-Cl-Ph | H |
| 6-937 | 2-(But-2-yliden-aminooxy)-eth-1-yl | Me | 4-Br-Ph | H |
| 6-938 | 2-(But-2-yliden-aminooxy)-eth-1-yl | Me | 5-Cl-pyridin-2-yl | H |
| 6-939 | 2-(But-2-yliden-aminooxy)-eth-1-yl | Me | 5-Br-pyridin-2-yl | H |
| 6-940 | 2-(But-2-yliden-aminooxy)-eth-1-yl | Me | 5-Cl-2-thienyl | H |
| 6-941 | 2-(But-2-yliden-aminooxy)-eth-1-yl | Me | 5-Br-2-thienyl | H |
| 6-942 | Oxetan-2-yl-methyl | Me | 4-Cl-Ph | H |
| 6-943 | Oxetan-2-yl-methyl | Me | 4-Br-Ph | H |
| 6-944 | Oxetan-2-yl-methyl | Me | 5-Cl-pyridin-2-yl | H |
| 6-945 | Oxetan-2-yl-methyl | Me | 5-Br-pyridin-2-yl | H |
| 6-946 | Oxetan-2-yl-methyl | Me | 5-Cl-2-thienyl | H |
| 6-947 | Oxetan-2-yl-methyl | Me | 5-Br-2-thienyl | H |
| 6-948 | 2,2-Dimethoxyethyl | Me | 4-Cl-Ph | H |
| 6-949 | 2,2-Dimethoxyethyl | Me | 4-Br-Ph | H |
| 6-950 | 2,2-Dimethoxyethyl | Me | 5-Cl-pyridin-2-yl | H |
| 6-951 | 2,2-Dimethoxyethyl | Me | 5-Br-pyridin-2-yl | H |
| 6-952 | 2,2-Dimethoxyethyl | Me | 5-Cl-2-thienyl | H |
| 6-953 | 2,2-Dimethoxyethyl | Me | 5-Br-2-thienyl | H |
| 6-954 | 1-Chlorpropyl | Me | 4-Cl-Ph | H |
| 6-955 | 1-Chlorpropyl | Me | 4-Br-Ph | H |
| 6-956 | 1-Chlorpropyl | Me | 5-Cl-pyridin-2-yl | H |
| 6-957 | 1-Chlorpropyl | Me | 5-Br-pyridin-2-yl | H |
| 6-958 | 1-Chlorpropyl | Me | 5-Cl-2-thienyl | H |
| 6-959 | 1-Chlorpropyl | Me | 5-Br-2-thienyl | H |
| 6-960 | 4-Chlorbutan-2-yl | Me | 4-Cl-Ph | H |
| 6-961 | 4-Chlorbutan-2-yl | Me | 4-Br-Ph | H |
| 6-962 | 4-Chlorbutan-2-yl | Me | 5-Cl-pyridin-2-yl | H |
| 6-963 | 4-Chlorbutan-2-yl | Me | 5-Br-pyridin-2-yl | H |
| 6-964 | 4-Chlorbutan-2-yl | Me | 5-Cl-2-thienyl | H |
| 6-965 | 4-Chlorbutan-2-yl | Me | 5-Br-2-thienyl | H |
| 6-966 | 3-Chlorpropan-2-yl | Me | 4-Cl-Ph | H |
| 6-967 | 3-Chlorpropan-2-yl | Me | 4-Br-Ph | H |
| 6-968 | 3-Chlorpropan-2-yl | Me | 5-Cl-pyridin-2-yl | H |
| 6-969 | 3-Chlorpropan-2-yl | Me | 5-Br-pyridin-2-yl | H |
| 6-970 | 3-Chlorpropan-2-yl | Me | 5-Cl-2-thienyl | H |
| 6-971 | 3-Chlorpropan-2-yl | Me | 5-Br-2-thienyl | H |
| 6-972 | 2-(2-Chlorethoxy)-ethyl | Me | 4-Cl-Ph | H |
| 6-973 | 2-(2-Chlorethoxy)-ethyl | Me | 4-Br-Ph | H |
| 6-974 | 2-(2-Chlorethoxy)-ethyl | Me | 5-Cl-pyridin-2-yl | H |
| 6-975 | 2-(2-Chlorethoxy)-ethyl | Me | 5-Br-pyridin-2-yl | H |
| 6-976 | 2-(2-Chlorethoxy)-ethyl | Me | 5-Cl-2-thienyl | H |
| 6-977 | 2-(2-Chlorethoxy)-ethyl | Me | 5-Br-2-thienyl | H |
| 6-978 | 2,2-Dichlorethyl | Me | 4-Cl-Ph | H |
| 6-979 | 2,2-Dichlorethyl | Me | 4-Br-Ph | H |
| 6-980 | 2,2-Dichlorethyl | Me | 5-Cl-pyridin-2-yl | H |
| 6-981 | 2,2-Dichlorethyl | Me | 5-Br-pyridin-2-yl | H |
| 6-982 | 2,2-Dichlorethyl | Me | 5-Cl-2-thienyl | H |
| 6-983 | 2,2-Dichlorethyl | Me | 5-Br-2-thienyl | H |
| 6-984 | 2,3-Dichlorpropyl | Me | 4-Cl-Ph | H |
| 6-985 | 2,3-Dichlorpropyl | Me | 4-Br-Ph | H |
| 6-986 | 2,3-Dichlorpropyl | Me | 5-Cl-pyridin-2-yl | H |
| 6-987 | 2,3-Dichlorpropyl | Me | 5-Br-pyridin-2-yl | H |
| 6-988 | 2,3-Dichlorpropyl | Me | 5-Cl-2-thienyl | H |
| 6-989 | 2,3-Dichlorpropyl | Me | 5-Br-2-thienyl | H |
| 6-990 | 1,3-Dichlorprop-2-yl | Me | 4-Cl-Ph | H |
| 6-991 | 1,3-Dichlorprop-2-yl | Me | 4-Br-Ph | H |
| 6-992 | 1,3-Dichlorprop-2-yl | Me | 5-Cl-pyridin-2-yl | H |
| 6-993 | 1,3-Dichlorprop-2-yl | Me | 5-Br-pyridin-2-yl | H |
| 6-994 | 1,3-Dichlorprop-2-yl | Me | 5-Cl-2-thienyl | H |
| 6-995 | 1,3-Dichlorprop-2-yl | Me | 5-Br-2-thienyl | H |
| 6-996 | 2-Chlor-2,2-difluorethyl | Me | 4-Cl-Ph | H |
| 6-997 | 2-Chlor-2,2-difluorethyl | Me | 4-Br-Ph | H |
| 6-998 | 2-Chlor-2,2-difluorethyl | Me | 5-Cl-pyridin-2-yl | H |
| 6-999 | 2-Chlor-2,2-difluorethyl | Me | 5-Br-pyridin-2-yl | H |
| 6-1000 | 2-Chlor-2,2-difluorethyl | Me | 5-Cl-2-thienyl | H |
| 6-1001 | 2-Chlor-2,2-difluorethyl | Me | 5-Br-2-thienyl | H |
| 6-1002 | 1-Chlor-2-methylpropan-2-yl | Me | 4-Cl-Ph | H |
| 6-1003 | 1-Chlor-2-methylpropan-2-yl | Me | 4-Br-Ph | H |
| 6-1004 | 1-Chlor-2-methylpropan-2-yl | Me | 5-Cl-pyridin-2-yl | H |
| 6-1005 | 1-Chlor-2-methylpropan-2-yl | Me | 5-Br-pyridin-2-yl | H |
| 6-1006 | 1-Chlor-2-methylpropan-2-yl | Me | 5-Cl-2-thienyl | H |
| 6-1007 | 1-Chlor-2-methylpropan-2-yl | Me | 5-Br-2-thienyl | H |
| 6-1008 | 1-Fluor-3-methoxypropan-2-yl | Me | 4-Cl-Ph | H |
| 6-1009 | 1-Fluor-3-methoxypropan-2-yl | Me | 4-Br-Ph | H |
| 6-1010 | 1-Fluor-3-methoxypropan-2-yl | Me | 5-Cl-pyridin-2-yl | H |
| 6-1011 | 1-Fluor-3-methoxypropan-2-yl | Me | 5-Br-pyridin-2-yl | H |
| 6-1012 | 1-Fluor-3-methoxypropan-2-yl | Me | 5-Cl-2-thienyl | H |
| 6-1013 | 1-Fluor-3-methoxypropan-2-yl | Me | 5-Br-2-thienyl | H |
| 6-1014 | 3,3,3-Trifluorpropyl | Me | 4-Cl-Ph | H |
| 6-1015 | 3,3,3-Trifluorpropyl | Me | 4-Br-Ph | H |
| 6-1016 | 3,3,3-Trifluorpropyl | Me | 5-Cl-pyridin-2-yl | H |
| 6-1017 | 3,3,3-Trifluorpropyl | Me | 5-Br-pyridin-2-yl | H |
| 6-1018 | 3,3,3-Trifluorpropyl | Me | 5-Cl-2-thienyl | H |
| 6-1019 | 3,3,3-Trifluorpropyl | Me | 5-Br-2-thienyl | H |
| 6-1020 | 2-Chlor-phenyl | Me | 4-Cl-Ph | H |
| 6-1021 | 2-Chlor-phenyl | Me | 4-Br-Ph | H |
| 6-1022 | 2-Chlor-phenyl | Me | 5-Cl-pyridin-2-yl | H |
| 6-1023 | 2-Chlor-phenyl | Me | 5-Br-pyridin-2-yl | H |
| 6-1024 | 2-Chlor-phenyl | Me | 5-Cl-2-thienyl | H |
| 6-1025 | 2-Chlor-phenyl | Me | 5-Br-2-thienyl | H |
| 6-1026 | 2-Chlorpyridin-3-yl | Me | 4-Cl-Ph | H |
| 6-1027 | 2-Chlorpyridin-3-yl | Me | 4-Br-Ph | H |
| 6-1028 | 2-Chlorpyridin-3-yl | Me | 5-Cl-pyridin-2-yl | H |
| 6-1029 | 2-Chlorpyridin-3-yl | Me | 5-Br-pyridin-2-yl | H |
| 6-1030 | 2-Chlorpyridin-3-yl | Me | 5-Cl-2-thienyl | H |
| 6-1031 | 2-Chlorpyridin-3-yl | Me | 5-Br-2-thienyl | H |
| 6-1032 | 3-Chlorpyridin-2-yl | Me | 4-Cl-Ph | H |
| 6-1033 | 3-Chlorpyridin-2-yl | Me | 4-Br-Ph | H |
| 6-1034 | 3-Chlorpyridin-2-yl | Me | 5-Cl-pyridin-2-yl | H |
| 6-1035 | 3-Chlorpyridin-2-yl | Me | 5-Br-pyridin-2-yl | H |
| 6-1036 | 3-Chlorpyridin-2-yl | Me | 5-Cl-2-thienyl | H |
| 6-1037 | 3-Chlorpyridin-2-yl | Me | 5-Br-2-thienyl | H |
| 6-1038 | Pentafluorethyl | Me | 4-Cl-Ph | H |
| 6-1039 | Pentafluorethyl | Me | 4-Br-Ph | H |
| 6-1040 | Pentafluorethyl | Me | 5-Cl-pyridin-2-yl | H |
| 6-1041 | Pentafluorethyl | Me | 5-Br-pyridin-2-yl | H |
| 6-1042 | Pentafluorethyl | Me | 5-Cl-2-thienyl | H |
| 6-1043 | Pentafluorethyl | Me | 5-Br-2-thienyl | H |
| 6-1044 | 1,2,2,2-Tetrafluorethyl | Me | 4-Cl-Ph | H |
| 6-1045 | 1,2,2,2-Tetrafluorethyl | Me | 4-Br-Ph | H |
| 6-1046 | 1,2,2,2-Tetrafluorethyl | Me | 5-Cl-pyridin-2-yl | H |
| 6-1047 | 1,2,2,2-Tetrafluorethyl | Me | 5-Br-pyridin-2-yl | H |
| 6-1048 | 1,2,2,2-Tetrafluorethyl | Me | 5-Cl-2-thienyl | H |
| 6-1049 | 1,2,2,2-Tetrafluorethyl | Me | 5-Br-2-thienyl | H |
| 6-1050 | 1,1,2,2-Tetrafluorethyl | Me | 4-Cl-Ph | H |
| 6-1051 | 1,1,2,2-Tetrafluorethyl | Me | 4-Br-Ph | H |
| 6-1052 | 1,1,2,2-Tetrafluorethyl | Me | 5-Cl-pyridin-2-yl | H |
| 6-1053 | 1,1,2,2-Tetrafluorethyl | Me | 5-Br-pyridin-2-yl | H |
| 6-1054 | 1,1,2,2-Tetrafluorethyl | Me | 5-Cl-2-thienyl | H |
| 6-1055 | 1,1,2,2-Tetrafluorethyl | Me | 5-Br-2-thienyl | H |
| 6-1056 | 1,1,2-Trifluorethyl | Me | 4-Cl-Ph | H |
| 6-1057 | 1,1,2-Trifluorethyl | Me | 4-Br-Ph | H |
| 6-1058 | 1,1,2-Trifluorethyl | Me | 5-Cl-pyridin-2-yl | H |
| 6-1059 | 1,1,2-Trifluorethyl | Me | 5-Br-pyridin-2-yl | H |
| 6-1060 | 1,1,2-Trifluorethyl | Me | 5-Cl-2-thienyl | H |
| 6-1061 | 1,1,2-Trifluorethyl | Me | 5-Br-2-thienyl | H |
| 6-1062 | 2-Methylbut-3-in-2-yl | Me | 4-Cl-Ph | H |
| 6-1063 | 2-Methylbut-3-in-2-yl | Me | 4-Br-Ph | H |
| 6-1064 | 2-Methylbut-3-in-2-yl | Me | 5-Cl-pyridin-2-yl | H |
| 6-1065 | 2-Methylbut-3-in-2-yl | Me | 5-Br-pyridin-2-yl | H |
| 6-1066 | 2-Methylbut-3-in-2-yl | Me | 5-Cl-2-thienyl | H |
| 6-1067 | 2-Methylbut-3-in-2-yl | Me | 5-Br-2-thienyl | H |
| 6-1068 | 1-(Ethoxycarbonyl)-eth-1-yl | Me | 4-Cl-Ph | H |
| 6-1069 | 1-(Ethoxycarbonyl)-eth-1-yl | Me | 4-Br-Ph | H |
| 6-1070 | 1-(Ethoxycarbonyl)-eth-1-yl | Me | 5-Cl-pyridin-2-yl | H |
| 6-1071 | 1-(Ethoxycarbonyl)-eth-1-yl | Me | 5-Br-pyridin-2-yl | H |
| 6-1072 | 1-(Ethoxycarbonyl)-eth-1-yl | Me | 5-Cl-2-thienyl | H |
| 6-1073 | 1-(Ethoxycarbonyl)-eth-1-yl | Me | 5-Br-2-thienyl | H |
| 6-1074 | 1,1,2,3,3,3-Hexafluorpropyl | Me | 4-Cl-Ph | H |
| 6-1075 | 1,1,2,3,3,3-Hexafluorpropyl | Me | 4-Br-Ph | H |
| 6-1076 | 1,1,2,3,3,3-Hexafluorpropyl | Me | 5-Cl-pyridin-2-yl | H |
| 6-1077 | 1,1,2,3,3,3-Hexafluorpropyl | Me | 5-Br-pyridin-2-yl | H |
| 6-1078 | 1,1,2,3,3,3-Hexafluorpropyl | Me | 5-Cl-2-thienyl | H |
| 6-1079 | 1,1,2,3,3,3-Hexafluorpropyl | Me | 5-Br-2-thienyl | H |
| 6-1080 | Isobutyl | Me | 4-Cl-Ph | H |
| 6-1081 | Isobutyl | Me | 4-Br-Ph | H |
| 6-1082 | Isobutyl | Me | 5-Cl-pyridin-2-yl | H |
| 6-1083 | Isobutyl | Me | 5-Br-pyridin-2-yl | H |
| 6-1084 | Isobutyl | Me | 5-Cl-2-thienyl | H |
| 6-1085 | Isobutyl | Me | 5-Br-2-thienyl | H |
| 6-1086 | n-Pentyl | Me | 4-Cl-Ph | H |
| 6-1087 | n-Pentyl | Me | 4-Br-Ph | H |
| 6-1088 | n-Pentyl | Me | 5-Cl-pyridin-2-yl | H |
| 6-1089 | n-Pentyl | Me | 5-Br-pyridin-2-yl | H |
| 6-1090 | n-Pentyl | Me | 5-Cl-2-thienyl | H |
| 6-1091 | n-Pentyl | Me | 5-Br-2-thienyl | H |
| 6-1092 | n-Heptyl | Me | 4-Cl-Ph | H |
| 6-1093 | n-Heptyl | Me | 4-Br-Ph | H |
| 6-1094 | n-Heptyl | Me | 5-Cl-pyridin-2-yl | H |
| 6-1095 | n-Heptyl | Me | 5-Br-pyridin-2-yl | H |
| 6-1096 | n-Heptyl | Me | 5-Cl-2-thienyl | H |
| 6-1097 | n-Heptyl | Me | 5-Br-2-thienyl | H |
| 6-1098 | n-Nonyl | Me | 4-Cl-Ph | H |
| 6-1099 | n-Nonyl | Me | 4-Br-Ph | H |
| 6-1100 | n-Nonyl | Me | 5-Cl-pyridin-2-yl | H |
| 6-1101 | n-Nonyl | Me | 5-Br-pyridin-2-yl | H |
| 6-1102 | n-Nonyl | Me | 5-Cl-2-thienyl | H |
| 6-1103 | n-Nonyl | Me | 5-Br-2-thienyl | H |
| 6-1104 | Cyclopentyl | Me | 4-Cl-Ph | H |
| 6-1105 | Cyclopentyl | Me | 4-Br-Ph | H |
| 6-1106 | Cyclopentyl | Me | 5-Cl-pyridin-2-yl | H |
| 6-1107 | Cyclopentyl | Me | 5-Br-pyridin-2-yl | H |
| 6-1108 | Cyclopentyl | Me | 5-Cl-2-thienyl | H |
| 6-1109 | Cyclopentyl | Me | 5-Br-2-thienyl | H |
| 6-1110 | Cyclohexyl | Me | 4-Cl-Ph | H |
| 6-1111 | Cyclohexyl | Me | 4-Br-Ph | H |
| 6-1112 | Cyclohexyl | Me | 5-Cl-pyridin-2-yl | H |
| 6-1113 | Cyclohexyl | Me | 5-Br-pyridin-2-yl | H |
| 6-1114 | Cyclohexyl | Me | 5-Cl-2-thienyl | H |
| 6-1115 | Cyclohexyl | Me | 5-Br-2-thienyl | H |
| 6-1116 | sBu | Me | 4-Cl-Ph | H |
| 6-1117 | sBu | Me | 4-Br-Ph | H |
| 6-1118 | sBu | Me | 5-Cl-pyridin-2-yl | H |
| 6-1119 | sBu | Me | 5-Br-pyridin-2-yl | H |
| 6-1120 | sBu | Me | 5-Cl-2-thienyl | H |
| 6-1121 | sBu | Me | 5-Br-2-thienyl | H |
| 6-1122 | Pentan-3-yl | Me | 4-Cl-Ph | H |
| 6-1123 | Pentan-3-yl | Me | 4-Br-Ph | H |
| 6-1124 | Pentan-3-yl | Me | 5-Cl-pyridin-2-yl | H |
| 6-1125 | Pentan-3-yl | Me | 5-Br-pyridin-2-yl | H |
| 6-1126 | Pentan-3-yl | Me | 5-Cl-2-thienyl | H |
| 6-1127 | Pentan-3-yl | Me | 5-Br-2-thienyl | H |
| 6-1128 | 1-(Methoxycarbonyl)-eth-1-yl | Me | 4-Cl-Ph | H |
| 6-1129 | 1-(Methoxycarbonyl)-eth-1-yl | Me | 4-Br-Ph | H |
| 6-1130 | 1-(Methoxycarbonyl)-eth-1-yl | Me | 5-Cl-pyridin-2-yl | H |
| 6-1131 | 1-(Methoxycarbonyl)-eth-1-yl | Me | 5-Br-pyridin-2-yl | H |
| 6-1132 | 1-(Methoxycarbonyl)-eth-1-yl | Me | 5-Cl-2-thienyl | H |
| 6-1133 | 1-(Methoxycarbonyl)-eth-1-yl | Me | 5-Br-2-thienyl | H |
| 6-1134 | 2,2,2-Trichlorethyl | Me | 4-Cl-Ph | H |
| 6-1135 | 2,2,2-Trichlorethyl | Me | 4-Br-Ph | H |
| 6-1136 | 2,2,2-Trichlorethyl | Me | 5-Cl-pyridin-2-yl | H |
| 6-1137 | 2,2,2-Trichlorethyl | Me | 5-Br-pyridin-2-yl | H |
| 6-1138 | 2,2,2-Trichlorethyl | Me | 5-Cl-2-thienyl | H |
| 6-1139 | 2,2,2-Trichlorethyl | Me | 5-Br-2-thienyl | H |
| 6-1140 | 3-Chlorpropyl | Me | 4-Cl-Ph | H |
| 6-1141 | 3-Chlorpropyl | Me | 4-Br-Ph | H |
| 6-1142 | 3-Chlorpropyl | Me | 5-Cl-pyridin-2-yl | H |
| 6-1143 | 3-Chlorpropyl | Me | 5-Br-pyridin-2-yl | H |
| 6-1144 | 3-Chlorpropyl | Me | 5-Cl-2-thienyl | H |
| 6-1145 | 3-Chlorpropyl | Me | 5-Br-2-thienyl | H |
| 6-1146 | 2-(2-Methoxyethoxy)-ethyl | Me | 4-Cl-Ph | H |
| 6-1147 | 2-(2-Methoxyethoxy)-ethyl | Me | 4-Br-Ph | H |
| 6-1148 | 2-(2-Methoxyethoxy)-ethyl | Me | 5-Cl-pyridin-2-yl | H |
| 6-1149 | 2-(2-Methoxyethoxy)-ethyl | Me | 5-Br-pyridin-2-yl | H |
| 6-1150 | 2-(2-Methoxyethoxy)-ethyl | Me | 5-Cl-2-thienyl | H |
| 6-1151 | 2-(2-Methoxyethoxy)-ethyl | Me | 5-Br-2-thienyl | H |
| 6-1152 | Butyl-2-yl-methyl | Me | 4-Cl-Ph | H |
| 6-1153 | Butyl-2-yl-methyl | Me | 4-Br-Ph | H |
| 6-1154 | Butyl-2-yl-methyl | Me | 5-Cl-pyridin-2-yl | H |
| 6-1155 | Butyl-2-yl-methyl | Me | 5-Br-pyridin-2-yl | H |
| 6-1156 | Butyl-2-yl-methyl | Me | 5-Cl-2-thienyl | H |
| 6-1157 | Butyl-2-yl-methyl | Me | 5-Br-2-thienyl | H |
| 6-1158 | But-3-in-1-yl | Me | 4-Cl-Ph | H |
| 6-1159 | But-3-in-1-yl | Me | 4-Br-Ph | H |
| 6-1160 | But-3-in-1-yl | Me | 5-Cl-pyridin-2-yl | H |
| 6-1161 | But-3-in-1-yl | Me | 5-Br-pyridin-2-yl | H |
| 6-1162 | But-3-in-1-yl | Me | 5-Cl-2-thienyl | H |
| 6-1163 | But-3-in-1-yl | Me | 5-Br-2-thienyl | H |
| 6-1164 | (2,2-Dichlorcyclopropyl)-methyl | Me | 4-Cl-Ph | H |
| 6-1165 | (2,2-Dichlorcyclopropyl)-methyl | Me | 4-Br-Ph | H |
| 6-1166 | (2,2-Dichlorcyclopropyl)-methyl | Me | 5-Cl-pyridin-2-yl | H |
| 6-1167 | (2,2-Dichlorcyclopropyl)-methyl | Me | 5-Br-pyridin-2-yl | H |
| 6-1168 | (2,2-Dichlorcyclopropyl)-methyl | Me | 5-Cl-2-thienyl | H |
| 6-1169 | (2,2-Dichlorcyclopropyl)-methyl | Me | 5-Br-2-thienyl | H |
| 6-1170 | 2-(N,N-Diethylamino)-eth-1-yl | Me | 4-Cl-Ph | H |
| 6-1171 | 2-(N,N-Diethylamino)-eth-1-yl | Me | 4-Br-Ph | H |
| 6-1172 | 2-(N,N-Diethylamino)-eth-1-yl | Me | 5-Cl-pyridin-2-yl | H |
| 6-1173 | 2-(N,N-Diethylamino)-eth-1-yl | Me | 5-Br-pyridin-2-yl | H |
| 6-1174 | 2-(N,N-Diethylamino)-eth-1-yl | Me | 5-Cl-2-thienyl | H |
| 6-1175 | 2-(N,N-Diethylamino)-eth-1-yl | Me | 5-Br-2-thienyl | H |
| 6-1176 | 2-Carboxy-phenyl | Me | 4-Cl-Ph | H |
| 6-1177 | 2-Carboxy-phenyl | Me | 4-Br-Ph | H |
| 6-1178 | 2-Carboxy-phenyl | Me | 5-Cl-pyridin-2-yl | H |
| 6-1179 | 2-Carboxy-phenyl | Me | 5-Br-pyridin-2-yl | H |
| 6-1180 | 2-Carboxy-phenyl | Me | 5-Cl-2-thienyl | H |
| 6-1181 | 2-Carboxy-phenyl | Me | 5-Br-2-thienyl | H |
| 6-1182 | tButyl | Me | 4-Cl-Ph | H |
| 6-1183 | tBu | Me | 4-Br-Ph | H |
| 6-1184 | tBu | Me | 5-Cl-pyridin-2-yl | H |
| 6-1185 | tBu | Me | 5-Br-pyridin-2-yl | H |
| 6-1186 | tBu | Me | 5-Cl-2-thienyl | H |
| 6-1187 | tBu | Me | 5-Br-2-thienyl | H |
| 6-1188 | 1-Methyl-cyclopropyl | Me | 4-Cl-Ph | H |
| 6-1189 | 1-Methyl-cyclopropyl | Me | 4-Br-Ph | H |
| 6-1190 | 1-Methyl-cyclopropyl | Me | 5-Cl-pyridin-2-yl | H |
| 6-1191 | 1-Methyl-cyclopropyl | Me | 5-Br-pyridin-2-yl | H |
| 6-1192 | 1-Methyl-cyclopropyl | Me | 5-Cl-2-thienyl | H |
| 6-1193 | 1-Methyl-cyclopropyl | Me | 5-Br-2-thienyl | H |
| 6-1194 | Trimethylsilylmethyl | Me | 4-Cl-Ph | H |
| 6-1195 | Trimethylsilylmethyl | Me | 4-Br-Ph | H |
| 6-1196 | Trimethylsilylmethyl | Me | 5-Cl-pyridin-2-yl | H |
| 6-1197 | Trimethylsilylmethyl | Me | 5-Br-pyridin-2-yl | H |
| 6-1198 | Trimethylsilylmethyl | Me | 5-Cl-2-thienyl | H |
| 6-1199 | Trimethylsilylmethyl | Me | 5-Br-2-thienyl | H |
| 6-1200 | 2,3-Dihydro-1H-inden-5-yl | Me | 4-Cl-Ph | H |
| 6-1201 | 2,3-Dihydro-1H-inden-5-yl | Me | 4-Br-Ph | H |
| 6-1202 | 2,3-Dihydro-1H-inden-5-yl | Me | 5-Cl-pyridin-2-yl | H |
| 6-1203 | 2,3-Dihydro-1H-inden-5-yl | Me | 5-Br-pyridin-2-yl | H |
| 6-1204 | 2,3-Dihydro-1H-inden-5-yl | Me | 5-Cl-2-thienyl | H |
| 6-1205 | 2,3-Dihydro-1H-inden-5-yl | Me | 5-Br-2-thienyl | H |
| 6-1206 | 1-Methylcyclobutyl | Me | 4-Cl-Ph | H |
| 6-1207 | 1-Methylcyclobutyl | Me | 4-Br-Ph | H |
| 6-1208 | 1-Methylcyclobutyl | Me | 5-Cl-pyridin-2-yl | H |
| 6-1209 | 1-Methylcyclobutyl | Me | 5-Br-pyridin-2-yl | H |
| 6-1210 | 1-Methylcyclobutyl | Me | 5-Cl-2-thienyl | H |
| 6-1211 | 1-Methylcyclobutyl | Me | 5-Br-2-thienyl | H |
| 6-1212 | 2-(Oxetan-3-yl)-eth-1-yl | Me | 4-Cl-Ph | H |
| 6-1213 | 2-(Oxetan-3-yl)-eth-1-yl | Me | 4-Br-Ph | H |
| 6-1214 | 2-(Oxetan-3-yl)-eth-1-yl | Me | 5-Cl-pyridin-2-yl | H |
| 6-1215 | 2-(Oxetan-3-yl)-eth-1-yl | Me | 5-Br-pyridin-2-yl | H |
| 6-1216 | 2-(Oxetan-3-yl)-eth-1-yl | Me | 5-Cl-2-thienyl | H |
| 6-1217 | 2-(Oxetan-3-yl)-eth-1-yl | Me | 5-Br-2-thienyl | H |
| 6-1218 | Bu | Me | 4-Cl-Ph | H |
| 6-1219 | Bu | Me | 4-Br-Ph | H |
| 6-1220 | Bu | Me | 5-Cl-pyridin-2-yl | H |
| 6-1221 | Bu | Me | 5-Br-pyridin-2-yl | H |
| 6-1222 | Bu | Me | 5-Cl-2-thienyl | H |
| 6-1223 | Bu | Me | 5-Br-2-thienyl | H |
| 6-1224 | 2-(N,N-Diethylamino)-eth-1-yl | Me | 4-Cl-Ph | H |
| 6-1225 | 2-(N,N-Diethylamino)-eth-1-yl | Me | 4-Br-Ph | H |
| 6-1226 | 2-(N,N-Diethylamino)-eth-1-yl | Me | 5-Cl-pyridin-2-yl | H |
| 6-1227 | 2-(N,N-Diethylamino)-eth-1-yl | Me | 5-Br-pyridin-2-yl | H |
| 6-1228 | 2-(N,N-Diethylamino)-eth-1-yl | Me | 5-Cl-2-thienyl | H |
| 6-1229 | 2-(N,N-Diethylamino)-eth-1-yl | Me | 5-Br-2-thienyl | H |
| 6-1230 | 2-(Morpholin-4-yl)-eth-1-yl | Me | 4-Cl-Ph | H |
| 6-1231 | 2-(Morpholin-4-yl)-eth-1-yl | Me | 4-Br-Ph | H |
| 6-1232 | 2-(Morpholin-4-yl)-eth-1-yl | Me | 5-Cl-pyridin-2-yl | H |
| 6-1233 | 2-(Morpholin-4-yl)-eth-1-yl | Me | 5-Br-pyridin-2-yl | H |
| 6-1234 | 2-(Morpholin-4-yl)-eth-1-yl | Me | 5-Cl-2-thienyl | H |
| 6-1235 | 2-(Morpholin-4-yl)-eth-1-yl | Me | 5-Br-2-thienyl | H |
| 6-1236 | 2-Chlor-thiophen-3-yl | Me | 4-Cl-Ph | H |
| 6-1237 | 2-Chlor-thiophen-3-yl | Me | 4-Br-Ph | H |
| 6-1238 | 2-Chlor-thiophen-3-yl | Me | 5-Cl-pyridin-2-yl | H |
| 6-1239 | 2-Chlor-thiophen-3-yl | Me | 5-Br-pyridin-2-yl | H |
| 6-1240 | 2-Chlor-thiophen-3-yl | Me | 5-Cl-2-thienyl | H |
| 6-1241 | 2-Chlor-thiophen-3-yl | Me | 5-Br-2-thienyl | H |
| 6-1242 | (N,N-Dimethylaminocarbonyl)-methyl | Me | 4-Cl-Ph | H |
| 6-1243 | (N,N-Dimethylaminocarbonyl)-methyl | Me | 4-Br-Ph | H |
| 6-1244 | (N,N-Dimethylaminocarbonyl)-methyl | Me | 5-Cl-pyridin-2-yl | H |
| 6-1245 | (N,N-Dimethylaminocarbonyl)-methyl | Me | 5-Br-pyridin-2-yl | H |
| 6-1246 | (N,N-Dimethylaminocarbonyl)-methyl | Me | 5-Cl-2-thienyl | H |
| 6-1247 | (N,N-Dimethylaminocarbonyl)-methyl | Me | 5-Br-2-thienyl | H |
| 6-1248 | 1-(t-Butylcarbonyloxy)-2-methylprop-1-yl | Me | 4-Cl-Ph | H |
| 6-1249 | 1-(t-Butylcarbonyloxy)-2-methylprop-1-yl | Me | 4-Br-Ph | H |
| 6-1250 | 1-(t-Butylcarbonyloxy)-2-methylprop-1-yl | Me | 5-Cl-pyridin-2-yl | H |
| 6-1251 | 1-(t-Butylcarbonyloxy)-2-methylprop-1-yl | Me | 5-Br-pyridin-2-yl | H |
| 6-1252 | 1-(t-Butylcarbonyloxy)-2-methylprop-1-yl | Me | 5-Cl-2-thienyl | H |
| 6-1253 | 1-(t-Butylcarbonyloxy)-2-methylprop-1-yl | Me | 5-Br-2-thienyl | H |
| 6-1254 | (5-Methyl-2-oxo-1,3-dioxol-4-yl)-methyl | Me | 4-Cl-Ph | H |
| 6-1255 | (5-Methyl-2-oxo-1,3-dioxol-4-yl)-methyl | Me | 4-Br-Ph | H |
| 6-1256 | (5-Methyl-2-oxo-1,3-dioxol-4-yl)-methyl | Me | 5-Cl-pyridin-2-yl | H |
| 6-1257 | (5-Methyl-2-oxo-1,3-dioxol-4-yl)-methyl | Me | 5-Br-pyridin-2-yl | H |
| 6-1258 | (5-Methyl-2-oxo-1,3-dioxol-4-yl)-methyl | Me | 5-Cl-2-thienyl | H |
| 6-1259 | (5-Methyl-2-oxo-1,3-dioxol-4-yl)-methyl | Me | 5-Br-2-thienyl | H |
| 6-1260 | [(t-Butoxycarbonyl)oxy]-methyl | Me | 4-Cl-Ph | H |
| 6-1261 | [(t-Butoxycarbonyl)oxy]-methyl | Me | 4-Br-Ph | H |
| 6-1262 | [(t-Butoxycarbonyl)oxy]-methyl | Me | 5-Cl-pyridin-2-yl | H |
| 6-1263 | [(t-Butoxycarbonyl)oxy]-methyl | Me | 5-Br-pyridin-2-yl | H |
| 6-1264 | [(t-Butoxycarbonyl)oxy]-methyl | Me | 5-Cl-2-thienyl | H |
| 6-1265 | [(t-Butoxycarbonyl)oxy]-methyl | Me | 5-Br-2-thienyl | H |
| 6-1266 | [(Isopropoxycarbonyl)oxy]-methyl | Me | 4-Cl-Ph | H |
| 6-1267 | [(Isopropoxycarbonyl)oxy]-methyl | Me | 4-Br-Ph | H |
| 6-1268 | [(Isopropoxycarbonyl)oxy]-methyl | Me | 5-Cl-pyridin-2-yl | H |
| 6-1269 | [(Isopropoxycarbonyl)oxy]-methyl | Me | 5-Br-pyridin-2-yl | H |
| 6-1270 | [(Isopropoxycarbonyl)oxy]-methyl | Me | 5-Cl-2-thienyl | H |
| 6-1271 | [(Isopropoxycarbonyl)oxy]-methyl | Me | 5-Br-2-thienyl | H |
| 6-1272 | [(Methoxycarbonyl)oxy]-methyl | Me | 4-Cl-Ph | H |
| 6-1273 | [(Methoxycarbonyl)oxy]-methyl | Me | 4-Br-Ph | H |
| 6-1274 | [(Methoxycarbonyl)oxy]-methyl | Me | 5-Cl-pyridin-2-yl | H |
| 6-1275 | [(Methoxycarbonyl)oxy]-methyl | Me | 5-Br-pyridin-2-yl | H |
| 6-1276 | [(Methoxycarbonyl)oxy]-methyl | Me | 5-Cl-2-thienyl | H |
| 6-1277 | [(Methoxycarbonyl)oxy]-methyl | Me | 5-Br-2-thienyl | H |
| 6-1278 | 1-[(Ethoxycarbonyl)oxy]-ethyl | Me | 4-Cl-Ph | H |
| 6-1279 | 1-[(Ethoxycarbonyl)oxy]-ethyl | Me | 4-Br-Ph | H |
| 6-1280 | 1-[(Ethoxycarbonyl)oxy]-ethyl | Me | 5-Cl-pyridin-2-yl | H |
| 6-1281 | 1-[(Ethoxycarbonyl)oxy]-ethyl | Me | 5-Br-pyridin-2-yl | H |
| 6-1282 | 1-[(Ethoxycarbonyl)oxy]-ethyl | Me | 5-Cl-2-thienyl | H |
| 6-1283 | 1-[(Ethoxycarbonyl)oxy]-ethyl | Me | 5-Br-2-thienyl | H |
| 6-1284 | 1-Acetoxy-eth-1-yl | Me | 4-CI-Ph | H |
| 6-1285 | 1-Acetoxy-eth-1-yl | Me | 4-Br-Ph | H |
| 6-1286 | 1-Acetoxy-eth-1-yl | Me | 5-Cl-pyridin-2-yl | H |
| 6-1287 | 1-Acetoxy-eth-1-yl | Me | 5-Br-pyridin-2-yl | H |
| 6-1288 | 1-Acetoxy-eth-1-yl | Me | 5-Cl-2-thienyl | H |
| 6-1289 | 1-Acetoxy-eth-1-yl | Me | 5-Br-2-thienyl | H |
| 6-1290 | 1-(2-Methylpropanoyloxy)-eth-1-yl | Me | 4-Cl-Ph | H |
| 6-1291 | 1-(2-Methylpropanoyloxy)-eth-1-yl | Me | 4-Br-Ph | H |
| 6-1292 | 1-(2-Methylpropanoyloxy)-eth-1-yl | Me | 5-Cl-pyridin-2-yl | H |
| 6-1293 | 1-(2-Methylpropanoyloxy)-eth-1-yl | Me | 5-Br-pyridin-2-yl | H |
| 6-1294 | 1-(2-Methylpropanoyloxy)-eth-1-yl | Me | 5-Cl-2-thienyl | H |
| 6-1295 | 1-(2-Methylpropanoyloxy)-eth-1-yl | Me | 5-Br-2-thienyl | H |
| 6-1296 | 1-Propanoyl-2-methyl-prop-1-yl | Me | 4-Cl-Ph | H |
| 6-1297 | 1-Propanoyl-2-methyl-prop-1-yl | Me | 4-Br-Ph | H |
| 6-1298 | 1-Propanoyl-2-methyl-prop-1-yl | Me | 5-Cl-pyridin-2-yl | H |
| 6-1299 | 1-Propanoyl-2-methyl-prop-1-yl | Me | 5-Br-pyridin-2-yl | H |
| 6-1300 | 1-Propanoyl-2-methyl-prop-1-yl | Me | 5-Cl-2-thienyl | H |
| 6-1301 | 1-Propanoyl-2-methyl-prop-1-yl | Me | 5-Br-2-thienyl | H |
| 6-1302 | 1-(Cyclohexoxycarbonyloxy)-eth-1-yl | Me | 4-Cl-Ph | H |
| 6-1303 | 1-(Cyclohexoxycarbonyloxy)-eth-1-yl | Me | 4-Br-Ph | H |
| 6-1304 | 1-(Cyclohexoxycarbonyloxy)-eth-1-yl | Me | 5-Cl-pyridin-2-yl | H |
| 6-1305 | 1-(Cyclohexoxycarbonyloxy)-eth-1-yl | Me | 5-Br-pyridin-2-yl | H |
| 6-1306 | 1-(Cyclohexoxycarbonyloxy)-eth-1-yl | Me | 5-Cl-2-thienyl | H |
| 6-1307 | 1-(Cyclohexoxycarbonyloxy)-eth-1-yl | Me | 5-Br-2-thienyl | H |
| 6-1308 | Cyclobutyl | Me | 4-Cl-Ph | H |
| 6-1309 | Cyclobutyl | Me | 4-Br-Ph | H |
| 6-1310 | Cyclobutyl | Me | 5-Cl-pyridin-2-yl | H |
| 6-1311 | Cyclobutyl | Me | 5-Br-pyridin-2-yl | H |
| 6-1312 | Cyclobutyl | Me | 5-Cl-2-thienyl | H |
| 6-1313 | Cyclobutyl | Me | 5-Br-2-thienyl | H |
| 6-1314 | CH₂(4-Me-Ph) | Me | 4-Cl-Ph | H |
| 6-1315 | CH₂(4-Me-Ph) | Me | 4-Br-Ph | H |
| 6-1316 | CH₂(4-Me-Ph) | Me | 5-Cl-pyridin-2-yl | H |
| 6-1317 | CH₂(4-Me-Ph) | Me | 5-Br-pyridin-2-yl | H |
| 6-1318 | CH₂(4-Me-Ph) | Me | 5-Cl-2-thienyl | H |
| 6-1319 | CH₂(4-Me-Ph) | Me | 5-Br-2-thienyl | H |
| 6-1320 | CHMe(4-Cl-Ph) | Me | 4-Cl-Ph | H |
| 6-1321 | CHMe(4-Cl-Ph) | Me | 4-Br-Ph | H |
| 6-1322 | CHMe(4-Cl-Ph) | Me | 5-Cl-pyridin-2-yl | H |
| 6-1323 | CHMe(4-Cl-Ph) | Me | 5-Br-pyridin-2-yl | H |
| 6-1324 | CHMe(4-Cl-Ph) | Me | 5-Cl-2-thienyl | H |
| 6-1325 | CHMe(4-Cl-Ph) | Me | 5-Br-2-thienyl | H |
| 6-1326 | CHMePh | Me | 4-Cl-Ph | H |
| 6-1327 | CHMePh | Me | 4-Br-Ph | H |
| 6-1328 | CHMePh | Me | 5-Cl-pyridin-2-yl | H |
| 6-1329 | CHMePh | Me | 5-Br-pyridin-2-yl | H |
| 6-1330 | CHMePh | Me | 5-Cl-2-thienyl | H |
| 6-1331 | CHMePh | Me | 5-Br-2-thienyl | H |
| 6-1332 | 1,1,1-Trifluorpropan-2-yl | Me | 4-Cl-Ph | H |
| 6-1333 | 1,1,1-Trifluorpropan-2-yl | Me | 4-Br-Ph | H |
| 6-1334 | 1,1,1-Trifluorpropan-2-yl | Me | 5-Cl-pyridin-2-yl | H |
| 6-1335 | 1,1,1-Trifluorpropan-2-yl | Me | 5-Br-pyridin-2-yl | H |
| 6-1336 | 1,1,1-Trifluorpropan-2-yl | Me | 5-Cl-2-thienyl | H |
| 6-1337 | 1,1,1-Trifluorpropan-2-yl | Me | 5-Br-2-thienyl | H |
| 6-1338 | (1-Ethyl-3-methyl-1 H-pyrazol-4-yl)methyl | Me | 4-Cl-Ph | H |
| 6-1339 | (1-Ethyl-3-methyl-1 H-pyrazol-4-yl)-methyl | Me | 4-Br-Ph | H |
| 6-1340 | (1-Ethyl-3-methyl-1 H-pyrazol-4-yl)-methyl | Me | 5-Cl-pyridin-2-yl | H |
| 6-1341 | (1-Ethyl-3-methyl-1 H-pyrazol-4-yl)-methyl | Me | 5-Br-pyridin-2-yl | H |
| 6-1342 | (1-Ethyl-3-methyl-1 H-pyrazol-4-yl)-methyl | Me | 5-Cl-2-thienyl | H |
| 6-1343 | (1-Ethyl-3-methyl-1 H-pyrazol-4-yl)-methyl | Me | 5-Br-2-thienyl | H |
| 6-1344 | Pr | Me | 4-Cl-Ph | H |
| 6-1345 | Pr | Me | 4-Br-Ph | H |
| 6-1346 | Pr | Me | 5-Cl-pyridin-2-yl | H |
| 6-1347 | Pr | Me | 5-Br-pyridin-2-yl | H |
| 6-1348 | Pr | Me | 5-Cl-2-thienyl | H |
| 6-1349 | Pr | Me | 5-Br-2-thienyl | H |
| 6-1350 | n-Octadecyl | Me | 4-Cl-Ph | H |
| 6-1351 | n-Octadecyl | Me | 4-Br-Ph | H |
| 6-1352 | n-Octadecyl | Me | 5-Cl-pyridin-2-yl | H |
| 6-1353 | n-Octadecyl | Me | 5-Br-pyridin-2-yl | H |
| 6-1354 | n-Octadecyl | Me | 5-Cl-2-thienyl | H |
| 6-1355 | n-Octadecyl | Me | 5-Br-2-thienyl | H |
| 6-1356 | n-Hexadecyl | Me | 4-Cl-Ph | H |
| 6-1357 | n-Hexadecyl | Me | 4-Br-Ph | H |
| 6-1358 | n-Hexadecyl | Me | 5-Cl-pyridin-2-yl | H |
| 6-1359 | n-Hexadecyl | Me | 5-Br-pyridin-2-yl | H |
| 6-1360 | n-Hexadecyl | Me | 5-Cl-2-thienyl | H |
| 6-1361 | n-Hexadecyl | Me | 5-Br-2-thienyl | H |
| 6-1362 | Oxetan-3-yl-methyl | Me | 4-Cl-Ph | H |
| 6-1363 | Oxetan-3-yl-methyl | Me | 4-Br-Ph | H |
| 6-1364 | Oxetan-3-yl-methyl | Me | 5-Cl-pyridin-2-yl | H |
| 6-1365 | Oxetan-3-yl-methyl | Me | 5-Br-pyridin-2-yl | H |
| 6-1366 | Oxetan-3-yl-methyl | Me | 5-Cl-2-thienyl | H |
| 6-1367 | Oxetan-3-yl-methyl | Me | 5-Br-2-thienyl | H |
| 6-1368 | 3-Methyloxetan-3-yl | Me | 4-Cl-Ph | H |
| 6-1369 | 3-Methyloxetan-3-yl | Me | 4-Br-Ph | H |
| 6-1370 | 3-Methyloxetan-3-yl | Me | 5-Cl-pyridin-2-yl | H |
| 6-1371 | 3-Methyloxetan-3-yl | Me | 5-Br-pyridin-2-yl | H |
| 6-1372 | 3-Methyloxetan-3-yl | Me | 5-Cl-2-thienyl | H |
| 6-1373 | 3-Methyloxetan-3-yl | Me | 5-Br-2-thienyl | H |
| 6-1374 | 2-Chlorprop-2-en-1-yl | Me | 4-Cl-Ph | H |
| 6-1375 | 2-Chlorprop-2-en-1-yl | Me | 4-Br-Ph | H |
| 6-1376 | 2-Chlorprop-2-en-1-yl | Me | 5-Cl-pyridin-2-yl | H |
| 6-1377 | 2-Chlorprop-2-en-1-yl | Me | 5-Br-pyridin-2-yl | H |
| 6-1378 | 2-Chlorprop-2-en-1-yl | Me | 5-Cl-2-thienyl | H |
| 6-1379 | 2-Chlorprop-2-en-1-yl | Me | 5-Br-2-thienyl | H |
| 6-1380 | (3E)-Pent-3-en-2-yl | Me | 4-Cl-Ph | H |
| 6-1381 | (3E)-Pent-3-en-2-yl | Me | 4-Br-Ph | H |
| 6-1382 | (3E)-Pent-3-en-2-yl | Me | 5-Cl-pyridin-2-yl | H |
| 6-1383 | (3E)-Pent-3-en-2-yl | Me | 5-Br-pyridin-2-yl | H |
| 6-1384 | (3E)-Pent-3-en-2-yl | Me | 5-Cl-2-thienyl | H |
| 6-1385 | (3E)-Pent-3-en-2-yl | Me | 5-Br-2-thienyl | H |
| 6-1386 | (2,2-Dimethylpropanoyloxy)-methyl | Me | 4-Cl-Ph | H |
| 6-1387 | (2,2-Dimethylpropanoyloxy)-methyl | Me | 4-Br-Ph | H |
| 6-1388 | (2,2-Dimethylpropanoyloxy)-methyl | Me | 5-Cl-pyridin-2-yl | H |
| 6-1389 | (2,2-Dimethylpropanoyloxy)-methyl | Me | 5-Br-pyridin-2-yl | H |
| 6-1390 | (2,2-Dimethylpropanoyloxy)-methyl | Me | 5-Cl-2-thienyl | H |
| 6-1391 | (2,2-Dimethylpropanoyloxy)-methyl | Me | 5-Br-2-thienyl | H |
| 6-1392 | 2-(Isopropoxycarbonyloxy)-eth-1-yl | Me | 4-Cl-Ph | H |
| 6-1393 | 2-(Isopropoxycarbonyloxy)-eth-1-yl | Me | 4-Br-Ph | H |
| 6-1394 | 2-(Isopropoxycarbonyloxy)-eth-1-yl | Me | 5-Cl-pyridin-2-yl | H |
| 6-1395 | 2-(Isopropoxycarbonyloxy)-eth-1-yl | Me | 5-Br-pyridin-2-yl | H |
| 6-1396 | 2-(Isopropoxycarbonyloxy)-eth-1-yl | Me | 5-Cl-2-thienyl | H |
| 6-1397 | 2-(Isopropoxycarbonyloxy)-eth-1-yl | Me | 5-Br-2-thienyl | H |

**Tabelle 7: Verbindungen der Formel (Ic")**

| | | | | | |
|---|---|---|---|---|---|
| 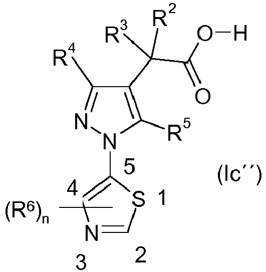 | | | | | |

| Nr. | R² | R³ | R⁴ | R⁵ | (R⁶)ₙ |
|---|---|---|---|---|---|
| 7-1 | H | H | Ph | Ph | H |
| 7-2 | H | H | Me | Ph | H |
| 7-3 | H | H | Me | 5-J-2-thienyl | H |
| 7-4 | H | H | Me | 2-Furyl | H |
| 7-5 | H | H | Me | Ph | 2-OMe |
| 7-6 | Me | H | Me | Ph | 4-Me |
| 7-7 | H | H | Me | Ph | 2-Cl |
| 7-8 | H | H | Me | Ph | 4-CF₃ |
| 7-9 | H | H | Me | Ph | 2-CF₃ |
| 7-10 | H | H | Me | Ph | 4-Me |
| 7-11 | H | H | Me | Ph | 2,4-Me₂ |
| 7-12 | H | H | Me | Ph | 2,4-Cl₂ |
| 7-13 | H | H | Me | 4-MeO-Ph | 4-Me |
| 7-14 | H | H | Me | 4-MeO-Ph | H |
| 7-15 | Me | H | Me | Ph | H |
| 7-16 | H | H | Me | 4-Me-Ph | 4-Me |
| 7-17 | H | H | Me | 4-Me-Ph | 4-Cl |
| 7-18 | H | H | Me | 4-Me-Ph | H |
| 7-19 | H | H | Me | 3-Cl-Ph | H |
| 7-20 | H | H | Me | 3-CF₃-Ph | H |
| 7-21 | H | H | Me | 3-CF₃-Ph | 4-Me |
| 7-22 | H | H | Me | 3,4-Cl₂-Ph | 4-Me |
| 7-23 | H | H | Me | 3-Cl-Ph | 4-Me |
| 7-24 | H | H | Me | 2-Cl-Ph | 4-Me |
| 7-25 | H | H | Me | 2,4-Cl₂-Ph | 4-Me |
| 7-26 | H | H | Me | 4-CF₃-Ph | 4-Me |
| 7-27 | H | H | Me | 4-Cl-Ph | 4-Me |
| 7-28 | H | H | Me | 4-Cl-Ph | 4-COOEt |
| 7-29 | H | H | Me | 4-Cl-Ph | H |
| 7-30 | H | H | Me | 3,4-Cl₂-Ph | H |
| 7-31 | H | H | Me | 4-CF₃-Ph | H |
| 7-32 | H | H | Me | 4-Cl-Ph | 4-Cl |
| 7-33 | H | H | Me | Ph | 4-Cl |
| 7-34 | H | H | Me | 2-Cl-Ph | H |
| 7-35 | H | H | Me | 4-tBu-Ph | 4-Me |
| 7-36 | H | H | Me | 3,5-Me₂-Ph | 4-Me |
| 7-37 | H | H | Me | Ph | 4-OMe |
| 7-38 | H | H | Me | 4-Cl-Ph | 4-OMe |
| 7-39 | H | H | Me | 4-Me-Ph | 4-Me |
| 7-40 | H | H | Me | 4-F-Ph | 4-Cl |
| 7-41 | H | H | Me | 4-F-Ph | 4-Me |
| 7-42 | H | H | Me | 3-Me-Ph | 4-Me |
| 7-43 | H | H | Me | 4-(COOH)-Ph | 4-Me |
| 7-44 | H | H | Me | 3-Br-Ph | 4-Me |
| 7-45 | H | H | Me | 4-Ph-Ph | 4-Me |
| 7-46 | H | H | Me | 4-(COOH)-Ph | H |
| 7-47 | H | H | Me | 3,5-Me₂-Ph | H |
| 7-48 | H | H | Me | Ph | 4-SMe |
| 7-49 | H | H | Me | 4-Cl-Ph | 4-SMe |
| 7-50 | H | H | Me | 3-Cl-4-Me-Ph | H |
| 7-51 | H | H | Me | 3-CF₃-4-Cl-Ph | H |
| 7-52 | H | H | Me | 3-CF₃-4-Cl-Ph | 4-Me |
| 7-53 | H | H | Me | 3-Cl-4-Me-Ph | 4-Me |
| 7-54 | H | H | Me | 2-Pyridyl | 4-Cl |
| 7-55 | H | H | Me | 4-CI-Ph | 4-F |
| 7-56 | H | H | Me | 2-Thienyl | 4-Me |
| 7-57 | H | H | Me | 3-Me-2-thienyl | 4-Me |
| 7-58 | H | H | Me | 4-Me-2-thienyl | 4-Me |
| 7-59 | H | H | Me | 5-Cl-2-thienyl | 4-Me |
| 7-60 | H | H | Me | 5-Cl-2-thienyl | 4-Cl |
| 7-61 | H | H | Me | 3-Thienyl | 4-Me |
| 7-62 | H | H | Me | 2-Thienyl | H |
| 7-63 | H | H | Me | 3-Me-2-thienyl | H |
| 7-64 | H | H | Me | 4-Me-2-thienyl | H |
| 7-65 | H | H | Me | 5-Cl-2-thienyl | H |
| 7-66 | H | H | Me | 5-Me-2-thienyl | H |
| 7-67 | H | H | Me | 6-MeO-pyridin-3-yl | H |
| 7-68 | H | H | Me | 5-Br-2-thienyl | H |
| 7-69 | H | H | Me | 5-Br-2-thienyl | 4-Me |
| 7-70 | H | H | Me | 3-Thienyl | H |
| 7-71 | H | H | Me | 4-Cl-Ph | 4-S(O)Me |
| 7-72 | H | H | Me | 4-Br-Ph | 4-Me |
| 7-73 | H | H | Me | 1,3-Benzodioxol-5-yl | 4-Me |
| 7-74 | H | H | Me | 4-J-Ph | 4-Me |
| 7-75 | H | H | Me | 3,5-Cl₂-Ph | 4-Me |
| 7-76 | H | H | Me | 4-PhO-Ph | 4-Me |
| 7-77 | H | H | Me | 6-OH-pyridin-3-yl | H |
| 7-78 | H | H | Me | Ph | 4-S(O)Me |
| 7-79 | H | H | H | Ph | H |
| 7-80 | H | H | H | Ph | 4-Me |
| 7-81 | H | H | Et | Ph | H |
| 7-82 | H | H | n-Pr | Ph | H |
| 7-83 | H | H | CH₂Cl | Ph | H |
| 7-84 | H | H | CHCl₂ | Ph | H |
| 7-85 | H | H | CH₂F | Ph | H |
| 7-86 | H | H | CHF₂ | Ph | H |
| 7-87 | H | H | Cl | Ph | H |
| 7-88 | H | H | Et | Ph | 4-Me |
| 7-89 | H | H | n-Pr | Ph | 4-Me |
| 7-90 | H | H | CH₂Cl | Ph | 4-Me |
| 7-91 | H | H | CHCl₂ | Ph | 4-Me |
| 7-92 | H | H | CH₂F | Ph | 4-Me |
| 7-93 | H | H | CHF₂ | Ph | 4-Me |
| 7-94 | H | H | Cl | Ph | 4-Me |
| 7-95 | H | H | Et | 4-Cl-Ph | H |
| 7-96 | H | H | n-Pr | 4-Cl-Ph | H |
| 7-97 | H | H | CH₂Cl | 4-Cl-Ph | H |
| 7-98 | H | H | CHCl₂ | 4-Cl-Ph | H |
| 7-99 | H | H | CH₂F | 4-Cl-Ph | H |
| 7-100 | H | H | CHF₂ | 4-Cl-Ph | H |
| 7-101 | H | H | Cl | 4-Cl-Ph | H |
| 7-102 | H | H | Et | 4-Me-Ph | H |
| 7-103 | H | H | n-Pr | 4-Me-Ph | H |
| 7-104 | H | H | CH₂Cl | 4-Me-Ph | H |
| 7-105 | H | H | CHCl₂ | 4-Me-Ph | H |
| 7-106 | H | H | CH₂F | 4-Me-Ph | H |
| 7-107 | H | H | CHF₂ | 4-Me-Ph | H |
| 7-108 | H | H | Cl | 4-Me-Ph | H |
| 7-109 | H | H | Et | 2-Pyridyl | H |
| 7-110 | H | H | n-Pr | 2-Pyridyl | H |
| 7-111 | H | H | CH₂Cl | 2-Pyridyl | H |
| 7-112 | H | H | CHCl₂ | 2-Pyridyl | H |
| 7-113 | H | H | CH₂F | 2-Pyridyl | H |
| 7-114 | H | H | CHF₂ | 2-Pyridyl | H |
| 7-115 | H | H | Cl | 2-Pyridyl | H |
| 7-116 | H | H | Me | 2-Pyridyl | H |
| 7-117 | H | H | Me | 5-Cl-pyridin-2-yl | H |
| 7-118 | H | H | Me | 5-Cl-pyridin-2-yl | 4-CI |
| 7-119 | H | H | Me | 5-Cl-pyridin-2-yl | 4-Me |
| 7-120 | H | H | Me | 5-Br-pyridin-2-yl | H |
| 7-121 | H | H | Me | 5-Br-pyridin-2-yl | 4-CI |
| 7-122 | H | H | Me | 5-Br-pyridin-2-yl | 4-Me |
| 7-123 | H | H | Me | 5-F-pyridin-2-yl | H |
| 7-124 | H | H | Me | 5-Me-pyridin-2-yl | H |
| 7-125 | H | H | Me | 5-Me-pyridin-2-yl | 4-Me |
| 7-126 | H | H | Me | 2,4-Cl₂-Ph | H |
| 7-127 | H | H | Me | 4-CH₂COOH-Ph | 4-Me |
| 7-128 | H | H | Me | 3,4-Me₂-Ph | 4-Me |
| 7-129 | H | H | Me | 4-Br-Ph | H |
| 7-130 | H | H | Me | 3,4-Me₂-Ph | H |
| 7-131 | H | H | Me | 3-Me-Ph | H |
| 7-132 | H | H | Me | 4-F-Ph | H |
| 7-133 | H | H | Me | 4-(Me-CO)-Ph | H |
| 7-134 | H | H | Me | 4-tBu-Ph | H |
| 7-135 | H | H | Me | 4-Cl-3-Me-Ph | H |
| 7-136 | H | H | n-Pr | 4-Cl-Ph | 4-Me |
| 7-137 | H | H | Me | 3-Pyridyl | H |
| 7-138 | H | H | Me | 4-Pyridyl | H |
| 7-139 | H | H | C(O)OMe | Ph | H |
| 7-140 | H | H | Me | 6-Me-pyridin-3-yl | H |
| 7-141 | H | H | Me | 4-Cl-Ph | 4-SO₂Me |
| 7-142 | H | H | Me | 3-Pyridyl | 4-Me |
| 7-143 | H | H | Me | 2,3-Cl₂-Ph | 4-Me |
| 7-144 | H | H | Me | 2-Pyridyl | 4-Me |
| 7-145 | H | H | H | 4-Cl-Ph | 4-Me |
| 7-146 | H | H | Me | 6-Cl-pyridin-3-yl | H |
| 7-147 | H | H | Me | Ph | 2-Me |
| 7-148 | H | H | Me | 4-Me-pyridin-2-yl | H |
| 7-149 | H | H | Me | 4-Me-pyridin-2-yl | 4-Me |
| 7-150 | H | H | Me | 4-Me-pyridin-2-yl | 4-Cl |
| 7-151 | H | H | Me | 4-Me-pyridin-2-yl | 4-F |
| 7-152 | H | H | Me | 4-F-pyridin-2-yl | H |
| 7-153 | H | H | Me | 4-Cl-pyridin-2-yl | H |
| 7-154 | H | H | Me | 4-Br-pyridin-2-yl | H |
| 7-155 | H | H | Me | 4-OMe-pyridin-2-yl | H |
| 7-156 | H | H | Me | 5-CF₃-pyridin-2-yl | H |
| 7-157 | H | H | Me | 6-OMe-pyridin-2-yl | H |
| 7-158 | H | H | cyPr | 4-Cl-Ph | H |
| 7-159 | H | H | CN | 4-Cl-Ph | H |
| 7-160 | H | H | CN | 4-Cl-Ph | 4-Me |
| 7-161 | H | H | CN | 4-Me-Ph | H |
| 7-162 | H | H | CN | 4-Me-Ph | 4-Me |
| 7-163 | H | H | CN | Ph | H |
| 7-164 | H | H | CN | Ph | 4-Me |
| 7-165 | H | H | CN | 2-pyridyl | H |
| 7-166 | H | H | CN | 3-pyridyl | H |
| 7-167 | H | H | CN | 5-Cl-pyridin-2-yl | H |
| 7-168 | H | H | CN | 5-Br-pyridin-2-yl | H |
| 7-169 | H | H | CN | 5-F-pyridin-2-yl | H |
| 7-170 | H | H | CN | 5-Me-pyridin-2-yl | H |
| 7-171 | H | H | CN | 6-Me-pyridin-3-yl | H |
| 7-172 | H | H | CN | 4-Me-pyridin-2-yl | H |
| 7-173 | H | H | CN | 4-F-pyridin-2-yl | H |
| 7-174 | H | H | CN | 4-Cl-pyridin-2-yl | H |
| 7-175 | H | H | CN | 4-Br-pyridin-2-yl | H |
| 7-176 | H | H | CN | 4-OMe-pyridin-2-yl | H |
| 7-177 | H | H | formyl | 4-Cl-Ph | H |
| 7-178 | H | H | formyl | 4-Cl-Ph | 4-Me |
| 7-179 | H | H | formyl | 4-Me-Ph | H |
| 7-180 | H | H | formyl | 4-Me-Ph | 4-Me |
| 7-181 | H | H | formyl | Ph | H |
| 7-182 | H | H | formyl | Ph | 4-Me |
| 7-183 | H | H | formyl | 2-pyridyl | H |
| 7-184 | H | H | formyl | 3-pyridyl | H |
| 7-185 | H | H | formyl | 5-Cl-pyridin-2-yl | H |
| 7-186 | H | H | formyl | 5-Br-pyridin-2-yl | H |
| 7-187 | H | H | formyl | 5-F-pyridin-2-yl | H |
| 7-188 | H | H | formyl | 5-Me-pyridin-2-yl | H |
| 7-189 | H | H | formyl | 6-Me-pyridin-3-yl | H |
| 7-190 | H | H | formyl | 4-Me-pyridin-2-yl | H |
| 7-191 | H | H | formyl | 4-F-pyridin-2-yl | H |
| 7-192 | H | H | formyl | 4-Cl-pyridin-2-yl | H |
| 7-193 | H | H | formyl | 4-Br-pyridin-2-yl | H |
| 7-194 | H | H | formyl | 4-OMe-pyridin-2-yl | H |
| 7-195 | H | H | CH₂OH | 5-Me-pyridin-2-yl | H |
| 7-196 | H | H | CH₂OH | 4-Cl-Ph | H |
| 7-197 | H | H | CH₂OH | 4-Me-pyridin-2-yl | H |
| 7-198 | H | H | CH₂OH | 4-Me-Ph | H |
| 7-199 | H | H | CH₂OH | Ph | H |
| 7-200 | H | H | CH₂OH | 2-Pyridyl | H |
| 7-201 | H | H | Me | 2-Thiazolyl | H |
| 7-202 | H | H | Me | 2-Thiazolyl | 4-Cl |
| 7-203 | H | H | Me | 2-Thiazolyl | 4-Me |
| 7-204 | H | H | Me | 4-Me-thiazol-2-yl | H |
| 7-205 | H | H | Me | 4-Me-thiazol-2-yl | 4-Cl |
| 7-206 | H | H | Me | 4-Me-thiazol-2-yl | 4-Me |
| 7-207 | H | H | Me | 5-Me-thiazol-2-yl | H |
| 7-208 | H | H | Me | 5-Br-thiazol-2-yl | H |
| 7-209 | H | H | Me | 5-Br-thiazol-2-yl | 4-Me |
| 7-210 | H | H | Me | 5-Cl-thiazol-2-yl | H |
| 7-211 | H | H | Me | 4,6-Me₂-pyridin-2-yl | H |
| 7-212 | H | H | Me | 4,6-Me₂-pyridin-2-yl | 4-Me |
| 7-213 | H | H | Me | 2-Pyridyl | 4-F |
| 7-214 | H | H | Me | 2-Pyrazinyl | H |
| 7-215 | H | H | Me | 5-Me-Pyrazin-2-yl | H |
| 7-216 | H | H | Me | 2-Pyrazinyl | 4-Me |
| 7-217 | H | H | Me | 1,3-Benzothiazol-2-yl | H |
| 7-218 | H | H | Me | 1,3-Benzothiazol-2-yl | 4-Me |
| 7-219 | H | H | Me | 7-Cl-1,3-benzothiazol-2-yl | H |
| 7-220 | H | H | Me | 1,5-Me₂-pyrazol-3-yl | H |
| 7-221 | H | H | Me | 1,5-Me₂-pyrazol-3-yl | 4-Me |
| 7-222 | H | H | Me | 4,5-Me₂-thiazol-2-yl | H |
| 7-223 | H | H | Me | 4,5-Cl₂-thiazol-2-yl | H |
| 7-224 | H | H | Me | 2-Pyrimidinyl | H |
| 7-225 | H | H | Me | 2-Pyrimidinyl | 4-Me |
| 7-226 | H | H | Me | 5-F-pyrimidin-2-yl | H |
| 7-227 | H | H | Me | 5-Cl-pyrimidin-2-yl | H |
| 7-228 | H | H | Me | 5-Br-pyrimidin-2-yl | H |
| 7-229 | H | H | Me | 5-Me-pyrimidin-2-yl | H |
| 7-230 | H | H | Me | 5-Me-pyrimidin-2-yl | 4-Me |
| 7-231 | H | H | Me | 4,6-Me₂-pyrimidin-2-yl | H |
| 7-232 | H | H | Me | 4,6-Me₂-pyrimidin-2-yl | 4-Me |
| 7-233 | H | H | Me | 3-Pyridazinyl | H |
| 7-234 | H | H | Me | 6-Me-Pyridazin-3-yl | H |
| 7-235 | H | H | Me | 1,2,4-Triazin-3-yl | H |
| 7-236 | H | H | Me | 6-Me-1,2,4-triazin-3-yl | H |
| 7-237 | H | H | Me | Chinolin-2-yl | H |
| 7-238 | H | H | Me | Isochinolin-3-yl | H |
| 7-239 | H | H | Me | 4-NO₂-Ph | H |
| 7-240 | H | H | Me | 3,5-Cl₂-Ph | H |
| 7-241 | H | H | Me | 2-Me-pyridin-4-yl | H |
| 7-242 | H | H | Me | 4-Cl-6-Me-pyridin-2-yl | H |
| 7-243 | H | H | Me | 4-Br-3-Me-Ph | H |
| 7-244 | H | H | Me | 5-Cl-pyridin-3-yl | H |
| 7-245 | H | H | Me | 5-Allyl-pyridin-2-yl | H |
| 7-246 | H | H | Me | 5-Cyclopropyl-pyridin-2-yl | H |
| 7-247 | H | H | Me | 5-Ethinyl-pyridin-2-yl | H |
| 7-248 | H | H | Me | 5-Ph-pyridin-2-yl | H |
| 7-249 | H | H | Me | 5-OH-pyridin-2-yl | H |
| 7-250 | H | H | Me | 5-OCHF₂-pyridin-2-yl | H |
| 7-251 | H | H | Me | 5-MeO-pyridin-2-yl | H |
| 7-252 | H | H | Me | 5-MeS-pyridin-2-yl | H |
| 7-253 | H | H | Me | 5-NHMe-pyridin-2-yl | H |
| 7-254 | H | H | Me | 5-NMe₂-pyridin-2-yl | H |
| 7-255 | H | H | Me | 6-Cl-1,3-benzoxazol-2-yl | H |
| 7-256 | H | H | Me | 6-Br-1,3-benzoxazol-2-yl | H |
| 7-257 | H | H | Me | 7-Cl-1,3-benzoxazol-2-yl | H |
| 7-258 | H | H | Me | 5-NH₂-pyridin-2-yl | H |
| 7-259 | H | H | Me | 2-Cl-thiazol-4-yl | H |
| 7-260 | H | H | Me | 2-Br-thiazol-4-yl | H |
| 7-261 | H | H | Me | 5-OSO₂Me-pyridin-2-yl | H |
| 7-262 | H | H | Me | 6-Cl-1,3-benzothiazol-2-yl | H |
| 7-263 | H | H | Me | 6-Br-1,3-benzothiazol-2-yl | H |
| 7-264 | H | H | Me | 1,3-Benzoxazol-2-yl | H |
| 7-265 | H | H | Me | 4-PhO-Ph | H |
| 7-266 | H | H | Me | 4-CH₂COOH-Ph | H |
| 7-267 | H | H | Me | 2,3-Cl2-Ph | H |
| 7-268 | H | H | Me | 5-I-pyridin-2-yl | H |
| 7-269 | H | H | Me | 5-I-pyrimidin-2-yl | H |
| 7-270 | H | H | Me | 3,4-F₂-Ph | H |
| 7-271 | H | H | Me | 1-Me-pyrazol-3-yl | H |
| 7-272 | H | H | Me | 1-Me-pyrazol-5-yl | H |
| 7-273 | H | H | Me | 3-Br-Ph | H |
| 7-274 | H | H | Me | 4-Ph-Ph | H |
| 7-275 | H | H | Me | 1,3-Benzodioxol-5-yl | H |
| 7-276 | H | H | Me | 4-J-Ph | H |
| 7-277 | H | H | Me | 5-Br-3-thienyl | H |
| 7-278 | H | H | Me | 5-Me-3-thienyl | H |
| 7-279 | H | H | Me | 2-F-Ph | H |
| 7-280 | H | H | Me | 2-CN-Ph | H |
| 7-281 | H | H | Me | 2-NO₂-Ph | H |
| 7-282 | H | H | Me | 2,4-F₂-Ph | H |
| 7-283 | H | H | Me | 5-Thiazolyl | H |
| 7-284 | H | H | Me | 2-Me-thiazol-4-yl | H |
| 7-285 | H | H | Me | 2-Me-thiazol-5-yl | H |
| 7-286 | H | H | Me | 5-Cl-3-thienyl | H |
| 7-287 | H | H | Me | 6-Br-pyridin-3-yl | H |
| 7-288 | H | H | Me | 4-Cl-3-thienyl | H |
| 7-289 | H | H | Me | 4-Br-3-thienyl | H |
| 7-290 | H | H | Me | 4-Me-3-thienyl | H |
| 7-291 | H | H | Me | 4-Thiazolyl | H |
| 7-292 | H | H | Me | 4-Me-5-Cl-pyridin-2-yl | H |

**Tabelle 8: Verbindungen der Formel (Ic'")**

| | | | | | |
|---|---|---|---|---|---|
| 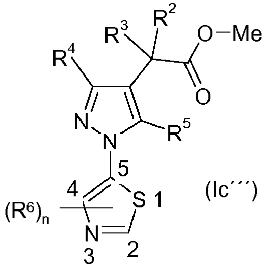 | | | | | |

| Nr. | R² | R³ | R⁴ | R⁵ | (R⁶)ₙ |
|---|---|---|---|---|---|
| 8-1 | H | H | Ph | Ph | H |
| 8-2 | H | H | Me | Ph | H |
| 8-3 | H | H | Me | 5-J-2-thienyl | H |
| 8-4 | H | H | Me | 2-Furyl | H |
| 8-5 | Me | H | Me | Ph | 2-OMe |
| 8-6 | Me | H | Me | Ph | 4-Me |
| 8-7 | Me | H | Me | Ph | 2-CI |
| 8-8 | H | H | Me | Ph | 2-CF₃ |
| 8-9 | H | H | Me | Ph | 4-CF₃ |
| 8-10 | H | H | Me | Ph | 4-Me |
| 8-11 | H | H | Me | Ph | 2,4-Me₂ |
| 8-12 | H | H | Me | Ph | 2,4-Cl₂ |
| 8-13 | H | H | Me | 4-MeO-Ph | 4-Me |
| 8-14 | H | H | Me | 4-MeO-Ph | H |
| 8-15 | Me | H | Me | Ph | H |
| 8-16 | H | H | Me | 4-Me-Ph | H |
| 8-17 | H | H | Me | 4-Me-Ph | 4-Me |
| 8-18 | H | H | Me | 4-Me-Ph | 4-Cl |
| 8-19 | H | H | Me | 3-Cl-Ph | H |
| 8-20 | H | H | Me | 3-CF₃-Ph | H |
| 8-21 | H | H | Me | 3-CF₃-Ph | 4-Me |
| 8-22 | H | H | Me | 3,4-Cl₂-Ph | 4-Me |
| 8-23 | H | H | Me | 3-Cl-Ph | 4-Me |
| 8-24 | H | H | Me | 2-Cl-Ph | 4-Me |
| 8-25 | H | H | Me | 2,4-Cl₂-Ph | 4-Me |
| 8-26 | H | H | Me | 4-CF₃-Ph | 4-Me |
| 8-27 | H | H | Me | 4-Cl-Ph | 4-Me |
| 8-28 | H | H | Me | 4-Cl-Ph | H |
| 8-29 | H | H | Me | 4-Cl-Ph | 2-Cl |
| 8-30 | H | H | Me | 4-Cl-Ph | 4-COOEt |
| 8-31 | H | H | Me | 3,4-Cl₂-Ph | H |
| 8-32 | H | H | Me | 4-CF₃-Ph | H |
| 8-33 | H | H | Me | 4-Cl-Ph | 4-Cl |
| 8-34 | H | H | Me | Ph | 4-Cl |
| 8-35 | H | H | Me | 2-Cl-Ph | H |
| 8-36 | H | H | Me | 4-tBu-Ph | 4-Me |
| 8-37 | H | H | Me | 3,5-Me₂-Ph | 4-Me |
| 8-38 | H | H | Me | Ph | 4-OMe |
| 8-39 | H | H | Me | 4-Cl-Ph | 4-OMe |
| 8-40 | H | H | Me | 4-Me-Ph | 4-Me |
| 8-41 | H | H | Me | 4-F-Ph | 4-Me |
| 8-42 | H | H | Me | 4-F-Ph | 4-Cl |
| 8-43 | H | H | Me | 3-Me-Ph | 4-Me |
| 8-44 | H | H | Me | 4-COOH-Ph | 4-Me |
| 8-45 | H | H | Me | 3-Br-Ph | 4-Me |
| 8-46 | H | H | Me | 4-Ph-Ph | 4-Me |
| 8-47 | H | H | Me | 4-COOH-Ph | H |
| 8-48 | H | H | Me | 3,5-Me₂-Ph | H |
| 8-49 | H | H | Me | Ph | 4-SMe |
| 8-50 | H | H | Me | 4-Cl-Ph | 4-SMe |
| 8-51 | H | H | Me | 3-Cl-4-Me-Ph | H |
| 8-52 | H | H | Me | 3-CF₃-4-Cl-Ph | H |
| 8-53 | H | H | Me | 3-CF₃-4-Cl-Ph | 4-Me |
| 8-54 | H | H | Me | 3-Cl-4-Me-Ph | 4-Me |
| 8-55 | H | H | Me | 2-Pyridyl | 4-Cl |
| 8-56 | H | H | Me | 4-Cl-Ph | 4-F |
| 8-57 | H | H | Me | 2-Thienyl | 4-Me |
| 8-58 | H | H | Me | 3-Me-2-thienyl | 4-Me |
| 8-59 | H | H | Me | 4-Me-2-thienyl | 4-Me |
| 8-60 | H | H | Me | 5-Cl-2-thienyl | 4-Me |
| 8-61 | H | H | Me | 5-Cl-2-thienyl | 4-Cl |
| 8-62 | H | H | Me | 3-Thienyl | 4-Me |
| 8-63 | H | H | Me | 2-Thienyl | H |
| 8-64 | H | H | Me | 3-Me-2-thienyl | H |
| 8-65 | H | H | Me | 4-Me-2-thienyl | H |
| 8-66 | H | H | Me | 5-Cl-2-thienyl | H |
| 8-67 | H | H | Me | 5-Me-2-thienyl | H |
| 8-68 | H | H | Me | 6-MeO-pyridin-3-yl | H |
| 8-69 | H | H | Me | 5-Br-2-thienyl | H |
| 8-70 | H | H | Me | 5-Br-2-thienyl | 4-Me |
| 8-71 | H | H | Me | 3-Thienyl | H |
| 8-72 | H | H | Me | 4-Cl-Ph | 4-S(O)Me |
| 8-73 | H | H | Me | 4-Br-Ph | 4-Me |
| 8-74 | H | H | Me | 1,3-Benzodioxol-5-yl | 4-Me |
| 8-75 | H | H | Me | 4-J-Ph | 4-Me |
| 8-76 | H | H | Me | 3,5-Cl₂-Ph | 4-Me |
| 8-77 | H | H | Me | 4-PhO-Ph | 4-Me |
| 8-78 | H | H | Me | 6-OH-pyridin-3-yl | H |
| 8-79 | H | H | Me | Ph | 4-S(O)Me |
| 8-80 | H | H | H | Ph | H |
| 8-81 | H | H | H | Ph | 4-Me |
| 8-82 | H | H | Et | Ph | H |
| 8-83 | H | H | n-Pr | Ph | H |
| 8-84 | H | H | CH₂Cl | Ph | H |
| 8-85 | H | H | CHCl₂ | Ph | H |
| 8-86 | H | H | CH₂F | Ph | H |
| 8-87 | H | H | CHF₂ | Ph | H |
| 8-88 | H | H | Cl | Ph | H |
| 8-89 | H | H | Et | Ph | 4-Me |
| 8-90 | H | H | n-Pr | Ph | 4-Me |
| 8-91 | H | H | CH₂Cl | Ph | 4-Me |
| 8-92 | H | H | CHCl₂ | Ph | 4-Me |
| 8-93 | H | H | CH₂F | Ph | 4-Me |
| 8-94 | H | H | CHF₂ | Ph | 4-Me |
| 8-95 | H | H | Cl | Ph | 4-Me |
| 8-96 | H | H | Et | 4-Cl-Ph | H |
| 8-97 | H | H | n-Pr | 4-Cl-Ph | H |
| 8-98 | H | H | CH₂Cl | 4-Cl-Ph | H |
| 8-99 | H | H | CHCl₂ | 4-Cl-Ph | H |
| 8-100 | H | H | CH₂F | 4-Cl-Ph | H |
| 8-101 | H | H | CHF₂ | 4-Cl-Ph | H |
| 8-102 | H | H | Cl | 4-Cl-Ph | H |
| 8-103 | H | H | Et | 4-Me-Ph | H |
| 8-104 | H | H | n-Pr | 4-Me-Ph | H |
| 8-105 | H | H | CH₂Cl | 4-Me-Ph | H |
| 8-106 | H | H | CHCl₂ | 4-Me-Ph | H |
| 8-107 | H | H | CH₂F | 4-Me-Ph | H |
| 8-108 | H | H | CHF₂ | 4-Me-Ph | H |
| 8-109 | H | H | Cl | 4-Me-Ph | H |
| 8-110 | H | H | Et | 2-Pyridyl | H |
| 8-111 | H | H | n-Pr | 2-Pyridyl | H |
| 8-112 | H | H | CH₂Cl | 2-Pyridyl | H |
| 8-113 | H | H | CHCl₂ | 2-Pyridyl | H |
| 8-114 | H | H | CH₂F | 2-Pyridyl | H |
| 8-115 | H | H | CHF₂ | 2-Pyridyl | H |
| 8-116 | H | H | Cl | 2-Pyridyl | H |
| 8-117 | H | H | Me | 2-Pyridyl | H |
| 8-118 | H | H | Me | 5-Cl-pyridin-2-yl | H |
| 8-119 | H | H | Me | 5-Cl-pyridin-2-yl | 4-Cl |
| 8-120 | H | H | Me | 5-Cl-pyridin-2-yl | 4-Me |
| 8-121 | H | H | Me | 5-Br-pyridin-2-yl | H |
| 8-122 | H | H | Me | 5-Br-pyridin-2-yl | 4-Cl |
| 8-123 | H | H | Me | 5-Br-pyridin-2-yl | 4-Me |
| 8-124 | H | H | Me | 5-F-pyridin-2-yl | H |
| 8-125 | H | H | Me | 5-Me-pyridin-2-yl | H |
| 8-126 | H | H | Me | 5-Me-pyridin-2-yl | 4-Me |
| 8-127 | H | H | Me | 2,4-Cl₂-Ph | H |
| 8-128 | H | H | Me | 4-(CH₂COOH)-Ph | 4-Me |
| 8-129 | H | H | Me | 3,4-Me₂-Ph | 4-Me |
| 8-130 | H | H | Me | 4-Br-Ph | H |
| 8-131 | H | H | Me | 3,4-Me₂-Ph | H |
| 8-132 | H | H | Me | 3-Me-Ph | H |
| 8-133 | H | H | Me | 4-F-Ph | H |
| 8-134 | H | H | Me | 4-(Me-CO)-Ph | H |
| 8-135 | H | H | Me | 4-tBu-Ph | H |
| 8-136 | H | H | Me | 4-Cl-3-Me-Ph | H |
| 8-137 | H | H | n-Pr | 4-Cl-Ph | 4-Me |
| 8-138 | H | H | Me | 3-Pyridyl | H |
| 8-139 | H | H | Me | 4-Pyridyl | H |
| 8-140 | H | H | C(O)OMe | Ph | H |
| 8-141 | H | H | Me | 6-Me-pyridin-3-yl | H |
| 8-142 | H | H | Me | 4-Cl-Ph | 4-SO₂Me |
| 8-143 | H | H | Me | 3-Pyridyl | 4-Me |
| 8-144 | H | H | Me | 2,3-Cl₂-Ph | 4-Me |
| 8-145 | H | H | Me | 2-Pyridyl | 4-Me |
| 8-146 | H | H | H | 4-Cl-Ph | 4-Me |
| 8-147 | H | H | Me | 6-Cl-pyridin-3-yl | H |
| 8-148 | H | H | Me | 4-Cl-Ph | 2-Me |
| 8-149 | H | H | Me | Ph | 2-Me |
| 8-150 | H | H | Me | 4-Me-pyridin-2-yl | H |
| 8-151 | H | H | Me | 4-Me-pyridin-2-yl | 4-Me |
| 8-152 | H | H | Me | 4-Me-pyridin-2-yl | 4-Cl |
| 8-153 | H | H | Me | 4-Me-pyridin-2-yl | 4-F |
| 8-154 | H | H | Me | 4-F-pyridin-2-yl | H |
| 8-155 | H | H | Me | 4-Cl-pyridin-2-yl | H |
| 8-156 | H | H | Me | 4-Br-pyridin-2-yl | H |
| 8-157 | H | H | Me | 4-OMe-pyridin-2-yl | H |
| 8-158 | H | H | Me | 5-CF₃-pyridin-2-yl | H |
| 8-159 | H | H | Me | 6-OMe-pyridin-2-yl | H |
| 8-160 | H | H | cyPr | 4-Cl-Ph | H |
| 8-161 | H | H | CN | 4-Cl-Ph | H |
| 8-162 | H | H | CN | 4-Cl-Ph | 4-Me |
| 8-163 | H | H | CN | 4-Me-Ph | H |
| 8-164 | H | H | CN | 4-Me-Ph | 4-Me |
| 8-165 | H | H | CN | Ph | H |
| 8-166 | H | H | CN | Ph | 4-Me |
| 8-167 | H | H | CN | 2-pyridyl | H |
| 8-168 | H | H | CN | 3-pyridyl | H |
| 8-169 | H | H | CN | 5-Cl-pyridin-2-yl | H |
| 8-170 | H | H | CN | 5-Br-pyridin-2-yl | H |
| 8-171 | H | H | CN | 5-F-pyridin-2-yl | H |
| 8-172 | H | H | CN | 5-Me-pyridin-2-yl | H |
| 8-173 | H | H | CN | 6-Me-pyridin-3-yl | H |
| 8-174 | H | H | CN | 4-Me-pyridin-2-yl | H |
| 8-175 | H | H | CN | 4-F-pyridin-2-yl | H |
| 8-176 | H | H | CN | 4-Cl-pyridin-2-yl | H |
| 8-177 | H | H | CN | 4-Br-pyridin-2-yl | H |
| 8-178 | H | H | CN | 4-OMe-pyridin-2-yl | H |
| 8-179 | H | H | Formyl | 4-CI-Ph | H |
| 8-180 | H | H | Formyl | 4-CI-Ph | 4-Me |
| 8-181 | H | H | Formyl | 4-Me-Ph | H |
| 8-182 | H | H | Formyl | 4-Me-Ph | 4-Me |
| 8-183 | H | H | Formyl | Ph | H |
| 8-184 | H | H | Formyl | Ph | 4-Me |
| 8-185 | H | H | Formyl | 2-pyridyl | H |
| 8-186 | H | H | Formyl | 3-pyridyl | H |
| 8-187 | H | H | Formyl | 5-Cl-pyridin-2-yl | H |
| 8-188 | H | H | Formyl | 5-Br-pyridin-2-yl | H |
| 8-189 | H | H | Formyl | 5-F-pyridin-2-yl | H |
| 8-190 | H | H | Formyl | 5-Me-pyridin-2-yl | H |
| 8-191 | H | H | Formyl | 6-Me-pyridin-3-yl | H |
| 8-192 | H | H | Formyl | 4-Me-pyridin-2-yl | H |
| 8-193 | H | H | Formyl | 4-F-pyridin-2-yl | H |
| 8-194 | H | H | Formyl | 4-Cl-pyridin-2-yl | H |
| 8-195 | H | H | Formyl | 4-Br-pyridin-2-yl | H |
| 8-196 | H | H | Formyl | 4-OMe-pyridin-2-yl | H |
| 8-197 | H | H | CH₂OH | 5-Me-pyridin-2-yl | H |
| 8-198 | H | H | CH₂OH | 4-Cl-Ph | H |
| 8-199 | H | H | CH₂OH | 4-Me-pyridin-2-yl | H |
| 8-200 | H | H | CH₂OH | 4-Me-Ph | H |
| 8-201 | H | H | CH₂OH | Ph | H |
| 8-202 | H | H | CH₂OH | 2-Pyridyl | H |
| 8-203 | H | H | Me | 2-Thiazolyl | H |
| 8-204 | H | H | Me | 2-Thiazolyl | 4-Cl |
| 8-205 | H | H | Me | 2-Thiazolyl | 4-Me |
| 8-206 | H | H | Me | 4-Me-thiazol-2-yl | H |
| 8-207 | H | H | Me | 4-Me-thiazol-2-yl | 4-Cl |
| 8-208 | H | H | Me | 4-Me-thiazol-2-yl | 4-Me |
| 8-209 | H | H | Me | 5-Me-thiazol-2-yl | H |
| 8-210 | H | H | Me | 5-Br-thiazol-2-yl | H |
| 8-211 | H | H | Me | 5-Br-thiazol-2-yl | 4-Me |
| 8-212 | H | H | Me | 5-Cl-thiazol-2-yl | H |
| 8-213 | H | H | Me | 4,6-Me₂-pyridin-2-yl | H |
| 8-214 | H | H | Me | 4,6-Me₂-pyridin-2-yl | 4-Me |
| 8-215 | H | H | Me | 2-Pyridyl | 4-F |
| 8-216 | H | H | Me | 2-Pyrazinyl | H |
| 8-217 | H | H | Me | 5-Me-Pyrazin-2-yl | H |
| 8-218 | H | H | Me | 2-Pyrazinyl | 4-Me |
| 8-219 | H | H | Me | 1,3-Benzothiazol-2-yl | H |
| 8-220 | H | H | Me | 1,3-Benzothiazol-2-yl | 4-Me |
| 8-221 | H | H | Me | 7-Cl-1,3-benzothiazol-2-yl | H |
| 8-222 | H | H | Me | 1,5-Me₂-pyrazol-3-yl | H |
| 8-223 | H | H | Me | 1,5-Me₂-pyrazol-3-yl | 4-Me |
| 8-224 | H | H | Me | 4,5-Me₂-thiazol-2-yl | H |
| 8-225 | H | H | Me | 4,5-Cl₂-thiazol-2-yl | H |
| 8-226 | H | H | Me | 2-Pyrimidinyl | H |
| 8-227 | H | H | Me | 2-Pyrimidinyl | 4-Me |
| 8-228 | H | H | Me | 5-F-pyrimidin-2-yl | H |
| 8-229 | H | H | Me | 5-Cl-pyrimidin-2-yl | H |
| 8-230 | H | H | Me | 5-Br-pyrimidin-2-yl | H |
| 8-231 | H | H | Me | 5-Me-pyrimidin-2-yl | H |
| 8-232 | H | H | Me | 5-Me-pyrimidin-2-yl | 4-Me |
| 8-233 | H | H | Me | 4,6-Me₂-pyrimidin-2-yl | H |
| 8-234 | H | H | Me | 4,6-Me₂-pyrimidin-2-yl | 4-Me |
| 8-235 | H | H | Me | 3-Pyridazinyl | H |
| 8-236 | H | H | Me | 6-Me-Pyridazin-3-yl | H |
| 8-237 | H | H | Me | 1,2,4-Triazin-3-yl | H |
| 8-238 | H | H | Me | 6-Me-1,2,4-triazin-3-yl | H |
| 8-239 | H | H | Me | Chinolin-2-yl | H |
| 8-240 | H | H | Me | Isochinolin-3-yl | H |
| 8-241 | H | H | Me | 2-Pyridyl | 4-COOEt |
| 8-242 | H | H | Me | 4-NO₂-Ph | H |
| 8-243 | H | H | Me | 3,5-Cl₂-Ph | H |
| 8-244 | H | H | Me | 2-Me-pyridin-4-yl | H |
| 8-245 | H | H | Me | 4-Cl-6-Me-pyridin-2-yl | H |
| 8-246 | H | H | Me | 4-Br-3-Me-Ph | H |
| 8-247 | H | H | Me | 5-Cl-pyridin-3-yl | H |
| 8-248 | H | H | Me | 5-Allyl-pyridin-2-yl | H |
| 8-249 | H | H | Me | 5-Cyclopropyl-pyridin-2-yl | H |
| 8-250 | H | H | Me | 5-Ethinyl-pyridin-2-yl | H |
| 8-251 | H | H | Me | 5-Ph-pyridin-2-yl | H |
| 8-252 | H | H | Me | 5-OH-pyridin-2-yl | H |
| 8-253 | H | H | Me | 5-OCHF₂-pyridin-2-yl | H |
| 8-254 | H | H | Me | 5-MeO-pyridin-2-yl | H |
| 8-255 | H | H | Me | 5-MeS-pyridin-2-yl | H |
| 8-256 | H | H | Me | 5-NHMe-pyridin-2-yl | H |
| 8-257 | H | H | Me | 5-NMe₂-pyridin-2-yl | H |
| 8-258 | H | H | Me | 6-Cl-1,3-benzoxazol-2-yl | H |
| 8-259 | H | H | Me | 6-Br-1,3-benzoxazol-2-yl | H |
| 8-260 | H | H | Me | 7-Cl-1,3-benzoxazol-2-yl | H |
| 8-261 | H | H | Me | 5-NH₂-pyridin-2-yl | H |
| 8-262 | H | H | Me | 2-Cl-thiazol-4-yl | H |
| 8-263 | H | H | Me | 2-Br-thiazol-4-yl | H |
| 8-264 | H | H | Me | 5-OSO₂Me-pyridin-2-yl | H |
| 8-265 | H | H | Me | 6-Cl-1,3-benzothiazol-2-yl | H |
| 8-266 | H | H | Me | 6-Br-1,3-benzothiazol-2-yl | H |
| 8-267 | H | H | Me | 1,3-Benzoxazol-2-yl | H |
| 8-268 | H | H | Me | 4-PhO-Ph | H |
| 8-269 | H | H | Me | 2,3-Cl₂-Ph | H |
| 8-270 | H | H | Me | 5-I-pyridin-2-yl | H |
| 8-271 | H | H | Me | 5-I-pyrimidin-2-yl | H |
| 8-272 | H | H | Me | 3,4-F₂-Ph | H |
| 8-273 | H | H | Me | 1-Me-pyrazol-3-yl | H |
| 8-274 | H | H | Me | 1-Me-pyrazol-5-yl | H |
| 8-275 | H | H | Me | 3-Br-Ph | H |
| 8-276 | H | H | Me | 4-Ph-Ph | H |
| 8-277 | H | H | Me | 1,3-Benzodioxol-5-yl | H |
| 8-278 | H | H | Me | 4-J-Ph | H |
| 8-279 | H | H | Me | 5-Br-3-thienyl | H |
| 8-280 | H | H | Me | 5-Me-3-thienyl | H |
| 8-281 | H | H | Me | 2-F-Ph | H |
| 8-282 | H | H | Me | 2-CN-Ph | H |
| 8-283 | H | H | Me | 2-NO₂-Ph | H |
| 8-284 | H | H | Me | 2,4-F₂-Ph | H |
| 8-285 | H | H | Me | 5-Thiazolyl | H |
| 8-286 | H | H | Me | 2-Me-thiazol-4-yl | H |
| 8-287 | H | H | Me | 2-Me-thiazol-5-yl | H |
| 8-288 | H | H | Me | 5-Cl-3-thienyl | H |
| 8-289 | H | H | Me | 6-Br-pyridin-3-yl | H |
| 8-290 | H | H | Me | 4-Cl-3-thienyl | H |
| 8-291 | H | H | Me | 4-Br-3-thienyl | H |
| 8-292 | H | H | Me | 4-Me-3-thienyl | H |
| 8-293 | H | H | Me | 4-Thiazolyl | H |
| 8-294 | H | H | Me | 4-Me-5-Cl-pyridin-2-yl | H |

Für einige Verbindungen der allgemeinen Formel (I) gemäß Tabelle 8 wurden ¹H-NMR-Spektren bei 400 MHz (CDCl₃) (¹H-Kernresonanz-Daten) gemessen. Nachfolgend sind charakteristische chemische Verschiebungen δ (ppm) für einige Beispielverbindungen aufgeführt (die Nummer der Verbindung gehört zur laufenden Nr. in Tabelle 8):
NMR Verbindung 8-28 (CDCl₃, 400 MHz, δ in ppm): 2.31 *(s,* 3H); 3.34 *(s,* 2H); 3.69 (*s,* 3H); 7.25 (*d*, 2H); 7.29 *(s,* 1H); 7.41 (*d*, 2H); 8.52 (*s*, 1H).
NMR Verbindung 8-32 (CDCl₃, 400 MHz, δ in ppm): 2.34 *(s,* 3H); 3.37 *(s,* 2H); 3.70 *(s,* 3H); 7.29 *(s,* 1 H); 7.45 (*d,* 2H); 7.69 (*d,* 2H); 8.56 (*s*, 1H).
NMR Verbindung 8-217 (CDCl₃, 400 MHz, δ in ppm): 2.33 (*s,* 3H); 2.61 *(s,* 3H); 3.54 *(s,* 2H); 3.69 (s, 3H); 7.49 *(s,* 1 H); 8.49 (*s,* 1H); 8.52 *(s,* 1 H); 8.63 (s, 1 H).
NMR Verbindung 8-117 (CDCl₃, 400 MHz, δ in ppm): 2.33 *(s,* 3H); 3.55 (s, 2H); 3.69 (*s,* 3H); 7.32 (*dd,* 1 H); 7,38 (*t*,1H); 7.41 (*s,* 1H); 7,75 (*t*,1H); 8.56 (s, 1 H); 8.69 (d, 1 H).
NMR Verbindung 8-2 (CDCl₃, 400 MHz, δ in ppm): 2.33 (*s, 3H);* 3.38 *(s,* 2H); 3.70 *(s,* 3H); 7.25 *(s,* 1 H); 7,30 (*m,* 2H); 7.46 *(m,* 3H); 8.50 (*s*, 1H).
NMR Verbindung 8-133 ([D₆]DMSO, 400 MHz, δ in ppm): 2.23 *(s,* 3H); 3.39 (*s,* 2H); 3.59 (*s,* 3H); 7.34 *(s,* 1 H); 7.37 *(m,* 5H); 8.87 (*s,* 1H).
NMR Verbindung 8-16 (CDCl₃, 400 MHz, δ in ppm): 2.31 *(s,* 3H); 2.40 *(s,* 3H); 3.36 (*s, 2*H); 3.69 (*s*, 3H); 7.19 (*d*, 2H); 7.23 *(s,* 1H); 7.25 (*d,* 2H); 8.49 *(s,* 1 H).
NMR Verbindung 8-230 (CDCl₃, 400 MHz, δ in ppm): 2.33 (*s,* 3H); 3.69 (*s,* 3H); 3.88 *(s,* 2H); 7.73 *(s,* 1 H); 8.72 *(s,* 1 H); 8.73 (*s, 2H).*
NMR Verbindung 8-69 (([D₆]DMSO, 400 MHz, δ in ppm): 2.22 (*s*, 3H); 3.49 (*s,* 2H); 3.62 (*s,* 3H); 7.08 (*d,* 1 H); 7.34 (*d,* 1 H); 7.67 (*s,* 1H); 8.99 (*s*, 1H).
NMR Verbindung 8-229 (CDCl₃, 400 MHz, δ in ppm): 2.35 (*s,* 3H); 3.69 (*s,* 3H); 3.88 *(s,* 2H); 7.72 *(s,* 1 H); 8.64 (*s*, 2H); 8.72 (*s,* 1H).
NMR Verbindung 8-121 (CDCl₃, 400 MHz, δ in ppm): 2.35 (*s,* 3H); 3.55 *(s,* 2H); 3.69 (*s,* 3H); 7.26 (*d,* 1 H); 7.48 (*s,* 1H); 7,87 (*dd*,1H); 8.62 *(s,* 1 H); 8.74 (*d,* 1 H).
NMR Verbindung 8-276 (CDCl₃, 400 MHz, δ in ppm): 2.35 (*s,* 3H); 3.42 *(s,* 2H); 3.71 *(s,* 3H); 7.38 (*m,* 4H); 7.49 (*m,* 2H); 7,65 (*m*,4H); 8.52 (s, 1 H).
NMR Verbindung 8-118 (CDCl₃, 400 MHz, δ in ppm): 2.35 (*s,* 3H); 3.55 *(s,* 2H); 3.69 (*s,* 3H); 7.32 (*d,* 1 H); 7.47 *(s,* 1 H); 7,71 (*dd*,1H); 8.61 *(s,* 1 H); 8.64 (*d,* 1 H).
NMR Verbindung 8-64 (CDCl₃, 400 MHz, δ in ppm): 1.98 *(s,* 3H); 2.35 (*s,* 3H); 3.48 (broad *s,* 2H); 3.68 (*s,* 3H); 6.97 (*d,* 1 H); 7.34 *(s,* 1 H); 7,47 (d,1 H); 8.48 (s, 1 H).
NMR Verbindung 8-4 (CDCl₃, 400 MHz, δ in ppm): 2.32 *(s,* 3H); 3.53 (*s,* 2H); 3.72 *(s,* 3H); 6.48 *(m,* 2H); 7.52 *(m,* 1 H); 7,58 (*s*,1H); 8.65 (s, 1H).
NMR Verbindung 8-294 (CDCl₃, 400 MHz, δ in ppm): 2.35 (*s,* 3H); 2.39 (*s,* 3H); 3.52 *(s,* 2H); 3.70 *(s,* 3H); 7.25 *(s,* 1 H); 7.44 *(s,* 1 H); 8.56 (s, 1 H); 8.60 (s, 1 H).
NMR Verbindung 8-219 (CDCl₃, 400 MHz, δ in ppm): 2.38 *(s,* 3H); 3.72 *(s,* 3H); 3.90 *(s,* 2H); 7.43 (*t*, 1 H); 7,52 (*t*,1H); 7.85 (*d,* 1 H); 7.89 (*s,* 1 H); 8.08 (*d,* 1 H); 8.81 *(s,* 1H).
NMR Verbindung 8-239 (CDCl₃, 400 MHz, δ in ppm): 2.38 *(s,* 3H); 3.65 (*s,* 3H); 3.68 (*s,* 2H); 7.38 (*d,* 1 H); 7,48 (*s*,1 H); 7.62 (*t*, 1 H); 7.78 (*t*, 1H); 7.88 *(d, 1H*); 8.10 *(d,* 1H); 8.19 (*d,* 1H); 8.68 *(s,* 1 H).
NMR Verbindung 8-240 (CDCl₃, 400 MHz, δ in ppm): 2.38 *(s,* 3H); 3.57 *(s,* 2H); 3.70 *(s,* 3H); 7.42 *(s,* 1 H); 7.71 *(t,* 1 H); 7.78 (*t*, 1 H); 7,83 (*s*,1H); 7.85 *(d, 1H);* 8.04 (*d,* 1 H); 8.53 *(s,* 1 H); 9.29 (*s,* 1 H).

**Tabelle 9: Verbindungen der Formel (Ic'''')**

| | | | | |
|---|---|---|---|---|
| 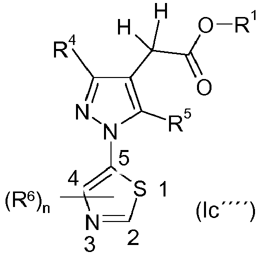 | | | | |

| Nr. | R¹ | R⁴ | R⁵ | (R⁶)ₙ |
|---|---|---|---|---|
| 9-1 | Et | Me | Ph | H |
| 9-2 | Et | Me | Ph | 4-Me |
| 9-3 | Et | Me | 3-Cl-Ph | H |
| 9-4 | Et | Me | 4-Cl-Ph | H |
| 9-5 | Et | Me | 4-Cl-Ph | 4-Me |
| 9-6 | Et | Me | 2-Thienyl | H |
| 9-7 | Et | Me | 3-Thienyl | H |
| 9-8 | Et | Me | 3-Me-2-thienyl | H |
| 9-9 | Et | Me | 4-Me-2-thienyl | H |
| 9-10 | Et | Me | 5-Br-2-thienyl | H |
| 9-11 | Et | Me | 5-Br-2-thienyl | 4-Me |
| 9-12 | Et | Me | 5-Cl-2-thienyl | H |
| 9-13 | Et | Me | 5-Cl-2-thienyl | 4-Me |
| 9-14 | Et | Me | 5-J-2-thienyl | H |
| 9-15 | Et | Me | 5-Me-2-thienyl | H |
| 9-16 | Et | Me | 3-Pyridyl | H |
| 9-17 | Et | Me | 6-MeO-pyridin-3-yl | H |
| 9-18 | Et | Me | 6-OH-pyridin-3-yl | H |
| 9-19 | Et | Me | 6-Me-pyridin-3-yl | H |
| 9-20 | Et | Me | 4-Me-Ph | H |
| 9-21 | Et | Me | 4-Me-Ph | 4-Me |
| 9-22 | Et | Me | 4-Br-Ph | H |
| 9-23 | Et | Me | 4-F-Ph | H |
| 9-24 | Et | Me | 4-F-Ph | 4-Me |
| 9-25 | Et | Me | 5-Cl-pyridin-2-yl | H |
| 9-26 | Et | Me | 5-Br-pyridin-2-yl | H |
| 9-27 | Et | Me | 5-F-pyridin-2-yl | H |
| 9-28 | Et | Me | 5-F-pyridin-2-yl | 4-Me |
| 9-29 | Et | Me | 5-Cl-pyridin-2-yl | 4-Me |
| 9-30 | Et | Me | 5-Br-pyridin-2-yl | 4-Me |
| 9-31 | Et | Me | 5-Me-pyridin-2-yl | H |
| 9-32 | Et | Me | 5-Me-pyridin-2-yl | 4-Me |
| 9-33 | Et | Me | 2-Pyridyl | 4-Me |
| 9-34 | Et | Me | 2-Pyridyl | H |
| 9-35 | Et | Me | 4-Pyridyl | H |
| 9-36 | Et | Me | 4-Me-pyridin-2-yl | H |
| 9-37 | Et | Me | 4-Me-pyridin-2-yl | 4-Me |
| 9-38 | Et | Me | 2-Thiazolyl | H |
| 9-39 | Et | Me | 4-Me-thiazol-2-yl | H |
| 9-40 | Et | Me | 5-Br-thiazol-2-yl | H |
| 9-41 | Et | Me | 5-Cl-thiazol-2-yl | H |
| 9-42 | Et | Me | 5-Me-thiazol-2-yl | H |
| 9-43 | Et | Me | 4,5-Me₂-thiazol-2-yl | H |
| 9-44 | Et | Me | 4,5-Cl₂-thiazol-2-yl | H |
| 9-45 | Et | Me | 4,6-Me₂-pyridin-2-yl | H |
| 9-46 | Et | Me | 2-Pyrazinyl | H |
| 9-47 | Et | Me | 2-Pyrimidinyl | H |
| 9-48 | Et | Me | 2-Pyrimidinyl | 4-Me |
| 9-49 | Et | Me | 5-Cl-pyrimidin-2-yl | H |
| 9-50 | Et | Me | 5-Br-pyrimidin-2-yl | H |
| 9-51 | Et | Me | 5-Me-pyrimidin-2-yl | H |
| 9-52 | Et | Me | 5-Me-pyrimidin-2-yl | 4-Me |
| 9-53 | Et | Me | 4,6-Me₂-pyrimidin-2-yl | H |
| 9-54 | Et | Me | 4,6-Me₂-pyrimidin-2-yl | 4-Me |
| 9-55 | Et | Me | 1,3-Benzothiazol-2-yl | H |
| 9-56 | Et | Me | 7-Cl-1,3-benzothiazol-2-yl | H |
| 9-57 | Et | Me | 1,5-Me₂-pyrazol-3-yl | H |
| 9-58 | Et | Me | 5-Me-Pyrazin-2-yl | H |
| 9-59 | Et | Me | 5-F-pyrimidin-2-yl | H |
| 9-60 | Et | Me | 3-Pyridazinyl | H |
| 9-61 | Et | Me | 6-Me-Pyridazin-3-yl | H |
| 9-62 | Et | Me | 1,2,4-Triazin-3-yl | H |
| 9-63 | Et | Me | 6-Me-1,2,4-triazin-3-yl | H |
| 9-64 | Et | Me | Chinolin-2-yl | H |
| 9-65 | Et | Me | Isochinolin-3-yl | H |
| 9-66 | Pr | Me | Ph | H |
| 9-67 | Pr | Me | 4-Cl-Ph | H |
| 9-68 | Pr | Me | 2-Thienyl | H |
| 9-69 | Pr | Me | 3-Pyridyl | H |
| 9-70 | Pr | Me | 6-Me-pyridin-3-yl | H |
| 9-71 | Pr | Me | 4-Me-Ph | H |
| 9-72 | Pr | Me | 4-Br-Ph | H |
| 9-73 | Pr | Me | 4-F-Ph | H |
| 9-74 | Pr | Me | 5-Cl-pyridin-2-yl | H |
| 9-75 | Pr | Me | 5-Br-pyridin-2-yl | H |
| 9-76 | Pr | Me | 5-F-pyridin-2-yl | H |
| 9-77 | Pr | Me | 5-Me-pyridin-2-yl | H |
| 9-78 | Pr | Me | 2-Pyridyl | H |
| 9-79 | Pr | Me | 4-Pyridyl | H |
| 9-80 | i-Pr | Me | Ph | H |
| 9-81 | i-Pr | Me | 4-Cl-Ph | H |
| 9-82 | i-Pr | Me | 2-Thienyl | H |
| 9-83 | i-Pr | Me | 3-Pyridyl | H |
| 9-84 | i-Pr | Me | 6-Me-pyridin-3-yl | H |
| 9-85 | i-Pr | Me | 4-Me-Ph | H |
| 9-86 | i-Pr | Me | 4-Br-Ph | H |
| 9-87 | i-Pr | Me | 4-F-Ph | H |
| 9-88 | i-Pr | Me | 5-Cl-pyridin-2-yl | H |
| 9-89 | i-Pr | Me | 5-Br-pyridin-2-yl | H |
| 9-90 | i-Pr | Me | 5-F-pyridin-2-yl | H |
| 9-91 | i-Pr | Me | 5-Me-pyridin-2-yl | H |
| 9-92 | i-Pr | Me | 2-Pyridyl | H |
| 9-93 | i-Pr | Me | 4-Pyridyl | H |
| 9-94 | CH₂Ph | Me | Ph | H |
| 9-95 | CH₂Ph | Me | 4-Cl-Ph | H |
| 9-96 | CH₂Ph | Me | 2-Thienyl | H |
| 9-97 | CH₂Ph | Me | 2-Pyridyl | H |
| 9-98 | prop-2-in-1-yl | Me | Ph | H |
| 9-99 | Prop-2-in-1-yl | Me | 4-Cl-Ph | H |
| 9-100 | Prop-2-in-1-yl | Me | 2-Thienyl | H |
| 9-101 | Prop-2-in-1-yl | Me | 3-Thienyl | H |
| 9-102 | Prop-2-in-1-yl | Me | 3-Me-2-thienyl | H |
| 9-103 | Prop-2-in-1-yl | Me | 4-Me-2-thienyl | H |
| 9-104 | Prop-2-in-1-yl | Me | 5-Cl-2-thienyl | H |
| 9-105 | Prop-2-in-1-yl | Me | 5-Me-2-thienyl | H |
| 9-106 | Prop-2-in-1-yl | Me | 3-Pyridyl | H |
| 9-107 | Prop-2-in-1-yl | Me | 6-MeO-pyridin-3-yl | H |
| 9-108 | Prop-2-in-1-yl | H | Ph | H |
| 9-109 | Prop-2-in-1-yl | Me | 6-Me-pyridin-3-yl | H |
| 9-110 | Prop-2-in-1-yl | Me | 4-Me-Ph | H |
| 9-111 | Prop-2-in-1-yl | Me | 4-Br-Ph | H |
| 9-112 | Prop-2-in-1-yl | Me | 4-F-Ph | H |
| 9-113 | Prop-2-in-1-yl | Me | 5-Cl-pyridin-2-yl | H |
| 9-114 | Prop-2-in-1-yl | Me | 5-Br-pyridin-2-yl | H |
| 9-115 | Prop-2-in-1-yl | Me | 5-F-pyridin-2-yl | H |
| 9-116 | Prop-2-in-1-yl | Me | 5-Me-pyridin-2-yl | H |
| 9-117 | Prop-2-in-1-yl | Me | 2-Pyridyl | H |
| 9-118 | Prop-2-in-1-yl | Me | 4-Pyridyl | H |
| 9-119 | Prop-2-in-1-yl | Me | 4-Cl-Ph | 4-Me |
| 9-120 | Prop-2-in-1-yl | Me | Ph | 4-Me |
| 9-121 | Cyclopropylmethyl | Me | Ph | H |
| 9-122 | Cyclopropylmethyl | Me | 4-Cl-Ph | H |
| 9-123 | Cyclopropylmethyl | Me | 2-Thienyl | H |
| 9-124 | Cyclopropylmethyl | Me | 3-Thienyl | H |
| 9-125 | Cyclopropylmethyl | Me | 3-Me-2-thienyl | H |
| 9-126 | Cyclopropylmethyl | Me | 3-Pyridyl | H |
| 9-127 | Cyclopropylmethyl | Me | 5-Cl-2-thienyl | H |
| 9-128 | Cyclopropylmethyl | Me | 5-Me-2-thienyl | H |
| 9-129 | Cyclopropylmethyl | Me | 4-Me-2-thienyl | H |
| 9-130 | Cyclopropylmethyl | Me | 6-MeO-pyridin-3-yl | H |
| 9-131 | Cyclopropylmethyl | Me | 6-OH-pyridin-3-yl | H |
| 9-132 | Cyclopropylmethyl | Me | 6-Me-pyridin-3-yl | H |
| 9-133 | Cyclopropylmethyl | Me | 4-Me-Ph | H |
| 9-134 | Cyclopropylmethyl | Me | 4-Br-Ph | H |
| 9-135 | Cyclopropylmethyl | Me | 4-F-Ph | H |
| 9-136 | Cyclopropylmethyl | Me | 5-Cl-pyridin-2-yl | H |
| 9-137 | Cyclopropylmethyl | Me | 5-Br-pyridin-2-yl | H |
| 9-138 | Cyclopropylmethyl | Me | 5-F-pyridin-2-yl | H |
| 9-139 | Cyclopropylmethyl | Me | 5-Me-pyridin-2-yl | H |
| 9-140 | Cyclopropylmethyl | Me | 2-Pyridyl | H |
| 9-141 | Cyclopropylmethyl | Me | 4-Pyridyl | H |
| 9-142 | Cyclopropylmethyl | Me | 4-Cl-Ph | 4-Me |
| 9-143 | Cyclopropylmethyl | Me | Ph | 4-Me |
| 9-144 | Cyclopropylmethyl | H | Ph | H |
| 9-145 | Cyclopropylmethyl | H | Chinolin-2-yl | H |
| 9-146 | Cyclopropylmethyl | H | Isochinolin-3-yl | H |
| 9-147 | 3,3-Dichlor-2-fluorprop-2-en-1-yl | Me | Ph | H |
| 9-148 | 3,3-Dichlor-2-fluorprop-2-en-1-yl | Me | 4-Cl-Ph | H |
| 9-149 | 3,3-Dichlor-2-fluorprop-2-en-1-yl | Me | 2-Thienyl | H |
| 9-150 | 3,3-Dichlor-2-fluorprop-2-en-1-yl | Me | 2-Pyridyl | H |
| 9-151 | (1-Methylcyclopropyl)-methyl | Me | Ph | H |
| 9-152 | (1-Methylcyclopropyl)-methyl | Me | 4-Cl-Ph | H |
| 9-153 | (1-Methylcyclopropyl)-methyl | Me | 2-Thienyl | H |
| 9-154 | (1-Methylcyclopropyl)-methyl | Me | 2-Pyridyl | H |
| 9-155 | 4-Chlorbut-2-in-1-yl | Me | Ph | H |
| 9-156 | 4-Chlorbut-2-in-1-yl | Me | 4-Cl-Ph | H |
| 9-157 | 4-Chlorbut-2-in-1-yl | Me | 2-Thienyl | H |
| 9-158 | 4-Chlorbut-2-in-1-yl | Me | 2-Pyridyl | H |
| 9-159 | (2,2-Dichlorcyclopropyl)-methyl | Me | Ph | H |
| 9-160 | (2,2-Dichlorcyclopropyl)-methyl | Me | 4-Cl-Ph | H |
| 9-161 | (2,2-Dichlorcyclopropyl)-methyl | Me | 2-Thienyl | H |
| 9-162 | (2,2-Dichlorcyclopropyl)-methyl | Me | 2-Pyridyl | H |
| 9-163 | But-2-in-1-yl | Me | Ph | H |
| 9-164 | But-2-in-1-yl | Me | 4-Cl-Ph | H |
| 9-165 | But-2-in-1-yl | Me | 2-Thienyl | H |
| 9-166 | But-2-in-1-yl | Me | 3-Thienyl | H |
| 9-167 | But-2-in-1-yl | Me | 3-Me-2-thienyl | H |
| 9-168 | But-2-in-1-yl | Me | 4-Me-2-thienyl | H |
| 9-169 | But-2-in-1-yl | Me | 5-Cl-2-thienyl | H |
| 9-170 | But-2-in-1-yl | Me | 5-Me-2-thienyl | H |
| 9-171 | But-2-in-1-yl | Me | 3-Pyridyl | H |
| 9-172 | But-2-in-1-yl | Me | 6-MeO-pyridin-3-yl | H |
| 9-173 | But-2-in-1-yl | H | Ph | H |
| 9-174 | But-2-in-1-yl | Me | 6-Me-pyridin-3-yl | H |
| 9-175 | But-2-in-1-yl | Me | 4-Me-Ph | H |
| 9-176 | But-2-in-1-yl | Me | 4-Br-Ph | H |
| 9-177 | But-2-in-1-yl | Me | 4-F-Ph | H |
| 9-178 | But-2-in-1-yl | Me | 5-Cl-pyridin-2-yl | H |
| 9-179 | But-2-in-1-yl | Me | 5-Br-pyridin-2-yl | H |
| 9-180 | But-2-in-1-yl | Me | 5-F-pyridin-2-yl | H |
| 9-181 | But-2-in-1-yl | Me | 5-Me-pyridin-2-yl | H |
| 9-182 | But-2-in-1-yl | Me | 2-Pyridyl | H |
| 9-183 | But-2-in-1-yl | Me | 4-Pyridyl | H |
| 9-184 | But-2-in-1-yl | Me | 4-Cl-Ph | 4-Me |
| 9-185 | But-2-in-1-yl | Me | Ph | 4-Me |
| 9-186 | 1-Methylprop-2-in-1-yl | Me | Ph | H |
| 9-187 | 1-Methylprop-2-in-1-yl | Me | 4-Cl-Ph | H |
| 9-188 | 1-Methylprop-2-in-1-yl | Me | 2-Thienyl | H |
| 9-189 | 1-Methylprop-2-in-1-yl | Me | 2-Pyridyl | H |
| 9-190 | 1-Cyclopropylethyl | Me | Ph | H |
| 9-191 | 1-Cyclopropylethyl | Me | 4-Cl-Ph | H |
| 9-192 | 1-Cyclopropylethyl | Me | 2-Thienyl | H |
| 9-193 | 1-Cyclopropylethyl | Me | 2-Pyridyl | H |
| 9-194 | Allyl | Me | Ph | H |
| 9-195 | Allyl | Me | 4-Cl-Ph | H |
| 9-196 | Allyl | Me | 2-Thienyl | H |
| 9-197 | Allyl | Me | 2-Pyridyl | H |
| 9-198 | 3-Methylbut-2-en-1-yl | Me | Ph | H |
| 9-199 | 3-Methylbut-2-en-1-yl | Me | 4-Cl-Ph | H |
| 9-200 | 3-Methylbut-2-en-1-yl | Me | 2-Thienyl | H |
| 9-201 | 3-Methylbut-2-en-1-yl | Me | 2-Pyridyl | H |
| 9-202 | 2-Methylprop-2-en-1-yl | Me | Ph | H |
| 9-203 | 2-Methylprop-2-en-1-yl | Me | 4-Cl-Ph | H |
| 9-204 | 2-Methylprop-2-en-1-yl | Me | 2-Thienyl | H |
| 9-205 | 2-Methylprop-2-en-1-yl | Me | 2-Pyridyl | H |
| 9-206 | (2E)-1-Methylbut-2-en-1-yl | Me | Ph | H |
| 9-207 | (2E)-1-Methylbut-2-en-1-yl | Me | 4-Cl-Ph | H |
| 9-208 | (2E)-1-Methylbut-2-en-1-yl | Me | 2-Thienyl | H |
| 9-209 | (2E)-1-Methylbut-2-en-1-yl | Me | 2-Pyridyl | H |
| 9-210 | 3-Phenylprop-2-in-1-yl | Me | Ph | H |
| 9-211 | 3-Phenylprop-2-in-1-yl | Me | 4-Cl-Ph | H |
| 9-212 | 3-Phenylprop-2-in-1-yl | Me | 2-Thienyl | H |
| 9-213 | 3-Phenylprop-2-in-1-yl | Me | 2-Pyridyl | H |
| 9-214 | Cyclobutylmethyl | Me | Ph | H |
| 9-215 | Cyclobutylmethyl | Me | 4-Cl-Ph | H |
| 9-216 | Cyclobutylmethyl | Me | 2-Thienyl | H |
| 9-217 | Cyclobutylmethyl | Me | 2-Pyridyl | H |
| 9-218 | Cyclopentylmethyl | Me | Ph | H |
| 9-219 | Cyclopentylmethyl | Me | 4-Cl-Ph | H |
| 9-220 | Cyclopentylmethyl | Me | 2-Thienyl | H |
| 9-221 | Cyclopentylmethyl | Me | 2-Pyridyl | H |
| 9-222 | Cyclohexylmethyl | Me | Ph | H |
| 9-223 | Cyclohexylmethyl | Me | 4-Cl-Ph | H |
| 9-224 | Cyclohexylmethyl | Me | 2-Thienyl | H |
| 9-225 | Cyclohexylmethyl | Me | 2-Pyridyl | H |
| 9-226 | But-3-en-1-yl | Me | Ph | H |
| 9-227 | But-3-en-1-yl | Me | 4-Cl-Ph | H |
| 9-228 | But-3-en-1-yl | Me | 2-Thienyl | H |
| 9-229 | But-3-en-1-yl | Me | 2-Pyridyl | H |
| 9-230 | 2-Ch loroprop-2-en-1-yl | Me | Ph | H |
| 9-231 | 2-Chloroprop-2-en-1-yl | Me | 4-Cl-Ph | H |
| 9-232 | 2-Chloroprop-2-en-1-yl | Me | 2-Thienyl | H |
| 9-233 | 2-Chloroprop-2-en-1-yl | Me | 3-Thienyl | H |
| 9-234 | 2-Chloroprop-2-en-1-yl | Me | 3-Me-2-thienyl | H |
| 9-235 | 2-Chloroprop-2-en-1-yl | Me | 4-Me-2-thienyl | H |
| 9-236 | 2-Chloroprop-2-en-1-yl | Me | 5-Cl-2-thienyl | H |
| 9-237 | 2-Chloroprop-2-en-1-yl | Me | 5-Me-2-thienyl | H |
| 9-238 | 2-Chloroprop-2-en-1-yl | Me | 3-Pyridyl | H |
| 9-239 | 2-Chloroprop-2-en-1-yl | Me | 6-MeO-pyridin-3-yl | H |
| 9-240 | 2-Chloroprop-2-en-1-yl | Me | 6-OH-pyridin-3-yl | H |
| 9-241 | 2-Chloroprop-2-en-1-yl | Me | 6-Me-pyridin-3-yl | H |
| 9-242 | 2-Chloroprop-2-en-1-yl | Me | 4-Me-Ph | H |
| 9-243 | 2-Chloroprop-2-en-1-yl | Me | 4-Br-Ph | H |
| 9-244 | 2-Chloroprop-2-en-1-yl | Me | 4-F-Ph | H |
| 9-245 | 2-Chloroprop-2-en-1-yl | Me | 5-Cl-pyridin-2-yl | H |
| 9-246 | 2-Chloroprop-2-en-1-yl | Me | 5-Br-pyridin-2-yl | H |
| 9-247 | 2-Chloroprop-2-en-1-yl | Me | 5-F-pyridin-2-yl | H |
| 9-248 | 2-Chloroprop-2-en-1-yl | Me | 5-Me-pyridin-2-yl | H |
| 9-249 | 2-Chloroprop-2-en-1-yl | Me | 2-Pyridyl | H |
| 9-250 | 2-Chloroprop-2-en-1-yl | Me | 4-Pyridyl | H |
| 9-251 | 2-Chloroprop-2-en-1-yl | Me | 4-Cl-Ph | 4-Me |
| 9-252 | 2-Chloroprop-2-en-1-yl | Me | Ph | 4-Me |
| 9-253 | 2-Chloroprop-2-en-1-yl | H | Ph | H |
| 9-254 | 2-Chloroprop-2-en-1-yl | H | Chinolin-2-yl | H |
| 9-255 | 2-Chloroprop-2-en-1-yl | H | Isochinolin-3-yl | H |
| 9-256 | 2-Methoxyethyl | Me | Ph | H |
| 9-257 | 2-Methoxyethyl | Me | 4-Cl-Ph | H |
| 9-258 | 2-Methoxyethyl | Me | 2-Thienyl | H |
| 9-259 | 2-Methoxyethyl | Me | 2-Pyridyl | H |
| 9-260 | Tetrahydrofuran-2-yl-methyl | Me | Ph | H |
| 9-261 | Tetrahydrofuran-2-yl-methyl | Me | 4-Cl-Ph | H |
| 9-262 | Tetrahydrofuran-2-yl-methyl | Me | 2-Thienyl | H |
| 9-263 | Tetrahydrofuran-2-yl-methyl | Me | 2-Pyridyl | H |
| 9-264 | 2-(Dimethylamino)ethyl | Me | Ph | H |
| 9-265 | 2-(Dimethylamino)ethyl | Me | 4-Cl-Ph | H |
| 9-266 | 2-(Dimethylamino)ethyl | Me | 2-Thienyl | H |
| 9-267 | 2-(Dimethylamino)ethyl | Me | 2-Pyridyl | H |
| 9-268 | Oxetan-3-yl | Me | Ph | H |
| 9-269 | Oxetan-3-yl | Me | 4-Cl-Ph | H |
| 9-270 | Oxetan-3-yl | Me | 2-Thienyl | H |
| 9-271 | Oxetan-3-yl | Me | 2-Pyridyl | H |
| 9-272 | (3-Methyloxetan-3-yl)methyl | Me | Ph | H |
| 9-273 | (3-Methyloxetan-3-yl)methyl | Me | 4-Cl-Ph | H |
| 9-274 | (3-Methyloxetan-3-yl)methyl | Me | 2-Thienyl | H |
| 9-275 | (3-Methyloxetan-3-yl)methyl | Me | 2-Pyridyl | H |
| 9-276 | 2,2,2-Trifluorethyl | Me | Ph | H |
| 9-277 | 2,2,2-Trifluorethyl | Me | 4-Cl-Ph | H |
| 9-278 | 2,2,2-Trifluorethyl | Me | 2-Thienyl | H |
| 9-279 | 2,2,2-Trifluorethyl | Me | 3-Pyridyl | H |
| 9-280 | 2,2,2-Trifluorethyl | Me | 6-Me-pyridin-3-yl | H |
| 9-281 | 2,2,2-Trifluorethyl | Me | 4-Me-Ph | H |
| 9-282 | 2,2,2-Trifluorethyl | Me | 4-Br-Ph | H |
| 9-283 | 2,2,2-Trifluorethyl | Me | 4-F-Ph | H |
| 9-284 | 2,2,2-Trifluorethyl | Me | 5-Cl-pyridin-2-yl | H |
| 9-285 | 2,2,2-Trifluorethyl | Me | 5-Br-pyridin-2-yl | H |
| 9-286 | 2,2,2-Trifluorethyl | Me | 5-F-pyridin-2-yl | H |
| 9-287 | 2,2,2-Trifluorethyl | Me | 5-Me-pyridin-2-yl | H |
| 9-288 | 2,2,2-Trifluorethyl | Me | 2-Pyridyl | H |
| 9-289 | 2,2,2-Trifluorethyl | Me | 4-Pyridyl | H |
| 9-290 | CH₂(4-Cl-Ph) | Me | Ph | H |
| 9-291 | CH₂(4-Cl-Ph) | Me | 4-Cl-Ph | H |
| 9-292 | CH₂(4-Cl-Ph) | Me | 2-Thienyl | H |
| 9-293 | CH₂(4-Cl-Ph) | Me | 3-Pyridyl | H |
| 9-294 | CH₂(4-Cl-Ph) | Me | 6-Me-pyridin-3-yl | H |
| 9-295 | CH₂(4-Cl-Ph) | Me | 4-Me-Ph | H |
| 9-296 | CH₂(4-Cl-Ph) | Me | 4-Br-Ph | H |
| 9-297 | CH₂(4-Cl-Ph) | Me | 4-F-Ph | H |
| 9-298 | CH₂(4-Cl-Ph) | Me | 5-Cl-pyridin-2-yl | H |
| 9-299 | CH₂(4-Cl-Ph) | Me | 5-Br-pyridin-2-yl | H |
| 9-300 | CH₂(4-Cl-Ph) | Me | 5-F-pyridin-2-yl | H |
| 9-301 | CH₂(4-Cl-Ph) | Me | 5-Me-pyridin-2-yl | H |
| 9-302 | CH₂(4-Cl-Ph) | Me | 2-Pyridyl | H |
| 9-303 | CH₂(4-Cl-Ph) | Me | 4-Pyridyl | H |
| 9-304 | CH₂(4-F-Ph) | Me | Ph | H |
| 9-305 | CH₂(4-F-Ph) | Me | 4-Cl-Ph | H |
| 9-306 | CH₂(4-F-Ph) | Me | 2-Thienyl | H |
| 9-307 | CH₂(4-F-Ph) | Me | 3-Pyridyl | H |
| 9-308 | CH₂(4-F-Ph) | Me | 6-Me-pyridin-3-yl | H |
| 9-309 | CH₂(4-F-Ph) | Me | 4-Me-Ph | H |
| 9-310 | CH₂(4-F-Ph) | Me | 4-Br-Ph | H |
| 9-311 | CH₂(4-F-Ph) | Me | 4-F-Ph | H |
| 9-312 | CH₂(4-F-Ph) | Me | 5-Cl-pyridin-2-yl | H |
| 9-313 | CH₂(4-F-Ph) | Me | 5-Br-pyridin-2-yl | H |
| 9-314 | CH₂(4-F-Ph) | Me | 5-F-pyridin-2-yl | H |
| 9-315 | CH₂(4-F-Ph) | Me | 5-Me-pyridin-2-yl | H |
| 9-316 | CH₂(4-F-Ph) | Me | 2-Pyridyl | H |
| 9-317 | CH₂(4-F-Ph) | Me | 4-Pyridyl | H |
| 9-318 | CH₂(4-OMe-Ph) | Me | Ph | H |
| 9-319 | CH₂(4-OMe-Ph) | Me | 4-Cl-Ph | H |
| 9-320 | CH₂(4-OMe-Ph) | Me | 2-Thienyl | H |
| 9-321 | CH₂(4-OMe-Ph) | Me | 3-Pyridyl | H |
| 9-322 | CH₂(4-OMe-Ph) | Me | 6-Me-pyridin-3-yl | H |
| 9-323 | CH₂(4-OMe-Ph) | Me | 4-Me-Ph | H |
| 9-324 | CH₂(4-OMe-Ph) | Me | 4-Br-Ph | H |
| 9-325 | CH₂(4-OMe-Ph) | Me | 4-F-Ph | H |
| 9-326 | CH₂(4-OMe-Ph) | Me | 5-Cl-pyridin-2-yl | H |
| 9-327 | CH₂(4-OMe-Ph) | Me | 5-Br-pyridin-2-yl | H |
| 9-328 | CH₂(4-OMe-Ph) | Me | 5-F-pyridin-2-yl | H |
| 9-329 | CH₂(4-OMe-Ph) | Me | 5-Me-pyridin-2-yl | H |
| 9-330 | CH₂(4-OMe-Ph) | Me | 2-Pyridyl | H |
| 9-331 | CH₂(4-OMe-Ph) | Me | 4-Pyridyl | H |
| 9-332 | 2,2-Difluorethyl | Me | Ph | H |
| 9-333 | 2,2-Difluorethyl | Me | 4-Cl-Ph | H |
| 9-334 | 2,2-Difluorethyl | Me | 2-Thienyl | H |
| 9-335 | 2,2-Difluorethyl | Me | 2-Pyridyl | H |
| 9-336 | Ph | Me | Ph | H |
| 9-337 | Ph | Me | 4-Cl-Ph | H |
| 9-338 | Ph | Me | 2-Thienyl | H |
| 9-339 | Ph | Me | 2-Pyridyl | H |
| 9-340 | 2-Fluorethyl | Me | Ph | H |
| 9-341 | 2-Fluorethyl | Me | 4-Cl-Ph | H |
| 9-342 | 2-Fluorethyl | Me | 2-Thienyl | H |
| 9-343 | 2-Fluorethyl | Me | 2-Pyridyl | H |
| 9-344 | 2,2,3,3,3-Pentafluorpropyl | Me | Ph | H |
| 9-345 | 2,2,3,3,3-Pentafluorpropyl | Me | 4-Cl-Ph | H |
| 9-346 | 2,2,3,3,3-Pentafluorpropyl | Me | 2-Thienyl | H |
| 9-347 | 2,2,3,3,3-Pentafluorpropyl | Me | 2-Pyridyl | H |
| 9-348 | 1-Ethyl-5-methyl-1 H-pyrazol-4-yl-methyl | Me | Ph | H |
| 9-349 | 1-Ethyl-5-methyl-1 H-pyrazol-4-yl-methyl | Me | 4-CI-Ph | H |
| 9-350 | 1-Ethyl-5-methyl-1 H-pyrazol-4-yl-methyl | Me | 2-Thienyl | H |
| 9-351 | 1-Ethyl-5-methyl-1 H-pyrazol-4-yl-methyl | Me | 2-Pyridyl | H |
| 9-352 | Et | Me | 6-Cl-1,3-benzothiazol-2-yl | H |
| 9-353 | Et | Me | 6-Br-1,3-benzothiazol-2-yl | H |
| 9-354 | Prop-2-in-1-yl | Me | Isochinolin-3-yl | H |
| 9-355 | Prop-2-in-1-yl | Me | Chinolin-2-yl | H |
| 9-356 | But-2-in-1-yl | Me | Isochinolin-3-yl | H |
| 9-357 | But-2-in-1-yl | Me | Chinolin-2-yl | H |
| 9-358 | 2,2-Difluorethyl | Me | 5-Cl-pyridin-2-yl | H |
| 9-359 | But-3-in-2-yl | Me | 5-Cl-pyridin-2-yl | H |
| 9-360 | But-3-in-2-yl | Me | Isochinolin-3-yl | H |
| 9-361 | But-3-in-2-yl | Me | Chinolin-2-yl | H |
| 9-362 | But-3-in-2-yl | Me | Ph | H |
| 9-363 | But-3-in-2-yl | Me | 4-Cl-Ph | H |
| 9-364 | But-3-in-2-yl | Me | 2-Thienyl | H |
| 9-365 | But-3-in-2-yl | Me | 3-Pyridyl | H |
| 9-366 | But-3-in-2-yl | Me | 6-Me-pyridin-3-yl | H |
| 9-367 | But-3-in-2-yl | Me | 4-Me-Ph | H |
| 9-368 | But-3-in-2-yl | Me | 4-Br-Ph | H |
| 9-369 | But-3-in-2-yl | Me | 4-F-Ph | H |
| 9-370 | But-3-in-2-yl | Me | 5-Br-pyridin-2-yl | H |
| 9-371 | But-3-in-2-yl | Me | 5-F-pyridin-2-yl | H |
| 9-372 | But-3-in-2-yl | Me | 5-Me-pyridin-2-yl | H |
| 9-373 | But-3-in-2-yl | Me | 2-Pyridyl | H |
| 9-374 | But-3-in-2-yl | Me | 4-Pyridyl | H |
| 9-375 | Pr | Me | Isochinolin-3-yl | H |
| 9-376 | Pr | Me | Chinolin-2-yl | H |
| 9-377 | iPr | Me | Isochinolin-3-yl | H |
| 9-378 | iPr | Me | Chinolin-2-yl | H |
| 9-379 | CH₂Ph | Me | Isochinolin-3-yl | H |
| 9-380 | CH₂Ph | Me | Chinolin-2-yl | H |
| 9-381 | 3,3-Dichlor-2-fluorprop-2-en-1-yl | Me | Isochinolin-3-yl | H |
| 9-382 | 3,3-Dichlor-2-fluorprop-2-en-1-yl | Me | Chinolin-2-yl | H |
| 9-383 | (1-Methylcyclopropyl)-methyl | Me | Isochinolin-3-yl | H |
| 9-384 | (1-Methylcyclopropyl)-methyl | Me | Chinolin-2-yl | H |
| 9-385 | 4-Chlorbut-2-in-1-yl | Me | Isochinolin-3-yl | H |
| 9-386 | 4-Chlorbut-2-in-1-yl | Me | Chinolin-2-yl | H |
| 9-387 | (2,2-Dichlorcyclopropyl)-methyl | Me | Isochinolin-3-yl | H |
| 9-388 | (2,2-Dichlorcyclopropyl)-methyl | Me | Chinolin-2-yl | H |
| 9-389 | 1-Methylprop-2-in-1-yl | Me | Isochinolin-3-yl | H |
| 9-390 | 1-Methylprop-2-in-1-yl | Me | Chinolin-2-yl | H |
| 9-391 | 1-Cyclopropylethyl | Me | Isochinolin-3-yl | H |
| 9-392 | 1-Cyclopropylethyl | Me | Chinolin-2-yl | H |
| 9-393 | Allyl | Me | Isochinolin-3-yl | H |
| 9-394 | Allyl | Me | Chinolin-2-yl | H |
| 9-395 | 3-Methylbut-2-en-1-yl | Me | Isochinolin-3-yl | H |
| 9-396 | 3-Methylbut-2-en-1-yl | Me | Chinolin-2-yl | H |
| 9-397 | Cyclobutylmethyl | Me | Isochinolin-3-yl | H |
| 9-398 | Cyclobutylmethyl | Me | Chinolin-2-yl | H |
| 9-399 | Cyclopentylmethyl | Me | Isochinolin-3-yl | H |
| 9-400 | Cyclopentylmethyl | Me | Chinolin-2-yl | H |
| 9-401 | Tetrahydrofuran-2-yl-methyl | Me | Isochinolin-3-yl | H |
| 9-402 | Tetrahydrofuran-2-yl-methyl | Me | Chinolin-2-yl | H |
| 9-403 | Tetrahydrofuran-2-yl-methyl | Me | 5-Cl-pyridin-2-yl | H |
| 9-404 | Tetrahydrofuran-2-yl-methyl | Me | 5-Br-pyridin-2-yl | H |
| 9-405 | Oxetan-3-yl | Me | Isochinolin-3-yl | H |
| 9-406 | Oxetan-3-yl | Me | Chinolin-2-yl | H |
| 9-407 | Oxetan-3-yl | Me | 5-Cl-pyridin-2-yl | H |
| 9-408 | Oxetan-3-yl | Me | 5-Br-pyridin-2-yl | H |
| 9-409 | (3-Methyloxetan-3-yl)methyl | Me | Isochinolin-3-yl | H |
| 9-410 | (3-Methyloxetan-3-yl)methyl | Me | Chinolin-2-yl | H |
| 9-411 | (3-Methyloxetan-3-yl)methyl | Me | 5-Cl-pyridin-2-yl | H |
| 9-412 | (3-Methyloxetan-3-yl)methyl | Me | 5-Br-pyridin-2-yl | H |
| 9-413 | 2,2,2-Trifluorethyl | Me | Isochinolin-3-yl | H |
| 9-414 | 2,2,2-Trifluorethyl | Me | Chinolin-2-yl | H |
| 9-415 | 2,2,2-Trifluorethyl | Me | 5-Cl-pyridin-2-yl | H |
| 9-416 | 2,2,2-Trifluorethyl | Me | 5-Br-pyridin-2-yl | H |
| 9-417 | 2,2-Difluorethyl | Me | Isochinolin-3-yl | H |
| 9-418 | 2,2-Difluorethyl | Me | Chinolin-2-yl | H |
| 9-419 | 2,2-Difluorethyl | Me | 5-Cl-pyridin-2-yl | H |
| 9-420 | 2,2-Difluorethyl | Me | 5-Br-pyridin-2-yl | H |
| 9-421 | Et | Me | 4-OMe-pyridin-2-yl | H |
| 9-422 | Et | Me | 4-F-pyridin-2-yl | H |
| 9-423 | Et | Me | 4-Cl-pyridin-2-yl | H |
| 9-424 | Et | Me | 4-Br-pyridin-2-yl | H |
| 9-425 | Et | Me | 6-Cl-pyridin-3-yl | H |
| 9-426 | Et | Me | 6-Br-pyridin-3-yl | H |
| 9-427 | Et | Me | 4-Cl-3-thienyl | H |
| 9-428 | Et | Me | 4-Br-3-thienyl | H |
| 9-429 | Et | Me | 4-Me-3-thienyl | H |
| 9-430 | Et | Me | 4-Thiazolyl | H |
| 9-431 | Et | Me | 5-Thiazolyl | H |
| 9-432 | Et | Me | 2-Me-thiazol-4-yl | H |
| 9-433 | Et | Me | 2-Me-thiazol-5-yl | H |
| 9-434 | Et | Me | 5-Cl-3-thienyl | H |
| 9-435 | Et | Me | 5-Br-3-thienyl | H |
| 9-436 | Et | Me | 5-Me-3-thienyl | H |
| 9-437 | Et | Me | 2-Cl-Ph | H |
| 9-438 | Et | Me | 2,4-Cl₂-Ph | H |
| 9-439 | Et | Me | 2-F-Ph | H |
| 9-440 | Et | Me | 2-CN-Ph | H |
| 9-441 | Et | Me | 2-NO₂-Ph | H |
| 9-442 | Et | Me | 2,4-F₂-Ph | H |
| 9-443 | Et | Me | 3,4-F₂-Ph | H |
| 9-444 | Et | Me | 1-Me-pyrazol-3-yl | H |
| 9-445 | Et | Me | 2-Furyl | H |
| 9-446 | Et | Me | 4-MeO-Ph | H |
| 9-447 | Et | Me | 3-CF₃-Ph | H |
| 9-448 | Et | Me | 3,4-Cl₂-Ph | H |
| 9-449 | Et | Me | 4-CF₃-Ph | H |
| 9-450 | Et | Me | 4-tBu-Ph | H |
| 9-451 | Et | Me | 3,5-Me₂-Ph | H |
| 9-452 | Et | Me | 3-Me-Ph | H |
| 9-453 | Et | Me | 3-Br-Ph | H |
| 9-454 | Et | Me | 4-Ph-Ph | H |
| 9-455 | Et | Me | 3-Cl-4-Me-Ph | H |
| 9-456 | Et | Me | 3-CF₃-4-Cl-Ph | H |
| 9-457 | Et | Me | 1,3-Benzodioxol-5-yl | H |
| 9-458 | Et | Me | 4-J-Ph | H |
| 9-459 | Et | Me | 3,5-Cl₂-Ph | H |
| 9-460 | Et | Me | 4-PhO-Ph | H |
| 9-461 | Et | Me | 3,4-Me₂-Ph | H |
| 9-462 | Et | Me | 4-(Me-CO)-Ph | H |
| 9-463 | Et | Me | 4-Cl-3-Me-Ph | H |
| 9-464 | Et | Me | 2,3-Cl₂-Ph | H |
| 9-465 | Et | Me | 5-CF₃-pyridin-2-yl | H |
| 9-466 | Et | Me | 6-OMe-pyridin-2-yl | H |
| 9-467 | Et | Me | 2-Me-pyridin-4-yl | H |
| 9-468 | Et | Me | 4-Cl-6-Me-pyridin-2-yl | H |
| 9-469 | Et | Me | 4-Br-3-Me-Ph | H |
| 9-470 | Et | Me | 5-Cl-pyridin-3-yl | H |
| 9-471 | Et | Me | 5-Allyl-pyridin-2-yl | H |
| 9-472 | Et | Me | 5-Cyclopropyl-pyridin-2-yl | H |
| 9-473 | Et | Me | 5-Ethinyl-pyridin-2-yl | H |
| 9-474 | Et | Me | 5-Ph-pyridin-2-yl | H |
| 9-475 | Et | Me | 5-I-pyridin-2-yl | H |
| 9-476 | Et | Me | 5-I-pyrimidin-2-yl | H |
| 9-477 | Et | Me | 2-Cl-thiazol-4-yl | H |
| 9-478 | Et | Me | 2-Br-thiazol-4-yl | H |
| 9-479 | Et | Me | 5-OSO₂Me-pyridin-2-yl | H |
| 9-480 | Et | Me | 1,3-Benzoxazol-2-yl | H |
| 9-481 | Et | Me | 6-Cl-1,3-benzoxazol-2-yl | H |
| 9-482 | Et | Me | 6-Br-1,3-benzoxazol-2-yl | H |
| 9-483 | Et | Me | 7-Cl-1,3-benzoxazol-2-yl | H |
| 9-484 | Et | Me | 5-NH₂-pyridin-2-yl | H |
| 9-485 | Et | Me | 5-OH-pyridin-2-yl | H |
| 9-486 | Et | Me | 5-OCHF₂-pyridin-2-yl | H |
| 9-487 | Et | Me | 5-MeO-pyridin-2-yl | H |
| 9-488 | Et | Me | 5-MeS-pyridin-2-yl | H |
| 9-489 | Et | Me | 5-NHMe-pyridin-2-yl | H |
| 9-490 | Et | Me | 5-NMe₂-pyridin-2-yl | H |
| 9-491 | Et | Me | 4-NO₂-Ph | H |
| 9-492 | Cyclopropylmethyl | Me | 4-Thiazolyl | H |
| 9-493 | Prop-2-in-1-yl | Me | 4-Thiazolyl | H |
| 9-494 | But-2-in-1-yl | Me | 4-Thiazolyl | H |
| 9-495 | But-3-in-2-yl | Me | 4-Thiazolyl | H |
| 9-496 | Pr | Me | 4-Thiazolyl | H |
| 9-497 | iPr | Me | 4-Thiazolyl | H |
| 9-498 | CH₂Ph | Me | 4-Thiazolyl | H |
| 9-499 | 3,3-Dichlor-2-fluorprop-2-en-1-yl | Me | 4-Thiazolyl | H |
| 9-500 | (1-Methylcyclopropyl)-methyl | Me | 4-Thiazolyl | H |
| 9-501 | 4-Chlorbut-2-in-1-yl | Me | 4-Thiazolyl | H |
| 9-502 | (2,2-Dichlorcyclopropyl)-methyl | Me | 4-Thiazolyl | H |
| 9-503 | 1-Methylprop-2-in-1-yl | Me | 4-Thiazolyl | H |
| 9-504 | 1-Cyclopropylethyl | Me | 4-Thiazolyl | H |
| 9-505 | Allyl | Me | 4-Thiazolyl | H |
| 9-506 | 3-Methylbut-2-en-1-yl | Me | 4-Thiazolyl | H |
| 9-507 | Cyclobutylmethyl | Me | 4-Thiazolyl | H |
| 9-508 | Cyclopentylmethyl | Me | 4-Thiazolyl | H |
| 9-509 | 2-Chloroprop-2-en-1-yl | Me | 4-Thiazolyl | H |
| 9-510 | Tetrahydrofuran-2-yl-methyl | Me | 4-Thiazolyl | H |
| 9-511 | (3-Methyloxetan-3-yl)-methyl | Me | 4-Thiazolyl | H |
| 9-512 | 2,2,2-Trifluorethyl | Me | 4-Thiazolyl | H |
| 9-513 | 2,2-Difluorethyl | Me | 4-Thiazolyl | H |
| 9-514 | Oxetan-3-yl | Me | 4-Thiazolyl | H |
| 9-515 | Cyclopropylmethyl | Me | 3-Br-Ph | H |
| 9-516 | Prop-2-in-1-yl | Me | 3-Br-Ph | H |
| 9-517 | But-2-in-1-yl | Me | 3-Br-Ph | H |
| 9-518 | But-3-in-2-yl | Me | 3-Br-Ph | H |
| 9-519 | Pr | Me | 3-Br-Ph | H |
| 9-520 | iPr | Me | 3-Br-Ph | H |
| 9-521 | CH₂Ph | Me | 3-Br-Ph | H |
| 9-522 | 3,3-Dichlor-2-fluorprop-2-en-1-yl | Me | 3-Br-Ph | H |
| 9-523 | (1-Methylcyclopropyl)-methyl | Me | 3-Br-Ph | H |
| 9-524 | 4-Chlorbut-2-in-1-yl | Me | 3-Br-Ph | H |
| 9-525 | (2,2-Dichlorcyclopropyl)-methyl | Me | 3-Br-Ph | H |
| 9-526 | 1-Methylprop-2-in-1-yl | Me | 3-Br-Ph | H |
| 9-527 | 1-Cyclopropylethyl | Me | 3-Br-Ph | H |
| 9-528 | Allyl | Me | 3-Br-Ph | H |
| 9-529 | 3-Methylbut-2-en-1-yl | Me | 3-Br-Ph | H |
| 9-530 | Cyclobutylmethyl | Me | 3-Br-Ph | H |
| 9-531 | Cyclopentylmethyl | Me | 3-Br-Ph | H |
| 9-532 | 2-Chloroprop-2-en-1-yl | Me | 3-Br-Ph | H |
| 9-533 | Tetrahydrofuran-2-yl-methyl | Me | 3-Br-Ph | H |
| 9-534 | (3-Methyloxetan-3-yl)-methyl | Me | 3-Br-Ph | H |
| 9-535 | 2,2,2-Trifluorethyl | Me | 3-Br-Ph | H |
| 9-536 | 2,2-Difluorethyl | Me | 3-Br-Ph | H |
| 9-537 | Oxetan-3-yl | Me | 3-Br-Ph | H |
| 9-538 | Cyclopropylmethyl | Me | 2-Cl-thiazol-4-yl | H |
| 9-539 | Prop-2-in-1-yl | Me | 2-Cl-thiazol-4-yl | H |
| 9-540 | But-2-in-1-yl | Me | 2-Cl-thiazol-4-yl | H |
| 9-541 | But-3-in-2-yl | Me | 2-Cl-thiazol-4-yl | H |
| 9-542 | Pr | Me | 2-Cl-thiazol-4-yl | H |
| 9-543 | iPr | Me | 2-Cl-thiazol-4-yl | H |
| 9-544 | CH₂Ph | Me | 2-Cl-thiazol-4-yl | H |
| 9-545 | 3,3-Dichlor-2-fluorprop-2-en-1-yl | Me | 2-Cl-thiazol-4-yl | H |
| 9-546 | (1-Methylcyclopropyl)-methyl | Me | 2-Cl-thiazol-4-yl | H |
| 9-547 | 4-Chlorbut-2-in-1-yl | Me | 2-Cl-thiazol-4-yl | H |
| 9-548 | (2,2-Dichlorcyclopropyl)-methyl | Me | 2-Cl-thiazol-4-yl | H |
| 9-549 | 1-Methylprop-2-in-1-yl | Me | 2-Cl-thiazol-4-yl | H |
| 9-550 | 1-Cyclopropylethyl | Me | 2-Cl-thiazol-4-yl | H |
| 9-551 | Allyl | Me | 2-Cl-thiazol-4-yl | H |
| 9-552 | 3-Methylbut-2-en-1-yl | Me | 2-Cl-thiazol-4-yl | H |
| 9-553 | Cyclobutylmethyl | Me | 2-Cl-thiazol-4-yl | H |
| 9-554 | Cyclopentylmethyl | Me | 2-Cl-thiazol-4-yl | H |
| 9-555 | 2-Chloroprop-2-en-1-yl | Me | 2-Cl-thiazol-4-yl | H |
| 9-556 | Tetrahydrofuran-2-yl-methyl | Me | 2-Cl-thiazol-4-yl | H |
| 9-557 | (3-Methyloxetan-3-yl)-methyl | Me | 2-Cl-thiazol-4-yl | H |
| 9-558 | 2,2,2-Trifluorethyl | Me | 2-Cl-thiazol-4-yl | H |
| 9-559 | 2,2-Difluorethyl | Me | 2-Cl-thiazol-4-yl | H |
| 9-560 | Oxetan-3-yl | Me | 2-Cl-thiazol-4-yl | H |
| 9-561 | Cyclopropylmethyl | Me | 2-Br-thiazol-4-yl | H |
| 9-562 | Prop-2-in-1-yl | Me | 2-Br-thiazol-4-yl | H |
| 9-563 | But-2-in-1-yl | Me | 2-Br-thiazol-4-yl | H |
| 9-564 | But-3-in-2-yl | Me | 2-Br-thiazol-4-yl | H |
| 9-565 | Pr | Me | 2-Br-thiazol-4-yl | H |
| 9-566 | iPr | Me | 2-Br-thiazol-4-yl | H |
| 9-567 | CH₂Ph | Me | 2-Br-thiazol-4-yl | H |
| 9-568 | 3,3-Dichlor-2-fluorprop-2-en-1-yl | Me | 2-Br-thiazol-4-yl | H |
| 9-569 | (1-Methylcyclopropyl)-methyl | Me | 2-Br-thiazol-4-yl | H |
| 9-570 | 4-Chlorbut-2-in-1-yl | Me | 2-Br-thiazol-4-yl | H |
| 9-571 | (2,2-Dichlorcyclopropyl)-methyl | Me | 2-Br-thiazol-4-yl | H |
| 9-572 | 1-Methylprop-2-in-1-yl | Me | 2-Br-thiazol-4-yl | H |
| 9-573 | 1-Cyclopropylethyl | Me | 2-Br-thiazol-4-yl | H |
| 9-574 | Allyl | Me | 2-Br-thiazol-4-yl | H |
| 9-575 | 3-Methylbut-2-en-1-yl | Me | 2-Br-thiazol-4-yl | H |
| 9-576 | Cyclobutylmethyl | Me | 2-Br-thiazol-4-yl | H |
| 9-577 | Cyclopentylmethyl | Me | 2-Br-thiazol-4-yl | H |
| 9-578 | 2-Chloroprop-2-en-1-yl | Me | 2-Br-thiazol-4-yl | H |
| 9-579 | Tetrahydrofuran-2-yl-methyl | Me | 2-Br-thiazol-4-yl | H |
| 9-580 | (3-Methyloxetan-3-yl)-methyl | Me | 2-Br-thiazol-4-yl | H |
| 9-581 | 2,2,2-Trifluorethyl | Me | 2-Br-thiazol-4-yl | H |
| 9-582 | 2,2-Difluorethyl | Me | 2-Br-thiazol-4-yl | H |
| 9-583 | Oxetan-3-yl | Me | 2-Br-thiazol-4-yl | H |
| 9-584 | Cyclopropylmethyl | Me | 5-Br-thiazol-2-yl | H |
| 9-585 | Prop-2-in-1-yl | Me | 5-Br-thiazol-2-yl | H |
| 9-586 | But-2-in-1-yl | Me | 5-Br-thiazol-2-yl | H |
| 9-587 | But-3-in-2-yl | Me | 5-Br-thiazol-2-yl | H |
| 9-588 | Pr | Me | 5-Br-thiazol-2-yl | H |
| 9-589 | iPr | Me | 5-Br-thiazol-2-yl | H |
| 9-590 | CH₂Ph | Me | 5-Br-thiazol-2-yl | H |
| 9-591 | 3,3-Dichlor-2-fluorprop-2-en-1-yl | Me | 5-Br-thiazol-2-yl | H |
| 9-592 | (1-Methylcyclopropyl)-methyl | Me | 5-Br-thiazol-2-yl | H |
| 9-593 | 4-Chlorbut-2-in-1-yl | Me | 5-Br-thiazol-2-yl | H |
| 9-594 | (2,2-Dichlorcyclopropyl)-methyl | Me | 5-Br-thiazol-2-yl | H |
| 9-595 | 1-Methylprop-2-in-1-yl | Me | 5-Br-thiazol-2-yl | H |
| 9-596 | 1-Cyclopropylethyl | Me | 5-Br-thiazol-2-yl | H |
| 9-597 | Allyl | Me | 5-Br-thiazol-2-yl | H |
| 9-598 | 3-Methylbut-2-en-1-yl | Me | 5-Br-thiazol-2-yl | H |
| 9-599 | Cyclobutylmethyl | Me | 5-Br-thiazol-2-yl | H |
| 9-600 | Cyclopentylmethyl | Me | 5-Br-thiazol-2-yl | H |
| 9-601 | 2-Chloroprop-2-en-1-yl | Me | 5-Br-thiazol-2-yl | H |
| 9-602 | Tetrahydrofuran-2-yl-methyl | Me | 5-Br-thiazol-2-yl | H |
| 9-603 | (3-Methyloxetan-3-yl)-methyl | Me | 5-Br-thiazol-2-yl | H |
| 9-604 | 2,2,2-Trifluorethyl | Me | 5-Br-thiazol-2-yl | H |
| 9-605 | 2,2-Difluorethyl | Me | 5-Br-thiazol-2-yl | H |
| 9-606 | Oxetan-3-yl | Me | 5-Br-thiazol-2-yl | H |
| 9-607 | Cyclopropylmethyl | Me | 5-Cl-thiazol-2-yl | H |
| 9-608 | Prop-2-in-1-yl | Me | 5-Cl-thiazol-2-yl | H |
| 9-609 | But-2-in-1-yl | Me | 5-Cl-thiazol-2-yl | H |
| 9-610 | But-3-in-2-yl | Me | 5-Cl-thiazol-2-yl | H |
| 9-611 | Pr | Me | 5-Cl-thiazol-2-yl | H |
| 9-612 | iPr | Me | 5-Cl-thiazol-2-yl | H |
| 9-613 | CH₂Ph | Me | 5-Cl-thiazol-2-yl | H |
| 9-614 | 3,3-Dichlor-2-fluorprop-2-en-1-yl | Me | 5-Cl-thiazol-2-yl | H |
| 9-615 | (1-Methylcyclopropyl)-methyl | Me | 5-Cl-thiazol-2-yl | H |
| 9-616 | 4-Chlorbut-2-in-1-yl | Me | 5-Cl-thiazol-2-yl | H |
| 9-617 | (2,2-Dichlorcyclopropyl)-methyl | Me | 5-Cl-thiazol-2-yl | H |
| 9-618 | 1-Methylprop-2-in-1-yl | Me | 5-Cl-thiazol-2-yl | H |
| 9-619 | 1-Cyclopropylethyl | Me | 5-Cl-thiazol-2-yl | H |
| 9-620 | Allyl | Me | 5-Cl-thiazol-2-yl | H |
| 9-621 | 3-Methylbut-2-en-1-yl | Me | 5-Cl-thiazol-2-yl | H |
| 9-622 | Cyclobutylmethyl | Me | 5-Cl-thiazol-2-yl | H |
| 9-623 | Cyclopentylmethyl | Me | 5-Cl-thiazol-2-yl | H |
| 9-624 | 2-Chloroprop-2-en-1-yl | Me | 5-Cl-thiazol-2-yl | H |
| 9-625 | Tetrahydrofuran-2-yl-methyl | Me | 5-Cl-thiazol-2-yl | H |
| 9-626 | (3-Methyloxetan-3-yl)-methyl | Me | 5-Cl-thiazol-2-yl | H |
| 9-627 | 2,2,2-Trifluorethyl | Me | 5-Cl-thiazol-2-yl | H |
| 9-628 | 2,2-Difluorethyl | Me | 5-Cl-thiazol-2-yl | H |
| 9-629 | Oxetan-3-yl | Me | 5-Cl-thiazol-2-yl | H |
| 9-630 | Cyclopropylmethyl | Me | 5-OSO₂Me-pyridin-2-yl | H |
| 9-631 | Prop-2-in-1-yl | Me | 5-OSO₂Me-pyridin-2-yl | H |
| 9-632 | But-3-in-2-yl | Me | 5-OSO₂Me-pyridin-2-yl | H |
| 9-633 | iPr | Me | 5-OSO₂Me-pyridin-2-yl | H |
| 9-634 | CH₂Ph | Me | 5-OSO₂Me-pyridin-2-yl | H |
| 9-635 | (2,2-Dichlorcyclopropyl)-methyl | Me | 5-OSO₂Me-pyridin-2-yl | H |
| 9-636 | Allyl | Me | 5-OSO₂Me-pyridin-2-yl | H |
| 9-637 | 2,2,2-Trifluorethyl | Me | 5-OSO₂Me-pyridin-2-yl | H |
| 9-638 | 2,2-Difluorethyl | Me | 5-OSO₂Me-pyridin-2-yl | H |
| 9-639 | Oxetan-3-yl | Me | 5-OSO₂Me-pyridin-2-yl | H |
| 9-640 | Cyclopropylmethyl | Me | 6-Cl-1,3-benzothiazol-2-yl | H |
| 9-641 | Prop-2-in-1-yl | Me | 6-Cl-1,3-benzothiazol-2-yl | H |
| 9-642 | But-3-in-2-yl | Me | 6-Cl-1,3-benzothiazol-2-yl | H |
| 9-643 | iPr | Me | 6-Cl-1,3-benzothiazol-2-yl | H |
| 9-644 | CH₂Ph | Me | 6-Cl-1,3-benzothiazol-2-yl | H |
| 9-645 | (2,2-Dichlorcyclopropyl)-methyl | Me | 6-Cl-1,3-benzothiazol-2-yl | H |
| 9-646 | Allyl | Me | 6-Cl-1,3-benzothiazol-2-yl | H |
| 9-647 | 2,2,2-Trifluorethyl | Me | 6-Cl-1,3-benzothiazol-2-yl | H |
| 9-648 | 2,2-Difluorethyl | Me | 6-Cl-1,3-benzothiazol-2-yl | H |
| 9-649 | Oxetan-3-yl | Me | 6-Cl-1,3-benzothiazol-2-yl | H |
| 9-650 | Cyclopropylmethyl | Me | 6-Br-1,3-benzothiazol-2-yl | H |
| 9-651 | Prop-2-in-1-yl | Me | 6-Br-1,3-benzothiazol-2-yl | H |
| 9-652 | But-3-in-2-yl | Me | 6-Br-1,3-benzothiazol-2-yl | H |
| 9-653 | iPr | Me | 6-Br-1,3-benzothiazol-2-yl | H |
| 9-654 | CH₂Ph | Me | 6-Br-1,3-benzothiazol-2-yl | H |
| 9-655 | (2,2-Dichlorcyclopropyl)-methyl | Me | 6-Br-1,3-benzothiazol-2-yl | H |
| 9-656 | Allyl | Me | 6-Br-1,3-benzothiazol-2-yl | H |
| 9-657 | 2,2,2-Trifluorethyl | Me | 6-Br-1,3-benzothiazol-2-yl | H |
| 9-658 | 2,2-Difluorethyl | Me | 6-Br-1,3-benzothiazol-2-yl | H |
| 9-659 | Oxetan-3-yl | Me | 6-Br-1,3-benzothiazol-2-yl | H |
| 9-660 | Cyclopropylmethyl | Me | 1,3-Benzoxazol-2-yl | H |
| 9-661 | Prop-2-in-1-yl | Me | 1,3-Benzoxazol-2-yl | H |
| 9-662 | But-3-in-2-yl | Me | 1,3-Benzoxazol-2-yl | H |
| 9-663 | iPr | Me | 1,3-Benzoxazol-2-yl | H |
| 9-664 | CH₂Ph | Me | 1,3-Benzoxazol-2-yl | H |
| 9-665 | (2,2-Dichlorcyclopropyl)-methyl | Me | 1,3-Benzoxazol-2-yl | H |
| 9-666 | Allyl | Me | 1,3-Benzoxazol-2-yl | H |
| 9-667 | 2,2,2-Trifluorethyl | Me | 1,3-Benzoxazol-2-yl | H |
| 9-668 | 2,2-Difluorethyl | Me | 1,3-Benzoxazol-2-yl | H |
| 9-669 | Oxetan-3-yl | Me | 1,3-Benzoxazol-2-yl | H |
| 9-670 | Cyclopropylmethyl | Me | 6-Cl-1,3-benzoxazol-2-yl | H |
| 9-671 | Prop-2-in-1-yl | Me | 6-Cl-1,3-benzoxazol-2-yl | H |
| 9-672 | But-3-in-2-yl | Me | 6-Cl-1,3-benzoxazol-2-yl | H |
| 9-673 | iPr | Me | 6-Cl-1,3-benzoxazol-2-yl | H |
| 9-674 | CH₂Ph | Me | 6-Cl-1,3-benzoxazol-2-yl | H |
| 9-675 | (2,2-Dichlorcyclopropyl)-methyl | Me | 6-Cl-1,3-benzoxazol-2-yl | H |
| 9-676 | Allyl | Me | 6-Cl-1,3-benzoxazol-2-yl | H |
| 9-677 | 2,2,2-Trifluorethyl | Me | 6-Cl-1,3-benzoxazol-2-yl | H |
| 9-678 | 2,2-Difluorethyl | Me | 6-Cl-1,3-benzoxazol-2-yl | H |
| 9-679 | Oxetan-3-yl | Me | 6-Cl-1,3-benzoxazol-2-yl | H |
| 9-680 | Cyclopropylmethyl | Me | 6-Br-1,3-benzoxazol-2-yl | H |
| 9-681 | Prop-2-in-1-yl | Me | 6-Br-1,3-benzoxazol-2-yl | H |
| 9-682 | But-3-in-2-yl | Me | 6-Br-1,3-benzoxazol-2-yl | H |
| 9-683 | iPr | Me | 6-Br-1,3-benzoxazol-2-yl | H |
| 9-684 | CH₂Ph | Me | 6-Br-1,3-benzoxazol-2-yl | H |
| 9-685 | (2,2-Dichlorcyclopropyl)-methyl | Me | 6-Br-1,3-benzoxazol-2-yl | H |
| 9-686 | Allyl | Me | 6-Br-1,3-benzoxazol-2-yl | H |
| 9-687 | 2,2,2-Trifluorethyl | Me | 6-Br-1,3-benzoxazol-2-yl | H |
| 9-688 | 2,2-Difluorethyl | Me | 6-Br-1,3-benzoxazol-2-yl | H |
| 9-689 | Oxetan-3-yl | Me | 6-Br-1,3-benzoxazol-2-yl | H |
| 9-690 | Cyclopropylmethyl | Me | 7-Cl-1,3-benzoxazol-2-yl | H |
| 9-691 | Prop-2-in-1-yl | Me | 7-Cl-1,3-benzoxazol-2-yl | H |
| 9-692 | But-3-in-2-yl | Me | 7-Cl-1,3-benzoxazol-2-yl | H |
| 9-693 | iPr | Me | 7-Cl-1,3-benzoxazol-2-yl | H |
| 9-694 | CH₂Ph | Me | 7-Cl-1,3-benzoxazol-2-yl | H |
| 9-695 | (2,2-Dichlorcyclopropyl)-methyl | Me | 7-Cl-1,3-benzoxazol-2-yl | H |
| 9-696 | Allyl | Me | 7-Cl-1,3-benzoxazol-2-yl | H |
| 9-697 | 2,2,2-Trifluorethyl | Me | 7-Cl-1,3-benzoxazol-2-yl | H |
| 9-698 | 2,2-Difluorethyl | Me | 7-Cl-1,3-benzoxazol-2-yl | H |
| 9-699 | Oxetan-3-yl | Me | 7-Cl-1,3-benzoxazol-2-yl | H |
| 9-700 | Cyclopropylmethyl | Me | 5-I-pyridin-2-yl | H |
| 9-701 | Prop-2-in-1-yl | Me | 5-I-pyridin-2-yl | H |
| 9-702 | But-3-in-2-yl | Me | 5-I-pyridin-2-yl | H |
| 9-703 | iPr | Me | 5-I-pyridin-2-yl | H |
| 9-704 | CH₂Ph | Me | 5-I-pyridin-2-yl | H |
| 9-705 | (2,2-Dichlorcyclopropyl)-methyl | Me | 5-I-pyridin-2-yl | H |
| 9-706 | Allyl | Me | 5-I-pyridin-2-yl | H |
| 9-707 | 2,2,2-Trifluorethyl | Me | 5-I-pyridin-2-yl | H |
| 9-708 | 2,2-Difluorethyl | Me | 5-I-pyridin-2-yl | H |
| 9-709 | Oxetan-3-yl | Me | 5-I-pyridin-2-yl | H |
| 9-710 | Cyclopropylmethyl | Me | 5-NH₂-pyridin-2-yl | H |
| 9-711 | Prop-2-in-1-yl | Me | 5-NH₂-pyridin-2-yl | H |
| 9-712 | But-3-in-2-yl | Me | 5-NH₂-pyridin-2-yl | H |
| 9-713 | iPr | Me | 5-NH₂-pyridin-2-yl | H |
| 9-714 | CH₂Ph | Me | 5-NH₂-pyridin-2-yl | H |
| 9-715 | (2,2-Dichlorcyclopropyl)-methyl | Me | 5-NH₂-pyridin-2-yl | H |
| 9-716 | Allyl | Me | 5-NH₂-pyridin-2-yl | H |
| 9-717 | 2,2,2-Trifluorethyl | Me | 5-NH₂-pyridin-2-yl | H |
| 9-718 | 2,2-Difluorethyl | Me | 5-NH₂-pyridin-2-yl | H |
| 9-719 | Oxetan-3-yl | Me | 5-NH₂-pyridin-2-yl | H |
| 9-720 | Cyclopropylmethyl | Me | 5-OH-pyridin-2-yl | H |
| 9-721 | Prop-2-in-1-yl | Me | 5-OH-pyridin-2-yl | H |
| 9-722 | But-3-in-2-yl | Me | 5-OH-pyridin-2-yl | H |
| 9-723 | iPr | Me | 5-OH-pyridin-2-yl | H |
| 9-724 | CH₂Ph | Me | 5-OH-pyridin-2-yl | H |
| 9-725 | (2,2-Dichlorcyclopropyl)-methyl | Me | 5-OH-pyridin-2-yl | H |
| 9-726 | Allyl | Me | 5-OH-pyridin-2-yl | H |
| 9-727 | 2,2,2-Trifluorethyl | Me | 5-OH-pyridin-2-yl | H |
| 9-728 | 2,2-Difluorethyl | Me | 5-OH-pyridin-2-yl | H |
| 9-729 | Oxetan-3-yl | Me | 5-OH-pyridin-2-yl | H |
| 9-730 | Cyclopropylmethyl | Me | 5-OCHF₂-pyridin-2-yl | H |
| 9-731 | Prop-2-in-1-yl | Me | 5-OCHF₂-pyridin-2-yl | H |
| 9-732 | But-3-in-2-yl | Me | 5-OCHF₂-pyridin-2-yl | H |
| 9-733 | iPr | Me | 5-OCHF₂-pyridin-2-yl | H |
| 9-734 | CH₂Ph | Me | 5-OCHF₂-pyridin-2-yl | H |
| 9-735 | (2,2-Dichlorcyclopropyl)-methyl | Me | 5-OCHF₂-pyridin-2-yl | H |
| 9-736 | Allyl | Me | 5-OCHF₂-pyridin-2-yl | H |
| 9-737 | 2,2,2-Trifluorethyl | Me | 5-OCHF₂-pyridin-2-yl | H |
| 9-738 | 2,2-Difluorethyl | Me | 5-OCHF₂-pyridin-2-yl | H |
| 9-739 | Oxetan-3-yl | Me | 5-OCHF₂-pyridin-2-yl | H |
| 9-740 | Cyclopropylmethyl | Me | 5-MeO-pyridin-2-yl | H |
| 9-741 | Prop-2-in-1-yl | Me | 5-MeO-pyridin-2-yl | H |
| 9-742 | But-3-in-2-yl | Me | 5-MeO-pyridin-2-yl | H |
| 9-743 | iPr | Me | 5-MeO-pyridin-2-yl | H |
| 9-744 | CH₂Ph | Me | 5-MeO-pyridin-2-yl | H |
| 9-745 | (2,2-Dichlorcyclopropyl)-methyl | Me | 5-MeO-pyridin-2-yl | H |
| 9-746 | Allyl | Me | 5-MeO-pyridin-2-yl | H |
| 9-747 | 2,2,2-Trifluorethyl | Me | 5-MeO-pyridin-2-yl | H |
| 9-748 | 2,2-Difluorethyl | Me | 5-MeO-pyridin-2-yl | H |
| 9-749 | Oxetan-3-yl | Me | 5-MeO-pyridin-2-yl | H |
| 9-750 | Cyclopropylmethyl | Me | 5-MeS-pyridin-2-yl | H |
| 9-751 | Prop-2-in-1-yl | Me | 5-MeS-pyridin-2-yl | H |
| 9-752 | But-3-in-2-yl | Me | 5-MeS-pyridin-2-yl | H |
| 9-753 | iPr | Me | 5-MeS-pyridin-2-yl | H |
| 9-754 | CH₂Ph | Me | 5-MeS-pyridin-2-yl | H |
| 9-755 | (2,2-Dichlorcyclopropyl)-methyl | Me | 5-MeS-pyridin-2-yl | H |
| 9-756 | Allyl | Me | 5-MeS-pyridin-2-yl | H |
| 9-757 | 2,2,2-Trifluorethyl | Me | 5-MeS-pyridin-2-yl | H |
| 9-758 | 2,2-Difluorethyl | Me | 5-MeS-pyridin-2-yl | H |
| 9-759 | Oxetan-3-yl | Me | 5-MeS-pyridin-2-yl | H |
| 9-760 | Cyclopropylmethyl | Me | 5-NHMe-pyridin-2-yl | H |
| 9-761 | Prop-2-in-1-yl | Me | 5-NHMe-pyridin-2-yl | H |
| 9-762 | But-3-in-2-yl | Me | 5-NHMe-pyridin-2-yl | H |
| 9-763 | iPr | Me | 5-NHMe-pyridin-2-yl | H |
| 9-764 | CH₂Ph | Me | 5-NHMe-pyridin-2-yl | H |
| 9-765 | (2,2-Dichlorcyclopropyl)-methyl | Me | 5-NHMe-pyridin-2-yl | H |
| 9-766 | Allyl | Me | 5-NHMe-pyridin-2-yl | H |
| 9-767 | 2,2,2-Trifluorethyl | Me | 5-NHMe-pyridin-2-yl | H |
| 9-768 | 2,2-Difluorethyl | Me | 5-NHMe-pyridin-2-yl | H |
| 9-769 | Oxetan-3-yl | Me | 5-NHMe-pyridin-2-yl | H |
| 9-770 | Cyclopropylmethyl | Me | 5-NMe₂-pyridin-2-yl | H |
| 9-771 | Prop-2-in-1-yl | Me | 5-NMe₂-pyridin-2-yl | H |
| 9-772 | But-3-in-2-yl | Me | 5-NMe₂-pyridin-2-yl | H |
| 9-773 | iPr | Me | 5-NMe₂-pyridin-2-yl | H |
| 9-774 | CH₂Ph | Me | 5-NMe₂-pyridin-2-yl | H |
| 9-775 | (2,2-Dichlorcyclopropyl)-methyl | Me | 5-NMe₂-pyridin-2-yl | H |
| 9-776 | Allyl | Me | 5-NMe₂-pyridin-2-yl | H |
| 9-777 | 2,2,2-Trifluorethyl | Me | 5-NMe₂-pyridin-2-yl | H |
| 9-778 | 2,2-Difluorethyl | Me | 5-NMe₂-pyridin-2-yl | H |
| 9-779 | Oxetan-3-yl | Me | 5-NMe₂-pyridin-2-yl | H |
| 9-780 | 3-Hydroxy-but-2-yl | Me | 4-Cl-Ph | H |
| 9-781 | 3-Hydroxy-but-2-yl | Me | 4-Br-Ph | H |
| 9-782 | 3-Hydroxy-but-2-yl | Me | 5-Cl-pyridin-2-yl | H |
| 9-783 | 3-Hydroxy-but-2-yl | Me | 5-Br-pyridin-2-yl | H |
| 9-784 | 3-Hydroxy-but-2-yl | Me | 5-Cl-2-thienyl | H |
| 9-785 | 3-Hydroxy-but-2-yl | Me | 5-Br-2-thienyl | H |
| 9-786 | 3-Ethylpent-1-in-3-yl | Me | 4-Cl-Ph | H |
| 9-787 | 3-Ethylpent-1-in-3-yl | Me | 4-Br-Ph | H |
| 9-788 | 3-Ethylpent-1-in-3-yl | Me | 5-Cl-pyridin-2-yl | H |
| 9-789 | 3-Ethylpent-1-in-3-yl | Me | 5-Br-pyridin-2-yl | H |
| 9-790 | 3-Ethylpent-1-in-3-yl | Me | 5-Cl-2-thienyl | H |
| 9-791 | 3-Ethylpent-1-in-3-yl | Me | 5-Br-2-thienyl | H |
| 9-792 | Difluormethyl | Me | 4-Cl-Ph | H |
| 9-793 | Difluormethyl | Me | 4-Br-Ph | H |
| 9-794 | Difluormethyl | Me | 5-Cl-pyridin-2-yl | H |
| 9-795 | Difluormethyl | Me | 5-Br-pyridin-2-yl | H |
| 9-796 | Difluormethyl | Me | 5-Cl-2-thienyl | H |
| 9-797 | Difluormethyl | Me | 5-Br-2-thienyl | H |
| 9-798 | 2,2,3,3-Tetrafluorpropyl | Me | 4-Cl-Ph | H |
| 9-799 | 2,2,3,3-Tetrafluorpropyl | Me | 4-Br-Ph | H |
| 9-800 | 2,2,3,3-Tetrafluorpropyl | Me | 5-Cl-pyridin-2-yl | H |
| 9-801 | 2,2,3,3-Tetrafluorpropyl | Me | 5-Br-pyridin-2-yl | H |
| 9-802 | 2,2,3,3-Tetrafluorpropyl | Me | 5-Cl-2-thienyl | H |
| 9-803 | 2,2,3,3-Tetrafluorpropyl | Me | 5-Br-2-thienyl | H |
| 9-804 | 4,4,4-Trifluorbutyl | Me | 4-Cl-Ph | H |
| 9-805 | 4,4,4-Trifluorbutyl | Me | 4-Br-Ph | H |
| 9-806 | 4,4,4-Trifluorbutyl | Me | 5-Cl-pyridin-2-yl | H |
| 9-807 | 4,4,4-Trifluorbutyl | Me | 5-Br-pyridin-2-yl | H |
| 9-808 | 4,4,4-Trifluorbutyl | Me | 5-Cl-2-thienyl | H |
| 9-809 | 4,4,4-Trifluorbutyl | Me | 5-Br-2-thienyl | H |
| 9-810 | Acetoxy-methyl | Me | 4-Cl-Ph | H |
| 9-811 | Acetoxy-methyl | Me | 4-Br-Ph | H |
| 9-812 | Acetoxy-methyl | Me | 5-Cl-pyridin-2-yl | H |
| 9-813 | Acetoxy-methyl | Me | 5-Br-pyridin-2-yl | H |
| 9-814 | Acetoxy-methyl | Me | 5-Cl-2-thienyl | H |
| 9-815 | Acetoxy-methyl | Me | 5-Br-2-thienyl | H |
| 9-816 | 2-Chlorethyl | Me | 4-Cl-Ph | H |
| 9-817 | 2-Chlorethyl | Me | 4-Br-Ph | H |
| 9-818 | 2-Chlorethyl | Me | 5-Cl-pyridin-2-yl | H |
| 9-819 | 2-Chlorethyl | Me | 5-Br-pyridin-2-yl | H |
| 9-820 | 2-Chlorethyl | Me | 5-Cl-2-thienyl | H |
| 9-821 | 2-Chlorethyl | Me | 5-Br-2-thienyl | H |
| 9-822 | 3-Fluorpropyl | Me | 4-Cl-Ph | H |
| 9-823 | 3-Fluorpropyl | Me | 4-Br-Ph | H |
| 9-824 | 3-Fluorpropyl | Me | 5-Cl-pyridin-2-yl | H |
| 9-825 | 3-Fluorpropyl | Me | 5-Br-pyridin-2-yl | H |
| 9-826 | 3-Fluorpropyl | Me | 5-Cl-2-thienyl | H |
| 9-827 | 3-Fluorpropyl | Me | 5-Br-2-thienyl | H |
| 9-828 | 2-Ethoxyethyl | Me | 4-Cl-Ph | H |
| 9-829 | 2-Ethoxyethyl | Me | 4-Br-Ph | H |
| 9-830 | 2-Ethoxyethyl | Me | 5-Cl-pyridin-2-yl | H |
| 9-831 | 2-Ethoxyethyl | Me | 5-Br-pyridin-2-yl | H |
| 9-832 | 2-methoxyethyl | Me | 5-Cl-2-thienyl | H |
| 9-833 | 2-Ethoxyethyl | Me | 5-Br-2-thienyl | H |
| 9-834 | 2-Propan-1-ol | Me | 4-Cl-Ph | H |
| 9-835 | 2-Propan-1-ol | Me | 4-Br-Ph | H |
| 9-836 | 1-Hydroxy-prop-2-yl | Me | 5-Cl-pyridin-2-yl | H |
| 9-837 | 1-Hydroxy-prop-2-yl | Me | 5-Br-pyridin-2-yl | H |
| 9-838 | 1-Hydroxy-prop-2-yl | Me | 5-Cl-2-thienyl | H |
| 9-839 | 1-Hydroxy-prop-2-yl | Me | 5-Br-2-thienyl | H |
| 9-840 | 2-Methoxybut-1-yl | Me | 4-Cl-Ph | H |
| 9-841 | 2-Methoxybut-1-yl | Me | 4-Br-Ph | H |
| 9-842 | 2-Methoxybut-1-yl | Me | 5-Cl-pyridin-2-yl | H |
| 9-843 | 2-Methoxybut-1-yl | Me | 5-Br-pyridin-2-yl | H |
| 9-844 | 2-Methoxybut-1-yl | Me | 5-Cl-2-thienyl | H |
| 9-845 | 2-Methoxybut-1-yl | Me | 5-Br-2-thienyl | H |
| 9-846 | 1,3-Difluorpropan-2-yl | Me | 4-Cl-Ph | H |
| 9-847 | 1,3-Difluorpropan-2-yl | Me | 4-Br-Ph | H |
| 9-848 | 1,3-Difluorpropan-2-yl | Me | 5-Cl-pyridin-2-yl | H |
| 9-849 | 1,3-Difluorpropan-2-yl | Me | 5-Br-pyridin-2-yl | H |
| 9-850 | 1,3-Difluorpropan-2-yl | Me | 5-Cl-2-thienyl | H |
| 9-851 | 1,3-Difluorpropan-2-yl | Me | 5-Br-2-thienyl | H |
| 9-852 | 2,3-Dimethoxypropyl | Me | 4-Cl-Ph | H |
| 9-853 | 2,3-Dimethoxypropyl | Me | 4-Br-Ph | H |
| 9-854 | 2,3-Dimethoxypropyl | Me | 5-Cl-pyridin-2-yl | H |
| 9-855 | 2,3-Dimethoxypropyl | Me | 5-Br-pyridin-2-yl | H |
| 9-856 | 2,3-Dimethoxypropyl | Me | 5-Cl-2-thienyl | H |
| 9-857 | 2,3-Dimethoxypropyl | Me | 5-Br-2-thienyl | H |
| 9-858 | 1,3-dioxolan-4-yl-methyl | Me | 4-Cl-Ph | H |
| 9-859 | 1,3-dioxolan-4-yl-methyl | Me | 4-Br-Ph | H |
| 9-860 | 1,3-dioxolan-4-yl-methyl | Me | 5-Cl-pyridin-2-yl | H |
| 9-861 | 1,3-dioxolan-4-yl-methyl | Me | 5-Br-pyridin-2-yl | H |
| 9-862 | 1,3-dioxolan-4-yl-methyl | Me | 5-Cl-2-thienyl | H |
| 9-863 | 1,3-dioxolan-4-yl-methyl | Me | 5-Br-2-thienyl | H |
| 9-864 | 1,1,1,4,4,4-Hexafluorbutan-2-yl | Me | 4-Cl-Ph | H |
| 9-865 | 1,1,1,4,4,4-Hexafluorbutan-2-yl | Me | 4-Br-Ph | H |
| 9-866 | 1,1,1,4,4,4-Hexafluorbutan-2-yl | Me | 5-Cl-pyridin-2-yl | H |
| 9-867 | 1,1,1,4,4,4-Hexafluorbutan-2-yl | Me | 5-Br-pyridin-2-yl | H |
| 9-868 | 1,1,1,4,4,4-Hexafluorbutan-2-yl | Me | 5-Cl-2-thienyl | H |
| 9-869 | 1,1,1,4,4,4-Hexafluorbutan-2-yl | Me | 5-Br-2-thienyl | H |
| 9-870 | 1,1-Difluorpropan-2-yl | Me | 4-Cl-Ph | H |
| 9-871 | 1,1-Difluorpropan-2-yl | Me | 4-Br-Ph | H |
| 9-872 | 1,1-Difluorpropan-2-yl | Me | 5-Cl-pyridin-2-yl | H |
| 9-873 | 1,1-Difluorpropan-2-yl | Me | 5-Br-pyridin-2-yl | H |
| 9-874 | 1,1-Difluorpropan-2-yl | Me | 5-Cl-2-thienyl | H |
| 9-875 | 1,1-Difluorpropan-2-yl | Me | 5-Br-2-thienyl | H |
| 9-876 | 1-Fluorpropan-2-yl | Me | 4-Cl-Ph | H |
| 9-877 | 1-Fluorpropan-2-yl | Me | 4-Br-Ph | H |
| 9-878 | 1-Fluorpropan-2-yl | Me | 5-Cl-pyridin-2-yl | H |
| 9-879 | 1-Fluorpropan-2-yl | Me | 5-Br-pyridin-2-yl | H |
| 9-880 | 1-Fluorpropan-2-yl | Me | 5-Cl-2-thienyl | H |
| 9-881 | 1-Fluorpropan-2-yl | Me | 5-Br-2-thienyl | H |
| 9-882 | 1-Brompropan-2-yl | Me | 4-Cl-Ph | H |
| 9-883 | 1-Brompropan-2-yl | Me | 4-Br-Ph | H |
| 9-884 | 1-Brompropan-2-yl | Me | 5-Cl-pyridin-2-yl | H |
| 9-885 | 1-Brompropan-2-yl | Me | 5-Br-pyridin-2-yl | H |
| 9-886 | 1-Brompropan-2-yl | Me | 5-Cl-2-thienyl | H |
| 9-887 | 1-Brompropan-2-yl | Me | 5-Br-2-thienyl | H |
| 9-888 | 1-Chlorpropan-2-yl | Me | 4-Cl-Ph | H |
| 9-889 | 1-Chlorpropan-2-yl | Me | 4-Br-Ph | H |
| 9-890 | 1-Chlorpropan-2-yl | Me | 5-Cl-pyridin-2-yl | H |
| 9-891 | 1-Chlorpropan-2-yl | Me | 5-Br-pyridin-2-yl | H |
| 9-892 | 1-Chlorpropan-2-yl | Me | 5-Cl-2-thienyl | H |
| 9-893 | 1-Chlorpropan-2-yl | Me | 5-Br-2-thienyl | H |
| 9-894 | 2-Isopropoxyethyl | Me | 4-Cl-Ph | H |
| 9-895 | 2-Isopropoxyethyl | Me | 4-Br-Ph | H |
| 9-896 | 2-Isopropoxyethyl | Me | 5-Cl-pyridin-2-yl | H |
| 9-897 | 2-Isopropoxyethyl | Me | 5-Br-pyridin-2-yl | H |
| 9-898 | 2-Isopropoxyethyl | Me | 5-Cl-2-thienyl | H |
| 9-899 | 2-Isopropoxyethyl | Me | 5-Br-2-thienyl | H |
| 9-900 | Tetrahydrofuran-3-yl | Me | 4-Cl-Ph | H |
| 9-901 | Tetrahydrofuran-3-yl | Me | 4-Br-Ph | H |
| 9-902 | Tetrahydrofuran-3-yl | Me | 5-Cl-pyridin-2-yl | H |
| 9-903 | Tetrahydrofuran-3-yl | Me | 5-Br-pyridin-2-yl | H |
| 9-904 | Tetrahydrofuran-3-yl | Me | 5-Cl-2-thienyl | H |
| 9-905 | Tetrahydrofuran-3-yl | Me | 5-Br-2-thienyl | H |
| 9-906 | 2-(2,2,2-Trifluorethoxy)ethyl | Me | 4-Cl-Ph | H |
| 9-907 | 2-(2,2,2-Trifluorethoxy)ethyl | Me | 4-Br-Ph | H |
| 9-908 | 2-(2,2,2-Trifluorethoxy)ethyl | Me | 5-Cl-pyridin-2-yl | H |
| 9-909 | 2-(2,2,2-Trifluorethoxy)ethyl | Me | 5-Br-pyridin-2-yl | H |
| 9-910 | 2-(2,2,2-Trifluorethoxy)ethyl | Me | 5-Cl-2-thienyl | H |
| 9-911 | 2-(2,2,2-Trifluorethoxy)ethyl | Me | 5-Br-2-thienyl | H |
| 9-912 | (3,3,4,4,4-Pentafluorbutan-2-yl | Me | 4-Cl-Ph | H |
| 9-913 | (3,3,4,4,4-Pentafluorbutan-2-yl | Me | 4-Br-Ph | H |
| 9-914 | (3,3,4,4,4-Pentafluorbutan-2-yl | Me | 5-Cl-pyridin-2-yl | H |
| 9-915 | (3,3,4,4,4-Pentafluorbutan-2-yl | Me | 5-Br-pyridin-2-yl | H |
| 9-916 | (3,3,4,4,4-Pentafluorbutan-2-yl | Me | 5-Cl-2-thienyl | H |
| 9-917 | (3,3,4,4,4-Pentafluorbutan-2-yl | Me | 5-Br-2-thienyl | H |
| 9-918 | 1-(N,N-Dimethylaminocarbonyl)-eth-1-yl | Me | 4-Cl-Ph | H |
| 9-919 | 1-(N,N-Dimethylaminocarbonyl)-eth-1-yl | Me | 4-Br-Ph | H |
| 9-920 | 1-(N,N-Dimethylaminocarbonyl)-eth-1-yl | Me | 5-Cl-pyridin-2-yl | H |
| 9-921 | 1-(N,N-Dimethylaminocarbonyl)-eth-1-yl | Me | 5-Br-pyridin-2-yl | H |
| 9-922 | 1-(N,N-Dimethylaminocarbonyl)-eth-1-yl | Me | 5-Cl-2-thienyl | H |
| 9-923 | 1-(N,N-Dimethylaminocarbonyl)-eth-1-yl | Me | 5-Br-2-thienyl | H |
| 9-924 | (1,3-dioxan-2-yl)-methyl | Me | 4-Cl-Ph | H |
| 9-925 | (1,3-dioxan-2-yl)-methyl | Me | 4-Br-Ph | H |
| 9-926 | (1,3-dioxan-2-yl)-methyl | Me | 5-Cl-pyridin-2-yl | H |
| 9-927 | (1,3-dioxan-2-yl)-methyl | Me | 5-Br-pyridin-2-yl | H |
| 9-928 | (1,3-dioxan-2-yl)-methyl | Me | 5-Cl-2-thienyl | H |
| 9-929 | (1,3-dioxan-2-yl)-methyl | Me | 5-Br-2-thienyl | H |
| 9-930 | 1,1,1-Trifluorbutan-2-yl | Me | 4-Cl-Ph | H |
| 9-931 | 1,1,1-Trifluorbutan-2-yl | Me | 4-Br-Ph | H |
| 9-932 | 1,1,1-Trifluorbutan-2-yl | Me | 5-Cl-pyridin-2-yl | H |
| 9-933 | 1,1,1-Trifluorbutan-2-yl | Me | 5-Br-pyridin-2-yl | H |
| 9-934 | 1,1,1-Trifluorbutan-2-yl | Me | 5-Cl-2-thienyl | H |
| 9-935 | 1,1,1-Trifluorbutan-2-yl | Me | 5-Br-2-thienyl | H |
| 9-936 | 2-(But-2-yliden-aminooxy)-eth-1-yl | Me | 4-Cl-Ph | H |
| 9-937 | 2-(But-2-yliden-aminooxy)-eth-1-yl | Me | 4-Br-Ph | H |
| 9-938 | 2-(But-2-yliden-aminooxy)-eth-1-yl | Me | 5-Cl-pyridin-2-yl | H |
| 9-939 | 2-(But-2-yliden-aminooxy)-eth-1-yl | Me | 5-Br-pyridin-2-yl | H |
| 9-940 | 2-(But-2-yliden-aminooxy)-eth-1-yl | Me | 5-Cl-2-thienyl | H |
| 9-941 | 2-(But-2-yliden-aminooxy)-eth-1-yl | Me | 5-Br-2-thienyl | H |
| 9-942 | Oxetan-2-yl-methyl | Me | 4-Cl-Ph | H |
| 9-943 | Oxetan-2-yl-methyl | Me | 4-Br-Ph | H |
| 9-944 | Oxetan-2-yl-methyl | Me | 5-Cl-pyridin-2-yl | H |
| 9-945 | Oxetan-2-yl-methyl | Me | 5-Br-pyridin-2-yl | H |
| 9-946 | Oxetan-2-yl-methyl | Me | 5-Cl-2-thienyl | H |
| 9-947 | Oxetan-2-yl-methyl | Me | 5-Br-2-thienyl | H |
| 9-948 | 2,2-Dimethoxyethyl | Me | 4-Cl-Ph | H |
| 9-949 | 2,2-Dimethoxyethyl | Me | 4-Br-Ph | H |
| 9-950 | 2,2-Dimethoxyethyl | Me | 5-Cl-pyridin-2-yl | H |
| 9-951 | 2,2-Dimethoxyethyl | Me | 5-Br-pyridin-2-yl | H |
| 9-952 | 2,2-Dimethoxyethyl | Me | 5-Cl-2-thienyl | H |
| 9-953 | 2,2-Dimethoxyethyl | Me | 5-Br-2-thienyl | H |
| 9-954 | 1-Chlorpropyl | Me | 4-Cl-Ph | H |
| 9-955 | 1-Chlorpropyl | Me | 4-Br-Ph | H |
| 9-956 | 1-Chlorpropyl | Me | 5-Cl-pyridin-2-yl | H |
| 9-957 | 1-Chlorpropyl | Me | 5-Br-pyridin-2-yl | H |
| 9-958 | 1-Chlorpropyl | Me | 5-Cl-2-thienyl | H |
| 9-959 | 1-Chlorpropyl | Me | 5-Br-2-thienyl | H |
| 9-960 | 4-Chlorbutan-2-yl | Me | 4-Cl-Ph | H |
| 9-961 | 4-Chlorbutan-2-yl | Me | 4-Br-Ph | H |
| 9-962 | 4-Chlorbutan-2-yl | Me | 5-Cl-pyridin-2-yl | H |
| 9-963 | 4-Chlorbutan-2-yl | Me | 5-Br-pyridin-2-yl | H |
| 9-964 | 4-Chlorbutan-2-yl | Me | 5-Cl-2-thienyl | H |
| 9-965 | 4-Chlorbutan-2-yl | Me | 5-Br-2-thienyl | H |
| 9-966 | 3-Chlorpropan-2-yl | Me | 4-Cl-Ph | H |
| 9-967 | 3-Chlorpropan-2-yl | Me | 4-Br-Ph | H |
| 9-968 | 3-Chlorpropan-2-yl | Me | 5-Cl-pyridin-2-yl | H |
| 9-969 | 3-Chlorpropan-2-yl | Me | 5-Br-pyridin-2-yl | H |
| 9-970 | 3-Chlorpropan-2-yl | Me | 5-Cl-2-thienyl | H |
| 9-971 | 3-Chlorpropan-2-yl | Me | 5-Br-2-thienyl | H |
| 9-972 | 2-(2-Chlorethoxy)-ethyl | Me | 4-Cl-Ph | H |
| 9-973 | 2-(2-Chlorethoxy)-ethyl | Me | 4-Br-Ph | H |
| 9-974 | 2-(2-Chlorethoxy)-ethyl | Me | 5-Cl-pyridin-2-yl | H |
| 9-975 | 2-(2-Chlorethoxy)-ethyl | Me | 5-Br-pyridin-2-yl | H |
| 9-976 | 2-(2-Chlorethoxy)-ethyl | Me | 5-Cl-2-thienyl | H |
| 9-977 | 2-(2-Chlorethoxy)-ethyl | Me | 5-Br-2-thienyl | H |
| 9-978 | 2,2-Dichlorethyl | Me | 4-Cl-Ph | H |
| 9-979 | 2,2-Dichlorethyl | Me | 4-Br-Ph | H |
| 9-980 | 2,2-Dichlorethyl | Me | 5-Cl-pyridin-2-yl | H |
| 9-981 | 2,2-Dichlorethyl | Me | 5-Br-pyridin-2-yl | H |
| 9-982 | 2,2-Dichlorethyl | Me | 5-Cl-2-thienyl | H |
| 9-983 | 2,2-Dichlorethyl | Me | 5-Br-2-thienyl | H |
| 9-984 | 2,3-Dichlorpropyl | Me | 4-Cl-Ph | H |
| 9-985 | 2,3-Dichlorpropyl | Me | 4-Br-Ph | H |
| 9-986 | 2,3-Dichlorpropyl | Me | 5-Cl-pyridin-2-yl | H |
| 9-987 | 2,3-Dichlorpropyl | Me | 5-Br-pyridin-2-yl | H |
| 9-988 | 2,3-Dichlorpropyl | Me | 5-Cl-2-thienyl | H |
| 9-989 | 2,3-Dichlorpropyl | Me | 5-Br-2-thienyl | H |
| 9-990 | 1,3-Dichlorprop-2-yl | Me | 4-Cl-Ph | H |
| 9-991 | 1,3-Dichlorprop-2-yl | Me | 4-Br-Ph | H |
| 9-992 | 1,3-Dichlorprop-2-yl | Me | 5-Cl-pyridin-2-yl | H |
| 9-993 | 1,3-Dichlorprop-2-yl | Me | 5-Br-pyridin-2-yl | H |
| 9-994 | 1,3-Dichlorprop-2-yl | Me | 5-Cl-2-thienyl | H |
| 9-995 | 1,3-Dichlorprop-2-yl | Me | 5-Br-2-thienyl | H |
| 9-996 | 2-Chlor-2,2-difluorethyl | Me | 4-Cl-Ph | H |
| 9-997 | 2-Chlor-2,2-difluorethyl | Me | 4-Br-Ph | H |
| 9-998 | 2-Chlor-2,2-difluorethyl | Me | 5-Cl-pyridin-2-yl | H |
| 9-999 | 2-Chlor-2,2-difluorethyl | Me | 5-Br-pyridin-2-yl | H |
| 9-1000 | 2-Chlor-2,2-difluorethyl | Me | 5-Cl-2-thienyl | H |
| 9-1001 | 2-Chlor-2,2-difluorethyl | Me | 5-Br-2-thienyl | H |
| 9-1002 | 1-Chlor-2-methylpropan-2-yl | Me | 4-Cl-Ph | H |
| 9-1003 | 1-Chlor-2-methylpropan-2-yl | Me | 4-Br-Ph | H |
| 9-1004 | 1-Chlor-2-methylpropan-2-yl | Me | 5-Cl-pyridin-2-yl | H |
| 9-1005 | 1-Chlor-2-methylpropan-2-yl | Me | 5-Br-pyridin-2-yl | H |
| 9-1006 | 1-Chlor-2-methylpropan-2-yl | Me | 5-Cl-2-thienyl | H |
| 9-1007 | 1-Chlor-2-methylpropan-2-yl | Me | 5-Br-2-thienyl | H |
| 9-1008 | 1-Fluor-3-methoxypropan-2-yl | Me | 4-Cl-Ph | H |
| 9-1009 | 1-Fluor-3-methoxypropan-2-yl | Me | 4-Br-Ph | H |
| 9-1010 | 1-Fluor-3-methoxypropan-2-yl | Me | 5-Cl-pyridin-2-yl | H |
| 9-1011 | 1-Fluor-3-methoxypropan-2-yl | Me | 5-Br-pyridin-2-yl | H |
| 9-1012 | 1-Fluor-3-methoxypropan-2-yl | Me | 5-Cl-2-thienyl | H |
| 9-1013 | 1-Fluor-3-methoxypropan-2-yl | Me | 5-Br-2-thienyl | H |
| 9-1014 | 3,3,3-Trifluorpropyl | Me | 4-Cl-Ph | H |
| 9-1015 | 3,3,3-Trifluorpropyl | Me | 4-Br-Ph | H |
| 9-1016 | 3,3,3-Trifluorpropyl | Me | 5-Cl-pyridin-2-yl | H |
| 9-1017 | 3,3,3-Trifluorpropyl | Me | 5-Br-pyridin-2-yl | H |
| 9-1018 | 3,3,3-Trifluorpropyl | Me | 5-Cl-2-thienyl | H |
| 9-1019 | 3,3,3-Trifluorpropyl | Me | 5-Br-2-thienyl | H |
| 9-1020 | 2-Chlor-phenyl | Me | 4-Cl-Ph | H |
| 9-1021 | 2-Chlor-phenyl | Me | 4-Br-Ph | H |
| 9-1022 | 2-Chlor-phenyl | Me | 5-Cl-pyridin-2-yl | H |
| 9-1023 | 2-Chlor-phenyl | Me | 5-Br-pyridin-2-yl | H |
| 9-1024 | 2-Chlor-phenyl | Me | 5-Cl-2-thienyl | H |
| 9-1025 | 2-Chlor-phenyl | Me | 5-Br-2-thienyl | H |
| 9-1026 | 2-Chlorpyridin-3-yl | Me | 4-Cl-Ph | H |
| 9-1027 | 2-Chlorpyridin-3-yl | Me | 4-Br-Ph | H |
| 9-1028 | 2-Chlorpyridin-3-yl | Me | 5-Cl-pyridin-2-yl | H |
| 9-1029 | 2-Chlorpyridin-3-yl | Me | 5-Br-pyridin-2-yl | H |
| 9-1030 | 2-Chlorpyridin-3-yl | Me | 5-Cl-2-thienyl | H |
| 9-1031 | 2-Chlorpyridin-3-yl | Me | 5-Br-2-thienyl | H |
| 9-1032 | 3-Chlorpyridin-2-yl | Me | 4-Cl-Ph | H |
| 9-1033 | 3-Chlorpyridin-2-yl | Me | 4-Br-Ph | H |
| 9-1034 | 3-Chlorpyridin-2-yl | Me | 5-Cl-pyridin-2-yl | H |
| 9-1035 | 3-Chlorpyridin-2-yl | Me | 5-Br-pyridin-2-yl | H |
| 9-1036 | 3-Chlorpyridin-2-yl | Me | 5-Cl-2-thienyl | H |
| 9-1037 | 3-Chlorpyridin-2-yl | Me | 5-Br-2-thienyl | H |
| 9-1038 | Pentafluorethyl | Me | 4-Cl-Ph | H |
| 9-1039 | Pentafluorethyl | Me | 4-Br-Ph | H |
| 9-1040 | Pentafluorethyl | Me | 5-Cl-pyridin-2-yl | H |
| 9-1041 | Pentafluorethyl | Me | 5-Br-pyridin-2-yl | H |
| 9-1042 | Pentafluorethyl | Me | 5-Cl-2-thienyl | H |
| 9-1043 | Pentafluorethyl | Me | 5-Br-2-thienyl | H |
| 9-1044 | 1,2,2,2-Tetrafluorethyl | Me | 4-Cl-Ph | H |
| 9-1045 | 1,2,2,2-Tetrafluorethyl | Me | 4-Br-Ph | H |
| 9-1046 | 1,2,2,2-Tetrafluorethyl | Me | 5-Cl-pyridin-2-yl | H |
| 9-1047 | 1,2,2,2-Tetrafluorethyl | Me | 5-Br-pyridin-2-yl | H |
| 9-1048 | 1,2,2,2-Tetrafluorethyl | Me | 5-Cl-2-thienyl | H |
| 9-1049 | 1,2,2,2-Tetrafluorethyl | Me | 5-Br-2-thienyl | H |
| 9-1050 | 1,1,2,2-Tetrafluorethyl | Me | 4-Cl-Ph | H |
| 9-1051 | 1,1,2,2-Tetrafluorethyl | Me | 4-Br-Ph | H |
| 9-1052 | 1,1,2,2-Tetrafluorethyl | Me | 5-Cl-pyridin-2-yl | H |
| 9-1053 | 1,1,2,2-Tetrafluorethyl | Me | 5-Br-pyridin-2-yl | H |
| 9-1054 | 1,1,2,2-Tetrafluorethyl | Me | 5-Cl-2-thienyl | H |
| 9-1055 | 1,1,2,2-Tetrafluorethyl | Me | 5-Br-2-thienyl | H |
| 9-1056 | 1,1,2-Trifluorethyl | Me | 4-Cl-Ph | H |
| 9-1057 | 1,1,2-Trifluorethyl | Me | 4-Br-Ph | H |
| 9-1058 | 1,1,2-Trifluorethyl | Me | 5-Cl-pyridin-2-yl | H |
| 9-1059 | 1,1,2-Trifluorethyl | Me | 5-Br-pyridin-2-yl | H |
| 9-1060 | 1,1,2-Trifluorethyl | Me | 5-Cl-2-thienyl | H |
| 9-1061 | 1,1,2-Trifluorethyl | Me | 5-Br-2-thienyl | H |
| 9-1062 | 2-Methylbut-3-in-2-yl | Me | 4-Cl-Ph | H |
| 9-1063 | 2-Methylbut-3-in-2-yl | Me | 4-Br-Ph | H |
| 9-1064 | 2-Methylbut-3-in-2-yl | Me | 5-Cl-pyridin-2-yl | H |
| 9-1065 | 2-Methylbut-3-in-2-yl | Me | 5-Br-pyridin-2-yl | H |
| 9-1066 | 2-Methylbut-3-in-2-yl | Me | 5-Cl-2-thienyl | H |
| 9-1067 | 2-Methylbut-3-in-2-yl | Me | 5-Br-2-thienyl | H |
| 9-1068 | 1-(Ethoxycarbonyl)-eth-1-yl | Me | 4-Cl-Ph | H |
| 9-1069 | 1-(Ethoxycarbonyl)-eth-1-yl | Me | 4-Br-Ph | H |
| 9-1070 | 1-(Ethoxycarbonyl)-eth-1-yl | Me | 5-Cl-pyridin-2-yl | H |
| 9-1071 | 1-(Ethoxycarbonyl)-eth-1-yl | Me | 5-Br-pyridin-2-yl | H |
| 9-1072 | 1-(Ethoxycarbonyl)-eth-1-yl | Me | 5-Cl-2-thienyl | H |
| 9-1073 | 1-(Ethoxycarbonyl)-eth-1-yl | Me | 5-Br-2-thienyl | H |
| 9-1074 | 1,1,2,3,3,3-Hexafluorpropyl | Me | 4-Cl-Ph | H |
| 9-1075 | 1,1,2,3,3,3-Hexafluorpropyl | Me | 4-Br-Ph | H |
| 9-1076 | 1,1,2,3,3,3-Hexafluorpropyl | Me | 5-Cl-pyridin-2-yl | H |
| 9-1077 | 1,1,2,3,3,3-Hexafluorpropyl | Me | 5-Br-pyridin-2-yl | H |
| 9-1078 | 1,1,2,3,3,3-Hexafluorpropyl | Me | 5-Cl-2-thienyl | H |
| 9-1079 | 1,1,2,3,3,3-Hexafluorpropyl | Me | 5-Br-2-thienyl | H |
| 9-1080 | Isobutyl | Me | 4-Cl-Ph | H |
| 9-1081 | Isobutyl | Me | 4-Br-Ph | H |
| 9-1082 | Isobutyl | Me | 5-Cl-pyridin-2-yl | H |
| 9-1083 | Isobutyl | Me | 5-Br-pyridin-2-yl | H |
| 9-1084 | Isobutyl | Me | 5-Cl-2-thienyl | H |
| 9-1085 | Isobutyl | Me | 5-Br-2-thienyl | H |
| 9-1086 | n-Pentyl | Me | 4-Cl-Ph | H |
| 9-1087 | n-Pentyl | Me | 4-Br-Ph | H |
| 9-1088 | n-Pentyl | Me | 5-Cl-pyridin-2-yl | H |
| 9-1089 | n-Pentyl | Me | 5-Br-pyridin-2-yl | H |
| 9-1090 | n-Pentyl | Me | 5-Cl-2-thienyl | H |
| 9-1091 | n-Pentyl | Me | 5-Br-2-thienyl | H |
| 9-1092 | n-Heptyl | Me | 4-Cl-Ph | H |
| 9-1093 | n-Heptyl | Me | 4-Br-Ph | H |
| 9-1094 | n-Heptyl | Me | 5-Cl-pyridin-2-yl | H |
| 9-1095 | n-Heptyl | Me | 5-Br-pyridin-2-yl | H |
| 9-1096 | n-Heptyl | Me | 5-Cl-2-thienyl | H |
| 9-1097 | n-Heptyl | Me | 5-Br-2-thienyl | H |
| 9-1098 | n-Nonyl | Me | 4-Cl-Ph | H |
| 9-1099 | n-Nonyl | Me | 4-Br-Ph | H |
| 9-1100 | n-Nonyl | Me | 5-Cl-pyridin-2-yl | H |
| 9-1101 | n-Nonyl | Me | 5-Br-pyridin-2-yl | H |
| 9-1102 | n-Nonyl | Me | 5-Cl-2-thienyl | H |
| 9-1103 | n-Nonyl | Me | 5-Br-2-thienyl | H |
| 9-1104 | Cyclopentyl | Me | 4-Cl-Ph | H |
| 9-1105 | Cyclopentyl | Me | 4-Br-Ph | H |
| 9-1106 | Cyclopentyl | Me | 5-Cl-pyridin-2-yl | H |
| 9-1107 | Cyclopentyl | Me | 5-Br-pyridin-2-yl | H |
| 9-1108 | Cyclopentyl | Me | 5-Cl-2-thienyl | H |
| 9-1109 | Cyclopentyl | Me | 5-Br-2-thienyl | H |
| 9-1110 | Cyclohexyl | Me | 4-Cl-Ph | H |
| 9-1111 | Cyclohexyl | Me | 4-Br-Ph | H |
| 9-1112 | Cyclohexyl | Me | 5-Cl-pyridin-2-yl | H |
| 9-1113 | Cyclohexyl | Me | 5-Br-pyridin-2-yl | H |
| 9-1114 | Cyclohexyl | Me | 5-Cl-2-thienyl | H |
| 9-1115 | Cyclohexyl | Me | 5-Br-2-thienyl | H |
| 9-1116 | sBu | Me | 4-Cl-Ph | H |
| 9-1117 | sBu | Me | 4-Br-Ph | H |
| 9-1118 | sBu | Me | 5-Cl-pyridin-2-yl | H |
| 9-1119 | sBu | Me | 5-Br-pyridin-2-yl | H |
| 9-1120 | sBu | Me | 5-Cl-2-thienyl | H |
| 9-1121 | sBu | Me | 5-Br-2-thienyl | H |
| 9-1122 | Pentan-3-yl | Me | 4-Cl-Ph | H |
| 9-1123 | Pentan-3-yl | Me | 4-Br-Ph | H |
| 9-1124 | Pentan-3-yl | Me | 5-Cl-pyridin-2-yl | H |
| 9-1125 | Pentan-3-yl | Me | 5-Br-pyridin-2-yl | H |
| 9-1126 | Pentan-3-yl | Me | 5-Cl-2-thienyl | H |
| 9-1127 | Pentan-3-yl | Me | 5-Br-2-thienyl | H |
| 9-1128 | 1-(Methoxycarbonyl)-eth-1-yl | Me | 4-Cl-Ph | H |
| 9-1129 | 1-(Methoxycarbonyl)-eth-1-yl | Me | 4-Br-Ph | H |
| 9-1130 | 1-(Methoxycarbonyl)-eth-1-yl | Me | 5-Cl-pyridin-2-yl | H |
| 9-1131 | 1-(Methoxycarbonyl)-eth-1-yl | Me | 5-Br-pyridin-2-yl | H |
| 9-1132 | 1-(Methoxycarbonyl)-eth-1-yl | Me | 5-Cl-2-thienyl | H |
| 9-1133 | 1-(Methoxycarbonyl)-eth-1-yl | Me | 5-Br-2-thienyl | H |
| 9-1134 | 2,2,2-Trichlorethyl | Me | 4-Cl-Ph | H |
| 9-1135 | 2,2,2-Trichlorethyl | Me | 4-Br-Ph | H |
| 9-1136 | 2,2,2-Trichlorethyl | Me | 5-Cl-pyridin-2-yl | H |
| 9-1137 | 2,2,2-Trichlorethyl | Me | 5-Br-pyridin-2-yl | H |
| 9-1138 | 2,2,2-Trichlorethyl | Me | 5-Cl-2-thienyl | H |
| 9-1139 | 2,2,2-Trichlorethyl | Me | 5-Br-2-thienyl | H |
| 9-1140 | 3-Chlorpropyl | Me | 4-Cl-Ph | H |
| 9-1141 | 3-Chlorpropyl | Me | 4-Br-Ph | H |
| 9-1142 | 3-Chlorpropyl | Me | 5-Cl-pyridin-2-yl | H |
| 9-1143 | 3-Chlorpropyl | Me | 5-Br-pyridin-2-yl | H |
| 9-1144 | 3-Chlorpropyl | Me | 5-Cl-2-thienyl | H |
| 9-1145 | 3-Chlorpropyl | Me | 5-Br-2-thienyl | H |
| 9-1146 | 2-(2-Methoxyethoxy)-ethyl | Me | 4-Cl-Ph | H |
| 9-1147 | 2-(2-Methoxyethoxy)-ethyl | Me | 4-Br-Ph | H |
| 9-1148 | 2-(2-Methoxyethoxy)-ethyl | Me | 5-Cl-pyridin-2-yl | H |
| 9-1149 | 2-(2-Methoxyethoxy)-ethyl | Me | 5-Br-pyridin-2-yl | H |
| 9-1150 | 2-(2-Methoxyethoxy)-ethyl | Me | 5-Cl-2-thienyl | H |
| 9-1151 | 2-(2-Methoxyethoxy)-ethyl | Me | 5-Br-2-thienyl | H |
| 9-1152 | Butyl-2-yl-methyl | Me | 4-Cl-Ph | H |
| 9-1153 | Butyl-2-yl-methyl | Me | 4-Br-Ph | H |
| 9-1154 | Butyl-2-yl-methyl | Me | 5-Cl-pyridin-2-yl | H |
| 9-1155 | Butyl-2-yl-methyl | Me | 5-Br-pyridin-2-yl | H |
| 9-1156 | Butyl-2-yl-methyl | Me | 5-Cl-2-thienyl | H |
| 9-1157 | Butyl-2-yl-methyl | Me | 5-Br-2-thienyl | H |
| 9-1158 | But-3-in-1-yl | Me | 4-Cl-Ph | H |
| 9-1159 | But-3-in-1-yl | Me | 4-Br-Ph | H |
| 9-1160 | But-3-in-1-yl | Me | 5-Cl-pyridin-2-yl | H |
| 9-1161 | But-3-in-1-yl | Me | 5-Br-pyridin-2-yl | H |
| 9-1162 | But-3-in-1-yl | Me | 5-Cl-2-thienyl | H |
| 9-1163 | But-3-in-1-yl | Me | 5-Br-2-thienyl | H |
| 9-1164 | (2,2-Dichlorcyclopropyl)-methyl | Me | 4-Cl-Ph | H |
| 9-1165 | (2,2-Dichlorcyclopropyl)-methyl | Me | 4-Br-Ph | H |
| 9-1166 | (2,2-Dichlorcyclopropyl)-methyl | Me | 5-Cl-pyridin-2-yl | H |
| 9-1167 | (2,2-Dichlorcyclopropyl)-methyl | Me | 5-Br-pyridin-2-yl | H |
| 9-1168 | (2,2-Dichlorcyclopropyl)-methyl | Me | 5-Cl-2-thienyl | H |
| 9-1169 | (2,2-Dichlorcyclopropyl)-methyl | Me | 5-Br-2-thienyl | H |
| 9-1170 | 2-(N,N-Diethylamino)-eth-1-yl | Me | 4-Cl-Ph | H |
| 9-1171 | 2-(N,N-Diethylamino)-eth-1-yl | Me | 4-Br-Ph | H |
| 9-1172 | 2-(N,N-Diethylamino)-eth-1-yl | Me | 5-Cl-pyridin-2-yl | H |
| 9-1173 | 2-(N,N-Diethylamino)-eth-1-yl | Me | 5-Br-pyridin-2-yl | H |
| 9-1174 | 2-(N,N-Diethylamino)-eth-1-yl | Me | 5-Cl-2-thienyl | H |
| 9-1175 | 2-(N,N-Diethylamino)-eth-1-yl | Me | 5-Br-2-thienyl | H |
| 9-1176 | 2-Carboxy-phenyl | Me | 4-Cl-Ph | H |
| 9-1177 | 2-Carboxy-phenyl | Me | 4-Br-Ph | H |
| 9-1178 | 2-Carboxy-phenyl | Me | 5-Cl-pyridin-2-yl | H |
| 9-1179 | 2-Carboxy-phenyl | Me | 5-Br-pyridin-2-yl | H |
| 9-1180 | 2-Carboxy-phenyl | Me | 5-Cl-2-thienyl | H |
| 9-1181 | 2-Carboxy-phenyl | Me | 5-Br-2-thienyl | H |
| 9-1182 | tButyl | Me | 4-Cl-Ph | H |
| 9-1183 | tBu | Me | 4-Br-Ph | H |
| 9-1184 | tBu | Me | 5-Cl-pyridin-2-yl | H |
| 9-1185 | tBu | Me | 5-Br-pyridin-2-yl | H |
| 9-1186 | tBu | Me | 5-Cl-2-thienyl | H |
| 9-1187 | tBu | Me | 5-Br-2-thienyl | H |
| 9-1188 | 1-Methyl-cyclopropyl | Me | 4-Cl-Ph | H |
| 9-1189 | 1-Methyl-cyclopropyl | Me | 4-Br-Ph | H |
| 9-1190 | 1-Methyl-cyclopropyl | Me | 5-Cl-pyridin-2-yl | H |
| 9-1191 | 1-Methyl-cyclopropyl | Me | 5-Br-pyridin-2-yl | H |
| 9-1192 | 1-Methyl-cyclopropyl | Me | 5-Cl-2-thienyl | H |
| 9-1193 | 1-Methyl-cyclopropyl | Me | 5-Br-2-thienyl | H |
| 9-1194 | Trimethylsilylmethyl | Me | 4-Cl-Ph | H |
| 9-1195 | Trimethylsilylmethyl | Me | 4-Br-Ph | H |
| 9-1196 | Trimethylsilylmethyl | Me | 5-Cl-pyridin-2-yl | H |
| 9-1197 | Trimethylsilylmethyl | Me | 5-Br-pyridin-2-yl | H |
| 9-1198 | Trimethylsilylmethyl | Me | 5-Cl-2-thienyl | H |
| 9-1199 | Trimethylsilylmethyl | Me | 5-Br-2-thienyl | H |
| 9-1200 | 2,3-Dihydro-1H-inden-5-yl | Me | 4-Cl-Ph | H |
| 9-1201 | 2,3-Dihydro-1 H-inden-5-yl | Me | 4-Br-Ph | H |
| 9-1202 | 2,3-Dihydro-1 H-inden-5-yl | Me | 5-Cl-pyridin-2-yl | H |
| 9-1203 | 2,3-Dihydro-1H-inden-5-yl | Me | 5-Br-pyridin-2-yl | H |
| 9-1204 | 2,3-Dihydro-1 H-inden-5-yl | Me | 5-Cl-2-thienyl | H |
| 9-1205 | 2,3-Dihydro-1 H-inden-5-yl | Me | 5-Br-2-thienyl | H |
| 9-1206 | 1-Methylcyclobutyl | Me | 4-Cl-Ph | H |
| 9-1207 | 1-Methylcyclobutyl | Me | 4-Br-Ph | H |
| 9-1208 | 1-Methylcyclobutyl | Me | 5-Cl-pyridin-2-yl | H |
| 9-1209 | 1-Methylcyclobutyl | Me | 5-Br-pyridin-2-yl | H |
| 9-1210 | 1 -Methylcyclobutyl | Me | 5-Cl-2-thienyl | H |
| 9-1211 | 1-Methylcyclobutyl | Me | 5-Br-2-thienyl | H |
| 9-1212 | 2-(Oxetan-3-yl)-eth-1-yl | Me | 4-Cl-Ph | H |
| 9-1213 | 2-(Oxetan-3-yl)-eth-1-yl | Me | 4-Br-Ph | H |
| 9-1214 | 2-(Oxetan-3-yl)-eth-1-yl | Me | 5-Cl-pyridin-2-yl | H |
| 9-1215 | 2-(Oxetan-3-yl)-eth-1-yl | Me | 5-Br-pyridin-2-yl | H |
| 9-1216 | 2-(Oxetan-3-yl)-eth-1-yl | Me | 5-Cl-2-thienyl | H |
| 9-1217 | 2-(Oxetan-3-yl)-eth-1-yl | Me | 5-Br-2-thienyl | H |
| 9-1218 | Bu | Me | 4-Cl-Ph | H |
| 9-1219 | Bu | Me | 4-Br-Ph | H |
| 9-1220 | Bu | Me | 5-Cl-pyridin-2-yl | H |
| 9-1221 | Bu | Me | 5-Br-pyridin-2-yl | H |
| 9-1222 | Bu | Me | 5-Cl-2-thienyl | H |
| 9-1223 | Bu | Me | 5-Br-2-thienyl | H |
| 9-1224 | 2-(N,N-Diethylamino)-eth-1-yl | Me | 4-Cl-Ph | H |
| 9-1225 | 2-(N,N-Diethylamino)-eth-1-yl | Me | 4-Br-Ph | H |
| 9-1226 | 2-(N,N-Diethylamino)-eth-1-yl | Me | 5-Cl-pyridin-2-yl | H |
| 9-1227 | 2-(N,N-Diethylamino)-eth-1-yl | Me | 5-Br-pyridin-2-yl | H |
| 9-1228 | 2-(N,N-Diethylamino)-eth-1-yl | Me | 5-Cl-2-thienyl | H |
| 9-1229 | 2-(N,N-Diethylamino)-eth-1-yl | Me | 5-Br-2-thienyl | H |
| 9-1230 | 2-(Morpholin-4-yl)-eth-1-yl | Me | 4-Cl-Ph | H |
| 9-1231 | 2-(Morpholin-4-yl)-eth-1-yl | Me | 4-Br-Ph | H |
| 9-1232 | 2-(Morpholin-4-yl)-eth-1-yl | Me | 5-Cl-pyridin-2-yl | H |
| 9-1233 | 2-(Morpholin-4-yl)-eth-1-yl | Me | 5-Br-pyridin-2-yl | H |
| 9-1234 | 2-(Morpholin-4-yl)-eth-1-yl | Me | 5-Cl-2-thienyl | H |
| 9-1235 | 2-(Morpholin-4-yl)-eth-1-yl | Me | 5-Br-2-thienyl | H |
| 9-1236 | 2-Chlor-thiophen-3-yl | Me | 4-Cl-Ph | H |
| 9-1237 | 2-Chlor-thiophen-3-yl | Me | 4-Br-Ph | H |
| 9-1238 | 2-Chlor-thiophen-3-yl | Me | 5-Cl-pyridin-2-yl | H |
| 9-1239 | 2-Chlor-thiophen-3-yl | Me | 5-Br-pyridin-2-yl | H |
| 9-1240 | 2-Chlor-thiophen-3-yl | Me | 5-Cl-2-thienyl | H |
| 9-1241 | 2-Chlor-thiophen-3-yl | Me | 5-Br-2-thienyl | H |
| 9-1242 | (N,N-Dimethylaminocarbonyl)-methyl | Me | 4-Cl-Ph | H |
| 9-1243 | (N,N-Dimethylaminocarbonyl)-methyl | Me | 4-Br-Ph | H |
| 9-1244 | (N,N-Dimethylaminocarbonyl)-methyl | Me | 5-Cl-pyridin-2-yl | H |
| 9-1245 | (N,N-Dimethylaminocarbonyl)-methyl | Me | 5-Br-pyridin-2-yl | H |
| 9-1246 | (N,N-Dimethylaminocarbonyl)-methyl | Me | 5-Cl-2-thienyl | H |
| 9-1247 | (N,N-Dimethylaminocarbonyl)-methyl | Me | 5-Br-2-thienyl | H |
| 9-1248 | 1-(t-Butylcarbonyloxy)-2-methylprop-1-yl | Me | 4-Cl-Ph | H |
| 9-1249 | 1-(t-Butylcarbonyloxy)-2-methylprop-1-yl | Me | 4-Br-Ph | H |
| 9-1250 | 1-(t-Butylcarbonyloxy)-2-methylprop-1-yl | Me | 5-Cl-pyridin-2-yl | H |
| 9-1251 | 1-(t-Butylcarbonyloxy)-2-methylprop-1-yl | Me | 5-Br-pyridin-2-yl | H |
| 9-1252 | 1-(t-Butylcarbonyloxy)-2-methylprop-1-yl | Me | 5-Cl-2-thienyl | H |
| 9-1253 | 1-(t-Butylcarbonyloxy)-2-methylprop-1-yl | Me | 5-Br-2-thienyl | H |
| 9-1254 | (5-Methyl-2-oxo-1,3-dioxol-4-yl)-methyl | Me | 4-Cl-Ph | H |
| 9-1255 | (5-Methyl-2-oxo-1,3-dioxol-4-yl)-methyl | Me | 4-Br-Ph | H |
| 9-1256 | (5-Methyl-2-oxo-1,3-dioxol-4-yl)-methyl | Me | 5-Cl-pyridin-2-yl | H |
| 9-1257 | (5-Methyl-2-oxo-1,3-dioxol-4-yl)-methyl | Me | 5-Br-pyridin-2-yl | H |
| 9-1258 | (5-Methyl-2-oxo-1,3-dioxol-4-yl)-methyl | Me | 5-Cl-2-thienyl | H |
| 9-1259 | (5-Methyl-2-oxo-1,3-dioxol-4-yl)-methyl | Me | 5-Br-2-thienyl | H |
| 9-1260 | [(t-Butoxycarbonyl)oxy]-methyl | Me | 4-Cl-Ph | H |
| 9-1261 | [(t-Butoxycarbonyl)oxy]-methyl | Me | 4-Br-Ph | H |
| 9-1262 | [(t-Butoxycarbonyl)oxy]-methyl | Me | 5-Cl-pyridin-2-yl | H |
| 9-1263 | [(t-Butoxycarbonyl)oxy]-methyl | Me | 5-Br-pyridin-2-yl | H |
| 9-1264 | [(t-Butoxycarbonyl)oxy]-methyl | Me | 5-Cl-2-thienyl | H |
| 9-1265 | [(t-Butoxycarbonyl)oxy]-methyl | Me | 5-Br-2-thienyl | H |
| 9-1266 | [(Isopropoxycarbonyl)oxy]-methyl | Me | 4-Cl-Ph | H |
| 9-1267 | [(Isopropoxycarbonyl)oxy]-methyl | Me | 4-Br-Ph | H |
| 9-1268 | [(Isopropoxycarbonyl)oxy]-methyl | Me | 5-Cl-pyridin-2-yl | H |
| 9-1269 | [(Isopropoxycarbonyl)oxy]-methyl | Me | 5-Br-pyridin-2-yl | H |
| 9-1270 | [(Isopropoxycarbonyl)oxy]-methyl | Me | 5-Cl-2-thienyl | H |
| 9-1271 | [(Isopropoxycarbonyl)oxy]-methyl | Me | 5-Br-2-thienyl | H |
| 9-1272 | [(Methoxycarbonyl)oxy]-methyl | Me | 4-Cl-Ph | H |
| 9-1273 | [(Methoxycarbonyl)oxy]-methyl | Me | 4-Br-Ph | H |
| 9-1274 | [(Methoxycarbonyl)oxy]-methyl | Me | 5-Cl-pyridin-2-yl | H |
| 9-1275 | [(Methoxycarbonyl)oxy]-methyl | Me | 5-Br-pyridin-2-yl | H |
| 9-1276 | [(Methoxycarbonyl)oxy]-methyl | Me | 5-Cl-2-thienyl | H |
| 9-1277 | [(Methoxycarbonyl)oxy]-methyl | Me | 5-Br-2-thienyl | H |
| 9-1278 | 1-[(Ethoxycarbonyl)oxy]-ethyl | Me | 4-Cl-Ph | H |
| 9-1279 | 1-[(Ethoxycarbonyl)oxy]-ethyl | Me | 4-Br-Ph | H |
| 9-1280 | 1-[(Ethoxycarbonyl)oxy]-ethyl | Me | 5-Cl-pyridin-2-yl | H |
| 9-1281 | 1-[(Ethoxycarbonyl)oxy]-ethyl | Me | 5-Br-pyridin-2-yl | H |
| 9-1282 | 1-[(Ethoxycarbonyl)oxy]-ethyl | Me | 5-Cl-2-thienyl | H |
| 9-1283 | 1-[(Ethoxycarbonyl)oxy]-ethyl | Me | 5-Br-2-thienyl | H |
| 9-1284 | 1-Acetoxy-eth-1-yl | Me | 4-Cl-Ph | H |
| 9-1285 | 1-Acetoxy-eth-1-yl | Me | 4-Br-Ph | H |
| 9-1286 | 1-Acetoxy-eth-1-yl | Me | 5-Cl-pyridin-2-yl | H |
| 9-1287 | 1-Acetoxy-eth-1-yl | Me | 5-Br-pyridin-2-yl | H |
| 9-1288 | 1-Acetoxy-eth-1-yl | Me | 5-Cl-2-thienyl | H |
| 9-1289 | 1-Acetoxy-eth-1-yl | Me | 5-Br-2-thienyl | H |
| 9-1290 | 1-(2-Methylpropanoyloxy)-eth-1-yl | Me | 4-Cl-Ph | H |
| 9-1291 | 1-(2-Methylpropanoyloxy)-eth-1-yl | Me | 4-Br-Ph | H |
| 9-1292 | 1-(2-Methylpropanoyloxy)-eth-1-yl | Me | 5-Cl-pyridin-2-yl | H |
| 9-1293 | 1-(2-Methylpropanoyloxy)-eth-1-yl | Me | 5-Br-pyridin-2-yl | H |
| 9-1294 | 1-(2-Methylpropanoyloxy)-eth-1-yl | Me | 5-Cl-2-thienyl | H |
| 9-1295 | 1-(2-Methylpropanoyloxy)-eth-1-yl | Me | 5-Br-2-thienyl | H |
| 9-1296 | 1-Propanoyl-2-methyl-prop-1-yl | Me | 4-Cl-Ph | H |
| 9-1297 | 1-Propanoyl-2-methyl-prop-1-yl | Me | 4-Br-Ph | H |
| 9-1298 | 1-Propanoyl-2-methyl-prop-1-yl | Me | 5-Cl-pyridin-2-yl | H |
| 9-1299 | 1-Propanoyl-2-methyl-prop-1-yl | Me | 5-Br-pyridin-2-yl | H |
| 9-1300 | 1-Propanoyl-2-methyl-prop-1-yl | Me | 5-Cl-2-thienyl | H |
| 9-1301 | 1-Propanoyl-2-methyl-prop-1-yl | Me | 5-Br-2-thienyl | H |
| 9-1302 | 1-(Cyclohexoxycarbonyloxy)-eth-1-yl | Me | 4-Cl-Ph | H |
| 9-1303 | 1-(Cyclohexoxycarbonyloxy)-eth-1-yl | Me | 4-Br-Ph | H |
| 9-1304 | 1-(Cyclohexoxycarbonyloxy)-eth-1-yl | Me | 5-Cl-pyridin-2-yl | H |
| 9-1305 | 1-(Cyclohexoxycarbonyloxy)-eth-1-yl | Me | 5-Br-pyridin-2-yl | H |
| 9-1306 | 1-(Cyclohexoxycarbonyloxy)-eth-1-yl | Me | 5-Cl-2-thienyl | H |
| 9-1307 | 1-(Cyclohexoxycarbonyloxy)-eth-1-yl | Me | 5-Br-2-thienyl | H |
| 9-1308 | Cyclobutyl | Me | 4-Cl-Ph | H |
| 9-1309 | Cyclobutyl | Me | 4-Br-Ph | H |
| 9-1310 | Cyclobutyl | Me | 5-Cl-pyridin-2-yl | H |
| 9-1311 | Cyclobutyl | Me | 5-Br-pyridin-2-yl | H |
| 9-1312 | Cyclobutyl | Me | 5-Cl-2-thienyl | H |
| 9-1313 | Cyclobutyl | Me | 5-Br-2-thienyl | H |
| 9-1314 | CH₂(4-Me-Ph) | Me | 4-Cl-Ph | H |
| 9-1315 | CH₂(4-Me-Ph) | Me | 4-Br-Ph | H |
| 9-1316 | CH₂(4-Me-Ph) | Me | 5-Cl-pyridin-2-yl | H |
| 9-1317 | CH₂(4-Me-Ph) | Me | 5-Br-pyridin-2-yl | H |
| 9-1318 | CH₂(4-Me-Ph) | Me | 5-Cl-2-thienyl | H |
| 9-1319 | CH₂(4-Me-Ph) | Me | 5-Br-2-thienyl | H |
| 9-1320 | CHMe(4-Cl-Ph) | Me | 4-Cl-Ph | H |
| 9-1321 | CHMe(4-Cl-Ph) | Me | 4-Br-Ph | H |
| 9-1322 | CHMe(4-Cl-Ph) | Me | 5-Cl-pyridin-2-yl | H |
| 9-1323 | CHMe(4-Cl-Ph) | Me | 5-Br-pyridin-2-yl | H |
| 9-1324 | CHMe(4-Cl-Ph) | Me | 5-Cl-2-thienyl | H |
| 9-1325 | CHMe(4-Cl-Ph) | Me | 5-Br-2-thienyl | H |
| 9-1326 | CHMePh | Me | 4-Cl-Ph | H |
| 9-1327 | CHMePh | Me | 4-Br-Ph | H |
| 9-1328 | CHMePh | Me | 5-Cl-pyridin-2-yl | H |
| 9-1329 | CHMePh | Me | 5-Br-pyridin-2-yl | H |
| 9-1330 | CHMePh | Me | 5-Cl-2-thienyl | H |
| 9-1331 | CHMePh | Me | 5-Br-2-thienyl | H |
| 9-1332 | 1,1,1-Trifluorpropan-2-yl | Me | 4-Cl-Ph | H |
| 9-1333 | 1,1,1-Trifluorpropan-2-yl | Me | 4-Br-Ph | H |
| 9-1334 | 1,1,1-Trifluorpropan-2-yl | Me | 5-Cl-pyridin-2-yl | H |
| 9-1335 | 1,1,1-Trifluorpropan-2-yl | Me | 5-Br-pyridin-2-yl | H |
| 9-1336 | 1,1,1-Trifluorpropan-2-yl | Me | 5-Cl-2-thienyl | H |
| 9-1337 | 1,1,1-Trifluorpropan-2-yl | Me | 5-Br-2-thienyl | H |
| 9-1338 | (1-Ethyl-3-methyl-1H-pyrazol-4-yl)methyl | Me | 4-Cl-Ph | H |
| 9-1339 | (1-Ethyl-3-methyl-1H-pyrazol-4-yl)-methyl | Me | 4-Br-Ph | H |
| 9-1340 | (1-Ethyl-3-methyl-1H-pyrazol-4-yl)-methyl | Me | 5-Cl-pyridin-2-yl | H |
| 9-1341 | (1-Ethyl-3-methyl-1H-pyrazol-4-yl)-methyl | Me | 5-Br-pyridin-2-yl | H |
| 9-1342 | (1-Ethyl-3-methyl-1H-pyrazol-4-yl)-methyl | Me | 5-Cl-2-thienyl | H |
| 9-1343 | (1-Ethyl-3-methyl-1H-pyrazol-4-yl)-methyl | Me | 5-Br-2-thienyl | H |
| 9-1344 | Pr | Me | 4-Cl-Ph | H |
| 9-1345 | Pr | Me | 4-Br-Ph | H |
| 9-1346 | Pr | Me | 5-Cl-pyridin-2-yl | H |
| 9-1347 | Pr | Me | 5-Br-pyridin-2-yl | H |
| 9-1348 | Pr | Me | 5-Cl-2-thienyl | H |
| 9-1349 | Pr | Me | 5-Br-2-thienyl | H |
| 9-1350 | n-Octadecyl | Me | 4-Cl-Ph | H |
| 9-1351 | n-Octadecyl | Me | 4-Br-Ph | H |
| 9-1352 | n-Octadecyl | Me | 5-Cl-pyridin-2-yl | H |
| 9-1353 | n-Octadecyl | Me | 5-Br-pyridin-2-yl | H |
| 9-1354 | n-Octadecyl | Me | 5-Cl-2-thienyl | H |
| 9-1355 | n-Octadecyl | Me | 5-Br-2-thienyl | H |
| 9-1356 | n-Hexadecyl | Me | 4-Cl-Ph | H |
| 9-1357 | n-Hexadecyl | Me | 4-Br-Ph | H |
| 9-1358 | n-Hexadecyl | Me | 5-Cl-pyridin-2-yl | H |
| 9-1359 | n-Hexadecyl | Me | 5-Br-pyridin-2-yl | H |
| 9-1360 | n-Hexadecyl | Me | 5-Cl-2-thienyl | H |
| 9-1361 | n-Hexadecyl | Me | 5-Br-2-thienyl | H |
| 9-1362 | Oxetan-3-yl-methyl | Me | 4-Cl-Ph | H |
| 9-1363 | Oxetan-3-yl-methyl | Me | 4-Br-Ph | H |
| 9-1364 | Oxetan-3-yl-methyl | Me | 5-Cl-pyridin-2-yl | H |
| 9-1365 | Oxetan-3-yl-methyl | Me | 5-Br-pyridin-2-yl | H |
| 9-1366 | Oxetan-3-yl-methyl | Me | 5-Cl-2-thienyl | H |
| 9-1367 | Oxetan-3-yl-methyl | Me | 5-Br-2-thienyl | H |
| 9-1368 | 3-Methyloxetan-3-yl | Me | 4-Cl-Ph | H |
| 9-1369 | 3-Methyloxetan-3-yl | Me | 4-Br-Ph | H |
| 9-1370 | 3-Methyloxetan-3-yl | Me | 5-Cl-pyridin-2-yl | H |
| 9-1371 | 3-Methyloxetan-3-yl | Me | 5-Br-pyridin-2-yl | H |
| 9-1372 | 3-Methyloxetan-3-yl | Me | 5-Cl-2-thienyl | H |
| 9-1373 | 3-Methyloxetan-3-yl | Me | 5-Br-2-thienyl | H |
| 9-1374 | 2-Chlorprop-2-en-1-yl | Me | 4-CI-Ph | H |
| 9-1375 | 2-Chlorprop-2-en-1-yl | Me | 4-Br-Ph | H |
| 9-1376 | 2-Chlorprop-2-en-1-yl | Me | 5-Cl-pyridin-2-yl | H |
| 9-1377 | 2-Chlorprop-2-en-1-yl | Me | 5-Br-pyridin-2-yl | H |
| 9-1378 | 2-Chlorprop-2-en-1-yl | Me | 5-Cl-2-thienyl | H |
| 9-1379 | 2-Chlorprop-2-en-1-yl | Me | 5-Br-2-thienyl | H |
| 9-1380 | (3E)-Pent-3-en-2-yl | Me | 4-Cl-Ph | H |
| 9-1381 | (3E)-Pent-3-en-2-yl | Me | 4-Br-Ph | H |
| 9-1382 | (3E)-Pent-3-en-2-yl | Me | 5-Cl-pyridin-2-yl | H |
| 9-1383 | (3E)-Pent-3-en-2-yl | Me | 5-Br-pyridin-2-yl | H |
| 9-1384 | (3E)-Pent-3-en-2-yl | Me | 5-Cl-2-thienyl | H |
| 9-1385 | (3E)-Pent-3-en-2-yl | Me | 5-Br-2-thienyl | H |
| 9-1386 | (2,2-Dimethylpropanoyloxy)-methyl | Me | 4-Cl-Ph | H |
| 9-1387 | (2,2-Dimethylpropanoyloxy)-methyl | Me | 4-Br-Ph | H |
| 9-1388 | (2,2-Dimethylpropanoyloxy)-methyl | Me | 5-Cl-pyridin-2-yl | H |
| 9-1389 | (2,2-Dimethylpropanoyloxy)-methyl | Me | 5-Br-pyridin-2-yl | H |
| 9-1390 | (2,2-Dimethylpropanoyloxy)-methyl | Me | 5-Cl-2-thienyl | H |
| 9-1391 | (2,2-Dimethylpropanoyloxy)-methyl | Me | 5-Br-2-thienyl | H |
| 9-1392 | 2-(Isopropoxycarbonyloxy)-eth-1-yl | Me | 4-Cl-Ph | H |
| 9-1393 | 2-(Isopropoxycarbonyloxy)-eth-1-yl | Me | 4-Br-Ph | H |
| 9-1394 | 2-(Isopropoxycarbonyloxy)-eth-1-yl | Me | 5-Cl-pyridin-2-yl | H |
| 9-1395 | 2-(Isopropoxycarbonyloxy)-eth-1-yl | Me | 5-Br-pyridin-2-yl | H |
| 9-1396 | 2-(Isopropoxycarbonyloxy)-eth-1-yl | Me | 5-Cl-2-thienyl | H |
| 9-1397 | 2-(Isopropoxycarbonyloxy)-eth-1-yl | Me | 5-Br-2-thienyl | H |

Für einige Verbindungen der allgemeinen Formel (I) gemäß Tabelle 9 wurden ¹H-NMR-Spektren bei 400 MHz (CDCl₃) (¹H-Kernresonanz-Daten) gemessen. Nachfolgend sind charakteristische chemische Verschiebungen δ (ppm) für einige Beispielverbindungen aufgeführt (die jeweilige Nummer der Verbindung gehört zur laufenden Nr. in Tabelle 9):
NMR Verbindung 9-4 (CDCl₃, 400 MHz, δ in ppm): 1.23 (*t*, 3H); 2.32 *(s,* 3H); 3.32 *(s,* 2H); 4.14 (*q,* 2H); 7.24 (*d*, 2H); 7.29 *(s,* 1H); 7.41 (*d,* 2H); 8.52 *(s,* 1 H).

**Tabelle 10: Verbindungen der Formel (III) (Zwischenprodukte)**

| | | | | | |
|---|---|---|---|---|---|
| 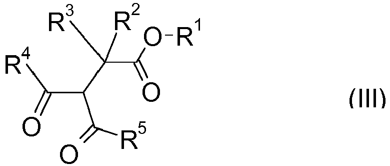 | | | | | |

| Nr. | R¹ | R² | R³ | R⁴ | R⁵ |
|---|---|---|---|---|---|
| 10-1 | Me | H | H | Ph | Ph |
| 10-2 | Me | H | H | Me | Ph |
| 10-3 | Me | H | H | Me | 2-Furyl |
| 10-4 | Me | Me | H | Me | Ph |
| 10-5 | Me | H | H | Me | 4-MeO-Ph |
| 10-6 | Me | H | H | Me | 4-Me-Ph |
| 10-7 | Me | H | H | Me | 3-CF₃-Ph |
| 10-8 | Me | H | H | Me | 3,4-Cl₂-Ph |
| 10-9 | Me | H | H | Me | 3-CI-Ph |
| 10-10 | Me | H | H | Me | 2-CI-Ph |
| 10-11 | Me | H | H | Me | 2,4-Cl₂-Ph |
| 10-12 | Me | H | H | Me | 4-CF₃-Ph |
| 10-13 | Me | H | H | Me | 4-CI-Ph |
| 10-14 | Me | H | H | Me | 4-CF₃-Ph |
| 10-15 | Me | H | H | Me | 4-tBu-Ph |
| 10-16 | Me | H | H | Me | 3,5-Me₂-Ph |
| 10-17 | Me | H | H | Me | 4-Me-Ph |
| 10-18 | Me | H | H | Me | 4-F-Ph |
| 10-19 | Me | H | H | Me | 3-Me-Ph |
| 10-20 | Me | H | H | Me | 4-COOH-Ph |
| 10-21 | Me | H | H | Me | 3-Br-Ph |
| 10-22 | Me | H | H | Me | 4-Ph-Ph |
| 10-23 | Me | H | H | Me | 3-Cl-4-Me-Ph |
| 10-24 | Me | H | H | Me | 3-CF₃-4-Cl-Ph |
| 10-25 | Me | H | H | Me | 2-Thienyl |
| 10-26 | Me | H | H | Me | 3-Me-2-thienyl |
| 10-27 | Me | H | H | Me | 4-Me-2-thienyl |
| 10-28 | Me | H | H | Me | 5-Cl-2-thienyl |
| 10-29 | Me | H | H | Me | 5-J-2-thienyl |
| 10-30 | Me | H | H | Me | 3-Thienyl |
| 10-31 | Me | H | H | Me | 3-Pyridyl |
| 10-32 | Me | H | H | Me | 5-Me-2-thienyl |
| 10-33 | Me | H | H | Me | 6-MeO-pyridin-3-yl |
| 10-34 | Me | H | H | Me | 5-Br-2-thienyl |
| 10-35 | Me | H | H | Me | 4-Br-Ph |
| 10-36 | Me | H | H | Me | 1,3-Benzodioxol-5-yl |
| 10-37 | Me | H | H | Me | 4-J-Ph |
| 10-38 | Me | H | H | Me | 4-PhO-Ph |
| 10-39 | Me | H | H | Me | 6-OH-pyridin-3-yl |
| 10-40 | Me | H | H | H | Ph |
| 10-41 | Me | H | H | Et | Ph |
| 10-42 | Me | H | H | n-Pr | Ph |
| 10-43 | Me | H | H | CH₂Cl | Ph |
| 10-44 | Me | H | H | CHCl₂ | Ph |
| 10-45 | Me | H | H | CH₂F | Ph |
| 10-46 | Me | H | H | CHF₂ | Ph |
| 10-47 | Me | H | H | Cl | Ph |
| 10-48 | Me | H | H | n-Pr | 4-CI-Ph |
| 10-49 | Me | H | H | CH₂Cl | 4-CI-Ph |
| 10-50 | Me | H | H | CHCl₂ | 4-CI-Ph |
| 10-51 | Me | H | H | CH₂F | 4-CI-Ph |
| 10-52 | Me | H | H | CHF₂ | 4-CI-Ph |
| 10-53 | Me | H | H | Cl | 4-CI-Ph |
| 10-54 | Me | H | H | Et | 4-Me-Ph |
| 10-55 | Me | H | H | n-Pr | 4-Me-Ph |
| 10-56 | Me | H | H | CH₂Cl | 4-Me-Ph |
| 10-57 | Me | H | H | CHCl₂ | 4-Me-Ph |
| 10-58 | Me | H | H | CH₂F | 4-Me-Ph |
| 10-59 | Me | H | H | CHF₂ | 4-Me-Ph |
| 10-60 | Me | H | H | Cl | 4-Me-Ph |
| 10-61 | Me | H | H | Et | 2-Pyridyl |
| 10-62 | Me | H | H | n-Pr | 2-Pyridyl |
| 10-63 | Me | H | H | CH₂Cl | 2-Pyridyl |
| 10-64 | Me | H | H | CHCl₂ | 2-Pyridyl |
| 10-65 | Me | H | H | CH₂F | 2-Pyridyl |
| 10-66 | Me | H | H | CHF₂ | 2-Pyridyl |
| 10-67 | Me | H | H | Cl | 2-Pyridyl |
| 10-68 | Me | H | H | Me | 2-Pyridyl |
| 10-69 | Me | H | H | Me | 5-Cl-pyridin-2-yl |
| 10-70 | Me | H | H | Me | 5-Br-pyridin-2-yl |
| 10-71 | Me | H | H | Me | 5-F-pyridin-2-yl |
| 10-72 | Me | H | H | Me | 5-Me-pyridin-2-yl |
| 10-73 | Me | H | H | Me | 4-Me-pyridin-2-yl |
| 10-74 | Me | H | H | Me | 4-CH₂COOH-Ph |
| 10-75 | Me | H | H | Me | 4-Me(CO)-Ph |
| 10-76 | Me | H | H | Me | 4-Cl-3-Me-Ph |
| 10-77 | Me | H | H | n-Pr | 4-CI-Ph |
| 10-78 | Me | H | H | Me | 3-Pyridyl |
| 10-79 | Me | H | H | Me | 4-Pyridyl |
| 10-80 | Me | H | H | C(O)OMe | Ph |
| 10-81 | Me | H | H | Me | 6-Me-pyridin-3-yl |
| 10-82 | Me | H | H | Me | 2,3-Cl₂-Ph |
| 10-83 | Me | H | H | H | 4-Cl-Ph |
| 10-84 | Me | H | H | Me | 6-Cl-pyridin-3-yl |
| 10-85 | Me | H | H | Me | 2-Thiazolyl |
| 10-86 | Me | H | H | Me | 4-Me-thiazol-2-yl |
| 10-87 | Me | H | H | Me | 5-Br-thiazol-2-yl |
| 10-88 | Me | H | H | Me | 5-Cl-thiazol-2-yl |
| 10-89 | Me | H | H | Me | 5-Me-thiazol-2-yl |
| 10-90 | Me | H | H | Me | 4,5-Me₂-thiazol-2-yl |
| 10-91 | Me | H | H | Me | 4,5-Cl₂-thiazol-2-yl |
| 10-92 | Me | H | H | Me | 4,6-Me₂-pyridin-2-yl |
| 10-93 | Me | H | H | Me | 2-Pyrazinyl |
| 10-94 | Me | H | H | Me | 2-Pyrimidinyl |
| 10-95 | Me | H | H | Me | 5-Cl-pyrimidin-2-yl |
| 10-96 | Me | H | H | Me | 5-Br-pyrimidin-2-yl |
| 10-97 | Me | H | H | Me | 5-Me-pyrimidin-2-yl |
| 10-98 | Me | H | H | Me | 4,6-Me₂-pyrimidin-2-yl |
| 10-99 | Me | H | H | Me | 1,3-Benzothiazol-2-yl |
| 10-100 | Me | H | H | Me | 7-Cl-1,3-benzothiazol-2-yl |
| 10-101 | Me | H | H | Me | 1,5-Me₂-pyrazol-3-yl |
| 10-102 | Me | H | H | Me | 5-Me-Pyrazin-2-yl |
| 10-103 | Me | H | H | Me | 5-F-pyrimidin-2-yl |
| 10-104 | Me | H | H | Me | 3-Pyridazinyl |
| 10-105 | Me | H | H | Me | 6-Me-Pyridazin-3-yl |
| 10-106 | Me | H | H | Me | 1,2,4-Triazin-3-yl |
| 10-107 | Me | H | H | Me | 6-Me-1,2,4-triazin-3-yl |
| 10-108 | Et | H | H | Ph | Ph |
| 10-109 | Et | H | H | Me | Ph |
| 10-110 | Et | H | H | Me | 2-Furyl |
| 10-111 | Et | Me | H | Me | Ph |
| 10-112 | Et | H | H | Me | 4-MeO-Ph |
| 10-113 | Et | H | H | Me | 4-Me-Ph |
| 10-114 | Et | H | H | Me | 3-CF₃-Ph |
| 10-115 | Et | H | H | Me | 3,4-Cl₂-Ph |
| 10-116 | Et | H | H | Me | 3-Cl-Ph |
| 10-117 | Et | H | H | Me | 2-Cl-Ph |
| 10-118 | Et | H | H | Me | 2,4-Cl₂-Ph |
| 10-119 | Et | H | H | Me | 4-CF₃-Ph |
| 10-120 | Et | H | H | Me | 4-Cl-Ph |
| 10-121 | Et | H | H | Me | 4-tBu-Ph |
| 10-122 | Et | H | H | Me | 3,5-Me₂-Ph |
| 10-123 | Et | H | H | Me | 4-Me-Ph |
| 10-124 | Et | H | H | Me | 4-F-Ph |
| 10-125 | Et | H | H | Me | 3-Me-Ph |
| 10-126 | Et | H | H | Me | 4-COOH-Ph |
| 10-127 | Et | H | H | Me | 3-Br-Ph |
| 10-128 | Et | H | H | Me | 4-Ph-Ph |
| 10-129 | Et | H | H | Me | 3-Cl-4-Me-Ph |
| 10-130 | Et | H | H | Me | 3-CF₃-4-Cl-Ph |
| 10-131 | Et | H | H | Me | 2-Thienyl |
| 10-132 | Et | H | H | Me | 3-Me-2-thienyl |
| 10-133 | Et | H | H | Me | 4-Me-2-thienyl |
| 10-134 | Et | H | H | Me | 5-Cl-2-thienyl |
| 10-135 | Et | H | H | Me | 5-J-2-thienyl |
| 10-136 | Et | H | H | Me | 3-Thienyl |
| 10-137 | Et | H | H | Me | 3-Pyridyl |
| 10-138 | Et | H | H | Me | 5-Me-2-thienyl |
| 10-139 | Et | H | H | Me | 6-MeO-pyridin-3-yl |
| 10-140 | Et | H | H | Me | 5-Br-2-thienyl |
| 10-141 | Et | H | H | Me | 4-Br-Ph |
| 10-142 | Et | H | H | Me | 1,3-Benzodioxol-5-yl |
| 10-143 | Et | H | H | Me | 4-J-Ph |
| 10-144 | Et | H | H | Me | 4-PhO-Ph |
| 10-145 | Et | H | H | Me | 6-OH-pyridin-3-yl |
| 10-146 | Et | H | H | H | Ph |
| 10-147 | Et | H | H | Et | Ph |
| 10-148 | Et | H | H | n-Pr | Ph |
| 10-149 | Et | H | H | CH₂Cl | Ph |
| 10-150 | Et | H | H | CHCl₂ | Ph |
| 10-151 | Et | H | H | CH₂F | Ph |
| 10-152 | Et | H | H | CHF₂ | Ph |
| 10-153 | Et | H | H | Cl | Ph |
| 10-154 | Et | H | H | n-Pr | 4-CI-Ph |
| 10-155 | Et | H | H | CH₂Cl | 4-CI-Ph |
| 10-156 | Et | H | H | CHCl₂ | 4-Cl-Ph |
| 10-157 | Et | H | H | CH₂F | 4-Cl-Ph |
| 10-158 | Et | H | H | CHF₂ | 4-Cl-Ph |
| 10-159 | Et | H | H | Cl | 4-Cl-Ph |
| 10-160 | Et | H | H | Et | 4-Me-Ph |
| 10-161 | Et | H | H | n-Pr | 4-Me-Ph |
| 10-162 | Et | H | H | CH₂Cl | 4-Me-Ph |
| 10-163 | Et | H | H | CHCl₂ | 4-Me-Ph |
| 10-164 | Et | H | H | CH₂F | 4-Me-Ph |
| 10-165 | Et | H | H | CHF₂ | 4-Me-Ph |
| 10-166 | Et | H | H | Cl | 4-Me-Ph |
| 10-167 | Et | H | H | Et | 2-Pyridyl |
| 10-168 | Et | H | H | n-Pr | 2-Pyridyl |
| 10-169 | Et | H | H | CH₂Cl | 2-Pyridyl |
| 10-170 | Et | H | H | CHCl₂ | 2-Pyridyl |
| 10-171 | Et | H | H | CH₂F | 2-Pyridyl |
| 10-172 | Et | H | H | CHF₂ | 2-Pyridyl |
| 10-173 | Et | H | H | Cl | 2-Pyridyl |
| 10-174 | Et | H | H | Me | 2-Pyridyl |
| 10-175 | Et | H | H | Me | 5-Cl-pyridin-2-yl |
| 10-176 | Et | H | H | Me | 5-Br-pyridin-2-yl |
| 10-177 | Et | H | H | Me | 5-F-pyridin-2-yl |
| 10-178 | Et | H | H | Me | 5-Me-pyridin-2-yl |
| 10-179 | Et | H | H | Me | 4-Me-pyridin-2-yl |
| 10-180 | Et | H | H | Me | 4-CH₂COOH-Ph |
| 10-181 | Et | H | H | Me | 4-Me(CO)-Ph |
| 10-182 | Et | H | H | Me | 4-Cl-3-Me-Ph |
| 10-183 | Et | H | H | n-Pr | 4-Cl-Ph |
| 10-184 | Et | H | H | Me | 3-Pyridyl |
| 10-185 | Et | H | H | Me | 4-Pyridyl |
| 10-186 | Et | H | H | C(O)OMe | Ph |
| 10-187 | Et | H | H | Me | 6-Me-pyridin-3-yl |
| 10-188 | Et | H | H | Me | 2,3-Cl₂-Ph |
| 10-189 | Et | H | H | H | 4-Cl-Ph |
| 10-190 | Et | H | H | Me | 6-Cl-pyridin-3-yl |
| 10-191 | Et | H | H | Me | 2-Thiazolyl |
| 10-192 | Et | H | H | Me | 4-Me-thiazol-2-yl |
| 10-193 | Et | H | H | Me | 5-Br-thiazol-2-yl |
| 10-194 | Et | H | H | Me | 5-Cl-thiazol-2-yl |
| 10-195 | Et | H | H | Me | 5-Me-thiazol-2-yl |
| 10-196 | Et | H | H | Me | 4,5-Me₂-thiazol-2-yl |
| 10-197 | Et | H | H | Me | 4,5-Cl₂-thiazol-2-yl |
| 10-198 | Et | H | H | Me | 4,6-Me₂-pyridin-2-yl |
| 10-199 | Et | H | H | Me | 2-Pyrazinyl |
| 10-200 | Et | H | H | Me | 2-Pyrimidinyl |
| 10-201 | Et | H | H | Me | 5-Cl-pyrimidin-2-yl |
| 10-202 | Et | H | H | Me | 5-Br-pyrimidin-2-yl |
| 10-203 | Et | H | H | Me | 5-Me-pyrimidin-2-yl |
| 10-204 | Et | H | H | Me | 4,6-Me₂-pyrimidin-2-yl |
| 10-205 | Et | H | H | Me | 2-(1,3-Benzothiazolyl) |
| 10-206 | Et | H | H | Me | 7-Cl-(1,3-benzothiazol-2-yl) |
| 10-207 | Et | H | H | Me | 3-(1,5-Me₂-pyrazolyl) |
| 10-208 | Et | H | H | Me | 5-Me-Pyrazin-2-yl |
| 10-209 | Et | H | H | Me | 5-F-pyrimidin-2-yl |
| 10-210 | Et | H | H | Me | 3-Pyridazinyl |
| 10-211 | Et | H | H | Me | 6-Me-Pyridazin-3-yl |
| 10-212 | Et | H | H | Me | 3-(1,2,4)-Triazinyl |
| 10-213 | Et | H | H | Me | 6-Me-(1,2,4)-Triazin-3-yl |
| 10-214 | Et | H | H | Me | Chinolin-2-yl |
| 10-215 | Et | H | H | Me | Isochinolin-3-yl |
| 10-216 | Me | H | H | Me | 5-Cl-pyridin-3-yl |
| 10-217 | Me | H | H | Me | 4-Br-3-Me-Ph |
| 10-218 | Me | H | H | Me | 4-Cl-6-Me-pyridin-2-yl |
| 10-219 | Me | H | H | Me | 2-Me-pyridin-4-yl |
| 10-220 | Me | H | H | Me | 3,5-Cl₂-Ph |
| 10-221 | Me | H | H | Me | Chinolin-2-yl |
| 10-222 | Me | H | H | Me | Isochinolin-3-yl |
| 10-223 | Me | H | H | Me | 5-CF₃₋pyridin-2-yl |
| 10-224 | Me | H | H | Me | 6-OMe-pyridin-2-yl |
| 10-225 | Me | H | H | Me | 4-OMe-pyridin-2-yl |
| 10-226 | Me | H | H | Me | 4-Br-pyridin-2-yl |
| 10-227 | Me | H | H | Me | 4-Cl-pyridin-2-yl |
| 10-228 | Me | H | H | Me | 4-F-pyridin-2-yl |
| 10-229 | Me | H | H | Me | 3,4-Me₂-Ph |
| 10-230 | Me | Me | H | Me | 4-Cl-Ph |
| 10-231 | Me | Me | H | Me | 5-Cl-pyridin-2-yl |
| 10-232 | Me | Me | H | Me | 5-Br-pyridin-2-yl |
| 10-233 | Me | Me | H | Me | 5-Br-pyrimidin-2-yl |
| 10-234 | Me | Me | H | Me | 5-Cl-pyrimidin-2-yl |
| 10-235 | Me | H | H | Me | 4-NO₂-Ph |
| 10-236 | Me | Me | H | Me | 2-Pyridinyl |
| 10-237 | Me | H | H | Me | 5-Allyl-pyridin-2-yl |
| 10-238 | Me | H | H | Me | 5-cyclopropy-pyridin-2-yl |
| 10-239 | Me | H | H | Me | 5-Ethinyl-pyridin-2-yl |
| 10-240 | Me | H | H | Me | 5-Ph-pyridin-2-yl |
| 10-241 | Me | H | H | Me | 6-Cl-1,3-benzothiazol-2-yl |
| 10-242 | Et | H | H | Me | 6-Cl-1,3-benzothiazol-2-yl |
| 10-243 | Me | H | H | Me | 6-Br-1,3-benzothiazol-2-yl |
| 10-244 | Et | H | H | Me | 6-Br-1,3-benzothiazol-2-yl |
| 10-245 | Et | H | H | Me | 4-OMe-pyridin-2-yl |
| 10-246 | Et | H | H | Me | 4-F-pyridin-2-yl |
| 10-247 | Et | H | H | Me | 4-Cl-pyridin-2-yl |
| 10-248 | Et | H | H | Me | 4-Br-pyridin-2-yl |
| 10-249 | Me | H | H | Me | 6-Br-pyridin-3-yl |
| 10-250 | Et | H | H | Me | 6-Br-pyridin-3-yl |
| 10-251 | Me | H | H | Me | 4-Cl-3-thienyl |
| 10-252 | Me | H | H | Me | 4-Br-3-thienyl |
| 10-253 | Me | H | H | Me | 4-Me-3-thienyl |
| 10-254 | Me | H | H | Me | 4-Thiazolyl |
| 10-255 | Me | H | H | Me | 5-Thiazolyl |
| 10-256 | Me | H | H | Me | 2-Me-thiazol-4-yl |
| 10-257 | Me | H | H | Me | 2-Me-thiazol-5-yl |
| 10-258 | Me | H | H | Me | 5-Cl-3-thienyl |
| 10-259 | Me | H | H | Me | 5-Br-3-thienyl |
| 10-260 | Me | H | H | Me | 5-Me-3-thienyl |
| 10-261 | Me | H | H | Me | 2-F-Ph |
| 10-262 | Me | H | H | Me | 2-CN-Ph |
| 10-263 | Me | H | H | Me | 2-NO₂-Ph |
| 10-264 | Me | H | H | Me | 2,4-F₂-Ph |
| 10-265 | Me | H | H | Me | 3,4-F₂-Ph |
| 10-266 | Me | H | H | Me | 1-Me-pyrazol-3-yl |
| 10-267 | Me | H | H | Me | 4-(Me-CO)-Ph |
| 10-268 | Me | H | H | Me | 5-l-pyridin-2-yl |
| 10-269 | Me | H | H | Me | 5-l-pyrimidin-2-yl |
| 10-270 | Me | H | H | Me | 2-Cl-thiazol-4-yl |
| 10-271 | Me | H | H | Me | 2-Br-thiazol-4-yl |
| 10-272 | Me | H | H | Me | 5-OSO₂Me-pyridin-2-yl |
| 10-273 | Me | H | H | Me | 1,3-Benzoxazol-2-yl |
| 10-274 | Me | H | H | Me | 6-Cl-1,3-benzoxazol-2-yl |
| 10-275 | Me | H | H | Me | 6-Br-1,3-benzoxazol-2-yl |
| 10-276 | Me | H | H | Me | 7-Cl-1,3-benzoxazol-2-yl |
| 10-277 | Me | H | H | Me | 5-NH₂-pyridin-2-yl |
| 10-278 | Me | H | H | Me | 5-OH-pyridin-2-yl |
| 10-279 | Me | H | H | Me | 5-OCHF₂-pyridin-2-yl |
| 10-280 | Me | H | H | Me | 5-MeO-pyridin-2-yl |
| 10-281 | Me | H | H | Me | 5-MeS-pyridin-2-yl |
| 10-282 | Me | H | H | Me | 5-NHMe-pyridin-2-yl |
| 10-283 | Me | H | H | Me | 5-NMe₂-pyridin-2-yl |
| 10-284 | Et | H | H | Me | 4-Cl-3-thienyl |
| 10-285 | Et | H | H | Me | 4-Br-3-thienyl |
| 10-286 | Et | H | H | Me | 4-Me-3-thienyl |
| 10-287 | Et | H | H | Me | 4-Thiazolyl |
| 10-288 | Et | H | H | Me | 5-Thiazolyl |
| 10-289 | Et | H | H | Me | 2-Me-thiazol-4-yl |
| 10-290 | Et | H | H | Me | 2-Me-thiazol-5-yl |
| 10-291 | Et | H | H | Me | 5-Cl-3-thienyl |
| 10-292 | Et | H | H | Me | 5-Br-3-thienyl |
| 10-293 | Et | H | H | Me | 5-Me-3-thienyl |
| 10-294 | Et | H | H | Me | 2-F-Ph |
| 10-295 | Et | H | H | Me | 2-CN-Ph |
| 10-296 | Et | H | H | Me | 2-NO₂-Ph |
| 10-297 | Et | H | H | Me | 2,4-F₂-Ph |
| 10-298 | Et | H | H | Me | 3,4-F₂-Ph |
| 10-299 | Et | H | H | Me | 1-Me-pyrazol-3-yl |
| 10-300 | Et | H | H | Me | 3,5-Cl₂-Ph |
| 10-301 | Et | H | H | Me | 3,4-Me₂-Ph |
| 10-302 | Et | H | H | Me | 4-(Me-CO)-Ph |
| 10-303 | Et | H | H | Me | 5-CF₃-pyridin-2-yl |
| 10-304 | Et | H | H | Me | 6-OMe-pyridin-2-yl |
| 10-305 | Et | H | H | Me | 2-Me-pyridin-4-yl |
| 10-306 | Et | H | H | Me | 4-Cl-6-Me-pyridin-2-yl |
| 10-307 | Et | H | H | Me | 4-Br-3-Me-Ph |
| 10-308 | Et | H | H | Me | 5-Cl-pyridin-3-yl |
| 10-309 | Et | H | H | Me | 5-Allyl-pyridin-2-yl |
| 10-310 | Et | H | H | Me | 5-cyclopropyl-pyridin-2-yl |
| 10-311 | Et | H | H | Me | 5-Ethinyl-pyridin-2-yl |
| 10-312 | Et | H | H | Me | 5-Ph-pyridin-2-yl |
| 10-313 | Et | H | H | Me | 5-l-pyridin-2-yl |
| 10-314 | Et | H | H | Me | 5-l-pyrimidin-2-yl |
| 10-315 | Et | H | H | Me | 2-Cl-thiazol-4-yl |
| 10-316 | Et | H | H | Me | 2-Br-thiazol-4-yl |
| 10-317 | Et | H | H | Me | 5-OSO₂Me-pyridin-2-yl |
| 10-318 | Et | H | H | Me | 1,3-Benzoxazol-2-yl |
| 10-319 | Et | H | H | Me | 6-Cl-1,3-benzoxazol-2-yl |
| 10-320 | Et | H | H | Me | 6-Br-1,3-benzoxazol-2-yl |
| 10-321 | Et | H | H | Me | 7-Cl-1,3-benzoxazol-2-yl |
| 10-322 | Et | H | H | Me | 5-NH₂-pyridin-2-yl |
| 10-323 | Et | H | H | Me | 5-OH-pyridin-2-yl |
| 10-324 | Et | H | H | Me | 5-OCHF₂-pyridin-2-yl |
| 10-325 | Et | H | H | Me | 5-MeO-pyridin-2-yl |
| 10-326 | Et | H | H | Me | 5-MeS-pyridin-2-yl |
| 10-327 | Et | H | H | Me | 5-NHMe-pyridin-2-yl |
| 10-328 | Et | H | H | Me | 5-NMe₂-pyridin-2-yl |
| 10-329 | Et | H | H | Me | 4-NO₂-Ph |

### (B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man 75 Gewichtsteile einer Verbindung der Formel (I),

| | |
|---|---|
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I),

| | |
|---|---|
| 5 " | 2,2'-dinaphthylmethan-6,6'-disulfonsaures Natrium |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### (C) Biologische Beispiele

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten erfindungsgemäßen Verbindungen (I) werden dann als wäßrige Suspension bzw. Emulsion mit einer Wasseraufwandmenge von umgerechnet 600 l/ha unter Zusatz von 0,2% Netzmittel auf die Oberfläche der Abdeckerde appliziert.
Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Erfindungsgemäße Verbindungen (I), wie beispielsweise die Verbindungen Nr. 1-67, 1-119, 2-2, 2-28, 2-68, 2-147, 2-116, 2-120, 5-2, 5-28, 5-115, 5-116, 5-119, 5-131, 5-216, 5-227, 5-238, 5-239, 5-240, 5-241, 7-29, 8-2, 8-4, 8-16, 8-28, 8-32, 8-64, 8-69, 8-117, 8-118, 8-121, 8-133, 8-217, 8-219, 8-229, 8-230, 8-239, 8-240, 8-241, 8-276, 8-294, 9-4 und 9-122, weisen eine gute herbizide Vorauflaufwirksamkeit gegen mehrere Schadpflanzen bei einer Aufwandmenge von 1 kg oder weniger Aktivsubstanz pro Hektar auf. Weiterhin haben Verbindungen aus Tabelle 1 bis 9, wie beispielsweise die Verbindung Nr. 8-217 sehr gute herbizide Wirkung (80% und mehr herbizide Wirkung) gegen Schadpflanzen wie Alopecurus myosuroides, Avena fatua, Echinochloa crus galli und Setaria viridis im Vorauflaufverfahren bei einer Aufwandmenge von 1,0 kg oder 0,32 kg Aktivsubstanz pro Hektar. Andere Verbindungen aus Tabelle 1 bis 9 wie beispielsweise die Verbindung Nr. 9-4 oder 8-229 weisen sehr gute herbizide Wirkung (70% und mehr) gegen Schadpflanzen wie Stellaria media, Veronica persica und Viola tricolor im Vorauflaufverfahren bei einer Aufwandmenge von 1,0 kg oder 0,32 kg Aktivsubstanz pro Hektar auf. Andere Verbindungen aus Tabelle 1 bis 9 wie beispielsweise die Verbindung Nr. 9-118 oder 8-121 weisen sehr gute herbizide Wirkung (70% und mehr) gegen Schadpflanzen wie Echinochloa crus-galli und Setaria viridis im Vorauflaufverfahren bei einer Aufwandmenge von 1,0 kg oder 0,32 kg Aktivsubstanz pro Hektar auf. In der Regel besitzen diese und andere Wirkstoffe aus den Tabellen 1 bis 9 aber noch ein wesentlich breiteres Wirkstoffspektrum.

Gleichzeitig lassen erfindungsgemäße Verbindungen zweikeimblättrige Kulturen wie Raps im Vorauflaufverfahren selbst bei hohen Wirkstoffdosierungen ungeschädigt und schonen darüber hinaus auch Gramineen-Kulturen wie Weizen, Mais und Reis. Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen.

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) formulierten erfindungsgemäßen Verbindungen (I) werden dann als wäßrige Suspension bzw. Emulsion mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen (I), wie beispielsweise die Verbindungen Nr. 1-67, 1-119, 2-2, 2-28, 2-68, 2-147, 2-116, 2-120, 5-2, 5-28, 5-115, 5-116, 5-119, 5-131, 5-216, 5-227, 5-238, 5-239, 5-240, 5-241, 7-29, 8-2, 8-4, 8-16, 8-28, 8-32, 8-64, 8-69, 8-117, 8-118, 8-121, 8-133, 8-217, 8-219, 8-229, 8-230, 8-239, 8-240, 8-241, 8-276, 8-294, 9-4 und 9-122 aus den Tabellen 1 bis 9, eine gute herbizide Nachauflaufwirksamkeit gegen ein breites Spektrum von Ungräsern und Unkräutern bei einer Aufwandmenge von 1 kg und weniger Aktivsubstanz pro Hektar auf. Weiterhin haben Verbindungen aus Tabelle 1 bis 9, wie beispielsweise die Verbindungen Nr. 7-29, 8-16, 8-28, 8-69, 8-117, 8-118, 8-121, 8-133, 8-217, 8-229, 8-230, 8-139, 8-240, 8-294, 9-4 und 9-122, sehr gute herbizide Wirkung (80% und mehr) gegen Schadpflanzen wie Avena fatua, Echinochloa crus galli und Setaria viridis bei 0,32 kg Aktivsubstanz pro Hektar. Weiterhin haben Verbindungen aus Tabelle 1 bis 9, wie z. B. die Verbindungen Nr. 8-2, 8-16, 8-28, 8-69, 8-117, 8-118, 8-121, 8-133, 8-217, 8-219, 8-229, 8-239, und 8-294 sehr gute herbizide Wirkung (80% und mehr) gegen Alopecurus myosuroides, jeweils im Nachauflaufverfahren bei einer Aufwandmenge von jeweils 0,32 kg Aktivsubstanz pro Hektar. Weiterhin haben Verbindungen aus Tabelle 1 bis 9, wie beispielseweise die Verbindungen Nr. 8-117, 8-118, 8-121, 8-217, 8-219, 8-229, 8-230, 8-239, 8-240 und 8-294 sehr gute herbizide Wirkung (80% und mehr) gegen Lolium multiflorum, jeweils im Nachauflaufverfahren bei einer Aufwandmenge von jeweils 0,32 kg Aktivsubstanz pro Hektar. Weiterhin haben Verbindungen aus Tabelle 1 bis 9, wie beispielseweise die Verbindungen Nr. 8-117, 8-118, 8-121, 8-229 und 8-240, sehr gute herbizide Wirkung (80% und mehr) gegen Veronica persica, jeweils im Nachauflaufverfahren bei einer Aufwandmenge von jeweils 0,32 kg Aktivsubstanz pro Hektar. Weiterhin haben Verbindungen aus Tabelle 1 bis 9, wie beispielseweise die Verbindungen Nr. 8-2, 8-16, 8-69, 8-117, 8-118, 8-121 und 8-229, sehr gute herbizide Wirkung (80% und mehr) gegen Viola tricolor, jeweils im Nachauflaufverfahren bei einer Aufwandmenge von jeweils 0,32 kg Aktivsubstanz pro Hektar.ln der Regel besitzen diese und andere Wirkstoffe aus den Tabellen 1 bis 9 aber noch ein wesentlich breiteres Wirkstoffspektrum.

## Patentansprüche

1. Verbindungen der Formel (I) oder deren Salze, 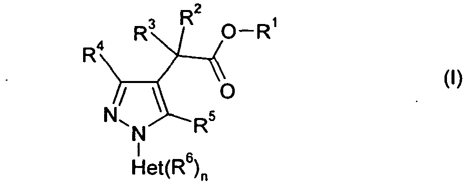 worin
Het einen fünfgliedrigen heteroaromatischen Rest mit zwei Heteroatomen als Ringatome, wobei eines der Heteroatome im Ring ein Stickstoffatom und das andere ein Schwefelatom ist und das Stickstoffatom im Ring in 1,3-Stellung zum Ring-C-atom steht, das am Pyrazolrest gebunden ist,
R¹ Wasserstoff oder einen hydrolysierbaren Rest,
R² Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist,
R³ Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist, oder
R² und R³ zusammen mit dem C-Atom, an das sie gebunden sind, einen carbocyclischen gesättigten oder teilweise ungesättigten Ring mit 3 bis 6 C-Atomen, der unsubstituiert oder durch einen oder mehrere Reste aus der Gruppe Halogen und (C₁-C₄)Alkyl substituiert ist, und
R⁴ Wasserstoff, Halogen, Cyano, (C₁-C₆)Alkyl, (C₂-C₆)Alkenyl oder (C₂-C₆)Alkinyl, wobei jeder der letztgenannten drei Reste unsubstituiert oder durch einen oder mehrere Reste aus der Gruppe bestehend aus Halogen, Cyano, Hydroxy, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₉)Cycloalkyl substituiert ist, oder
(C₃-C₉)Cycloalkyl, (C₅-C₉)Cycloalkenyl oder (C₅-C₉)Cycloalkinyl, wobei jeder der 3 letztgenannten Reste unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, oder
Phenyl, das unsubstituiert oder durch einen oder mehrere Reste aus der Gruppe bestehend aus Halogen, Cyano, Nitro, Carboxy, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₁-C₄)Alkanoyl, (C₁-C₄)Haloalkanoyl, [(C₁-C₄)Alkoxy]-carbonyl und [(C₁-C₄)Haloalkoxy]-carbonyl substituiert ist, oder
(C₁-C₆)Alkanoyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₁-C₄)Alkoxy]-carbonyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₃-C₉)Cycloalkoxy]-carbonyl, das unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist,
R⁵ einen Phenylrest oder einen 5- oder 6-gliedrigen heteroaromatischen Rest mit 1 bis 3 Heteroringatomen aus der Gruppe N, O und S, wobei der Phenylrest oder der heterocyclische Rest unsubstituiert oder durch einen oder mehrere Reste aus der Gruppe bestehend aus den Resten
(a) Halogen, Hydroxy, Amino, Nitro, Carboxy, Cyano und Carbamoyl,
(b) (C₁-C₆)Alkyl, (C₂-C₆)Alkenyl, (C₂-C₆)Alkinyl, (C₁-C₆)Alkoxy, (C₁-C₆)Alkenyloxy und (C₁-C₆)Alkinyloxy, wobei jeder der letztgenannten 6 Reste unsubstituiert oder durch einen oder mehrere Reste aus der Gruppe Halogen, (C₁-C₄)Alkoxy, (C₁-C₆)Haloalkoxy, (C₁-C₄)Alkylthio, Mono- und Di-[(C₁-C₄)alkyl]-amino, Hydroxy, Carboxy, [(C₁-C₄)Alkoxyl-carbonyl, [(C₁-C₄)Haloalkoxy]-carbonyl, Mono- und Di-[(C₁-C₄)alkyl]-aminocarbonyl und Cyano substituiert ist,
(c) (C₁-C₆)Alkylthio, [(C₁-C₆)Alkoxy]-carbonyl, [(C₁-C₆)Haloalkoxy] carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₁-C₆)Alkylsulfinyloxy, (C₁-C₆)Haloalkylsulfinyloxy, (C₁-C₆)Alkylsulfonyloxy, (C₁-C₆)Haloalkylsulfonyloxy, (C₁-C₆)Alkylsulfato, (C₁-C₆)Haloalkylsulfato und
(d) (C₃-C₆)Cycloalkyl, (C₃-C₆)Cycloalkyloxy, Phenyl und Phenoxy, wobei jeder der letztgenannten 4 Reste unsubstituiert oder durch einen oder mehrere Reste aus der Gruppe bestehend aus Halogen, (C₁-C₄)Alkyl, (C₁-C₄)Haloalkyl, (C₁-C₄)Alkoxy, (C₁-C₆)Haloalkoxy und (C₁-C₄)Alkylthio substituiert ist, substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₆)Haloalkoxy und (C₁-C₄)Alkylthio substituiert ist, wobei der Phenylrest inklusive Substituenten 6 bis 30 C-Atome aufweist und der heteroaromatische Rest inklusive Substituenten 1 bis 30 C-Atome aufweist, und
(R⁶)ₙ n Substituenten R⁶, wobei R⁶, im Falle dass n = 1 ist, oder jeder der Substituenten R⁶ unabhängig voneinander, im Falle dass n größer als 1 ist, einen Rest Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- oder Di-[(C₁-C₄)alkyl]-aminoalkyl, Hydroxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₁-C₆)Haloalkoxy, (C₁-C₄)Alkoxy-(C₁-C₄)alkoxy, (C₁-C₆)Alkylthio, (C₁-C₆)Haloalkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- oder Di-[(C₁-C₄)alkyl]-aminocarbonyl, Mono- oder Di-[(C₁-C₆)acyl]-amino, Mono- oder Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₉)Cycloalkyl oder (C₅-C₉)Cycloalkenyl, wobei jeder der letztgenannten beiden Reste unsubstituiert oder durch einen oder mehrere Reste aus der Gruppe bestehend aus Halogen, (C₁-C₄)Alkyl und (C₁-C₄)Haloalkyl substituiert ist, bedeutet, und
n 0, 1, oder 2
bedeuten.

2. Verbindungen oder deren Salze gemäß Anspruch 1, **dadurch gekennzeichnet, dass**
R¹ Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, aufweist, oder
einen Rest der Formel SiR^{a}R^{b}R^{c}, -NR^{a}R^{b} oder -N=CR^{c}R^{d}, wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet oder R^{a} und R^{b} zusammen mit dem N-Atom einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten,
wobei jeder der Reste R^{a}, R^{b}, R^{c} und R^{d} inklusive Substituenten bis 30 C-Atome aufweist.

3. Verbindungen oder deren Salze gemäß Anspruch 1, **dadurch gekennzeichnet, dass**
R¹ Wasserstoff, Alkyl, Alkenyl, Alkinyl, Cycloalkyl, Cycloalkenyl, Cycloalkinyl oder Aryl, wobei jeder der letztgenannten 7 Reste unsubstituiert oder substituiert ist und inklusive Substituenten bis zu 30 C-Atome aufweist, oder einen Heterocyclylrest mit 3 bis 9 Ringatomen, der 1 bis 4 Heteroatome aus der Gruppe N, O und S enthält und der unsubstituiert oder oder substituiert ist und der inklusive Substituenten 1 bis 30 C-Atome aufweist, bedeutet.

4. Verbindungen oder deren Salze gemäß Anspruch 1, **dadurch gekennzeichnet, dass**
R¹ H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, Carboxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, die letztgenannten 7 Reste nur im Falle cyclischer Basisreste, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, Reste der Formeln -NR*R**, -CO-NR*R** und -O-CO-NR*R**,
wobei jeder der Reste R* und R** in den letztgenannten 3 Formeln unabhängig voneinander H, (C₁-C₈)Alkyl, (C₂-C₈)Alkenyl, (C₂-C₈)Alkinyl, Benzyl, substituiertes Benzyl, Phenyl oder substituiertes Phenyl, oder zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeutet,
und [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl, wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist,
und Phenyl, Phenyl-(C₁-C₆)alkoxy, Phenyl-[(C₁-C₆)alkoxy]-carbonyl, Phenoxy, Phenoxy-(C₁-C₆)alkoxy, Phenoxy-[(C₁-C₆)alkoxy]-carbonyl, Phenoxycarbonyl, Phenylcarbonyloxy, Phenoxycarbonyloxy, Phenylcarbonylamino, Phenyl-[(C₁-C₆)alkyl]-carbonylamino, Phenyl-[(C1-C6)alkyl]-carbonyloxy, Phenyl-[(C₁-C₆)alkoxy]-carbonyloxy, (C₃-C₇)Cycloalkyl, (C₃-C₇)Cycloalkoxy, (C₃-C₆)Cycloalkyl-(C₁-C₆)alkoxy, (C₃-C₆)Cycloalkyl-[(C₁-C₆)alkoxy]-carbonyl, (C₃-C₆)Cycloalkoxy-(C₁-C₆)alkoxy, (C₃-C₆)Cycloalkoxy-[(C₁-C₆)alkoxy]-carbonyl, (C₃-C₆)Cycloalkoxy-carbonyl, (C₃-C₆)Cycloalkyl-carbonyloxy, (C₃-C₆)Cycloalkoxy-carbonyloxy, (C₃-C₆)Cycloalkyl-[(C₁-C₆)alkoxy]-carbonyloxy, (C₃--C₆)Cycloalkyl-carbonylamino, (C₃-C₆)Cycloalkyl-[(C₁-C₆)alkyl]-carbonylamino und (C₃-C₆)Cycloalkyl-[(C₁-C₆)alkyl]-carbonyloxy,
wobei jeder der letztgenannten 26 Reste gegebenenfalls mit einem carbocyclischen Ring mit 3 bis 6 C-Atomen oder einem hetereocyclischen Ring mit 5 oder 6 Ringatomen und 1 bis 3 Heteroringatomen aus der Gruppe N, O und S kondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)m-CH(OR')₂,
in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR'"CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten,
und Reste der Formel Het¹, wobei Het¹ jeweils unabhängig voneinander einen gesättigten, teilweise ungesättigten oder heteroaromatischen Heterocyclylrest mit 3 bis 9 Ringatomen bedeutet, wobei der jeweilige heterocyclische Rest 1 bis 4 Heteroatome aus der Gruppe N, O und S enthält und gegebenenfalls mit einem carbocyclischen Ring mit 3 bis 6 C-Atomen oder einem hetereocyclischen Ring mit 5 oder 6 Ringatomen und 1 bis 3 Heteroringatomen aus der Gruppe N, O und S kondensiert ist, und im Ring oder im mehrcyclischen System unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
substituiert ist,
oder
R¹ einen mehrcyclischen Rest auf Basis von (C₃-C₉)Cycloalkyl, (C₅-C₉)Cycloalkenyl, (C₅-C₉)Cycloalkinyl oder Phenyl, wobei der Basisring mit einem 5- oder 6-gliedrigen Ring mit 0 oder 1 bis 3 Heteroringatomen aus der Gruppe N, O und S kondensiert ist, und wobei der Basisring oder das mehrcyclische System unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist, oder
R¹ einen gesättigten, teilweise ungesättigten oder heteroaromatischen Heterocyclylrest mit 3 bis 9 Ringatomen, vorzugsweise mit 5 oder 6 Ringatomen, der 1 bis 4 Heteroatome, vorzugsweise 1 bis 3 Heteroringatome aus der Gruppe N, O und S enthält, gegebenenfalls mit einem carbocyclischen oder heterocyclischen Ring, vorzugsweise einem 5- oder 6-gliedrigen Ring mit o oder 1 bis 3 Heteroringatomen aus der Gruppe N, O und S kondensiert ist, vorzugsweise benzokondensiert ist, und der im Ring oder im mehrcyclischen System unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-Ca)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxyl-carbonyl und Oxo substituiert ist,
bedeutet.

5. Verbindungen oder deren Salze gemäß Anspruch 1, **dadurch gekennzeichnet, dass**
R¹ H, (C₁-C₁₈)Alkyl, (C₂-C₁₈)Alkenyl, (C₂-C₁₈)Alkinyl, (C₃-C₉)cycloalkyl, (C₅-C₉)Cycloalkenyl, (C₅-C₉)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, die letztgenannten 7 Reste nur im Falle cyclischer Basisreste, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, Reste der Formeln -NR*R**, -CO-NR*R** und -O-CO-NR*R**,
wobei jeder der Reste R* und R** in den letztgenannten 3 Formeln unabhängig voneinander H, (C₁-C₈)Alkyl, (C₂-C₈)Alkenyl, (C₂-C₈)Alkinyl, Benzyl, substituiertes Benzyl, Phenyl oder substituiertes Phenyl, oder zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeutet,
und [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist,
und Phenyl, Phenyl-(C₁-C₆)alkoxy, Phenyl-[(C₁-C₆)alkoxy]-carbonyl, Phenoxy, Phenoxy-(C₁-C₆)alkoxy, Phenoxy-[(C₁-C₆)alkoxy]-carbonyl, Phenoxycarbonyl, Phenylcarbonyloxy, Phenylcarbonylamino, Phenyl-[(C₁-C₆)alkyl]-carbonylamino, Phenyl-[(C₁-C₆)alkyl]-carbonyloxy, (C₃-C₇)Cycloalkyl und (C₃-C₇)Cycloalkoxy,
wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)ₘ-CH(OR')₂,
in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR'"CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten, substituiert ist,
bedeutet.

6. Verbindungen oder deren Salz gemäß einem der Ansprüche 1 bis 5, **dadurch gekennzeichnet, dass**
R² Wasserstoff, Halogen oder (C₁-C₄)Alkyl, das unsubstituiert oder durch einen oder mehrere Reste aus der Gruppe Halogen substituiert ist, bedeutet und
R³ Wasserstoff, Halogen oder (C₁-C₄)Alkyl, das unsubstituiert oder durch einen oder mehrere Reste aus der Gruppe Halogen substituiert ist, bedeutet oder
R² und R³ zusammen mit dem C-Atom, an das sie gebunden sind, (C₃-C₆)Cycloalkyl oder (C₅-C₆)Cycloalkenyl, wobei jeder der letztgenannten 2 Reste unsubstituiert oder durch einen oder mehrere Reste aus der Gruppe Halogen und (C₁-C₄)Alkyl substituiert ist, bedeuten.

7. Verbindungen oder deren Salze gemäß einem der Ansprüche 1 bis 6, **dadurch gekennzeichnet, dass**
R⁴ Wasserstoff, Halogen, Cyano, (C₁-C₄)Alkyl, (C₂-C₄)Alkenyl oder (C₂-C₄)Alkinyl, wobei jeder der letztgenannten drei Reste unsubstituiert oder durch einen oder mehrere Reste aus der Gruppe bestehend aus Halogen und Hydroxy substituiert ist, vorzugsweise unsubstituiert oder durch einen oder mehrere Reste aus der Gruppe Halogen, wie Fluor und Chlor substituiert ist, oder
(C₃-C₆)Cycloalkyl, das unsubstituiert oder durch einen oder mehrere Reste aus der Gruppe bestehend aus Halogen und (C₁-C₄)Alkyl substituiert ist, oder
Phenyl, das unsubstituiert oder durch einen oder mehrere Reste aus der Gruppe bestehend aus Halogen, Nitro, (C₁-C₄)Alkyl, (C₁-C₄)Haloalkyl, (C₁-C₄)Alkoxy, (C₁-C₄)Haloalkoxy, (C₁-C₄)Alkoxy-(C₁-C₄)alkoxy, (C₁-C₄)Alkylthio, [(C₁-C₄)Alkoxy]-carbonyl und [(C₁-C₄)Haloalkoxy]-carbonyl substituiert ist, oder
(C₁-C₄)Alkanoyl, das unsubstituiert oder durch einen oder mehrere Reste aus der Gruppe bestehend aus Halogen, wie Fluor und Chlor, Cyano, (C₁-C₄)Alkoxy, (C₁-C₄)Haloalkoxy und (C₁-C₂)Alkoxy-(C₁-C₂)alkoxy substituiert ist, vorzugsweise Formyl, oder
[(C₁-C₄)Alkoxy]-carbonyl, das unsubstituiert oder durch einen oder mehrere Reste aus der Gruppe bestehend aus Halogen, wie Fluor und Chlor, substituiert ist, oder
[(C₃-C₆)Cycloalkoxy]-carbonyl, das unsubstituiert oder durch einen oder mehrere Reste aus der Gruppe bestehend aus Halogen und (C₁-C₄)Alkyl substituiert ist,
bedeutet.

8. Verbindungen oder deren Salze gemäß einem der Ansprüche 1 bis 7, **dadurch gekennzeichnet, dass**
R⁵ Phenyl, das unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- und Di-[(C₁-C₄)alkyl]-aminoalkyl, Hydroxy-(C₁-C₄)alkyl, Carboxy-(C₁-C₄)alkyl, Cyano-(C₁-C₄)alkyl, (C₁-C₆)Alkoxy, das gegebenenfalls halogeniert sein kann [= (C₁-C₆)Haloalkoxy)], (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist, substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
oder
R⁵ einen 5- oder 6-gliedrigen heteroaromatischen Rest mit 1 bis 3 Heteroringatomen aus der Gruppe N, O und S, der unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- und Di-[(C₁-C₄)alkyl]-aminoalkyl, Hydroxy-(C₁-C₄)alkyl, Carboxy-(C₁-C₄)alkyl, Cyano-(C₁-C₄)alkyl, (C₁-C₆)Alkoxy, das gegebenenfalls halogeniert sein kann [= (C₁-C₆)Haloalkoxy], (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist, substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
bedeutet.

9. Verbindungen oder deren Salze gemäß einem der Ansprüche 1 bis 8, **dadurch gekennzeichnet, dass**
(R⁶)ₙ n Substituenten R⁶, wobei R⁶, im Falle dass n = 1 ist, oder jeder der Substituenten R⁶ unabhängig voneinander, im Falle dass n größer als 1 ist, einen Rest Halogen, Cyano, (C₁-C₄)Alkyl, (C₁-C₄)Haloalkyl, (C₁-C₄)Alkoxy, (C₁-C₄)Alkylthio, (C₁-C₄)Alkylsulfinyl, (C₁-C₄)Haloalkylsulfinyl, (C₁-C₄)Alkylsulfonyl, (C₁-C₄)Alkoxycarbonyl oder (C₁-C₄)Haloalkylsulfonyl bedeutet, und
n 0, 1, oder 2
bedeuten.

10. Verfahren zur Herstellung einer Verbindung der Formel (I), wie sie gemäß einem der Ansprüche 1 bis 9 definiert ist, oder deren Salz, **dadurch gekennzeichnet, dass** man
(a) eine Verbindung der Formel (II),
H₂N-NH-Het(R⁶)ₙ (II)
worin Het und (R⁶)ₙ wie in Formel (I) definiert ist,
mit einer Verbindung der Formel (III), 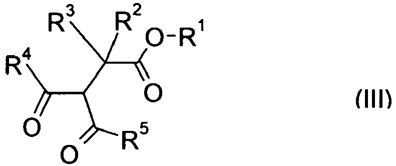 worin R¹, R², R³, R⁴ und R⁵ wie in Formel (I) definiert sind,
zur Verbindung der Formel (I) oder deren Salz umsetzt,
(b) im Falle, dass R¹ in Formel (I) von Wasserstoff verschieden ist, eine Verbindung der Formel (I'), 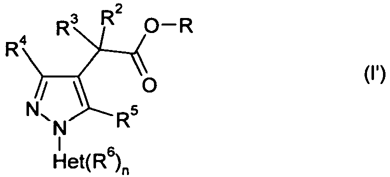 in welcher Het, R², R³, R⁴, R⁵ und R⁶ wie in Formel (I) definiert sind und
R einen vom Rest R¹ unterschiedlichen, von Wasserstoff verschiedenen Rest, der aus der Gruppe der Reste, wie sie für R¹ definiert ist, ausgewählt ist, oder ein Anhydrid, Säurehalogenid oder einen aktivierten Ester der Verbindung der Formel (I'), worin R = H bedeutet, bedeutet,
mit einer Verbindung der Formel (IV),
R¹ - OH (IV)
worin R¹ wie in Formel (I) definiert ist,
zur Verbindung der Formel (I) umsetzt
oder
(c) im Falle, dass R¹ in Formel (I) von Wasserstoff verschieden ist, eine Verbindung der Formel (I") 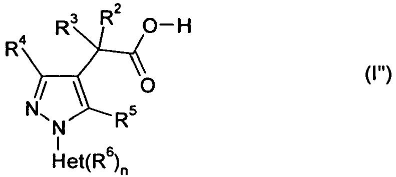 in welcher Het, R², R³, R⁴, R⁵ und R⁶ wie in Formel (I) definiert sind mit einer Verbindung der Formel (IV),
R¹ - OH (IV)
worin R¹ wie in Formel (I) definiert ist,
zur Verbindung der Formel (I) verestert
oder
(d) im Falle, dass die Verbindung der Formel (I), worin R¹ = H, oder deren Salz hergestellt wird, eine Verbindung der Formel (I') [siehe Definition in Variante (b)] zur Verbindung der Formel (I) oder deren Salz hydrolysiert, oder
g) eine Verbindung der allgemeinen Formel (XI) mit einem Bor-Derivat der Formel (XII) in Gegenwart eines Cu(I) oder Cu(II) Salzes und einer organischen Base umsetzt, 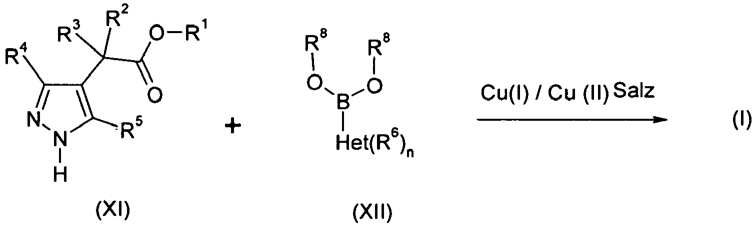 wobei in den Formeln (XI) und (XII) Het, R¹, R², R³, R⁴, R⁵, R⁶, die gemäß Formel (I) angegebenen Bedeutungen haben, und R⁸ gleich H oder (C₁-C₆)-Alkyl oder beide Alkylreste R⁸ zyklisch miteinander verknüpft sind,
oder
h) eine Verbindung der allgemeinen Formel (XI) mit einer Verbindung der Formel (XIII), worin R⁶ die gemäß Formel (I) zuvor angegebene Bedeutung hat, in Gegenwart eines geeigneten Katalysator/Liganden-Systems, mit einer geeigneten Base und in einem geeigneten Lösemittel zur Verbindung der Formel (I) oder deren Salz umsetzt, 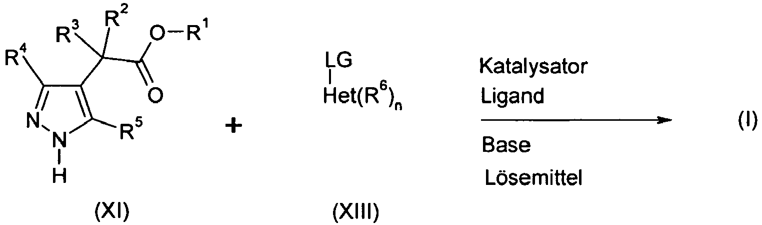 wobei in den Formeln (XI) und (XIII) die Reste Het, R¹, R², R³, R⁴, R⁵, R⁶, die gemäß Formel (I) angegebenen Bedeutungen haben und LG eine Abgangsgruppe bedeutet, oder
i) eine Verbindung der allgemeinen Formel (XV) mit einer Verbindung der allgemeinen Formel (III) in Gegenwart einer Säure zur Verbindung der Formel (I) oder deren Salz umsetzt, 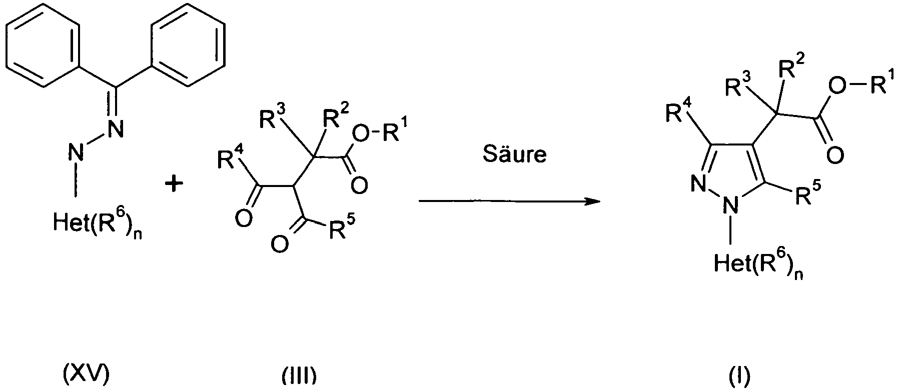 wobei in den Formeln (XV) und (III) die Reste Het, R¹, R², R³, R⁴, R⁵, R⁶ wie in Formel (I) definiert sind und LG eine Abgangsgruppe bedeutet,
oder
j) eine Verbindung der allgemeinen Formel (XVI), wobei R⁶ wie in Formel (I) definiert ist, mit Di-tert-butyl-azo-dicarboxylat (DBAD, XVII) in Gegenwart eines Kupfer-Salzes zu einer Verbindung der Formel (XVIII) umsetzt, worin R⁶ wie in Formel (I) definiert ist, welche anschließend über eine Verbindung der Formel (II) oder deren Salze, worin R⁶ wie in Formel (I) definiert ist, und gemäß Verfahren a) zu Verbindungen der Formel (I) umgesetzt wird: 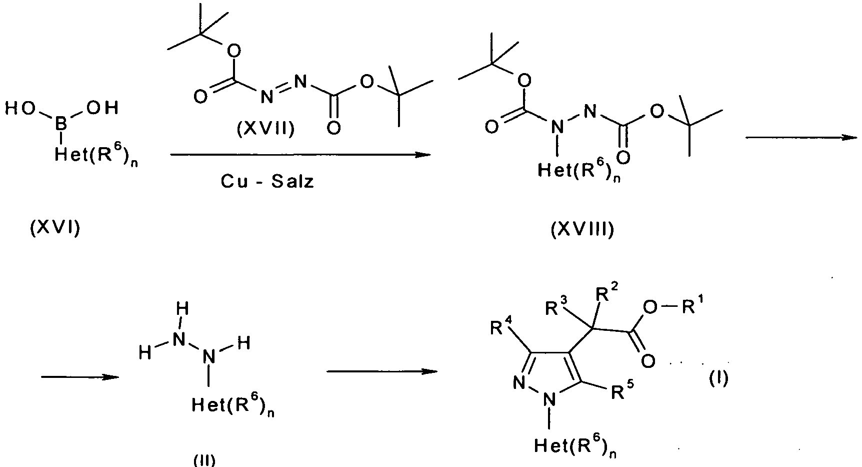 wobei in den Formeln (XVI), (XVII), (XVIII) und (II) die Reste Het, R¹, R², R³, R⁴, R⁵, R⁶ wie in Formel (I) definiert sind.

11. Herbizides oder pflanzenwachstumsregulierendes Mittel, **dadurch gekennzeichnet, dass** es eine oder mehrere Verbindungen der Formel (I) oder deren Salze, wie sie nach einem der Ansprüche 1 bis 9 definiert sind, und im Pflanzenschutz übliche Formulierungshilfsmittel enthält.

12. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen, wie sie nach einem der Ansprüche 1 bis 9 definiert sind, auf die Pflanzen, Pflanzensamen, den Boden, in dem oder auf dem die Pflanzen wachsen, oder die Anbaufläche appliziert.

13. Verfahren nach Anspruch 12, **dadurch gekennzeichnet, dass** die Verbindungen der Formel (I) oder deren Salze zur Bekämpfung von Schadpflanzen oder zur Wachstumsregulierung in Kulturen von Nutz- oder Zierpflanzen eingesetzt werden.

14. Verfahren nach Anspruch 13, **dadurch gekennzeichnet, dass** die Kulturpflanzen transgene Kulturpflanzen sind.

15. Verwendung der Verbindungen der Formel (I) oder deren Salze gemäß einem Ansprüche 1 bis 9 als Herbizide oder Pflanzenwachstumsregulatoren.

## Claims

1. A compound of the formula (I) or a salt thereof 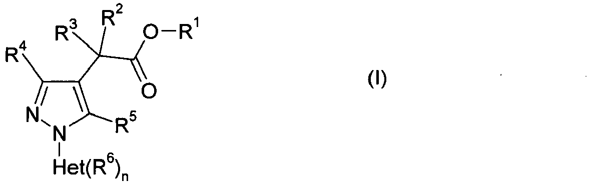 in which
Het is a five-membered heteroaromatic radical having two heteroatoms as ring atoms, where one of the heteroatoms in the ring is a nitrogen atom and the other is a sulfur atom and the nitrogen atom in the ring is located in the 1,3-position to the ring carbon atom attached to the pyrazole radical,
R¹ is hydrogen or a hydrolyzable radical,
R² is hydrogen, halogen or (C₁-C₆)-alkyl which is unsubstituted or substituted by one or more radicals from the group consisting of halogen, (C₁-C₄ )-alkoxy, (C₁-C₄)-alkylthio and (C₁-C₄)-haloalkoxy,
R³ is hydrogen, halogen or (C₁-C₆)-alkyl which is unsubstituted or substituted by one or more radicals from the group consisting of halogen, (C₁-C₄)-alkoxy, (C₁-C₄)-alkylthio and (C₁-C₄)-haloalkoxy, or
R² and R³ together with the carbon atom to which they are attached are a carbocyclic saturated or partially unsaturated ring having 3 to 6 carbon atoms which is unsubstituted or substituted by one or more radicals from the group consisting of halogen and (C₁-C₄)-alkyl, and
R⁴ is hydrogen, halogen, cyano, (C₁-C₆)-alkyl, (C₂-C₆)-alkenyl or (C₂-C₆)-alkynyl, where each of the three lastmentioned radicals is unsubstituted or substituted by one or more radicals from the group consisting of halogen, cyano, hydroxyl, (C₁-C₄)-alkoxy, (C₁-C₄)-haloalkoxy, (C₁-C₄) -alkoxy- (C₁-C₄)-alkoxy, (C₁-C₄)-alkylthio and optionally halogen-, cyano-, (C₁-C₄)-alkyl- or (C₁-C₄)-haloalkyl-substituted (C₃-C₉)-cycloalkyl, or
(C₃-C₉)-cycloalkyl, (C₅-C₉)-cycloalkenyl or (C₅-C₉)-cycloalkynyl, where each of the 3 lastmentioned radicals is unsubstituted or substituted by one or more radicals from the group consisting of halogen, (C₁-C₄)-alkyl, (C₁-C₄)-haloalkyl, (C₂-C₄)-alkenyl, (C₂-C₄)-alkynyl, (C₁-C₄)-alkoxy, (C₁-C₄)-haloalkoxy and (C₁-C₄)-alkylthio, or
phenyl which is unsubstituted or substituted by one or more radicals from the group consisting of halogen, cyano, nitro, carboxyl, (C₁-C₄)-alkyl, (C₁-C₄)-haloalkyl, (C₂-C₄)-alkenyl, (C₂-C₄)-alkynyl, (C₁-C₄)-alkoxy, (C₁-C₄)-haloalkoxy, (C₁-C₄) -alkoxy- (C₁-C₄)-alkoxy, (C₁-C₄)-alkylthio, (C₁-C₄)-alkanoyl, (C₁-C₄)-haloalkanoyl, [(C₁-C₄)-alkoxy] carbonyl and [(C₁-C₄)-haloalkoxy]carbonyl, or
(C₁-C₆)-alkanoyl which is unsubstituted or substituted by one or more radicals from the group consisting of halogen, cyano, (C₁-C₄)-alkoxy, (C₁-C₄)-haloalkoxy, (C₁-C₄)-alkoxy- (C₁-C₄)-alkoxy, (C₁-C₄)-alkylthio and optionally halogen-, cyano-, (C₁-C₄)-alkyl- or (C₁-C₄)-haloalkyl-substituted (C₃-C₆)-cycloalkyl, or
[(C₁-C₄)-alkoxy]carbonyl is unsubstituted or substituted by one or more radicals from the group consisting of halogen, cyano, (C₁-C₄)-alkoxy, (C₁-C₄)-haloalkoxy, (C₁-C₄)-alkoxy- (C₁-C₄)-alkoxy, (C₁-C₄)-alkylthio and optionally halogen-, cyano-, (C₁-C₄)-alkyl- or (C₁-C₄)-haloalkyl-substituted (C₃-C₆)-cycloalkyl, or
[(C₃-C₉)-cycloalkoxy]carbonyl which is unsubstituted or substituted by one or more radicals from the group consisting of halogen, (C₁-C₄)-alkyl, (C₁-C₄)-haloalkyl, (C₂-C₄)-alkenyl, (C₂-C₄)-alkynyl, (C₁-C₄)-alkoxy, (C₁-C₄)-haloalkoxy and (C₁-C₄)-alkylthio,
R⁵ is a phenyl radical or a 5- or 6-membered heteroaromatic radical having 1 to 3 hetero ring atoms from the group consisting of N, O and S, where the phenyl radical or the heterocyclic radical is unsubstituted or substituted by one or more radicals from the group consisting of the radicals
(a) halogen, hydroxyl, amino, nitro, carboxy, cyano and carbamoyl,
(b) (C₁-C₆)-alkyl, (C₂-C₆)-alkenyl, (C₂-C₆)-alkynyl, (C₁-C₆)-alkoxy, (C₁-C₆)-alkenyloxy and (C₁-C₆)-alkynyloxy, where each of the 6 lastmentioned radicals is unsubstituted or substituted by one or more radicals from the group consisting of halogen, (C₁-C₄)-alkoxy, (C₁-C₆)-haloalkoxy, (C₁-C₄)-alkylthio, mono-and di-[(C₁-C₄)-alkyl]amino, hydroxyl, carboxy, [(C₁-C₄)-alkoxy]carbonyl, [(C₁-C₄)-haloalkoxy] carbonyl, mono- and di-[(C₁-C₄)-alkyl]aminocarbonyl and cyano,
(c) (C₁-C₆)-alkylthio, [(C₁-C₆)-alkoxy] carbonyl, [(C₁-C₆)-haloalkoxy] carbonyl, (C₁-C₆)-alkanoyl, (C₁-C₆)-haloalkanoyl, mono- and di-[(C₁-C₄)-alkyl]aminocarbonyl, mono- and di-[(C₁-C₆)acyl]amino, mono- and di-[(C₁-C₄)-alkyl] amino, N-[(C₁-C₆)-acyl]-N-[(C₁-C₆)-alkyl] amino, (C₁-C₆)-alkylsulfinyl, (C₁-C₆)-haloalkylsulfinyl, (C₁-C₆)-alkylsulfonyl, (C₁-C₆)-haloalkylsulfonyl, (C₁-C₆)-alkylsulfinyloxy, (C₁-C₆)-haloalkylsulfinyloxy, (C₁-C₆)-alkylsulfonyloxy, (C₁-C₆)-haloalkylsulfonyloxy, (C₁-C₆)-alkylsulfato, (C₁-C₆)-haloalkylsulfato and
(d) (C₃-C₆)-cycloalkyl, (C₃-C₆)-cycloalkyloxy, phenyl and phenoxy, where each of the 4 lastmentioned radicals is unsubstituted or substituted by one or more radicals from the group consisting of halogen, (C₁-C₄)-alkyl, (C₁-C₄)-haloalkyl, (C₁-C₄)-alkoxy, (C₁-C₆)-haloalkoxy and (C₁-C₄)-alkylthio, and where two adjacent substituents may form a fused-on 5- or 6-membered ring which is carbocyclic or may also contain 1 to 3 hetero ring atoms from the group consisting of N, O and S and which is unsubstituted or substituted by one or more radicals from the group consisting of halogen, (C₁-C₄)-alkyl, (C₁-C₄)-haloalkyl, (C₁-C₄)-alkoxy, (C₁-C₆)-haloalkoxy and (C₁-C₄)-alkylthio,
where the phenyl radical, including substituents, has 6 to 30 carbon atoms and the heteroaromatic radical, including substituents, has 1 to 30 carbon atoms, and
(R⁶)ₙ are n substituents R⁶, where R⁶, in the case that n = 1, or each of the substituents R⁶ independently of the others, in the case that n is greater than 1, is a radical halogen, hydroxyl, amino, nitro, carboxy, cyano, carbamoyl, (C₁-C₆)-alkyl, (C₁-C₆)-haloalkyl, (C₁-C₄)-alkoxy- (C₁-C₄)-alkyl, (C₁-C₄)-alkylthio-(C₁-C₄)-alkyl, mono- or di-[(C₁-C₄)-alkyl]aminoalkyl, hydroxy-(C₁-C₄)-alkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₁-C₆)-haloalkoxy, (C₁-C₄)-alkoxy- (C₁-C₄)-alkoxy, (C₁-C₆)-alkylthio, (C₁-C₆)-haloalkylthio, [(C₁-C₆)-alkoxy]carbonyl, [(C₁-C₆)-haloalkoxy]carbonyl, (C₁-C₆)-alkanoyl, (C₁-C₆)-haloalkanoyl, mono- or di-[(C₁₋C₄)-alkyl]aminocarbonyl, mono- or di-[(C₁-C₆)acyl]amino, mono- or di-[(C₁-C₄)-alkyl] amino, N-[(C₁-C₆)-acyl]-N-[(C₁-C₆)-alkyl]amino, (C₁-C₆)-alkylsulfinyl, (C₁-C₆)-haloalkylsulfinyl, (C₁-C₆)-alkylsulfonyl, (C₁-C₆)-haloalkylsulfonyl, (C₃-C₉)-cycloalkyl or (C₅-C₉)-cycloalkenyl, where each of the two lastmentioned radicals is unsubstituted or substituted by one or more radicals from the group consisting of halogen, (C₁-C₄)-alkyl and (C₁-C₄)-haloalkyl, and
n is 0, 1, or 2.

2. The compound or a salt thereof as claimed in claim 1 wherein
R¹ is hydrogen or an optionally substituted hydrocarbon radical or an optionally substituted heterocyclyl radical, where each of the two lastmentioned carbon-containing radicals including substituents has 1 to 30 carbon atoms, or
a radical of the formula SiR^{a}R^{b}R^{c}, -NR^{a}R^{b} or -N=CR^{c}R^{d}, where in the 3 lastmentioned formulae each of the radicals R^{a}, R^{b}, R^{c} and R^{d} independently of the other is hydrogen or an optionally substituted hydrocarbon radical or R^{a} and R^{b} together with the nitrogen atom are a 3- to 9-membered heterocycle which in addition to the nitrogen atom may contain one or two further hetero ring atoms from the group consisting of N, O and S and which is unsubstituted or substituted, or R^{c} and R^{d} together with the carbon atom are a 3- to 9-membered carbocyclic radical or a heterocyclic radical which may contain 1 to 3 hetero ring atoms from the group consisting of N, O and S, where the carbocyclic or heterocyclic radical is unsubstituted or substituted, where each of the radicals R^{a}, R^{b}, R^{c} and R^{d} including substituents has up to 30 carbon atoms.

3. The compound or a salt thereof as claimed in claim 1 wherein
R¹ is hydrogen, alkyl, alkenyl, alkynyl, cycloalkyl, cycloalkenyl, cycloalkynyl or aryl, where each of the 7 lastmentioned radicals is unsubstituted or substituted and including substituents has up to 30 carbon atoms, or a heterocyclyl radical having 3 to 9 ring atoms which contains 1 to 4 heteroatoms from the group consisting of N, O and S and which is unsubstituted or substituted and which, including substituents, has 1 to 30 carbon atoms.

4. The compound or a salt thereof as claimed in claim 1 wherein
R¹ is H, (C₁-C₁₈)-alkyl, (C₂-C₁₈)-alkenyl, (C₂-C₁₈)-alkynyl, (C₃-C₉)-cycloalkyl, (C₅-C₉)-cycloalkenyl, (C₅-C₉)-cycloalkynyl or phenyl, where each of the 7 lastmentioned radicals is unsubstituted or substituted by one or more radicals from the group consisting of halogen, cyano, thio, nitro, hydroxyl, carboxy, (C₁-C₈)-alkyl, (C₁-C₈)-haloalkyl, (C₁-C₄)-alkoxy-(C₁-C₄)-alkyl, (C₂-C₈)-alkenyl, (C₂-C₈)-haloalkenyl, (C₂-C₈)-alkynyl, (C₂-C₈)-haloalkynyl, the 7 lastmentioned radicals only in the case of cyclic basic radicals, (C₁-C₈)-alkoxy, (C₂-C₈)-alkenyloxy, (C₂-C₈)-alkynyloxy, (C₁-C₈)-haloalkoxy, (C₁-C₄)-alkoxy-(C₁-C₄)-alkoxy, (C₁-C₈)-alkylthio, (C₂-C₈)-alkenylthio, (C₂-C₈)-alkynylthio, radicals of the formulae -NR*R**, -CO-NR*R** and -O-CO-NR*R**,
where each of the radicals R* and R** in the lastmentioned 3 formulae independently of the other is H, (C₁-C₈)-alkyl, (C₂-C₈)-alkenyl, (C₂-C₈)-alkynyl, benzyl, substituted benzyl, phenyl or substituted phenyl, or together with the nitrogen atom are a 3- to 8-membered heterocycle which in addition to the nitrogen atom may contain one or two further hetero ring atoms from the group consisting of N, O and S and which is unsubstituted or substituted by one or more radicals from the group consisting of (C₁-C₄)-alkyl and (C₁-C₄)-haloalkyl,
and [(C₁-C₈)-alkoxy]carbonyl, [(C₁-C₈)-alkoxy]thiocarbonyl, [(C₂-C₈)-alkenyloxy]carbonyl, [(C₂-C₈)-alkynyloxy] carbonyl, [(C₁-C₈)-alkylthio]carbonyl, [(C₂-C₈)-alkenylthio]carbonyl, [(C₂-C₈)-alkynylthio]carbonyl, (C₁-C₈)-alkanoyl, [(C₂-C₈)-alkenyl] carbonyl, [(C₂-C₈)-alkynyl]carbonyl, (C₁-C₄)-alkylimino, (C₁-C₄)-alkoxyimino, [(C₁-C₈)-alkyl]carbonylamino, [(C₂-C₈)-alkenyl]carbonylamino, [(C₂-C₈)-alkynyl]carbonylamino, [(C₁-C₈)-alkoxy]carbonylamino, [(C₂-C₈)-alkenyloxy]carbonylamino, [(C₂-C₈)-alkynyloxy] carbonylamino, [(C₁-C₈)-alkylamino]carbonylamino, [(C₁-C₆)-alkyl]carbonyloxy, [(C₂-C₆)-alkenyl]carbonyloxy, [(C₂-C₆)-alkynyl]carbonyloxy, [(C₁-C₈)-alkoxy] carbonyloxy, [(C₂-C₈)-alkenyloxy]carbonyloxy, [(C₂-C₈)-alkynyloxy]carbonyloxy, (C₁-C₈)-alkylsulfinyl and (C₁-C₈)-alkylsulfonyl,
where each of the 27 lastmentioned radicals is unsubstituted or substituted by one or more radicals from the group consisting of halogen, NO₂, (C₁-C₄)-alkoxy and optionally substituted phenyl,
and phenyl, phenyl-(C₁-C₆)-alkoxy, phenyl-[(C₁-C₆)-alkoxy]carbonyl, phenoxy, phenoxy-(C₁-C₆)-alkoxy, phenoxy-[(C₁-C₆)-alkoxy]carbonyl, phenoxycarbonyl,
phenylcarbonyloxy, phenoxycarbonyloxy, phenylcarbonylamino, phenyl-[(C₁-C₆)-alkyl]carbonylamino, phenyl-[(C₁-C₆)-alkyl]carbonyloxy, phenyl-[(C₁-C₆)-alkoxy]carbonyloxy, (C₃-C₇)-cycloalkyl, (C₃-C₇)-cycloalkoxy, (C₃-C₆)-cycloalkyl- (C₁-C₆)-alkoxy, (C₃-C₆)-cycloalkyl- [(C₁-C₆)-alkoxy] carbonyl, (C₃-C₆)-cycloalkoxy- (C₁-C₆)-alkoxy, (C₃-C₆)-cycloalkoxy-[(C₁-C₆)-alkoxy]carbonyl, (C₃-C₆)-cycloalkoxycarbonyl, (C₃-C₆)-cycloalkylcarbonyloxy, (C₃-C₆)-cycloalkoxycarbonyloxy, (C₃-C₆)-cycloalkyl-[(C₁-C₆)-alkoxy] carbonyloxy, (C₃--C₆)-cycloalkylcarbonylamino, (C₃-C₆-cycloalkyl-[(C₁-C₆)-alkyl]carbonylamino and (C₃-C₆)-cycloalkyl-[(C₁-C₆)-alkyl]carbonyloxy,
where each of the 26 lastmentioned radicals is optionally fused with a carbocyclic ring having 3 to 6 carbon atoms or a hetereocyclic ring having 5 or 6 ring atoms and 1 to 3 hetero ring atoms from the group consisting of N, O and S, and is unsubstituted or substituted in the ring or in the polycyclic system by one or more radicals from the group consisting of halogen, (C₁-C₄)-alkyl, (C₁-C₄)-alkoxy, (C₁-C₄)-haloalkyl, (C₁-C₄)-haloalkoxy and nitro,
and radicals of the formulae -SiR'₃, -O-SiR'₃, (R')₃Si-(C₁-C₆)-alkoxy, -CO-O-NR'₂, -O-N=CR'₂, -N=CR'₂, -O-NMR'₂, -CH(OR')₂ and -O-(CH₂)ₘ-CH(OR')₂,
in which each of the radicals R' independently of the others is H, (C₁-C₄)-alkyl or phenyl which is unsubstituted or substituted by one or more radicals from the group consisting of halogen, (C₁-C₄)-alkyl, (C₁-C₄)-alkoxy, (C₁-C₄)-haloalkyl, (C₁-C₄)-haloalkoxy and nitro or substituted in two adjacent positions by a (C₂-C₆)-alkylene bridge, and m is an integer of from 0 to 6,
and radicals of the formula R"O-CHR'"CH(OR")-(C₁-C₆)-alkoxy, in which each of the radicals R" independently of the others is H or (C₁-C₄)-alkyl or the radicals R" together are a (C₁-C₆)-alkylene group and R"' is H or (C₁-C₄)-alkyl,
and radicals of the formula Het¹, where Het¹ is in each case independently of the others a saturated, partially unsaturated or heteroaromatic heterocyclyl radical having 3 to 9 ring atoms, where the heterocyclic radical in question contains 1 to 4 heteroatoms from the group consisting of N, O and S and is optionally fused with a carbocyclic ring having 3 to 6 carbon atoms or a hetereocyclic ring having 5 or 6 ring atoms and 1 to 3 hetero ring atoms from the group consisting of N, O and S, and is unsubstituted or substituted in the ring or in the polycyclic system by one or more radicals from the group consisting of halogen, cyano, thio, nitro, hydroxyl, carboxy, (C₁-C₆)-alkyl, (C₁-C₆)-haloalkyl, (C₁-C₄)-alkoxy- (C₁-C₄)-alkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₂-C₆)-alkenyloxy, (C₂-C₆)-alkynyloxy, (C₁-C₆)-haloalkoxy, (C₁-C₄)-alkoxy-(C₁-C₄)-alkoxy, (C₁-C₆)-alkylthio, (C₂-C₆)-alkenylthio, (C₂-C₆)-alkynylthio, (C₃-C₆)-cycloalkyl, (C₃-C₆)-cycloalkoxy, [(C₁-C₈)-alkoxy] carbonyl, [(C₁-C₆)-haloalkoxy]carbonyl and oxo, or
R¹ is a polycyclic radical based on (C₃-C₉)-cycloalkyl, (C₅-C₉)-cycloalkenyl, (C₅-C₉)-cycloalkynyl or phenyl, where the basic ring is fused with a 5- or 6-membered ring having 0 or 1 to 3 hetero ring atoms from the group consisting of N, O and S, and where the basic ring or the polycyclic system is unsubstituted or substituted by one or more radicals from the group consisting of halogen, cyano, thio, nitro, hydroxyl, carboxy, (C₁-C₆)-alkyl, (C₁-C₆)-haloalkyl, (C₁-C₄)-alkoxy-(C₁-C₄)-alkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₂-C₆)-alkenyloxy, (C₂-C₆)-alkynyloxy, (C₁-C₆)-haloalkoxy, (C₁-C₄)-alkoxy-(C₁-C₄)-alkoxy, (C₁-C₆)-alkylthio, (C₂-C₆)-alkenylthio, (C₂-C₆)-alkynylthio, (C₃-C₆)-cycloalkyl, (C₃-C₆)-cycloalkoxy, [(C₁-C₈)-alkoxy]carbonyl, [(C₁-C₆)-haloalkoxy]carbonyl and oxo, or
R¹ is a saturated, partially unsaturated or heteroaromatic heterocyclyl radical having 3 to 9 ring atoms, preferably 5 or 6 ring atoms, which contains 1 to 4 heteroatoms, preferably 1 to 3 hetero ring atoms, from the group consisting of N, O and S, optionally also fused, preferably benzofused, with a carbocyclic or heterocyclic ring, preferably a 5- or 6-membered ring having 0 or 1 to 3 hetero ring atoms from the group consisting of N, O and S, and which is unsubstituted or substituted in the ring or in the polycyclic system by one or more radicals from the group consisting of halogen, cyano, thio, nitro, hydroxyl, carboxy, (C₁-C₆)-alkyl, (C₁-C₆)-haloalkyl, (C₁-C₄)-alkoxy-(C₁-C₄)-alkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₂-C₆)-alkenyloxy, (C₂-C₆)-alkynyloxy, (C₁-C₆)-haloalkoxy, (C₁-C₄)-alkoxy- (C₁-C₄)-alkoxy, (C₁-C₆)-alkylthio, (C₂-C₆)-alkenylthio, (C₂-C₆)-alkynylthio, (C₃-C₆)-cycloalkyl, (C₃-C₆)-cycloalkoxy, [(C₁-C₈)-alkoxy]carbonyl, [(C₁-C₆)-haloalkoxy]carbonyl and oxo.

5. The compound or a salt thereof as claimed in claim 1 wherein
R¹ is H, (C₁-C₁₈)-alkyl, (C₂-C₁₈)-alkenyl, (C₂-C₁₈)-alkynyl, (C₃-C₉)-cycloalkyl, (C₅-C₉)-cycloalkenyl, (C₅-C₉)-cycloalkynyl or phenyl, where each of the 7 lastmentioned radicals is unsubstituted or substituted by one or more radicals from the group consisting of halogen, cyano, thio, nitro, hydroxyl, (C₁-C₈)-alkyl, (C₁-C₈-haloalkyl, (C₁-C₄)-alkoxy-(C₁-C₄)-alkyl, (C₂-C₈)-alkenyl, (C₂-C₈)-haloalkenyl, (C₂-C₈)-alkynyl, (C₂-C₈)-haloalkynyl, the 7 lastmentioned radicals only in the case of cyclic base radicals, (C₁-C₈)-alkoxy, (C₂-C₈)-alkenyloxy, (C₂-C₈)-alkynyloxy, (C₁-C₈)-haloalkoxy, (C₁-C₄)-alkoxy- (C₁-C₄)-alkoxy, (C₁-C₈)-alkylthio, (C₂-C₈)-alkenylthio, (C₂-C₈)-alkynylthio, radicals of the formulae -NR*R**, -CO-NR*R** and -O-CO-NR*R**,
where each of the radicals R* and R** in the 3 lastmentioned formulae independently of the others is H, (C₁-C₈)-alkyl, (C₂-C₈)-alkenyl, (C₂-C₈)-alkynyl, benzyl, substituted benzyl, phenyl or substituted phenyl, or together with the nitrogen atom is a 3- to 8-membered heterocycle which in addition to the nitrogen atom may contain one or two further hetero ring atoms from the group consisting of N, O and S and which is unsubstituted or substituted by one or more radicals from the group consisting of (C₁-C₄)-alkyl and (C₁-C₄)-haloalkyl,
and (C₁-C₈)-alkoxy] carbonyl, [(C₁-C₈)-alkoxy]thiocarbonyl, [(C₂-C₈)-alkenyloxy]carbonyl, [(C₂-C₈)-alkynyloxy] carbonyl, [(C₁-C₈)-alkylthio]carbonyl, [(C₂-C₈)-alkenylthio]carbonyl, [(C₂-C₈)-alkynylthio]carbonyl, (C₁-C₈)-alkanoyl, [(C₂-C₈)-alkenyl]carbonyl, [(C₂-C₈)-alkynyl]carbonyl, (C₁-C₄)-alkylimino, (C₁-C₄)-alkoxyimino, [(C₁-C₈)-alkyl]carbonylamino, [(C₂-C₈)-alkenyl]carbonylamino, [(C₂-C₈)-alkynyl]carbonylamino, [(C₁-C₈)-alkoxy]carbonylamino, [(C₂-C₈)-alkenyloxy]carbonylamino, [(C₂-C₈)-alkynyloxy]carbonylamino, [(C₁-C₈)-alkylamino]carbonylamino, [(C₁-C₆)-alkyl]-carbonyloxy, [(C₂-C₆)-alkenyl]carbonyloxy, [(C₂-C₆-alkynyl]carbonyloxy, [(C₁-C₈)-alkoxy]carbonyloxy, [(C₂-C₈)-alkenyloxy] carbonyloxy, [(C₂-C₈)-alkynyloxy]carbonyloxy, (C₁-C₈)-alkylsulfinyl and (C₁-C₈)-alkylsulfonyl,
where each of the 27 lastmentioned radicals is unsubstituted or substituted by one or more radicals from the group consisting of halogen, NO₂, (C₁-C₄)-alkoxy and optionally substituted phenyl,
and phenyl, phenyl-(C₁-C₆)-alkoxy, phenyl-[(C₁-C₆)-alkoxy]carbonyl, phenoxy, phenoxy-(C₁-C₆)-alkoxy, phenoxy-[(C₁₋C₆)-alkoxy]carbonyl, phenoxycarbonyl, phenylcarbonyloxy, phenylcarbonylamino, phenyl-[(C₁-C₆)-alkyl]carbonylamino, phenyl-[(C₁-C₆)-alkyl]carbonyloxy, (C₃-C₇)-cycloalkyl and (C₃-C₇)-cycloalkoxy,
where each of the 13 radicals in the ring is unsubstituted or substituted by one or more radicals from the group consisting of halogen, (C₁-C₄)-alkyl, (C₁-C₄) -alkoxy, (C₁-C₄)-haloalkyl, (C₁-C₄)-haloalkoxy and nitro,
and radicals of the formulae -SiR'₃, -O-SiR'₃, (R')₃Si-(C₁-C₆)-alkoxy, -CO-O-NR'₂, -O-N=CR'₂, -N=CR'₂, -O-NR'₂, -CH(OR')₂ and -O-(CH₂)ₘ-CH(OR')₂,
in which each of the radicals R' independently of the others is H, (C₁-C₄)-alkyl or phenyl which is unsubstituted or substituted by one or more radicals from the group consisting of halogen, (C₁-C₄)-alkyl, (C₁-C₄)-alkoxy, (C₁-C₄)-haloalkyl, (C₁-C₄)-haloalkoxy and nitro or substituted at two adjacent positions by a (C₂-C₆)-alkylene bridge, and m is an integer from 0 to 6,
and radicals of the formula R"O-CHR'"CH(OR")-(C₁-C₆)-alkoxy,
in which each of the radicals R" independently of the others is H or (C₁-C₄)-alkyl or the radicals R" together are a (C₁-C₆-alkylene group and R'" is H or (C₁-C₄)-alkyl.

6. The compound or a salt thereof as claimed in any of claims 1 to 5 wherein
R² is hydrogen, halogen or (C₁-C₄)-alkyl which is unsubstituted or substituted by one or more radicals from the group consisting of halogen and
R³ is hydrogen, halogen or (C₁-C₄)-alkyl which is unsubstituted or substituted by one or more radicals from the group consisting of halogen or
R² and R³ together with the carbon atom to which they are attached are (C₃-C₆)-cycloalkyl or (C₅-C₆)-cycloalkenyl, where each of the 2 lastmentioned radicals is unsubstituted or substituted by one or more radicals from the group consisting of halogen and (C₁-C₄)-alkyl.

7. The compound or a salt thereof as claimed in any of claims 1 to 6 wherein
R⁴ is hydrogen, halogen, cyano, (C₁-C₄)-alkyl, (C₂-C₄)-alkenyl or (C₂-C₄)-alkynyl, where each of the three lastmentioned radicals is unsubstituted or substituted by one or more radicals from the group consisting of halogen and hydroxyl, preferably unsubstituted or substituted by one or more radicals from the group consisting of halogen, such as fluorine and chlorine, or
(C₃-C₆)-cycloalkyl which is unsubstituted or substituted by one or more radicals from the group consisting of halogen and (C₁-C₄)-alkyl, or
phenyl which is unsubstituted or substituted by one or more radicals from the group consisting of halogen, nitro, (C₁-C₄)-alkyl, (C₁-C₄)-haloalkyl, (C₁-C₄)-alkoxy, (C₁-C₄)-haloalkoxy, (C₁-C₄)-alkoxy-(C₁-C₄)-alkoxy, (C₁-C₉)-alkylthio, [(C₁-C₄)-alkoxy]carbonyl and [(C₁-C₄)-haloalkoxy]carbonyl, or
(C₁-C₄)-alkanoyl which is unsubstituted or substituted by one or more radicals from the group consisting of halogen, such as fluorine and chlorine, cyano, (C₁-C₄)-alkoxy, (C₁-C₄)-haloalkoxy and (C₁-C₂)-alkoxy-(C₁-C₂)-alkoxy, preferably formyl, or
[(C₁₋C₄)-alkoxy]carbonyl which is unsubstituted or substituted by one or more radicals from the group consisting of halogen, such as fluorine and chlorine, or
[(C₃-C₆)-cycloalkoxy]carbonyl which is unsubstituted or substituted by one or more radicals from the group consisting of halogen and (C₁-C₄)-alkyl.

8. The compound or a salt thereof as claimed in any of claims 1 to 7 wherein
R⁵ is phenyl, which is unsubstituted or preferably substituted by one or more radicals from the group consisting of halogen, hydroxyl, amino, nitro, carboxyl, cyano, carbamoyl, (C₁-C₆)-alkyl, (C₁-C₆)-haloalkyl, (C₁-C₄)-alkoxy-(C₁-C₄)-alkyl, (C₁-C₄)-alkylthio- (C₁-C₄)-alkyl, mono- and di-[(C₁-C₄)-alkyl]amino-alkyl, hydroxy-(C₁-C₄)-alkyl, carboxy-(C₁-C₄)-alkyl, cyano-(C₁-C₄)-alkyl, (C₁-C₆)-alkoxy, which may optionally also be halogenated [= (C₁-C₆)-haloalkoxy)], (C₁-C₄)-alkoxy-(C₁-C₄)-alkoxy, (C₁-C₆)-alkylthio, [(C₁-C₆)-alkoxy] carbonyl, [(C₁-C₆)-haloalkoxy]carbonyl, (C₁-C₆)-alkanoyl, (C₁-C₆)-haloalkanoyl, mono- and di-[(C₁-C₄)-alkyl]aminocarbonyl, mono- and di-[(C₁-C₆)acyl]amino, mono- and di-[(C₁-C₄)-alkyl] amino, N-[(C₁-C₆)-acyl]-N-[(C₁-C₆)-alkyl] amino, (C₁-C₆)-alkylsulfinyl, (C₁-C₆)-haloalkylsulfinyl, (C₁-C₆)-alkylsulfonyl, (C₁-C₆)-haloalkylsulfonyl, (C₃-C₆)-cycloalkyl, (C₃-C₆)-cycloalkyloxy, phenyl and phenoxy,
where each of the 4 lastmentioned radicals is unsubstituted or substituted by one or more radicals from the group consisting of halogen, (C₁-C₄)-alkyl and (C₁-C₄)-haloalkyl,
where two adjacent substituents may form a fused-on 5- or 6-membered ring which is carbocyclic or may also contain 1 to 3 hetero ring atoms from the group consisting of N, O and S and which is unsubstituted or substituted by one or more radicals from the group consisting of halogen and (C₁-C₆)-alkyl, or
R⁵ is a 5- or 6-membered heteroaromatic radical having 1 to 3 hetero ring atoms from the group consisting of N, O and S,
which is unsubstituted or substituted by one or more radicals from the group consisting of halogen, hydroxyl, amino, nitro, carboxy, cyano, carbamoyl, (C₁-C₆)-alkyl, (C₁-C₆)-haloalkyl, (C₁-C₄)-alkoxy-(C₁-C₄)-alkyl, (C₁-C₄)-alkylthio-(C₁₋C₄)-alkyl, mono- and di-[(C₁-C₄)-alkyl]aminoalkyl hydroxy-(C₁-C₄)-alkyl, carboxy-(C₁-C₄)-alkyl, cyano-(C₁-C₄)-alkyl, (C₁-C₆)-alkoxy, which may optionally also be halogenated [= (C₁-C₆)-haloalkoxy], (C₁-C₄)-alkoxy- (C₁-C₄)-alkoxy, (C₁-C₆)-alkylthio, [(C₁-C₆)-alkoxy] carbonyl, [(C₁-C₆)-haloalkoxy] carbonyl, (C₁-C₆)-alkanoyl, (C₁-C₆)-haloalkanoyl, mono- and di-[(C₁-C₄)-alkyl]aminocarbonyl, mono- and di-[(C₁-C₆)acyl]amino, mono- and di-[(C₁-C₄)-alkyl] amino, N-[(C₁-C₆-acyl]-N-[(C₁-C₆)-alkyl] amino, (C₁-C₆)-alkylsulfinyl, (C₁-C₆)-haloalkylsulfinyl, (C₁-C₆)-alkylsulfonyl, (C₁-C₆)-haloalkylsulfonyl, (C₃-C₆)-cycloalkyl, (C₃-C₆)-cycloalkyloxy, phenyl and phenoxy,
where each of the 4 lastmentioned radicals is unsubstituted or substituted by one or more radicals from the group consisting of halogen, (C₁-C₄)-alkyl and (C₁-C₄)-haloalkyl,
and where two adjacent substituents may form a fused-on 5- or 6-membered ring which is carbocyclic or may also contain 1 to 3 hetero ring atoms from the group consisting of N, O and S and which is unsubstituted or substituted by one or more radicals from the group consisting of halogen and (C₁-C₆)-alkyl.

9. The compound or a salt thereof as claimed in any of claims 1 to 8 wherein
(R⁶)ₙ is n substituents R⁶, where R⁶, in the case that n = 1, or each of the substituents R⁶ independently of one another, in the case that n is greater than 1, is a radical halogen, cyano, (C₁-C₄)-alkyl, (C₁-C₄) -haloalkyl, (C₁-C₄)-alkoxy, (C₁-C₄)-alkylthio, (C₁-C₄)-alkylsulfinyl, (C₁-C₄)-haloalkylsulfinyl, (C₁-C₄)-alkylsulfonyl or (C₁-C₄)-haloalkylsulfonyl, (C₁-C₄)-alkoxycarbonyl, and
n 0, 1, or 2,

10. A process for preparing a compound of the formula (I) as defined in any of claims 1 to 9 or a salt thereof wherein
(a) a compound of the formula (II),
H₂N-NH-Het(R⁶)ₙ (II)
in which Het and (R⁶)ₙ are as defined for formula (I),
is reacted with a compound of the formula (III), 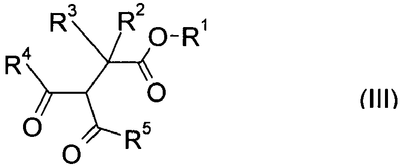 in which R¹, R², R³, R⁴ and R⁵ are as defined for formula (I),
to give the compound of the formula (I) or its salt,
(b) in the case that R¹ in formula (I) is different from hydrogen, a compound of the formula (I'), 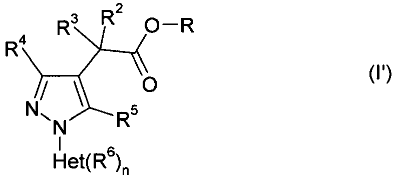 in which Het, R², R³, R⁴, R⁵ and R⁶ are as defined for formula (I) and
R is a radical different from the radical R¹ and different from hydrogen selected from the group of the radicals as defined for R¹, or an anhydride, acid halide or an activated ester of the compound of the formula (I') in which R = H,
is reacted with a compound of the formula (IV),
R¹ - OH (IV)
in which R¹ is as defined for formula (I),
to give the compound of the formula (I)
or
(c) in the case that R¹ in formula (I) is different from hydrogen, a compound of the formula (I") 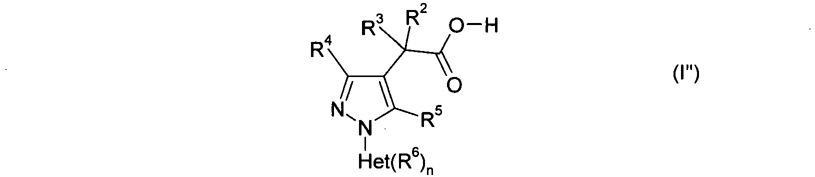 in which Het, R², R³, R⁴, R⁵ and R⁶ are as defined for formula (I) is esterified with a compound of the formula (IV),
R¹ - OH (IV)
in which R¹ is as defined for formula (I), to give the compound of the formula (I)
or
(d) in the case that the compound of the formula (I) in which R¹ = H or a salt thereof is prepared, a compound of the formula (I') [see definition in variant (b)] is hydrolyzed to give the compound of the formula (I) or a salt thereof,
or
g) a compound of the general formula (XI) is reacted with a boron derivative of the formula (XII), in the presence of a Cu(I) or Cu(II) salt and an organic base, 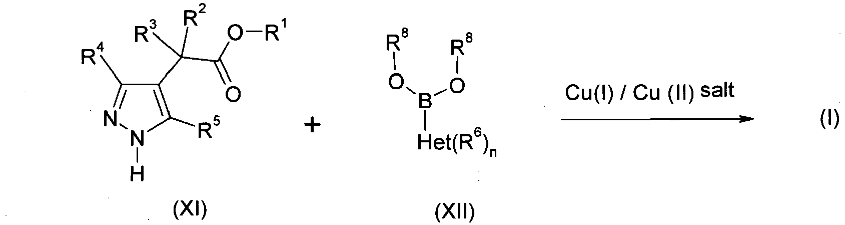 where in the formulae (XI) and (XII) Het, R¹, R², R³, R⁴, R⁵, R⁶ have the meanings given for formula (I) and R⁸ is H or (C₁-C₆)-alkyl or both alkyl radicals R⁸ are cyclically attached to one another,
or
h) a compound of the general formula (XI) is reacted with a compound of the formula (XIII) in which R⁶ has the meanings given above for formula (I) in the presence of a suitable catalyst/ligand system with a suitable base and in a suitable solvent to give the compound of the formula (I) or a salt thereof, 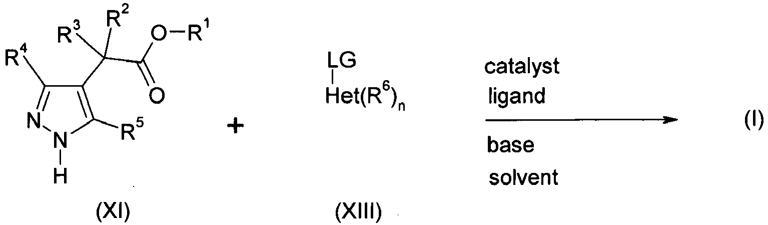 where in the formulae (XI) and (XIII) the radicals Het, R¹, R², R³, R⁴, R⁵, R⁶ have the meaning given above for formula (I) and LG is a leaving group, or
i) a compound of the general formula (XV) is reacted with a compound of the general formula (III) in the presence of an acid to give the compound of the formula (I) or a salt thereof, 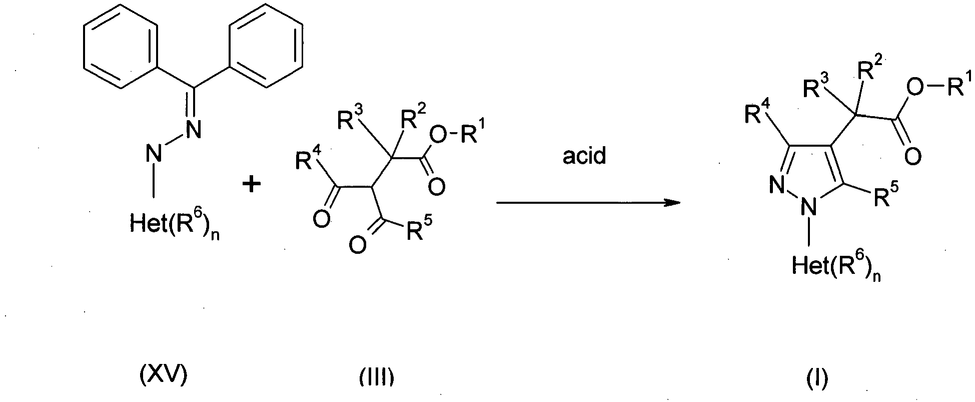 where in the formulae (XV) and (III) the radicals Het, R¹, R², R³, R⁴, R⁵, R⁶ are as defined for formula (I) and LG is a leaving group,
or
j) a compound of the general formula (XVI) where R⁶ is as defined for formula (I) is reacted with di-tert-butyl azodicarboxylate (DBAD, XVII) in the presence of a copper salt to give a compound of the formula (XVIII) in which R⁶ is as defined for formula (I) which is subsequently via a compound of the formula (II) or a salt thereof in which R⁶ is as defined for formula (I) and according to process a) converted into a compound of the formula (I): 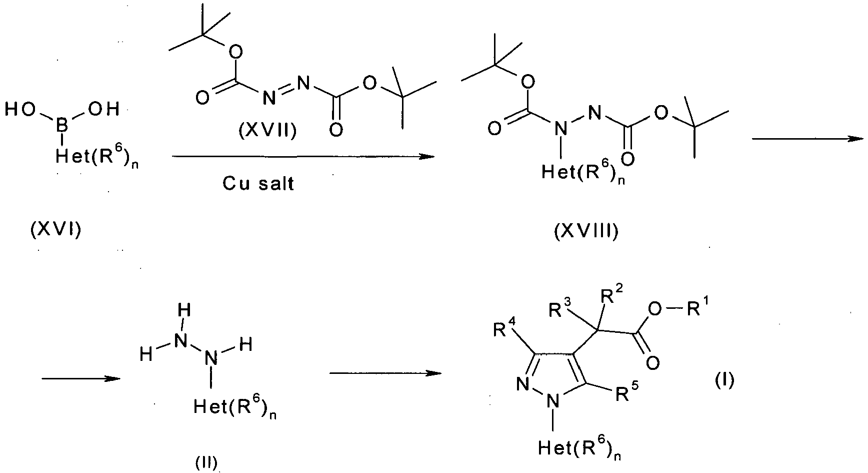 where in the formulae (XVI), (XVII), (XVIII) and (II) the radicals Het, R¹, R², R³, R⁴, R⁵, R⁶ are as defined for formula (I).

11. A herbicidal or plant growth-regulating composition which comprises one or more compounds of the formula (I) or salts thereof as defined in any of claims 1 to 9 and formulation auxiliaries customary in crop protection.

12. A method for controlling harmful plants or for regulating the growth of plants which comprises applying an effective amount of one or more compounds of the formula (I) or salts thereof as defined in any of claims 1 to 9 onto the plants, plant seeds, the soil in which or on which the plants grow or the area under cultivation.

13. The method as claimed in claim 12 wherein the compounds of the formula (I) or salts thereof are employed for controlling harmful plants or for regulating the growth in crops of useful plants or ornamental plants.

14. The method as claimed in claim 13 wherein the crop plants are transgenic crop plants.

15. The use of a compound of the formula (I) or a salt thereof as claimed in any of claims 1 to 9 as herbicides or plant growth regulators.

## Revendications

1. Composés de formule (I) ou leurs sels, 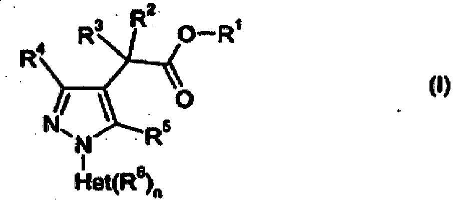 formule dans laquelle
Het représente un radical hétéroaromatique à cinq chaînons, comportant deux hétéroatomes en tant qu'atomes formant le cycle, l'un des hétéroatomes dans le cycle étant un atome d'azote et l'autre un atome de soufre et l'atome d'azote dans le cycle se trouvant en position 1,3 par rapport à l'atome de carbone du cycle, qui est lié au radical pyrazole,
R¹ représente un atome de d'hydrogène ou un radical hydrolysable,
R² représente un atome d'hydrogène ou d'halogène, ou un groupe alkyle en C₁-C₆ qui est non substitué ou substitué par un ou plusieurs radicaux choisis dans le groupe qui est constitué par halogéno, alcoxy en C₁-C₄, alkyl(C₁-C₄)thio et halogéno-alcoxy(C₁-C₄),
R³ représente un atome d'hydrogène ou d'halogène, ou un groupe alkyle en C₁-C₆ qui est non substitué ou substitué par un ou plusieurs radicaux choisis dans le groupe qui est constitué par halogéno, alcoxy en C₁-C₄, alkyl(C₁-C₄)thio et halogéno-alcoxy(C₁-C₄), ou
R² et R³ représentent ensemble avec l'atome de carbone, auquel ils sont liés, un cycle carbocyclique saturé ou partiellement insaturé ayant de 3 à 6 atomes de carbone, qui est non substitué ou substitué par un ou plusieurs radicaux choisis dans le groupe constitué par halogéno et alkyle en C₁-C₄, et
R⁴ représente un atome d'hydrogène ou d'halogène, un groupe cyano, alkyle en C₁-C₆, alcényle en C₂-C₆ ou alcynyle en C₂-C₆, chacun des trois radicaux nommés en dernier étant non substitué ou substitué par un ou plusieurs radicaux choisis dans le groupe constitué par halogéno, cyano, hydroxy, alcoxy en C₁-C₄, halogénoalcoxy(C₁-C₄), alcoxy(C₁-C₄)-alcoxy(C₁-C₄), alkyl(C₁-C₄)thio et un groupe cycloalkyle en C₃-C₉ éventuellement substitué par halogéno, cyano, alkyle en C₁-C₄ ou halogénoalkyle(C₁-C₄), ou un groupe cycloalkyle en C₃-C₉, cycloalcényle en C₅-C₉ ou cycloalcynyle en C₅-C₉, chacun des trois radicaux nommés en dernier étant non substitué ou substitué par un ou plusieurs radicaux choisis dans le groupe constitué par halogéno, alkyle en C₁-C₄, halogénoalkyle(C₁-C₄), alcényle en C₂-C₄, alcynyle en C₂-C₄, alcoxy en C₁-C₄, halogénoalcoxy(C₁-C₄) et alkyl(C₁-C₄)thio, ou un groupe phényle qui est non substitué ou substitué par un ou plusieurs radicaux choisis dans le groupe constitué par halogéno, cyano, nitro, carboxy, alkyle en C₁-C₄, halogénoalkyle(C₁-C₄), alcényle en C₂-C₄, alcynyle en C₂-C₄, alcoxy en C₁-C₄, halogénoalcoxy(C₁-C₄), alcoxy(C₁-C₄)-alcoxy(C₁-C₄), alkyl(C₁-C₄)thio, alcanoyle en C₁-C₄, halogénoalcanoyle(C₁-C₄), [alcoxy(C₁-C₄)]-carbonyle et [halogéno-alcoxy(C₁-C₄)]-carbonyle, ou
un groupe alcanoyle en C₁-C₆ qui est non substitué ou substitué par un ou plusieurs radicaux choisis dans le groupe constitué par halogéno, cyano, alcoxy en C₁-C₄, halogénoalcoxy(C₁-C₄), alcoxy(C₁-C₄)-alcoxy(C₁-C₄), alkyl(C₁-C₄)thio et un groupe cycloalkyle en C₃-C₆ éventuellement substitué par halogéno, cyano, alkyle en C₁-C₄ ou halogénoalkyle(C₁-C₄), ou
un groupe [alcoxy(C₁-C₄)]-carbonyle qui est non substitué ou substitué par un ou plusieurs radicaux choisis dans le groupe constitué par halogéno, cyano, alcoxy en C₁-C₄, halogénoalcoxy(C₁-C₄), alcoxy(C₁-C₄)-alcoxy(C₁-C₄), alkyl(C₁-C₄)thio et un groupe cycloalkyle en C₃-C₆ éventuellement substitué par halogéno, cyano, alkyle en C₁-C₄ ou halogénoalkyle(C₁-C₄), ou
un groupe [cycloalcoxy(C₃-C₉)]-carbonyle qui est non substitué ou substitué par un ou plusieurs radicaux choisis dans le groupe constitué par
halogéno, alkyle en C₁-C₄, halogéno-alkyle(C₁-C₄), alcényle en C₂-C₄, alcynyle en C₂-C₄, alcoxy en C₁-C₄, halogénoalcoxy(C₁-C₄) et alkyl(C₁-C₄)thio,
R⁵ représente un radical phényle ou un radical hétéroaromatique à 5 ou 6 chaînons, comportant 1 à 3 hétéroatomes formant le cycle, choisis dans le groupe constitué par N, 0 et S, le radical phényle ou le radical hétérocyclique étant non substitué ou substitué par un ou plusieurs radicaux choisis dans le groupe constitué par les radicaux
(a) halogéno, hydroxy, amino, nitro, carboxy, cyano et carbamoyle,
(b) alkyle en C₁-C₆, alcényle en C₂-C₆, alcynyle en C₂-C₆, alcoxy en C₁-C₆, alcényloxy en C₁-C₆ et alcynyloxy en C₁-C₆, chacun des 6 radicaux nommés en dernier étant non substitué ou substitué par un ou plusieurs radicaux choisis dans le groupe constitué par halogéno, alcoxy en C₁-C₄, halogéno-alcoxy(C₁-C₆), alkyl(C₁-C₄)thio, mono- et di-[alkyl(C₁-C₄)]-amino, hydroxy, carboxy, [alcoxy(C₁-C₄)]-carbonyle, [halogéno-alcoxy(C₁-C₄)]carbonyle, mono- et di-[alkyl(C₁-C₄)]-aminocarbonyle et cyano,
(c) alkyl(C₁-C₆)thio, [alcoxy(C₁-C₆)]-carbonyle, [halogénoalcoxy(C₁-C₆)]-carbonyle, alcanoyle en C₁-C₆, halogénoalcanoyle(C₁-C₆), mono- et di-[alkyl(C₁-C₄)]-aminocarbonyle, mono- et di-[acyl(C₁-C₆)]-amino, mono- et di-[alkyl(C₁-C₄)]-amino, N-[acyl(C₁-C₆)]-N-[alkyl(C₁-C₆)]-amino, alkyl(C₁-C₆)sulfinyle, halogénoalkyl (C₁-C₆)sulfinyle, alkyl(C₁-C₆)-sulfonyle, halogénoalkyl(C₁-C₆)sulfonyle, alkyl(C₁-C₆)sulfinyloxy, halogénoalkyl(C₁-C₆)-sulfinyloxy, alkyl(C₁-C₆)sulfonyloxy, halogénoalkyl(C₁-C₆)sulfonyloxy, alkyl(C₁-C₆)-sulfato, halogénoalkyl(C₁-C₆)sulfato et
(d) cycloalkyle en C₃-C₆, cycloalkyloxy en C₃-C₆, phényle et phénoxy, chacun des 4 radicaux nommés en dernier étant non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₆) et alkyl(C₁-C₄)thio,
et deux substituants contigus pouvant former un cycle soudé à 5 ou 6 chaînons, qui est carbocyclique ou peut contenir encore 1 à 3 hétéroatomes formant le cycle, choisis dans le groupe N, O et S et qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₆) et alkyl(C₁-C₄)thio, le radical phényle ayant, substituants inclus, de 6 à 30 atomes de carbone et le radical hétéroaromatique ayant, substituants inclus, de 1 à 30 atomes de carbone, et
(R⁶)ₙ représente n substituants R⁶, R⁶, dans le cas où n est égal à 1, ou chacun des substituants R⁶ indépendamment les uns des autres, dans le cas où n est supérieur à 1, représentant un radical halogéno, hydroxy, amino, nitro, carboxy, cyano, carbamoyle, alkyle en C₁-C₆, halogénoalkyle(C₁-C₆), alcoxy(C₁-C₄)-alkyle(C₁-C₄), alkyl(C₁-C₄)thio-alkyle(C₁-C₄), mono- ou di-[alkyl(C₁-C₄)]-aminoalkyle, hydroxy-alkyle(C₁-C₄), alcényle en C₂-C₆, halogénoalcényle(C₂-C₆), alcynyle en C₂-C₆, halogénoalcynyle(C₂-C₆), alcoxy en C₁-C₆, halogénoalcoxy(C₁-C₆), alcoxy(C₁-C₄)-alcoxy(C₁-C₄), alkyl(C₁-C₆)thio, halogénoalkyl(C₁-C₆)thio, [alcoxy(C₁-C₆)]-carbonyle, [halogénoalcoxy(C₁-C₆)]-carbonyle, alcanoyle en C₁-C₆, halogéno-alcanoyle(C₁-C₆), mono- ou di-[alkyl(C₁-C₄)]-amino-carbonyle, mono- ou di-[acyl(C₁-C₆)]-amino, mono-ou di-[alkyl(C₁-C₄)-amino, N-[acyl(C₁-C₆)]-N-[alkyl(C₁-C₆)]-amino, alkyl(C₁-C₆)sulfinyle, halogénoalkyl(C₁-C₆)sulfinyle, alkyl(C₁-C₆)-sulfonyle, halogénoalkyl(C₁-C₆)sulfonyle, cycloalkyle en C₃-C₉ ou cycloalcényle en C₅-C₉, chacun des deux radicaux nommés en dernier étant non substitué ou substitué par un ou plusieurs radicaux choisis dans le groupe constitué par halogéno, alkyle en C₁-C₄ et halogénoalkyle(C₁-C₄), et
n représente 0, 1 ou 2.

2. Composés ou leurs sels selon la revendication 1, **caractérisés en ce que**
R¹ représente un atome d'hydrogène ou un radical hydrocarboné éventuellement substitué ou un radical hétérocyclyle éventuellement substitué, chacun des deux radicaux carbonés nommés en dernier comportant, substituants inclus, de 1 à 30 atomes de carbone, ou un radical de formule SiR^{a}R^{b}R^{c}, -NR^{a}R^{b} ou -N=CR^{c}R^{d}, dans les 3 formules nommées en dernier chacun des radicaux R^{a}, R^{b}, R^{c} et R^{d} indépendamment les uns des autres représentant un atome d'hydrogène ou un radical hydrocarboné éventuellement substitué ou R^{a} et R^{b} représentant ensemble avec l'atome d'azote un hétérocycle à 3 à 9 chaînons, qui en plus de l'atome d'azote peut contenir un ou deux hétéroatomes supplémentaires formant le cycle, choisis dans le groupe constitué par N, O et S et qui est substitué ou non substitué, ou R^{c} et R^{d} représentant ensemble avec l'atome de carbone un radical carbocyclique à 3 à 9 chaînons ou un radical hétérocyclique qui peut contenir 1 à 3 hétéroatomes formant le cycle, choisis dans le groupe constitué par N, 0 et S, le radical carbocyclique ou hétérocyclique étant substitué ou non substitué,
chacun des radicaux R^{a}, R^{b}, R^{c} et R^{d}, substituants inclus, comportant jusqu'à 30 atomes de carbone.

3. Composés ou leurs sels selon la revendication 1, **caractérisés en ce que**
R¹ représente un atome d'hydrogène, un groupe alkyle, alcényle, alcynyle, cycloalkyle, cycloalcényle, cycloalcynyle ou aryle, chacun des 7 radicaux nommés en dernier étant substitué ou non substitué en comportant, substituants inclus, jusqu'à 30 atomes de carbone, ou un radical hétérocyclyle ayant de 3 à 9 atomes formant le cycle, qui contient 1 à 4 hétéroatomes choisis dans le groupe constitué par N, 0 et S et qui est substitué ou non substitué et qui comporte, substituants inclus, de 1 à 30 atomes de carbone.

4. Composés ou leurs sels selon la revendication 1, **caractérisés en ce que**
R¹ représente H, un groupe alkyle en C₁-C₁₈, alcényle en C₂-C₁₈, alcynyle en C₂-C₁₈, cycloalkyle en C₃-C₉, cycloalcényle en C₅-C₉, cycloalcynyle en C₅-C₉ ou phényle, chacun des 7 radicaux nommés en dernier étant non substitué ou substitué par un ou plusieurs radicaux choisis dans le groupe constitué par halogéno, cyano, thio, nitro, hydroxy, carboxy, alkyle en C₁-C₈, halogénoalkyle(C₁-C₈), alcoxy(C₁-C₄)-alkyle(C₁-C₄), alcényle en C₂-C₈, halogénoalcényle(C₂-C₈), alcynyle en C₂-C₈, halogénoalcynyle(C₂-C₈), les 7 radicaux nommés en dernier seulement dans le cas de radicaux cycliques de base, alcoxy en C₁-C₈, alcényloxy en C₂-C₈, alcynyloxy en C₂-C₈, halogénoalcoxy(C₁-C₈), alcoxy(C₁-C₄)-alcoxy(C₁-C₄), alkyl(C₁-C₈)thio, alcényl(C₂-C₈)thio, alcynyl(C₂-C₈)thio, des radicaux de formules -NR*R**, -CO-NR*R** et -O-CO-NR*R**,
les radicaux R* et R** dans les 3 formules nommées en dernier représentant chacun indépendamment H, un groupe alkyle en C₁-C₈, alcényle en C₂-C₈, alcynyle en C₂-C₈, benzyle, benzyle substitué, phényle ou phényle substitué, ou formant ensemble avec l'atome d'azote un hétérocycle à 3 à 8 chaînons, qui en plus de l'atome d'azote peut contenir un ou deux hétéroatomes supplémentaires formant le cycle, choisis dans le groupe constitué par N, O et S et qui est non substitué ou substitué par un ou plusieurs radicaux choisis dans le groupe constitué par alkyle en C₁-C₄ et halogénoalkyle(C₁-C₄),
et [alcoxy(C₁-C₈)]-carbonyle, [alcoxy(C₁-C₈)]-thio-carbonyle, [alcényloxy(C₂-C₈)]-carbonyle, [alcynyloxy(C₂-C₈)]-carbonyle, [alkyl(C₁-C₈)thio]-carbonyle, [alcényl(C₂-C₈)thio]-carbonyle, [alcynyl(C₂-C₈)thio]-carbonyle, alcanoyle en C₁-C₈, [alcényl(C₂-C₈)]-carbonyle, [alcynyl(C₂-C₈)]-carbonyle, alkyl(C₁-C₄)imino, alcoxy(C₁-C₄)imino, [alkyl(C₁-C₈)]-carbonylamino, [alcényl(C₂-C₈)]-carbonylamino, [alcynyl(C₂-C₈)]-carbonylamino, [alcoxy(C₁-C₈)]-carbonylamino, [alcényloxy(C₂-C₈)]-carbonylamino, [alcynyloxy(C₂-C₈)]-carbonylamino, [alkyl(C₁-C₈)amino]-carbonylamino, [alkyl(C₁-C₆)]-carbonyloxy, [alcényl(C₂-C₆)]-carbonyloxy, [alcynyl(C₂-C₆)]-carbonyloxy, [alcoxy(C₁-C₈)]-carbonyloxy, [alcényloxy(C₂-C₈)]-carbonyloxy, [alcynyloxy(C₂-C₈)]-carbonyloxy, alkyl(C₁-C₈)-sulfinyle et alkyl(C₁-C₈)sulfonyle,
chacun des 27 radicaux nommés en dernier étant non substitué ou substitué par un ou plusieurs radicaux choisis dans le groupe constitué par halogéno, NO₂, alcoxy en C₁-C₄ et phényle éventuellement substitué,
et phényle, phényl-alcoxy(C₁-C₆), phényl-[alcoxy(C₁-C₆] -carbonyle, phénoxy, phénoxy-alcoxy(C₁-C₆), phénoxy[alcoxy(C₁-C₆)]-carbonyle, phénoxycarbonyle, phénylcarbonyloxy, phénoxy-carbonyloxy, phénylcarbonylamino, phényl-[alkyl(C₁-C₆)]-carbonylamino, phényl[alkyl(C₁-C₆)]-carbonyloxy, phényl[alcoxy(C₁-C₆)]-carbonyloxy, cycloalkyle en C₃-C₇, cycloalcoxy en C₃-C₇, cycloalkyl(C₃-C₆)-alcoxy(C₁-C₆), cycloalkyl(C₃-C₆)-[alcoxy(C₁-C₆)]-carbonyle, cycloalcoxy(C₃-C₆)-alcoxy(C₁-C₆), cycloalcoxy(C₃-C₆)-[alcoxy(C₁-C₆)]-carbonyle, cycloalcoxy(C₃-C₆)-carbonyle, cycloalkyl(C₃-C₆)-carbonyloxy, cycloalcoxy(C₃-C₆)-carbonyloxy, cycloalkyl(C₃-C₆)-[alcoxy(C₁-C₆)]-carbonyloxy, cycloalkyl(C₃-C₆)-carbonylamino, cycloalkyl(C₃-C₆)-[alkyl(C₁-C₆)]-carbonylamino et cycloalkyl(C₃-C₆)-[alkyl(C₁-C₆)]-carbonyloxy,
chacun des 26 radicaux nommés en dernier étant éventuellement condensé avec un cycle carbocyclique ayant de 3 à 6 atomes de carbone ou un cycle hétérocyclique ayant 5 ou 6 atomes formant le cycle et comportant 1 à 3 hétéroatomes dans le cycle, choisis dans le groupe constitué par N, O et S, et étant non substitué ou substitué dans le cycle ou dans le système polycyclique par un ou plusieurs radicaux choisis dans le groupe constitué par halogéno, alkyle en C₁-C₄, alcoxy en C₁-C₄, halogénoalkyle(C₁-C₄), halogénoalcoxy(C₁-C₄) et nitro,
et des radicaux de formules -SiR'₃, -O-SiR'₃, (R')₃Si-alcoxy(C₁-C₆), -CO-O-NR'₂, -O-N=CR'₂, -N=CR'₂, -O-NR'₂, -CH(OR')₂ et -O-(_{C}H₂)ₘ-CH(OR')₂,
dans lesquels chacun des radicaux R' indépendamment les uns des autres représente H, un groupe alkyle en C₁-C₄ ou phényle qui est non substitué ou substitué par un ou plusieurs radicaux choisis dans le groupe constitué par halogéno, alkyle en C₁-C₄, alcoxy en C₁-C₄, halogénoalkyle(C₁-C₄), halogénoalcoxy(C₁-C₄) et nitro ou en deux positions contiguës par un pont alkylène en C₂-C₆, et m représente un nombre entier valant de 0 à 6,
et des radicaux de formule R"O-CHR'"CH(OR")-alcoxy(C₁-C₆), dans laquelle les radicaux R" représentent chacun indépendamment l'un de l'autre H ou un groupe alkyle en C₁-C₄ ou forment ensemble un groupe alkylène en C₁-C₆ et R'" représente H ou un groupe alkyle en C₁-C₄,
et des radicaux de formule Het¹, Het¹ représentant chaque fois indépendamment un radical hétérocyclyle saturé, partiellement insaturé ou hétéroaromatique ayant de 3 à 9 atomes formant le cycle, le cycle hétérocyclique respectif contenant 1 à 4 hétéroatomes choisis dans le groupe constitué par N, 0 et S et étant éventuellement condensé avec un cycle carbocyclique ayant de 3 à 6 atomes de carbone ou un cycle hétérocyclique ayant 5 ou 6 atomes formant le cycle et comportant 1 à 3 hétéroatomes dans le cycle, choisis dans le groupe constitué par N, 0 et S, et étant non substitué ou substitué dans le cycle ou dans le système polycyclique par un ou plusieurs radicaux choisis dans le groupe constitué par halogéno, cyano, thio, nitro, hydroxy, carboxy, alkyle en C₁-C₆, halogéno-alkyle(C₁-C₆), alcoxy(C₁-C₄)-alkyle(C₁-C₄), alcényle en C₂-C₆, halogénoalcényle(C₂-C₆), alcynyle en C₂-C₆, halogénoalcynyle(C₂-C₆), alcoxy en C₁-C₆, alcényloxy en C₂-C₆, alcynyloxy en C₂-C₆, halogénoalcoxy(C₁-C₆), alcoxy(C₁-C₄)-alcoxy(C₁-C₄), alkyl(C₁-C₆)thio, alcényl(C₂-C₆)thio, alcynyl(C₂-C₆)thio, cycloalkyle en C₃-C₆, cycloalcoxy en C₃-C₆, [alcoxy(C₁-C₈)]-carbonyle, [halogéno-alcoxy(C₁-C₆)]-carbonyle et oxo, ou
R¹ représente un radical polycyclique à base de cycloalkyle en C₃-C₉, cycloalcényle en C₅-C₉, cycloalcynyle en C₅-C₉ ou phényle, le cycle de base étant condensé avec un cycle à 5 ou 6 chaînons comportant 0 ou 1 à 3 hétéroatomes dans le cycle, choisis dans le groupe constitué par N, 0 et S, et le cycle de base ou le système polycyclique étant non substitué ou substitué par un ou plusieurs radicaux choisis dans le groupe constitué par halogéno, cyano, thio, nitro, hydroxy, carboxy, alkyle en C₁-C₆, halogénoalkyle(C₁-C₆), alcoxy(C₁-C₄)-alkyle(C₁-C₄), alcényle en C₂-C₆, halogénoalcényle(C₂-C₆), alcynyle en C₂-C₆, halogénoalcynyle(C₂-C₆), alcoxy en C₁-C₆, alcényloxy en C₂-C₆, alcynyloxy en C₂-C₆, halogénoalcoxy(C₁-C₆), alcoxy(C₁-C₄)-alcoxy(C₁-C₄), alkyl(C₁-C₆)thio, alcényl(C₂-C₆)thio, alcynyl(C₂-C₆)thio, cycloalkyle en C₃-C₆, cycloalcoxy(C₃-C₆), [alcoxy(C₁-C₈)]-carbonyle, [halogénoalcoxy(C₁-C₆)]-carbonyle et oxo, ou
R¹ représente un radical hétérocyclyle saturé, partiellement insaturé ou hétéroaromatique ayant de 3 à 9 atomes formant le cycle, de préférence ayant 5 ou 6 atomes formant le cycle, qui contient 1 à 4 hétéroatomes, de préférence 1 à 3 hétéroatomes dans le cycle, choisis dans le groupe constitué par N, 0 et S, est éventuellement condensé avec un cycle carbocyclique ou hétérocyclique, de préférence avec un cycle à 5 ou 6 chaînons comportant 0 ou 1 à 3 hétéroatomes dans le cycle, choisis dans le groupe constitué par N, O et S, de préférence est condensé avec un cycle benzénique, et qui est non substitué ou substitué dans le cycle ou dans le système polycyclique par un ou plusieurs radicaux choisis dans le groupe constitué par halogéno, cyano, thio, nitro, hydroxy, carboxy, alkyle en C₁-C₆, halogénoalkyle(C₁-C₆), alcoxy(C₁-C₄)-alkyle(C₁-C₄), alcényle en C₂-C₆, halogénoalcényle(C₂-C₆), alcynyle en C₂-C₆, halogénoalcynyle(C₂-C₆), alcoxy en C₁-C₆, alcényloxy en C₂-C₆, alcynyloxy en C₂-C₆, halogénoalcoxy(C₁-C₆), alcoxy(C₁-C₄)-alcoxy(C₁-C₄), alkyl(C₁-C₆)thio, alcényl(C₂-C₆)thio, alcynyl(C₂-C₆)thio, cycloalkyle en C₃-C₆, cycloalcoxy en C₃-C₆, [alcoxy(C₁-C₈)]-carbonyle, [halogénoalcoxy(C₁-C₆)]-carbonyle et oxo.

5. Composés ou leurs sels selon la revendication 1, **caractérisés en ce que**
R¹ représente H, un groupe alkyle en C₁-C₁₈, alcényle en C₂-C₁₈, alcynyle en C₂-C₁₈, cycloalkyle en C₃-C₉, cycloalcényle en C₅-C₉, cycloalcynyle en C₅-C₉ ou phényle,
chacun des 7 radicaux nommés en dernier étant non substitué ou substitué par un ou plusieurs radicaux choisis dans le groupe constitué par halogéno, cyano, thio, nitro, hydroxy, alkyle en C₁-C₈, halogénoalkyle(C₁-C₈), alcoxy(C₁-C₄)-alkyle(C₁-C₄), alcényle en C₂-C₈, halogéno-alcényle(C₂-C₈), alcynyle en C₂-C₈, halogéno-alcynyle(C₂-C₈), les 7 radicaux nommés en dernier seulement dans le cas des radicaux cycliques de base, alcoxy en C₁-C₈, alcényloxy en C₂-C₈, alcynyloxy en C₂-C₈, halogénoalcoxy(C₁-C₈), alcoxy(C₁-C₄)-alcoxy(C₁-C₄), alkyl(C₁-C₈)thio, alcényl(C₂-C₈)thio, alcynyl(C₂-C₈)thio, des radicaux de formules -NR*R**, -CO-NR*R** et -O-CO-NR*R**,
les radicaux R* et R** dans les 3 formules nommées en dernier représentant chacun indépendamment H, un groupe alkyle en C₁-C₈, alcényle en C₂-C₈, alcynyle en C₂-C₈, benzyle, benzyle substitué, phényle ou phényle substitué, ou formant ensemble avec l'atome d'azote un hétérocycle à 3 à 8 chaînons, qui en plus de l'atome d'azote peut contenir un ou deux autres hétéroatomes formant le cycle, choisis dans le groupe constitué par N, 0 et S et qui est non substitué ou substitué par un ou plusieurs radicaux choisis dans le groupe constitué par alkyle en C₁-C₄ et halogénoalkyle(C₁-C₄),
et [alcoxy(C₁-C₈)]-carbonyle, [alcoxy(C₁-C₈)]-thio-carbonyle, [alcényloxy(C₂-C₈)]-carbonyle, [alcynyloxy(C₂-C₈)]-carbonyle, [alkyl(C₁-C₈)thio]-carbonyle, [alcényl(C₂-C₈)thio]-carbonyle, [alcynyl(C₂-C₈)thio]-carbonyle, alcanoyle en C₁-C₈, [alcényl(C₂-C₈)]-carbonyle, [alcynyl(C₂-C₈)]-carbonyle, alkyl(C₁-C₄)imino, alcoxy(C₁-C₄)imino, [alkyl(C₁-C₈)]-carbonylamino, [alcényl(C₂-C₈)]-carbonylamino, [alcynyl(C₂-C₈)]-carbonylamino, [alcoxy(C₁-C₈)]-carbonylamino, [alcényloxy(C₂-C₈)]-carbonylamino, [alcynyloxy(C₂-C₈)]-carbonylamino, [alkyl(C₁-C₈)amino]-carbonylamino, [alkyl(C₁-C₆)]-carbonyloxy, [alcényl(C₂-C₆)]-carbonyloxy, [alcynyl(C₂-C₆)]-carbonyloxy, [alcoxy(C₁-C₈)]-carbonyloxy, [alcényloxy(C₂-C₈)]-carbonyloxy, [alcynyloxy(C₂-C₈)]-carbonyloxy, alkyl(C₁-C₈)-sulfinyle et alkyl(C₁-C₈)sulfonyle,
chacun des 27 radicaux nommés en dernier étant non substitué ou substitué par un ou plusieurs radicaux choisis dans le groupe constitué par halogéno, NO₂, alcoxy en C₁-C₄ ou phényle éventuellement substitué,
et phényle, phényl-alcoxy(C₁-C₆), phényl-[alcoxy(C₁-C₆)]-carbonyle, phénoxy, phénoxy-alcoxy(C₁-C₆), phénoxy[alcoxy(C₁-C₆)]-carbonyle, phénoxycarbonyle, phénylcarbonyloxy, phényl-carbonylamino, phényl-[alkyl(C₁-C₆)]-carbonylamino, phényl[alkyl(C₁-C₆)]-carbonyloxy, cycloalkyle en C₃-C₇ et cycloalcoxy en C₃-C₇,
chacun des 13 radicaux nommés en dernier étant non substitué ou substitué dans le cycle par un ou plusieurs radicaux choisis dans le groupe constitué par halogéno, alkyle en C₁-C₄, alcoxy en C₁-C₄, halogéno-alkyle(C₁-C₄), halogénoalcoxy(C₁-C₄) et nitro,
et des radicaux de formules -SiR'₃, -O-SiR'₃, (R')₃Si-alcoxy(C₁-C₆), -CO-O-NR'₂, -O-N=CR'₂, -N=CR'₂, -O-NR'₂, -CH(OR')₂ et -O-(CH₂)ₘ-CH(OR')₂,
dans lesquels chacun des radicaux R' indépendamment les uns des autres représente H, un groupe alkyle en C₁-C₄ ou phényle, qui est non substitué ou substitué par un ou plusieurs radicaux choisis dans le groupe constitué par halogéno, alkyle en C₁-C₄, alcoxy en C₁-C₄, halogénoalkyle(C₁-C₄), halogénoalcoxy(C₁-C₄) et nitro ou en deux positions contiguës par un pont alkylène en C₂-C₆, et m représente un nombre entier valant de 0 à 6,
et des radicaux de formule R"O-CHR'"CH(OR")-alcoxy(C₁-C₆) dans laquelle les radicaux R" représentent chacun indépendamment l'un de l'autre H ou un groupe alkyle en C₁-C₄ ou forment ensemble un groupe alkylène en C₁-C₆ et R'" représente H ou un groupe alkyle en C₁-C₄.

6. Composés ou leurs sels selon l'une quelconque des revendications 1 à 5, **caractérisés en ce que**
R² représente un atome d'hydrogène ou d'halogène, ou un groupe alkyle en C₁-C₄ qui est non substitué ou substitué par un ou plusieurs radicaux choisis dans le groupe halogéno, et
R³ représente un atome d'hydrogène ou d'halogène ou un groupe alkyle en C₁-C₄ qui est non substitué ou substitué par un ou plusieurs radicaux choisis dans le groupe halogéno ou
R² et R³ représentent ensemble avec l'atome de carbone, auquel ils sont liés, un groupe cycloalkyle en C₃-C₆ ou cycloalcényle en C₅-C₆, chacun des 2 radicaux nommés en dernier étant non substitué ou substitué par un ou plusieurs radicaux choisis dans le groupe constitué par halogéno et alkyle en C₁-C_{4.}

7. Composés ou leurs sels selon l'une quelconque des revendications 1 à 6, **caractérisés en ce que**
R⁴ représente un atome d'hydrogène ou d'halogène, un groupe cyano, alkyle en C₁-C₄, alcényle en C₂-C₄ ou alcynyle en C₂-C₄,
chacun des trois radicaux nommés en dernier étant non substitué ou substitué par un ou plusieurs radicaux choisis dans le groupe constitué par halogéno et hydroxy, de préférence étant non substitué ou substitué par un ou plusieurs radicaux choisis dans le groupe halogéno, tels que fluoro et chloro, ou
un groupe cycloalkyle en C₃-C₆, qui est non substitué ou substitué par un ou plusieurs radicaux choisis dans le groupe constitué par halogéno et alkyle en C₁-C₄, ou
un groupe phényle qui est non substitué ou substitué par un ou plusieurs radicaux choisis dans le groupe constitué par halogéno, nitro, alkyle en C₁-C₄, halogénoalkyle(C₁-C₄), alcoxy en C₁-C₄, halogénoalcoxy(C₁-C₄), alcoxy(C₁-C₄)-alcoxy(C₁-C₄), alkyl(C₁-C₄)thio, [alcoxy(C₁-C₄)]-carbonyle et [halogéno-alcoxy(C₁-C₄)]-carbonyle, ou
un groupe alcanoyle en C₁-C₄ qui est non substitué ou substitué par un ou plusieurs radicaux choisis dans le groupe constitué par halogéno, tel que fluoro ou chloro, cyano, alcoxy en C₁-C₄, halogénoalcoxy(C₁-C₄) et alcoxy(C₁-C₂)-alcoxy(C₁-C₂), de préférence formyle, ou
un groupe [alcoxy(C₁-C₄)]-carbonyle qui est non substitué ou substitué par un ou plusieurs radicaux choisis dans le groupe constitué par halogéno, tel que fluoro ou chloro, ou
un groupe [cycloalcoxy(C₃-C₆)]-carbonyle qui est non substitué ou substitué par un ou plusieurs radicaux choisis dans le groupe constitué par halogéno et alkyle en C₁-C₄.

8. Composés ou leurs sels selon l'une quelconque des revendications 1 à 7, **caractérisés en ce que**
R⁵ représente un radical phényle qui est non substitué ou substitué par un ou plusieurs radicaux choisis dans le groupe constitué par halogéno, hydroxy, amino, nitro, carboxy, cyano, carbamoyle, alkyle en C₁-C₆, halogénoalkyle(C₁-C₆), alcoxy(C₁-C₄)-alkyle(C₁-C₄) alkyl(C₁-C₄)thio-alkyle(C₁-C₄), mono- et di-[alkyl(C₁-C₄)]-aminoalkyle, hydroxy-alkyle(C₁-C₄), carboxy-alkyle(C₁-C₄), cyano-alkyle(C₁-C₄), alcoxy en C₁-C₆ qui peut éventuellement être halogéné [= halogéno-alcoxy(C₁-C₆)], alcoxy(C₁-C₄)-alcoxy(C₁-C₄), alkyl(C₁-C₆)thio, [alcoxy(C₁-C₆)]-carbonyle, [halogénoalcoxy(C₁-C₆)]carbonyle, alcanoyle en C₁-C₆, halogénoalcanoyle(C₁-C₆), mono- et di-[alkyl(C₁-C₄)]-aminocarbonyle, mono- et di-[acyl(C₁-C₆)]-amino, mono- et di-[alkyl(C₁-C₄)]-amino, N-[acyl(C₁-C₆)]-N-[alkyl(C₁-C₆)]-amino, alkyl(C₁-C₆)sulfinyle, halogénoalkyl(C₁-C₆)sulfinyle, alkyl(C₁-C₆)-sulfonyle, halogénoalkyl(C₁-C₆)sulfonyle, cycloalkyle en C₃-C₆, cycloalkyloxy en C₃-C₆, phényle et phénoxy,
chacun des 4 radicaux nommés en dernier étant non substitué ou substitué par un ou plusieurs radicaux choisis dans le groupe constitué par halogéno, alkyle en C₁-C₄ et halogénoalkyle(C₁-C₄),
et deux substituants contigus pouvant former un cycle soudé à 5 ou 6 chaînons, qui est carbocyclique ou peut contenir encore 1 à 3 hétéroatomes formant le cycle, choisis dans le groupe N, 0 et S et qui est non substitué ou substitué par un ou plusieurs radicaux choisis dans le groupe constitué par halogéno et alkyle en C₁-C₆, ou
R⁵ représente un radical hétéroaromatique à 5 ou 6 chaînons, comportant 1 à 3 hétéroatomes formant le cycle, choisis dans le groupe constitué par N, O et S,
qui est non substitué ou substitué par un ou plusieurs radicaux choisis dans le groupe constitué par halogéno, hydroxy, amino, nitro, carboxy, cyano, carbamoyle, alkyle en C₁-C₆, halogénoalkyle(C₁-C₆), alcoxy(C₁-C₄)-alkyle(C₁-C₄) alkyl(C₁-C₄)thio-alkyle(C₁-C₄), mono- et di-[alkyl(C₁-C₄)]-aminoalkyle, hydroxy-alkyle(C₁-C₄), carboxy-alkyle(C₁-C₄), cyano-alkyle(C₁-C₄), alcoxy en C₁-C₆ qui peut éventuellement être halogéné [= halogéno-alcoxy(C₁-C₆)], alcoxy(C₁-C₄)-alcoxy(C₁-C₄), alkyl(C₁-C₆)thio, [alcoxy(C₁-C₆)]-carbonyle, [halogénoalcoxy(C₁-C₆)]carbonyle, alcanoyle en C₁-C₆, halogénoalcanoyle(C₁-C₆), mono- et di-[alkyl(C₁-C₄)]-aminocarbonyle, mono- et di-[acyl(C₁-C₆)]-amino, mono- et di-[alkyl(C₁-C₄)]-amino, N-[acyl(C₁-C₆)]-N-[alkyl(C₁-C₆)]-amino, alkyl(C₁-C₆)sulfinyle, halogénoalkyl (C₁-C₆)sulfinyle, alkyl(C₁-C₆)-sulfonyle, halogénoalkyl(C₁-C₆)sulfonyle, cycloalkyle en C₃-C₆, cycloalkyloxy en C₃-C₆, phényle et phénoxy,
chacun des 4 radicaux nommés en dernier étant non substitué ou substitué par un ou plusieurs radicaux choisis dans le groupe constitué par halogéno, alkyle en C₁-C₄ et halogénoalkyle(C₁-C₄),
et deux substituants contigus pouvant former un cycle soudé à 5 ou 6 chaînons, qui est carbocyclique ou peut contenir encore 1 à 3 hétéroatomes formant le cycle, choisis dans le groupe N, O et S et qui est non substitué ou substitué par un ou plusieurs radicaux choisis dans le groupe constitué par halogéno et alkyle en C₁-C₆,

9. Composés ou leurs sels selon l'une quelconque des revendications 1 à 8, **caractérisés en ce que**
(R⁶)ₙ représente n substituants R⁶, R⁶, dans le cas où n est égal à 1, ou chacun des substituants R⁶ indépendamment les uns des autres, dans le cas où n est supérieur à 1, représentant un radical halogéno, cyano, alkyle en C₁-C₄, halogéno-alkyle(C₁-C₄), alcoxy en C₁-C₄, alkyl(C₁-C₄)thio, alkyl(C₁-C₄)sulfinyle, halogénoalkyl(C₁-C₄)-sulfinyle, alkyl(C₁-C₄)sulfonyle, alcoxy(C₁-C₄)-carbonyle ou halogénoalkyl(C₁-C₄)sulfonyle, et
n représente 0, 1 ou 2.

10. Procédé pour la préparation d'un composé de formule (I) tel qu'il est défini selon l'une quelconque des revendications 1 à 9, ou d'un sel d'un tel composé, **caractérisé en ce que**
(a) on fait réagir un composé de formule (II),
H₂N-NH-Het(R⁶)ₙ (II)
dans laquelle Het et (R⁶)ₙ sont tels que définis dans la formule (I),
avec un composé de formule (III), 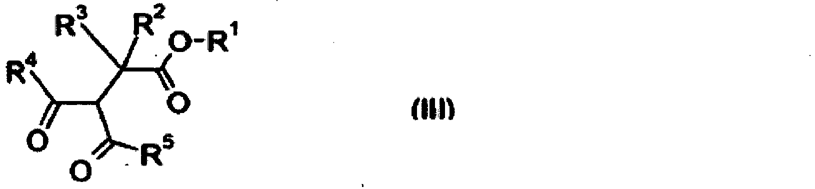 dans laquelle R¹, R², R³, R⁴ et R⁵ sont tels que définis dans la formule (I),
pour aboutir au composé de formule (I) ou à un sel d'un tel composé,
(b) dans le cas où R¹ dans la formule (I) est différent d'un atome d'hydrogène, on fait réagir un composé de formule (I') 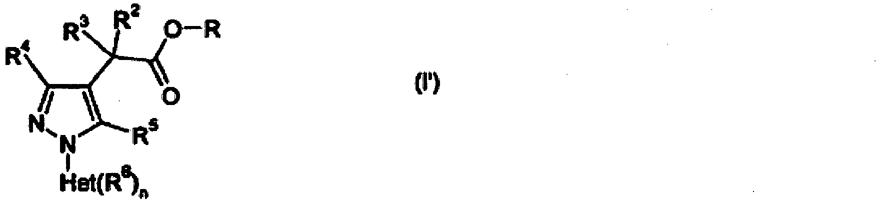 dans laquelle Het, R², R³, R⁴, R⁵ et R⁶ sont tels que définis dans la formule (I) et
R représente un radical différent d'un atome d'hydrogène, différent du radical R¹, qui est choisi dans le groupe des radicaux tels qu'ils sont définis pour R¹,
ou un anhydride, halogénure d'acide ou ester activé du composé de formule (I'), où R représente H,
avec un composé de formule (IV),
R¹-OH (IV)
dans laquelle R¹ est tel que défini dans la formule (I),
pour aboutir au composé de formule (I)
ou
(c) dans le cas où R¹ dans la formule (I) est différent d'un atome d'hydrogène, on estérifie un composé de formule (I") 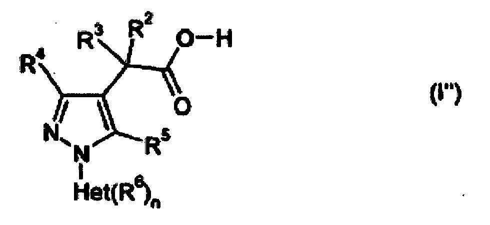 dans laquelle Het, R², R³, R⁴, R⁵ et R⁶ sont tels que définis dans la formule (I),
avec un composé de formule (IV),
R¹-OH (IV)
dans laquelle R¹ est tel que défini dans la formule (I),
pour aboutir au composé de formule (I)
ou
(d) dans le cas où l'on prépare le composé de formule (I) dans lequel R¹ = H, ou un sel d'un tel composé, on hydrolyse un composé de formule (I') [voir définition dans la variante (b)] pour aboutir au composé de formule (I) ou à un sel d'un tel composé,
ou
g) on fait réagir un composé de formule générale (XI) avec un dérivé de bore de formule (XII) en présence d'un sel de Cu(I) ou Cu(II) et d'une base organique, 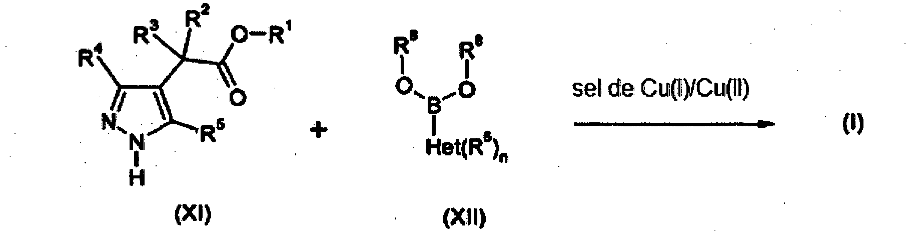 dans les formules (XI) et (XII) Het, R¹, R², R³, R⁴, R⁵, R⁶ ayant les significations indiquées selon la formule (I), et R⁸ représentant H ou un groupe alkyle en C₁-C₆ ou les deux radicaux alkyle R⁸ étant reliés l'un à l'autre pour former un cycle, ou
h) on fait réagir un composé de formule générale (XI) avec un composé de formule (XIII), dans laquelle R⁶ a la signification indiquée précédemment selon la formule (I), en présence d'un système catalyseur/ligand approprié, avec une base appropriée et dans un solvant convenable, pour aboutir au composé de formule (I) ou à un sel d'un tel composé, 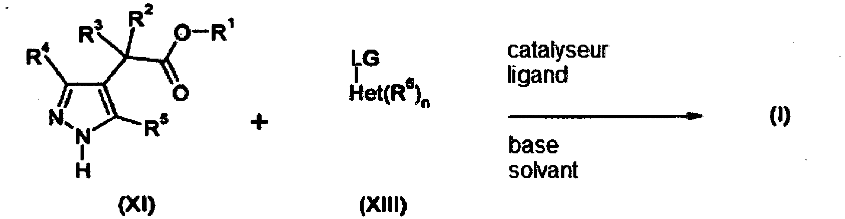 dans les formules (XI) et (XIII) les radicaux Het, R¹, R², R³, R⁴, R⁵, R⁶ ayant les significations indiquées selon la formule (I), et LG représentant un groupe partant, ou
i) on fait réagir un composé de formule générale (XV) avec un composé de formule générale (III) en présence d'un acide, pour aboutir au composé de formule (I) ou à un sel d'un tel composé, 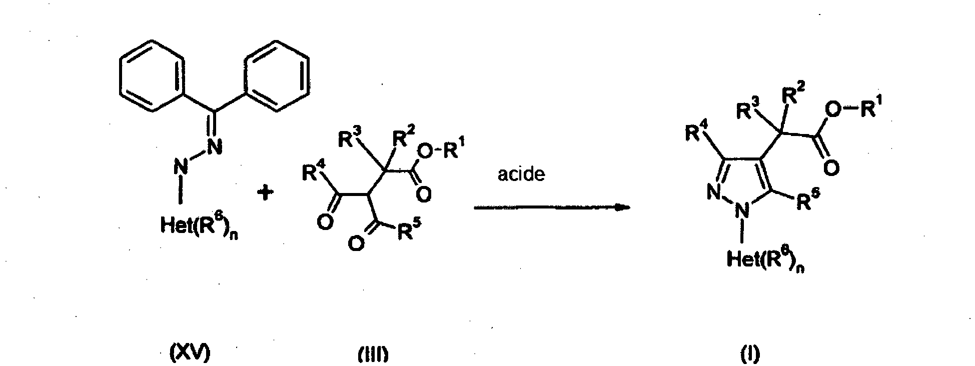 dans les formules (XV) et (III) les radicaux Het, R¹, R², R³, R⁴, R⁵, R⁶ étant tels que définis dans la formule (I) et LG représentant un groupe partant,
ou
j) on fait réagir un composé de formule générale (XVI), où R⁶ est tel que défini dans la formule (I), avec de l'azodicarboxylate de di-tert-butyle (DBAD, XVII) en présence d'un sel de cuivre, pour aboutir à un composé de formule (XVIII), dans laquelle R⁶ est tel que défini dans la formule (I), qui est ensuite converti, via un composé de formule (II) ou ses sels, formule dans laquelle R⁶ est tel que défini dans la formule (I), et selon le procédé a), en des composés de formule (I) : 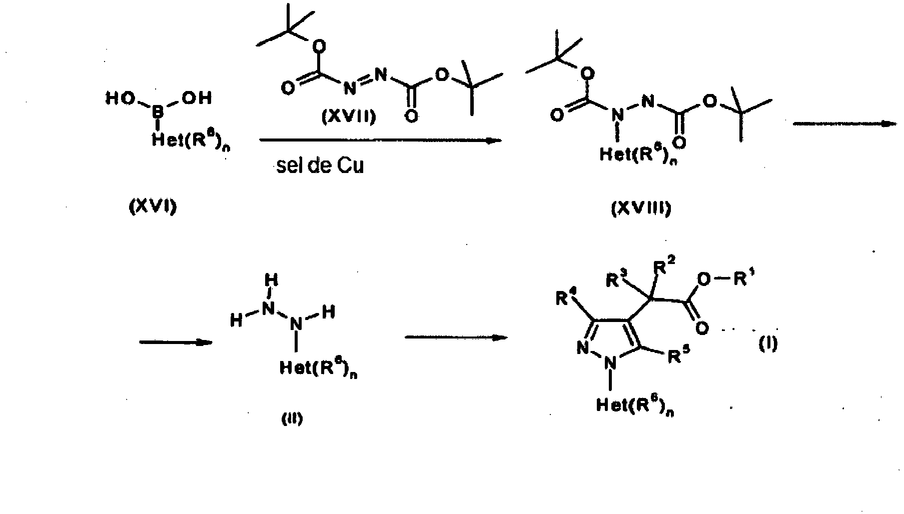 dans les formules (XVI), (XVII), (XVIII) et (II) les radicaux Het, R¹, R², R³, R⁴, R⁵, R⁶ étant tels que définis dans la formule (I).

11. Composition herbicide ou régulatrice de croissance végétale, **caractérisée en ce qu'**elle contient un ou plusieurs composés de formule (I) ou sel(s) de tel(s) composé(s), tels qu'il(s) est/sont défini(s) selon l'une quelconque des revendications 1 à 9, et contient des adjuvants de formulation usuels dans la protection des plantes.

12. Procédé pour la lutte contre des plantes nuisibles ou pour la régulation de la croissance de plantes, **caractérisé en ce qu'**on applique sur les plantes, semences de plantes, le sol dans lequel ou sur lequel poussent les plantes, ou l'aire de culture une quantité efficace d'un ou de plusieurs composés de formule (I) ou sel(s) de tel(s) composé(s), tel(s) qu'il(s) est/sont défini(s) selon l'une quelconque des revendications 1 à 9.

13. Procédé selon la revendication 12, **caractérisé en ce qu'**on utilise les composés de formule (I) ou leurs sels pour la lutte contre des pantes nuisibles ou pour la régulation de la croissance dans des cultures de plantes utiles ou ornementales.

14. Procédé selon la revendication 13, **caractérisé en ce que** les plantes cultivées sont des plantes cultivées transgéniques.

15. Utilisation des composés de formule (I) ou de leurs sels selon l'une quelconque des revendications 1 à 9, en tant qu'herbicides ou régulateurs de croissance végétale.
